# EUROPEAN PATENT APPLICATION

(11) **EP 4 831 169 A1**
(43) Date of publication of application: **28.10.2026**
(21) Application number: 25742254.3
(22) Date of filing: 16.01.2025
(51) Int. Cl.: C07D 403/14, A61K 31/4184, A61P 35/00, A61P 29/00, A61P 37/00, C07D 405/14, C07D 401/14, C07D 413/14, C07D 403/04, C07D 471/04

(54) **NOVEL COMPOUND USED AS CBP/P300 INHIBITOR, AND PHARMACEUTICAL COMPOSITION FOR PREVENTING OR TREATING CANCER, INFLAMMATORY DISORDERS, OR AUTOIMMUNE DISEASES COMPRISING SAME AS ACTIVE INGREDIENT**

(30) Priority: 17.01.2024 KR 20240007499
(71) Applicant: LG Chem, Ltd., Seoul 07336 (KR)
(72) Inventor: NOH, Seungjoo, Daejeon 34122 (KR); KANG, Esder, Daejeon 34122 (KR); KIM, Rira, Daejeon 34122 (KR); PAK, Gyungah, Daejeon 34122 (KR); KIM, Byung Gyu, Daejeon 34122 (KR); LEE, Soyun, Daejeon 34122 (KR); HWANG, Youson, Daejeon 34122 (KR)
(74) Representative: Hoffmann Eitle
(86) International application number: PCT/KR2025/000963
(87) International publication number: WO 2025/155115

(57) **Abstract**

The present invention provides: a novel compound used as a CBP/p300 inhibitor, a method for preparing same; an isomer thereof, a pharmaceutically acceptable salt thereof, a hydrate thereof, or a solvate thereof; and a pharmaceutical composition for preventing or treating cancer, inflammatory disorders or autoimmune diseases, comprising at least one of these as an active ingredient.

## Description

### Technical Field

This application claims the benefit of priority to Korean Patent Application No. 2024-0007499, filed on January 17, 2024, the entire disclosure of which is incorporated herein by reference. This disclosure relates to a novel compound used as a CBP/p300 inhibitor, and a pharmaceutical composition for preventing or treating cancer, inflammatory disorders, or autoimmune diseases containing the same as an active ingredient.

### Background Art

CBP [CREB (cyclic-AMP response element binding protein) binding protein] and the paralog thereof, p300, are transcription coactivators composed of multiple functional domains including a histone acetyl-transferase (HATs) domain and a bromodomain (BRD). CBP and p300 are major epigenetic modulators that recognize and induce histone lysine acetylation at specific loci such as enhancers and promoters on chromatin, thereby regulating the expression of various genes.

In consideration of high structural similarity and functional redundancy, overexpression or mutations of CBP and p300, often referred to as CBP/p300, have been associated with various diseases, especially malignant tumors. For example, CBP and p300 bind to chromatin through BRD and HAT and acetylate histones at enhancer sites of oncogenes, thereby relaxing a chromatin structure and allowing transcriptional functional proteins including RNA polymerase II to assemble. As a result, tumor cells increase the expression of oncogenes such as MYC and IRF4 in tumor cells, thereby forming and growing tumors. Inhibition of the HAT domain causes an overall decrease in acetylation, which is important for transcription, and acts in the same way not only on tumor cells but also on normal cells, especially stem cells. Accordingly, it has been reported that it is difficult to secure a therapeutic window, and toxicity may be induced, so it is expected that the concerns may be overcome through a bromodomain inhibitor strategy.

Accordingly, various studies have been conducted to develop inhibitors that selectively inhibit the bromodomain of CBP/p300 and reduce the transcription of oncogenes activated in tumor cells. For example, CCS1477, a CBP/p300 bromodomain inhibitor developed by CellCentric, is in phase 1b/2a clinical trials for the treatment of hematological malignancies and advanced drug-resistant prostate cancer, and various CBP/p300 activity modulators are also disclosed in Patent Documents 1 to 3 below.

However, the development of effective and selective therapeutic agents targeting CBP/p300 is still a difficult task. Therefore, the development of more chemotypes of CBP/p300 inhibitors is needed.
[Patent Document 1] WO2018073587
[Patent Document 2] WO2019191667
[Patent Document 3] WO2017205538

### Disclosure of Invention

### Technical Goals

The present disclosure has a purpose of providing a novel compound used as a CBP/p300 inhibitor.

The present disclosure also has another purpose of providing an isomer of the compound, a pharmaceutically acceptable salt thereof, a hydrate thereof, or a solvate thereof.

The present disclosure also has another purpose of providing a pharmaceutical composition for preventing or treating cancer, inflammatory disorders, or autoimmune diseases, which includes at least one of the compound, the isomer thereof, the pharmaceutically acceptable salt thereof, the hydrate thereof, and the solvate thereof, as an active ingredient.

### Technical solutions

To solve the task, an aspect of the present disclosure relates to a compound represented by the following Formula I: 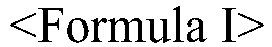 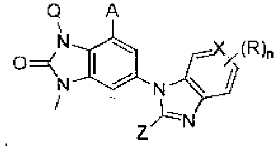 where
X is C or N,
n is an integer from 0 to 3, where if n is an integer of 2 or more, Rs are the same or different,
Rs are each independently C₁₋₇ alkyl, C₃₋₇ cycloalkyl, C₁₋₅ haloalkyl, C₆₋₁₀ aryl, 5- to 9-membered heteroaryl, 3- to 14-membered heterocycloalkyl, 4- to 9-membered heterocycloalkenyl, halogen, cyano, carbonyl, formaldoxime, NR^{a}R^{b}, SO₂NHR^{c}, CONR^{d}R^{e}, CO₂R^{f}, NHCOR^{g} or OR^{h}, and R^{a} to R^{f} are each independently selected from the group consisting of hydrogen, C₁₋₅ alkyl, C₃₋₇ cycloalkyl, C₁₋₄ haloalkyl, C₆₋₁₀ aryl, halogen, carbonyl and a Boc group,
Q is hydrogen, C₁₋₇ alkyl or C₃₋₇ cycloalkyl,
A is hydrogen, C₁₋₈ alkyl, C₂₋₈ alkenyl, C₂₋₆ alkynyl, C₃₋₁₀ cycloalkyl, C₄₋₁₀ cycloalkenyl, C₆₋₁₀ aryl, 3- to 16-membered heterocycloalkyl, 3- to 16-membered heterocycloalkenyl, 5- to 9-membered heteroaryl, halogen, carbonyl, NRⁱR^{j} or OR^{k}, and Rⁱ to R^{k} are each independently selected from the group consisting of hydrogen, C₁₋₈ alkyl, C₃₋₁₀ cycloalkyl, C₆₋₁₀ aryl, 5- to 10-membered heteroaryl, 3- to 10-membered heterocycloalkyl and a Boc group, and
Z is C₁₋₈ alkyl, C₃₋₇ cycloalkyl, C₆₋₁₀ aryl, 5- to 13-membered heteroaryl or 3- to 8-membered heterocycloalkyl,
where R, R^{a} to R^{k}, Q, A and Z are each substituted or unsubstituted with Substituent Group I.

Another aspect of the present disclosure relates to an isomer of the compound above, a pharmaceutically acceptable salt thereof, a hydrate thereof or a solvate thereof.

Another aspect of the present disclosure relates to a pharmaceutical composition for preventing or treating cancer, inflammatory disorders or autoimmune diseases, the composition including at least one among the compound above, an isomer thereof, a pharmaceutically acceptable salt thereof, a hydrate thereof and a solvate thereof, as an active ingredient.

### Effects

According to the present disclosure, it has been found that the compound represented by Formula I has excellent CBP/p300 inhibitory activity. Therefore, the compound represented by Formula I of the present disclosure; an isomer of the compound, a pharmaceutically acceptable salt thereof, a hydrate thereof or a solvate thereof; and a pharmaceutical composition including at least one of them as an active ingredient may be useful for inhibition, such as the prevention or treatment of cancer, inflammatory disorders or autoimmune diseases.

The effects of the present disclosure are not limited to the above-described effects, and are understood to include all effects that may be inferred from the configuration of the invention described in the description or claims of the present disclosure.

### Best Mode for Carrying Out the Invention

Hereinafter, in order to assist the understanding of the present disclosure, the present disclosure will be described in more detail. In this case, the terms or words used in this specification and claims should not be interpreted as limited to usual or dictionary meanings, and should be interpreted as meanings and concepts that conform to the technical idea of the present disclosure based on the principle that the inventor may appropriately define the concept of the term to explain his own invention in the best way.

The present disclosure may provide a compound represented by the following Formula I:

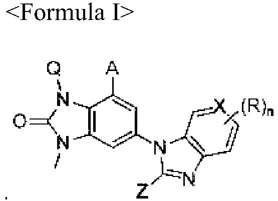

where X may be C or N, n may be an integer from 0 to 3, where if n is an integer of 2 or more, Rs may be the same or different, Rs may be each independently C₁₋₇ alkyl, C₃₋₇ cycloalkyl, C₁₋₅ haloalkyl, C₆₋₁₀ aryl, 5- to 9-membered heteroaryl, 3- to 14-membered heterocycloalkyl, 4- to 9-membered heterocycloalkenyl, halogen, cyano, carbonyl, formaldoxime, NR^{a}R^{b}, SO₂NHR^{c}, CONR^{d}R^{e}, CO₂R^{f}, NHCOR^{g} or OR^{h}, and R^{a} to R^{f} may be each independently selected from the group consisting of hydrogen, C₁₋₅ alkyl, C₃₋₇ cycloalkyl, C₁₋₄ haloalkyl, C₆₋₁₀ aryl, halogen, carbonyl and a Boc group, Q may be hydrogen, C₁₋₇ alkyl or C₃₋₇ cycloalkyl, A may be hydrogen, C₁₋₈ alkyl, C₂₋₈ alkenyl, C₂₋₆ alkynyl, C₃₋₁₀ cycloalkyl, C₄₋₁₀ cycloalkenyl, C₆₋₁₀ aryl, 3- to 16-membered heterocycloalkyl, 3- to 16-membered heterocycloalkenyl, 5- to 9-membered heteroaryl, halogen, carbonyl, NRⁱR^{j} or OR^{k}, and Rⁱ to R^{k} may be each independently selected from the group consisting of hydrogen, C₁₋₈ alkyl, C₃₋₁₀ cycloalkyl, C₆₋₁₀ aryl, 5- to 10-membered heteroaryl, 3- to 10-membered heterocycloalkyl and a Boc group, and Z may be C₁₋₈ alkyl, C₃₋₇ cycloalkyl, C₆₋₁₀ aryl, 5- to 13-membered heteroaryl or 3- to 8-membered heterocycloalkyl, where R, R^{a} to R^{k}, Q, A and Z may be each substituted or unsubstituted with Substituent Group I.

Substituent Group I: substituted or unsubstituted C₁₋₁₀ alkyl, substituted or unsubstituted C₂₋₁₀ alkenyl, substituted or unsubstituted C₂₋₁₀ alkynyl, substituted or unsubstituted C₃₋₂₀ carbocycle, substituted or unsubstituted C₃₋₂₀ cycloalkyl, substituted or unsubstituted C₆₋₃₀ aryl, substituted or unsubstituted 3- to 30-membered heterocycle, substituted or unsubstituted C₁₋₁₀ alkoxy, substituted or unsubstituted C₂₋₁₀ alkenyloxy, substituted or unsubstituted C₃₋₁₀ cycloalkyloxy, substituted or unsubstituted C₆₋₃₀ aryloxy, substituted or unsubstituted 3- to 30-membered heterocycleoxy, substituted or unsubstituted C₁₋₁₀ alkylketone, substituted or unsubstituted C₂₋₁₀ alkenylketone, substituted or unsubstituted C₃₋₁₀ cycloalkylketone, substituted or unsubstituted C₆₋₃₀ arylketone, substituted or unsubstituted 3- to 30-membered heterocycleketone, substituted or unsubstituted C₁₋₁₀ alkylthio, substituted or unsubstituted C₂₋₁₀ alkenylthio, substituted or unsubstituted C₃₋₁₀ cycloalkylthio, substituted or unsubstituted C₆₋₃₀ arylthio, substituted or unsubstituted 3- to 30-membered heterocyclethio, substituted or unsubstituted carbonyl, carboxy, halogen, substituted or unsubstituted C₁₋₁₀ haloalkyl, hydroxy, substituted or unsubstituted amino, imine, cyano, nitro, amide, thiol, sulfone and phosphoric acid, where a C₁₋₁₀ alkyl moiety may be interposed by a heteroatom group selected from -O-, -S-, -SO-, -SO₂-, -NR^{g}- (R^{g} is hydrogen or substituted or unsubstituted C₁₋₁₀ alkyl), -N=, =N-, -POR^{g}- and -PO₄R^{g}-, and a heterocycle moiety may include at least one of S, N, P and O as a ring-forming atom.

In defining the compound represented by Formula I throughout this specification, the defined concepts as follows are used. The definitions apply to terms used individually or as part of a larger group throughout this specification, unless otherwise specified. Terms and abbreviations used herein have their original meanings unless otherwise defined.

In this specification, the term "substituted" may mean that a hydrogen atom bonded to the compound or each substituent listed in the substituent group is replaced and bonded with an arbitrary substituent. In this case, the position to be substituted is not limited as long as the position is where a hydrogen atom bonded to the compound or each substituent listed in the substituent group exists, i.e., the position where the hydrogen atom may be substituted with a substituent. If two or more are substituted, the two or more substituents may be the same or different from each other. In contrast, "unsubstituted" may mean that no hydrogen atom bonded to a carbon atom is replaced with an arbitrary substituent.

Arbitrary substituent applicable to the "substitution" may be appropriately selected from the substituents listed in the substituent group as described in this specification, but the scope of the substituents available for the substitution is not limited to the substituent group.

If a substituent is not separately described or mentioned in the chemical formulas described in this specification, it may be considered that hydrogen is bonded, and in some cases, the substituent may be absent depending on the ring-forming elements of a heterocycle.

In this specification, the term "alkyl" by itself or as a part of another substituent means a straight or branched monovalent hydrocarbon having an indicated number of carbon atoms (i.e., C₁₋₁₀ means 1 to 10 carbon atoms), unless otherwise referred to.

Examples of the alkyl include methyl, ethyl, n-propyl, isopropyl, n-butyl, isobutyl, tert-butyl, sec-butyl, 1-methyl-butyl, 1-ethyl-butyl, n-pentyl, isopentyl, neopentyl, tert-pentyl, n-hexyl, 1-methylpentyl, 2-methylpentyl, 4-methyl-2-pentyl, 3,3-dimethylbutyl, 2-ethylbutyl, n-heptyl, 1-methylhexyl, n-octyl, tert-octyl, 1-methylheptyl, 2-ethylhexyl, 2-propylpentyl, n-nonyl, 2,2-dimethylheptyl, 1-ethylpropyl, 1,1-dimethyl-propyl, isohexyl, 2-methylpentyl, 4-methylhexyl and 5-methylhexyl, but these are non-limiting examples and the scope of the present disclosure is not limited thereto.

In this specification, the term "alkenyl" may be a straight or branched chain. Alkenyl includes, for example, ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl or 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl, 1-ethyl-2-methyl-2-propenyl, 1-heptenyl, 2-heptenyl, 3-heptenyl, 1-octenyl, 2-octenyl, 3-octenyl, 4-octenyl, 1-nonenyl, 2-nonenyl, 3-nonenyl, 4-nonenyl, 1-decenyl, 2-decenyl, 3-decenyl, 4-decenyl, and 5-decenyl, but is not limited thereto.

In this specification, the term "alkynyl" may be a straight or branched chain. Alkynyl includes, for example, acetylenyl and trimethylsilylacetylenyl, but is not limited thereto.

In this specification, the term "cycloalkyl" means a monovalent aliphatic carbocycle in which one hydrogen atom is missing from an arbitrary position among the carbon atoms constituting the ring. Examples of cycloalkyl include, but are not limited to, cyclopropyl, cyclobutyl, cyclopentyl, 3-methylcyclopentyl, 2,3-dimethylcyclopentyl, cyclohexyl, cyclohexylene 3-methylcyclohexyl, 4-methylcyclohexyl, 2,3-dimethylcyclohexyl, 3,4,5-trimethylcyclohexyl, 4-tert-butylcyclohexyl, cycloheptyl, and cyclooctyl.

In this specification, the term "cycloalkenyl" means a cyclic hydrocarbon having a double bond. Examples of cycloalkenyl include, but are not limited to, cyclohexenyl. In this specification, the term "aryl" refers to a ring containing a multiply unsaturated, typically aromatic hydrocarbon ring, where the aryl may be monocycle or polycycle. The case of polycycle includes a form in which only aromatic hydrocarbon rings are fused and a form in which an aliphatic hydrocarbon ring is fused with an aromatic hydrocarbon. Examples of the aryl include monocyclic aryls such as phenyl, biphenyl, terphenyl and quaterphenyl, and polycyclic aryls such as naphthyl, anthracenyl, phenanthrenyl, pyrenyl and benzopyrenyl.

In this specification, the term "heterocycle" refers to a ring including, together with carbon atoms, 1 to 5 heteroatoms other than carbon atoms, i.e., O, N, P and S, as ring-forming atoms. Here, the heterocycle may be aromatic, aliphatic, monocyclic or polycyclic and include, but is not limited to, thiophenyl, furanyl, pyrrolyl, imidazolyl, thiazolyl, oxazolyl, oxadiazolyl, pyridinyl, bipyridinyl, pyrimidinyl, triazinyl, triazolyl, pyridazinyl, pyrazinyl, pyrido pyrimidyl, pyrido pyrazinyl, pyrazino pyrazinyl, isoxazolyl and thiadiazolyl, but the scope of the present disclosure is not limited thereto.

In this specification, the term "heteroaryl" means an aromatic heterocycle and may be a monocycle or polycycle. The polycycle includes not only a form in which multiple aromatic rings are fused, but also a form in which an aromatic ring and an aliphatic ring are fused. Examples of the heteroaryl include pyrrolyl, pyrazolyl, imidazolyl, triazolyl, oxazolyl, isoxazolyl, thiazolyl, tetrazolyl, isothiazolyl, furyl, thienyl, pyridinyl, pyridyl N-oxide, pyrazinyl, pyrimidinyl, pyridazinyl, indolyl, benzothiazolyl, benzoxazolyl, benzothienyl, quinolinyl, quinazolinyl, quinoxalinyl, phthalazinyl, isoquinolinyl, thiophenyl, acridyl, indazolyl, carbazolyl, benzimidazolyl, benzocarbazolyl, benzofuranyl, benzothiophenyl, dibenzothiophenyl, pyrazolopyrimidinyl, oxadiazolyl or azaindolyl, but the scope of the present disclosure is not limited thereto.

In this specification, the term "heterocycloalkyl" means partially or fully saturated hydrocarbon containing one or more heteroatoms selected from O, N, P and S as a ring-forming atom and forming a monocycle or a fused ring. Preferably, the heteroatom may be 1, 1 to 2, or 1 to 3. For example, piperazinyl, piperazinedionyl, piperidinyl, pyrrolidinyl, morpholinyl, thiomorpholinyl, thiomorpholinedioxadinyl, oxaazaspiroheptyl, hexahydrofuropyrrolyl, oxaazabicycloheptyl, imidazolidinyl, imidazolidinonyl, lactamyl, tetrahydropyranyl, tetrahydrofuranyl, diazabicyclooctanyl, oxaindolyl, benzoxazinonyl, oxacyclobutanyl, azetidinyl, or diazaspiroctanyl may be included, without limitation.

In this specification, the term "heterocycloalkenyl" means unsaturated hydrocarbon including one or more heteroatoms selected from O, N, P and S as ring-forming atoms and forming a monocycle or a fused ring. Preferably, the heteroatom may be 1, 1 to 2, or 1 to 3. For example, tetrahydropyridinyl, dihydropyrazinyl, and dihydroquinoline may be included, without limitation.

In this specification, the term "halogen" includes, for example, fluoro, chloro, bromo or iodo, but is not limited thereto.

In this specification, the term "carbonyl" is a substituent which may be represented by "-C(=O)R^{car}", where R^{car} may be, but is not limited to, hydrogen, substituted or unsubstituted, C₁₋₁₀ alkyl, C₂₋₁₀ alkenyl, substituted or unsubstituted C₃₋₁₀ carbocycle, C₃₋₁₀ cycloalkyl, C₆₋₃₀ aryl, 3- to 30-membered heterocycle, or C₁₋₁₀ haloalkyl, each of which is defined as described above.

In this specification, the term "Boc group" means a tert-butyloxycarbonyl protecting group.

In this specification, the term "amino" is intended to encompass monovalent primary amine, monovalent secondary amine, and monovalent tertiary amine. That is, amino refers to both a monovalent group in which two hydrogen atoms are bonded to a nitrogen atom and a monovalent group in which at least one hydrogen atom is substituted with another substituent. In the above, the group in which two hydrogen atoms are bonded to a nitrogen atom may be an unsubstituted amino, and the group in which at least one hydrogen atom is substituted with another substituent may be a substituted amino. Meanwhile, for dialkylamino, alkyl moieties may be the same or different.

In this specification, the term "imine or imino" is intended to encompass monovalent primary ketimines (-C(=NH)Rⁱ⁻¹), secondary ketimines (-C(N=Rⁱ⁻²)Rⁱ⁻¹), primary aldimines (-C(=NH)H), and secondary aldimines (-C(=NRⁱ⁻¹)H), where Rⁱ⁻¹ and Rⁱ⁻² may be, but are not limited to, each independently, C₁₋₁₀ alkyl, C₂₋₁₀ alkenyl, C₃₋₁₀ carbocycle, C₃₋₁₀ cycloalkyl, C₆₋₃₀ aryl, 3- to 30-membered heterocycle, or C₁₋₁₀ haloalkyl, and each of which may be substituted or unsubstituted, but is not limited thereto.

In this specification, the terms "carbocycle" and "carbocyclic" mean a ring in which ring-forming atoms are composed of carbon. The carbocycle may be aliphatic or aromatic, saturated or unsaturated, and monocyclic or polycyclic. The polycyclic ring may be a fused, bridged, or spiro polycyclic ring. A monocyclic carbocycle may have 3 to 17 carbon atoms, specifically 3 to 14 carbon atoms, more specifically 3 to 10 carbon atoms, and particularly specifically 3 to 7 carbon atoms.

In this specification, the term "alkoxy" refers to a substituent having oxygen bonded to alkyl and may be a straight, branched, or cyclic chain. Examples of alkoxy include, but are not limited to, methoxy, ethoxy, n-propoxy, isopropoxy, i-propyloxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy, and monohalogenated and polyhalogenated variants thereof.

In this specification, the term "oxy" refers to a substituent which may be represented by "-OR^{oxy}", where R^{oxy} may be, but is not limited to, substituted or unsubstituted, C₁₋₁₀ alkyl, C₂₋₁₀ alkenyl, C₃₋₁₀ carbocycle, C₃₋₁₀ cycloalkyl, C₆₋₃₀ aryl, 3- to 30-membered heterocycle, or C₁₋₁₀ haloalkyl.

In this specification, the term "thio" is a substituent which may be represented by "-SR^{thio}", where R^{thio} may be, but is not limited to, substituted or unsubstituted, C₁₋₁₀ alkyl, C₂₋₁₀ alkenyl, C₃₋₁₀ carbocycle, C₃₋₁₀ cycloalkyl, C₆₋₃₀ aryl, 3- to 30-membered heterocycle, or C₁₋₁₀ haloalkyl.

In this specification, the term "haloalkyl" is synonymous with "halogenated alkyl" and "halogen-substituted alkyl", and may mean alkyl in which an arbitrary hydrogen atom in the alkyl is substituted with an arbitrary halogen atom. For example, -CF₃, -CH₂Cl, -CH₂CF₃, and -C(Cl₂)CF₃ are haloalkyl, but the scope of the present disclosure is not limited thereto.

In this specification, the term "amide" is a substituent which may be represented, for example, by R-C(=O)-NR'R", where R, R' and R" are each, but are not limited to, hydrogen, substituted or unsubstituted C₁₋₁₀ alkyl, substituted or unsubstituted C₃₋₁₀ carbocycle, substituted or unsubstituted C₃₋₁₀ cycloalkyl, substituted or unsubstituted C₆₋₃₀ aryl, substituted or unsubstituted 3- to 30-membered heterocycle, or substituted or unsubstituted C₁₋₁₀ haloalkyl.

In this specification, the term "sulfonyl" refers to a substituent which may be represented by "-S(=O)₂R^{s1}", where R^{s1} may be, but is not limited to, substituted or unsubstituted, C₁₋₁₀ alkyl, C₂₋₁₀ alkenyl, C₃₋₁₀ carbocycle, C₃₋₁₀ cycloalkyl, C₆₋₃₀ aryl, 3- to 30-membered heterocycle, or C₁₋₁₀ haloalkyl.

In this specification, the term "thioamide" refers to a substituent which may be represented by "-C(=S)NR^{ta}₂", "-C(=S)NHR^{ta}" and "-C(=S)NH₂", where R^{ta}s are independent from each other and each may be, but are not limited to, substituted or unsubstituted, C₁₋₁₀ alkyl, C₂₋₁₀ alkenyl, C₃₋₁₀ carbocycle, C₃₋₁₀ cycloalkyl, C₆₋₃₀ aryl, 3- to 30-membered heterocycle, or C₁₋₁₀ haloalkyl.

In this specification, the term "sulfinyl" refers to a substituent which may be represented by "-S(=O)R^{s2}", where R^{s2} may be, but is not limited to, substituted or unsubstituted, C₁₋₁₀ alkyl, C₂₋₁₀ alkenyl, C₃₋₁₀ carbocycle, C₃₋₁₀ cycloalkyl, C₆₋₃₀ aryl, 3- to 30-membered heterocycle, or C₁₋₁₀ haloalkyl, and each of which is defined as described above. Sulfinyl may also be used interchangeably with "thiocarbonyl", and in a narrower sense may also be referred to as alkylthiocarbonyl in a case where R^{s2} is alkyl.

In this specification, the term "phosphoric acid" refers to a substituent which may be represented by "-OP(=O)(OH)₂" or "-OP(=O)(OH)(OR^{P})", where R^{P} may be, but is not limited to, substituted or unsubstituted, C₁₋₁₀ alkyl, C₂₋₁₀ alkenyl, C₃₋₁₀ carbocycle, C₃₋₁₀ cycloalkyl, C₆₋₃₀ aryl, 3- to 30-membered heterocycle, or C₁₋₁₀ haloalkyl.

In this specification, the term "ester" is a substituent which may be represented by "-OC(=O)R^{ester}", where R^{ester} may be, but is not limited to, substituted or unsubstituted, C₁₋₁₀ alkyl, C₃₋₁₀ carbocycle, C₃₋₁₀ cycloalkyl, C₆₋₃₀ aryl, 3- to 30-membered heterocycle, or C₁₋₁₀ haloalkyl.

In an embodiment, R, R^{a} to R^{k}, Q, A and Z each may be substituted or unsubstituted with Substituent Group I-1.

Substituent Group I-1: substituted or unsubstituted C₁₋₆ alkyl, substituted or unsubstituted C₁₋₅ alkenyl, substituted or unsubstituted C₂₋₇ alkynyl, substituted or unsubstituted C₃₋₆ cycloalkyl, substituted or unsubstituted C₁₋₅ haloalkyl, substituted or unsubstituted C₆₋₈ aryl, substituted or unsubstituted carbonyl, substituted or unsubstituted 3- to 9-membered heterocycloalkyl, halogen, cyano, a Boc group, OR^{p1}, NR^{p2}R^{p3}, CONR^{p4}R^{p5}, NHCOR^{p6} or CO₂R^{p7}, where R^{p1} to R^{p7} may be each independently hydrogen, substituted or unsubstituted methyl, substituted or unsubstituted ethyl, substituted or unsubstituted propyl, substituted or unsubstituted propenyl, substituted or unsubstituted mesylate, substituted or unsubstituted carbonyl and a Boc group.

In an embodiment, the compound of Formula I may be represented by any one of the following Formula I-1 or I-2:

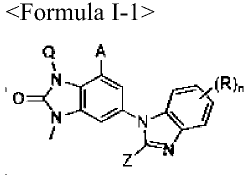

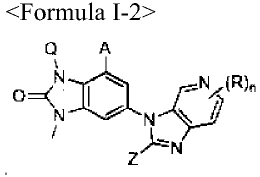

where n, R, Q, A and Z may be the same as defined above.

In an embodiment, the compound of Formula I-1 may be represented by any one of the following Formula I-1-1 or I-1-2: 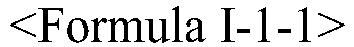 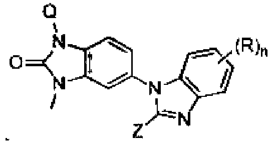 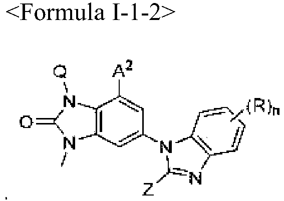 where A² may be C₁₋₈ alkyl, C₂₋₈ alkenyl, C₂₋₆ alkynyl, C₃₋₁₀ cycloalkyl, C₄₋₁₀ cycloalkenyl, C₆₋₁₀ aryl, 3- to 16-membered heterocycloalkyl, 5- to 9-membered heteroaryl, halogen, carbonyl, NRⁱR^{j} or OR^{k}, which is substituted or unsubstituted with substituent group I, and Rⁱ to R^{k}, n, R, Q and Z may be the same as defined above.

In an embodiment, A² may be chloro, hydroxy, substituted or unsubstituted carbonyl, substituted or unsubstituted ethyl, substituted or unsubstituted propyl, substituted or unsubstituted butyl, substituted or unsubstituted propenyl, substituted or unsubstituted butenyl, trimethylsilylacetylenyl, acetylenyl, substituted or unsubstituted cyclopropyl, substituted or unsubstituted cyclohexyl, substituted or unsubstituted cyclohexenyl, substituted or unsubstituted cyclohexanonyl, substituted or unsubstituted phenyl, substituted or unsubstituted azetidinyl, substituted or unsubstituted pyrrolidinyl, substituted or unsubstituted piperazinyl, substituted or unsubstituted pyridinyl, substituted or unsubstituted piperidinyl, substituted or unsubstituted piperidinonyl, substituted or unsubstituted dihydropyranyl, substituted or unsubstituted aziridinyl, substituted or unsubstituted tetrahydropyranyl, substituted or unsubstituted tetrahydropyridinyl, substituted or unsubstituted morpholinyl, substituted or unsubstituted thiomorpholinyl, substituted or unsubstituted thiomorpholine dioxide, substituted or unsubstituted oxaazaspiroheptyl, substituted or unsubstituted hexahydrofuropyrrolyl, substituted or unsubstituted oxaazabicycloheptyl, substituted or unsubstituted pyrazolyl, NRⁱR^{j} or OR^{k}, where Rⁱ to R^{k} may be hydrogen, substituted or unsubstituted methyl, substituted or unsubstituted ethyl, substituted or unsubstituted propyl, substituted or unsubstituted butyl, substituted or unsubstituted cyclopropyl, substituted or unsubstituted cyclobutyl, substituted or unsubstituted cyclopentyl, substituted or unsubstituted cyclohexyl, substituted or unsubstituted cyclohexylmethyl, substituted or unsubstituted oxacyclobutanyl, substituted or unsubstituted dimethyloxazolidinonyl, substituted or unsubstituted cyclohexanonylmethyl, substituted or unsubstituted phenyl, substituted or unsubstituted piperidinyl, substituted or unsubstituted pyrimidinyl or substituted or unsubstituted tetrahydropyranyl.

In an embodiment, the atom that connects A² with the 2-oxo-benzimidazole of a parent nucleus may be C, and may also be a heteroatom such as N or O. For example, if A² is substituted or unsubstituted azetidinyl, substituted or unsubstituted aziridinyl, substituted or unsubstituted pyrrolidinyl, substituted or unsubstituted piperazinyl, substituted or unsubstituted piperidinyl, substituted or unsubstituted morpholinyl, substituted or unsubstituted thiomorpholinyl, substituted or unsubstituted thiomorpholine dioxide, substituted or unsubstituted oxaazaspiroheptyl, substituted or unsubstituted hexahydrofuropyrrolyl or substituted or unsubstituted oxaazabicycloheptyl, A² and the 2-oxo-benzimidazole of the parent nucleus may be connected by N.

In an embodiment, the compound of Formula I-2 may be represented by any one of the following Formula I-2-1 or I-2-2:

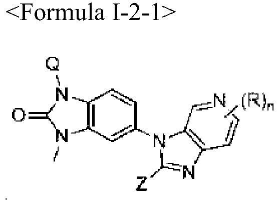

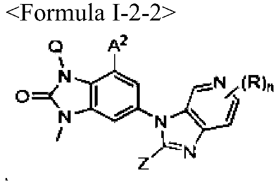

where A², n, R, Q and Z may be the same as defined above.

In an embodiment, the compound of Formula I-1-1 may be represented by any one of the following Formula I-1-1-1 or I-1-1-2:

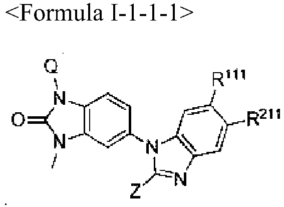

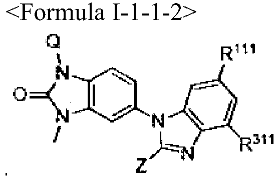

where R¹¹¹, R²¹¹ and R³¹¹ may be each independently C₁₋₅ alkyl, C₁₋₃ haloalkyl, C₆₋₈ aryl, 5- to 7-membered heteroaryl, halogen, cyano, carbonyl, CONR^{d}R^{e} or CO₂R^{f}, which may be substituted or unsubstituted with substituent group I, and R^{d} to R^{f}, Q and Z may be the same as defined above.

In an embodiment, R¹¹¹, R²¹¹ and R³¹¹ may be each independently methyl, propyl, CF₂H, phenyl, pyrazolyl, bromo, fluoro, cyano, aldehyde, CONR^{d}R^{e} or CO₂R^{f}, where R^{d} to R^{f} may be each independently hydrogen or substituted or unsubstituted methyl, and R¹¹¹ and R²¹¹ may be each independently substituted or unsubstituted with Substituent Group I-1-1.

Substituent Group I-1-1: substituted or unsubstituted C₁₋₃ alkyl, halogen, OR^{p1}, NR^{p2}R^{p3}, CONR^{p4}R^{p5} or NHCOR^{p6}, where R^{p1} to R^{p6} may be each independently hydrogen, substituted or unsubstituted methyl or substituted or unsubstituted propyl.

In an embodiment, the compound of Formula I-1-2 may be represented by any one of the following Formula I-1-2-1 to I-1-2-3:

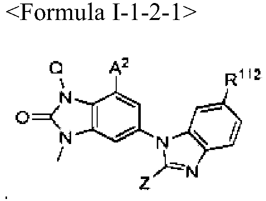

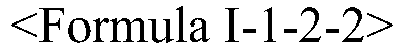

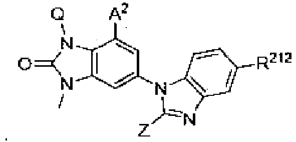

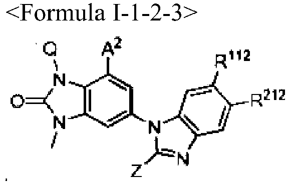

where A² may be the same as defined above, R¹¹² and R²¹² may be each independently C₁₋₅ alkyl, C₃₋₇ cycloalkyl, C₁₋₅ haloalkyl, C₆₋₁₀ aryl, 5- to 9-membered heteroaryl, 3- to 14-membered heterocycloalkyl, 4- to 9-membered heterocycloalkenyl, halogen, cyano, carbonyl, formaldoxime, NR^{a}R^{b}, SO₂NHR^{c}, CONR^{d}R^{e}, CO₂R^{f}, NHCOR^{g} or OR^{h}, where R¹¹² and ^{R212} may be each substituted or unsubstituted with Substituent Group I, and R^{a} to R^{h}, Q and Z may be the same as defined above.

In an embodiment, R¹¹² and R²¹² may be each independently methyl, cyclopropyl, CF₃, CF₂H, CFH₂, phenyl, oxazolyl, pyrazolyl, thiazolyl, pyridinyl, oxadiazolyl, imidazolyl, triazolyl, lactamyl, oxindolyl, quinolinonyl, dihydroquinolinonyl, benzoxazinonyl, dihydropyranyl, fluoro, chloro, bromo, cyano, aldehyde, formaldoxime, NR^{a}R^{b}, SO₂NHR^{c}, CONR^{d}R^{e}, CO₂R^{f}, NHCOR^{g} or OR^{h}, where R^{a} to R^{h} may be each independently hydrogen, substituted or unsubstituted methyl, CF₂H, CF₃, substituted or unsubstituted phenyl, substituted or unsubstituted benzyl, substituted or unsubstituted carbonyl or a Boc group, and R¹¹² and R²¹² may be each independently substituted or unsubstituted with Substituent Group I-1-2.

Substituent Group I-1-2: substituted or unsubstituted C₁₋₅ alkyl, substituted or unsubstituted C₃₋₅ cycloalkyl, substituted or unsubstituted C₁₋₅ alkenyl, substituted or unsubstituted C₁₋₄ haloalkyl, halogen, 4- to 8-membered heterocycloalkyl, OR^{p1}, NR^{p2}R^{p3}, CONR^{p4}R^{p5} or NHCOR^{p6}, where R^{p1} to R^{p6} may be each independently hydrogen, substituted or unsubstituted methyl or substituted or unsubstituted carbonyl.

In an embodiment, the compound of Formula I-2-1 may be represented by any one of the following Formula I-2-1-1 or 1-2-1-2:

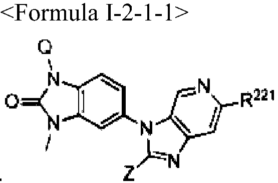

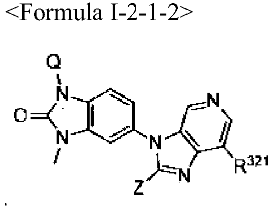

where R²²¹ and R³¹² may be each independently C₆₋₉ aryl, 5- to 9-membered heteroaryl, halogen or CONR^{d}R^{e}, where R²²¹ and R³¹² may be each substituted or unsubstituted with Substituent Group I, and R^{d} to R^{e}, Q and Z may be the same as defined above.

In an embodiment, R²²¹ and R³¹² may be each independently phenyl, pyrazolyl, bromo or CONR^{d}R^{e}, where R^{d} to R^{e} may be each independently hydrogen or substituted or unsubstituted methyl, and R²²¹ and R³¹² may be each independently substituted or unsubstituted with Substituent Group 1-1-3.

Substituent Group I-1-3: substituted or unsubstituted C₁₋₃ alkyl, halogen or CONR^{p4}R^{p5}, where R^{p4} to R^{p5} may be each independently hydrogen or substituted or unsubstituted methyl.

In an embodiment, the compound of Formula I-2-2 may be represented by any one of the following Formula I-2-2-1 or I-2-2-2:

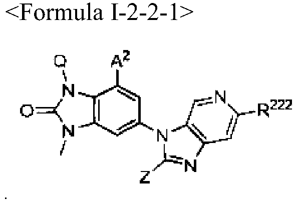

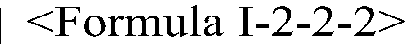

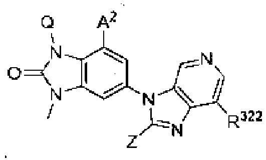

where A² may be the same as defined above, R²²² and R³²² may be each independently 5- to 9-membered heteroaryl, where R²²² and R³²² may be each substituted or unsubstituted with Substituent Group I, and Q and Z may be the same as defined above.

In an embodiment, R²²² and R³²² may be each independently pyrazolyl or thiazolyl, where R²²² and R³²² may be each independently substituted or unsubstituted with Substituent Group I-1-4.

Substituent Group I-1-4: substituted or unsubstituted C₁₋₃ alkyl or substituted or unsubstituted C₁₋₃ haloalkyl.

In an embodiment, Q may be hydrogen, substituted or unsubstituted methyl, substituted or unsubstituted ethyl or substituted or unsubstituted propyl.

In an embodiment, Z may be substituted or unsubstituted methyl, substituted or unsubstituted ethyl, substituted or unsubstituted propyl, substituted or unsubstituted butyl, substituted or unsubstituted cyclopropyl, substituted or unsubstituted cyclobutyl, substituted or unsubstituted phenyl, substituted or unsubstituted imidazolyl, substituted or unsubstituted indolyl, substituted or unsubstituted thiophenyl, substituted or unsubstituted pyridinyl, substituted or unsubstituted pyrimidinyl, substituted or unsubstituted thiazolyl, or substituted or unsubstituted oxetanyl.

In an embodiment, the compound may be any one selected from Table 2 below.

In an embodiment, the compound represented by Formula I may be used as a CBP/p300 inhibitor. In this specification, CBP/p300 may mean CBP and/or p300. The CBP refers to CREB (cyclic-AMP response element binding protein) binding protein, and the p300 refers to a paralog of CBP.

The CBP/p300 induces histone H3 lysine 27 acetylation (H3K27ac) at target gene promoters, enhancers and super enhancers, and at the same time, recognizes acetylated histones via BRD to activate gene transcription. According to the overexpression of the CBP/p300 in cancer cells and drug-resistant cancer cells, oncogene transcription is activated and cancer cell proliferation, survival, tumor formation, metastasis, immune evasion or drug resistance is induced. Therefore, it is necessary to inhibit the overexpression of CBP/p300 in cancer cells, or the like. The use of the compound represented by Formula I as a CBP/p300 inhibitor in this specification may mean that the compound has the inhibition activity of the overexpression of CBP/p300 as described above. The compound represented by Formula I of the present disclosure may, for example, reduce the H3K27ac and down-regulate oncogene transcription, thereby inducing cancer cell growth inhibition and apoptosis, activating immune responses, overcoming drug resistance, and inhibiting tumor progression in vivo. The compound represented by Formula I of the present disclosure may also, for example, enhance the anticancer efficacy of chemotherapy, radiotherapy, and epigenetic anticancer agents by inhibiting BRD.

The present disclosure also provides an isomer of the compound represented by Formula I, a pharmaceutically acceptable salt thereof, a hydrate thereof, or a solvate thereof.

In this specification, the term "isomer" may include an enantiomer, a diastereomer thereof, a tautomer thereof, and a geometric isomer thereof. In this specification, enantiomers refer to two stereoisomers of a compound that are non-superimposable mirror images of each other.

In this specification, diastereomers refer to stereoisomers having two or more chiral centers and whose molecules are not mirror images of each other. Diastereomers have different physical properties such as melting points, boiling points, spectral characteristics, and reactivities. Mixtures of diastereomers may be separated under high-resolution analytical procedures such as electrophoresis and chromatography.

In this specification, geometric isomers refer to cis forms, in which the same type of atoms or groups of atoms are on the same side of a double bond, and trans forms, in which the same type of atoms or groups of atoms are on opposite sides of a double bond.

In this specification, tautomers or tautomeric forms refer to structural isomers with different energies that are compatible through a low energy barrier. For example, proton tautomers (also known as protonic tautomers) include interconversions via the transfer of a proton, including keto-enol and imine-enamine isomerization. Atomic tautomers include interconversions by retransfer of some bonding electrons.

In this specification, an arbitrary atom present in the compound of the present disclosure may be present in any of naturally occurring isotopic forms. For example, isotopes of hydrogen, carbon, nitrogen, oxygen, phosphorus, sulfur, fluorine, chlorine and iodine may be ²H ("D"), ³H, ¹¹C, ¹²C, ¹³C, ¹⁴C, ¹³N, ¹⁵N, ¹⁵O, ¹⁷O, ¹⁸O, ³²P, ³³P, ³⁵S, ¹⁸F, ³⁶Cl, ¹²³I and ¹²⁵I.

In this specification, the term "acceptable salt" means a salt of an active compound prepared with an acid or a base, which depends on the particular substituent based on the compound described in this specification. If the compound according to the present disclosure contains a relatively acidic functional group, a base addition salt may be obtained by contacting such a compound in a neutral state with a sufficient amount of a desired base in the absence of a solvent or in the presence of a suitable inert solvent.

Acceptable salts include, for example, alkali metal salts, for example, sodium or potassium salt; alkaline earth metal salts, for example, calcium or magnesium salt; ammonium salt; aliphatic amine salts, for example, trimethylamine, triethylamine, dicyclohexylamine, ethanolamine, diethanolamine, triethanolamine or procaine salt; aralkyl amine salts, for example, N,N-dibenzylethylenediamine salt; heterocyclic aromatic amine salts, for example, pyridine salt, picolino salt, quinoline salt or isoquinoline salt; quaternary ammonium salts, for example, tetramethylammonium salt, tetraethylammonium salt, benzyltrimethylammonium salt, benzyltriethylammonium salt, benzyltributylammonium salt, methyltrioctylammonium salt or tetrabutylammonium salt; and basic amino acid salts, for example, arginine salt or lysine salt. The acid salts may be, for example, inorganic acid salts, for example, hydrochloride, sulfate salt, nitrate salt, phosphate salt, carbonate salt, hydrogencarbonate or perchlorate; organic acid salts, for example, acetate, propionate, lactate, malate, fumarate, tartrate, malate, fumarate, citrate, ascorbate, or formic acid; sulfonates, for example, methanesulfonate, isothionate, benzenesulfonate or p-toluenesulfonate; and acidic amino acid salts, for example, aspartate or glutamate.

The term "hydrate" in this specification refers to a compound in which water (H₂O) is bonded to another compound.

In this specification, the term "solvate" refers to an aggregate or composite of one or more solvent molecules with the compound of the present disclosure. Examples of the solvent forming a solvate include, but are not limited to, water, isopropanol, ethanol, methanol, dimethyl sulfoxide, ethyl acetate, acetic acid, and ethanolamine.

The present disclosure also provides a pharmaceutical composition for preventing or treating cancer, inflammatory disorders, or autoimmune diseases, including at least one of the compound represented by Formula I, an isomer thereof, a pharmaceutically acceptable salt thereof, a hydrate thereof, and a solvate thereof, as an active ingredient.

In an embodiment, the cancer may be cancer expressing AR, tumor harboring a loss of function mutation of CBP or p300, or cancer disease with activation of CBP and/or p300 function.

In an embodiment, the cancer disease may be one or more selected from the group consisting of blood cancer, prostate cancer, breast cancer, lung cancer, pancreatic cancer, colorectal cancer, and melanoma.

In an embodiment, the cancer disease may be one or more selected from the group consisting of acoustic neuroma, acute leukemia, acute lymphoblastic leukemia, acute myeloid leukemia, acute T-cell leukemia, basal cell carcinoma, cholangiocarcinoma, bladder cancer, brain cancer, breast cancer, bronchial carcinoma, cervical cancer, chondrosarcoma, chordoma, choriocarcinoma, chronic leukemia, chronic lymphocytic leukemia, chronic myeloid leukemia, chronic myeloid leukemia, colon cancer, colon cancer, craniopharyngioma, cystadenocarcinoma, diffuse large B-cell lymphoma, dysproliferative changes, embryonal carcinoma, endometrial cancer, endotheliosarcoma, ependymoma, epithelial carcinoma, erythroleukemia, esophageal cancer, estrogen receptor-positive breast cancer, essential thrombocythemia, Ewing's tumor, fibrosarcoma, follicular lymphoma, germ cell testicular cancer, glioma, glioblastoma, pedunculated tumor, heavy chain disease head and neck cancer, hemangioblastoma, hepatoma, hepatocellular carcinoma, hormone-insensitive prostate cancer, leiomyosarcoma, leukemia, liposarcoma, lung cancer, lymphoendotheliosarcoma, lymphangiosarcoma, lymphocytic leukemia, lymphoma, lymphoid malignancy of T-cell or B-cell origin, medullary carcinoma, medulloblastoma, melanoma, meningioma, mesothelioma, multiple myeloma, myeloid leukemia, myeloma, myxosarcoma, neuroblastoma, NUT midline carcinoma (NMC), non-small cell lung cancer, oligodendroglioma, oral cancer, osteosarcoma, ovarian cancer, pancreatic cancer, papillary adenocarcinoma, papillary carcinoma, pinealoma, polycythemia vera, prostate cancer, rectal cancer, renal cell carcinoma, retinoblastoma, rhabdomyosarcoma, sarcoma, sebaceous carcinoma, seminoma, skin cancer, small cell lung carcinoma, solid tumors (carcinomas and sarcomas), small cell lung cancer, stomach cancer, squamous cell carcinoma, synovioma, sweat gland carcinoma, thyroid cancer, Waldenstrom macroglobulinemia, testicular tumor, uterine cancer and Wilms tumor.

In an embodiment, the inflammatory disorder or autoimmune disease may be one or more selected from the group consisting of Addison's disease, acute gout, ankylosing spondylitis, asthma, atherosclerosis, Behcet's disease, bullous skin disease, chronic obstructive pulmonary disease, Crohn's disease, dermatitis, eczema, giant cell arteritis, fibrosis, glomerulonephritis, hepatic vascular occlusion, hepatitis, hypophysitis, immunodeficiency syndrome, inflammatory bowel disease, Kawasaki disease, lupus nephritis, multiple sclerosis, myocarditis, myositis, nephritis, organ transplant rejection, osteoarthritis, pancreatitis, pericarditis, polyarteritis nodosa pneumonia, primary biliary cirrhosis, psoriasis, psoriatic arthritis, rheumatoid arthritis, scleritis, sclerosing cholangitis, sepsis, systemic lupus erythematosus, Takayasu's arteritis, toxic shock, thyroiditis, type 1 diabetes, ulcerative colitis, uveitis, vitiligo, vasculitis, and Wegener's granulomatosis.

In an embodiment, the pharmaceutical composition may further include a pharmaceutically acceptable diluent or carrier. Specifically, the pharmaceutical composition of the present disclosure may include a pharmaceutically acceptable carrier, and may be formulated in the form of oral dosage forms such as powders, granules, tablets, capsules, suspensions, emulsions, syrups and aerosols, external preparations, suppositories and sterile injectable solutions, respectively, according to common methods. The pharmaceutically acceptable carrier may include lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinyl pyrrolidone, water, methyl hydroxybenzoate, propyl hydroxybenzoate, talc, magnesium stearates and mineral oil. In addition, the carrier may include diluents or excipients, such as fillers, weighting agents, binders, wetting agents, disintegrating agents and surfactants. An oral solid preparation may include tablets, pills, powders, granules and capsules, and the solid preparation may include one or more excipients, for example, starch, calcium carbonate, sucrose or lactose, and gelatin, and may include a lubricant such as magnesium stearate and talc. An oral liquid preparation may include suspensions, solutions, emulsions and syrups, and may include diluents such as water and liquid paraffin, wetting agents, sweeteners, flavoring agents and preservatives. A parenteral preparation may include sterile aqueous solutions, non-aqueous solvents, suspensions, emulsions, lyophilized preparations and suppositories, and the non-aqueous solvent and suspension may include propylene glycol, polyethylene glycol, vegetable oils such as olive oil, and injectable esters such as ethyl oleate. As a suppository base, witepsol, macrogol, tween 61, cacao butter, laurin butter, glycerogelatin, or the like may be used.

In an embodiment, the dosage of the compound represented by Formula I, the isomer thereof, the pharmaceutically acceptable salt thereof, the hydrate thereof or the solvate thereof, contained in the pharmaceutical composition of the present disclosure varies depending on the patient's condition, weight, the degree of the disease, the drug form, the administration route and period, but may be appropriately selected by a person skilled in the art. For example, the compound represented by Formula I, or the pharmaceutically acceptable salt thereof may be administered at a dosage of 0.0001 to 1000 mg/kg, preferably 0.01 to 1000 mg/kg per day, and the administration may be performed once a day or in several divided doses. In addition, the pharmaceutical composition of the present disclosure may contain the compound represented by Formula I, or the pharmaceutically acceptable salt thereof, in a weight percentage of 0.001 to 90% based on the total weight of the composition.

In an embodiment, the pharmaceutical composition of the present disclosure may be administered to mammals such as rats, mice, livestock, and humans by various routes, for example, orally, intraperitoneally, rectally, or by intravenous, intramuscular, subcutaneous, intrauterine or intracerebroventricular injection.

Hereinafter, the present disclosure will be described in more detail through preparation examples, examples, and experimental examples. However, the examples and experimental examples below are intended to illustrate the present disclosure, and the scope of the present disclosure is not limited thereto.

The compound having the structure of Formula I may be synthesized by the method below.

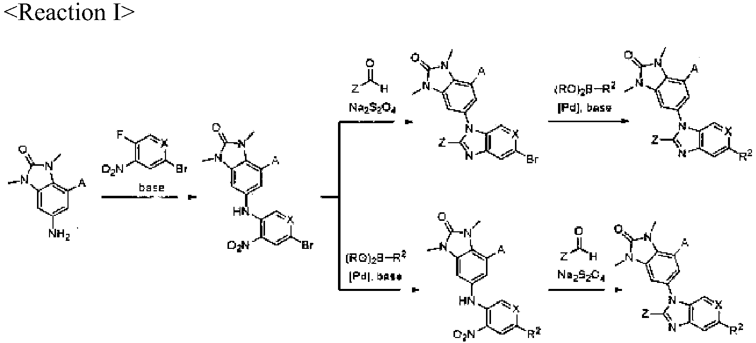

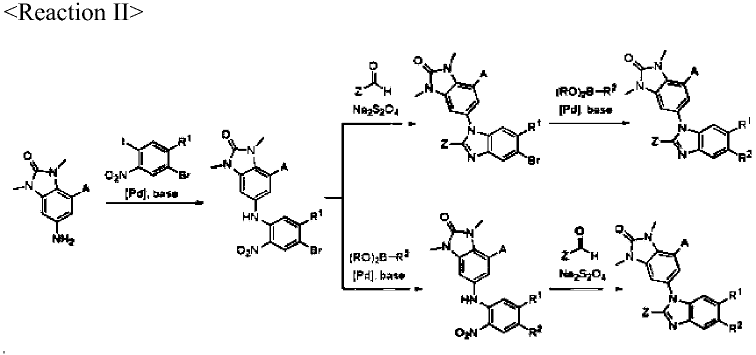

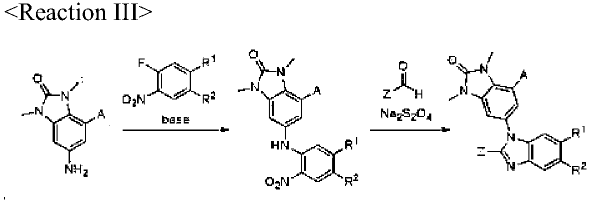

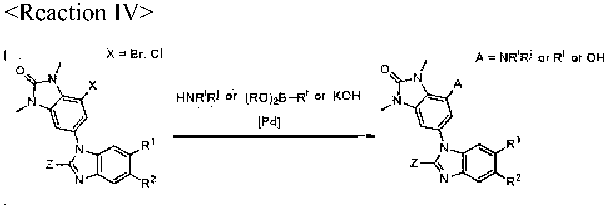

The analyses of the compounds prepared in the Preparation Examples and Examples were performed as follows: Nuclear magnetic resonance (NMR) spectroscopy was performed on a Bruker 500 MHz or Jeol 400 MHz spectrometer. Mass spectrometry was performed using LC/MS (Agilent 1260 infinity II/Agilent infinity lab LC/MSD, API-ES mode, UV detection at 220 nm/254 nm). (Column: ZORBAX RRHT StableBond C18, 2.1 x 50 mm, 1.8 µm, 80 Å, mobile phase: water/acetonitrile/formic acid (30/70/0.1%), isocratic elution for 3 min.)

In addition, medium pressure liquid chromatography (MPLC, CombiFlash Nextgen 300+) or prepHPLC (Thermo Ultimate 3000 equipped with a diode array detector, UV detection: 215 nm, 254 nm, 280 nm) was used for the purification of the compounds. The prepHPLC conditions were as follows. (Column: Phenomenex Luna, 10 µm, C18(2), 100 Å, 250 x 21.20 mm, mobile phase A: water/0.1% trifluoroacetic acid, mobile phase B: acetonitrile/0.1% trifluoroacetic acid, gradient elution for 30 min, A:B = 75:25-65:35)
Each starting material is a known compound and was synthesized according to the literature or purchased commercially.

### Intermediate Synthetic Method

### Intermediate I-1: Preparation of 5-amino-1,3-dimethyl-1,3-dihydro-2H-benzo[d]imidazol-2-one

### Step 1) Preparation of 5-nitro-1,3-dihydro-2H-benzo[d]imidazol-2-one

4-Nitrobenzene-1,2-diamine (10 g, 65.3 mmol) was dissolved in tetrahydrofuran (365 mL) and cooled to 0°C, and 1,1'-carbonyldiimidazole (21 g, 130.6 mmol) was added. The reaction solution was stirred at room temperature for 2 hours. The reaction solution was distilled under reduced pressure, and the obtained solid was washed with diethyl ether, 1 N hydrochloric acid, and water. The solid was dried under vacuum to obtain the title compound. (9.59 g, 53.5 mmol, 82% yield)
¹H-NMR (500 MHz, DMSO-d₆) δ 11.43 (s, 1H), 11.20 (s, 1H), 7.94 (dd, J = 8.6, 2.3 Hz, 1H), 7.71 (d, J = 2.2 Hz, 1H), 7.10 (d, J = 8.6 Hz, 1H).

### Step 2: Preparation of 1,3-dimethyl-5-nitro-1,3-dihydro-2H-benzo[d]imidazol-2-one

5-Nitro-1,3-dihydro-2H-benzo[d]imidazol-2-one (4.9 g, 27.5 mmol) obtained in Step 1 was dissolved in dimethylformamide (70 mL) and cooled to 0°C. Sodium hydride (dispersed in mineral oil into 60%, 4.4 g, 110 mmol) was added little by little and iodomethane (11.7 g, 5.13 mL, 83.0 mmol) was added. The reaction solution was stirred at 60°C for 2 hours and then water was added to terminate the reaction. The reaction solution was diluted with ethyl acetate and washed with 0.1 N hydrochloric acid and brine. An organic layer was distilled under reduced pressure, and the obtained solid was washed with hexane and dried under vacuum to obtain the title compound. (4.95 g, 23.4 mmol, 87% yield)
¹H-NMR (500 MHz, Chloroform-d) δ 8.12 (dd, J = 8.6, 2.2 Hz, 1H), 7.89 (d, J = 2.1 Hz, 1H), 7.02 (d, J = 8.6 Hz, 1H), 3.50 (s, 3H), 3.49 (s, 3H).

### Step 3: Preparation of 5-amino-1,3-dimethyl-1,3-dihydro-2H-benzo[d]imidazol-2-one

1,3-Dimethyl-5-nitro-1,3-dihydro-2H-benzo[d]imidazol-2-one (3.5 g, 16.9 mmol) obtained in Step 2 was dissolved in ethyl acetate (50 mL) and methanol (50 mL), and palladium/carbon (10 wt%, 0.7 g) was added. After the reaction system was replaced with hydrogen using a hydrogen balloon, the reaction solution was stirred under hydrogen conditions for 3 hours. After the reaction was completed, the reaction solution was filtered through a celite pad to remove a catalyst. The filtrate was distilled under reduced pressure to obtain the title compound, which was used in a subsequent reaction without further purification. (2.79 g, 15.7 mmol, 93% yield)
¹H-NMR (500 MHz, DMSO-d₆) δ 6.78 (d, J = 8.2 Hz, 1H), 6.35 (d, J = 2.0 Hz, 1H), 6.31 (dd, J = 8.2, 2.1 Hz, 1H), 4.77 (s, 2H), 3.22 (s, 3H), 3.21 (s, 3H).

### Intermediate I-2 (Synthetic Method I-A): Preparation of 6-amino-4-chloro-1,3-dimethyl-1,3-dihydro-2H-benzo[d]imidazol-2-one

### Step 1: Preparation of 3-chloro-5-nitrobenzene-1,2-diamine

Sodium sulfide pentahydrate (3.95 g, 37.2 mmol) and sulfur (1.194 g, 37.2 mmol) were added to a solution of water (25 mL) and ethanol (6 mL), followed by stirring at 100°C for 15 minutes. A mixture of 2-chloro-4,6-dinitroaniline (5.4 g, 24.82 mmol) and ammonium chloride (1.991 g, 37.2 mmol), dissolved in ethanol (50 mL) and water (25 mL) was added dropwisely to the reaction solution over 10 minutes. The reaction solution was stirred at 65°C for 1 hour. Then, 6 N sodium hydroxide (12.41 mL, 74.5 mmol) was added dropwisely over 10 minutes and stirred at 65°C for 1 hour. The reaction solution was cooled to 0°C, 6 N hydrochloric acid (11.5 mL, 69 mmol) was added over 20 minutes, and water (25 mL) was added. The reaction solution was stirred at 0°C for 40 minutes and a suspension was filtered. The filter cake was washed with water and ethanol and dried under nitrogen to obtain a yellow solid. (4 g, 21.32 mmol, 86% yield)
¹H-NMR (500 MHz, Chloroform-d) δ 7.88 (d, J = 2.4 Hz, 1H), 7.58 (d, J = 2.1 Hz, 1H), 4.47 (s, 2H), 3.69-3.48 (m, 2H).

### Step 2: Preparation of 4-chloro-6-nitro-1,3-dihydro-2H-benzo[d]imidazol-2-one

3-Chloro-5-nitrobenzene-1,2-diamine (4 g, 21.32 mmol) obtained in Step 1 was dissolved in dimethylformamide (80 mL), and 1,1'-carbonyldiimidazole (5.19 g, 32.0 mmol) was added at room temperature. The mixture was stirred at room temperature for 18 hours. The reaction solution was cooled to 0°C, and water (80 mL) and 6 N hydrochloric acid (10.7 mL) were added, followed by stirring for 30 minutes. A suspension was filtered, and the filter cake was washed with methyl t-butyl ether and dried under nitrogen blowing conditions to obtain the title compound. (3.6 g, 16.86 mmol, 79% yield)
¹H-NMR (500 MHz, DMSO-d₆) δ 7.98 (d, J = 1.8 Hz, 1H), 7.70-7.66 (m, 1H).

### Step 3: Preparation of 4-chloro-1,3-dimethyl-6-nitro-1,3-dihydro-2H-benzo[d]imidazol-2-one

4-Chloro-6-nitro-1,3-dihydro-2H-benzo[d]imidazol-2-one (7.2 g, 33.7 mmol) obtained in Step 2 was dissolved in dimethylformamide (100 mL), and potassium carbonate (13.98 g, 101 mmol) and iodomethane (8.39 mL, 135 mmol) were added. The reaction solution was stirred for 3 hours. After the mixture was cooled to 0°C, water (100 mL) was added, and the resultant was stirred for 20 minutes. The solid formed during the reaction was filtered, and the filtered solid was washed with water, finally with methyl t-butyl ether, and dried with nitrogen to obtain the title compound. (5.9 g, 24.42 mmol, 72.4% yield)
¹H-NMR (500 MHz, DMSO-d₆) δ 8.07 (d, J = 1.8 Hz, 1H), 8.03-8.00 (1H), 3.66 (s, 3H), 3.43 (s, 3H).

### Step 4: Preparation of 6-amino-4-chloro-1,3-dimethyl-1,3-dihydro-2H-benzo[d]imidazol-2-one

4-Chloro-1,3-dimethyl-6-nitro-1,3-dihydro-2H-benzo[d]imidazol-2-one (3 g, 12.42 mmol) obtained in Step 3 and stannous chloride dihydrate (8.40 g, 37.2 mmol) were added to ethanol (30 mL) and stirred at 70°C for 5 hours under reflux conditions. The reaction solution was cooled to room temperature, water (40 mL) was added, and 6 N sodium hydroxide (40-60 mL) was added at 0°C to adjust the pH of the solution to about 10-12. After stirring for 20 minutes, an organic layer was extracted with ethyl acetate and washed with brine. The solid obtained by concentration under reduced pressure was used in a subsequent reaction without further purification. (2.7 g, 12.76 mmol, 100% yield)
¹H-NMR (500 MHz, Chloroform-d) δ 6.38 (s, 1H), 6.22 (s, 1H), 3.66 (s, 3H), 3.59 (s, 2H), 3.33 (s, 3H).

### Intermediate I-4 (Synthetic Method I-B): Preparation of 6-amino-1,3-dimethyl-4-(tetrahydro-2H-pyran-4-yl)-1,3-dihydro-2H-benzo[d]imidazol-2-one

### Step 1: Preparation of 4-(3,6-dihydro-2H-pyran-4-yl)-1,3-dimethyl-6-nitro-1,3-dihydro-2H-benzo[d]imidazol-2-one

4-Chloro-1,3-dimethyl-6-nitro-1,3-dihydro-2H-benzo[d]imidazol-2-one (the compound of Step 3 of Synthetic Method I-A, 1.2 g, 4.97 mmol) was added to 1,4-dioxane (20 mL), and 2-(3,6-dihydro-2H-pyran-4-yl)-4,4,5,5-tetramethyl-1,3,2-dioxaborolane (1.148 g, 5.46 mmol), potassium carbonate (1.373 g, 9.93 mmol), XPhos Pd G2 (0.195 g, 0.248 mmol) and XPhos (0.237 g, 0.497 mmol) were added respectively, followed by bubbling with nitrogen for 5 minutes. The reaction solution was stirred at 90°C under nitrogen conditions for 18 hours. After cooling the reaction solution to room temperature, water was added, the resultant was extracted with ethyl acetate, and an organic layer was washed with brine. The organic layer was concentrated under reduced pressure and separated by MPLC to obtain the title compound.

¹H-NMR (500 MHz, Chloroform-d) δ 7.85 (d, J = 2.1 Hz, 1H), 7.80 (d, J = 2.1 Hz, 1H), 5.86 (s, 1H), 4.35 (t, J = 2.6 Hz, 2H), 3.99 (t, J = 5.3 Hz, 2H), 3.56-3.52 (3H), 3.51 (s, 3H), 2.46 (d, J = 2.1 Hz, 2H)

### Step 2: Preparation of 6-amino-1,3-dimethyl-4-(tetrahydro-2H-pyran-4-yl)-1,3-dihydro-2H-benzo[d]imidazol-2-one

4-(3,6-Dihydro-2H-pyran-4-yl)-1,3-dimethyl-6-nitro-1,3-dihydro-2H-benzo[d]imidazol-2-one (1.45 g, 5.01 mmol) obtained in Step 1 was added to methanol (2 mL), and dihydroxypalladium (0.352 g, 2.506 mmol) was added. The solution was stirred at 60°C for 4 hours under hydrogen conditions. The reaction solution was filtered through a celite pad, and the filtrate was concentrated under reduced pressure. The obtained material was used in a subsequent reaction without further purification.

¹H-NMR (500 MHz, DMSO-d₆) δ 6.26 (s, 1H), 6.20 (s, 1H), 4.78 (s, 2H), 3.99-3.84 (m, 2H), 3.49 (d, J = 11.0 Hz, 4H), 3.19 (s, 3H), 1.74-1.59 (m, 4H).

### Intermediate I-8 (Synthetic Method I-C): Preparation of 6-amino-1,3-dimethyl-4-(propylamino)-1,3-dihydro-2H-benzo[d]imidazol-2-one

### Step 1: Preparation of 1,3-dimethyl-6-nitro-4-(propylamino)-1,3-dihydro-2H-benzo[d]imidazol-2-one

6-Amino-4-bromo-1,3-dimethyl-1,3-dihydro-2H-benzo[d]imidazol-2-one (Intermediate I-3, 100 mg, 0.350 mmol) and propan-1-amine (0.057 mL, 0.699 mmol) were stirred together with tris(dibenzylideneacetone)dipalladium(0) (28.5 mg, 0.035 mmol), Xantphos (60.7 mg, 0.105 mmol) and cesium carbonate (297 mg, 1.398 mmol) in a toluene (3.5 mL) solvent at 100°C for 18 hours. The reaction product was diluted with ethyl acetate, washed with water, dried over anhydrous magnesium sulfate, and concentrated under reduced pressure. The residue was purified by MPLC to obtain the title compound. (66 mg, 0.250 mmol, 71.4% yield)

### Step 2: Preparation of 6-amino-1,3-dimethyl-4-(propylamino)-1,3-dihydro-2H-benzo[d]imidazol-2-one

1,3-Dimethyl-6-nitro-4-(propylamino)-1,3-dihydro-2H-benzo[d]imidazol-2-one (66 mg, 0.250 mmol) obtained in Step 1 and stannous chloride dihydrate (169 mg, 0.749 mmol) were added in ethanol (1 mL) and stirred at 60°C for 18 hours. After quenching the reaction using 1 N sodium hydroxide, an organic layer was extracted using ethyl acetate, washed sufficiently with water, dried over anhydrous magnesium sulfate, and concentrated under reduced pressure. The residue obtained was purified by MPLC to obtain the title compound. (50 mg, 0.213 mmol, 85% yield)
¹H-NMR (500 MHz, DMSO-d₆) δ 5.74 (s, 2H), 4.76 (t, J = 5.6 Hz, 1H), 4.63 (s, 2H), 3.48 (s, 3H), 3.13 (s, 3H), 2.93 (dd, J = 13.0, 6.9 Hz, 2H), 1.60 (p, 2H), 0.94 (t, J = 7.4 Hz, 3H).

### Intermediate I-12: Preparation of 6-amino-1-methyl-1,3-dihydro-2H-benzo[d]imidazol-2-one

### Step 1: Preparation of N1-methyl-5-nitrobenzene-1,2-diamine

4-Nitrobenzene-1,2-diamine (1.53 g, 9.99 mmol) and potassium carbonate (2.76 g, 19.98 mmol), with iodomethane (0.625 mL, 9.99 mmol) were stirred at room temperature for 16 hours in a solution of dimethylformamide (15 mL). The reaction mixture was diluted with ethyl acetate, washed once with water, twice with brine, and dried over anhydrous sodium sulfate. The filtrate was concentrated under reduced pressure, and the residue was purified by MPLC to obtain the title compound. (720 mg, 4.31 mmol, 43.1% yield)
¹H-NMR (500 MHz, DMSO-d₆) δ 7.50 (d, 1H), 7.11 (s, 1H), 6.56 (s, 1H), 6.12 (br s, 2H), 5.20 (s, 1H), 2.79 (d, 3H); LC/MS: 168 (M+H)

### Step 2: Preparation of 1-methyl-6-nitro-1,3-dihydro-2H-benzo[d]imidazol-2-one

N1-Methyl-5-nitrobenzene-1,2-diamine (720 mg, 4.31 mmol) obtained in Step 1 was dissolved in tetrahydrofuran (30 mL), 1,1'-carbonyldiimidazole (838 mg, 5.17 mmol) was added, and the resultant was stirred at 80°C for 6 hours and at room temperature for 3 days. After cooling to room temperature, the reaction solution was concentrated under reduced pressure, and the residue was precipitated by trituration with ethyl acetate and dried to obtain the title compound. (614 mg, 3.18 mmol, 73.8% yield)
¹H-NMR (500 MHz, DMSO-d₆) δ 11.63 (br s, 1H), 8.01 (d, 1H), 7.99 (s, 1H), 7.15 (d, 1H), 3.78 (s, 3H); LC/MS: 192 (M-H).

### Step 3: Preparation of 6-amino-1-methyl-1,3-dihydro-2H-benzo[d]imidazol-2-one

The title compound was obtained in a similar manner to Step 3 of the synthesis of Intermediate I-1, using 1-methyl-6-nitro-1,3-dihydro-2H-benzo[d]imidazol-2-one (400 mg, 2.071 mmol) obtained in Step 2 as a starting material. (331 mg, 2.028 mmol, 98% yield)
LC/MS: 164 (M+H).

### Intermediate I-13 (Synthetic Method I-D): Preparation of 6-amino-1,3-dimethyl-4-(pyrrolidin-1-yl)-1,3-dihydro-2H-benzo[d]imidazol-2-one

### Step 1: Preparation of 1,3-dimethyl-6-nitro-4-(pyrrolidin-1-yl)-1,3-dihydro-2H-benzo[d]imidazol-2-one

The title compound was obtained in a similar manner to Step 1 of Synthetic Method I-C, using 6-amino-4-bromo-1,3-dimethyl-1,3-dihydro-2H-benzo[d]imidazol-2-one (Intermediate I-3, 300 mg, 1.049 mmol) and pyrrolidine (0.086 mL, 1.049 mmol). (160 mg, 0.579 mmol, 55.2% yield)

### Step 2: Preparation of 6-amino-1,3-dimethyl-4-(pyrrolidin-1-yl)-1,3-dihydro-2H-benzo[d]imidazol-2-one

1,3-Dimethyl-6-nitro-4-(pyrrolidin-1-yl)-1,3-dihydro-2H-benzo[d]imidazol-2-one (131 mg, 0.474 mmol) obtained in Step 1 was stirred with palladium/carbon (50.5 mg, 0.047 mmol) in a solvent of methanol (1.2 mL) and ethyl acetate (1.2 mL) under hydrogen gas for 18 hours. After the reaction was completed, the reaction product was filtered through celite and concentrated under reduced pressure, and the residue was purified by MPLC to obtain the title compound. (110 mg, 0.474 mmol, 94% yield)
¹H-NMR (500 MHz, Chloroform-d) δ 6.26 (br s, 1H), 6.10 (br s, 1H), 3.61 (s, 3H), 3.54 (br s, 2H), 3.33 (s, 3H), 3.11 (br s, 2H), 3.02 (br s, 2H), 2.01 (br s, 2H), 1.93 (br s, 2H).

### Intermediate I-18 (Synthetic Method I-E): Preparation of 6-amino-4-isopropoxy-1,3-dimethyl-1,3-dihydro-2H-benzo[d]imidazol-2-one

### Step 1: Preparation of 4-hydroxy-1,3-dimethyl-6-nitro-1,3-dihydro-2H-benzo[d]imidazol-2-one

To a solution of 4-bromo-1,3-dimethyl-6-nitro-1,3-dihydro-2H-benzo[d]imidazol-2-one (15 g, 52.4 mmol) and potassium hydroxide (3.53 g, 62.9 mmol), dissolved in 1,4-dioxane (300 mL), tris(dibenzylideneacetone)dipalladium(0) (4.80 g, 5.24 mmol) and 2-di-tert-butylphosphino-2',4',6'-triisopropylbiphenyl (t-BuXPhos) (4.45 g, 10.5 mmol) were added, and nitrogen was blown in, followed by stirring at 100°C for 18 hours. The reaction mixture was cooled to room temperature and filtered, and the filtered solid was eluted while washing with 1,4-dioxane. The filtrate was concentrated under reduced pressure, and the residue was purified by column chromatography to obtain the title compound. (5 g, 21.7 mmol, 41.4% yield)
¹H-NMR (400 MHz, Chloroform-d) δ 10.75 (s, 1H), 7.61 (d, J = 2.1 Hz, 1H), 7.47 (d, J = 2.2 Hz, 1H), 3.53 (s, 3H), 3.35 (s, 3H); LC/MS: 224 (M+H).

### Step 2: Preparation of 4-isopropoxy-1,3-dimethyl-6-nitro-1,3-dihydro-2H-benzo[d]imidazol-2-one

To a solution of 4-hydroxy-1,3-dimethyl-6-nitro-1,3-dihydro-2H-benzo[d]imidazol-2-one (1.116 g, 5.0 mmol) and potassium carbonate (0.829 g, 6.00 mmol), dissolved in acetonitrile (25.00 mL), 2-iodopropane (0.549 mL, 5.50 mmol) was added, followed by stirring at 82°C for 10 hours. The reaction mixture was cooled to room temperature, diluted with water, and extracted with ethyl acetate. The obtained organic layer was washed with brine, dried over anhydrous sodium sulfate, and concentrated under reduced pressure. The residue was purified by MPLC to obtain the title compound. (1.065 g, 4.01 mmol, 80% yield)
¹H-NMR (500 MHz, Chloroform-d) δ 7.64 (s, 1H), 7.59 (s, 1H), 4.79-4.74 (m, 1H), 3.71 (s, 3H), 3.48 (s, 3H), 1.46 (d, J = 5.8 Hz, 6H); LC/MS: 266 (M+H)

### Step 3: Preparation of 6-amino-4-isopropoxy-1,3-dimethyl-1,3-dihydro-2H-benzo[d]imidazol-2-one

The title compound was obtained in a similar manner to Step 2 of Synthetic Method I-D, using 4-isopropoxy-1,3-dimethyl-6-nitro-1,3-dihydro-2H-benzo[d]imidazol-2-one (1.06 g, 4.00 mmol) obtained in Step 2 as a starting material. (812 mg, 3.45 mmol, 86% yield)
¹H-NMR (500 MHz, Chloroform-d) δ 6.04 (s, 1H), 5.98 (s, 1H), 4.57-4.50 (m, 1H), 3.56 (d, J = 7.6 Hz, 5H), 3.31 (s, 3H), 1.36 (d, J = 6.1 Hz, 6H); LC/MS: 236 (M+H).

### Intermediate I-24: Preparation of 4-acetyl-6-amino-1,3-dimethyl-1,3-dihydro-2H-benzo[d]imidazol-2-one

### Step 1: Preparation of 4-(1-ethoxyvinyl)-1,3-dimethyl-6-nitro-1,3-dihydro-2H-benzo[d]imidazol-2-one

4-Bromo-1,3-dimethyl-6-nitro-1,3-dihydro-2H-benzo[d]imidazol-2-one (400 mg, 1.398 mmol) was added to 1,4-dioxane (6 mL), and tributyl(1-ethoxyvinyl)stannane (0.566 mL, 1.678 mmol) and bis(triphenylphosphine)palladium(II) dichloride (49.1 mg, 0.070 mmol) were added. The solution was bubbled with nitrogen for 5 minutes. The reaction solution was stirred at 90°C under nitrogen conditions for 18 hours. Water and 1 N hydrochloric acid were added to the reaction solution, and the resultant was extracted twice with ethyl acetate. An organic layer was washed with water and brine, dried over anhydrous magnesium sulfate and concentrated under reduced pressure, and the residue was purified by MPLC to obtain the title compound. (218.8 mg, 0.789 mmol, 56.4% yield).

¹H-NMR (500 MHz, Chloroform-d) δ 8.03 (s, 1H), 7.85 (s, 1H), 4.51 (s, 1H), 4.41 (s, 1H), 3.98 (q, J = 6.9 Hz, 2H), 3.53 (s, 3H), 3.51 (s, 3H), 1.42 (t, J = 6.9 Hz, 3H).

### Step 2: Preparation of 4-acetyl-6-amino-1,3-dimethyl-1,3-dihydro-2H-benzo[d]imidazol-2-one

The title compound was obtained in a similar manner to Step 2 of Synthetic Method I-C, using 4-(1-ethoxyvinyl)-1,3-dimethyl-6-nitro-1,3-dihydro-2H-benzo[d]imidazol-2-one (125 mg, 0.451 mmol) obtained in Step 1 as a starting material. (73.5 mg, 0.335 mmol, 74.4% yield)
¹H-NMR (500 MHz, Chloroform-d) δ 6.68 (s, 1H), 6.49 (s, 1H), 3.67 (s, 2H), 3.42-3.35 (6H), 2.62 (s, 3H); LC/MS: 220(M+H).

### Intermediate I-43 (Synthetic Method I-F): Preparation of 6-amino-1,3-dimethyl-4-(pyridin-4-yl)-1,3-dihydro-2H-benzo[d]imidazol-2-one

### Step 1: Preparation of 1,3-dimethyl-6-nitro-4-(pyridin-4-yl)-1,3-dihydro-2H-benzo[d]imidazol-2-one

4-Bromo-1,3-dimethyl-6-nitro-1,3-benzodiazol-2-one (5 g, 17.48 mmol) and pyridin-4-yl boronic acid (4.30 g, 34.95 mmol) were added to 1,4-dioxane (150 mL) and water (25 mL), and tetrakis(triphenylphosphine)palladium (2.02 g, 1.748 mmol), and sodium carbonate (7.41 g, 69.91 mmol) were added and stirred at 100°C for 24 hours. The reaction solution was cooled to room temperature, filtered, and washed with dichloromethane. The filtrate was washed with brine, and an organic layer was dried over anhydrous sodium sulfate and concentrated under reduced pressure. The title compound was obtained by separation using MPLC. (3 g, 9.97 mmol, 57.06% yield)
LC/MS: 285 (M+H).

### Step 2: Preparation of 6-amino-1,3-dimethyl-4-(pyridin-4-yl)-1,3-dihydro-2H-benzo[d]imidazol-2-one

The title compound was obtained in a similar manner to Step 2 of Synthetic Method I-D using 1,3-dimethyl-6-nitro-4-(pyridin-4-yl)-1,3-dihydro-2H-benzo[d]imidazol-2-one (2.5 g, 8.79 mmol) obtained in Step 1 as a starting material. (1.5 g, 5.69 mmol, 64.73% yield)
¹H-NMR (400 MHz, DMSO-d₆) δ 8.69-8.53 (m, 2H), 7.50-7.29 (m, 2H), 6.44 (d, J = 2.1 Hz, 1H), 6.16 (d, J = 2.1 Hz, 1H), 4.99 (s, 2H), 3.26 (s, 3H), 2.82 (s, 3H).

### Intermediate I-50: Preparation of 1-bromo-2-(difluoromethyl)-4-fluoro-5-nitrobenzene

### Step 1: Preparation of (2-bromo-5-fluorophenyl)methyl 2,2-dimethylproponate

(2-Bromo-5-fluorophenyl)methanol (200 g, 0.976 mol) and triethylamine (370.7 mL, 2.93 mol) were dissolved in dichloromethane (3 L), and then trimethylacetyl chloride (141.15 g, 1.17 mol) was slowly added dropwisely under nitrogen conditions at 0°C. The reaction solution was stirred at room temperature for 2 hours. After the reaction was completed, water was added, and an organic layer was extracted with dichloromethane. The organic layer was washed with water and brine, dried over anhydrous magnesium sulfate, and concentrated under reduced pressure. The residue was purified by MPLC to obtain the title compound. (144 g, 0.498 mol, 51.1% yield)

### Step 2: Preparation of (2-bromo-5-fluoro-4-nitrophenyl)methanol

(2-Bromo-5-fluorophenyl)methyl 2,2-dimethylproponate (160 g, 553.36 mmol) obtained in Step 1 was dissolved in sulfuric acid (1.6 L), and potassium nitrate (67.13 g, 664.03 mmol) was slowly added dropwisely at 0°C under nitrogen conditions. The reaction solution was stirred at 0°C for 3 hours. After the reaction was completed, ice water was poured into the reaction solution, and an organic layer was extracted with dichloromethane. The organic layer was washed with water and brine, dried over anhydrous magnesium sulfate and concentrated under reduced pressure, and the residue was purified by MPLC to obtain the title compound. (50 g, 199.98 mmol, 36.14% yield)

### Step 3: Preparation of 2-bromo-5-fluoro-4-nitrobenzaldehyde

(2-Bromo-5-fluoro-4-nitrophenyl)methanol (40 g, 159.99 mmol) obtained in Step 2 was dissolved in dichloromethane (500 mL), and Dess-Martin (135.71 g, 319.97 mmol) was slowly added dropwisely at 0°C under nitrogen conditions. The reaction solution was stirred at room temperature for 3 hours. After the reaction was completed, a saturated aqueous sodium thiosulfate solution was added at room temperature, and an organic layer was extracted with dichloromethane. The organic layer was washed with water and brine, dried over anhydrous magnesium sulfate and concentrated under reduced pressure, and the residue was purified by MPLC to obtain the title compound. (30.8 g, 124.2 mmol, 77.63% yield)
¹H-NMR (400 MHz, DMSO-d₆) δ 10.16 (d, J = 2.6 Hz, 1H), 8.55 (d, J = 6.5 Hz, 1H), 7.93 (d, J = 10.8 Hz, 1H).

### Step 4: Preparation of 1-bromo-2-(difluoromethyl)-4-fluoro-5-nitrobenzene

2-Bromo-5-fluoro-4-nitrobenzaldehyde (31.5 g, 127 mmol) obtained in Step 3 was dissolved in dichloromethane (300 mL), and then diethylaminosulfur trifluoride (35.2 mL, 267 mmol) was added at 0°C. The reaction solution was stirred for 4 hours. After the reaction was completed, water was slowly added at 0°C to terminate the reaction, and an organic layer was extracted with dichloromethane. The organic layer was washed with a saturated aqueous sodium bicarbonate solution, dried over anhydrous magnesium sulfate and concentrated under reduced pressure, and the residue was purified by MPLC to obtain the title compound. (32.4 g, 120 mmol, 94% yield)
¹H-NMR (500 MHz, Chloroform-d) δ 8.33 (d, J = 6.7 Hz, 1H), 7.64 (d, J = 10.4 Hz, 1H), 6.87 (t, J = 54.0 Hz, 1H).

### Intermediate I-51: Preparation of 1-bromo-2-(difluoromethoxy)-4-fluoro-5-nitrobenzene

### Step 1: Preparation of 1-bromo-2-(difluoromethoxy)-4-fluorobenzene

2-Bromo-5-fluorophenol (25 g, 130.89 mmol) was dissolved in dimethylformamide, and then sodium carbonate (27.75 g, 261.78 mmol) was slowly added at room temperature, and 2-chloro-2,2-difluoroacetic acid (25.62 g, 196.34 mmol) was slowly added thereto. The reaction solution was stirred for 3 hours at room temperature under nitrogen conditions. After the reaction was completed, water was added, and an organic layer was extracted with ethyl acetate. The organic layer was washed with water and brine, dried over anhydrous magnesium sulfate, and concentrated under reduced pressure. The residue was purified by MPLC to obtain the title compound. (16.6 g, 68.85 mmol, 52.62% yield)

### Step 2: Preparation of 1-bromo-2-(difluoromethoxy)-4-fluoro-5-nitrobenzene

1-Bromo-2-(difluoromethoxy)-4-fluorobenzene (7.8 g, 32.36 mmol) obtained in Step 1 was dissolved in sulfuric acid, and potassium nitrate (4.58 g, 45.31 mmol) was added little by little at -10°C. The reaction solution was stirred under nitrogen conditions at 0°C for 3 hours. After the reaction was completed, ice was added to terminate the reaction. Water was added, and an organic layer was extracted with ethyl acetate. The organic layer was washed with water, dried over anhydrous magnesium sulfate and concentrated under reduced pressure, and the residue was purified by MPLC to obtain the title compound. (5.1 g, 17.80 mmol, 55.10% yield)
¹H-NMR (400 MHz, Chloroform-d) δ 8.40 (d, J = 7.8 Hz, 1H), 7.23 (d, J = 11.0 Hz, 1H), 6.71 (t, 1H).

### Intermediate I-52: Preparation of 1-bromo-4-fluoro-5-nitro-2-(trifluoromethoxy)benzene

### Step 1: Preparation of 2-fluoro-1-nitro-4-(trifluoromethoxy)benzene and 4-fluoro-1-nitro-2-(trifluoromethoxy)benzene

A mixture of the two title compounds was obtained in a similar manner to Step 2 of the synthesis of Intermediate I-51, using 1-fluoro-3-(trifluoromethoxy)benzene (15 g, 83.29 mmol) as a starting material. (12.1 g, 53.72 mmol, 64.54% yield)
LC/MS: 226 (M+H).

### Step 2: Preparation of 1-bromo-4-fluoro-5-nitro-2-(trifluoromethoxy)benzene and 1-bromo-2-fluoro-5-nitro-4-(trifluoromethoxy)benzene

A mixture of 2-fluoro-1-nitro-4-(trifluoromethoxy)benzene and 4-fluoro-1-nitro-2-(trifluoromethoxy)benzene, obtained in Step 1 (15 g, 66.64 mmol) and sulfuric acid (3 mL) were dissolved in trifluoroacetic acid (150 mL), and then N-bromosuccinimide (35.58 g, 199.91 mmol) was slowly added little by little at room temperature. The reaction solution was stirred at 80°C for 18 hours. After the reaction was completed, water was added and an organic layer was extracted with ethyl acetate. The organic layer was washed with water and brine, dried over anhydrous magnesium sulfate and concentrated under reduced pressure, and the residue was purified by reverse phase chromatography to obtain a mixture of the two title compounds. (10.2 g, 33.72 mmol, 50.6% yield)
¹H-NMR (400 MHz, Chloroform-d) δ 8.33 (d, J = 7.7 Hz, 1H), 8.23 (d, J = 6.7 Hz, 1H), 7.30-7.16 (m, 2H); LC/MS: 304 (M+H) (isomer mixture).

### Intermediate I-53: Preparation of 2-bromo-5-fluoro-4-nitrophenol

1-Bromo-4-fluoro-2-methoxy-5-nitrobenzene (5 g, 20 mmol) was dissolved in 1,2-dichloroethane (100 mL), and then boron tribromide (7.51 g, 30 mmol) was slowly added at 0°C. The reaction solution was stirred at 60°C under nitrogen conditions for 18 hours. After the reaction was completed, ice and water were added at 0°C, and an organic layer was extracted with ethyl acetate. The organic layer was washed with water and brine, dried over anhydrous magnesium sulfate, and concentrated under reduced pressure. The residue was purified by MPLC to obtain the title compound. (2.3 g, 9.82 mmol, 49.1% yield)
¹H-NMR (400 MHz, DMSO-d₆) δ 12.48 (s, 1H), 8.35 (d, J = 8.3 Hz, 1H), 6.94 (d, J = 13.0 Hz, 1H).

### Intermediate I-54: Preparation of 1-(benzyloxy)-2-bromo-5-fluoro-4-nitrobenzene

To a solution of 2-bromo-5-fluoro-4-nitrophenol (Intermediate I-53, 354 mg, 1.50 mmol), potassium carbonate (415 mg, 3.00 mmol), and potassium iodide (24.9 mg, 0.15 mmol), dissolved in acetone (6.00 mL), benzyl bromide (0.214 mL, 1.80 mmol) was added, followed by stirring at 56°C for 18 hours. The reaction mixture was cooled to room temperature, diluted with water, and extracted with ethyl acetate. The obtained organic layer was washed with brine, dried over anhydrous sodium sulfate and concentrated under reduced pressure, and the residue was purified by MPLC to obtain the title compound. (456 mg, 1.40 mmol, 93% yield)
¹H-NMR (500 MHz, Chloroform-d) δ 8.39 (d, J = 7.9 Hz, 1H), 7.46-7.37 (m, 5H), 6.80 (d, J = 12.2 Hz, 1H), 5.24 (s, 2H); LC/MS: 326 (M+H).

### Intermediate I-55: Preparation of 4-(2,4-difluoro-5-nitrophenyl)-1-methyl-1H-pyrazole

Bromo-2,4-difluoro-5-nitrobenzene (3 g, 12.61 mmol), 1-methyl-4-(4,4,5,5,-tetramethyl-1,3,2-dioxaborolan-2-yl)-1H-pyrazole (3.67 g, 17.65 mmol), tetrakis(triphenylphosphine)palladium (0.728 g, 0.630 mmol) and sodium carbonate (2.67 g, 25.2 mmol) were stirred in a 1,4-dioxane (40 mL) and water (10 mL) solvent at 90°C for 18 hours. The reaction product was diluted in ethyl acetate, washed with water, dried over anhydrous magnesium sulfate, and concentrated under reduced pressure. The residue was purified by MPLC to obtain the title compound. (2.75 g, 11.5 mmol, 91% yield)
¹H-NMR (500 MHz, Chloroform-d) δ 8.30 (t, J = 7.71 Hz, 1H), 7.87 (s, 1H), 7.81 (s, 1H), 7.10 (t, J = 10.22 Hz, 1H), 3.99 (s, 3H); LC/MS: 240 (M+H).

### Intermediate I-56: Preparation of ((2,4-difluoro-5-nitrophenyl)ethynyl)trimethylsilane

1-Bromo-2,4-difluoro-5-nitrobenzene (213 mg, 0.895 mmol), bis(triphenylphosphine)palladium(II) dichloride (62.8 mg, 0.090 mmol), copper(I) iodide (25.6 mg, 0.134 mmol), and triethylamine (624 µL, 4.48 mmol) were dissolved in toluene (4.8 mL), and then ethynyltrimethylsilane (371 µL, 2.69 mmol) was added. The reaction solution was stirred at room temperature for 7 hours. After the reaction was completed, water was added, and an organic layer was extracted with ethyl acetate. The organic layer was washed with water and brine, dried over anhydrous magnesium sulfate and concentrated under reduced pressure, and the residue was purified by MPLC to obtain the title compound. (197 mg, 0.772 mmol, 86% yield)
¹H-NMR (500 MHz, Chloroform-d) δ 8.25 (t, J = 7.5 Hz, 1H), 7.04 (t, J = 9.3 Hz, 1H), 0.28 (s, 9H).

### Intermediate I-57: Preparation of 5-(2,4-difluoro-5-nitrophenyl)-3-methyl-1,2,4-oxadiazole

### Step 1: Preparation of 2,4-difluoro-5-nitrobenzoic acid

Methyl 2,4-difluoro-5-nitrobenzoate (1 g, 4.61 mmol) was dissolved in tetrahydrofuran (10 mL) and water (5 mL), and lithium hydroxide (0.221 g, 9.21 mmol) was slowly added. The reaction solution was stirred at room temperature for 3 hours. After the reaction was completed, the reaction solution was concentrated under reduced pressure and diluted with ethyl acetate. The pH of the reaction solution was adjusted to 5-6 with 1 N hydrochloric acid solution, and an organic layer was extracted. The organic layer was washed with water and brine, dried over anhydrous magnesium sulfate, and concentrated under reduced pressure to obtain the title compound, which was used directly in a subsequent step without further purification. (955 mg, 4.70 mmol, 100% yield)
¹H-NMR (500 MHz, DMSO-d₆) δ 13.86 (s, 1H), 8.61 (t, J = 7.8 Hz, 1H), 7.87 (t, J = 10.8 Hz, 1H).

### Step 2: Preparation of 5-(2,4-difluoro-5-nitrophenyl)-3-methyl-1,2,4-oxadiazole

2,4-Difluoro-5-nitrobenzoic acid (200 mg, 0.99 mmol) and N-hydroxyacetimidamide (76 mg, 0.99 mmol) obtained in Step 1 were dissolved in ethyl acetate (2.5 mL), triethylamine (0.4 mL, 2.95 mmol) was added, and 2,4,6-tripropyl-1,3,5,2,4,6-trioxatriphosphinane 2,4,6-trioxide (50 wt% in ethyl acetate, 5 mL, 2.46 mmol) was slowly added. The reaction solution was stirred at 80°C for 18 hours. After the reaction was completed, water was added, and an organic layer was extracted with ethyl acetate. The organic layer was washed with a saturated sodium bicarbonate aqueous solution and brine, dried over anhydrous magnesium sulfate and concentrated under reduced pressure, and the residue was purified by MPLC to obtain the title compound. (68.8 mg, 0.285 mmol, 29.0% yield)
¹H-NMR (500 MHz, Chloroform-d) δ 8.92 (t, J = 7.2 Hz, 1H), 7.28 (t, J = 9.6 Hz, 1H), 2.49 (s, 3H).

### Intermediate I-58: Preparation of 2,2-dimethyl-3-oxopropanenitrile

### Step 1: Preparation of 3-hydroxy-2,2-dimethylpropanenitrile

Sodium borohydride (8.93 g, 235.96 mmol) was dissolved in methanol (100 mL) and stirred at 0°C for 5 minutes. Methyl 2-cyano-2,2-dimethylacetate (10 g, 78.65 mmol) was slowly added at 0°C over 5 minutes. The reaction solution was stirred at room temperature for 18 hours. After the reaction was completed, the reaction solution was concentrated under reduced pressure, water and dichloromethane were added, and an organic layer was extracted. The organic layer was washed with a saturated ammonium chloride aqueous solution, dried over anhydrous magnesium sulfate and concentrated under reduced pressure, and the residue was purified by MPLC to obtain the title compound. (6.4 g, 64.6 mmol, 82.1% yield)
¹H-NMR (400 MHz, DMSO-d₆) δ 5.43 (t, J = 5.7 Hz, 1H), 3.36 (d, J = 5.4 Hz, 2H), 1.23 (s, 6H).

### Step 2: Preparation of 2,2-dimethyl-3-oxopropanenitrile

Oxalyl chloride (8.25 mL, 96.84 mmol) was dissolved in dichloromethane (80 mL), and then dimethyl sulfoxide (8.60 mL, 121.05 mmol) was added at -78°C under nitrogen conditions. 3-Hydroxy-2,2-dimethylpropanenitrile (8 g, 80.7 mmol) obtained in Step 1 was slowly added at -78°C. The reaction solution was stirred at -78°C for 40 minutes under nitrogen conditions. Triethylamine (56.09 mL, 403.5 mmol) was slowly added at -78°C for 5 minutes. The reaction solution was further stirred at room temperature for 2 hours. After the reaction was completed, the reaction solution was concentrated under reduced pressure, water and ethyl acetate were added, and an organic layer was extracted. The organic layer was washed with brine, dried over anhydrous magnesium sulfate, and concentrated under reduced pressure to obtain the title compound, which was used directly in a subsequent step without further purification. (4 g, 41.16 mmol, 51.0% yield)
¹H-NMR (400 MHz, DMSO-d₆) δ 9.41 (s, 1H), 1.32-1.00 (m, 6H).

In addition, intermediates synthesized in a similar manner as above and the synthetic methods are listed in Table 1.

**[Table 1]**

| **No.** | **Compound name** | **¹H-NMR / MS** | **Synthetic method** |
|---|---|---|---|
| I-3 | 6-amino-4-bromo-1,3-dimethyl-1,3-dihydro-2H-benzo[d]imidazol-2-one | ¹H-NMR (500 MHz, Chloroform-d) δ 6.59 (s, 1H), 6.29 (s, 1H), 3.90 (s, 3H), 3.60 (s, 2H), 3.35 (s, 3H); m/z N.D. | I-A |
| I-5 | tert-butyl 4-(6-amino-1,3-dimethyl-2-oxo-2,3-dihydro-1H-benzo[d]imidazol-4-yl)-3,6-dihydropyridin-1(2H)-carboxylate | ¹H-NMR (500 MHz, DMSO-d₆) δ 6.90 (d, J = 2.1 Hz, 1H), 6.61 (d, J = 2.1 Hz, 1H), 5.96 (s, 1H), 4.78 (s, 2H), 3.89 (s, 3H), 3.87 (s, 3H), 3.55-3.42 (m, 2H), 3.33 (t, J = 5.5 Hz, 2H), 2.07 (s, 2H), 1.42 (s, 9H); m/z N.D. | I-F |
| I-6 | 6-amino-1,3-dimethyl-4-((2-morpholinoethyl)amino)-1,3-dihydro-2H-benzo[d]imidazol-2-one | ¹H-NMR (500 MHz, Methanol-d₄) δ 6.01 (t, J = 2.3 Hz, 2H), 3.75-3.68 (m, 4H), 3.65 (s, 3H), 3.28 (s, 3H), 3.22 (t, J = 6.2 Hz, 2H), 2.68 (t, J = 6.2 Hz, 2H), 2.53 (s, 4H); m/z N.D. | I-C |
| I-7 | 6-amino-4-(cyclopentylamino)-1,3-dimethyl-1,3-dihydro-2H-benzo[d]imidazol-2-one | ¹H-NMR (500 MHz, Chloroform-d) δ 6.15-6.13 (m, 1H), 6.12 (s, 1H), 3.81-3.75 (m, 1H), 3.65 (s, J = 5.7 Hz, 3H), 3.30 (s, 3H), 2.07-2.00 (m, 2H), 1.73 (dt, J = 12.3, 3.7 Hz, 2H), 1.68-1.59 (m, 2H), 1.55-1.48 (m, 2H); m/z N.D. | I-C |
| I-9 | 6-amino-4-(cyclopropylamino)-1,3-dimethyl-1,3-dihydro-2H-benzo[d]imidazol-2-one | ¹H-NMR (500 MHz, Methanol-d₄) δ 6.43 (d, J = 1.9 Hz, 1H), 6.05 (d, J = 1.9 Hz, 1H), 3.56 (s, 3H), 3.28 (s, 3H), 2.45-2.39 (m, 1H), 0.78-0.72 (m, 2H), 0.53-0.48 (m, 2H); m/z N.D. | I-C |
| I-10 | 6-amino-1,3-dimethyl-4-morpholino-1,3-dihydro-2H-benzo[d]imidazol-2-one | ¹H-NMR (500 MHz, DMSO-d₆) δ 6.16 (d, J = 1.9 Hz, 1H), 6.11 (d, J = 1.9 Hz, 1H), 4.81 (s, 2H), 3.73 (s, 4H), 3.49 (s, 3H), 3.17 (s, 3H), 2.83 (s, 4H); m/z N.D. | I-C |
| I-11 | 6-amino-1,3-dimethyl-4-(1-methyl-1,2,3,6-tetrahydropyridin -4-yl)-1,3-dihydro-2H-benzo[d]imidazol-2-one | ¹H-NMR (500 MHz, DMSO-d₆) δ 6.25 (d, J = 2.1 Hz, 1H), 6.03 (d, J = 2.1 Hz, 1H), 5.58-5.55 (m, 1H), 4.79 (s, 2H), 3.22 (s, 3H), 3.20 (s, 3H), 2.97 (dd, J = 5.8, 2.8 Hz, 2H), 2.55 (t, J = 5.6 Hz, 2H), 2.32 (d, J = 1.8 Hz, 2H), 2.27 (s, 3H); m/z N.D. | I-F |
| I-14 | 6-amino-4-(isopropylamino)-1,3-dimethyl-1,3-dihydro-2H-benzo[d]imidazol-2-one | ¹H-NMR (500 MHz, Chloroform-d) δ 5.85 (s, 2H), 3.64 (s, 3H), 3.60-3.55 (m, 2H), 3.29 (s, 3H), 1.24 (d, J = 5.8 Hz, 6H); m/z N.D. | I-D |
| I-15 | 6-amino-4-(cyclopentylamino)-1,3-dimethyl-1,3-dihydro-2H-benzo[d]imidazol-2-one | ¹H-NMR (500 MHz, Chloroform-d) δ 5.86 (d, J = 9.61 Hz, 2H), 3.78 (br. s., 1H), 3.64 (s, 4H), 3.30 (s, 3H), 2.04 (d, J = 7.02 Hz, 2H), 1.75 (br. s., 2H), 1.66 (br. s., 2H); m/z N.D. | I-D |
| I-16 | 6-amino-4-(cyclobutylamino)-1,3-dimethyl-1,3-dihydro-2H-benzo[d]imidazol-2-one | ¹H-NMR (500 MHz, Chloroform-d) δ 5.85 (s, 1H), 5.73 (s, 1H), 4.00-3.82 (m, 2H), 3.66 (s, 3H), 3.52 (br. s., 1H), 3.30 (s, 3H), 2.46 (br. s., 2H), 1.98-1.74 (m, 4H); m/z N.D. | I-D |
| I-17 | 6-amino-4-(tert-butylamino)-1,3-dimethyl-1,3-dihydro-2H-benzo[d]imidazol-2-one | ¹H-NMR (500 MHz, Methanol-d₄) δ 6.32 (s, 1H), 6.22 (s, 1H), 3.65 (s, 3H), 1.28 (s, 9H); m/z N.D. | I-D |
| I-19 | 6-amino-1,3-dimethyl-4-(phenylamino)-1,3-dihydro-2H-benzo[d]imidazol-2-one | ¹H-NMR (500 MHz, Chloroform-d) δ 7.20 (t, J = 7.55 Hz, 2H), 6.83 (t, J = 7.32 Hz, 1H), 6.70 (d, J = 7.93 Hz, 2H), 6.23 (s, 1H), 6.25 (s, 1H), 5.29 (s, 1H), 3.57 (br. s., 2H), 3.41 (s, 3H), 3.36 (s, 3H); m/z N.D. | I-D |
| I-20 | 6-amino-4-((4-fluorophenyl)amino)-1,3-dimethyl-1,3-dihydro-2H-benzo[d]imidazol-2-one | ¹H-NMR (500 MHz, Chloroform-d) δ 6.91 (t, J = 8.16 Hz, 2H), 6.66 (br. s., 2H), 6.21 (d, J = 9.77 Hz, 2H), 3.58 (br. s., 2H), 3.41 (s, 3H), 3.36 (s, 3H); m/z N.D. | I-D |
| I-21 | 6-amino-4-((4-(dimethylamino)phenyl)ami no)-1,3-dimethyl-1,3-dihydro-2H-benzo[d]imidazol-2-one | ¹H-NMR (500 MHz, Chloroform-d) δ 6.85-6.74 (m, J = 8.54 Hz, 2H), 6.74-6.67 (m, J = 8.54 Hz, 2H), 6.10 (s, 1H), 6.12 (s, 1H), 3.49 (s, 5H), 3.35 (s, 3H), 2.89 (s, 6H); m/z N.D. | I-D |
| I-22 | 6-amino-4-((4-methoxyphenyl)amino)-1,3-dimethyl-1,3-dihydro-2H-benzo[d]imidazol-2-one | ¹H-NMR (500 MHz, Chloroform-d) δ 6.85-6.78 (m, J = 7.48 Hz, 2H), 6.78-6.68 (m, J = 7.48 Hz, 2H), 6.16 (s, 2H), 3.77 (s, 3H), 3.55 (br. s., 2H), 3.45(s, 3H), 3.36 (s, 3H); m/z N.D. | I-D |
| I-23 | 6-amino-4-isopropyl-1,3-dimethyl-1,3-dihydro-2H-benzo[d]imidazol-2-one | ¹H-NMR (500 MHz, Chloroform-d) δ 6.39 (s, 1H), 6.22 (s, 1H), 3.61 (s, 3H), 3.57-3.51 (m, 1H), 3.33 (s, 3H), 1.28 (d, J = 6.7 Hz, 6H); m/z 220. | I-B |
| I-25 | 6-amino-4-(cyclohexylamino)-1,3-dimethyl-1,3-dihydro-2H-benzo[d]imidazol-2-one | ¹H-NMR (500 MHz, Chloroform-d) δ 5.85 (d, J = 11.90 Hz, 2H), 3.65 (s, 4H), 3.52 (br. s., 2H), 3.30 (s, 3H), 3.24 (br. s., 1H), 2.07 (d, J = 13.58 Hz, 2H), 1.77 (d, J = 13.43 Hz, 2H), 1.66 (d, J = 13.58 Hz, 1H), 1.44-1.33 (m, 2H), 1.30-1.18 (m, 3H); m/z N.D. | I-D |
| I-26 | 6-amino-4-(sec-butylamino)-1,3-dimethyl-1,3-dihydro-2H-benzo[d]imidazol-2-one | ¹H-NMR (500 MHz, Chloroform-d) δ 5.84 (s, 2H), 3.69-3.58 (m, 4H), 3.52 (br. s., 2H), 3.38 (d, J = 6.10 Hz, 1H), 3.30 (s, 3H), 1.63 (dd, J = 6.79, 13.96 Hz, 1H), 1.53 (dd, J = 7.17, 14.19 Hz, 1H), 1.21 (d, J = 6.26 Hz, 3H), 0.98 (t, J = 7.48 Hz, 3H); m/z N.D. | I-D |
| I-27 | 6-amino-4-(3,3-difluoroazetidin-1-yl)-1,3-dimethyl-1,3-dihydro-2H-benzo[d]imidazol-2-one | ¹H-NMR (300 MHz, DMSO-d₆) δ 6.09-5.99 (m, 2H), 4.24 (s, 1H), 4.18 (d, J = 12.5 Hz, 3H), 3.43 (s, 3H), 3.32 (s, 2H), 3.17 (s, 3H); m/z N.D. | I-D |
| I-28 | 6-amino-4-(azetidin-1-yl)-1,3-dimethyl-1,3-dihydro-2H-benzo[d]imidazol-2-one | ¹H-NMR (300 MHz, Methanol-d₄) δ 6.16 (dd, J = 11.7, 2.0 Hz, 2H), 3.82 (t, J = 7.1 Hz, 4H), 3.59 (s, 3H), 3.28 (s, 3H), 2.25 (p, J = 7.1 Hz, 2H); m/z N.D. | I-D |
| I-29 | 6-amino-4-(3-hydroxy-3-methylazetidin-1-yl)-1,3-dimethyl-1,3-dihydro-2H-benzo[d]imidazol-2-one | ¹H-NMR (500 MHz, Chloroform-d) δ 6.03 (s, 1H), 5.99 (s, 1H), 3.78-3.68 (m, 4H), 3.61 (s, 3H), 3.57 (br. s., 2H), 3.31 (s, 3H), 1.94 (s, 1H), 1.65 (s, 3H); m/z N.D. | I-D |
| I-30 | 6-amino-4-(3-hydroxyazetidin-1-yl)-1,3-dimethyl-1,3-dihydro-2H-benzo[d]imidazol-2-one | N.D.; | I-D |
| | | m/z 249. | |
| I-31 | tert-butyl 4-(6-amino-1,3-dimethyl-2-oxo-2,3-dihydro-1H-benzo[d]imidazol-4-yl)piperidin-1-carboxylate | ¹H-NMR (400 MHz, DMSO-d₆) δ 6.23 (s, 2H), 4.75 (s, 2H), 4.06 (d, J = 13.1 Hz, 2H), 3.48 (s, 3H), 3.27 (d, J = 3.2 Hz, 1H), 3.19 (s, 3H), 2.86 (s, 2H), 1.75 (d, J = 12.9 Hz, 2H), 1.42 (s, 11H); m/z 361. | I-B |
| I-32 | (R)-6-amino-1,3-dimethyl-4-(3-methylmorpholino)-1,3-dihydro-2H-benzo[d]imidazol-2-one | ¹H-NMR (500 MHz, Chloroform-d) δ 6.24 (s, 1H), 6.15 (s, 1H), 3.96 (d, 1H), 3.86-3.80 (m, 2H), 3.65 (s, 3H), 3.60 (br s, 2H), 3.32 (s, 3H), 2.97 (d, 1H), 2.93-2.86 (m, 2H), 2.58-2.53 (m, 1H), 1.20 (d, 3H); m/z 277. | I-D |
| I-33 | (S)-6-amino-1,3-dimethyl-4-(3-methylmorpholino)-1,3-dihydro-2H-benzo[d]imidazol-2-one | ¹H-NMR (500 MHz, Chloroform-d) δ 6.26 (s, 1H), 6.17 (s, 1H), 3.98 (d, 1H), 3.88-3.82 (m, 2H), 3.66 (s, 3H), 3.62 (br s, 2H), 3.34 (s, 3H), 2.99 (d, 1H), 2.95-2.88 (m, 2H), 2.60-2.55 (m, 1H), 1.22 (d, 3H); m/z 277. | I-D |
| I-34 | 6-amino-1,3-dimethyl-4-[(2R)-2-methylazetidin-1-yl]-1,3-benzodiazol-2-one | ¹H-NMR (300 MHz, DMSO-d₆) δ 5.97 (s, 2H), 4.12 (p, J = 6.7 Hz, 1H), 3.84 (ddd, J = 8.5, 6.4, 3.3 Hz, 1H), 3.45 (s, 3H), 3.34-3.26 (m, 1H), 3.15 (s, 3H), 2.21 (dtd, J = 10.6, 7.7, 3.2 Hz, 1H), 2.02-1.80 (m, 1H), 1.18 (d, J = 6.1 Hz, 3H); m/z N.D. | I-D |
| I-35 | tert-butyl 4-(6-amino-1,3-dimethyl-2-oxo-2,3-dihydro-1H-benzo[d]imidazol-4-yl)piperazin-1-carboxylate | ¹H-NMR (500 MHz, Chloroform-d) δ 6.24 (s, 1H), 6.17 (s, 1H), 4.23-4.00 (m, 2H), 3.67 (s, 3H), 3.59 (br. s., 2H), 3.43-3.29 (m, 3H), 3.04 (br. s., 4H), 2.79 (br. s., 2H), 1.51 (s, 9H); m/z N.D. | I-D |
| I-36 | 6-amino-1,3-dimethyl-4-(2-morpholinoethoxy)-1,3-dihydro-2H-benzo[d]imidazol-2-one | ¹H-NMR (500 MHz, Chloroform-d) δ 6.03 (s, 1H), 6.01 (s, 1H), 4.14 (t, J =5.0 Hz, 2H), 3.73 (s, 4H), 3.58 (s, 3H), 3.31 (s, 3H), 2.83-2.82 (m, 2H), 2.57 (s, 4H); m/z 307. | I-E |
| I-37 | 6-amino-1,3-dimethyl-4-(oxetan-3-yloxy)-1,3-dihydro-2H-benzo[d]imidazol-2-one | ¹H-NMR (500 MHz, Chloroform-d) δ 6.02 (s, 1H), 5.54 (s, 1H), 5.22 (dd, J = 16.3, 5.3 Hz, 1H), 4.99 (t, J = 6.6 Hz, 2H), 4.80-4.77 (m, 2H), 3.61 (s, 3H), 3.56 (s, 2H), 3.32 (s, 3H); m/z 250. | I-E |
| I-38 | 6-amino-4-((4S)-2-oxa-5-azabicyclo[2.2.1]heptan-5-yl)-1,3-dimethyl-1,3-dihydro-2H-benzo[d]imidazol-2-one | ¹H-NMR (500 MHz, Chloroform-d) δ 6.31 (s, 1H), 6.10 (s, 1H), 4.62 (br. s., 1H), 4.04 (d, J = 7.63 Hz, 1H), 3.87 (s, 1H), 3.67 (d, J = 7.78 Hz, 1H), 3.65 (s, 3H), 3.57 (br. s., 2H), 3.50 (d, J = 10.22 Hz, 1H), 3.32 (s, 3H), 3.17 (d, J = 10.07 Hz, 1H), 2.08 (d, J = 9.77 Hz, 1H), 1.94 (d, J = 9.61 Hz, 1H); m/z N.D. | I-D |
| I-39 | 6-amino-4-(benzyloxy)-1,3-dimethyl-1,3-dihydro-2H-benzo[d]imidazol-2-one | ¹H-NMR (400 MHz, DMSO-d₆) δ 7.50-7.44 (m, 2H), 7.44-7.38 (m, 2H), 7.37-7.30 (m, 1H), 6.15 (d, J = 1.7 Hz, 1H), 5.99 (d, J = 1.7 Hz, 1H), 5.09 (s, 2H), 4.86 (s, 2H), 3.39 (s, 3H), 3.17 (s, 3H); m/z 284. | I-E |
| I-40 | 6-amino-1,3-dimethyl-4-thiomorpholino-1,3-dihydro-2H-benzo[d]imidazol-2-one | ¹H-NMR (500 MHz, Chloroform-d) δ 6.26 (s, 1H), 6.16 (s, 1H), 3.68-3.62 (m, 3H), 3.58 (br. s., 2H), 3.44-3.27 (m, 5H), 3.18 (br. s., 1H), 3.05 (br. s., 4H), 2.57 (br. s., 2H); m/z N.D. | I-D |
| I-41 | (R)-6-amino-1,3-dimethyl-4-(2-methylazetidin-1-yl)-1,3-dihydro-2H-benzo[d]imidazol-2-one | ¹H-NMR (300 MHz, DMSO-d₆) δ 5.97 (s, 2H), 4.12 (p, J = 6.7 Hz, 1H), 3.84 (ddd, J = 8.5, 6.4, 3.3 Hz, 1H), 3.45 (s, 3H), 3.34-3.26 (m, 1H), 3.15 (s, 3H), 2.21 (dtd, J = 10.6, 7.7, 3.2 Hz, 1H), 2.02-1.80 (m, 1H), 1.18 (d, J = 6.1 Hz, 3H); m/z N.D. | I-D |
| I-42 | 6-amino-4-(3-hydroxypyrrolidin-1-yl)-1,3-dimethyl-1,3-dihydro-2H-benzo[d]imidazol-2-one | ¹H-NMR (500 MHz, Chloroform-d) δ 6.27 (d, J = 1.98 Hz, 1H), 6.13 (d, J = 1.98 Hz, 1H), 4.55 (br. s., 1H), 3.64 (s, 3H), 3.57 (br. s., 2H), 3.33 (s, 3H), 3.30 (dd, J = 7.93, 15.26 Hz, 1H), 3.22 (dd, J = 5.04, 10.22 Hz, 1H), 3.07 (dd, J = 1.83, 10.22 Hz, 1H), 2.93 (dt, J = 5.87, 8.81 Hz, 1H), 2.36-2.29 (m, 1H), 1.95-1.88 (m, 1H), 1.79 (d, J = 5.49 Hz, 1H); m/z N.D. | I-D |
| I-44 | 6-amino-4-(3-hydroxy-3-(trifluoromethyl)azetidin-1-yl)-1,3-dimethyl-1,3-dihydro-2H-benzo[d]imidazol-2-one | ¹H-NMR (400 MHz, DMSO-d₆) δ 7.20 (s, 1H), 6.12-5.91 (m, 2H), 4.84 (s, 2H), 4.10-3.97 (m, 2H), 3.81 (d, J = 8.6 Hz, 2H), 3.42 (s, 3H), 3.17 (d, J = 5.3 Hz, 3H); m/z N.D. | I-D |
| I-45 | 6-amino-4-(4-hydroxy-4-methylpiperidin-1-yl)-1,3-dimethyl-1,3-dihydro-2H-benzo[d]imidazol-2-one | ¹H-NMR (500 MHz, Chloroform-d) δ 6.31 (br. s., 1H), 6.13 (d, J = 1.98 Hz, 1H), 3.72-3.64 (m, 3H), 3.60 (br. s., 2H), 3.33 (s, 3H), 3.04 (br. s., 2H), 2.94 (br. s., 2H), 1.84 (br. s., 2H), 1.70 (br. s., 2H), 1.34 (s, 3H); m/z N.D. | I-D |
| I-46 | 6-amino-1,3-dimethyl-4-(1,4-dioxa-8-azaspiro[4.5]decan-8-yl)-1,3-dihydro-2H-benzo[d]imidazol-2-one | ¹H-NMR (400 MHz, DMSO-d₆) δ 6.18 (d, J = 1.9 Hz, 1H), 6.09 (d, J = 1.9 Hz, 1H), 4.77 (s, 2H), 3.91 (s, 4H), 3.49 (s, 3H), 3.17 (s, 3H), 3.05-2.71 (m, 4H), 1.77 (s, 4H); m/z 319. | I-D |
| I-47 | tert-butyl (1-(6-amino-1,3-dimethyl-2-oxo-2,3-dihydro-1H-benzo[d]imidazol-4-yl)-3-methylazetidin-3-yl)carbamate | ¹H-NMR (400 MHz, DMSO-d₆) δ 7.35 (s, 1H), 5.95 (d, J = 1.9 Hz, 1H), 5.88 (d, J = 1.9 Hz, 1H), 4.79 (s, 2H), 3.73 (d, J = 6.3 Hz, 2H), 3.60-3.56 (m, 2H), 3.41 (s, 3H), 3.15 (s, 3H), 1.55 (s, 3H), 1.39 (s, 9H); m/z 362. | I-D |
| I-48 | 6-amino-1,3-dimethyl-4-(1,4-dioxaspiro[4.5]decan-8-yl)-1,3-dihydro-2H-benzo[d]imidazol-2-one | ¹H-NMR (300 MHz, DMSO-d₆) δ 6.26 (d, J = 2.1 Hz, 1H), 6.17 (d, J = 2.0 Hz, 1H), 4.76 (s, 2H), 3.89 (d, J = 1.6 Hz, 4H), 3.47 (s, 3H), 3.18 (s, 4H), 1.71 (qd, J = 9.6, 3.8 Hz, 8H); m/z 318. | I-B |
| I-49 | 6-amino-4-(3-hydroxy-3-methylpyrolidin-1-yl)-1,3-dimethyl-1,3-dihydro-2H-benzo[d]imidazol-2-one | ¹H-NMR (500 MHz, Chloroform-d) δ 6.27 (d, J = 1.98 Hz, 1H), 6.13 (d, J = 1.98 Hz, 1H), 3.64 (s, 3H), 3.56 (br. s., 2H), 3.38-3.30 (m, 4H), 3.11-3.07 (m, 1H), 3.03-2.95 (m, 2H), 2.05 (t, J = 7.10 Hz, 2H), 2.01 (s, 1H), 1.49 (s, 3H); m/z N.D. | I-D |

The synthetic method commonly used in each example is as follows.

### Example 1 (Synthetic Method A): Preparation of 3-(7-chloro-1,3-dimethyl-2-oxo-benzimidazol-5-yl)-N-methyl-2-(6-methyl-1H-indol-3-yl)benzimidazole-5-sulfonamide

### Step 1: Preparation of 3-fluoro-N-methyl-4-nitrobenzenesulfonamide

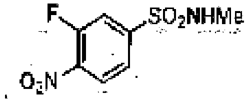

A solution of 3-fluoro-4-nitrobenzenesulfonyl chloride (10 g, 41.7 mmol) dissolved in dichloromethane (100 mL) was cooled to 0°C, and then a methanamine solution (2 M in tetrahydrofuran, 52.2 mL, 104 mmol) was slowly added dropwisely, followed by stirring at room temperature for 2 hours. After the reaction was completed, a methylamine salt was filtered and washed with dichloromethane. The residue was concentrated under reduced pressure and used in a subsequent reaction without further purification. (11.4 g, 48.6 mmol, 100% yield)
¹H-NMR (400 MHz, Chloroform-d) δ 8.26-8.14 (m, 2H), 7.80 (t, J = 9.3 Hz, 2H), 4.56 (s, 1H), 2.83-2.74 (m, 3H).

### Step 2: Preparation of 3-((7-chloro-1,3-dimethyl-2-oxo-2,3-dihydro-1H-benzo[d]imidazol-5-yl)amino)-N-methyl-4-nitrobenzenesulfonamide

.

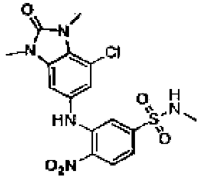

6-Amino-4-chloro-1,3-dimethyl-1,3-dihydro-2H-benzo[d]imidazol-2-one (Intermediate I-2, 0.43 g 2.05 mmol) and 3-fluoro-N-methyl-4-nitrobenzenesulfonamide (0.4 g, 1.71 mmol) obtained in Step 1 were added to 1,4-dioxane (10 mL), and diisopropylethylamine (0.6 mL, 3.42 mmol) was added, followed by stirring at 80°C for 18 hours. The reaction resultant was concentrated under reduced pressure, and the produced solid was washed with dichloromethane and methanol and filtered. The filter cake was washed with methanol, dried under nitrogen, and used in a subsequent reaction without further purification. (430 mg, 0.73 mmol, 59% yield)

### Step 3: Preparation of 4-amino-3-((7-chloro-1,3-dimethyl-oxo-2,3-dihydro-1H-benzo[d]imidazol-5-yl)amino)-N-methylbenzenesulfonamide

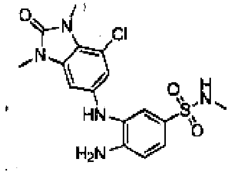

3-((7-Chloro-1,3-dimethyl-2-oxo-2,3-dihydro-1H-benzo[d]imidazol-5-yl)amino)-N-methyl-4-nitrobenzenesulfonamide (430 mg, 1.01 mmol) obtained in Step 2 and iron (169 mg, 3.03 mmol) were added to methanol (6 mL) and water (3 mL), and ammonium chloride (270 mg, 5.05 mmol) was added, and then the reaction solution was stirred at 100°C for 2 hours. The reaction solution was cooled to room temperature, and ethyl acetate (30 mL) was added, followed by stirring at room temperature for 2 hours. The reaction solution was filtered through a celite pad, the filtrate was washed with brine, and water was removed with anhydrous magnesium sulfate. An organic layer was concentrated under reduced pressure and purified by MPLC to obtain the title compound. (220 mg, 0.56 mmol, 55% yield)
¹H-NMR (400 MHz, DMSO-d₆) δ 8.49 (s, 1H), 7.02 (m, 2H), 6.96 (m, 1H), 6.90 (m, 1H), 6.73(s, 1H), 6.14(m, 1H), 4.41(d, J = 6.5 Hz, 2H), 3.57 (s, 3H), 3.27(s, 3H), 2.37 (d, J = 5 Hz, 3H).

### Step 4: Preparation of 3-(7-chloro-1,3-dimethyl-2-oxo-benzimidazol-5-yl)-N-methyl-2-(6-methyl-1H-indol-3-yl)benzimidazole-5-sulfonamide

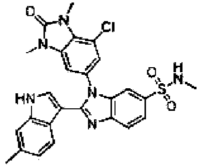

To a solution of 4-amino-3-((7-chloro-1,3-dimethyl-oxo-2,3-dihydro-1H-benzo[d]imidazol-5-yl)amino)-N-methylbenzenesulfonamide (200 mg, 0.51 mmol) obtained in Step 3 and 6-methyl-1H-indole-3-carbaldehyde (80 mg, 0.51 mmol) dissolved in acetic acid (7 mL), copper(II) acetate (92 mg, 0.51 mmol) was added. The solution was stirred at 80°C for 3 hours. The reaction solution was cooled to room temperature, water and 1 N sodium hydroxide were added, and an organic layer was extracted with ethyl acetate and then washed with brine. The organic layer was concentrated under reduced pressure and separated by MPLC to obtain the title compound. (16 mg, 0.03 mmol, 5.92% yield)
¹H-NMR (500 MHz, DMSO-d₆) δ 11.27 (s, 1H), 8.49 (d, 1H), 8.12 (s, 1H), 7.62 (m, 2H), 7.43 (s, 1H), 7.37 (m, 1H), 7.25 (m, 2H), 7.08 (d, 1H), 6.68 (d, 1H), 3.73 (s, 3H), 3.35 (s, 3H), 2.43 (s, 6H).

### Examples 3 and 4 (Synthetic Method B): Preparation of methyl 6-bromo-3-(1,3-dimethyl-2-oxo-benzimidazol-5-yl)-2-(3-methylimidazol-4-yl)benzimidazole-5-carboxylate and methyl 3-(1,3-dimethyl-2-oxo-benzimidazol-5-yl)-6-[3-fluoro-4-(methylcarbamoyl)phenyl]-2-(3-methylimidazol-4-yl)benzimidazole-5-carboxylate

### Step 1: Preparation of methyl 2-bromo-5-((1,3-dimethyl-2-oxo-2,3-dihydro-1H-benzo[d]imidazol-5-yl)amino)-4-nitrobenzoate

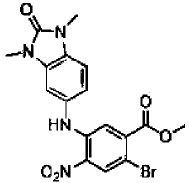

5-Amino-1,3-dimethyl-1,3-dihydro-2H-benzo[d]imidazol-2-one (Intermediate I-1, 0.637 g, 3.60 mmol) was dissolved in 1,4-dioxane (9 mL), and methyl 2-bromo-5-fluoro-4-nitrobenzoate (1 g, 3.60 mmol) and diisopropylethylamine (0.942 mL, 5.40 mmol) were added. The reaction solution was stirred at 100°C for 18 hours. After the reaction was completed, water was added, and an organic layer was extracted with dichloromethane. The organic layer was washed with water and brine, dried over anhydrous magnesium sulfate and concentrated under reduced pressure, and the residue was purified by MPLC to obtain the title compound. (1.5 g, 3.45 mmol, 96% yield)
¹H-NMR (400 MHz, DMSO-d₆) δ 9.55 (s, 1H), 8.33 (s, 1H), 7.24 (s, 1H), 7.22 (d, J = 8.7 Hz, 1H), 7.19 (d, J = 1.8 Hz, 1H), 7.05 (dd, J = 8.2, 1.8 Hz, 1H), 3.76 (s, 3H), 3.31 (s, 3H).

### Step 2: Preparation of methyl 4-amino-2-bromo-5-((1,3-dimethyl-2-oxo-2,3-dihydro-1H-benzo[d]imidazol-5-yl)amino)benzoate

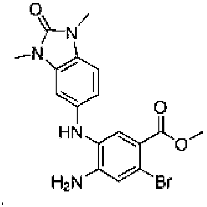

Methyl 2-bromo-5-((1,3-dimethyl-2-oxo-2,3-dihydro-1H-benzo[d]imidazol-5-yl)amino)-4-nitrobenzoate (1.5 g, 3.45 mmol) obtained in Step 1 was dissolved in ethanol (11.50 mL) and water (5.70 mL), and then iron (1.155 g, 20.68 mmol) and ammonium chloride (0.221 g, 4.14 mmol) were added. The reaction solution was stirred at 100°C for 2 hours. After the reaction was completed, the reaction product was passed through a celite filter and concentrated, and purified by MPLC to obtain the title compound. (1.3 g, 3.21 mmol, 93% yield)
¹H-NMR (500 MHz, DMSO-d₆) δ 7.51 (s, 1H), 7.03-6.98 (m, 3H), 6.66-6.60 (m, 2H), 3.67 (s, 3H), 3.28 (s, 3H), 3.24 (s, 3H).

### Step 3: Preparation of methyl 6-bromo-3-(1,3-dimethyl-2-oxo-benzimidazol-5-yl)-2-(3-methylimidazol-4-yl)benzimidazole-5-carboxylate (Example 3)

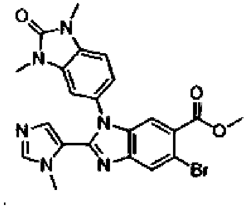

1-Methyl-1H-imidazole-5-carbaldehyde (54.3 mg, 0.494 mmol) and methyl 4-amino-2-bromo-5-((1,3-dimethyl-2-oxo-2,3-dihydro-1H-benzo[d]imidazol-5-yl)amino)benzoate (200 mg, 0.494 mmol) obtained in Step 2 were dissolved in acetic acid (8.2 mL), and then copper(II) acetate (90 mg, 0.494 mmol) was added. The reaction solution was stirred at 80°C for 30 minutes. After the reaction was completed, water and 1 N sodium hydroxide aqueous solution were added, and an organic layer was extracted with dichloromethane. The organic layer was washed with water and brine, dried over anhydrous magnesium sulfate and concentrated under reduced pressure, and the residue was purified by MPLC to obtain the title compound. (102 mg, 0.206 mmol, 41.7% yield)
¹H-NMR (500 MHz, Chloroform-d) δ 8.09 (s, 1H), 7.56 (brs, 1H), 7.51 (s, 1H), 7.16-7.08 (m, 2H), 6.92 (s, 1H), 6.47 (br s, 1H), 4.17 (s, 3H), 3.88 (s, 3H), 3.53 (s, 3H), 3.41 (s, 3H).

### Step 4: Preparation of methyl 3-(1,3-dimethyl-2-oxo-benzimidazol-5-yl)-6-[3-fluoro-4-(methylcarbamoyl)phenyl]-2-(3-methylimidazol-4-yl)benzimidazole-5-carboxylate (Example 4)

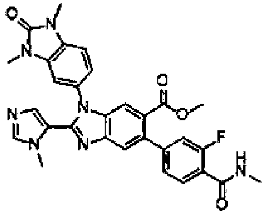

Methyl 6-bromo-3-(1,3-dimethyl-2-oxo-benzimidazol-5-yl)-2-(3-methylimidazol-4-yl)benzimidazole-5-carboxylate (102 mg, 0.206 mmol) obtained in Step 3, (3-fluoro-4-(methylcarbamoyl)phenyl)boronic acid (48.7 mg, 0.247 mmol) and tripotassium phosphate (87 mg, 0.412 mmol) were dissolved in 1,4-dioxane (0.8 mL) and water (0.2 mL), and [1,1'-bis(diphenylphosphino)ferrocene]palladium(II) dichloride dichloromethane adduct (16.82 mg, 0.021 mmol) was added. The reaction solution was stirred under nitrogen conditions at 100°C for 3 hours. After the reaction was completed, water was added, and an organic layer was extracted with dichloromethane. The organic layer was washed with water and brine, dried over anhydrous magnesium sulfate and concentrated under reduced pressure, and the residue was purified by MPLC and PTLC to obtain the title compound. (111 mg, 0.196 mmol, 95% yield)
¹H-NMR (500 MHz, Chloroform-d) δ 8.12 (t, J = 8.1 Hz, 1H), 7.73 (s, 1H), 7.62 (s, 1H), 7.53 (s, 1H), 7.23 (d, J = 8.2 Hz, 1H), 7.17-7.12 (m, 3H), 6.96 (s, 1H), 6.83-6.80 (m, 1H), 6.47 (s, 1H), 4.17 (s, 3H), 3.59 (s, 3H), 3.52 (s, 3H), 3.41 (s, 3H), 3.05 (d, J = 4.3 Hz, 3H); LC/MS: 568 (M+H).

### Example 6 (Synthetic Method C): Preparation of 3-(1,3-dimethyl-2-oxo-benzimidazol-5-yl)-2-(3-methylimidazol-4-yl)-6-(1-methylpyrazol-4-yl)benzimidazole-5-carbonitrile

### Step 1: Preparation of 4-amino-2-bromo-5-((1,3-dimethyl-2-oxo-2,3-dihydro-1H-benzo[d]imidazol-5-yl)amino)benzonitrile

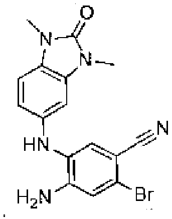

The title compound was obtained in a similar manner to Step 1 and Step 2 in Synthetic Method B using 5-amino-1,3-dimethyl-1,3-dihydro-2H-benzo[d]imidazol-2-one (Intermediate I-1, 56 mg, 0.316 mmol) and 5-fluoro-4-nitrobenzonitrile (77 mg, 0.316 mmol) as starting materials. (70.5% yield over 2 steps)

### Step 2: Preparation of 4-amino-5-((1,3-dimethyl-2-oxo-2,3-dihydro-1H-benzo[d]imidazol-5-yl)amino)-2-(1-methyl-1H-pyrazol-4-yl)benzonitrile

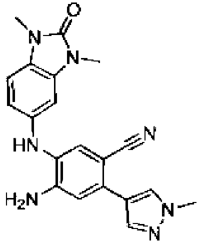

4-Amino-2-bromo-5-((1,3-dimethyl-2-oxo-2,3-dihydro-1H-benzo[d]imidazol-5-yl)amino)benzonitrile (78 mg, 0.210 mmol) obtained in Step 1, potassium carbonate (57.9 mg, 0.419 mmol), and 1-methyl-4-(4,4,5,5-tetramethyl-1,3,2-dioxaborolan-2-yl)-1H-pyrazole (52.3 mg, 0.251 mmol) were dissolved in 1,4-dioxane (0.8 mL) and water (0.2 mL), and then [1,1'-bis(diphenylphosphino)ferrocene]palladium(II) dichloride dichloromethane adduct (17.11 mg, 0.021 mmol) was added. The reaction solution was stirred at 100°C for 18 hours under nitrogen conditions. After the reaction was completed, water was added, and an organic layer was extracted with dichloromethane. The organic layer was washed with water and brine, dried over anhydrous magnesium sulfate and concentrated under reduced pressure, and the residue was purified by MPLC to obtain the title compound. (54 mg, 0.145 mmol, 69.0% yield)
¹H-NMR (500 MHz, Methanol-d₄) δ 8.02 (s, 1H), 7.84 (s, 1H), 7.25 (s, 1H), 7.02 (d, J = 8.5 Hz, 1H), 6.95 (s, 1H), 6.89 (d, J = 7.0 Hz, 1H), 6.69 (dd, J = 8.5, 1.8 Hz, 1H), 6.64 (d, J = 1.8 Hz, 1H), 6.58 (s, 2H), 3.95 (s, 3H), 3.39 (s, 3H), 3.35 (s, 3H).

### Step 3: Preparation of 3-(1,3-dimethyl-2-oxo-benzimidazol-5-yl)-2-(3-methylimidazol-4-yl)-6-(1-methylpyrazol-4-yl)benzimidazole-5-carbonitrile

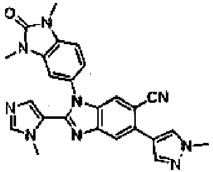

The title compound was obtained in a similar manner to Step 3 of Synthetic Method B using 4-amino-5-((1,3-dimethyl-2-oxo-2,3-dihydro-1H-benzo[d]imidazol-5-yl)amino)-2-(1-methyl-1H-pyrazol-4-yl)benzonitrile (27 mg, 0.072 mmol) obtained in Step 2 and 1-methyl-1H-imidazole-5-carbaldehyde (7.96 mg, 0.072 mmol) as starting materials. (19 mg, 0.041 mmol, 56.7% yield)

¹H-NMR (400 MHz, Methanol-d₄) δ 8.06 (s, 1H), 7.90 (s, 1H), 7.89 (s, 1H), 7.81 (s, 1H), 7.49 (s, 1H), 7.35 (d, J = 8.2 Hz, 1H), 7.32 (d, J = 1.8 Hz, 1H), 7.20 (dd, J = 8.2, 1.8 Hz, 1H), 6.51 (s, 1H), 4.11 (s, 3H), 3.96 (s, 3H), 3.49 (s, 3H), 3.40 (s, 3H).

### Example 7 (Synthetic Method D): Preparation of 5-[6-(hydroxymethyl)-2-(6-methyl-1H-indol-3-yl)-5-(1-methylpyrazol-4-yl)benzimidazol-1-yl]-1,3-dimethyl-benzimidazol-2-one

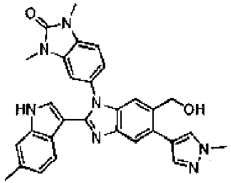

Methyl 3-(1,3-dimethyl-2-oxo-benzimidazol-5-yl)-2-(6-methyl-1H-indol-3-yl)-6-(1-methylpyrazol-4-yl)benzimidazole-5-carboxylate (Example 5, 300 mg, 0.550 mmol) was dissolved in tetrahydrofuran (5 mL) and cooled to 0°C, and diisobutylaluminum hydride (DIBAL-H) (1 M in tetrahydrofuran, 2.2 mL, 2.2 mmol) was slowly added. The reaction solution was stirred at room temperature for 4 hours. The reaction solution was diluted with ethyl acetate and washed with water and brine. Anhydrous sodium sulfate was added to an organic layer to remove water, then filtered, and the filtrate was concentrated under reduced pressure and purified by MPLC to obtain the title compound. (125 mg, 0.242 mmol, 43.9% yield)
¹H-NMR (400 MHz, DMSO-d₆) δ 11.11(s, 1H), 8.52 (d, 1H), 7.96 (d, 1H), 7.72 (d, 2H), 7.46 (m, 2H), 7.22 (m, 2H), 7.12 (s, 1H), 7.04 (d, 1H), 6.50 (s, 1H), 5.12 (t, 1H), 4.55 (d, 2H), 3.92 (s, 3H), 3.47 (s, 3H), 3.38 (s, 3H), 2.50 (s, 3H); LC/MS: 518 (M+H).

### Example 8 (Synthetic Method E): Preparation of 3-(1,3-dimethyl-2-oxo-benzimidazol-5-yl)-2-(6-methyl-1H-indol-3-yl)-6-(1-methylpyrazol-4-yl)benzimidazole-5-carbaldehyde

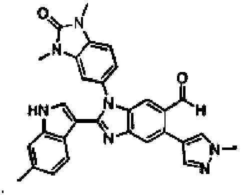

5-[6-(Hydroxymethyl)-2-(6-methyl-1H-indol-3-yl)-5-(1-methylpyrazol-4-yl)benzimidazol-1-yl]-1,3-dimethyl-benzimidazol-2-one (Example 7, 100 mg, 0.193 mmol) was dissolved in dichloromethane (1 mL), and manganese dioxide (33.6 mg, 0.386 mmol) was added. The reaction solution was stirred at 45°C for 4 hours. The reaction solution was filtered to remove a catalyst, and the filtrate was concentrated under reduced pressure. The title compound was obtained by purification using MPLC. (90 mg, 0.193 mmol, 90.0% yield)
¹H-NMR (400 MHz, Chloroform-d) δ 10.22 (s, 1H), 8.59 (d, 1H), 8.21 (m, 1H), 7.88 (d, 1H), 7.74 (d, 1H), 7.68 (s, 1H), 7.55 (d, 1H), 7.24 (m, 3H), 7.03 (s, 1H), 6.64 (d, 1H), 4.05 (s, 3H), 3.57 (s, 3H), 3.42 (s, 3H), 2.51 (s, 3H); LC/MS: 516 (M+H).

### Example 9: Preparation of 5-[6-(difluoromethyl)-2-(6-methyl-1H-indol-3-yl)-5-(1-methylpyrazol-4-yl)benzimidazol-1-yl]-1,3-dimethyl-benzimidazol-2-one

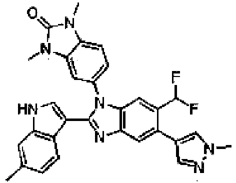

3-(1,3-Dimethyl-2-oxo-benzimidazol-5-yl)-2-(6-methyl-1H-indol-3-yl)-6-(1-methylpyrazol-4-yl)benzimidazole-5-carbaldehyde (Example 8, 90 mg, 0.175 mmol) was dissolved in dichloromethane (2 mL) and cooled to 0°C, and diethylaminosulfur trifluoride (98 mg, 0.61 mmol) was slowly added. The reaction solution was stirred at room temperature for 12 hours. Saturated sodium bicarbonate solution was added to the reaction solution, and the resultant was extracted with dichloromethane. Anhydrous sodium sulfate was added to a collected organic layer to remove water, then filtered, and the filtrate was concentrated under reduced pressure and purified by MPLC to obtain the title compound. (15 mg, 0.028 mmol, 16.0% yield)
¹H-NMR (400 MHz, DMSO-d₆) δ 11.22 (s, 1H), 8.53 (d, 1H), 7.92 (d, 1H), 7.79 (s, 1H), 7.67 (d, 1H), 7.50 (m, 2H), 7.28 (d, 1H), 7.22 (d, 2H), 7.07 (m, 2H), 6.54 (d, 1H), 3.94 (s, 3H), 3.47 (s, 3H), 3.36 (s, 3H), 2.43 (s, 3H); LC/MS: 538 (M+H).

### Example 10 (Synthetic Method F): Preparation of 3-(1,3-dimethyl-2-oxo-benzimidazol-5-yl)-2-(6-methyl-1H-indol-3-yl)-6-(1-methylpyrazol-4-yl)benzimidazole-5-carboxylic acid

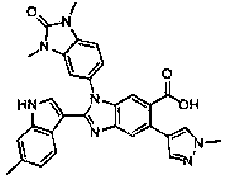

Methyl 3-(1,3-dimethyl-2-oxo-benzimidazol-5-yl)-2-(6-methyl-1H-indol-3-yl)-6-(1-methylpyrazol-4-yl)benzimidazole-5-carboxylate (Example 5, 20 mg, 0.037 mmol) was dissolved in methanol (1 mL), and 1 N sodium hydroxide (0.11 mL, 0.11 mmol) was slowly added. The reaction solution was stirred at room temperature for 3 hours. The reaction solution was neutralized with 1 N hydrochloric acid. The reaction solution was diluted with water and extracted with ethyl acetate. Anhydrous sodium sulfate was added to an organic layer to remove water and filtered, and the filtrate was concentrated under reduced pressure and purified by MPLC to obtain the title compound. (11 mg, 21 µmol, 56.5% yield)
¹H-NMR (400 MHz, Methanol-d₄) δ 8.00 (d, 1H), 7.83 (s, 1H), 7.75 (s, 1H), 7.65 (s, 1H), 7.60 (m, 1H), 7.45 (m, 2H), 7.38 (d, 1H), 7.29 (m, 1H), 7.12 (d, 1H), 7.06 (s, 1H), 3.98 (s, 3H), 3.55 (s, 3H), 3.35 (s, 3H), 2.47 (s, 3H); LC/MS: 532 (M+H).

### Example 12 (Synthetic Method G): Preparation of 5-[6-(1-hydroxy-1-methyl-ethyl)-2-(6-methyl-1H-indol-3-yl)-5-(1-methylpyrazol-4-yl)benzimidazol-1-yl]-1,3-dimethyl-benzimidazol-2-one

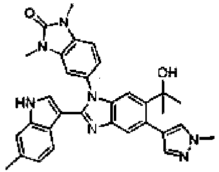

Methyl 3-(1,3-dimethyl-2-oxo-benzimidazol-5-yl)-2-(6-methyl-1H-indol-3-yl)-6-(1-methylpyrazol-4-yl)benzimidazole-5-carboxylate (Example 5, 30 mg, 0.055 mmol) was dissolved in tetrahydrofuran (1 mL), and methylmagnesium bromide (3 M in diethyl ether, 0.055 mL, 0.165 mmol) was slowly added. The reaction solution was stirred at room temperature for 24 hours. Ice was added to the reaction solution, and the resultant was diluted with saturated ammonium chloride aqueous solution and extracted with ethyl acetate. Anhydrous sodium sulfate was added to an organic layer to remove water, and then filtered. The filtrate was concentrated under reduced pressure and purified by MPLC to obtain the title compound. (4.3 mg, 0.0079 mmol, 14.3% yield)

¹H-NMR (400 MHz, Chloroform-d) δ 8.39 (m, 2H), 7.66 (d, 1H), 7.48 (d, 1H), 7.36 (d, 1H), 7.28 (d, 1H), 7.21 (m, 1H), 7.15 (m, 2H), 7.08 (d, 1H), 6.98 (s, 1H), 6.54 (s, 1H), 4.01 (s, 3H), 3.78 (s, 1H), 3.54 (s, 3H), 3.38 (s, 3H), 2.47 (s, 3H), 1.53 (s, 6H); LC/MS: 546 (M+H).

### Example 26 (Synthetic Method H): Preparation of 3-(1,3-dimethyl-2-oxo-benzimidazol-5-yl)-6-[3-fluoro-4-(methylcarbamoyl)phenyl]-N-methyl-2-(3-methylimidazol-4-yl)benzimidazole-5-carboxamide

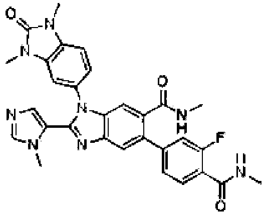

3-(1,3-Dimethyl-2-oxo-benzimidazol-5-yl)-6-[3-fluoro-4-(methylcarbamoyl)phenyl]-2-(3-methylimidazol-4-yl)benzimidazole-5-carboxylic acid (Example 22, 78 mg, 0.141 mmol), 1-ethyl-3-(3-dimethylaminopropyl)carbodiimide (40.5 mg, 0.211 mmol), and hydroxybenzotriazole (32.4 mg, 0.211 mmol) were dissolved in dimethylformamide (1.4 mL), and then methanamine (2 M in tetrahydrofuran, 176 µL, 0.352 mmol) was added. The reaction solution was stirred at room temperature for 6 hours. After the reaction was completed, water was added, and an organic layer was extracted with dichloromethane. The organic layer was washed with water and brine, dried over anhydrous magnesium sulfate and concentrated under reduced pressure, and the residue was purified by MPLC to obtain the title compound. (54 mg, 0.095 mmol, 67.6% yield)
¹H-NMR (500 MHz, Chloroform-d) δ 8.15 (t, J = 8.1 Hz, 1H), 7.78 (s, 1H), 7.53 (s, 1H), 7.39 (s, 1H), 7.37 (d, J = 8.2 Hz, 1H), 7.27-7.24 (m, 1H), 7.14-7.09 (m, 2H), 6.95 (s, 1H), 6.82-6.78 (m, 1H), 6.47 (s, 1H), 5.36 (d, J = 4.9 Hz, 1H), 4.17 (s, 3H), 3.52 (s, 3H), 3.41 (s, 3H), 3.06 (d, J = 4.9 Hz, 3H), 2.69 (d, J = 4.9 Hz, 3H).

### Example 47 (Synthetic Method I): Preparation of 6-[6-fluoro-2-(3-methylimidazol-4-yl)-5-(1-methylpyrazol-4-yl)benzimidazol-1-yl]-1,3-dimethyl-4-morpholino-benzimidazol-2-one

### Step 1: Preparation of 6-((4-bromo-5-fluoro-2-nitrophenyl)amino)-1,3-dimethyl-4-morpholino-1,3-dihydro-2H-benzo[d]imidazol-2-one

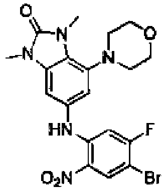

6-Amino-1,3-dimethyl-4-morpholino-1,3-dihydro-2H-benzo[d]imidazol-2-one (Intermediate I-10, 47 mg, 0.179 mmol) and 1-bromo-2,4-difluoro-5-nitrobenzene (42.6 mg, 0.179 mmol) were dissolved in 1,4-dioxane (2.00 mL), and diisopropylethylamine (0.064 mL, 0.358 mmol) was added dropwisely, followed by stirring at 100°C for 18 hours. The reaction solution was diluted in dichloromethane, washed with water, dried over anhydrous magnesium sulfate, and concentrated under reduced pressure. The residue was purified by MPLC to obtain the title compound. (77.6 mg, 0.162 mmol, 90% yield)
¹H-NMR (500 MHz, Chloroform-d) δ 9.54 (s, 1 H), 8.51 (s, 1 H), 6.79 (dd, 3 H), 3.92 (br. s., 4 H), 3.79 (s, 3 H), 3.42 (s, 3H), 3.01 (s, 4H).

### Step 2: Preparation of 5-bromo-6-fluoro-1',3'-dimethyl-2-(1-methyl-1H-imidazol-5-yl)-7'-morpholino-1',3'-dihydro-2'H-[1,5'-bibenzo[d]imidazol]-2'-one

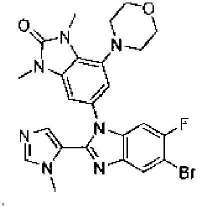

6-((4-Bromo-5-fluoro-2-nitrophenyl)amino)-1,3-dimethyl-4-morpholino-1,3-dihydro-2H-benzo[d]imidazol-2-one] (77.6 mg, 0.162 mmol) obtained in Step 1 and sodium dithionite (99 mg, 0.485 mmol) were stirred together with 1-methyl-1H-imidazole-5-carbaldehyde (175 mg, 0.162 mmol) in a dimethyl sulfoxide (2.3 mL) and water (0.23 mL) solvent at 90°C for 1 hour. After diluting the reaction solution with water and ethyl acetate, 25% ammonia water was added to terminate the reaction. An organic layer was washed with water, dried over anhydrous sodium sulfate and concentrated under reduced pressure, and the residue obtained was purified by MPLC to obtain the title compound. (48 mg, 0.162 mmol, 55% yield)
¹H-NMR(500 MHz, Chloroform-d) δ 8.01 (d, 1H), 7.53 (s, 1H), 6.88 (d, 1H), 6.84 (s, 1H), 6.74 (s, 1H), 6.45 (s, 1H), 4.16 (s, 3 H), 3.89 (br, s, 4H), 3.83 (s, 3H), 3.40 (s, 3H), 2.97 (s, 4H).

### Step 3: Preparation of 6-[6-fluoro-2-(3-methylimidazol-4-yl)-5-(1-methylpyrazol-4-yl)benzimidazol-1-yl]-1,3-dimethyl-4-morpholino-benzimidazol-2-one

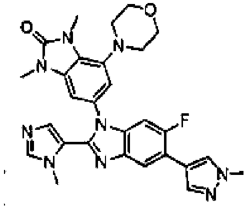

The title compound was obtained in a similar manner to Step 4 of Synthetic Method B using 5-bromo-6-fluoro-1',3'-dimethyl-2-(1-methyl-1H-imidazol-5-yl)-7'-morpholino-1',3'-dihydro-2'H-[1,5'-bibenzo[d]imidazol]-2'-one (7.5 mg, 0.014 mmol) obtained in Step 2 and 1-methyl-4-(4,4,5,5-tetramethyl-1,3,2-dioxaborolan-2-yl)-1H-pyrazole (4.04 mg, 0.019 mmol) as starting materials. (0.8 mg, 0.014 mmol, 10.6% yield)
¹H-NMR (500 MHz, Chloroform-d) δ 7.92 (s, 1H), 7.79 (br. s., 1H), 7.53 (s, 1H), 6.87 (s, 2H), 6.77 (s, 1H), 6.46 (s, 1H), 4.18 (s, 3H), 3.99 (s, 3H), 3.83 (s, 3H), 3.40 (s, 3H), 2.98 (br. s., 4H); LC/MS: 542 (M+H).

### Example 49 (Synthetic Method J): Preparation of 1-ethyl-5-[6-fluoro-2-(3-methylimidazol-4-yl)-5-(1-methylpyrazol-4-yl)benzimidazol-1-yl]-3-methyl-benzimidazol-2-one

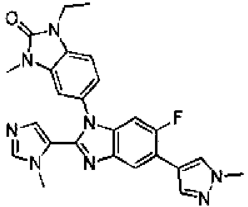

To a solution of 5-[6-fluoro-2-(3-methylimidazol-4-yl)-5-(1-methylpyrazol-4-yl)benzimidazol-1-yl]-3-methyl-1H-benzimidazol-2-one (Example 48, 25 mg, 0.057 mmol) dissolved in dimethylformamide (2 mL), potassium carbonate (23.43 mg, 0.170 mmol) and bromoethane (6.33 µL, 0.085 mmol) were added, followed by stirring at room temperature for 3 days. The reaction product was diluted with ethyl acetate and water, and a separated aqueous layer was extracted with ethyl acetate. An organic layer was washed with brine, dried over anhydrous sodium sulfate and concentrated under reduced pressure, and the residue was purified by PTLC to obtain the title compound. (17.6 mg, 0.037 mmol, 66.2% yield).

¹H-NMR (500 MHz, DMSO-d₆) δ 8.15 (s, 1H), 8.10 (d, 1H), 7.98 (s, 1H), 7.82 (s, 1H), 7.45-7.42 (m, 2H), 7.18 (d, 1H), 6.98 (d, 1H), 6.37 (s, 1H), 4.05 (s, 3H), 3.99-3.97 (m, 2H), 3.91 (s, 3H), 3.35 (s, 3H), 1.29 (t, 3H); LC/MS: 471 (M+H).

### Example 54 (Synthetic Method K): Preparation of 6-[6-fluoro-2-(3-methylimidazol-4-yl)-5-(1-methylpyrazol-4-yl)benzimidazol-1-yl]-4-isopropenyl-1,3-dimethyl-benzimidazol-2-one

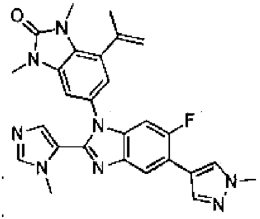

4-Chloro-6-[6-fluoro-2-(3-methylimidazol-4-yl)-5-(1-methylpyrazol-4-yl)benzimidazol-1-yl]-1,3-dimethyl-benzimidazol-2-one (Example 53, 30 mg, 0.061 mmol), 4,4,5,5-tetramethyl-2-(prop-1-en-2-yl)-1,3,2-dioxaborolane (20.54 mg, 0.122 mmol), and 2 M sodium carbonate aqueous solution (0.122 mL, 0.244 mmol) were added to 1,4-dioxane (1 mL) and stirred for 5 minutes while bubbling with nitrogen. Tetrakis(triphenylphosphine)palladium(0) (7.06 mg, 6.11 µmol) was added to the reaction solution and stirred at 90°C for 18 hours under nitrogen conditions. After the reaction was completed, water was added, the resultant was extracted with ethyl acetate, and an organic layer was washed with brine and concentrated under reduced pressure. The title compound was obtained by purification by MPLC and PTLC. (26 mg, 0.052 mmol, 86% yield)
¹H-NMR (500 MHz, Chloroform-d) δ 8.12-7.98 (m, 2H), 7.83 (s, 1H), 7.08-6.75 (m, 4H), 6.72 (s, 1H), 5.44 (s, 1H), 5.10 (s, 1H), 4.40 (s, 3H), 4.04 (s, 3H), 3.60 (s, 3H), 3.45 (s, 3H), 2.16 (s, 3H); LC/MS: 497(M+H).

### Example 58 (Synthetic Method L): Preparation of 6-[6-fluoro-2-(3-methylimidazol-4-yl)-5-(1-methylpyrazol-4-yl)benzimidazol-1-yl]-4-isopropyl-1,3-dimethyl-benzimidazol-2-one

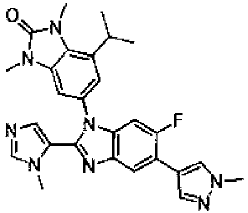

6-[6-Fluoro-2-(3-methylimidazol-4-yl)-5-(1-methylpyrazol-4-yl)benzimidazol-1-yl]-4-isopropenyl-1,3-dimethyl-benzimidazol-2-one (Example 54, 30 mg, 0.060 mmol) and palladium hydroxide on carbon (21.21 mg, 0.030 mmol) were added to ethanol (1 mL)/methanol (1 mL) and stirred at 50°C under hydrogen balloon pressure for 18 hours. The reaction solution was filtered through a celite pad to remove a catalyst, and the resultant was concentrated under reduced pressure and separated by MPLC and PTLC to obtain the title compound. (18 mg, 0.036 mmol, 59.8% yield)
¹H-NMR (500 MHz, Chloroform-d) δ 7.95 (d, J = 6.4 Hz, 1H), 7.91 (s, 1H), 7.78 (s, 1H), 7.51 (s, 1H), 7.02 (s, 1H), 6.84 (d, J= 10.4 Hz, 1H), 6.77 (s, 1H), 6.45 (s, 1H), 4.23-4.12 (3H), 3.98 (s, 3H), 3.77 (s, 3H), 3.75-3.65 (m, 1H), 3.39 (s, 3H), 1.33 (d, J = 6.7 Hz, 6H); LC/MS: 499(M+H).

### Example 59 (Synthetic Method M): Preparation of 6-[6-fluoro-2-(3-methylimidazol-4-yl)-5-(1-methylpyrazol-4-yl)benzimidazol-1-yl]-4-(isopropylamino)-1,3-dimethyl-benzimidazol-2-one

### Step 1: Preparation of 6-((4-bromo-5-fluoro-2-nitrophenyl)amino)-4-(isopropylamino)-1,3-dimethyl-1,3-dihydro-2H-benzo[d]imidazol-2-one

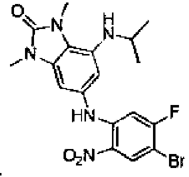

The title compound was obtained in a similar manner to Step 1 of Synthetic Method B using 6-amino-4-(isopropylamino)-1,3-dimethyl-1,3-dihydro-2H-benzo[d]imidazol-2-one (Intermediate I-14, 235 mg, 1.003 mmol) and 1-bromo-2,4-difluoro-5-nitrobenzene (239 mg, 1.003 mmol) as starting materials. (378 mg, 0.836 mmol, 83% yield)
¹H-NMR (500 MHz, Chloroform-d) δ 9.52 (s, 1H), 8.49 (d, J = 8.9 Hz, 1H), 6.83 (d, J = 13.4 Hz, 1H), 6.37 (s, 1H), 6.33 (s, 1H), 3.83 (s, 1H), 3.77 (s, 3H), 3.63-3.59 (m, 1H), 3.39 (s, 3H), 1.29 (d, J = 7.9 Hz, 6H); LC/MS: 452, 454 (M+H).

### Step 2: Preparation of 6-((5-fluoro-4-(1-methyl-1H-pyrazol-4-yl)-2-nitrophenyl)amino)-4-(isopropylamino)-1,3-dimethyl-1,3-dihydro-2H-benzo[d]imidazol-2-one

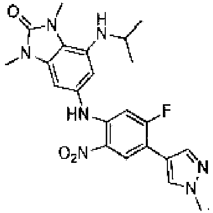

The title compound was obtained in a similar manner to Step 4 of Synthetic Method B using 6-((4-bromo-5-fluoro-2-nitrophenyl)amino)-4-(isopropylamino)-1,3-dimethyl-1,3-dihydro-2H-benzo[d]imidazol-2-one (701 mg, 1.550 mmol) obtained in Step 1 and 1-methyl-4-(4,4,5,5-tetramethyl-1,3,2-dioxaborolan-2-yl)-1H-pyrazole (387 mg, 1.860 mmol) as starting materials. (487 mg, 1.074 mmol, 69.3% yield)
¹H-NMR (500 MHz, Chloroform-d) δ 9.49 (s, 1H), 8.42 (d, J = 7.6 Hz, 1H), 7.81 (s, 1H), 7.69 (s, 1H), 6.82 (d, J = 13.1 Hz, 1H), 6.38 (s, 1H), 6.34 (s, 1H), 3.95 (s, 3H), 3.75 (s, 3H), 3.61-3.59 (m, 1H), 3.37 (s, 3H), 1.27 (d, J = 6.1 Hz, 6H); LC/MS: 454 (M+H).

### Step 3: Preparation of 6-[6-fluoro-2-(3-methylimidazol-4-yl)-5-(1-methylpyrazol-4-yl)benzimidazol-1-yl]-4-(isopropylamino)-1,3-dimethyl-benzimidazol-2-one

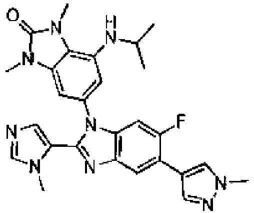

6-((5-Fluoro-4-(1-methyl-1H-pyrazol-4-yl)-2-nitrophenyl)amino)-4-(isopropylamino)-1,3-dimethyl-1,3-dihydro-2H-benzo[d]imidazol-2-one (358 mg, 0.789 mmol) obtained in Step 2 and sodium dithionite (412 mg, 2.368 mmol) were stirred together with 1-methyl-1H-imidazol-5-carbaldehyde (87 mg, 0.789 mmol) at 90°C for 1 hour in a solvent of ethanol (11.8 mL) and water (3.93 mL). The reaction was terminated with 25% ammonia water. The reaction product was diluted with ethyl acetate, washed with water, dried over anhydrous sodium sulfate, and concentrated under reduced pressure. The residue was purified by MPLC to obtain the title compound. (263 mg, 0.512 mmol, 64.9% yield)
¹H-NMR (500 MHz, Chloroform-d) δ 7.94 (d, J = 6.1 Hz, 1H), 7.91 (s, 1H), 7.77 (s, 1H), 7.51 (s, 1H), 6.88 (d, J = 10.4 Hz, 1H), 6.62 (s, 1H), 6.39 (d, J = 7.6 Hz, 2H), 4.17 (s, 3H), 3.98 (s, 3H), 3.90 (d, J = 7.0 Hz, 1H), 3.81 (s, 3H), 3.54-3.52 (m, 1H), 3.35 (s, 3H), 1.22 (d, J = 6.1 Hz, 6H); LC/MS: 514 (M+H).

### Example 60 (Synthetic Method N): Preparation of 6-[6-fluoro-2-(3-methylimidazol-4-yl)-5-(1-methylpyrazol-4-yl)benzimidazol-1-yl]-1,3-dimethyl-4-(propylamino)benzimidazol-2-one

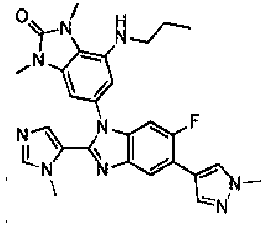

4-Chloro-6-[6-fluoro-2-(3-methylimidazol-4-yl)-5-(1-methylpyrazol-4-yl)benzimidazol-1-yl]-1,3-dimethyl-benzimidazol-2-one (Example 53, 38 mg, 0.077 mmol) and sodium tert-butoxide (22.32 mg, 0.232 mmol) were added to 1,4-dioxane (1 mL), and propane-1-amine (0.025 mL, 0.310 mmol) and PEPPSI-iPr catalyst (5.26 mg, 7.74 µmol) were added. The mixture was stirred at 120°C for 40 minutes using a microwave. Water was added to the reaction solution, the resultant was extracted with ethyl acetate, and an organic layer was concentrated under reduced pressure. The title compound was obtained by separation using MPLC and PTLC. (14 mg, 0.027 mmol, 35.2% yield)
¹H-NMR (500 MHz, Chloroform-d) δ 7.94 (d, J = 6.1 Hz, 1H), 7.91 (s, 1H), 7.77 (s, 1H), 7.53 (s, 1H), 6.88 (d, J = 10.4 Hz, 1H), 6.62 (s, 1H), 6.41 (s, 1H), 6.37 (s, 1H), 4.16 (s, 3H), 3.98 (s, 3H), 3.82 (s, 3H), 3.35 (s, 3H), 3.03 (t, J = 7.0 Hz, 2H), 1.74-1.59 (m, 2H), 1.00 (t, J = 7.3 Hz, 3H); LC/MS: 514(M+H).

### Example 97: Preparation of 6-[6-fluoro-2-(3-methylimidazol-4-yl)-5-(1-methylpyrazol-4-yl)benzimidazol-1-yl]-4-hydroxy-1,3-dimethyl-benzimidazol-2-one

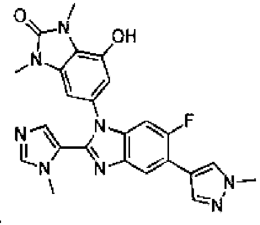

To a reaction vessel containing 4-chloro-6-[6-fluoro-2-(3-methylimidazol-4-yl)-5-(1-methylpyrazol-4-yl)benzimidazol-1-yl]-1,3-dimethyl-benzimidazol-2-one (Example 53, 25 mg, 0.050 mmol), tris(dibenzylideneacetone)dipalladium(0) (2.289 mg, 2.500 µmol), 2-di-tert-butylphosphino-2',4',6'-triisopropylbiphenyl (5.89 mg, 10.00 µmol), and potassium hydroxide (8.42 mg, 0.150 mmol), 1,4-dioxane (0.10 mL) and water (0.10 mL) were added, nitrogen was blown in, and stirring was performed at 100°C for 18 hours. The reaction mixture was cooled to room temperature, acidified with 1 N hydrochloric acid, and extracted with ethyl acetate. The obtained organic layer was washed with brine, dried over anhydrous sodium sulfate, and concentrated under reduced pressure. The residue was purified by MPLC to obtain the title compound. (2.3 mg, 4.87 µmol, 9.7% yield)
¹H-NMR (500 MHz, Chloroform-d) δ 7.88 (d, J = 7.0 Hz, 2H), 7.75 (s, 1H), 7.61 (s, 1H), 6.77 (d, J = 11.6 Hz, 2H), 6.49 (s, 1H), 6.35 (s, 1H), 4.13 (s, 3H), 3.97 (s, 3H), 3.79 (s, 3H), 3.34 (s, 3H); LC/MS: 473 (M+H).

### Example 99 (Synthetic Method O): Preparation of 6-[6-fluoro-2-(3-methylimidazol-4-yl)-5-(1-methylpyrazol-4-yl)benzimidazol-1-yl]-1,3-dimethyl-4-propoxy-benzimidazol-2-one

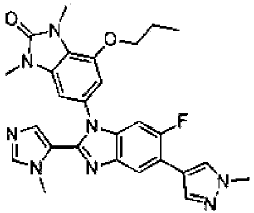

To a reaction vessel containing 4-chloro-6-[6-fluoro-2-(3-methylimidazol-4-yl)-5-(1-methylpyrazol-4-yl)benzimidazol-1-yl]-1,3-dimethyl-benzimidazol-2-one (Example 53, 49 mg, 0.100 mmol), tris(dibenzylideneacetone)dipalladium(0) (4.57 mg, 4.99 µmol), 2-di-tert-butylphosphino-2',4',6'-triisopropylbiphenyl (11.75 mg, 0.020 mmol) and potassium hydroxide (16.80 mg, 0.299 mmol), 1,4-dioxane (0.10 mL) and water (0.10 mL) were added, nitrogen was blown in, and stirring was performed at 100°C for 18 hours. The reaction mixture was cooled to room temperature, and cetyltrimethylammonium bromide (3.6 mg, 9.98 µmol) and 1-bromopropane (0.018 mL, 0.200 mmol) were added, and the reaction mixture was stirred at 100°C for additional 1 hour. The reaction mixture was cooled to room temperature, diluted with water, and extracted with ethyl acetate. The obtained organic layer was washed with brine, dried over anhydrous sodium sulfate and concentrated under reduced pressure, and the residue was purified by MPLC to obtain the title compound. (16.1 mg, 0.031 mmol, 31.3% yield)
¹H-NMR (500 MHz, Chloroform-d) δ 7.94 (d, J = 6.4 Hz, 1H), 7.91 (s, 1H), 7.78 (s, 1H), 7.53 (s, 1H), 6.85 (d, J = 10.4 Hz, 1H), 6.61 (d, J = 7.0 Hz, 2H), 6.54 (s, 1H), 4.17 (s, 3H), 3.98 (s, 3H), 3.95 (t, J = 6.1 Hz, 2H), 3.75 (s, 3H), 3.38 (s, 3H), 1.85 (td, J = 13.6, 6.5 Hz, 2H), 1.07 (t, J = 7.3 Hz, 3H); LC/MS: 515 (M+H).

### Example 107: Preparation of 6-[6-fluoro-2-(3-methylimidazol-4-yl)-5-(1-methylpyrazol-4-yl)benzimidazol-1-yl]-4-[isopropyl(methyl)amino]-1,3-dimethyl-benzimidazol-2-one

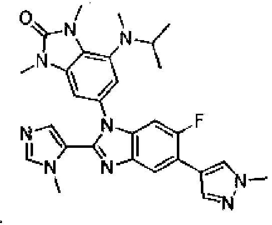

To a solution of 6-[6-fluoro-2-(3-methylimidazol-4-yl)-5-(1-methylpyrazol-4-yl)benzimidazol-1-yl]-4-(isopropylamino)- 1,3-dimethyl-benzimidazol-2-one (Example 59, 29 mg, 0.056 mmol) dissolved in 1,2-dichloroethane (0.565 mL), formaldehyde (37 wt% in water, 0.021 mL, 0.282 mmol), acetic acid (0.016 mL, 0.282 mmol), and sodium triacetoxyborohydride (35.9 mg, 0.169 mmol) were added, and stirred at room temperature for 24 hours. The reaction was terminated by adding a saturated aqueous sodium bicarbonate solution, and the resultant was extracted with dichloromethane. The obtained organic layer was washed with brine, dried over anhydrous magnesium sulfate and concentrated under reduced pressure, and the residue was purified by MPLC to obtain the title compound. (7 mg, 0.013 mmol, 23.5% yield)
¹H-NMR (500 MHz, Chloroform-d) δ 7.95 (d, J = 6.4 Hz, 1H), 7.91 (s, 1H), 7.78 (s, 1H), 7.52 (s, 1H), 6.86 (s, 1H), 6.83 (d, J = 10.7 Hz, 1H), 6.69 (s, 1H), 6.50 (s, 1H), 4.16 (s, 3H), 3.98 (s, 3H), 3.79 (s, 3H), 3.38 (s, 3H), 3.34-3.29 (m, 1H), 2.64 (s, 3H), 1.11 (d, J = 4.6 Hz, 6H); LC/MS: 528 (M+H).

### Example 116 (Synthetic Method P): Preparation of 6-[6-fluoro-2-(1-methylcyclopropyl)-5-(1-methylpyrazol-4-yl)benzimidazol-1-yl]-4-isopropyl-1,3-dimethyl-benzimidazol-2-one

### Step 1: Preparation of 6-((2-amino-5-fluoro-4-(1-methyl-1H-pyrazol-4-yl)phenyl)amino)-4-isopropyl-1,3-dimethyl-1,3-dihydro-2H-benzo[d]imidazol-2-one

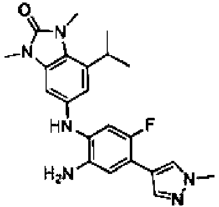

The title compound was synthesized in a similar manner to Step 1 and Step 2 of Synthetic Method C using 6-amino-4-isopropyl-1,3-dimethyl-1,3-dihydro-2H-benzo[d]imidazol-2-one (Intermediate I-23, 540 mg, 2.463 mmol) and 1-bromo-2,4-difluoro-5-nitrobenzene (310 µL, 2.463 mmol) as starting materials. (50.9% yield over 2 steps)
¹H-NMR (500 MHz, Chloroform-d) δ 7.77 (s, 1H), 7.69 (s, 1H), 6.95 (d, J = 7.3 Hz, 1H), 6.85 (d, J = 12.2 Hz, 1H), 6.63 (s, 1H), 6.42 (s, 1H), 3.94 (s, 3H), 3.65 (s, 3H), 3.62-3.57 (m, 1H), 3.34 (s, 3H), 1.31 (d, J = 6.7 Hz, 6H); LC/MS: 409 (M+H).

### Step 2: Preparation of 6-[6-fluoro-2-(1-methylcyclopropyl)-5-(1-methylpyrazol-4-yl)benzimidazol-1-yl]-4-isopropyl-1,3-dimethyl-benzimidazol-2-one

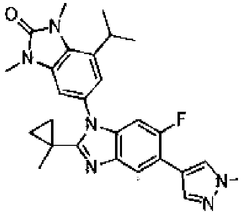

6-((2-amino-5-fluoro-4-(1-methyl-1H-pyrazol-4-yl)phenyl)amino)-4-isopropyl-1,3-dimethyl-1,3-dihydro-2H-benzo[d]imidazol-2-one (39 mg, 0.095 mmol) obtained in Step 1, 1-methylcyclopropane-1-carboxylic acid (10.51 mg, 0.105 mmol), and diisopropylethylamine (0.033 mL, 0.191 mmol) were dissolved in ethyl acetate (1 mL), and 2,4,6-tripropyl-1,3,5,2,4,6-trioxatriphosphinane 2,4,6-trioxide (50 wt% in ethyl acetate, 0.284 mL, 0.477 mmol) was added. The reaction solution was stirred at 110°C for 3 hours using a microwave. After the reaction was completed, the reaction solution was diluted with water, 1 N aqueous sodium hydroxide solution was added, and an organic layer was extracted with ethyl acetate. The organic layer was washed with water and brine, dried over anhydrous magnesium sulfate and concentrated under reduced pressure, and the residue was purified by MPLC and PTLC to obtain the title compound. (8.3 mg, 0.018 mmol, 18.40% yield)
¹H-NMR (500 MHz, Chloroform-d) δ 7.85-7.84 (m, 2H), 7.72 (s, 1H), 7.11 (s, 1H), 6.89 (s, 1H), 6.83 (d, J = 10.4 Hz, 1H), 3.96 (s, 3H), 3.76 (s, 3H), 3.75-3.71 (m, 1H), 3.45 (s, 3H), 1.37 (d, J = 6.7 Hz, 7H), 1.30 (s, 2H), 1.14 (s, 3H), 0.72 (s, 2H); LC/MS: 473 (M+H).

### Example 118: Preparation of 6-[6-fluoro-2-(3-methyloxetan-3-yl)-5-(1-methylpyrazol-4-yl)benzimidazol-1-yl]-4-isopropyl-1,3-dimethyl-benzimidazol-2-one

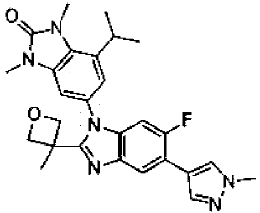

(3-Methyloxetan-3-yl)methanol (15.00 mg, 0.147 mmol) was dissolved in ethyl acetate (0.5 mL) and dimethyl sulfoxide (0.25 mL), and then 2,4,6-tripropyl-1,3,5,2,4,6-trioxatriphosphinane 2,4,6-trioxide (50 wt% in ethyl acetate, 0.131 mL, 0.220 mmol) was added at 0°C. The reaction solution was stirred at room temperature for 2 hours. After the oxidation reaction of alcohol was completed, 6-((2-amino-5-fluoro-4-(1-methyl-1H-pyrazol-4-yl)phenyl)amino)-4-isopropyl-1,3-dimethyl-1,3-dihydro-2H-benzo[d]imidazol-2-one (compound of Step 1 of Synthetic Method P, 30 mg, 0.073 mmol) was added, followed by stirring for an additional 1 hour. After the reaction was completed, water was added, and an organic layer was extracted with dichloromethane. The organic layer was washed with water and brine, dried over anhydrous magnesium sulfate and concentrated under reduced pressure, and the residue was purified by MPLC and PTLC to obtain the title compound. (11.3 mg, 0.023 mmol, 31.5% yield)
¹H-NMR (400 MHz, Chloroform-d) δ 7.90 (d, J = 6.4 Hz, 1H), 7.87 (s, 1H), 7.74 (d, J = 2.3 Hz, 1H), 6.92 (d, J = 2.3 Hz, 1H), 6.79-6.76 (m, 2H), 5.22 (d, J = 5.9 Hz, 2H), 4.27-4.26 (m, 2H), 3.97 (s, 3H), 3.76 (s, 3H), 3.73-3.68 (m, 1H), 3.43 (s, 3H), 1.76 (s, 3H), 1.34 (d, J = 6.9 Hz, 6H); LC/MS: 489 (M+H).

### Example 122: Preparation of 6-[6-fluoro-2-(3-methylimidazol-4-yl)-5-(1-methylpyrazol-4-yl)benzimidazol-1-yl]-1,3-dimethyl-4-(2-trimethylsilylethynyl)benzimidazol-2-one

### Step 1: Preparation of 4-bromo-6-((5-fluoro-4-(1-methyl-1H-pyrazol-4-yl)-2-nitrophenyl)amino)-1,3-dimethyl-1,3-dihydro-2H-benzo[d]imidazol-2-one

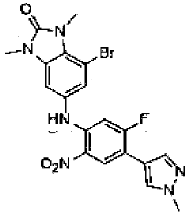

6-Amino-4-bromo-1,3-dimethyl-1,3-dihydro-2H-benzo[d]imidazol-2-one (Intermediate I-3, 100 mg, 0.390 mmol) and 4-(2,4-difluoro-5-nitrophenyl)-1-methyl-1H-pyrazole (Intermediate I-55, 112 mg, 0.469 mmol) were reacted together with anhydrous potassium fluoride (85 mg, 0.586 mmol) in a dimethylformamide (1.5 mL) solvent at 160°C for 4 h with a microwave. The reaction product was diluted with ethyl acetate, washed with water, dried over anhydrous magnesium sulfate, and concentrated under reduced pressure. The residue was purified by MPLC to obtain the title compound. (75.4 mg, 0.159 mmol, 40.6% yield)
¹H-NMR (500 MHz, Chloroform-d) δ 9.46 (br, s, 1 H), 8.43 (d, J=7.78 Hz, 1 H), 7.82(s, 1 H), 7.71 (s, 1 H), 7.17 (s, 1 H), 6.85 (s, 1 H), 6.75 (d, J=12.97 Hz, 1 H), 3.97 (s, 3 H), 3.79 (s, 3 H), 3.43 (s, 3 H).

### Step 2: Preparation of 7'-bromo-6-fluoro-1',3'-dimethyl-2-(1-methyl-1H-imidazol-5-yl)-5-(1-methyl-1H-pyrazol-4-yl)-1',3'-dihydro-2'H-[1,5'-bibenzo[d]imidazol]-2'-one

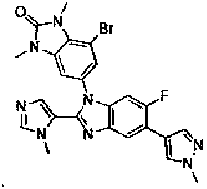

4-Bromo-6-((5-fluoro-4-(1-methyl-1H-pyrazol-4-yl)-2-nitrophenyl)amino)-1,3-dimethyl-1,3-dihydro-2H-benzo[d]imidazol-2-one (75.4 mg, 0.159 mmol) obtained in Step 1, 1-methyl-1H-imidazole-5-carbaldehyde (17.5 mg, 0.159 mmol), and sodium sulfate (83 mg, 0.476 mmol) were reacted in a dimethyl sulfoxide (1 mL, 0.786 mmol) solvent at 90°C for 3 hours. The reaction product was quenched with 25% ammonium hydroxide, diluted in ethyl acetate, washed with water, dried over anhydrous magnesium sulfate, and concentrated under reduced pressure. The residue was purified by MPLC to obtain the title compound. (35.4 mg, 0.066 mmol, 41.7% yield)
¹H-NMR (500 MHz, Chloroform-d) δ 7.96(d, 2H) 7.79(s, 1H) 7.54(s, 1H) 6.89(s, 1H) 6.86(d, 1H) 6.55(s, 1H) 4.17(s, 1H) 3.99(s, 1H) 3.86(s, 3H) 3.41(s, 3H); LC/MS: 535, 537 (M+H).

### Step 3: Preparation of 6-[6-fluoro-2-(3-methylimidazol-4-yl)-5-(1-methylpyrazol-4-yl)benzimidazol-1-yl]-1,3-dimethyl-4-(2-trimethylsilylethynyl)benzimidazol-2-one

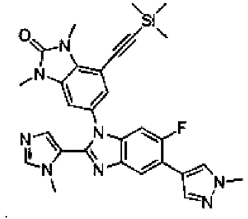

7'-Bromo-6-fluoro-1',3'-dimethyl-2-(1-methyl-1H-imidazol-5-yl)-5-(1-methyl-1H-pyrazol-4-yl)-1',3'-dihydro-2'H-[1,5'-bibenzo[d]imidazol]-2'-one (50 mg, 0.093 mmol) obtained in Step 2 was dissolved in dimethylformamide (1 mL) and ethynyltrimethylsilane (0.026 mL, 0.187 mmol), copper(I) iodide (1.779 mg, 9.34 µmol), triethylamine (0.130 mL, 0.934 mmol), and bis(triphenylphosphine)palladium(II) dichloride (6.56 mg, 9.34 µmol) were added. The reaction solution was stirred at 100°C for 18 hours. The reaction solution was cooled to room temperature, water and ethyl acetate were added, and an organic layer was extracted and then washed with brine. The organic layer was concentrated under reduced pressure and purified by MPLC and PTLC to obtain the title compound. (17 mg, 0.031 mmol, 32.9% yield)
¹H-NMR (500 MHz, Chloroform-d) δ 7.94 (d, J = 6.4 Hz, 1H), 7.91 (s, 1H), 7.78 (s, 1H), 7.53 (s, 1H), 7.18 (s, 1H), 6.85 (s, 1H), 6.82 (d, J = 10.4 Hz, 1H), 6.53 (s, 1H), 4.20-4.13 (3H), 4.02-3.94 (3H), 3.86 (s, 3H), 3.41-3.35 (3H), 0.30-0.22 (9H); LC/MS: 553 (M+H).

### Example 123: Preparation of 4-ethynyl-6-[6-fluoro-2-(3-methylimidazol-4-yl)-5-(1-methylpyrazol-4-yl)benzimidazol-1-yl]-1,3-dimethyl-benzimidazol-2-one

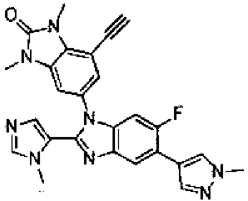

6-[6-Fluoro-2-(3-methylimidazol-4-yl)-5-(1-methylpyrazol-4-yl)benzimidazol-1-yl]-1,3-dimethyl-4-(2-trimethylsilylethynyl)benzimidazol-2-one (Example 122, 14 mg, 0.025 mmol) was added to tetrahydrofuran (0.5 mL), and tetra-n-butylammonium fluoride (1 M in tetrahydrofuran, 0.076 mL, 0.076 mmol) was added. The reaction solution was stirred at room temperature for 18 hours. Water was added to the reaction solution, an organic layer was extracted with ethyl acetate and washed with brine, and moisture was removed with anhydrous magnesium sulfate. The solution was filtered, concentrated under reduced pressure, and purified by PTLC to obtain the title compound. (9.7 mg, 0.020 mmol, 80% yield)
¹H-NMR (500 MHz, Chloroform-d) δ 7.95 (d, J = 6.4 Hz, 1H), 7.91 (s, 1H), 7.78 (s, 1H), 7.53 (s, 1H), 7.21 (s, 1H), 6.90 (s, 1H), 6.83 (d, J = 10.4 Hz, 1H), 6.56-6.49 (1H), 4.16 (s, 3H), 3.98 (s, 3H), 3.90-3.81 (3H), 3.40 (s, 3H), 3.36 (s, 1H); LC/MS: 481 (M+H).

### Example 125: Preparation of 6-[6-fluoro-2-(3-methylimidazol-4-yl)-5-(1-methylpyrazol-4-yl)benzimidazol-1-yl]-4-(1-hydroxyethyl)-1,3-dimethyl-benzimidazol-2-one

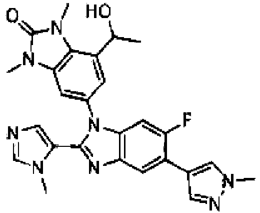

4-Acetyl-6-[6-fluoro-2-(3-methylimidazol-4-yl)-5-(1-methylpyrazol-4-yl)benzimidazol-1-yl]-1,3-dimethyl-benzimidazol-2-one (Example 124, 20 mg, 0.040 mmol) was dissolved in tetrahydrofuran (0.5 mL) and methanol (0.2 mL), and sodium borohydride (3.04 mg, 0.080 mmol) was added at 0°C. The reaction solution was stirred at room temperature for 3 hours. Water was added to the reaction solution, and an organic layer was extracted with ethyl acetate and then concentrated under reduced pressure. The title compound was obtained by purification using PTLC. (20 mg, 0.040 mmol, 100% yield)
¹H-NMR (500 MHz, Chloroform-d) δ 7.94 (d, J = 6.4 Hz, 1H), 7.89 (s, 1H), 7.76 (s, 1H), 7.49 (s, 1H), 7.28 (s, 1H), 6.87 (s, 1H), 6.83 (d, J = 10.4 Hz, 1H), 6.43 (s, 1H), 5.55 (q, J = 6.1 Hz, 1H), 4.14 (s, 3H), 3.97 (s, 3H), 3.77 (s, 3H), 3.44-3.36 (3H), 1.59 (d, J = 6.4 Hz, 3H); LC/MS: 501 (M+H).

### Example 126: Preparation of 6-[6-fluoro-2-(3-methylimidazol-4-yl)-5-(1-methylpyrazol-4-yl)benzimidazol-1-yl]-4-(1-hydroxy-1-methyl-ethyl)-1,3-dimethyl-benzimidazol-2-one

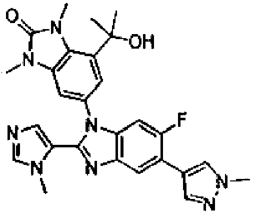

4-Acetyl-6-[6-fluoro-2-(3-methylimidazol-4-yl)-5-(1-methylpyrazol-4-yl)benzimidazol-1-yl]-1,3-dimethyl-benzimidazol-2-one (Example 124, 25 mg, 0.050 mmol) was added to tetrahydrofuran (0.5 mL), the resultant was cooled to 0°C, and methylmagnesium bromide (3.4 M in methyltetrahydrofuran, 0.074 mL, 0.251 mmol) was added. The reaction solution was stirred at room temperature for 18 hours. Water was added to the reaction solution, and an organic layer was extracted with ethyl acetate and then concentrated under reduced pressure. The title compound was obtained by purification by PTLC. (11.5 mg, 0.022 mmol, 44.6% yield)
¹H-NMR (500 MHz, Chloroform-d) δ 7.95 (d, J = 6.4 Hz, 1H), 7.90 (s, 1H), 7.77 (s, 1H), 7.51 (s, 1H), 7.04 (s, 1H), 6.90 (s, 1H), 6.83 (d, J = 10.4 Hz, 1H), 6.43 (s, 1H), 4.16 (s, 3H), 3.98 (s, 3H), 3.96 (s, 3H), 3.41 (s, 3H), 2.85 (br, 1H), 1.71 (s, 6H); LC/MS: 515 (M+H).

### Example 128 (Synthetic Method Q): Preparation of 6-[6-fluoro-2-(3-methylimidazol-4-yl)-5-(1-methylpyrazol-4-yl)benzimidazol-1-yl]-4-(1-methoxyethyl)-1,3-dimethyl-benzimidazol-2-one

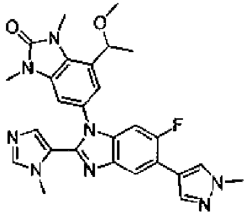

6-[6-Fluoro-2-(3-methylimidazol-4-yl)-5-(1-methylpyrazol-4-yl)benzimidazol-1-yl]-4-(1-hydroxyethyl)-1,3-dimethyl-benzimidazol-2-one (Example 125, 22 mg, 0.044 mmol) was dissolved in tetrahydrofuran (0.5 mL), the resultant was cooled to 0°C, and then sodium hydride (dispersed in mineral oil into 60%, 2.64 mg, 0.066 mmol) was added. The solution was stirred for 10 minutes, and then iodomethane (0.027 mL, 0.440 mmol) was added, followed by stirring at room temperature for 18 hours. Water was added to the reaction solution, and an organic layer was extracted with ethyl acetate and washed with brine. The organic layer was concentrated under reduced pressure and purified by PTLC to obtain the title compound. (6 mg, 0.012 mmol, 26.5% yield)
¹H-NMR (500 MHz, Chloroform-d) δ 7.96 (d, J = 6.7 Hz, 1H), 7.91 (s, 1H), 7.78 (s, 1H), 7.52 (s, 1H), 7.17 (s, 1H), 6.88 (s, 1H), 6.85 (d, J = 10.4 Hz, 1H), 6.44 (s, 1H), 5.10-4.93 (m, 1H), 4.16 (s, 3H), 3.98 (s, 3H), 3.82-3.69 (3H), 3.42 (s, 3H), 3.26 (s, 3H), 1.54 (d, J = 6.1 Hz, 3H); LC/MS: 515 (M+H).

### Example 130 (Synthetic Method R): Preparation of 4-isopropyl-1,3-dimethyl-6-[2-(3-methylimidazol-4-yl)-5-(1-methylpyrazol-4-yl)-6-(trifluoromethyl)benzimidazol-1-yl] benzimidazol-2-one

### Step 1: Preparation of 6-((4-bromo-2-nitro-5-(trifluoromethyl)phenyl)amino)-4-isopropyl-1,3-dimethyl-1,3-dihydro-2H-benzo[d]imidazol-2-one

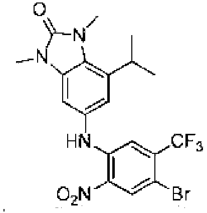

A solution of 6-amino-4-isopropyl-1,3-dimethyl-1,3-dihydro-2H-benzo[d]imidazol-2-one (121 mg, 0.550 mmol), 1-bromo-4-iodo-5-nitro-2-(trifluoromethyl)benzene (198 mg, 0.500 mmol), tris(dibenzylideneacetone)dipalladium(O) (11.45 mg, 0.013 mmol), xanthos (28.9 mg, 0.050 mmol), and potassium tert-butoxide (67.3 mg, 0.600 mmol) dissolved in toluene (1.000 mL) was stirred at 80°C for 18 hours. The reaction mixture was cooled to room temperature, diluted with water, and extracted with ethyl acetate. The obtained organic layer was washed with brine, dried over anhydrous sodium sulfate and concentrated under reduced pressure, and the residue was purified by MPLC to obtain the title compound. (192 mg, 0.394 mmol, 79% yield)
¹H-NMR (500 MHz, Chloroform-d) δ 9.51 (s, 1H), 8.52 (s, 1H), 7.48 (s, 1H), 6.92 (s, 1H), 6.71 (s, 1H), 3.71 (s, 3H), 3.69-3.64 (m, 1H), 3.41 (s, 3H), 1.32 (d, J = 6.7 Hz, 6H); LC/MS: 487, 489 (M+H).

### Step 2: Preparation of 4-isopropyl-1,3-dimethyl-6-((4-(1-methyl-1H-pyrazol-4-yl)-2-nitro-5-(trifluoromethyl)phenyl)amino)-1,3-dihydro-2H-benzo[d]imidazol-2-one

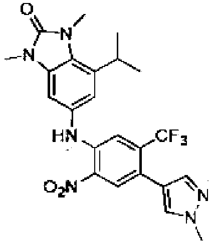

The title compound was obtained in a similar manner to Step 4 of Synthetic Method B using 6-((4-bromo-2-nitro-5-(trifluoromethyl)phenyl)amino)-4-isopropyl-1,3-dimethyl-1,3-dihydro-2H-benzo[d]imidazol-2-one (146 mg, 0.300 mmol) obtained in Step 1 and 1-methyl-4-(4,4,5,5-tetramethyl-1,3,2-dioxaborolan-2-yl)-1H-pyrazole (94 mg, 0.450 mmol) as starting materials. (125.3 mg, 0.257 mmol, 86% yield)
¹H-NMR (500 MHz, Chloroform-d) δ 9.50 (s, 1H), 8.23 (s, 1H), 7.57 (s, 1H), 7.54 (s, 1H), 7.47 (s, 1H), 6.96 (s, 1H), 6.75 (s, 1H), 3.97 (s, 3H), 3.72 (s, 3H), 3.70-3.64 (m, 1H), 3.41 (s, 3H), 1.33 (d, J = 6.7 Hz, 6H); LC/MS: 489 (M+H).

### Step 3: Preparation of 4-isopropyl-1,3-dimethyl-6-[2-(3-methylimidazol-4-yl)-5-(1-methylpyrazol-4-yl)-6-(trifluoromethyl)benzimidazol-1-yl]benzimidazol-2-one

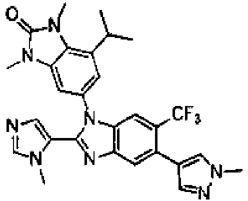

A mixture of 4-isopropyl-1,3-dimethyl-6-((4-(1-methyl-1H-pyrazol-4-yl)-2-nitro-5-(trifluoromethyl)phenyl)amino)-1,3-dihydro-2H-benzo[d]imidazol-2-one (19.3 mg, 0.040 mmol) obtained in Step 2 and sodium dithionite (20.64 mg, 0.119 mmol) dissolved in ethanol (0.3 mL) and water (0.1 mL) was stirred at 90°C. After confirming the exhaustion of the starting material on TLC (approximately 30 minutes), 1-methyl-1H-imidazole-5-carbaldehyde (4.35 mg, 0.040 mmol) was added, followed by stirring at 90°C for 18 hours. The reaction mixture was cooled to room temperature, quenched with 25% ammonia water, diluted with water, and extracted with ethyl acetate. The obtained organic layer was washed with brine, dried over anhydrous sodium sulfate and concentrated under reduced pressure, and the residue was purified by MPLC to obtain the title compound. (15.5 mg, 0.028 mmol, 71.5% yield)
¹H-NMR (500 MHz, Chloroform-d) δ 7.81 (s, 1H), 7.63 (s, 1H), 7.54 (s, 1H), 7.51 (s, 1H), 7.44 (s, 1H), 7.03 (s, 1H), 6.78 (s, 1H), 6.48 (s, 1H), 4.19 (s, 3H), 3.99 (s, 3H), 3.79 (s, 3H), 3.76-3.71 (m, 1H), 3.40 (s, 3H), 1.33 (d, J = 6.4 Hz, 6H); LC/MS: 549 (M+H).

### Example 146 (Synthetic Method S): Preparation of 6-[6-fluoro-2-(3-methylimidazol-4-yl)-5-(1-methylpyrazol-4-yl)benzimidazol-1-yl]-1,3-dimethyl-4-(1-piperidyl)benzimidazol-2-one

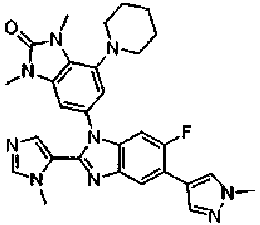

6-[6-Fluoro-2-(3-methylimidazol-4-yl)-5-(1-methylpyrazol-4-yl)benzimidazol-1-yl]-1,3-dimethyl-4-(2-trimethylsilylethynyl)benzimidazol-2-one (a compound synthesized in Step 2 of Example 122, 0.03 g, 0.056 mmol), piperidine (0.022 mL, 0.224 mmol), tris(dibenzylideneacetone)dipalladium(O) (4.58 mg, 0.006 mmol), xanthos (9.73 mg, 0.017 mmol), and cesium carbonate (0.055 g, 0.168 mmol) were stirred in a toluene (3 mL) solvent at 100°C for 18 hours. The reaction product was diluted with ethyl acetate, washed with water, dried over anhydrous magnesium sulfate, and concentrated under reduced pressure. The residue obtained was purified by MPLC to obtain the title compound. (8.4 mg, 0.016 mmol, 27.8% yield)
¹H-NMR (500 MHz, Chloroform-d) δ 7.96 (d, J = 5.80 Hz, 1H), 7.92 (s., 1H), 7.79 (s., 2H), 7.52 (s., 2H), 6.85 (s, 1H), 6.87 (s, 1H), 6.69 (s., 1H), 6.50 (s., 1H), 4.17 (s., 4H), 3.99 (br. s., 3H), 3.83 (br. s., 3H), 3.38 (br. s., 3H), 1.76 (br. s., 4H), 1.26 (br. s., 6H); LC/MS: 540 (M+H).

### Example 178 (Synthetic Method T): Preparation of 6-[6-fluoro-2-(3-methylimidazol-4-yl)-5-(1-methylpyrazol-4-yl)benzimidazol-1-yl]-1,3-dimethyl-4-(4-piperidyl)benzimidazol-2-one

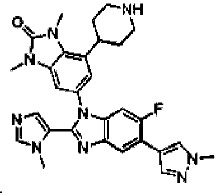

Tert-butyl 4-[6-[6-fluoro-2-(3-methylimidazol-4-yl)-5-(1-methylpyrazol-4-yl)benzimidazol-1-yl]-1,3-dimethyl-2-oxo-benzimidazol-4-yl]piperidine-1-carboxylate (Example 174, 190 mg, 0.297 mmol) was added to tetrahydrofuran (3 mL) and methanol (1 mL), and hydrochloric acid (4 M in 1,4-dioxane, 0.742 mL, 2.97 mmol) was added. The reaction solution was stirred at room temperature for 18 hours. The reaction solution was concentrated under reduced pressure and purified by prepHPLC to obtain the title compound. (150 mg, 0.278 mmol, 94% yield)
¹H-NMR (500 MHz, Chloroform-d) δ 7.95 (d, J = 6.4 Hz, 1H), 7.91 (s, 1H), 7.78 (s, 1H), 7.51 (s, 1H), 7.00 (s, 1H), 6.83 (d, J = 13.7 Hz, 2H), 6.41 (s, 1H), 4.16 (s, 3H), 3.99 (s, 3H), 3.76 (s, 3H), 3.40 (s, 4H), 3.24 (d, J = 11.9 Hz, 2H), 2.79 (d, J = 12.2 Hz, 2H), 1.90 (d, J = 16.5 Hz, 2H), 1.70 (br, 2H); LC/MS: 540 (M+H).

### Example 188 (Synthetic Method U): Preparation of 4-(1-acetyl-4-piperidyl)-6-[6-fluoro-2-(3-methylimidazol-4-yl)-5-(1-methylpyrazol-4-yl)benzimidazol-1-yl]-1,3-dimethyl-benzimidazol-2-one

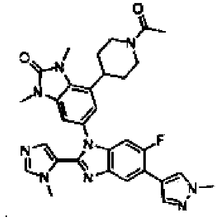

Tert-butyl 4-[6-[6-fluoro-2-(3-methylimidazol-4-yl)-5-(1-methylpyrazol-4-yl)benzimidazol-1-yl]-1,3-dimethyl-2-oxo-benzimidazol-4-yl]piperidine-1-carboxylate (Example 174, 30 mg, 0.056 mmol) was dissolved in dichloromethane (1 mL), and acetyl chloride (4.35 µL, 0.061 mmol) and pyridine (5.40 µL, 0.067 mmol) were added at 0°C. The reaction solution was stirred at room temperature for 18 hours. Water was added to the reaction solution, and an organic layer was extracted with dichloromethane and then concentrated under reduced pressure. The title compound was obtained by separation using MPLC and PTLC. (17 mg, 0.029 mmol, 52.6% yield)
¹H-NMR (400 MHz, Chloroform-d) δ 7.95 (d, J = 6.9 Hz, 1H), 7.91 (s, 1H), 7.78 (d, J = 2.7 Hz, 1H), 7.51 (s, 1H), 6.93-6.90 (m, 1H), 6.86 (dd, J = 6.4, 1.8 Hz, 1H), 6.81 (d, J = 10.5 Hz, 1H), 6.36 (d, J = 3.7 Hz, 1H), 4.82 (d, J = 13.3 Hz, 1H), 4.17 (s, 3H), 3.98 (s, 4H), 3.78 (s, 3H), 3.50 (d, J = 11.9 Hz, 1H), 3.41 (s, 3H), 3.24-3.18 (m, 1H), 2.67-2.62 (m, 1H), 2.09 (s, 3H), 1.96 (t, J = 14.2 Hz, 2H), 1.67 (d, J = 12.8 Hz, 2H); LC/MS: 582 (M+H).

### Example 189 (Synthetic Method V): Preparation of 6-[6-fluoro-2-(3-methylimidazol-4-yl)-5-(1-methylpyrazol-4-yl)benzimidazol-1-yl]-1,3-dimethyl-4-(1-methyl-4-piperidyl)benzimidazol-2-one

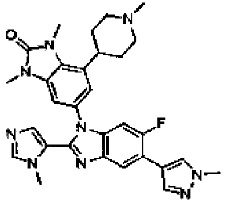

Tert-butyl 4-[6-[6-fluoro-2-(3-methylimidazol-4-yl)-5-(1-methylpyrazol-4-yl)benzimidazol-1-yl]-1,3-dimethyl-2-oxo-benzimidazol-4-yl]piperidine-1-carboxylate (Example 174, 50 mg, 0.093 mmol) was added to acetonitrile (1 mL), and formaldehyde (37 wt% in water, 15.90 mg, 0.185 mmol), sodium triacetoxyborohydride (29.5 mg, 0.139 mmol), and acetic acid (5.56 mg, 0.093 mmol) were added. The reaction solution was stirred at room temperature for 5 hours. 1 N sodium hydroxide was added to adjust the pH to about 9-11, and an organic layer was extracted with dichloromethane. The collected organic layer was concentrated under reduced pressure and separated and purified by PTLC to obtain the title compound. (18 mg, 0.033 mmol, 35.1% yield)
¹H-NMR (400 MHz, Chloroform-d) δ 7.95 (d, J = 6.9 Hz, 1H), 7.91 (s, 1H), 7.78 (d, J = 2.3 Hz, 1H), 7.51 (s, 1H), 6.99 (d, J = 2.1 Hz, 1H), 6.86-6.83 (m, 2H), 6.40 (s, 1H), 4.15 (s, 3H), 3.98 (s, 3H), 3.75 (s, 3H), 3.41 (s, 3H), 3.28-3.24 (m, 1H), 3.10-3.04 (m,2H), 2.36 (s, 3H), 2.18 (s, 2H), 1.90 (s, 4H); LC/MS: 554 (M+H).

### Example 191: Preparation of 6-[2-tert-butyl-6-fluoro-5-(5-methyl-1,2,4-oxadiazol-3-yl)benzimidazol-1-yl]-4-(isopropylamino)-1,3-dimethyl-benzimidazol-2-one

### Step 1: Preparation of 5-bromo-2-(tert-butyl)-6-fluoro-7'-(isopropylamino)-1',3'-dimethyl-1',3'-dihydro-2'H-[1,5'-bibenzo[d]imidazol]-2'-one

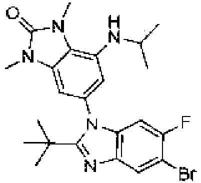

The title compound was obtained in a similar manner to Step 1 and Step 2 of Synthetic Method I using 6-amino-4-(isopropylamino)-1,3-dimethyl-1,3-dihydro-2H-benzo[d]imidazol-2-one (Intermediate I-14, 474 mg, 2.023 mmol) and 1-bromo-2,4-difluoro-nitrobenzene (481 mg, 2.023 mmol) as starting materials. (33.5% yield over 2 steps)
¹H-NMR (500 MHz, Chloroform-d) δ 7.93 (d, J = 5.8 Hz, 1H), 6.61 (d, J = 8.2 Hz, 1H), 6.42 (s, 1H), 6.40-6.34 (m, 1H), 3.80 (s, 3H), 3.59-3.50 (m, 1H), 3.37 (s, 3H), 1.39 (s, 9H), 1.28-1.16 (m, 6H); LC/MS: 488, 490 (M+H).

### Step 2: Preparation of 2-(tert-butyl)-6-fluoro-7'-(isopropylamino)-1',3'-dimethyl-2'-oxo-2',3'-dihydro-1'H-[1,5'-bibenzo[d]imidazole]-5-carbonitrile

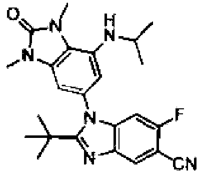

5-Bromo-2-(tert-butyl)-6-fluoro-7'-(isopropylamino)-1',3'-dimethyl-1',3'-dihydro-2'H-[1,5'-bibenzo[d]imidazol]-2'-one (50 mg, 0.102 mmol) obtained in Step 1 was dissolved in anhydrous dimethylformamide (1 mL), and then dicyanozinc (36.1 mg, 0.307 mmol) and tetrakis(triphenylphosphine)palladium (11.83 mg, 10.24 µmol) were added. The reaction solution was stirred under a nitrogen balloon at 100°C for 18 hours. After the reaction was completed, water was added and an organic layer was extracted with ethyl acetate. The organic layer was washed with water and brine, dried over anhydrous magnesium sulfate and concentrated under reduced pressure, and the residue was purified by MPLC to obtain the title compound. (44 mg, 0.101 mmol, 99% yield)
¹H-NMR (500 MHz, Chloroform-d) δ 7.92 (d, 1H), 6.60 (d, 1H), 6.40 (s, 1H), 6.30 (s, 1H), 3.93 (s, 1H), 3.78 (s, 3H), 3.53 (s, 1H), 3.34 (s, 3H), 1.38 (s, 9H), 1.23 (t, 6H); LC/MS: 435 (M+H).

### Step 3: Preparation of 6-[2-tert-butyl-6-fluoro-5-(5-methyl-1,2,4-oxadiazol-3-yl)benzimidazol-1-yl]-4-(isopropylamino)-1,3-dimethyl-benzimidazol-2-one

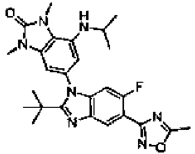

2-(Tert-butyl)-6-fluoro-7'-(isopropylamino)-1',3'-dimethyl-2'-oxo-2',3'-dihydro-1'H-[1,5'-bibenzo[d]imidazole]-5-carbonitrile (44 mg, 0.101 mmol) obtained in Step 2 and potassium carbonate (28.0 mg, 0.203 mmol) were dissolved in ethanol (1 mL), and then hydroxylamine aqueous solution (0.012 mL, 0.203 mmol) was added. The reaction solution was stirred at 80°C for 18 hours. After confirming that the starting material had disappeared, the solvent was removed by concentrating under reduced pressure, the resultant was dissolved in pyridine (1.0 mL), and acetic anhydride (10.73 µL, 0.111 mmol) was added. The reaction solution was stirred at 80°C for 3 hours. After the reaction was completed, water was added and an organic layer was extracted with ethyl acetate. The organic layer was washed with water and brine, dried over anhydrous magnesium sulfate and concentrated under reduced pressure, and the residue was purified by MPLC and PTLC to obtain the title compound. (8.5 mg, 0.017 mmol, 17.08% yield)
¹H-NMR (500 MHz, Chloroform-d) δ 8.45-8.42 (m, 1H), 6.69-6.66 (m, 1H), 6.46 (s, 1H), 6.39 (s, 1H), 3.80 (s, 3H), 3.57-3.52 (m, 1H), 3.38 (s, 3H), 2.67 (s, 3H), 1.42 (s, 9H), 1.25 (d, J = 5.8 Hz, 6H); LC/MS: 492 (M+H).

### Example 192 (Synthetic Method W): Preparation of 6-[2-tert-butyl-6-fluoro-5-(3-methyl-1,2,4-oxadiazol-5-yl)benzimidazol-1-yl]-4-(isopropylamino)-1,3-dimethyl-benzimidazol-2-one

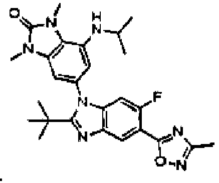

The title compound was obtained in a similar manner to Step 1 and Step 2 of Synthetic Method I using 6-amino-4-(isopropylamino)-1,3-dimethyl-1,3-dihydro-2H-benzo[d]imidazol-2-one (Intermediate I-14, 42.8 mg, 0.182 mmol) and 5-(2,4-difluoro-5-nitrophenyl)-3-methyl-1,2,4-oxadiazole (Intermediate I-57, 40 mg, 0.166 mmol) as starting materials. (23.5% yield over 2 steps)
¹H-NMR (500 MHz, Chloroform-d) δ 8.45 (s, 1H), 6.68 (d, J = 16.5 Hz, 1H), 6.45 (s, 1H), 6.38 (s, 1H), 3.80 (s, 3H), 3.55-3.53 (m, 1H), 3.37 (s, 3H), 2.49 (s, 3H), 1.41 (s, 9H), 1.24 (s, 6H); LC/MS: 492 (M+H).

### Example 219 (Synthetic Method X): Preparation of 6-[6-(difluoromethyl)-2-(3-methylimidazol-4-yl)-5-(1-methylpyrazol-4-yl)benzimidazol-1-yl]-4-(3-methoxy-3-methyl-azetidin-1-yl)-1,3-dimethyl-benzimidazol-2-one

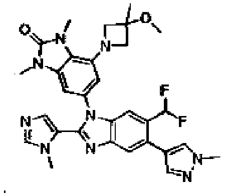

6-[6-(Difluoromethyl)-2-(3-methylimidazol-4-yl)-5-(1-methylpyrazol-4-yl)benzimidazol-1-yl]-4-(3-hydroxy-3-methyl-azetidin-1-yl)-1,3-dimethyl-benzimidazol-2-one (Example 218, 25 mg, 0.044 mmol) was added to tetrahydrofuran (0.5 mL) and sodium hydride (2.61 mg, 0.065 mmol) was added at 0°C. After stirring for 30 minutes, iodomethane (0.027 mL, 0.436 mmol) was added and stirred at room temperature for 18 hours. Water was added to the reaction solution, and an organic layer was extracted with ethyl acetate and washed with brine. The organic layer was concentrated under reduced pressure and separated by PTLC to obtain the title compound. (7 mg, 0.012 mmol, 27.3% yield)
¹H-NMR (500 MHz, Chloroform-d) δ 7.81 (s, 1H), 7.66 (s, 1H), 7.54 (s, 2H), 7.48 (s, 1H), 6.88-6.58 (m, 3H), 6.56 (s, 1H), 4.19 (s, 3H), 4.01 (s, 3H), 3.86-3.80 (2H), 3.78 (s, 3H), 3.69 (d, J = 7.0 Hz, 2H), 3.37 (s, 3H), 3.24 (s, 3H), 1.60 (s, 3H); LC/MS: 588(M+H).

### Example 243 (Synthetic Method Y): Preparation of 6-[2-tert-butyl-5-(2-methylthiazol-5-yl)-6-(trifluoromethyl)benzimidazol-1-yl]-4-(3-hydroxy-3-methyl-azetidin-1-yl)-1,3-dimethyl-benzimidazol-2-one

### Step 1: Preparation of 6-((4-bromo-2-nitro-5-(trifluoromethyl)phenyl)amino)-4-(3-hydroxy-3-methylazetidin-1-yl)-1,3-dimethyl-1,3-dihydro-2H-benzo[d]imidazol-2-one

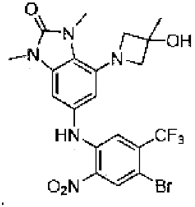

The title compound was obtained in a similar manner to Step 1 of Synthetic Method R using 6-amino-4-(3-hydroxy-3-methylazetidin-1-yl)-1,3-dimethyl-1,3-dihydro-2H-benzo[d]imidazol-2-one (Intermediate I-29, 13.12 g, 50 mmol) and 1-bromo-4-iodo-5-nitro-2-(trifluoromethyl)benzene (21.77 g, 55.0 mmol) as starting materials. (15.64 g, 29.5 mmol, 59.0% yield)
¹H-NMR (500 MHz, Chloroform-d) δ 9.46 (s, 1H), 8.51 (s, 1H), 7.45 (s, 1H), 6.54 (s, 1H), 6.48 (s, 1H), 3.78 (dd, J = 12.4, 7.5 Hz, 4H), 3.71 (s, 3H), 3.37 (s, 3H), 2.01 (s, 1H), 1.67 (s, 3H); LC/MS: 530, 532 (M+H).

### Step 2: Preparation of 5-bromo-2-(tert-butyl)-7'-(3-hydroxy-3-methylazetidin-1-yl)-1',3'-dimethyl-6-(trifluoromethyl)-1',3'-dihydro-2'H-[1,5'-bibenzo[d]imidazol]-2'-one

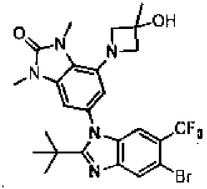

The title compound was obtained in a similar manner to Step 3 of Synthetic Method R using 6-((4-Bromo-2-nitro-5-(trifluoromethyl)phenyl)amino)-4-(3-hydroxy-3-methylazetidin-1-yl)-1,3-dimethyl-1,3-dihydro-2H-benzo[d]imidazol-2-one (135 mg, 0.255 mmol) obtained in Step 1 and pivaldehyde (55.3 µL, 0.509 mmol) as starting materials. (28 mg, 0.049 mmol, 19.4% yield)
¹H-NMR (500 MHz, Chloroform-d) δ 8.11 (s, 1H), 7.15 (s, 1H), 6.62 (s, 1H), 6.54 (s, 1H), 3.83-3.77 (m, 4H), 3.77(s, 3H), 3.40 (s, 3H), 1.68 (s, 3H), 1.38 (s, 9H); LC/MS: 566, 568 (M+H).

### Step 3: Preparation of 6-[2-tert-butyl-5-(2-methylthiazol-5-yl)-6-(trifluoromethyl)benzimidazol-1-yl]-4-(3-hydroxy-3-methyl-azetidin-1-yl)-1,3-dimethyl-benzimidazol-2-one

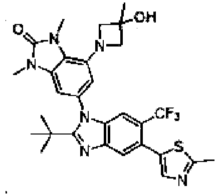

The title compound was obtained in a similar manner to Step 4 of Synthetic Method B using 5-bromo-2-(tert-butyl)-7'-(3-hydroxy-3-methylazetidin-1-yl)-1',3'-dimethyl-6-(trifluoromethyl)-1',3'-dihydro-2'H-[1,5'-bibenzo[d]imidazol]-2'-one (15 mg, 0.026 mmol) obtained in Step 2 and 2-methyl-5-(4,4,5,5-tetramethyl-1,3,2-dioxaborolan-2-yl)thiazole (8.94 mg, 0.040 mmol) as starting materials. (4.6 mg, 7.87 µmol, 29.7% yield)
¹H-NMR (500 MHz, Chloroform-d) δ 7.84 (s, 1H), 7.55 (s, 1H), 7.21 (s, 1H), 6.64 (s, 1H), 6.57 (s, 1H), 3.83-3.78 (m, 4H), 3.78 (s, 3H), 3.40 (s, 3H), 2.75 (s, 3H), 2.11 (s, 1H), 1.68 (s, 3H), 1.39 (s, 9H); LC/MS: 585 (M+H).

### Example 244: Preparation of 6-[5-(1-cyclopropylpyrazol-4-yl)-6-fluoro-2-(3-methylimidazol-4-yl)benzimidazol-1-yl]-1,3-dimethyl-4-morpholino-benzimidazol-2-one

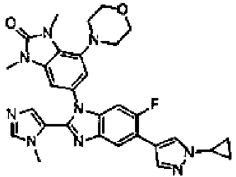

6-[6-Fluoro-2-(3-methylimidazol-4-yl)-5-(1H-pyrazol-4-yl)benzimidazol-1-yl]-1,3-dimethyl-4-morpholino-benzimidazol-2-one (Example 230, 6.8 mg, 0.013 mmol), cyclopropylboronic acid (4.43 mg, 0.052 mmol), pyridine (10.4 µL, 0.129 mmol), and triethylamine (9.0 µL, 0.064 mmol) were dissolved in tetrahydrofuran (0. 8 mL), and copper (II) acetate (7.02 mg, 0.039 mmol) was added. The reaction solution was stirred under a nitrogen balloon at 100°C for 1 hour. After the reaction was completed, the reaction solution was diluted with ethyl acetate and then passed through a celite filter, and the filtrate was concentrated. The residue was purified by PTLC to obtain the title compound. (1.5 mg, 2.64 µmol, 20.50% yield)
¹H-NMR (500 MHz, Chloroform-d) δ 7.95 (d, J = 6.7 Hz, 1H), 7.90 (s, 1H), 7.86 (s, 1H), 7.52 (s, 1H), 6.85 (m, 2H), 6.76 (s, 1H), 6.45 (s, 1H), 4.17 (s, 3H), 3.88 (s, 4H), 3.82 (s, 3H), 3.65-3.68 (m, 1H), 3.39 (s, 3H), 2.97 (s, 4H), 2.36-2.33 (m, 2H), 2.30-2.28 (m, 2H); LC/MS: 568 (M+H).

### Example 261: Preparation of 6-[2-tert-butyl-6-(methoxymethyl)-5-(1-methylpyrazol-4-yl)benzimidazol-1-yl]-1,3-dimethyl-4-morpholino-benzimidazol-2-one

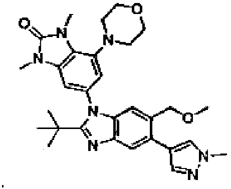

6-[2-Tert-butyl-6-(hydroxymethyl)-5-(1-methylpyrazol-4-yl)benzimidazol-1-yl]-1,3-dimethyl-4-morpholino-benzimidazol-2-one (Example 260, 15 mg, 0.028 mmol) was added to tetrahydrofuran (0.3 mL) and cooled to 0°C, and then sodium hydride (dispersed in mineral oil into 60%, 1.699 mg, 0.042 mmol) was added. After stirring for 30 minutes, iodomethane (0.018 mL, 0.283 mmol) was added to the reaction solution. The reaction solution was stirred at room temperature for 18 hours. After the reaction was completed, water was added, and an organic layer was extracted with ethyl acetate and washed with brine. The organic layer was concentrated under reduced pressure and purified by MPLC and PTLC to obtain the title compound. (11.5 mg, 0.021 mmol, 74.7% yield)
¹H-NMR (400 MHz, Chloroform-d) δ 7.80 (s, 1H), 7.65 (d, J = 0.9 Hz, 1H), 7.55 (s, 1H), 6.90 (t, J = 1.6 Hz, 2H), 6.80 (d, J = 2.3 Hz, 1H), 4.37 (s, 2H), 3.98 (s, 3H), 3.90 (s, 4H), 3.83 (s, 3H), 3.41 (s, 3H), 3.34 (s, 3H), 3.00-2.96 (m, 4H), 1.38 (s, 9H); LC/MS: 544 (M+H).

### Example 262: Preparation of 6-[2-tert-butyl-6-(fluoromethyl)-5-(1-methylpyrazol-4-yl)benzimidazol-1-yl]-1,3-dimethyl-4-morpholino-benzimidazol-2-one

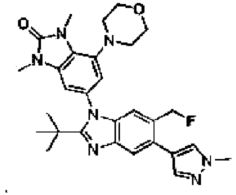

6-[2-Tert-butyl-6-(hydroxymethyl)-5-(1-methylpyrazol-4-yl)benzimidazol-1-yl]-1,3-dimethyl-4-morpholino-benzimidazol-2-one (Example 260, 15 mg, 0.028 mmol) was added to dichloromethane (0.5 mL), and diethylaminosulfur trifluoride (0.011 mL, 0.085 mmol) was added dropwisely at 0°C. The reaction solution was stirred at 0°C for 2 hours. Water and saturated sodium bicarbonate solution were added to the reaction solution, and an organic layer was extracted with dichloromethane and then washed with brine. The organic layer was concentrated under reduced pressure and separated by MPLC to obtain the title compound. (7.5 mg, 0.014 mmol, 49.8% yield)
¹H-NMR (400 MHz, Chloroform-d) δ 7.86 (s, 1H), 7.66 (s, 1H), 7.57 (s, 1H), 6.94 (d, J = 2.7 Hz, 1H), 6.91 (d, J = 1.8 Hz, 1H), 6.80 (d, J = 1.8 Hz, 1H), 5.39 (s, 1H), 5.27 (s, 1H), 3.99 (s, 3H), 3.90 (s, 4H), 3.84 (s, 3H), 3.42 (s, 3H), 2.99 (s, 4H), 1.39 (s, 9H); LC/MS: 532(M+H).

### Example 272: Preparation of methyl 2-tert-butyl-1-(1,3-dimethyl-7-morpholino-2-oxo-benzimidazol-5-yl)-6-fluoro-benzimidazole-5-carboxylate

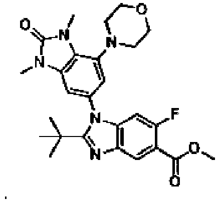

The title compound was obtained in a similar manner to Step 1 and Step 2 of Synthetic Method I using 6-amino-1,3-dimethyl-4-morpholino-1,3-dihydro-2H-benzo[d]imidazol-2-one (Intermediate I-10, 133 mg, 0.507 mmol) and methyl 2,4-difluoro-5-nitrobenzoate (100 mg, 0.461 mmol) as starting materials. (68.9% yield over 2 steps)
¹H-NMR (500 MHz, Chloroform-d) δ 8.36 (d, J = 5.5 Hz, 1H), 6.85 (s, 1H), 6.76 (s, 1H), 6.50 (d, J = 12.8 Hz, 1H), 3.91 (s, 4H), 3.87 (s, 3H), 3.79 (s, 3H), 3.40 (s, 3H), 2.96 (s, 4H), 1.35 (s, 9H); LC/MS: 496 (M+H).

### Example 273: Preparation of 2-tert-butyl-1-(1,3-dimethyl-7-morpholino-2-oxo-benzimidazol-5-yl)-6-fluoro-benzimidazole-5-carboxylic acid

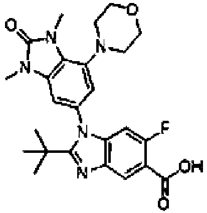

Methyl 2-tert-butyl-1-(1,3-dimethyl-7-morpholino-2-oxo-benzimidazol-5-yl)-6-fluoro-benzimidazole-5-carboxylate (Example 272, 160 mg, 0.323 mmol) was dissolved in methanol (2 mL) and water (1 mL), and then lithium hydroxide hydrate (135 mg, 3.23 mmol) was added. The reaction solution was stirred at 50°C for 1 hour. After the reaction was completed, the reaction was terminated with 1 N hydrochloric acid aqueous solution, and the pH of the reaction solution was adjusted to 5-6. An organic layer was extracted using dichloromethane. The organic layer was washed with water and brine, dried over anhydrous magnesium sulfate and concentrated under reduced pressure, and the residue was purified by PTLC to obtain the title compound.

¹H-NMR (500 MHz, Chloroform-d) δ 8.51 (d, J = 6.1 Hz, 1H), 6.88 (s, 1H), 6.79 (s, 1H), 6.54 (d, J = 10.4 Hz, 1H), 3.90 (s, 4H), 3.83 (s, 3H), 3.42 (s, 3H), 2.99 (s, 4H), 1.38 (s, 9H); LC/MS: 482 (M+H).

### Example 274 (Synthetic Method Z): Preparation of 2-tert-butyl-1-(1,3-dimethyl-7-morpholino-2-oxo-benzimidazol-5-yl)-6-fluoro-N-methylbenzimidazole-5-carboxamide

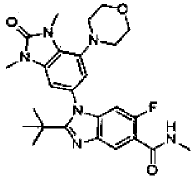

2-Tert-butyl-1-(1,3-dimethyl-7-morpholino-2-oxo-benzimidazol-5-yl)-6-fluoro-benzimidazole-5-carboxylic acid (Example 273, 35 mg, 0.073 mmol), 1-ethyl-3-(3-dimethylaminopropyl)carbodiimide (20.90 mg, 0.109 mmol), and hydroxybenzotriazole (16.70 mg, 0.109 mmol) were dissolved in dichloromethane (0.8 mL), and then methanamine (2 M in tetrahydrofuran, 0.109 mL, 0.218 mmol) was added. The reaction solution was stirred at room temperature for 18 hours. After the reaction was completed, water was added and an organic layer was extracted with dichloromethane. The organic layer was washed with water and brine, dried over anhydrous magnesium sulfate and concentrated under reduced pressure, and the residue was purified by MPLC and PTLC to obtain the title compound. (35.6 mg, 0.072 mmol, 99% yield)
¹H-NMR (500 MHz, Chloroform-d) δ 8.55 (d, J = 6.7 Hz, 1H), 6.87 (s, 1H), 6.77 (s, 1H), 6.73 (d, J = 11.6 Hz, 1H), 6.50 (d, J = 12.2 Hz, 1H), 3.90 (s, 4H), 3.82 (s, 3H), 3.41 (s, 3H), 3.04 (d, J = 4.3 Hz, 3H), 2.99 (s, 4H), 1.37 (s, 9H); LC/MS: 495 (M+H).

### Example 288: Preparation of 6-[2-tert-butyl-6-(difluoromethyl)-5-(1-methylpyrazol-4-yl)benzimidazol-1-yl]-4-hydroxy-1,3-dimethyl-benzimidazol-2-one

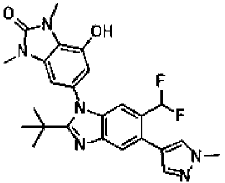

To a solution of 4-benzyloxy-6-[2-tert-butyl-6-(difluoromethyl)-5-(1-methylpyrazol-4-yl)benzimidazol-1-yl]-1,3-dimethyl-benzimidazol-2-one (Example 286, 221 mg, 0.387 mmol) dissolved in ethyl acetate (0.968 mL) and methanol (0.968 mL), palladium/carbon (10 wt%, 41.2 mg, 0.039 mmol) was added. The reactants were subjected to hydrogen/vacuum exchange three times to sufficiently fill the inside of a reaction vessel with hydrogen, and stirred for 4 hours at room temperature in the presence of a hydrogen balloon. The reaction mixture was passed through celite and filtered while eluting with dichloromethane. The filtrate was concentrated under reduced pressure, and the resulting residue was used in a subsequent reaction without further purification. (156 mg, 0.325 mmol, 84% yield)
¹H-NMR (500 MHz, Chloroform-d) δ 7.62 (s, 1H), 7.47 (s, 1H), 7.34 (s, 1H), 7.17 (s, 1H), 6.67-6.45 (m, 3H), 3.93 (s, 3H), 3.84 (s, 3H), 3.41 (s, 3H), 1.31 (s, 9H); LC/MS: 481 (M+H).

### Example 290 (Synthetic Method AA): Preparation of 6-[2-tert-butyl-6-(difluoromethyl)-5-(3-methylpyrazol-1-yl)benzimidazol-1-yl]-4-(3-hydroxy-3-methyl-azetidin-1-yl)-1,3-dimethyl-benzimidazol-2-one

### Step 1: Preparation of 5-bromo-2-(tert-butyl)-6-(difluoromethyl)-7'-(3-hydroxy-3-methylazetidin-1-yl)-1',3'-dimethyl-1',3'-dihydro-2'H-[1,5'-bibenzo[d]imidazol]-2'-one

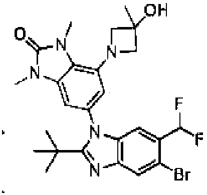

The title compound was obtained in a similar manner to Step 1 and Step 2 of Synthetic Method I using 6-amino-4-(3-hydroxy-3-methylazetidin-1-yl)-1,3-dimethyl-1,3-dihydro-2H-benzo[d]imidazol-2-one (Intermediate I-29, 180 mg, 0.686 mmol) and 1-bromo-2-(difluoromethyl)-4-fluoro-5-nitrobenzene (Intermediate I-50, 185 mg, 0.686 mmol) as starting materials. (24.4% yield over 2 steps)
¹H-NMR (400 MHz, Chloroform-d) δ 8.01 (s, 1H), 7.13 (s, 1H), 6.97 (t, J = 55.3 Hz, 1H), 6.63 (d, J = 1.8 Hz, 1H), 6.58 (d, J = 1.8 Hz, 1H), 3.82 (dd, J = 11.2, 7.1 Hz, 4H), 3.76 (s, 3H), 3.38 (s, 3H), 1.69 (s, 3H), 1.37 (s, 9H); LC/MS: 548, 550 (M+H).

### Step 2: Preparation of 6-[2-tert-butyl-6-(difluoromethyl)-5-(3-methylpyrazol-1-yl)benzimidazol-1-yl]-4-(3-hydroxy-3-methyl-azetidin-1-yl)-1,3-dimethyl-benzimidazol-2-one

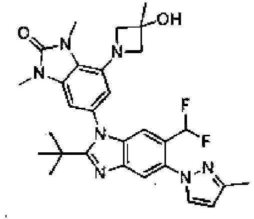

5-Bromo-2-(tert-butyl)-6-(difluoromethyl)-7'-(3-hydroxy-3-methylazetidin-1-yl)-1',3'-dimethyl-1',3'-dihydro-2'H-[1,5'-bibenzo[d]imidazol]-2'-one (32 mg, 0.058 mmol) obtained in Step 1 was dissolved in toluene (0.8 mL). (1R,2R)-N1,N2-Dimethylcyclohexane-1,2-diamine (1.84 µL, 0.012 mmol), 3-methyl-1H-pyrazole (9.58 mg, 0.117 mmol), potassium carbonate (16.13 mg, 0.117 mmol), and copper(I) iodide (0.556 mg, 2.92 µmol) were added. The mixture was stirred at 150°C for 8 h using a microwave. After the reaction was completed, water was added, and an organic layer was extracted with ethyl acetate. The organic layer was washed with water and brine, dried over anhydrous magnesium sulfate, and concentrated under reduced pressure. The residue was purified by MPLC and PTLC to obtain the title compound. (1.5 mg, 2.73 µmol, 4.68% yield)
¹H-NMR (500 MHz, Chloroform-d) δ 7.75 (s, 1H), 7.57 (s, 1H), 7.21 (s, 1H), 6.92 (t, J = 55.5 Hz, 1H), 6.66 (s, 1H), 6.58 (s, 1H), 6.25 (s, 1H), 3.83-3.80 (m, 4H), 3.78 (s, 3H), 3.41 (s, 3H), 2.37 (s, 3H), 2.04 (s, 3H), 1.68 (s, 3H), 1.39 (s, 9H); LC/MS : 550 (M+H).

### Example 292: Preparation of 6-[2-tert-butyl-6-fluoro-5-(5-methyl-1,3,4-oxadiazol-2-yl)benzimidazol-1-yl]-1,3-dimethyl-4-morpholino-benzimidazol-2-one

### Step 1: Preparation of N'-acetyl-2-(tert-butyl)-6-fluoro-1',3'-dimethyl-7'-morpholino-2'-oxo-2',3'-dihydro-1'H-[1,5'-bibenzo[d]imidazole]-5-carbohydrazide

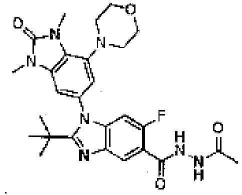

2-Tert-butyl-1-(1,3-dimethyl-7-morpholino-2-oxo-benzimidazol-5-yl)-6-fluoro-benzimidazole-5-carboxylic acid (Example 273, 42.3 mg, 0.088 mmol), 1-ethyl-3-(3-dimethylaminopropyl)carbodiimide (25.3 mg, 0.132 mmol), and hydroxybenzotriazole (20.18 mg, 0.132 mmol) were dissolved in dimethylformamide (1 mL), and acetohydrazide (19.52 mg, 0.264 mmol) was added. The reaction solution was stirred at room temperature for 18 hours. After the reaction was completed, water was added, and an organic layer was extracted with dichloromethane. The organic layer was washed with water and brine, dried over anhydrous magnesium sulfate and concentrated under reduced pressure, and the residue was purified by MPLC to obtain the title compound. (44 mg, 0.082 mmol, 9% yield)
¹H-NMR (500 MHz, Chloroform-d) δ 9.47 (q, J = 6.4 Hz, 1H), 9.33 (s, 1H), 8.52 (d, J = 6.7 Hz, 1H), 6.86 (s, 1H), 6.77 (s, 1H), 6.56 (d, J = 11.6 Hz, 1H), 3.90 (s, 4H), 3.82 (s, 3H), 3.42 (s, 3H), 2.99 (s, 4H), 2.14 (s, 3H), 1.37 (s, 9H); LC/MS: 538 (M+H).

### Step 2: Preparation of 6-[2-tert-butyl-6-fluoro-5-(5-methyl-1,3,4-oxadiazol-2-yl)benzimidazol-1-yl]-1,3-dimethyl-4-morpholino-benzimidazol-2-one

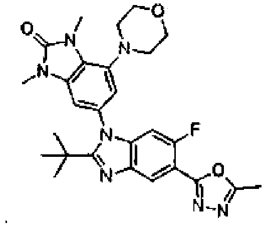

N'-Acetyl-2-(tert-butyl)-6-fluoro-1',3'-dimethyl-7'-morpholino-2'-oxo-2',3'-dihydro-1'H-[1,5'-bibenzo[d]imidazole]-5-carbohydrazide (41 mg, 0.076 mmol) obtained in Step 1 was dissolved in tetrahydrofuran (0.8 mL), and then Burgess reagent (72.6 mg, 0.306 mmol) was added. The reaction solution was stirred at 100°C for 2 hours. After the reaction was completed, water was added and an organic layer was extracted with ethyl acetate. The organic layer was washed with water and brine, dried over anhydrous magnesium sulfate and concentrated under reduced pressure, and the residue was purified by MPLC to obtain the title compound. (30 mg, 0.058 mmol, 76% yield)
¹H-NMR (500 MHz, Chloroform-d) δ 8.32 (d, J = 5.8 Hz, 1H), 6.88 (s, 1H), 6.79 (s, 1H), 6.62 (d, J = 10.1 Hz, 1H), 3.88 (s, 4H), 3.81 (s, 3H), 3.41 (s, 3H), 2.98 (s, 4H), 2.61 (s, 3H), 1.36 (s, 9H); LC/MS: 520 (M+H).

### Example 299: Preparation of (5E)-2-tert-butyl-3-(1,3-dimethyl-7-morpholino-2-oxo-benzimidazol-5-yl)-6-(1-methylpyrazol-4-yl)benzimidazole-5-carbaldehyde oxime

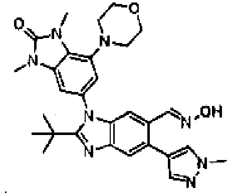

2-Tert-butyl-3-(1,3-dimethyl-7-morpholino-2-oxo-benzimidazol-5-yl)-6-(1-methylpyrazol-4-yl)benzimidazole-5-carbaldehyde (Example 285, 12 mg, 0.023 mmol) was added to methanol (0.5 mL), and hydroxylamine hydrochloride (1.738 mg, 0.025 mmol) and potassium carbonate (3.46 mg, 0.025 mmol) were added. Stirring was performed at room temperature for 2 hours. Water was added to the reaction solution and an organic layer was extracted with ethyl acetate. The collected organic layer was concentrated under reduced pressure and purified by MPLC to obtain the title compound. (11.1 mg, 0.020 mmol, 90% yield)
¹H-NMR (500 MHz, Chloroform-d) δ 8.34 (s, 1H), 7.77 (s, 1H), 7.57-7.53 (1H), 7.39 (s, 1H), 7.32-7.28 (1H), 6.91-6.84 (1H), 6.78 (s, 1H), 4.00-3.96 (3H), 3.89 (s, 4H), 3.82 (s, 3H), 3.40 (s, 3H), 2.98 (s, 4H), 1.38 (s, 9H); LC/MS: 543 (M+H).

### Example 300: Preparation of 6-[2-tert-butyl-6-(difluoromethyl)-5-(1-methylpyrazol-4-yl)benzimidazol-1-yl]-4-(6-chloropyrimidin-4-yl)oxy-1,3-dimethyl-benzimidazol-2-one

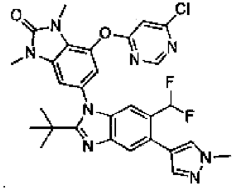

A reaction solution of 6-[2-tert-butyl-6-(difluoromethyl)-5-(1-methylpyrazol-4-yl)benzimidazol-1-yl]-4-hydroxy-1,3-dimethyl-benzimidazol-2-one (Example 288, 15 mg, 0.031 mmol), 4,6-dichloropyrimidine (5.12 mg, 0.034 mmol), and potassium carbonate (12.94 mg, 0.094 mmol) dissolved in dimethylformamide (0.312 mL) was stirred at 100°C for 3 hours. The reaction mixture was cooled to room temperature, diluted with water, and extracted with ethyl acetate. The obtained organic layer was washed with brine, dried over anhydrous sodium sulfate and concentrated under reduced pressure, and the residue was purified by MPLC to obtain the title compound. (8.9 mg, 0.015 mmol, 48.1% yield)
¹H-NMR (500 MHz, Chloroform-d) δ 8.57 (s, 1H), 7.77 (s, 1H), 7.60 (s, 1H), 7.47 (s, 1H), 7.26 (s, 1H), 7.12 (s, 1H), 6.94 (s, 1H), 6.90 (s, 1H), 6.68 (t, J = 55.5 Hz, 1H), 3.99 (s, 3H), 3.52 (s, 3H), 3.47 (s, 3H), 1.43 (s, 9H); LC/MS: 593 (M+H).

### Example 301 (Synthetic Method AB): Preparation of 6-[2-tert-butyl-6-(difluoromethyl)-5-(1-methylpyrazol-4-yl)benzimidazol-1-yl]-4-(4-methoxyphenoxy)-1,3-dimethyl-benzimidazol-2-one

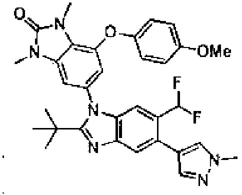

To a solution of 6-[2-tert-butyl-6-(difluoromethyl)-5-(1-methylpyrazol-4-yl)benzimidazol-1-yl]-4-hydroxy-1,3-dimethyl-benzimidazol-2-one (Example 288, 15 mg, 0.031 mmol), (4-methoxyphenyl)boronic acid (14.2 mg, 0.094 mmol), and copper(II) acetate (5.67 mg, 0.031 mmol), dissolved in dichloromethane (1.56 mL), 4 Å molecular sieves were added, followed by stirring at room temperature for 10 minutes. Triethylamine (26.1 µL, 0.187 mmol) was added to the reaction solution and stirred at room temperature for 18 hours after allowing air to pass through a reaction vessel. The reaction mixture was diluted with dichloromethane and filtered through celite. The filtrate was concentrated under reduced pressure, and the residue was purified by MPLC to obtain the title compound. (15.2 mg, 0.026 mmol, 83% yield)
¹H-NMR (400 MHz, Chloroform-d) δ 7.73 (s, 1H), 7.58 (s, 1H), 7.46 (s, 1H), 7.17 (s, 1H), 7.00 (d, J = 9.1 Hz, 2H), 6.86 (d, J = 9.1 Hz, 2H), 6.80-6.52 (m, 2H), 6.51 (d, J = 1.8 Hz, 1H), 3.98 (s, 3H), 3.76 (s, 3H), 3.74 (s, 3H), 3.45 (s, 3H), 1.35 (s, 9H); LC/MS: 587 (M+H).

### Example 307: Preparation of 6-[2-tert-butyl-6-fluoro-5-(5-methyl-4H-1,2,4-triazol-3-yl)benzimidazol-1-yl]-1,3-dimethyl-4-morpholino-benzimidazol-2-one

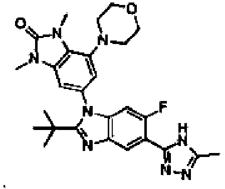

6-[2-Tert-butyl-6-fluoro-5-(5-methyl-1,3,4-oxadiazol-2-yl)benzimidazol-1-yl]-1,3-dimethyl-4-morpholino-benzimidazol-2-one (Example 292, 17 mg, 0.033 mmol) was dissolved in acetic acid (0.6 mL), and then ammonium acetate (50.4 mg, 0.654 mmol) was added. The reaction solution was stirred using a microwave at 150°C for 12 hours. After the reaction was completed, 1 N sodium hydroxide aqueous solution was added and an organic layer was extracted with ethyl acetate. The organic layer was washed with water and brine, dried over anhydrous magnesium sulfate and concentrated under reduced pressure, and the residue was purified by PTLC to obtain the title compound. (8 mg, 0.015 mmol, 47.1% yield)
¹H-NMR (500 MHz, Chloroform-d) δ 8.64 (s, 1H), 6.89 (s, 1H), 6.80 (s, 1H), 6.60 (d, J = 11.3 Hz, 1H), 3.91 (s, 4H), 3.83 (s, 3H), 3.42 (s, 3H), 3.00 (s, 4H), 2.52 (s, 3H), 1.39 (s, 9H); LC/MS: 519 (M+H).

### Example 311: Preparation of 6-[2-tert-butyl-6-(difluoromethyl)-5-(1-methylpyrazol-4-yl)benzimidazol-1-yl]-4-(1,1-dioxo-1,4-thiazinan-4-yl)-1,3-dimethyl-benzimidazol-2-one

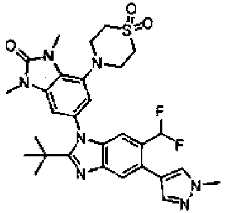

6-[2-Tert-butyl-6-(difluoromethyl)-5-(1-methylpyrazol-4-yl)benzimidazol-1-yl]-1,3-dimethyl-4-thiomorpholino-benzimidazol-2-one (Example 298, 8.6 mg, 0.015 mmol) and 3-chlorobenzoperoxoic acid (15.33 mg, 0.068 mmol) were stirred at 10-15°C for 3 hours in chloroform (1 mL). The reaction was terminated with a saturated sodium bicarbonate aqueous solution. The reaction product was diluted with ethyl acetate, washed with water, dried over anhydrous magnesium sulfate, and concentrated under reduced pressure. The residue was purified by MPLC to obtain the title compound. (6 mg, 0.010 mmol, 66.0% yield)
¹H-NMR (500 MHz, Chloroform-d) δ 7.80 (s, 1H), 7.62 (s, 1H), 7.50 (s, 1H), 7.14 (s, 1H), 6.99 (d, J = 1.68 Hz, 1H), 6.88 (d, J = 1.53 Hz, 1H), 6.68 (t, 1H), 4.01 (s, 3H), 3.84 (s, 3H), 3.55 (br. s., 4H), 3.50-3.36 (m, 5H), 3.16 (br. s., 2H); LC/MS: 598 (M+H).

### Example 315: Preparation of 6-[2-tert-butyl-6-fluoro-5-(1-methyltriazol-4-yl)benzimidazol-1-yl]-1,3-dimethyl-4-morpholino-benzimidazol-2-one

### Step 1: Preparation of 6-((4-ethynyl-5-fluoro-2-nitrophenyl)amino)-1,3-dimethyl-4-morpholino-1,3-dihydro-2H-benzo[d]imidazol-2-one

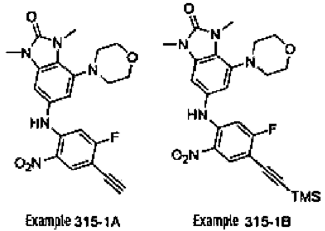

A mixture of two title compounds was obtained in the same manner as Step 1 of Synthetic Method I using 6-amino-1,3-dimethyl-4-morpholino-1,3-dihydro-2H-benzo[d]imidazol-2-one (Intermediate I-10, 362 mg, 1.379 mmol) and ((2,4-difluoro-5-nitrophenyl)ethynyl)trimethylsilane (Intermediate I-56, 320 mg, 1.253 mmol) as starting materials. (202 mg, 0.475 mmol, 37.9% yield)
Example 315-1A: ¹H-NMR (500 MHz, Chloroform-d) δ 9.65 (s, 1H), 8.43 (d, J = 7.0 Hz, 1H), 6.77 (s, 1H), 6.69 (s, 1H), 6.65 (d, J = 11.0 Hz, 1H), 3.90 (s, 4H), 3.76 (s, 3H), 3.40 (s, 3H), 3.22 (s, 1H), 2.98 (s, 4H); LC/MS: 426 (M+H).

Example 315-1B: ¹H-NMR (500 MHz, Chloroform-d) δ 9.63 (s, 1H), 8.40 (d, J = 7.0 Hz, 1H), 6.79 (s, 1H), 6.70 (s, 1H), 6.63 (d, 1H), 3.90 (s, 4H), 3.76 (s, 3H), 3.40 (s, 3H), 2.98 (s, 4H), 0.25 (s, 9H).

### Step 2: Preparation of 2-(tert-butyl)-5-ethynyl-6-fluoro-1',3'-dimethyl-7'-morpholino-1',3'-dihydro-2'H-[1,5'-benzo[d]imidazol]-2'-one and 2-(tert-butyl)- 6-fluoro-1',3'-dimethyl-7'-morpholino-5-((trimethylsily)ethynyl)-1',3'-dihydro-2'H-[1,5'-benzo[d]imidazol]-2'-one

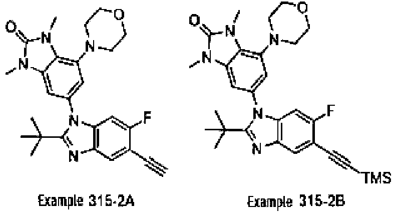

A mixture of the two title compounds was obtained in the same manner as in Step 2 of Synthetic Method I using a mixture of 6-((4-ethynyl-5-fluoro-2-nitrophenyl)amino)-1,3-dimethyl-4-morpholino-1,3-dihydro-2H-benzo[d]imidazol-2-one obtained in Step 1, and 6-((5-fluoro-2-nitro-4-((trimethylsilyl)ethynyl)phenyl)amino)-1,3-dimethyl-4-morpholino-1,3-dihydro-2H-benzo[d]imidazol-2-one (202 mg, 0.475 mmol), and pivaldehyde (155 µL, 1.424 mmol) as starting materials. (83 mg, 0.180 mmol, 37% yield)
Example 315-2A : ¹H-NMR (500 MHz, Chloroform-d) δ 7.87 (d, J = 5.8 Hz, 1H), 6.86 (s, 1H), 6.76 (s, 1H), 6.49 (d, J = 8.9 Hz, 1H), 3.89 (s, 4H), 3.81 (s, 3H), 3.41 (s, 3H), 3.25 (s, 1H), 2.98 (s, 4H), 1.36 (s, 9H); LC/MS: 462 (M+H).

Example 315-2B : ¹H-NMR (500 MHz, Chloroform-d) δ 7.80 (d, J = 5.8 Hz, 1H), 6.85 (s, 1H), 6.75 (s, 1H), 6.44 (d, J = 8.9 Hz, 1H), 3.89 (s, 4H), 3.80 (s, 3H), 3.40 (s, 3H), 2.97 (s, 4H), 1.34 (s, 9H), 0.25 (s, 9H); LC/MS: 534 (M+H).

### Step 3: Preparation of 2-(tert-butyl)-5-ethynyl-6-fluoro-1',3'-dimethyl-7'-morpholino-1',3'-dihydro-2'H-[1,5'-bibenzo[d]imidazol]-2'-one

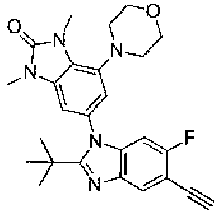

A mixture of 2-(tert-butyl)-5-ethynyl-6-fluoro-1',3'-dimethyl-7'-morpholino-1',3'-dihydro-2'H-[1,5'-benzo[d]imidazol]-2'-one and 2-(tert-butyl)-6-fluoro-1',3'-dimethyl-7'-morpholino-5-((trimethylsilyl)ethynyl)-1',3'-dihydro-2'H-[1,5'-bibenzo[d]imidazol]-2'-one obtained in Step 2 (83 mg, 0.156 mmol) was dissolved in tetrahydrofuran (0.8 mL), and then tetra-n-butylammonium fluoride (1 M in tetrahydrofuran, 0.311 mL, 0.311 mmol) was added. The reaction solution was stirred at room temperature for 1 hour. After the reaction was completed, the solvent was removed by concentration under reduced pressure, and ethyl acetate was added to dilute. Water was added, and an organic layer was extracted with ethyl acetate. The organic layer was washed with a saturated ammonium chloride aqueous solution and brine, dried over anhydrous magnesium sulfate and concentrated under reduced pressure, and the residue was purified by MPLC to obtain the title compound. (61 mg, 0.132 mmol, 85% yield)
¹H-NMR (500 MHz, Chloroform-d) δ 7.85 (d, J = 5.2 Hz, 1H), 6.85 (s, 1H), 6.76 (s, 1H), 6.48 (d, J = 8.9 Hz, 1H), 3.88 (s, 4H), 3.80 (s, 3H), 3.39 (s, 3H), 3.24 (s, 1H), 2.97 (s, 4H), 1.34 (s, 9H); LC/MS: 462 (M+H).

### Step 4: Preparation of 2-(tert-butyl)-6-fluoro-1',3'-dimethyl-7'-morpholino-5-(1-((trimethylsilyl)methyl)-1H-1,2,3-triazol-4-yl)-1',3'-dihydro-2'H-[1,5'-bibenzo[d]imidazol]-2'-one

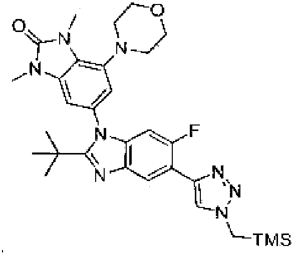

2-(Tert-butyl)-5-ethynyl-6-fluoro-1',3'-dimethyl-7'-morpholino-1',3'-dihydro-2'H-[1,5'-bibenzo[d]imidazol]-2'-one (32 mg, 0.069 mmol) obtained in Step 3 was dissolved in dimethylformamide (0.8 mL), and copper(I) iodide (2.64 mg, 0.014 mmol) and diisopropylethylamine (0.012 mL, 0.069 mmol) were added. After 5 minutes, (azidomethyl)trimethylsilane (0.051 mL, 0.347 mmol) was added, and the reaction solution was stirred at room temperature for 8 hours. After the reaction was completed, water was added, and an organic layer was extracted with ethyl acetate. The organic layer was washed with water and brine, dried over anhydrous magnesium sulfate and concentrated under reduced pressure, and the residue was purified by MPLC to obtain the title compound. (29 mg, 0.049 mmol, 70.8% yield)
¹H-NMR (500 MHz, Chloroform-d) δ 8.62 (d, J = 6.1 Hz, 1H), 7.72 (s, 1H), 6.89 (s, 1H), 6.79 (s, 1H), 6.53 (d, J = 10.1 Hz, 1H), 3.93 (s, 2H), 3.89 (s, 4H), 3.81 (s, 3H), 3.41 (s, 3H), 2.99 (s, 4H), 1.37 (s, 9H), 0.17 (s, 9H); LC/MS: 591 (M+H).

### Step 5: Preparation of 6-[2-tert-butyl-6-fluoro-5-(1-methyltriazol-4-yl)benzimidazol-1-yl]-1,3-dimethyl-4-morpholino-benzimidazol-2-one

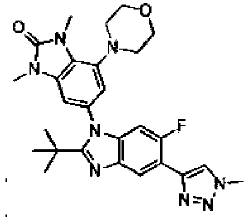

The title compound was obtained in a similar manner to Step 3 above using 2-(tert-butyl)-6-fluoro-1',3'-dimethyl-7'-morpholino-5-(1-((trimethylsilyl)methyl)-1H-1,2,3-triazol-4-yl)-1',3'-dihydro-2'H-[1,5'-bibenzo[d]imidazol]-2'-one (29 mg, 0.049 mmol) obtained in Step 4 as a starting material. (23 mg, 0.044 mmol, 90% yield)
¹H-NMR (500 MHz, Chloroform-d) δ 8.62 (s, 1H), 7.83 (s, 1H), 6.89 (s, 1H), 6.79 (s, 1H), 6.54 (d, J = 10.1 Hz, 1H), 4.13 (s, 3H), 3.89 (s, 4H), 3.81 (s, 3H), 3.41 (s, 3H), 2.98 (s, 4H), 1.36 (s, 9H); LC/MS: 519 (M+H).

### Example 317 (Synthetic Method AC): Preparation of 6-[6-amino-2-tert-butyl-5-(1-methylpyrazol-4-yl)benzimidazol-1-yl]-1,3-dimethyl-4-morpholino-benzimidazol-2-one

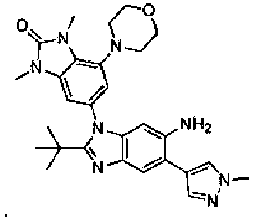

Tert-butyl N-[2-tert-butyl-3-(1,3-dimethyl-7-morpholino-2-oxo-benzimidazol-5-yl)-6-(1-methylpyrazol-4-yl)benzimidazol-5-yl]carbamate (Example 316, 160 mg, 0.260 mmol) was dissolved in dichloromethane (2 mL), and hydrochloric acid (4 M in 1,4-dioxane, 0.651 mL, 2.60 mmol) was added. The reaction solution was stirred at room temperature for 1 hour. The pH of the reaction solution was adjusted to 10 by adding 1 N sodium hydroxide and the reaction solution was extracted with dichloromethane. An organic layer was concentrated under reduced pressure and used in a subsequent reaction without further purification. (120 mg, 0.233 mmol, 90% yield)
¹H-NMR (400 MHz, Chloroform-d) δ 7.67 (s, 1H), 7.59 (s, 1H), 7.54 (s, 1H), 6.91 (d, J = 1.8 Hz, 1H), 6.80 (d, J = 1.8 Hz, 1H), 6.15 (s, 1H), 3.97 (s, 3H), 3.91 (s, 4H), 3.82 (s, 3H), 3.65 (s, 2H), 3.41 (s, 3H), 2.99 (s, 4H), 1.36 (s, 9H); LC/MS: 515 (M+H).

### Example 318 (Synthetic Method AD): Preparation of 6-[2-tert-butyl-6-(methylaminomethyl)-5-(1-methylpyrazol-4-yl)benzimidazol-1-yl]-1,3-dimethyl-4-morpholino-benzimidazol-2-one

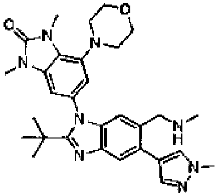

2-Tert-butyl-3-(1,3-dimethyl-7-morpholino-2-oxo-benzimidazol-5-yl)-6-(1-methylpyrazol-4-yl)benzimidazole-5-carbaldehyde (Example 285, 20 mg, 0.038 mmol) was added to dichloroethane (0.5 mL), and methanamine (2 M in tetrahydrofuran, 0.038 mL, 0.076 mmol), acetic acid (2.170 µL, 0.038 mmol) and sodium triacetoxyborohydride (10.44 mg, 0.049 mmol) were added. The reaction solution was stirred at room temperature for 4 hours. After the reaction was completed, water and 1 N sodium hydroxide were added to adjust the pH to 9-11, and an organic layer was extracted with dichloromethane. The collected organic layer was concentrated under reduced pressure and separated by PTLC to obtain the title compound. (7 mg, 0.013 mmol, 34.0% yield)
¹H-NMR (400 MHz, Chloroform-d) δ 7.75 (s, 1H), 7.63 (s, 1H), 7.56 (s, 1H), 6.90 (d, J = 1.8 Hz, 1H), 6.85 (s, 1H), 6.80 (d, J = 1.4 Hz, 1H), 3.98 (s, 3H), 3.91 (s, 4H), 3.83 (s, 3H), 3.80-3.73 (m, 2H), 3.42 (s, 3H), 2.99 (s, 4H), 2.36 (s, 3H), 2.23 (s, 1H), 1.37 (s, 9H); LC/MS: 543 (M+H).

### Example 321: Preparation of N-[2-tert-butyl-3-(1,3-dimethyl-7-morpholino-2-oxo-benzimidazol-5-yl)-6-(1-methylpyrazol-4-yl)benzimidazol-5-yl]acetamide

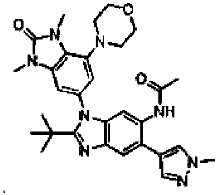

6-[6-Amino-2-tert-butyl-5-(1-methylpyrazol-4-yl)benzimidazol-1-yl]-1,3-dimethyl-4-morpholino-benzimidazol-2-one (Example 317, 30 mg, 0.058 mmol) was added to dichloromethane (1 mL), and acetyl chloride (4.16 µL, 0.058 mmol) and diisopropylethylamine (0.020 mL, 0.117 mmol) were added. Stirring was performed at room temperature for 18 hours. Water was added to the reaction solution, and an organic layer was extracted with dichloromethane and then concentrated under reduced pressure. The title compound was obtained by purification using MPLC. (22.3 mg, 0.040 mmol, 68.7% yield)
¹H-NMR (400 MHz, Chloroform-d) δ 7.70 (s, 1H), 7.66 (s, 1H), 7.59 (s, 1H), 7.47 (s, 1H), 7.38 (s, 1H), 6.90 (d, J = 1.8 Hz, 1H), 6.80 (d, J = 1.8 Hz, 1H), 4.01 (s, 3H), 3.89 (s, 4H), 3.82 (s, 3H), 3.40 (s, 3H), 2.99 (d, J = 4.6 Hz, 4H), 2.05 (s, 3H), 1.37 (s, 9H); LC/MS: 557 (M+H).

### Example 325 (Synthetic Method AE): Preparation of 6-[2-tert-butyl-6-fluoro-5-(1-methylpyrazol-4-yl)benzimidazol-1-yl]-1,3-dimethyl-4-(oxetan-3-ylmethoxy)benzimidazol-2-one

### Step 1: Preparation of 7'-(benzyloxy)-2-(tert-butyl)-6-fluoro-1',3'-dimethyl-5-(1-methyl-1H-pyrazol-4-yl)-1',3'-dihydro-2'H-[1,5'-bibenzo[d]imidazol]-2'-one

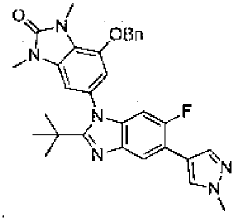

The title compound was obtained in a similar manner to Synthetic Method M using 6-amino-4-(benzyloxy)-1,3-dimethyl-1,3-dihydro-2H-benzo[d]imidazol-2-one (4.53 g, 16 mmol), 1-bromo-2,4-difluoro-5-nitrobenzene (3.81 g, 16.00 mmol), 1-methyl-4-(4,4,5,5-tetramethyl-1,3,2-dioxaborolan-2-yl)-1H-pyrazole (2.70 g, 13.00 mmol), and pivaldehyde (2.12 mL, 19.50 mmol) as starting materials. (2.71 g, 5.03 mmol, 52% yield over 3 steps)
¹H-NMR (400 MHz, Chloroform-d) δ 7.91 (d, J = 6.4 Hz, 1H), 7.85 (s, 1H), 7.72 (s, 1H), 7.38-7.34 (m, 5H), 6.65 (s, 2H), 6.50 (d, J = 10.5 Hz, 1H), 5.19 (d, J = 12.3 Hz, 1H), 5.11 (d, J = 11.9 Hz, 1H), 3.96 (s, 3H), 3.75 (s, 3H), 3.41 (s, 3H), 1.31 (s, 9H); LC/MS: 539 (M+H).

### Step 2: Preparation of 2-(tert-butyl)-6-fluoro-7'-hydroxy-1',3'-dimethyl-5-(1-methyl-1H-pyrazol-4-yl)-1',3'-dihydro-2'H-[1,5'-bibenzo[d]imidazol]-2'-one

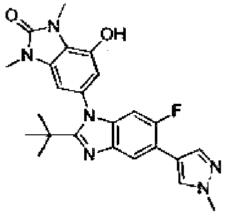

The title compound was obtained in a similar manner to the synthesis of Example 288 using 7'-(benzyloxy)-2-(tert-butyl)-6-fluoro-1',3'-dimethyl-5-(1-methyl-1H-pyrazol-4-yl)-1',3'-dihydro-2'H-[1,5'-bibenzo[d]imidazol]-2'-one (2.478 g, 4.6 mmol) obtained in Step 1 as a starting material. (2.04 g, 4.55 mmol, 99% yield)
¹H-NMR (400 MHz, Chloroform-d) δ 7.75 (s, 1H), 7.74 (d, J = 6.4 Hz, 1H), 7.66 (d, J = 2.3 Hz, 1H), 6.52 (d, J = 1.4 Hz, 1H), 6.46 (d, J = 10.1 Hz, 1H), 6.13 (s, 1H), 3.94 (s, 3H), 3.84 (s, 3H), 3.40 (s, 3H), 1.26 (s, 9H); LC/MS: 449 (M+H).

### Step 3: Preparation of 6-[2-tert-butyl-6-fluoro-5-(1-methylpyrazol-4-yl)benzimidazol-1-yl]-1,3-dimethyl-4-(oxetan-3-ylmethoxy)benzimidazol-2-on

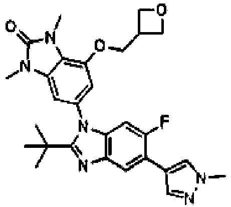

To a solution of 2-(tert-butyl)-6-fluoro-7'-hydroxy-1',3'-dimethyl-5-(1-methyl-1H-pyrazol-4-yl)-1',3'-dihydro-2'H-[1,5'-bibenzo[d]imidazol]-2'-one (27.0 mg, 0.060 mmol) obtained in Step 2 and cesium carbonate (23.5 mg, 0.072 mmol) dissolved in dimethylformamide (0.300 mL), 3-(bromomethyl)oxetane (9.06 mg, 0.060 mmol) was added, followed by stirring at 100°C for 18 hours. The reaction mixture was cooled to room temperature, diluted with water, and extracted with ethyl acetate. The obtained organic layer was washed with brine, dried over anhydrous sodium sulfate and concentrated under reduced pressure, and the residue was purified by MPLC to obtain the title compound. (26.7 mg, 0.051 mmol, 86% yield)
¹H-NMR (500 MHz, Chloroform-d) δ 7.92 (d, J = 6.7 Hz, 1H), 7.86 (s, 1H), 7.72 (s, 1H), 6.70 (s, 1H), 6.65 (s, 1H), 6.56 (d, J = 10.4 Hz, 1H), 4.94-4.91 (m, 2H), 4.64 (t, J = 6.1 Hz, 2H), 4.26-4.20 (m, 2H), 3.96 (s, 3H), 3.74 (s, 3H), 3.52-3.46 (m, 1H), 3.42 (s, 3H), 1.39-1.42 (9H); LC/MS: 519 (M+H).

### Example 326 (Synthetic Method AF): Preparation of 6-[2-tert-butyl-6-fluoro-5-(1-methylpyrazol-4-yl)benzimidazol-1-yl]-1,3-dimethyl-4-[(1-methyl-4-piperidyl)oxy]benzimidazol-2-one

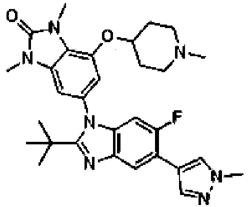

To a solution of 2-(tert-butyl)-6-fluoro-7'-hydroxy-1',3'-dimethyl-5-(1-methyl-1H-pyrazol-4-yl)-1',3'-dihydro-2'H-[1,5'-bibenzo[d]imidazol]-2'-one (a compound of Step 2 of Synthetic Method AE, 22.0 mg, 0.050 mmol) dissolved in anhydrous tetrahydrofuran (0.500 mL), 1-methylpiperidin-4-ol (6.91 mg, 0.060 mmol) and triphenylphosphine (19.7 mg, 0.075 mmol) were added. The temperature of the reactants was lowered to 0°C, diisopropyl azodicarboxylate (14.6 µL, 0.075 mmol) was added, and the resultant was stirred at room temperature for 18 hours. The reaction mixture was diluted with water and extracted with ethyl acetate. The obtained organic layer was washed with brine, dried over anhydrous sodium sulfate and concentrated under reduced pressure, and the residue was purified by MPLC to obtain the title compound. (15.9 mg, 0.029 mmol, 58.3% yield)
¹H-NMR (500 MHz, Chloroform-d) δ 7.92 (d, J = 6.7 Hz, 1H), 7.86 (s, 1H), 7.72 (d, J = 2.1 Hz, 1H), 6.64 (d, J = 1.5 Hz, 1H), 6.61 (d, J = 1.5 Hz, 1H), 6.56 (d, J = 10.4 Hz, 1H), 4.37 (s, 1H), 3.96 (s, 3H), 3.75 (s, 3H), 3.41 (s, 3H), 2.68 (s, 2H), 2.34-2.29 (m, 5H), 2.05 (t, J = 6.9 Hz, 3H), 1.95-1.87 (m, 3H), 1.41-1.39 (9H); LC/MS: 546 (M+H).

### Examples 331 and 332: Synthesis of 6-[2-tert-butyl-6-(methylamino)-5-(1-methylpyrazol-4-yl)benzimidazol-1-yl]-1,3-dimethyl-4-morpholino-benzimidazol-2-one and 6-[2-tert-butyl-6-(dimethylamino)-5-(1-methylpyrazol-4-yl)benzimidazol-1-yl]-1,3-dimethyl-4-morpholino-benzimidazol-2-one

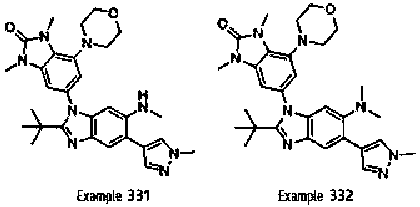

6-[6-Amino-2-tert-butyl-5-(1-methylpyrazol-4-yl)benzimidazol-1-yl]-1,3-dimethyl-4-morpholino-benzimidazol-2-one (Example 317, 30 mg, 0.058 mmol) was added to 1,2-dichloroethane (0.5 mL), and formaldehyde (37 wt% in water, 4.34 µL, 0.058 mmol), acetic acid (3.34 µL, 0.058 mmol), and sodium triacetoxyborohydride (16.06 mg, 0.076 mmol) were added. Stirring was performed at room temperature for 3 hours. When the reaction was completed, water and 1 N sodium hydroxide were added to adjust the pH to 9-11, and an organic layer was extracted with dichloromethane. The collected organic layer was concentrated under reduced pressure and separated by PTLC to obtain two title compounds, Example 331 (7 mg, 0.013 mmol, 22.71% yield) and Example 332 (5 mg, 0.009 mmol, 15.9% yield)
Example 331: ¹H-NMR (400 MHz, Chloroform-d) δ 7.63 (s, 1H), 7.56 (s, 1H), 7.48 (s, 1H), 6.92 (d, J = 1.4 Hz, 1H), 6.82 (d, J = 1.4 Hz, 1H), 5.92 (s, 1H), 3.98 (s, 3H), 3.90 (s, 4H), 3.84 (s, 3H), 3.42 (s, 3H), 2.99 (s, 4H), 2.66 (s, 3H), 1.35 (s, 9H); LC/MS: 529 (M+H).

Example 332: ¹H-NMR (400 MHz, Chloroform-d) δ 7.90 (s, 1H), 7.83 (s, 1H), 7.81 (s, 1H), 6.92 (s, 1H), 6.83 (s, 1H), 6.46 (s, 1H), 3.96 (s, 3H), 3.92 (s, 4H), 3.84 (s, 3H), 3.43 (s, 3H), 3.00 (s, 4H), 2.53 (s, 6H), 1.35 (s, 9H); LC/MS: 543 (M+H).

### Example 333: Preparation of 6-[2-tert-butyl-6-(difluoromethyl)-5-(1-methylpyrazol-4-yl)benzimidazol-1-yl]-4-[(3-chloro-2-hydroxy-2-methylpropyl)amino]-1,3-dimethyl-benzimidazol-2-one

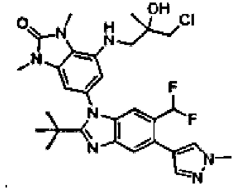

6-[2-Tert-butyl-6-(difluoromethyl)-5-(1-methylpyrazol-4-yl)benzimidazol-1-yl]-4-(3-hydroxy-3-methyl-azetidin-1-yl)-1,3-dimethyl-benzimidazol-2-one (Example 183, 100 mg, 0.18 mmol) was added to dichloromethane (2 mL), and hydrochloric acid (4 M in 1,4-dioxane, 0.045 mL, 0.18 mmol) was added, followed by stirring for 1 hour. The reaction solution was concentrated under reduced pressure and separated by MPLC to obtain the title compound.

¹H-NMR (500 MHz, Chloroform-d) δ 7.78 (s, 1H), 7.60 (s, 1H), 7.48 (s, 1H), 7.22 (s, 1H), 6.67 (t, J = 55.7 Hz, 1H), 6.53 (s, 1H), 6.42 (s, 1H), 4.70 (s, 1H), 3.99 (s, 3H), 3.84 (s, 3H), 3.76-3.56 (m, 2H), 3.40 (s, 3H), 3.23 (d, J = 5.8 Hz, 1H), 3.13 (t, J = 5.6 Hz, 1H), 2.70 (s, 1H), 1.42 (s, 9H); LC/MS: 586 (M+H).

### Example 338 (Synthetic Method AG): Preparation of 6-[2-tert-butyl-6-hydroxy-5-(1-methylpyrazol-4-yl)benzimidazol-1-yl]-4-(3-hydroxy-3-methyl-azetidin-1-yl)-1,3-dimethyl-benzimidazol-2-one

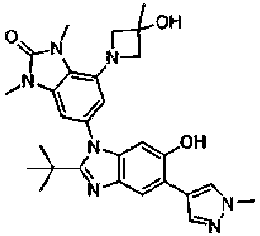

To a solution of 6-[6-benzyloxy-2-tert-butyl-5-(1-methylpyrazol-4-yl)benzimidazol-1-yl]-4-(3-hydroxy-3-methyl-azetidin-1-yl)-1,3-dimethyl-benzimidazol-2-one (Example 337, 43 mg, 0.071 mmol) dissolved in methanol (0.355 mL), palladium/carbon (10 wt%, 7.6 mg, 7.10 µmol) was added. The reactants were subjected to hydrogen/vacuum exchange three times to sufficiently fill the inside of a reaction vessel with hydrogen, and stirred for 4 hours at room temperature in the presence of a hydrogen balloon. The reaction mixture was passed through celite and filtered while eluting with dichloromethane. The filtrate was concentrated under reduced pressure, and the residue was purified by PTLC to obtain the title compound. (31 mg, 0.060 mmol, 85% yield)
¹H-NMR (500 MHz, Chloroform-d) δ 7.74 (s, 1H), 7.73 (s, 1H), 7.66 (s, 1H), 6.64 (d, J = 1.8 Hz, 1H), 6.59 (d, J = 1.8 Hz, 1H), 6.36 (s, 1H), 3.98 (s, 3H), 3.81-3.73 (m, 7H), 3.37 (s, 3H), 1.67 (s, 3H), 1.36 (s, 9H); LC/MS: 516 (M+H).

### Example 339: Preparation of [2-tert-butyl-3-[7-(3-hydroxy-3-methyl-azetidin-1-yl)-1,3-dimethyl-2-oxo-benzimidazol-5-yl]-6-(1-methylpyrazol-4-yl)benzimidazol-5-yl] acetate

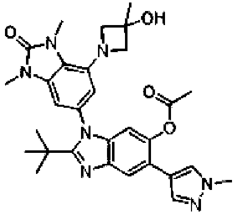

To a solution of 6-[2-tert-butyl-6-hydroxy-5-(1-methylpyrazol-4-yl)benzimidazol-1-yl]-4-(3-hydroxy-3-methyl-azetidin-1-yl)-1,3-dimethyl-benzimidazol-2-one (Example 338, 16 mg, 0.031 mmol) dissolved in dichloromethane (0.155 mL), pyridine (5.02 µL, 0.062 mmol) was added. The temperature of the reaction mixture was lowered to 0°C, acetyl chloride (3.31 µL, 0.047 mmol) was added, and stirring was performed at room temperature for 18 hours. Water was added to the reaction mixture to terminate the reaction, and the resultant was extracted with ethyl acetate. The obtained organic layer was washed with brine, dried over anhydrous sodium sulfate and concentrated under reduced pressure, and the residue was purified by MPLC to obtain the title compound. (7.7 mg, 0.014 mmol, 44.5% yield)

¹H-NMR (500 MHz, Chloroform-d) δ 7.84 (s, 1H), 7.70 (s, 1H), 7.53 (s, 1H), 6.68 (d, J = 1.8 Hz, 1H), 6.61 (d, J = 1.8 Hz, 1H), 6.58 (s, 1H), 3.95 (s, 3H), 3.82-3.73 (m, 7H), 3.39 (s, 3H), 2.20 (s, 3H), 2.06 (s, 1H), 1.68 (s, 3H), 1.38 (s, 9H); LC/MS: 558 (M+H).

### Example 342: Preparation of 6-[2-tert-butyl-6-fluoro-5-(2-methyloxazol-5-yl)benzimidazol-1-yl]-1,3-dimethyl-4-morpholino-benzimidazol-2-one

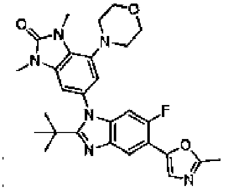

2-(Tert-butyl)-5-ethynyl-6-fluoro-1',3'-dimethyl-7'-morpholino-1',3'-dihydro-2'H-[1,5'-bibenzo[d]imidazol]-2'-one (a compound of Step 3, synthesized in Example 315, 33 mg, 0.071 mmol) was dissolved in acetonitrile (0.8 mL), and then triphenylphosphingold(I) bis(trifluoromethanesulfonyl)imidate (14.80 mg, 0.093 mmol) was added. The reaction solution was stirred at 60°C for 18 hours. After the reaction was completed, the solvent was removed by concentrating under reduced pressure, and the obtained residue was purified by PTLC to obtain the title compound. (18.2 mg, 0.035 mmol, 49.1% yield)
¹H-NMR (500 MHz, Chloroform-d) δ 8.11 (d, J = 6.4 Hz, 1H), 7.27 (s, 1H), 6.88 (d, J = 1.8 Hz, 1H), 6.78 (d, J = 1.8 Hz, 1H), 6.56 (d, J = 10.4 Hz, 1H), 3.90 (s, 4H), 3.82 (s, 3H), 3.42 (s, 3H), 3.00-2.98 (m, 4H), 2.55 (s, 3H), 1.38 (s, 9H); LC/MS: 519 (M+H).

### Examples 345 and 346: Preparation of 6-[2-tert-butyl-6-(difluoromethyl)-5-(1-vinylpyrazol-4-yl)benzimidazol-1-yl]-4-[3-(dimethylamino)-3-methyl-azetidin-1-yl]-1,3-dimethyl-benzimidazol-2-one and 6-[2-tert-butyl-6-(difluoromethyl)-5-[1-[2-(dimethylamino)ethyl]pyrazol-4-yl]benzimidazol-1-yl]-4-[3-(dimethylamino)-3-methyl-azetidin-1-yl]-1,3-dimethyl-benzimidazol-2-one

### Step 1: Preparation of 1-(2-(tert-butyl)-6-(difluoromethyl)-1',3'-dimethyl-5-(1-(2-((methylsulfonyl)oxy)ethyl)-1H-pyrazol-4-yl)-2'-oxo-2',3'-dihydro-1'H-[1,5'-bibenzo[d]imidazol]-7'-yl)-3-methylazetidin-3-yl methanesulfonate

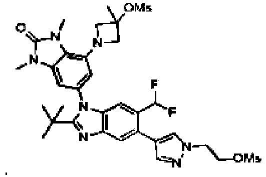

After dissolving 6-[2-tert-butyl-6-(difluoromethyl)-5-[1-(2-hydroxyethyl)pyrazol-4-yl]benzimidazol-1-yl]-4-(3-hydroxy-3-methyl-azetidin-1-yl)-1,3-dimethyl-benzimidazol-2-one (Example 313, 42.4 mg, 0.073 mmol) in dichloromethane (0.8 mL), methanesulfonyl chloride (0.0165 mL, 0.219 mmol) and triethylamine (0.051 mL, 0.366 mmol) were added at 0°C. The reaction solution was stirred at room temperature for 1 hour. After the reaction was completed, water and dichloromethane were added, and an organic layer was extracted. The organic layer was washed with water and brine, dried over anhydrous magnesium sulfate and concentrated under reduced pressure, and the residue was purified by MPLC to obtain the title compound. (54.2 mg, 0.074 mmol, 100% yield)
¹H-NMR (400 MHz, Chloroform-d) δ 7.75 (s, 1H), 7.66 (s, 1H), 7.60 (s, 1H), 7.19 (s, 1H), 6.79-6.51 (m, 3H), 4.63 (t, J = 4.8 Hz, 2H), 4.50 (t, J = 4.8 Hz, 2H), 4.16-4.11 (m, 2H), 3.93-3.87 (m, 2H), 3.75 (s, 3H), 3.39 (s, 3H), 3.04 (s, 3H), 2.87 (s, 3H), 1.99 (s, 3H), 1.37 (s, 9H); LC/MS: 735 (M+H).

### Step 2: Preparation of 6-[2-tert-butyl-6-(difluoromethyl)-5-(1-vinylpyrazol-4-yl)benzimidazol-1-yl]-4-[3-(dimethylamino)-3-methyl-azetidin-1-yl]-1,3-dimethyl-benzimidazol-2-one and 6-[2-tert-butyl-6-(difluoromethyl)-5-[1-[2-(dimethylamino)ethyl]pyrazol-4-yl]benzimidazol-1-yl]-4-[3-(dimethylamino)-3-methyl-azetidin-1-yl]-1,3-dimethyl-benzimidazol-2-one

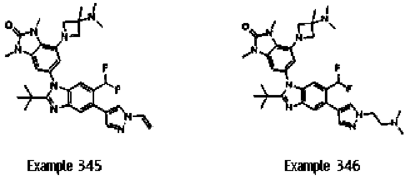

After dissolving 1-(2-(tert-butyl)-6-(difluoromethyl)-1',3'-dimethyl-5-(1-(2-((methylsulfonyl)oxy)ethyl)-1H-pyrazol-4-yl)-2'-oxo-2',3'-dihydro-1'H-[1,5'-bibenzo[d]imidazol]-7'-yl)-3-methylazetidin-3-yl methanesulfonate (27 mg, 0.037 mmol) obtained in Step 1 in acetonitrile (0.8 mL), dimethylamine (0.022 mL, 0.044 mmol) and cesium carbonate (17.93 mg, 0.055 mmol) were added. The reaction solution was stirred at 80°C for 18 hours. After the reaction was completed, water was added, and an organic layer was extracted with ethyl acetate. The organic layer was washed with water and brine, dried over anhydrous magnesium sulfate and concentrated under reduced pressure, and the residue was purified by MPLC and PTLC to obtain the title compounds, respectively.

Example 345: ¹H-NMR (500 MHz, Chloroform-d) δ 7.81 (s, 1H), 7.75 (s, 1H), 7.72 (s, 1H), 7.22 (s, 1H), 7.10 (dd, J = 15.9, 8.9 Hz, 1H), 6.78-6.56 (m, 3H), 5.60 (d, J = 15.9 Hz, 1H), 4.92 (d, J = 8.9 Hz, 1H), 3.78 (s, 3H), 3.74-3.69 (m, 2H), 3.63-3.57 (m, 2H), 3.40 (s, 3H), 2.15 (s, 6H), 1.43 (s, 3H), 1.39 (s, 9H); LC/MS: 589 (M+H).

Example 346: ¹H-NMR (500 MHz, Chloroform-d) δ 7.79 (s, 1H), 7.63 (s, 1H), 7.61 (s, 1H), 7.20 (s, 1H), 6.79-6.57 (m, 3H), 4.30 (t, J = 6.7 Hz, 2H), 3.77 (s, 3H), 3.75-3.70 (m, 2H), 3.62-3.57 (m, 2H), 3.39 (s, 3H), 2.85 (t, J = 6.6 Hz, 2H), 2.32 (s, 6H), 2.15 (s, 6H), 1.43 (s, 3H), 1.39 (s, 9H); LC/MS: 634 (M+H).

### Example 347: Preparation of [1-[6-[2-tert-butyl-6-(difluoromethyl)-5-[1-(2-morpholinoethyl)pyrazol-4-yl]benzimidazol-1-yl]-1,3-dimethyl-2-oxo-benzimidazol-4-yl]-3-methyl-azetidin-3-yl] methanesulfonate

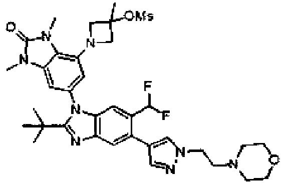

1-(2-(Tert-butyl)-6-(difluoromethyl)-1',3'-dimethyl-5-(1-(2-((methylsulfonyl)oxy)ethyl)-1H-pyrazol-4-yl)-2'-oxo-2',3'-dihydro-1'H-[1,5'-bibenzo[d]imidazol]-7'-yl)-3-methylazetidin-3-yl methanesulfonate (compound of Step 1 in Examples 345 and 346, 27 mg, 0.037 mmol) was dissolved in morpholine (0.5 mL, 5.80 mmol) and stirred at room temperature for 18 hours. After the reaction was completed, the solvent was removed by concentration under reduced pressure, water and ethyl acetate were added, and an organic layer was extracted. The organic layer was washed with water and brine, dried over anhydrous magnesium sulfate and concentrated under reduced pressure, and the residue was purified by MPLC and PTLC to obtain the title compound. (16.5 mg, 0.023 mmol, 61.9% yield)
¹H-NMR (500 MHz, Chloroform-d) δ 7.79 (s, 1H), 7.64 (s, 1H), 7.61 (s, 1H), 7.20 (s, 1H), 6.79-6.57 (m, 3H), 4.32 (s, 2H), 4.18-4.13 (m, 2H), 3.91 (dd, J = 22.4, 8.4 Hz, 2H), 3.76 (s, 3H), 3.71 (s, 4H), 3.40 (s, 3H), 3.05 (s, 3H), 2.88 (s, 2H), 2.52 (s, 4H), 2.01 (s, 3H), 1.39 (s, 9H); LC/MS: 727 (M+H).

### Example 348: Preparation of 6-[2-tert-butyl-6-(difluoromethyl)-5-[1-(2-morpholinoethyl)pyrazol-4-yl]benzimidazol-1-yl]-4-(3-hydroxy-3-methyl-azetidin-1-yl)-1,3-dimethyl-benzimidazol-2-one

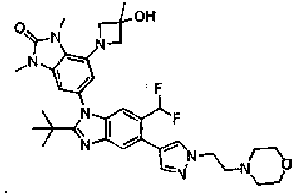

[1-[6-[2-Tert-butyl-6-(difluoromethyl)-5-[1-(2-morpholinoethyl)pyrazol-4-yl]benzimidazol-1-yl]-1,3-dimethyl-2-oxo-benzimidazol-4-yl]-3-methyl-azetidin-3-yl] methanesulfonate (Example 347, 7.4 mg, 10.18 µmol) was dissolved in dimethyl sulfoxide (0.3 mL), and 1 M triethylammonium acetate aqueous solution (0.015 mL, 0.015 mmol) was added, followed by stirring at 60°C for 18 hours. After the reaction was completed, water was added and an organic layer was extracted with ethyl acetate. The organic layer was washed with water and brine, dried over anhydrous magnesium sulfate and concentrated under reduced pressure, and the residue was purified by PTLC to obtain the title compound. (3.5 mg, 5.39 µmol, 53.0% yield)
¹H-NMR (500 MHz, Chloroform-d) δ 7.79 (s, 1H), 7.63 (s, 1H), 7.62 (s, 1H), 7.21 (s, 1H), 6.79-6.57 (m, 3H), 4.31 (t, J = 6.3 Hz, 2H), 3.83-3.76 (m, 7H), 3.70 (t, J = 4.4 Hz, 4H), 3.40 (s, 3H), 2.86 (t, J = 6.3 Hz, 2H), 2.51 (s, 4H), 1.68 (s, 3H), 1.39 (s, 9H); LC/MS: 649 (M+H).

### Example 357: Preparation of 6-[6-amino-2-tert-butyl-5-(1-methylpyrazol-4-yl)benzimidazol-1-yl]-4-[(2,3-dihydroxy-2-methyl-propyl)amino]-1,3-dimethyl-benzimidazol-2-one

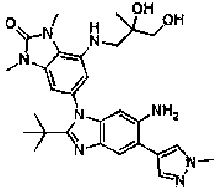

Tert-butyl N-[2-tert-butyl-3-[7-(3-hydroxy-3-methyl-azetidin-1-yl)-1,3-dimethyl-2-oxo-benzimidazol-5-yl]-6-(1-methylpyrazol-4-yl)benzimidazol-5-yl]carbamate (Example 343, 36 mg, 0.059 mmol) and trifluoroacetic acid (0.031 mL, 0.468 mmol) were stirred in a dichloromethane (0.3 mL) solvent for 1 hour. After diluting the reaction solution with dichloromethane (3 mL), a saturated sodium bicarbonate aqueous solution was added dropwisely at 0°C. The reaction solution was extracted with dichloromethane, dried over anhydrous magnesium sulfate, and concentrated under reduced pressure. The residue was purified by MPLC to obtain the title compound. (1 mg, 0.002 mmol, 3.3% yield)
¹H-NMR (500 MHz, Chloroform-d) δ 7.67 (s, 1H), 7.59 (br. s., 1H), 7.54 (s, 1H), 6.50 (s, 1H), 6.40 (s, 1H), 6.17 (d, J = 2.59 Hz, 1H), 5.03 (br. s., 1H), 4.03-3.92 (m, 4H), 3.86-3.79 (m, 3H), 3.77-3.72 (m, 2H), 3.70-3.62 (m, 2H), 3.44-3.34 (m, 4H), 3.21-3.14 (m, 1H), 3.12-3.05 (m, 1H), 1.46-1.35 (m, 9H), 1.30 (d, J = 1.83 Hz, 3H); LC/MS: 533 (M+H).

### Example 359 (Synthetic Method AH): Preparation of 6-[2-tert-butyl-6-(difluoromethyl)-5-(1-methylpyrazol-4-yl)benzimidazol-1-yl]-1,3-dimethyl-4-(4-oxo-1-piperidyl)benzimidazol-2-one

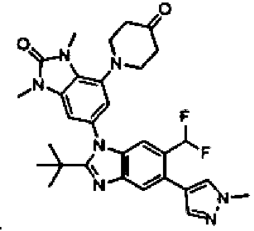

6-[2-Tert-butyl-6-(difluoromethyl)-5-(1-methylpyrazol-4-yl)benzimidazol-1-yl]-4-(1,4-dioxa-8-azaspiro[4.5]decan-8-yl)-1,3-dimethyl-benzimidazol-2-one (Example 361, 220 mg, 0.363 mmol) was dissolved in tetrahydrofuran (1.8 mL) and then 1 M hydrochloric acid (2.54 mL, 2.54 mmol) was added to perform a reaction at room temperature for 18 hours. The reaction solution was neutralized with an aqueous sodium hydroxide solution, then diluted with ethyl acetate, washed with water, dried over anhydrous magnesium sulfate, and concentrated under reduced pressure, and the residue was purified by MPLC to obtain the title compound. (141 mg, 0.251 mmol, 69.1% yield)
¹H-NMR (500 MHz, Chloroform-d) δ 7.80 (s, 1H), 7.61 (s, 1H), 7.49 (s, 1H), 7.18 (s, 1H), 6.92 (d, J = 1.68 Hz, 1H), 6.84 (d, J = 1.68 Hz, 1H), 6.68 (t, 1H), 4.00 (s, 3H), 3.96-3.88 (m, 3H), 3.54 (br. s., 1H), 3.50 (br. s., 1H), 3.48-3.40 (m, 3H), 3.14 (br. s., 2H), 2.84 (br. s., 2H), 2.53 (br. s., 2H), 1.48-1.32 (m, 9H); LC/MS: 562 (M+H).

### Example 362 (Synthetic Method AI): Preparation of 6-[2-tert-butyl-6-(difluoromethyl)-5-(1-methylpyrazol-4-yl)benzimidazol-1-yl]-4-(4-hydroxy-1-piperidyl)-1,3-dimethyl-benzimidazol-2-one

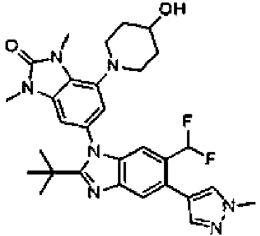

6-[2-Tert-butyl-6-(difluoromethyl)-5-(1-methylpyrazol-4-yl)benzimidazol-1-yl]-1,3-dimethyl-4-(4-oxo-1-piperidyl)benzimidazol-2-one (Example 359, 22 mg, 0.039 mmol) was stirred in an ethanol (0.2 mL) solvent, and sodium borohydride (2.67 mg, 0.071 mmol) was added at 0°C, followed by stirring for 2 hours. The reaction was terminated using a saturated ammonium chloride aqueous solution, and the reaction solution was diluted with ethyl acetate, washed with water, dried over anhydrous magnesium sulfate, and concentrated under reduced pressure. The residue was purified by MPLC to obtain the title compound. (9.7 mg, 0.017 mmol, 43.9% yield)
¹H-NMR (500 MHz, Chloroform-d) δ 7.79 (s, 1H), 7.61 (s, 1H), 7.49 (s, 1H), 7.19 (s, 1H), 6.77 (d, J = 1.68 Hz, 1H), 6.68 (t, 1H), 4.00 (s, 3H), 3.84 (s, 3H), 3.75 (br. s., 1H), 3.42 (s, 3H), 3.19 (br. s., 2H), 2.75 (br. s., 2H), 2.12-2.06 (m, 2H), 1.87-1.76 (m, 3H), 1.72-1.64 (m, 1H); LC/MS: 564 (M+H).

### Example 366: Preparation of 4-[[[6-[2-tert-butyl-6-(difluoromethyl)-5-(1-methylpyrazol-4-yl)benzimidazol-1-yl]-1,3-dimethyl-2-oxo-benzimidazol-4-yl]amino]methyl]-4-methyl-oxazolidin-2-one

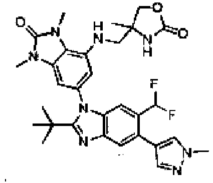

Tert-butyl N-[1-[6-[2-tert-butyl-6-(difluoromethyl)-5-(1-methylpyrazol-4-yl)benzimidazol-1-yl]-1,3-dimethyl-2-oxo-benzimidazol-4-yl]-3-methyl-azetidin-3-yl]carbamate (Example 364, 28 mg, 0.043 mmol) was dissolved in dichloromethane (0.3 mL), and trifluoroacetic acid (33 µL, 0.432 mmol) was added. The reaction solution was stirred at room temperature for 4 hours. After the reaction was completed, 1 N aqueous sodium hydroxide solution and ethyl acetate were added, and an organic layer was extracted. The organic layer was washed with water and brine, dried over anhydrous magnesium sulfate and concentrated under reduced pressure, and the residue was purified by MPLC and PTLC to obtain the title compound. (16.3 mg, 0.028 mmol, 63.7% yield)
¹H-NMR (500 MHz, Chloroform-d) δ 7.78 (s, 1H), 7.60 (s, 1H), 7.48 (s, 1H), 7.19 (d, J = 7.6 Hz, 1H), 6.68-6.56 (m, 2H), 6.41 (s, 1H), 5.35 (d, J = 11.9 Hz, 1H), 4.52-4.45 (m, 1H), 4.33-4.32 (m, 1H), 4.17 (d, J = 8.9 Hz, 1H), 3.99 (s, 3H), 3.81 (s, 3H), 3.40 (s, 3H), 3.19-3.08 (m, 2H), 1.47 (s, 3H), 1.41 (s, 9H); LC/MS: 593 (M+H).

### Example 375 (Synthetic Method AJ): Preparation of 6-[2-tert-butyl-6-(difluoromethyl)-5-(1-methylpyrazol-4-yl)benzimidazol-1-yl]-4-(4-hydroxy-4-methyl-1-piperidyl)-1,3-dimethyl-benzimidazol-2-one

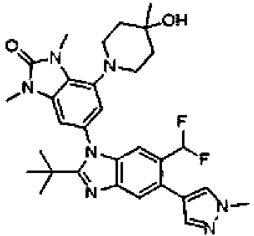

6-[2-Tert-butyl-6-(difluoromethyl)-5-(1-methylpyrazol-4-yl)benzimidazol-1-yl]-1,3-dimethyl-4-(4-oxo-1-piperidyl)benzimidazol-2-one (Example 359, 30 mg, 0.053 mmol) was stirred at 0°C in a tetrahydrofuran (0.4 mL) solvent, and at the same temperature, methylmagnesium bromide (3 M in diethyl ether, 0.036 mL, 0.107 mmol) was added dropwisely, followed by stirring at room temperature for 1 hour. The reaction was quenched with water, and an organic layer was extracted with dichloromethane, dried over anhydrous magnesium sulfate and concentrated under reduced pressure. The residue was purified by MPLC to obtain the title compound. (11.7 mg, 0.020 mmol, 37.9% yield)

¹H-NMR (500 MHz, Chloroform-d) δ 7.79 (s, 1H), 7.62 (s, 1H), 7.49 (s, 1H), 7.20 (s, 1H), 6.93 (br. s., 1H), 6.76 (d, J = 1.68 Hz, 1H), 6.68 (t, 1H), 4.00 (s, 3H), 3.83 (s, 3H), 3.42 (s, 3H), 3.03 (br. s., 4H), 1.89 (br. s., 2H), 1.72 (br. s., 2H), 1.40 (s, 9H), 1.35 (s, 3H); LC/MS: 578 (M+H).

### Example 384: Preparation of 6-[5-(3,6-dihydro-2H-pyran-4-yl)-6-fluoro-2-(trifluoromethyl)benzimidazol-1-yl]-1,3-dimethyl-4-tetrahydropyran-4-yl-benzimidazol-2-one

### Step 1: Preparation of 6-((2-amino-4-(3,6-dihydro-2H-pyran-4-yl)-5-fluorophenyl)amino)-1,3-dimethyl-4-(tetrahydro-2H-pyran-4-yl)-1,3-dihydro-2H-benzo[d]imidazol-2-one

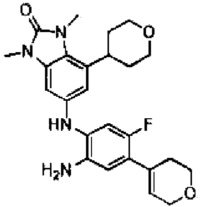

The title compound was obtained in a similar manner to Step 1 and Step 2 of Synthetic Method C using 6-amino-1,3-dimethyl-4-(tetrahydro-2H-pyran-4-yl)-1,3-dihydro-2H-benzo[d]imidazol-2-one (Intermediate I-4, 340 mg, 1.301 mmol) and 1-bromo-2,4-difluoro-5-nitrobenzene (310 mg, 1.301 mmol). (46.0% yield over 2 steps)
¹H-NMR (500 MHz, Chloroform-d) δ 6.76 (d, J = 12.5 Hz, 1H), 6.69 (d, J = 7.6 Hz, 1H), 6.59 (d, J = 1.8 Hz, 1H), 6.45 (d, J = 1.8 Hz, 1H), 5.99 (s, 1H), 5.37 (s, 1H), 4.31 (q, J = 2.7 Hz, 2H), 4.18 (q, J = 2.8 Hz, 1H), 3.90 (t, J = 5.3 Hz, 2H), 3.74 (t, J = 5.3 Hz, 1H), 3.65 (s, 3H), 3.57-3.53 (m, 3H), 3.34 (s, 3H), 2.49-2.49 (m, 2H), 2.23-2.20 (m, 1H), 1.92-1.84 (m, 2H), 1.78-1.75 (m, 2H); LC/MS: 453 (M+H).

### Step 2: Preparation of 6-[5-(3,6-dihydro-2H-pyran-4-yl)-6-fluoro-2-(trifluoromethyl)benzimidazol-1-yl]-1,3-dimethyl-4-tetrahydropyran-4-yl-benzimidazol-2-one

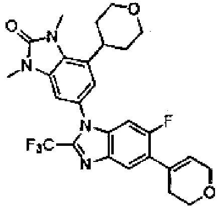

6-((2-Amino-4-(3,6-dihydro-2H-pyran-4-yl)-5-fluorophenyl)amino)-1,3-dimethyl-4-(tetrahydro-2H-pyran-4-yl)-1,3-dihydro-2H-benzo[d]imidazol-2-one (87 mg, 0.192 mmol) obtained in Step 1 was dissolved in trifluoroacetic acid (1 mL). The reaction solution was stirred at 100°C for 3 hours. After the reaction was completed, the reaction solution was diluted with water, 1 N sodium hydroxide aqueous solution was added, and an organic layer was extracted with ethyl acetate. The organic layer was washed with water and brine, dried over anhydrous magnesium sulfate and concentrated under reduced pressure, and the residue was purified by MPLC and PTLC to obtain the title compound. (8 mg, 0.015 mmol, 7.84% yield)
¹H-NMR (500 MHz, Chloroform-d) δ 7.80 (d, J = 6.7 Hz, 1H), 7.04 (d, J = 1.8 Hz, 1H), 6.88 (d, J = 1.8 Hz, 1H), 6.83 (d, J = 10.4 Hz, 1H), 6.05 (s, 1H), 4.36-4.34 (m, 2H), 4.13-4.11 (m, 2H), 3.97-3.95 (m, 2H), 3.77 (s, 3H), 3.60-3.52 (m, 3H), 3.44 (s, 3H), 2.55-2.55 (m, 2H), 1.93-1.82 (m, 4H); LC/MS: 531 (M+H).

### Example 391: Preparation of 6-[2-tert-butyl-6-(difluoromethyl)-5-(1-methylpyrazol-4-yl)benzimidazol-1-yl]-4-[(2,3-dihydroxy-2-methylpropyl)amino]-1,3-dimethyl-benzimidazol-2-one

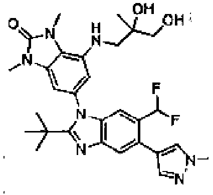

6-[2-Tert-butyl-6-(difluoromethyl)-5-(1-methylpyrazol-4-yl)benzimidazol-1-yl]-4-(3-hydroxy-3-methyl-azetidin-1-yl)-1,3-dimethyl-benzimidazol-2-one (Example 183, 25 mg, 0.045 mmol) was dissolved in 1,4-dioxane (0.7 mL) and then hydrochloric acid (4 M in 1,4-dioxane, 0.017 mL, 0.068 mmol) was added. The reaction solution was stirred at room temperature for 1 hour. After the reaction was completed, 1 N sodium hydroxide (0.227 mL, 0.227 mmol) was added, and stirring was performed at room temperature for 30 minutes. After the reaction was completed, the reaction solution was dissolved in dimethylformamide (0.5 mL), and 1 N sodium hydroxide (0.227 mL, 0.227 mmol) was additionally added, followed by stirring at 100°C for 7 hours. The reaction solution was diluted with water, and an organic layer was extracted with ethyl acetate. The organic layer was washed with water and brine, dried over anhydrous magnesium sulfate and concentrated under reduced pressure, and the residue was purified by PTLC to obtain the title compound. (9 mg, 0.016 mmol, 34.9% yield)
¹H-NMR (500 MHz, Chloroform-d) δ 7.77 (s, 1H), 7.60 (s, 1H), 7.48 (s, 1H), 7.22 (s, 1H), 6.67 (t, J = 55.7 Hz, 1H), 6.51 (s, 1H), 6.40 (s, 1H), 5.05-4.98 (m, 1H), 3.99 (s, 3H), 3.83 (s, 3H), 3.75-3.62 (m, 2H), 3.39 (s, 3H), 3.21-3.05 (m, 2H), 2.54 (d, J = 40.0 Hz, 1H), 2.10-2.07 (m, 1H), 1.41 (s, 9H), 1.29 (d, J = 4.3 Hz, 3H); LC/MS: 568 (M+H).

### Examples 2, 5, 11, 13-25, 27-46, 48, 50-53, 55-57, 61-96, 98, 100-106, 108-115, 117, 119-121, 124, 127, 129, 131-145, 147-177, 179-187, 190, 193-218, 220-242, 245-260, 263-271, 275-287, 289, 291, 293-298, 302-306, 308-310, 312-314, 316, 319, 320, 322-324, 327-330, 334-337, 340-341, 343-344, 349-356, 358, 360, 361, 363-365, 367-374, 376-383, 385-390, and 392-404

Syntheses were performed similarly using the same synthetic methods as the representative examples above.

Specifically, Example 2 was similarly prepared by A, Examples 5, 11, 13 to 15, 21, 23, 24, 27 to 46, 53 and 67 to 74 were similarly prepared by B, Examples 16, 18 and 20 were similarly prepared by C, Example 260 was similarly prepared by D, Example 285 was similarly prepared by E, Examples 17, 19, 22, 105 and 278 were similarly prepared by F, Example 25 was similarly prepared by G, Example 279 was similarly prepared by H, Examples 48, 78 to 80, 83, 84, 86 to 89, 104, 124, 152, 161, 162, 179 to 182, 198, 205 to 208, 214, 215, 218, 230 to 234, 240, 241, 247 to 249, 251 to 254, 263 to 265, 280 to 282, 286, 294, 303 to 305, 312, 313, 316, 322 to 324, 337, 340, 361, 370, 376, 377 and 395 to 399 were similarly prepared by I, Examples 50 to 52 were similarly prepared by J, Examples 55 to 57, 64, 65, 76, 77, 103, 139 and 153 were similarly prepared by K, Examples 63 and 154 were similarly prepared by L, and Examples 61, 62, 85, 92 to 95, 102, 106, 108 to 115, 127, 134 to 138, 140 to 143, 150, 151, 156, 157, 163, 164, 169, 174, 175, 183, 194, 195, 197, 200, 201, 209 to 213, 216, 217, 238, 239, 250, 257 to 259, 266 to 271, 277, 283, 284, 287, 289, 295 to 298, 306, 308, 310, 327 to 330, 343, 350, 354 to 356, 358, 360, 363 to 365 and 400 to 404 were similarly prepared by M, Examples 66, 81, 82, 90, 91, 101, 155 and 173 were similarly prepared by N, Examples 96, 98, 100, 120, 121 and 144 were similarly prepared by O, Examples 117, 119, 148, 149, 160, 176, 177, 190, 204, 349 and 383 were similarly prepared by P, Example 129 was similarly prepared by Q, Examples 131 to 133, 158, 159, 167, 168, 202, 203, 224, 225, 227, 235 to 237, 255 and 256 were similarly prepared by R, Examples 145, 147, 165, 166, 170, 171, 184, 185, 196, 199, 226, 228, 229 and 309 were similarly prepared by S, Examples 75, 187, 352 and 367 were similarly prepared by T, Examples 353 and 368 were similarly prepared by U, Example 369 was similarly prepared by V, Example 193 was similarly prepared by W, Examples 172, 186, 220 to 223, 245 and 246 were similarly prepared by X, Example 242 was similarly prepared by Y, Examples 275 and 276 were similarly prepared by Z, Examples 291, 293 and 314 were similarly prepared by AA, Examples 301 and 302 were similarly prepared by AB, Examples 317 and 344 were similarly prepared by AC, Examples 318 to 320 were similarly prepared by AD, Example 351 was similarly prepared by AE, Example 334 was similarly prepared by AF, Examples 338 and 341 were similarly prepared by AG, Examples 371, 385 and 386 were similarly prepared by AH, Examples 372, 378, 389, 390 and 392 to 394 were similarly prepared by AI, and Examples 335, 336, 373, 374, 379 to 382, 387 and 388 were similarly prepared by AJ.

The structure, name, NMR and synthetic method of the compound synthesized from each of the Examples are listed in Table 2 below.

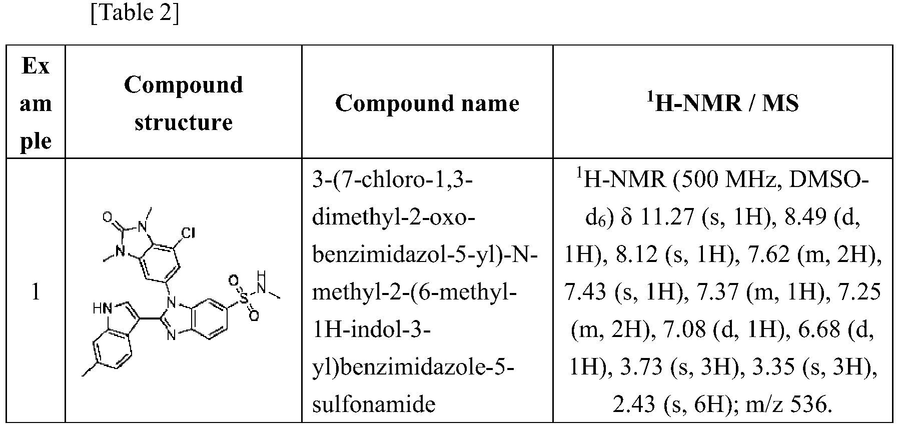

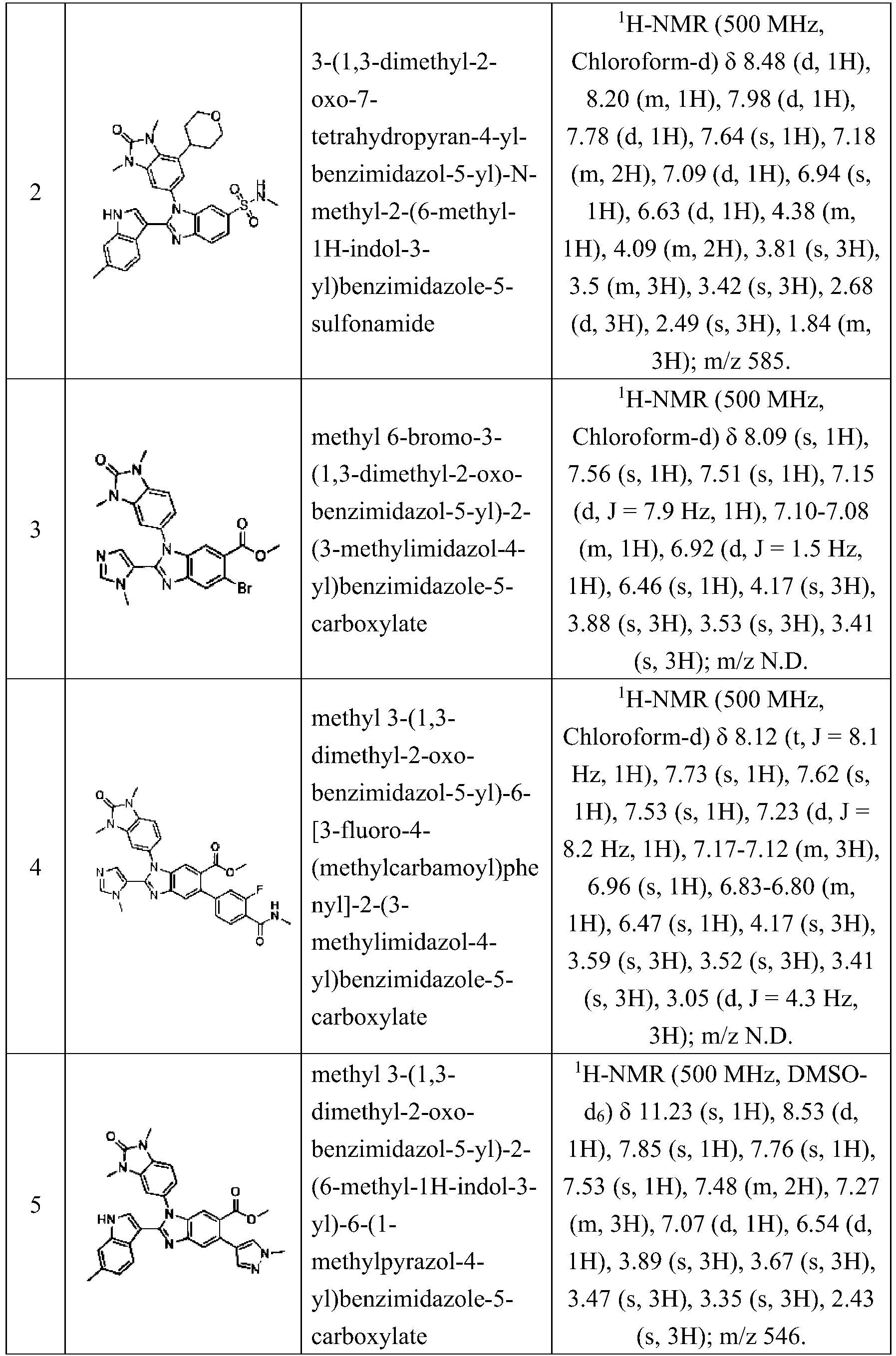

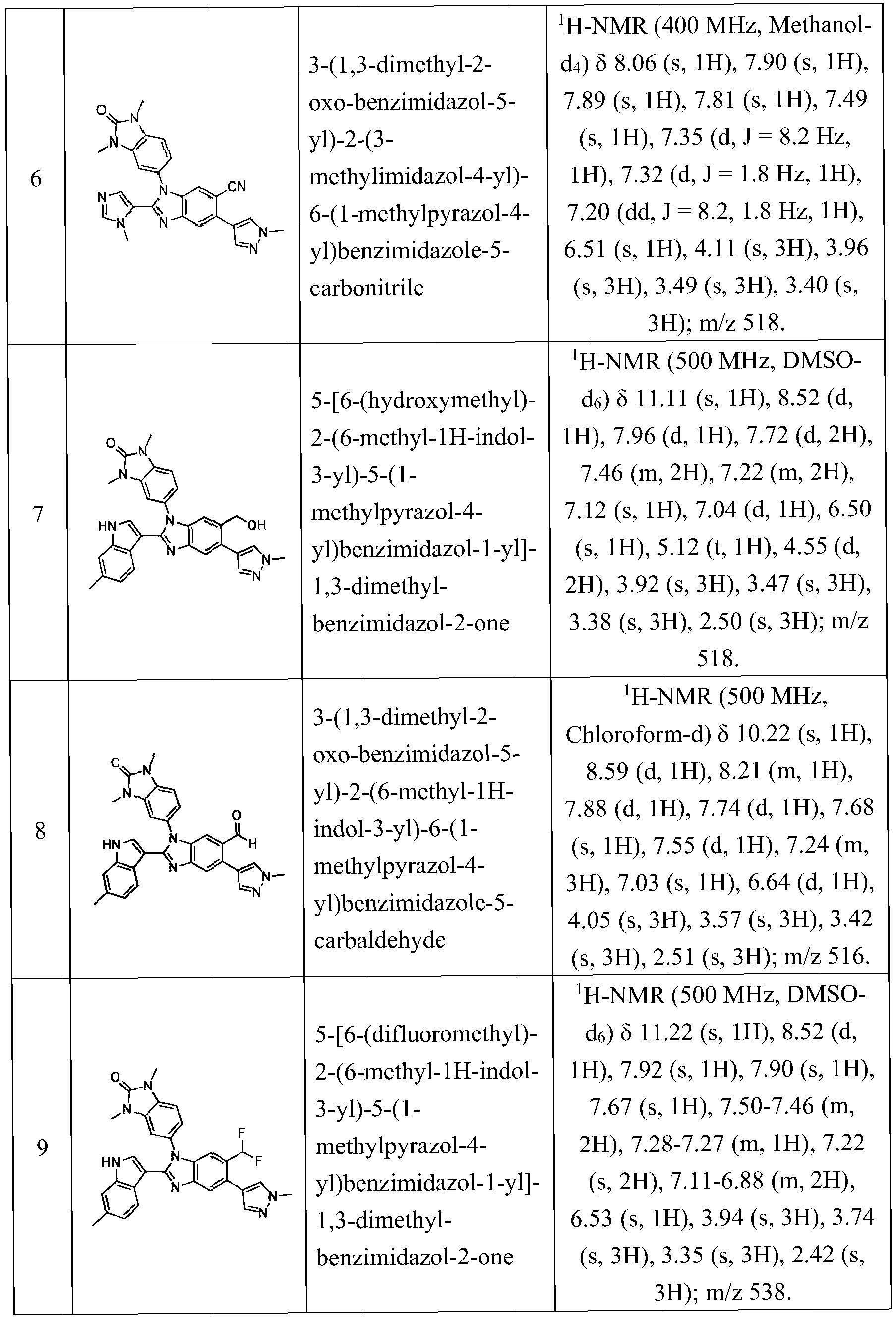

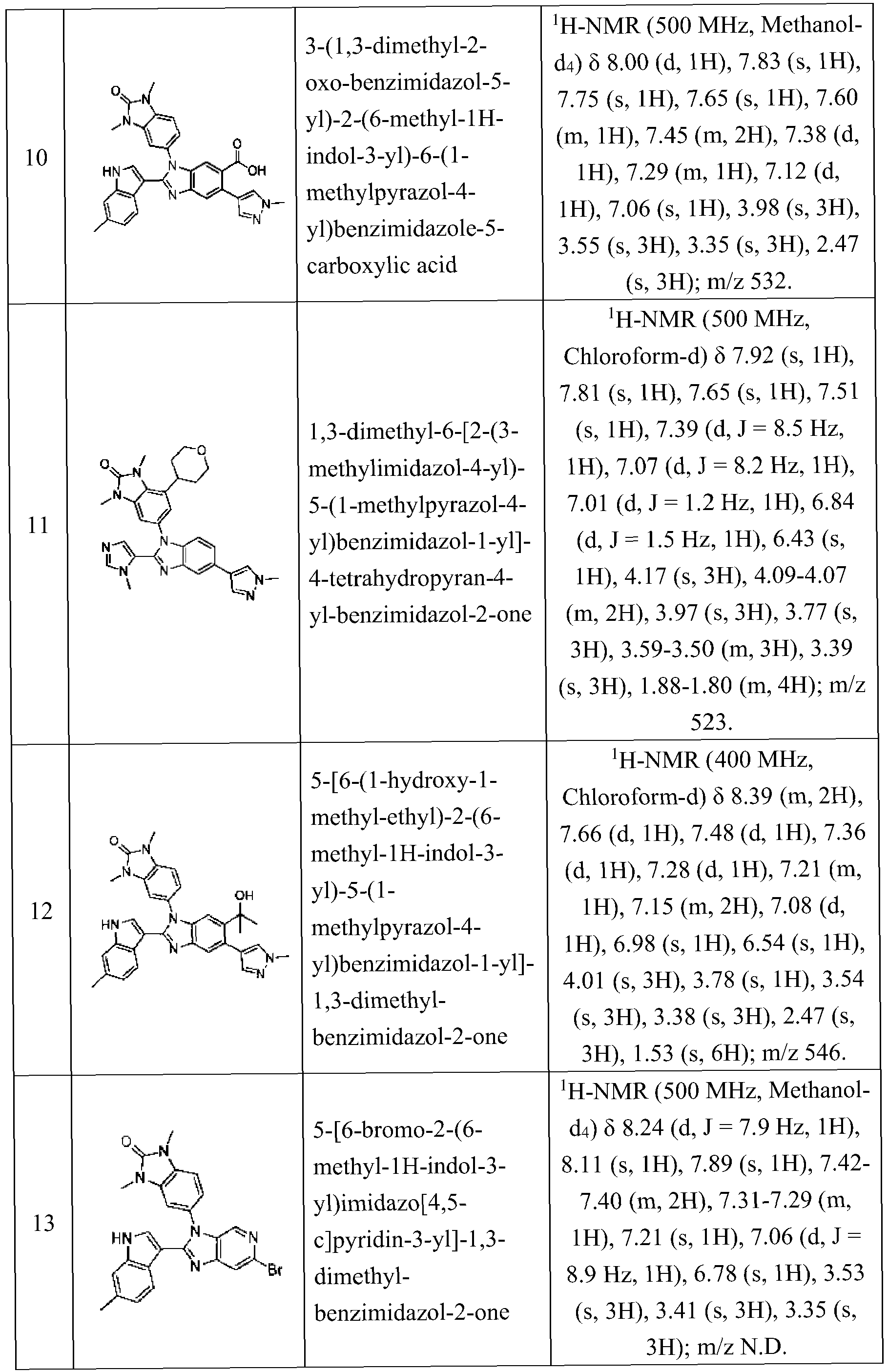

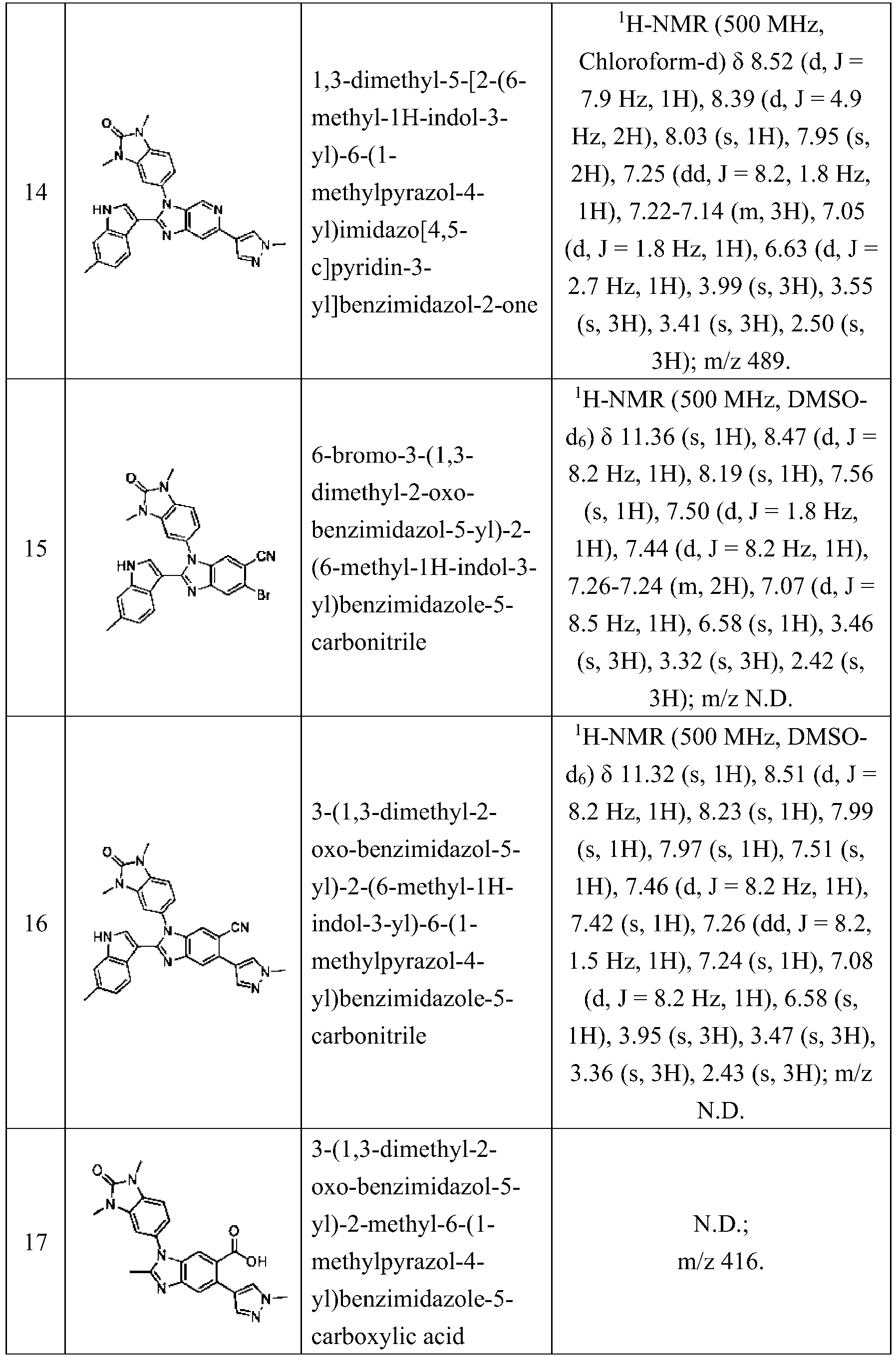

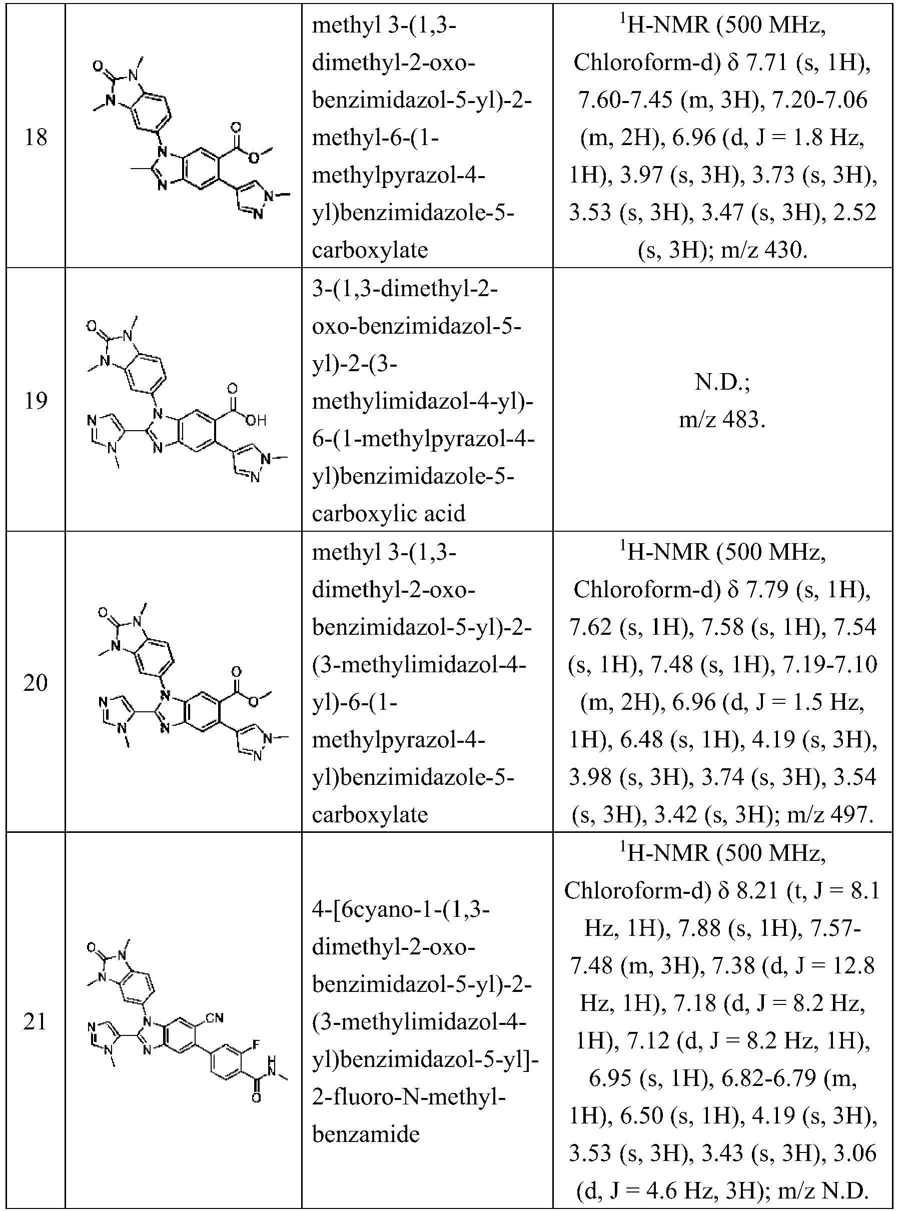

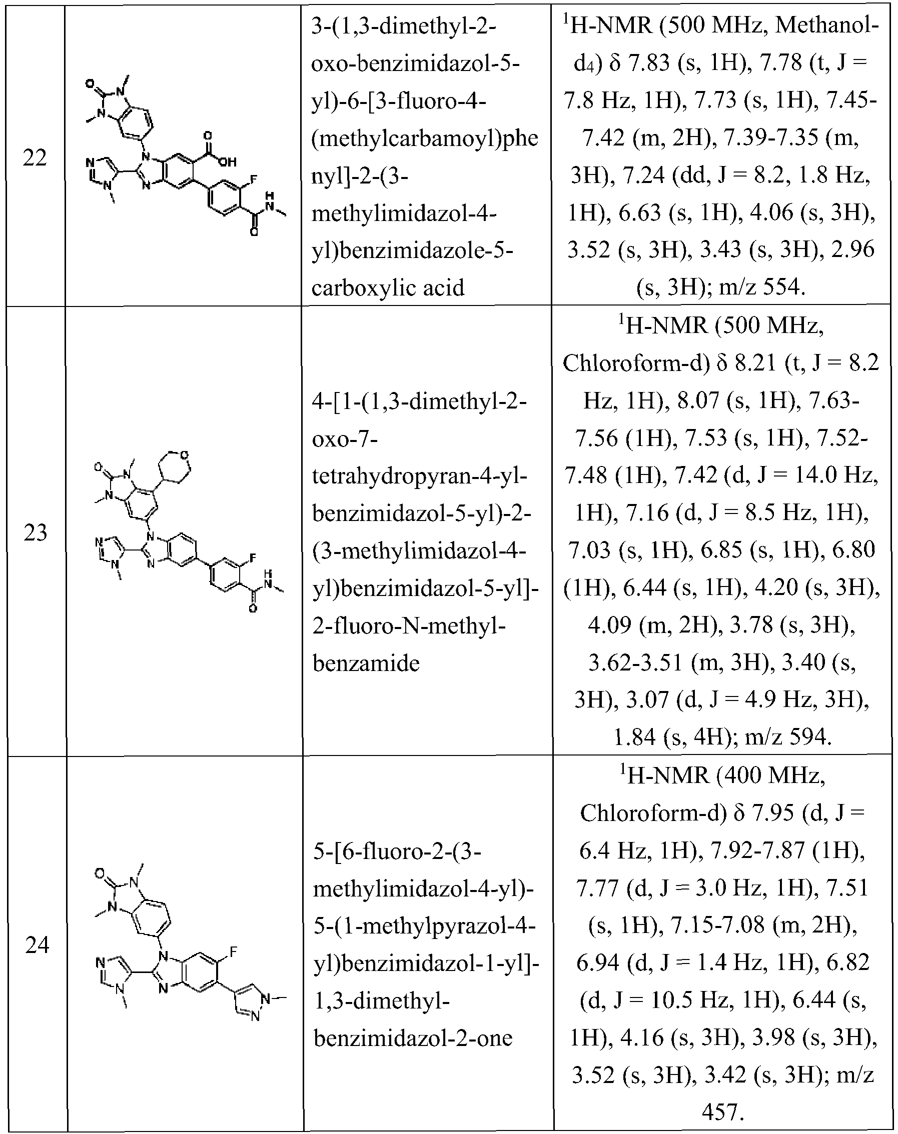

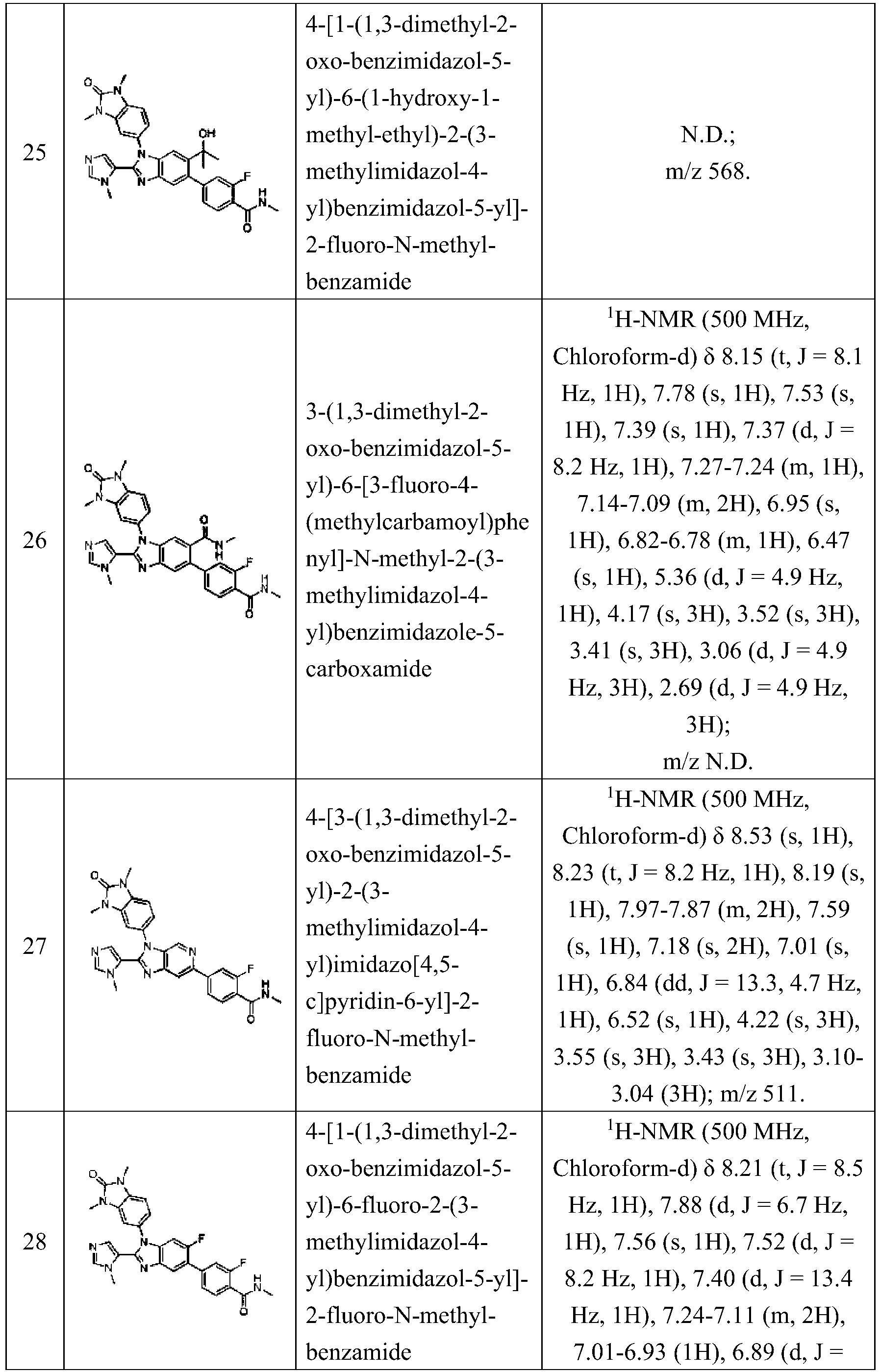

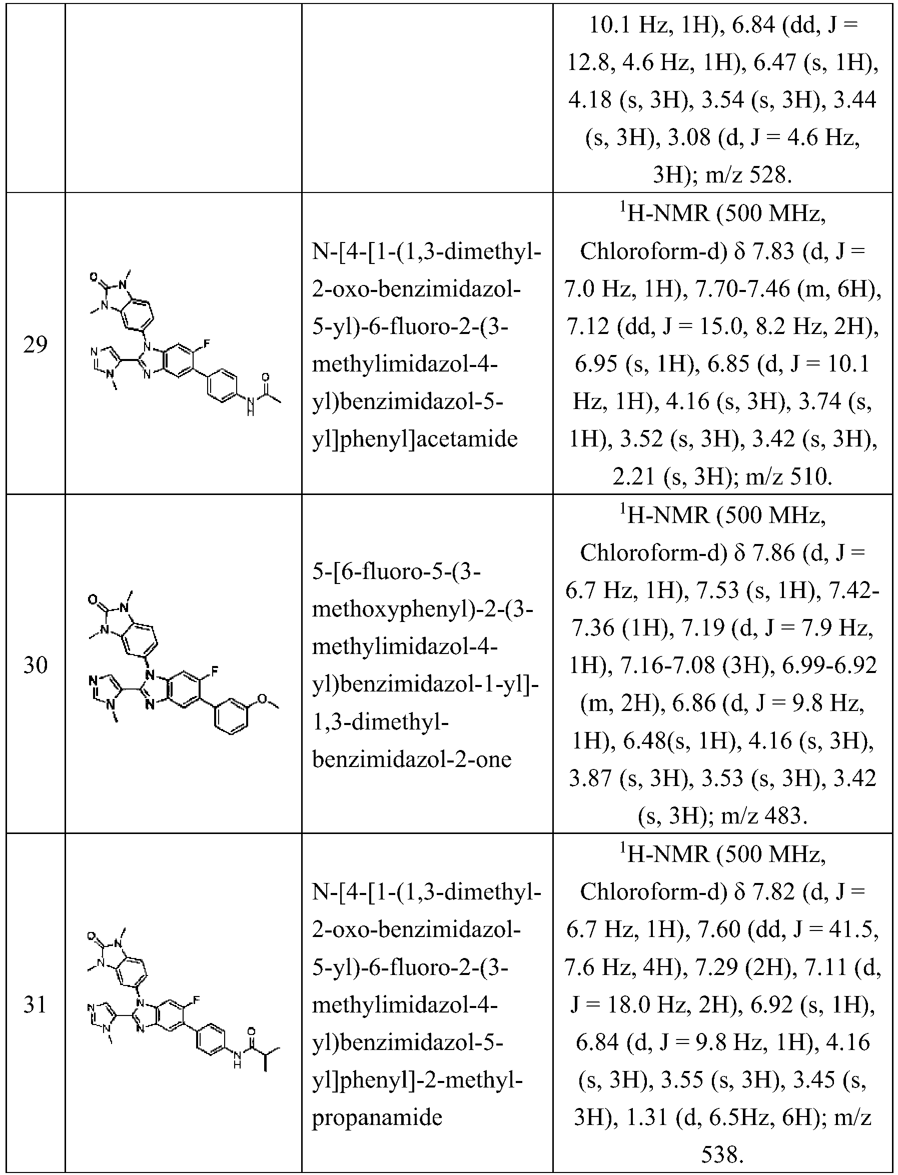

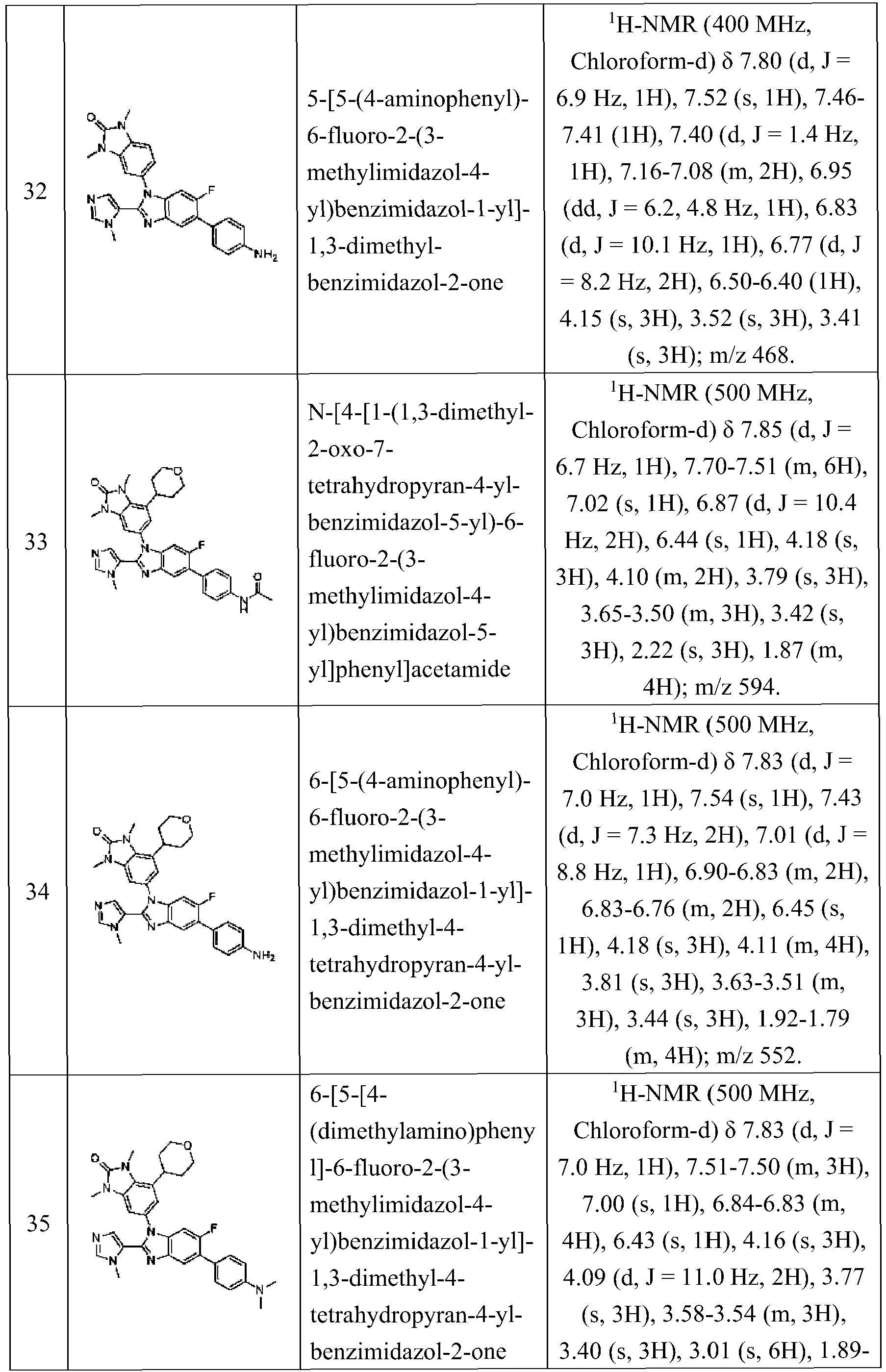

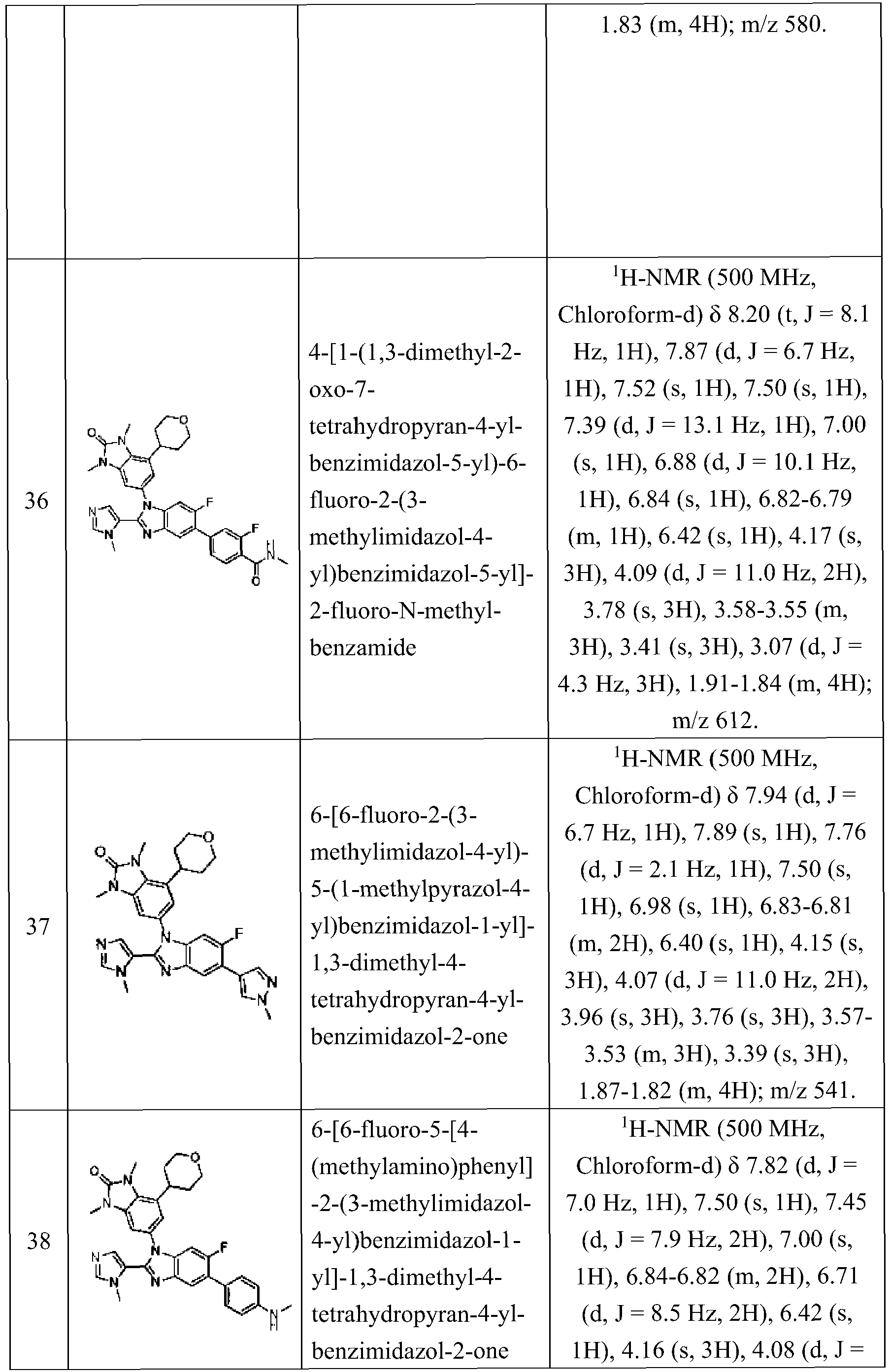

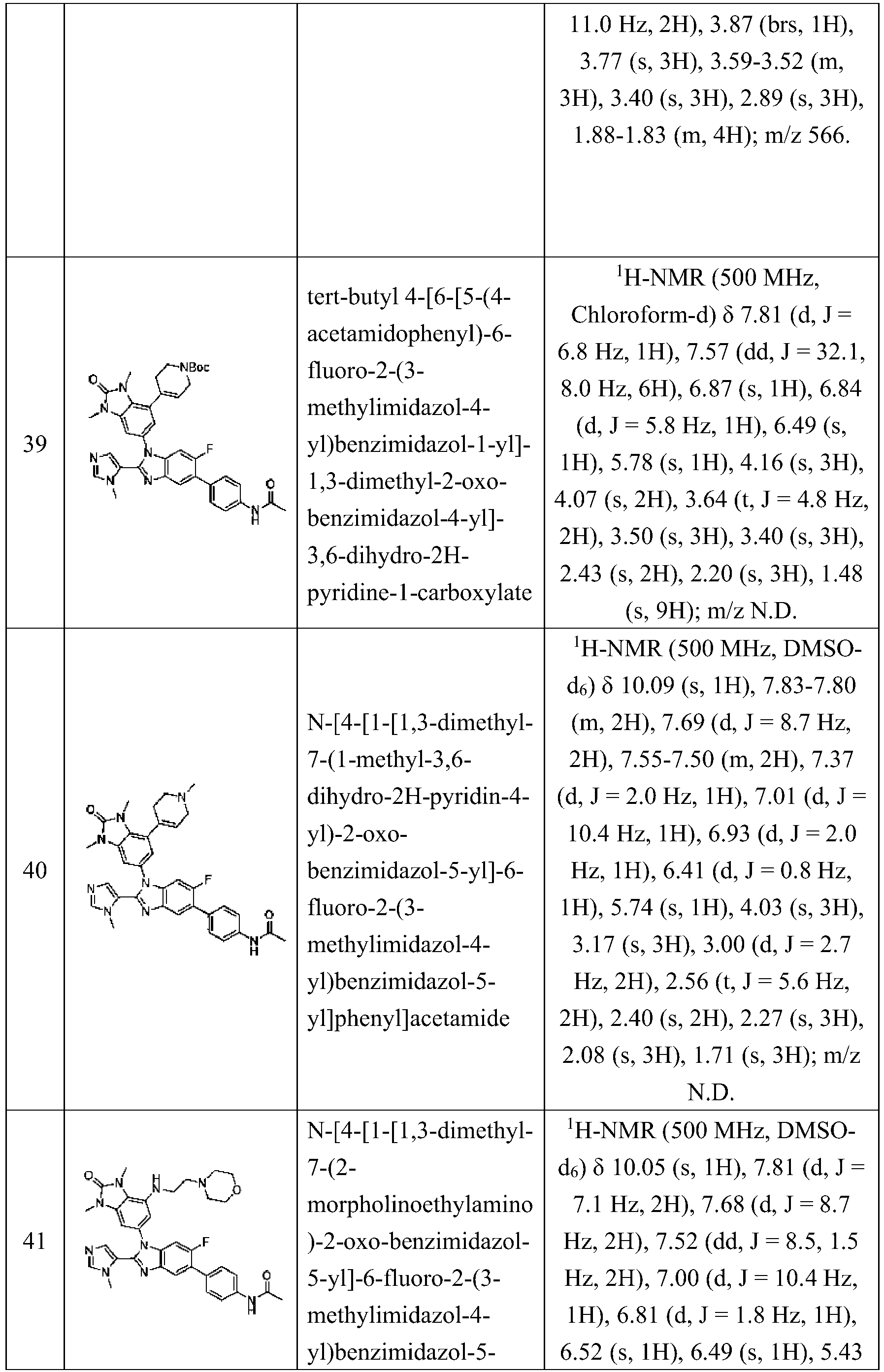

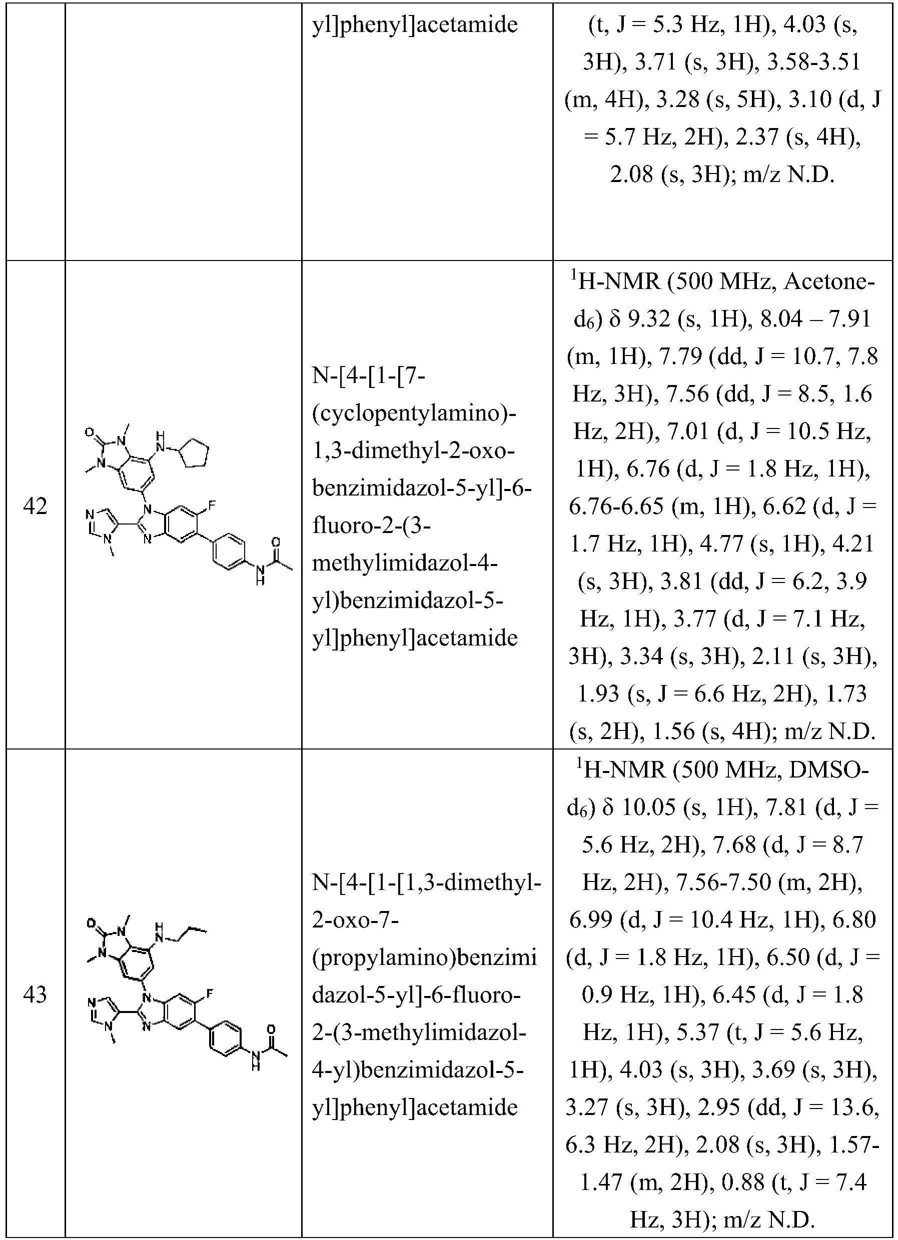

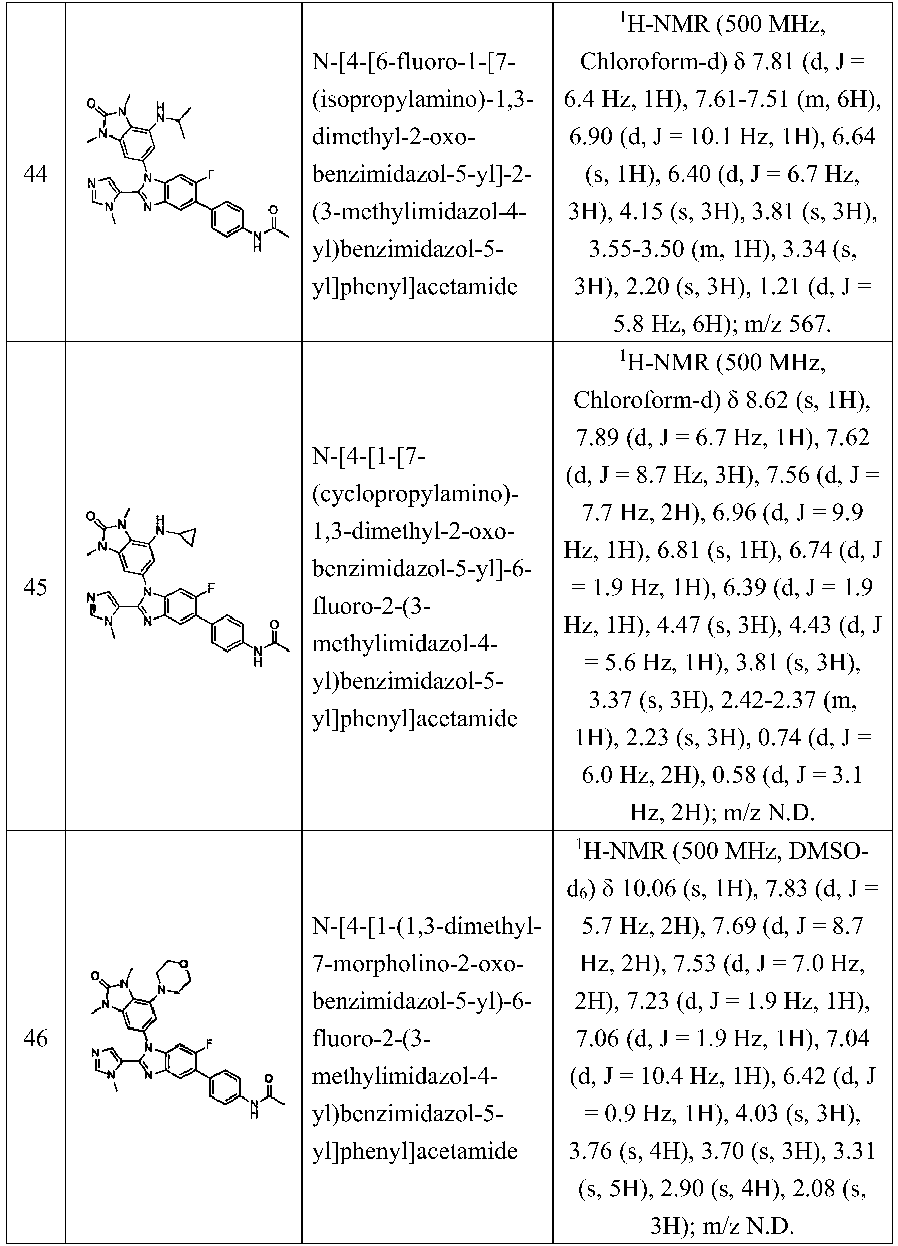

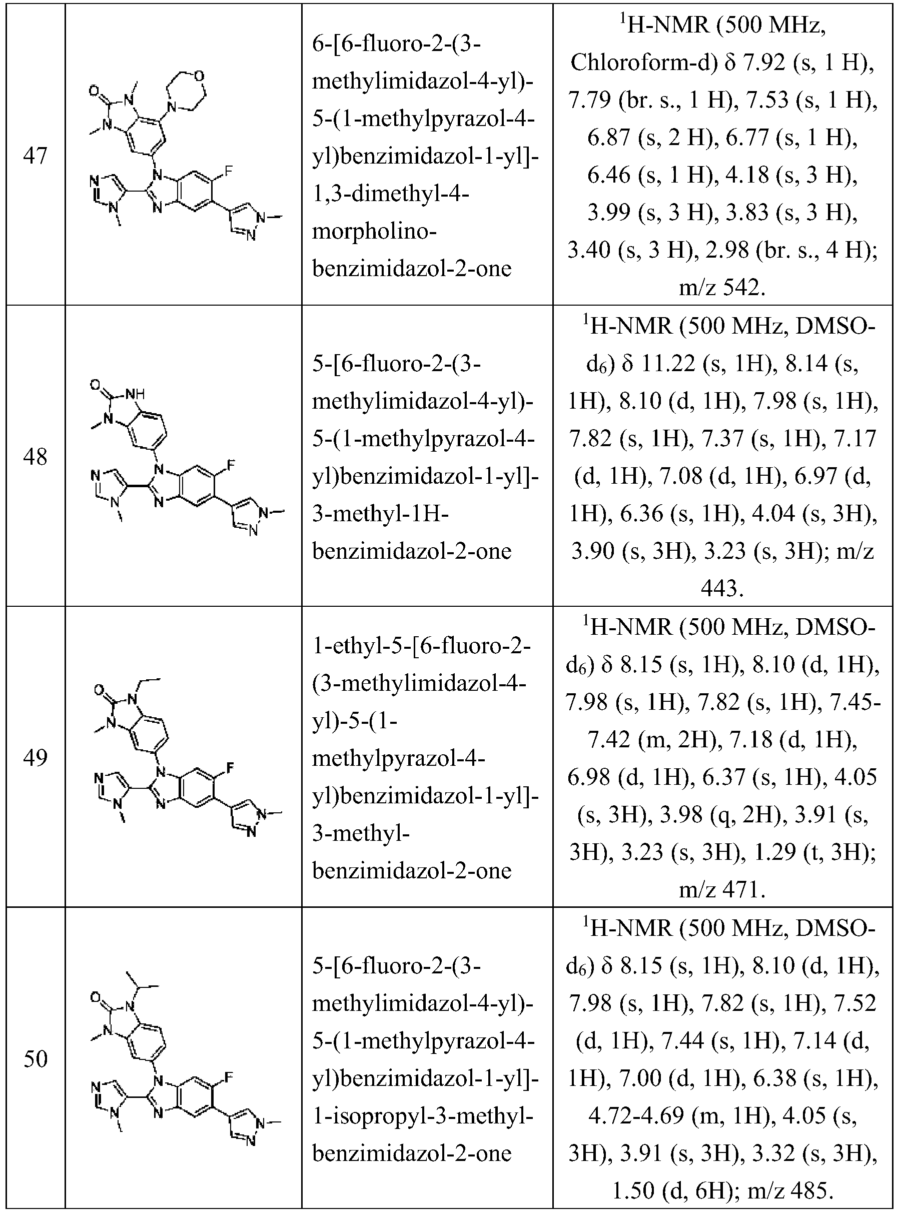

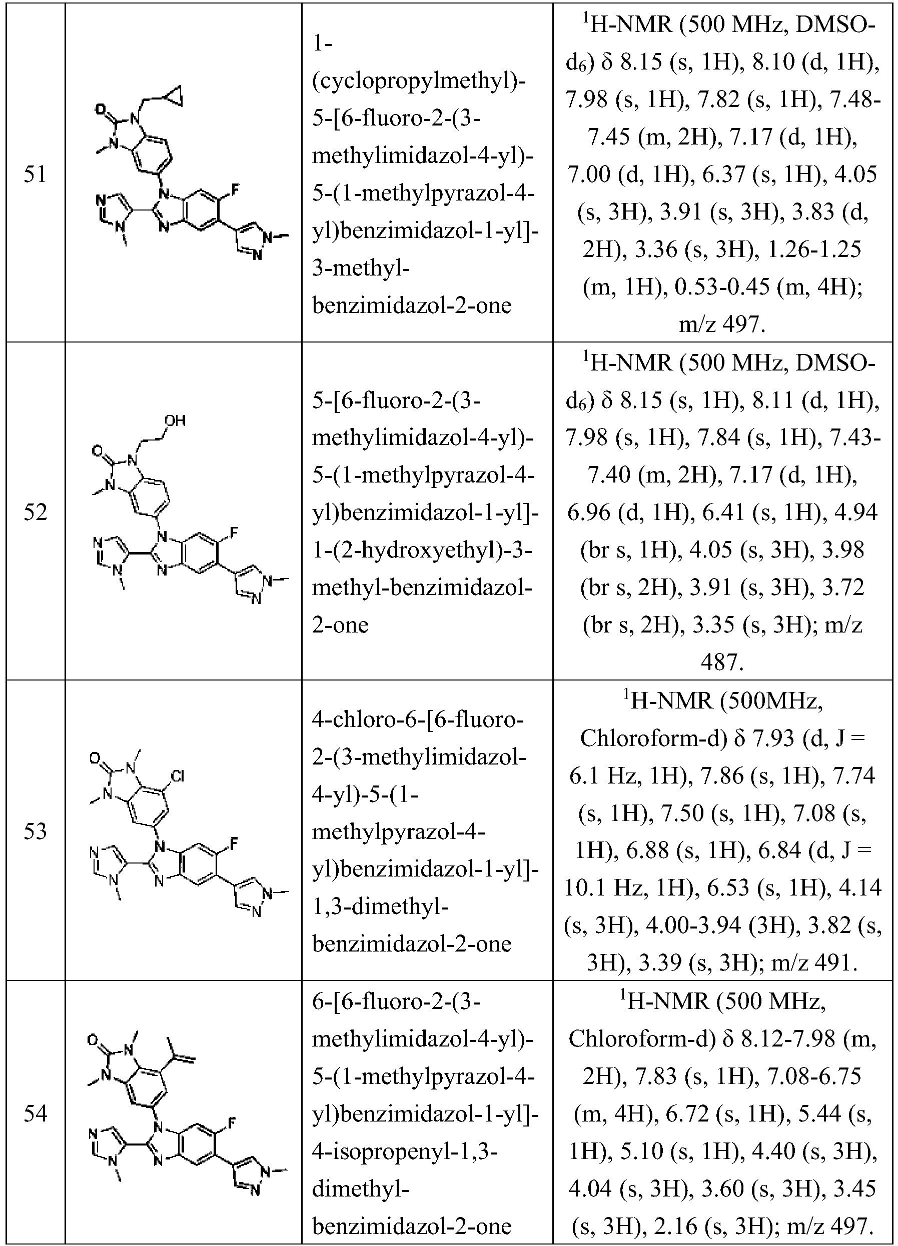

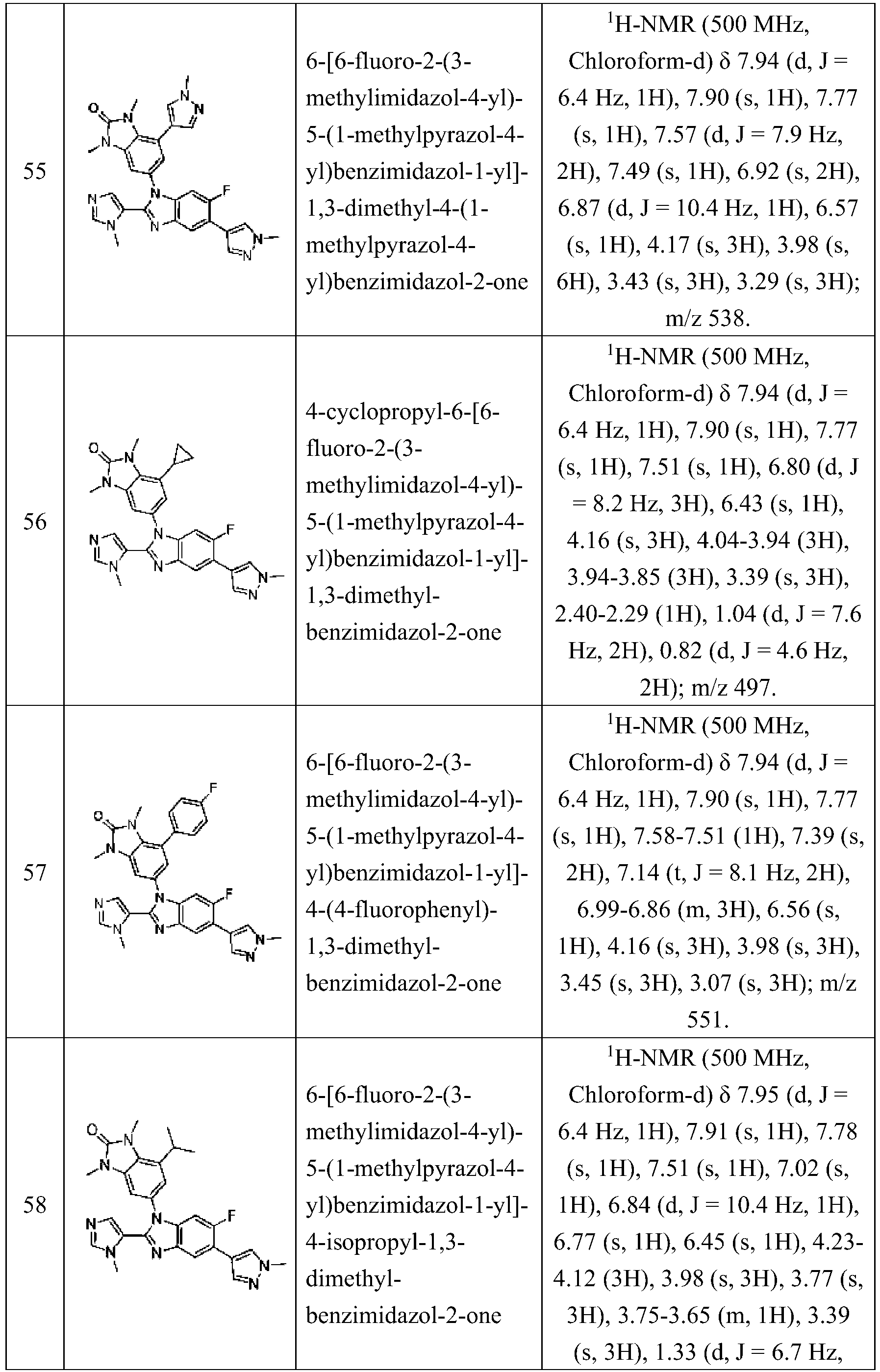

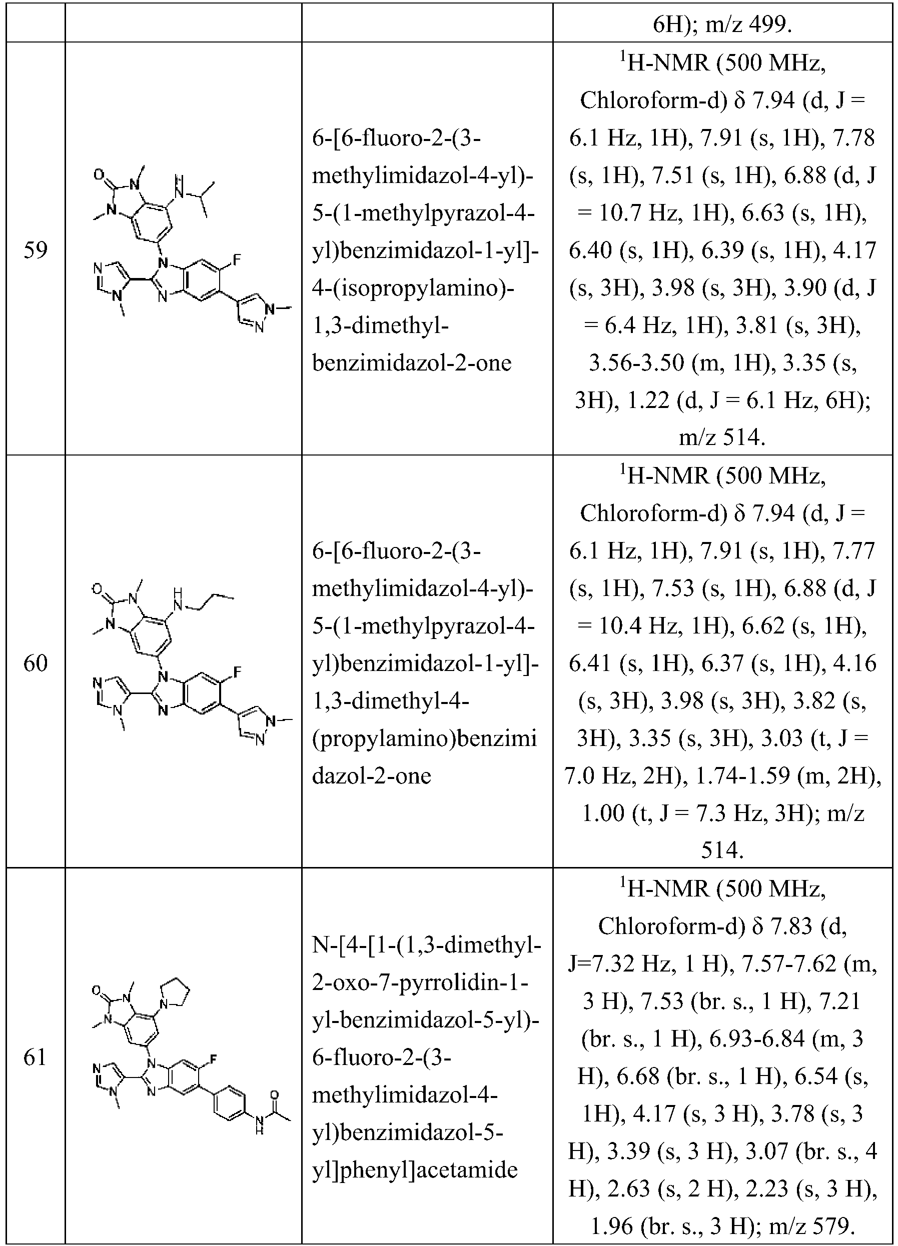

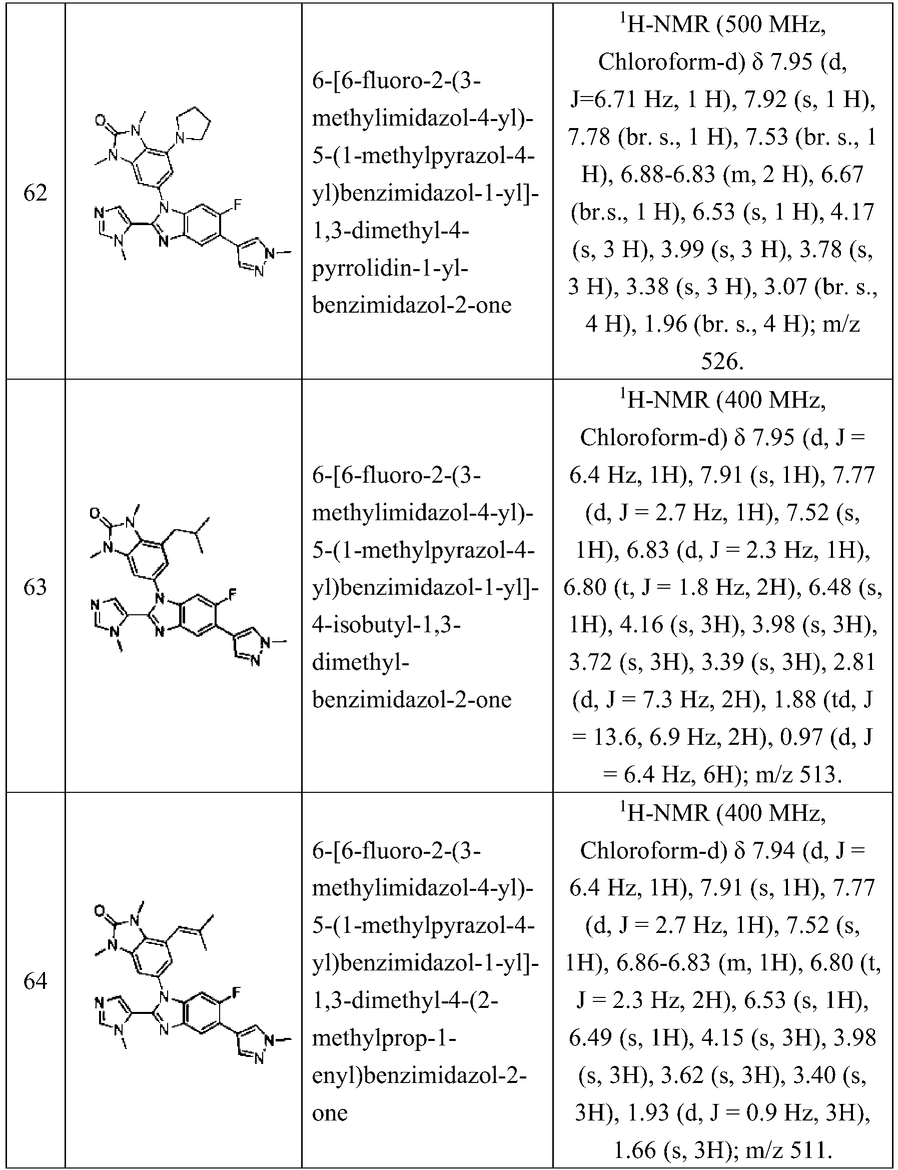

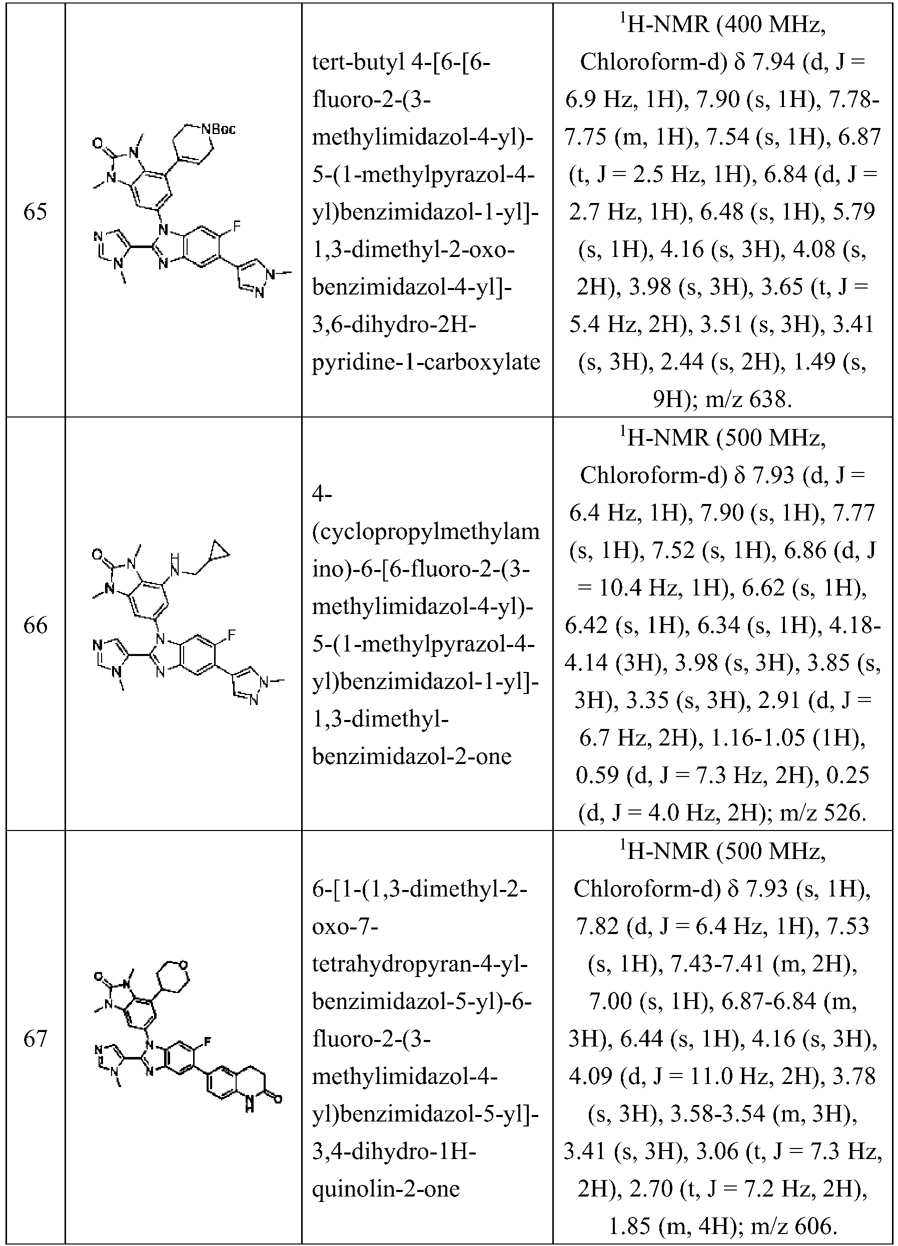

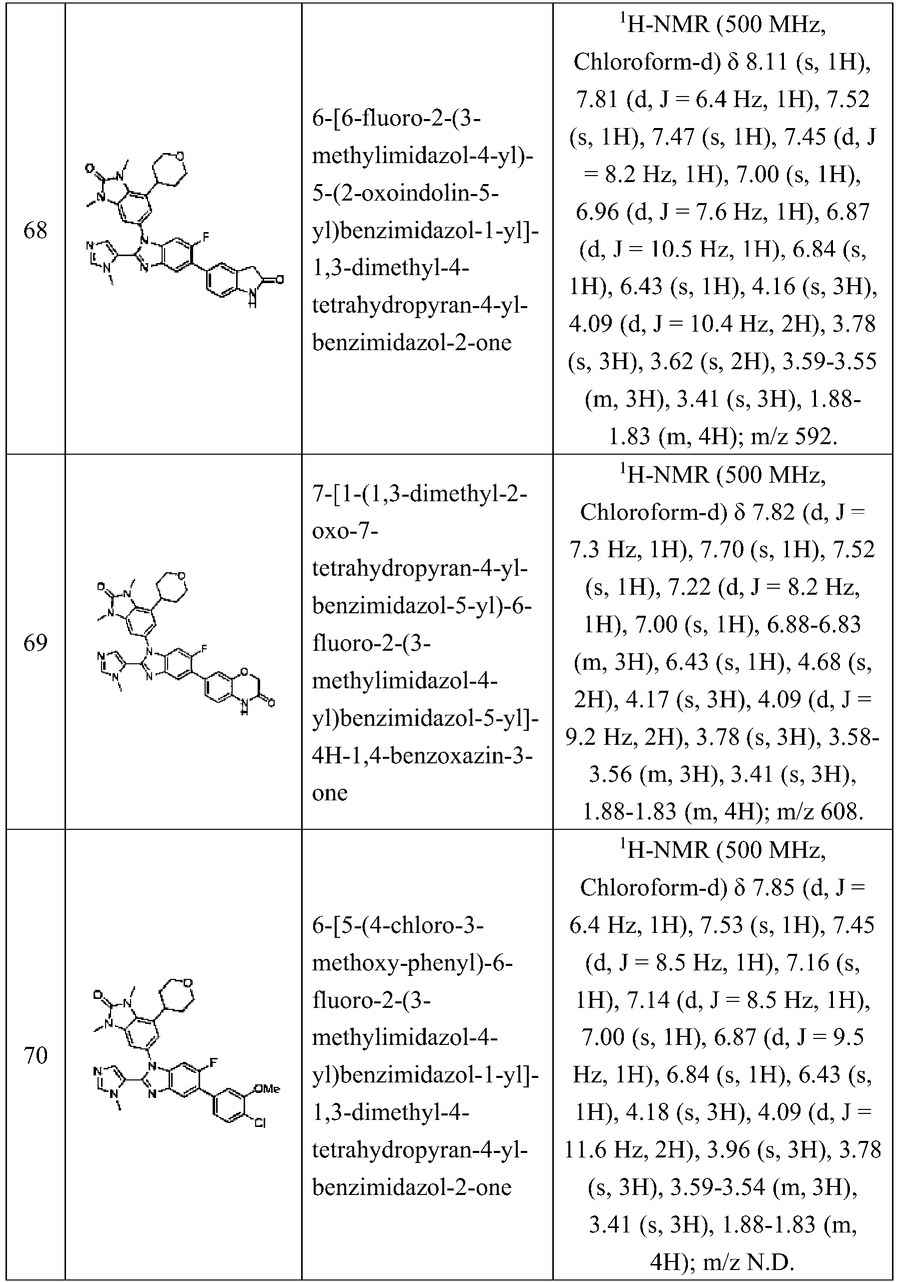

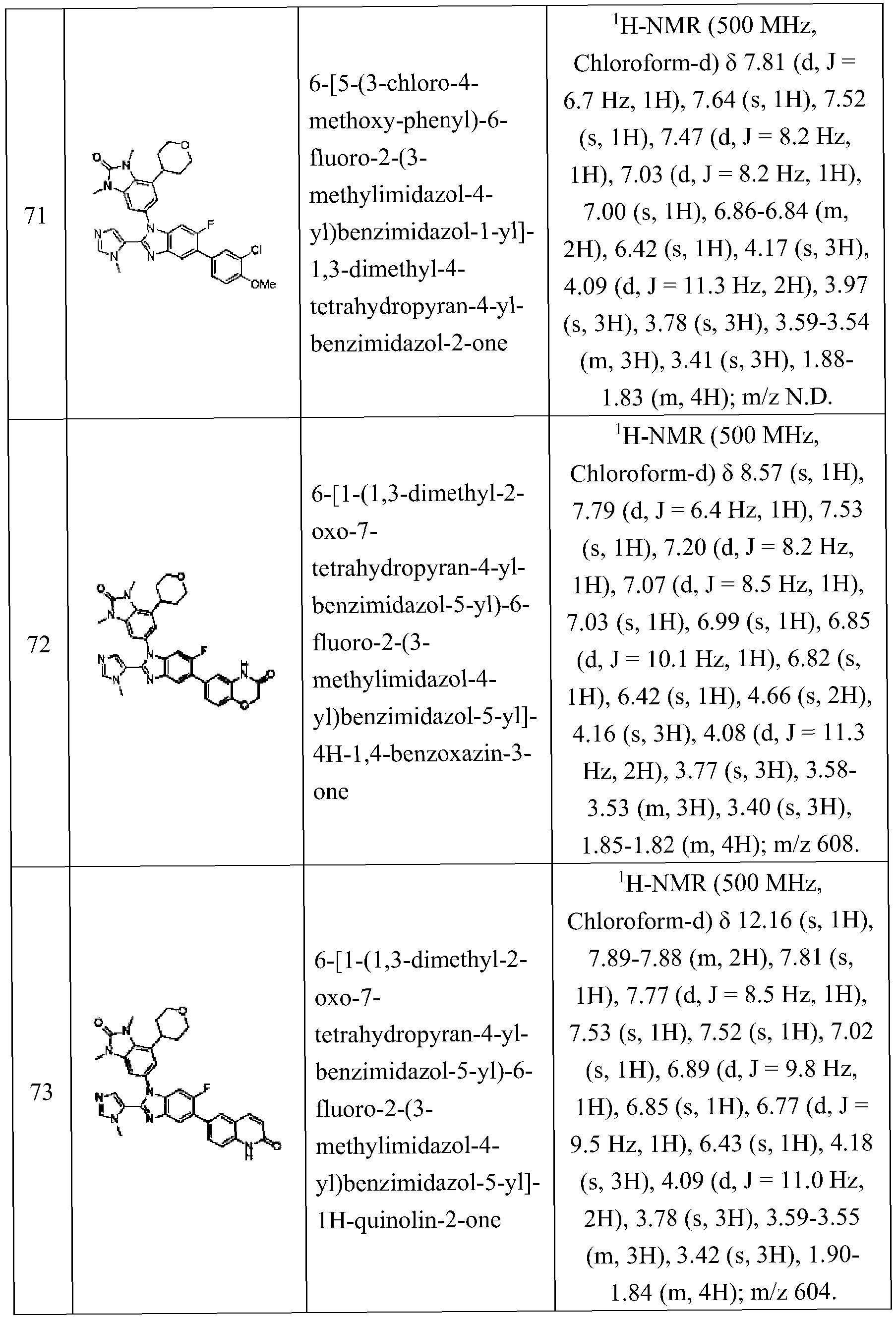

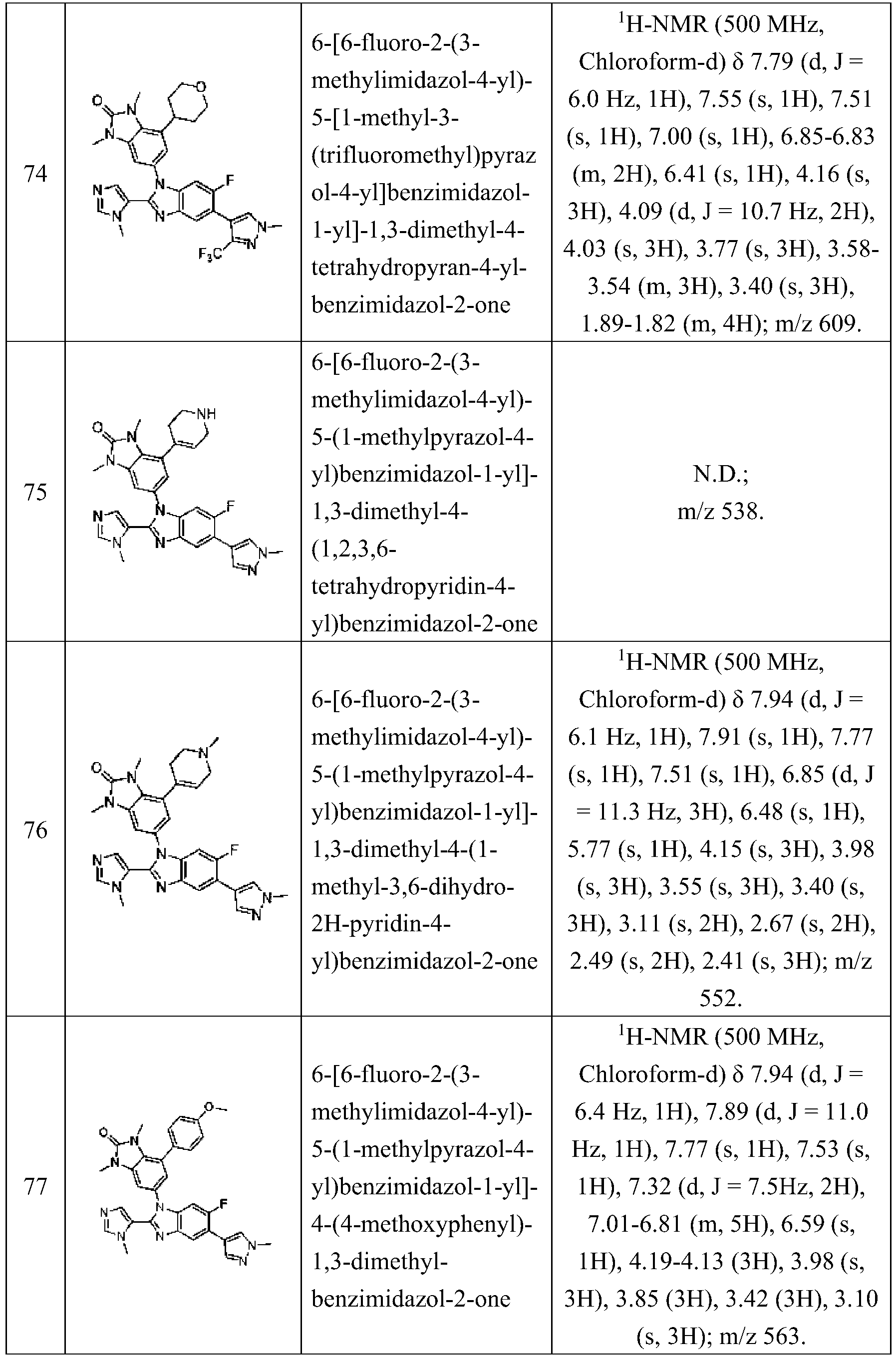

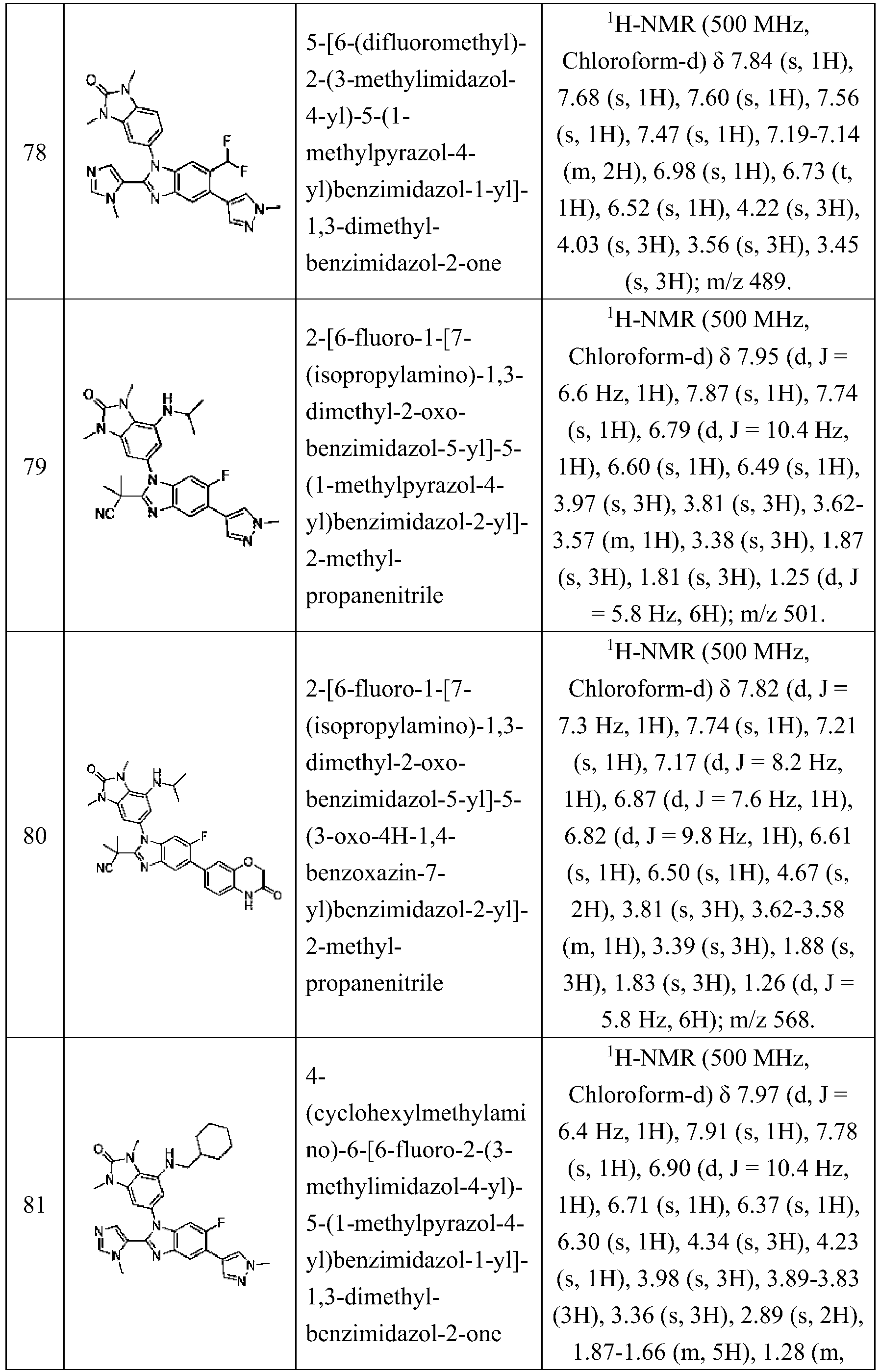

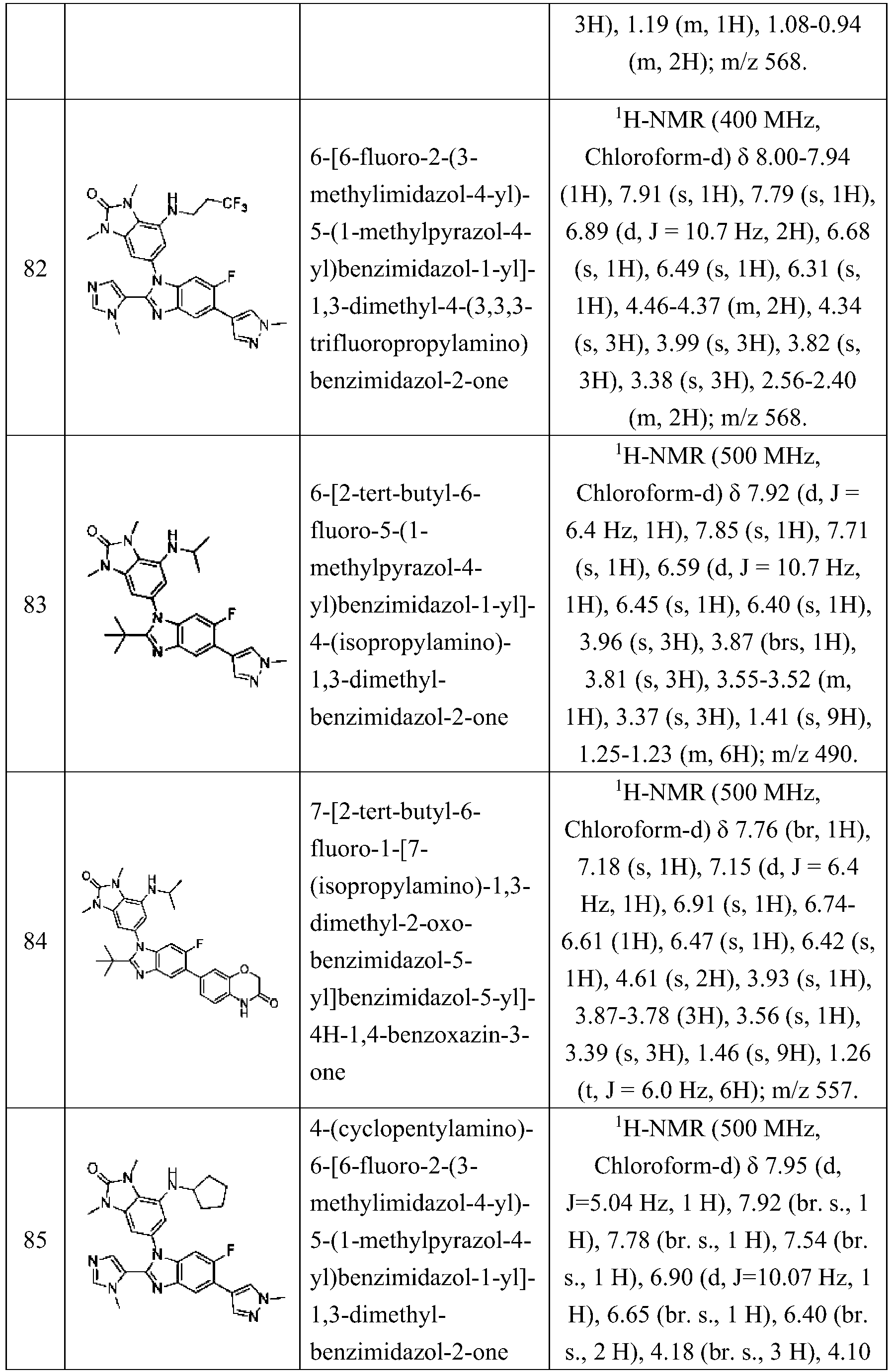

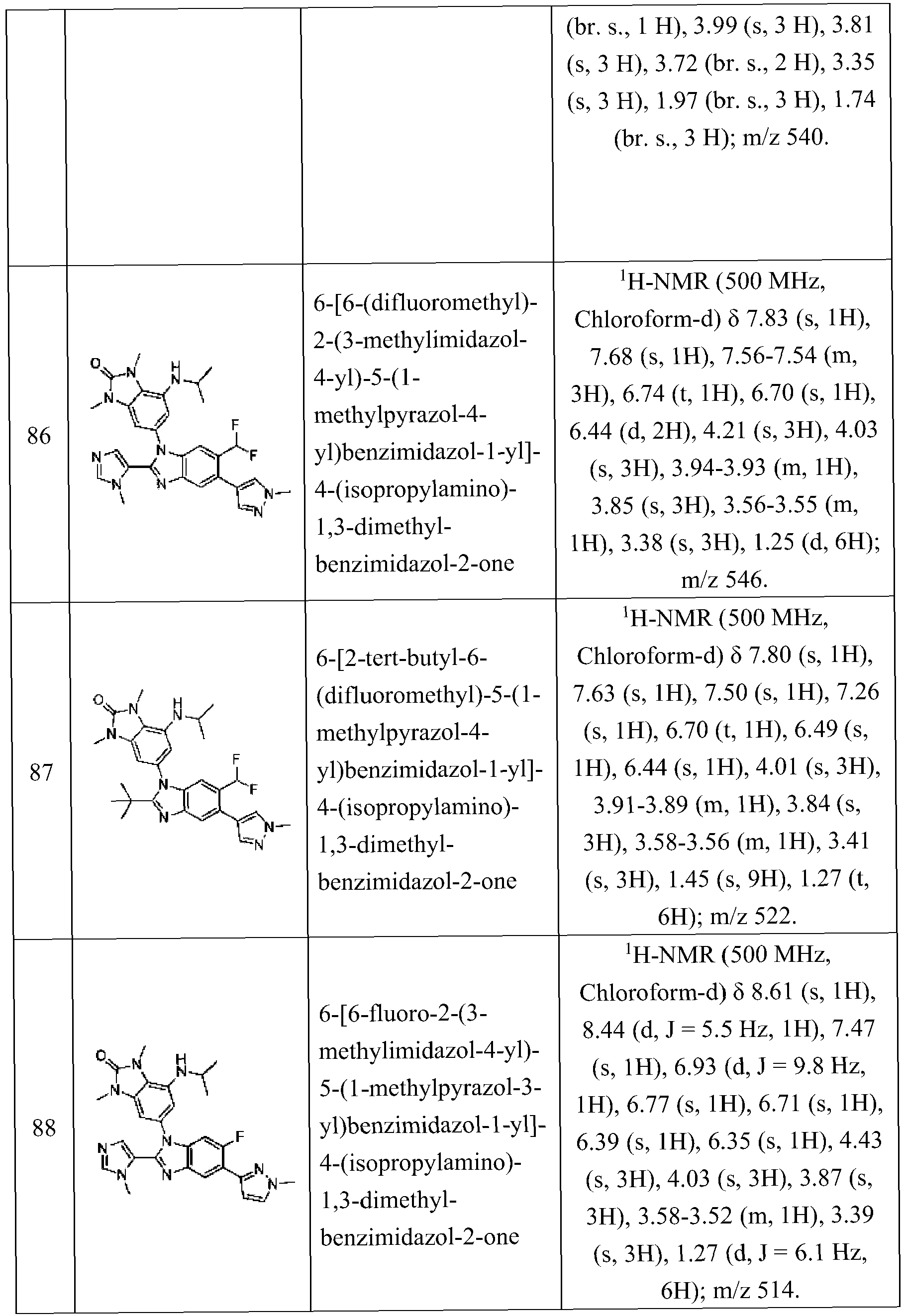

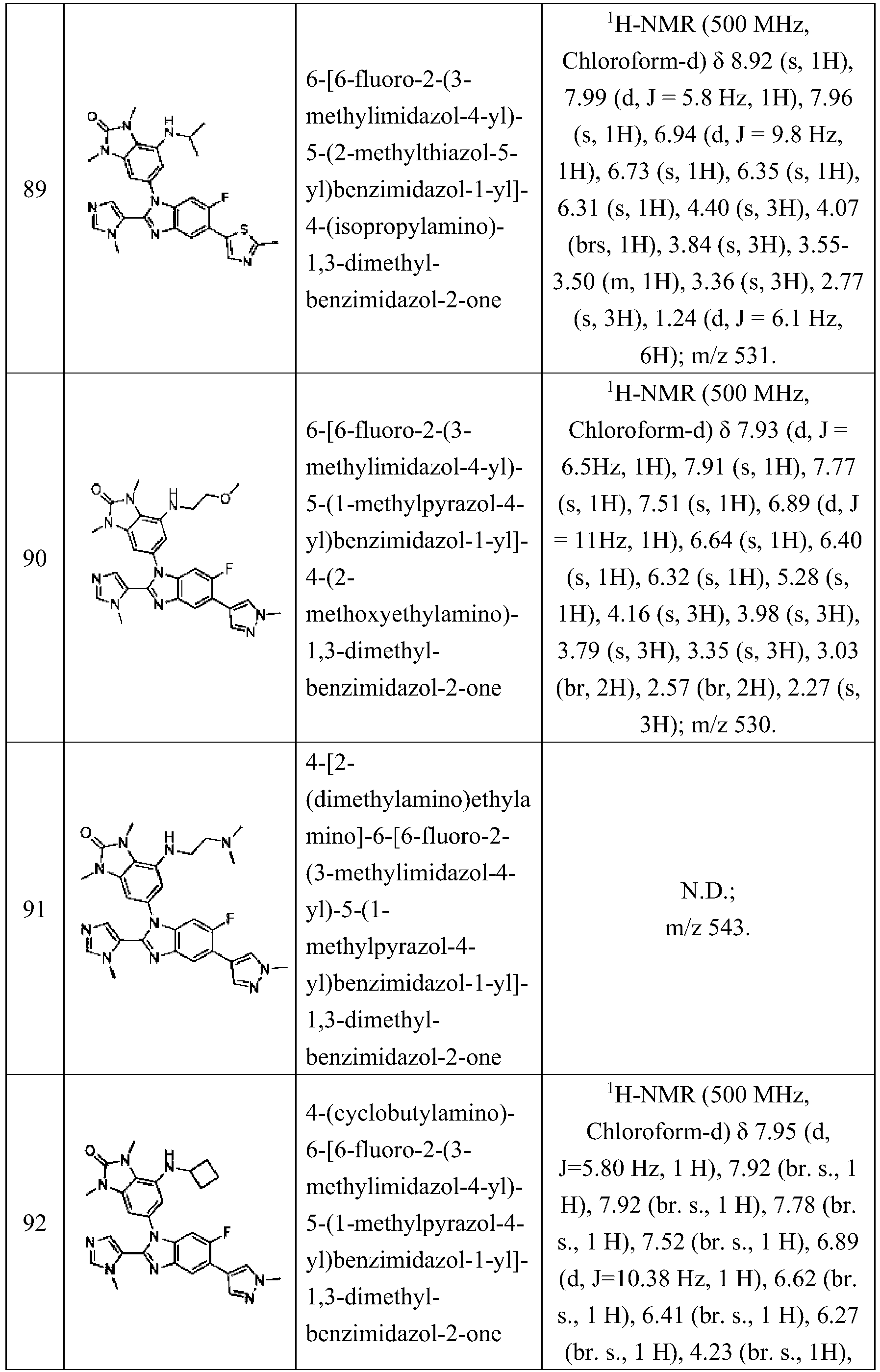

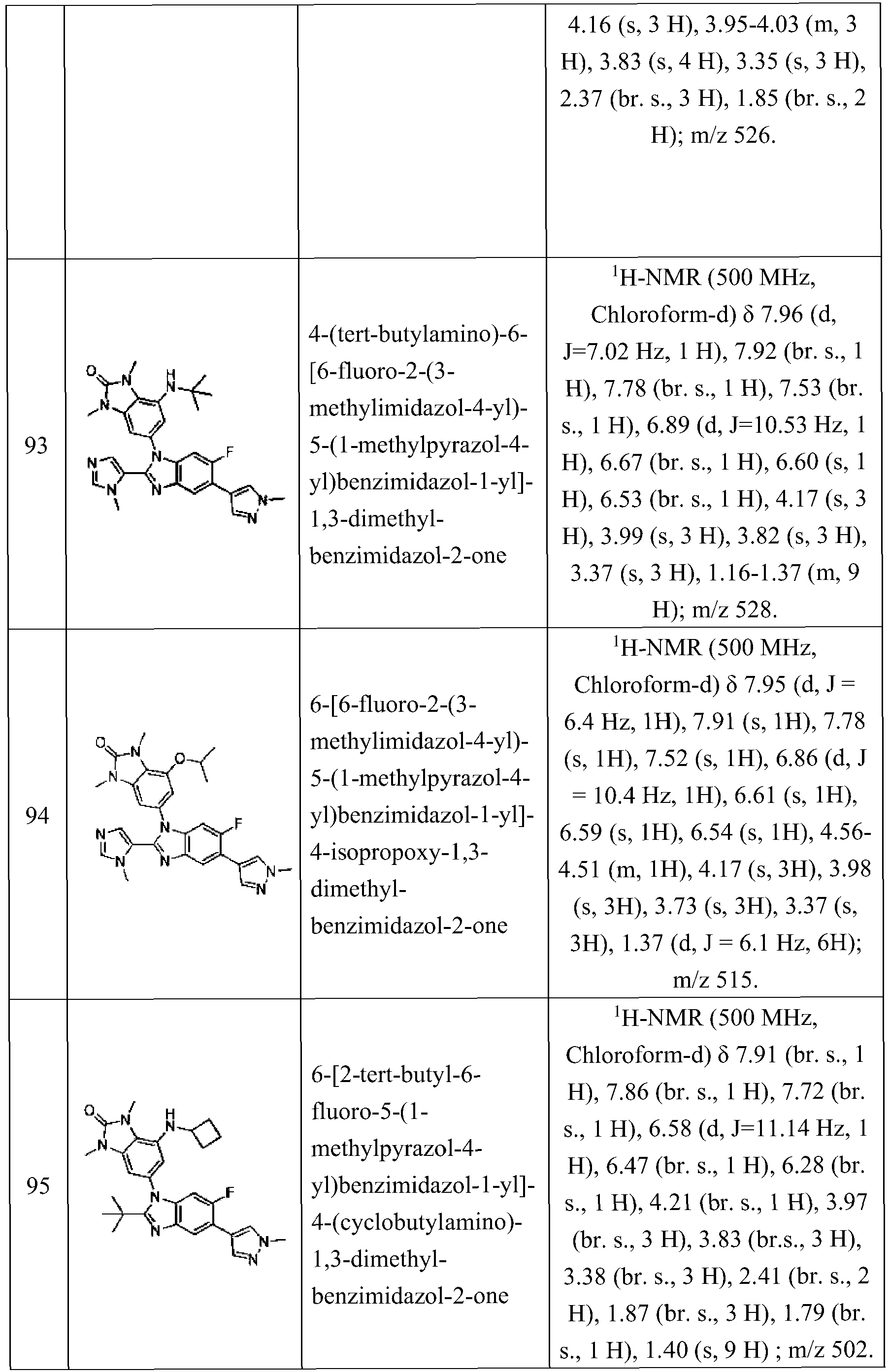

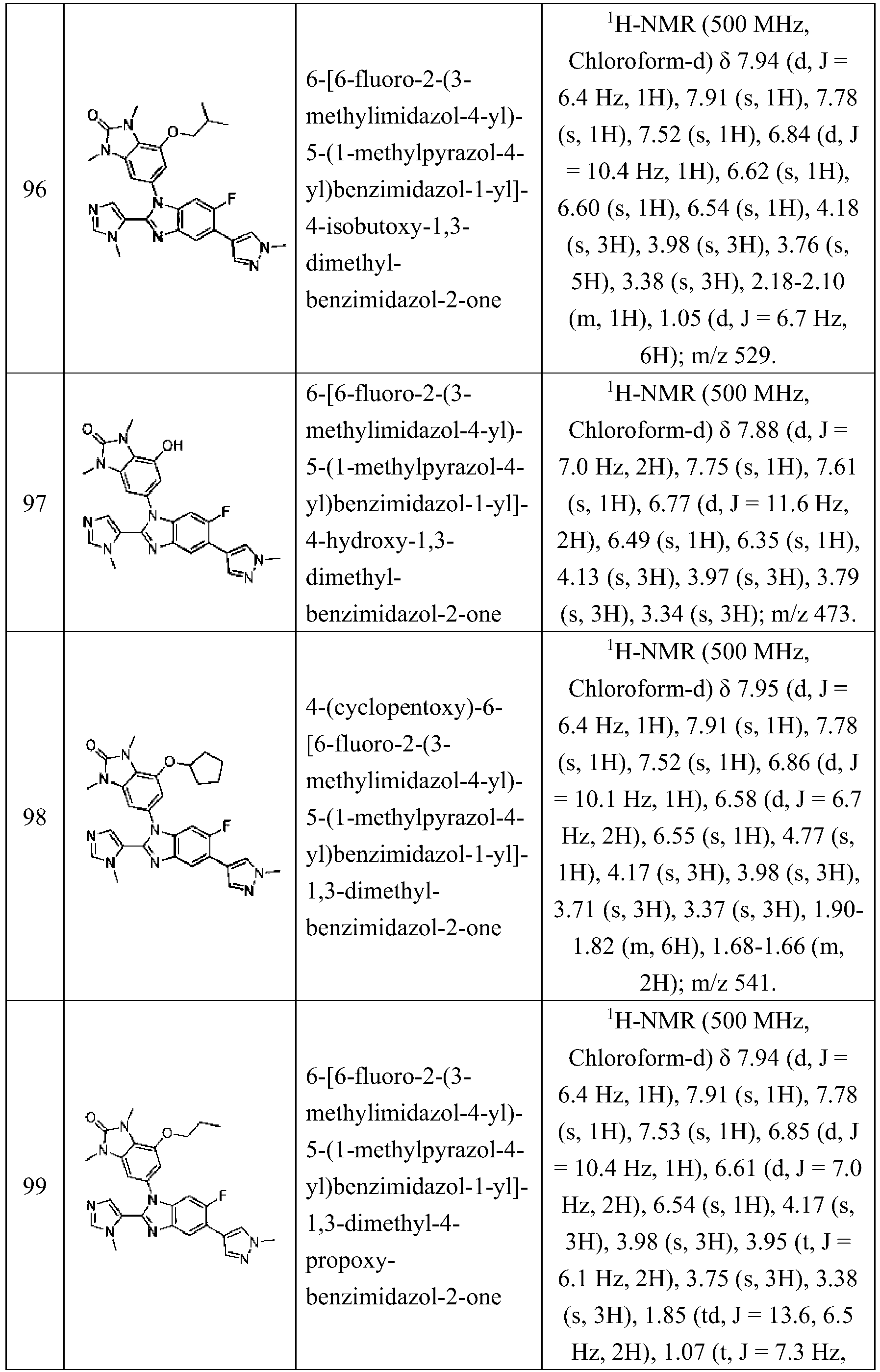

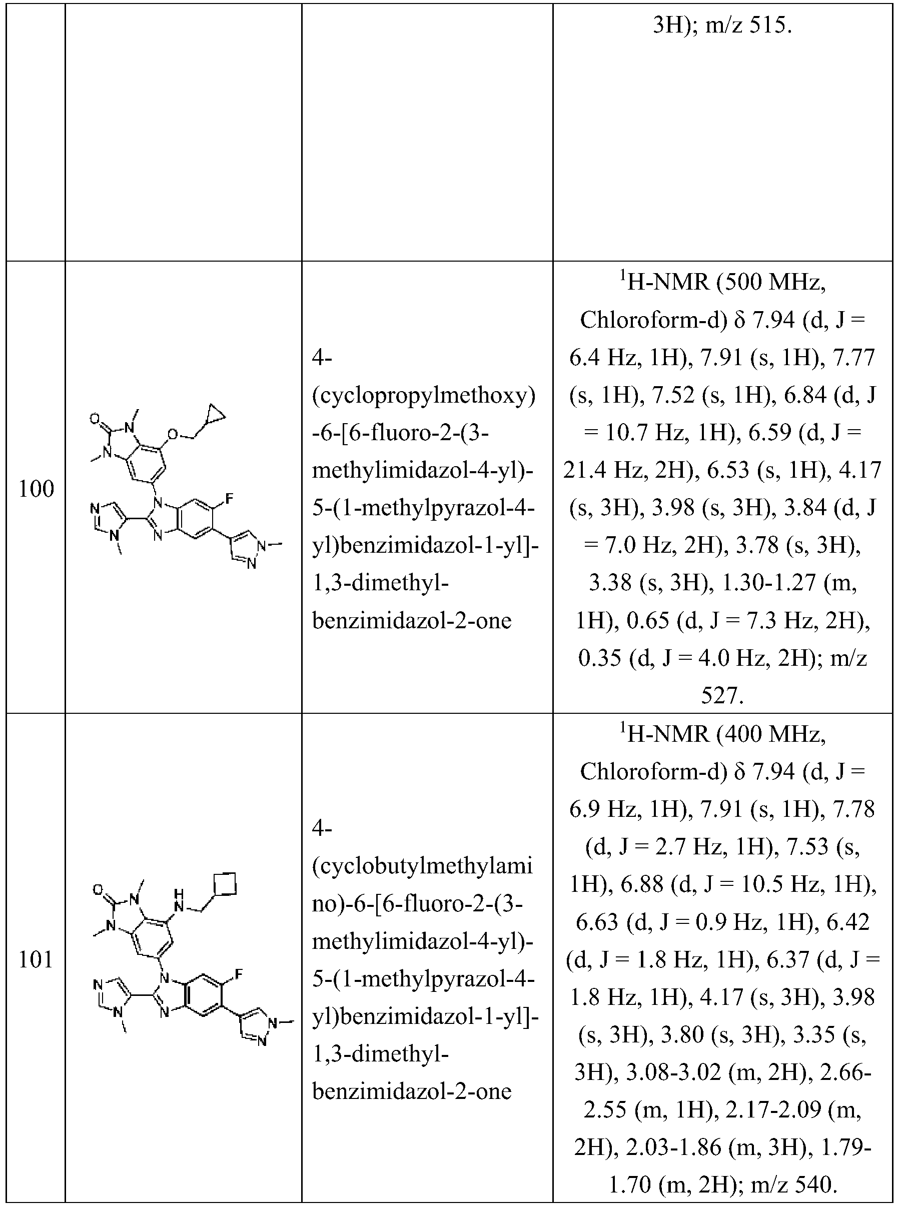

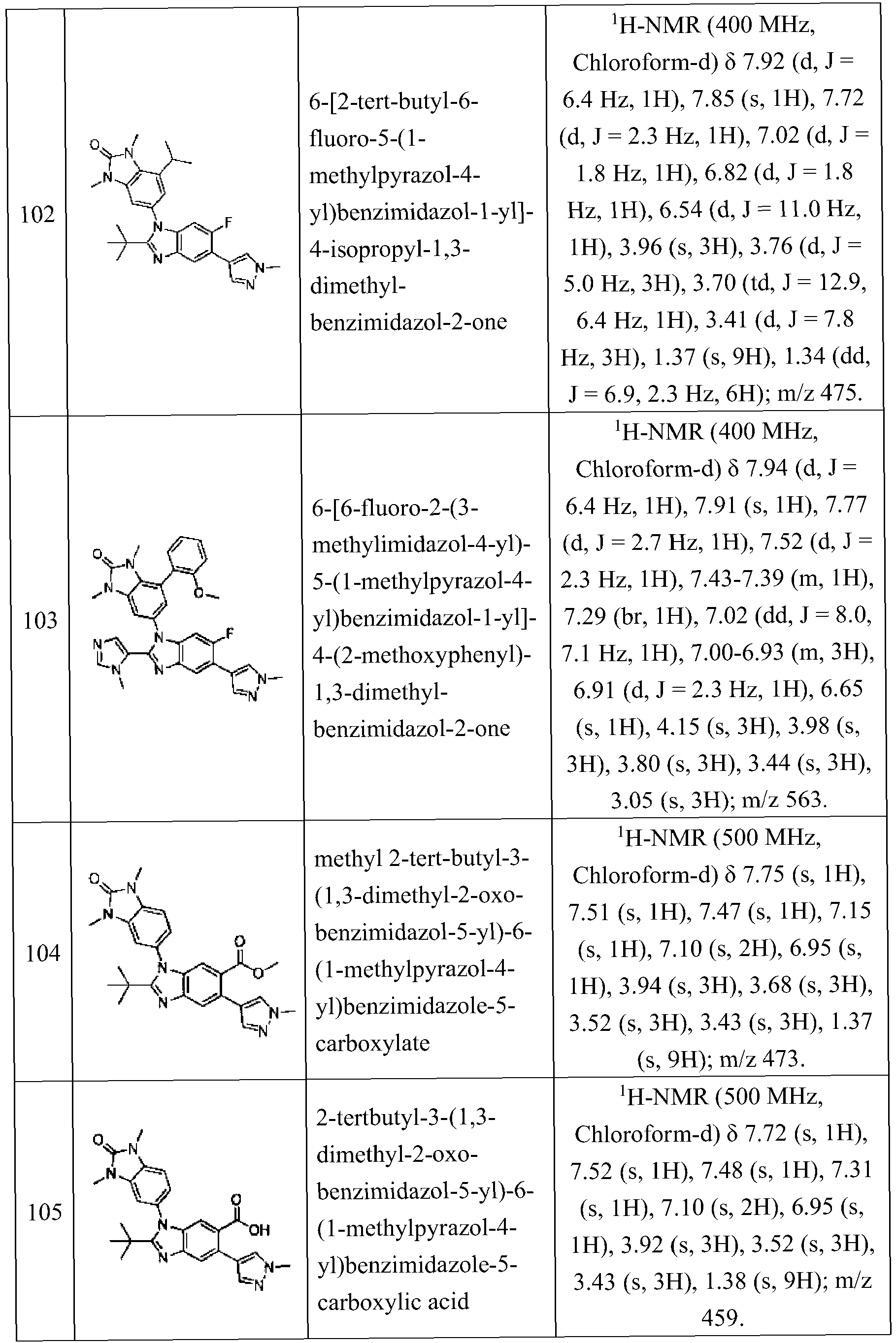

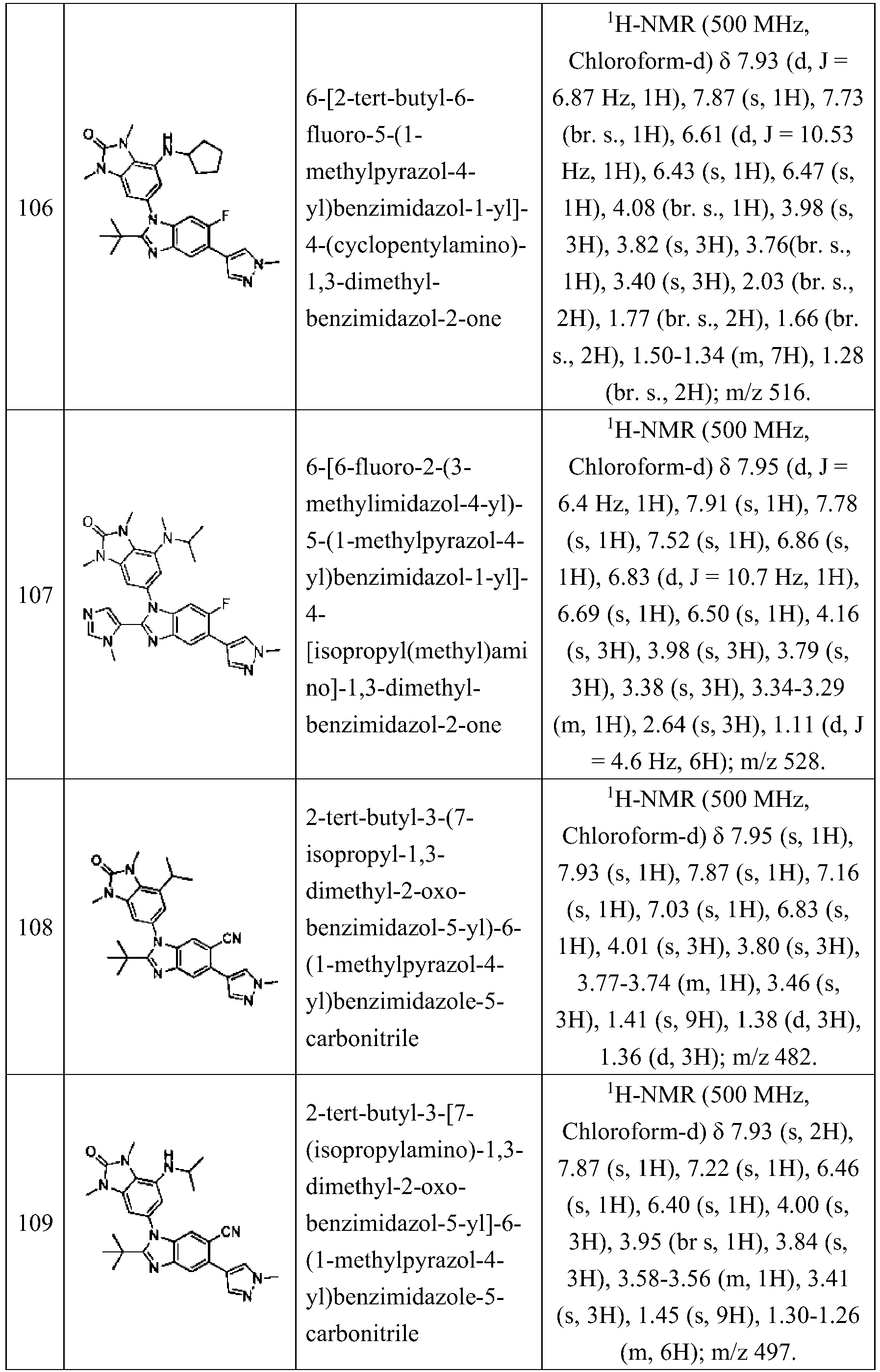

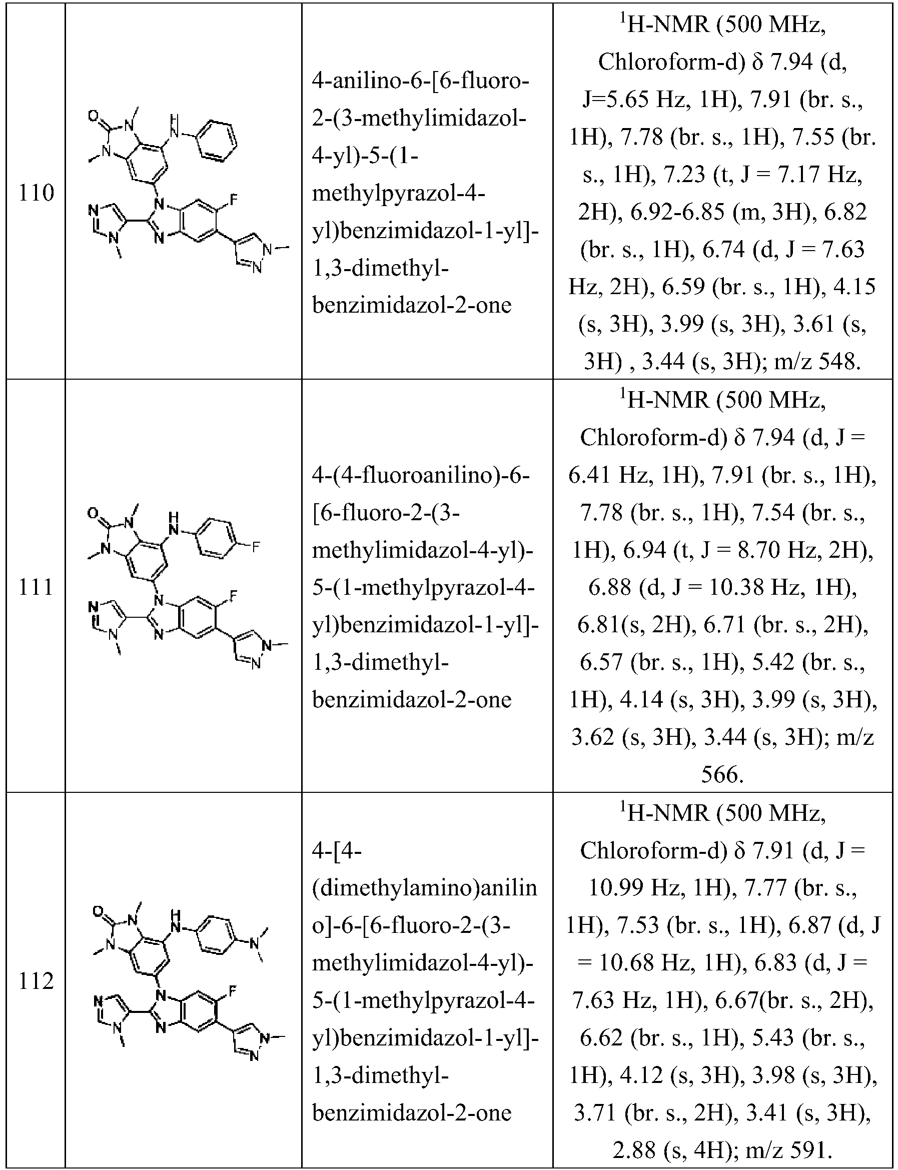

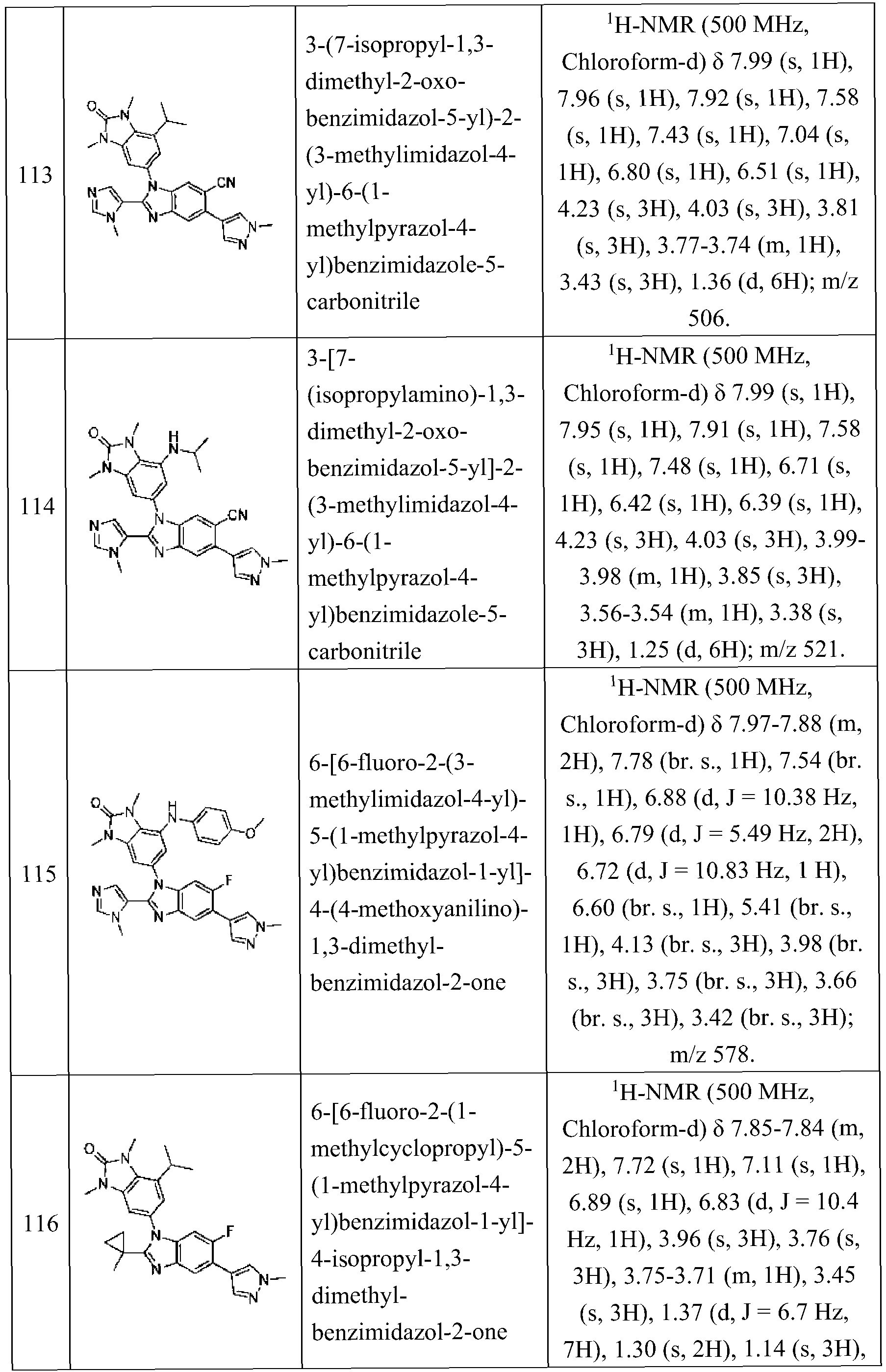

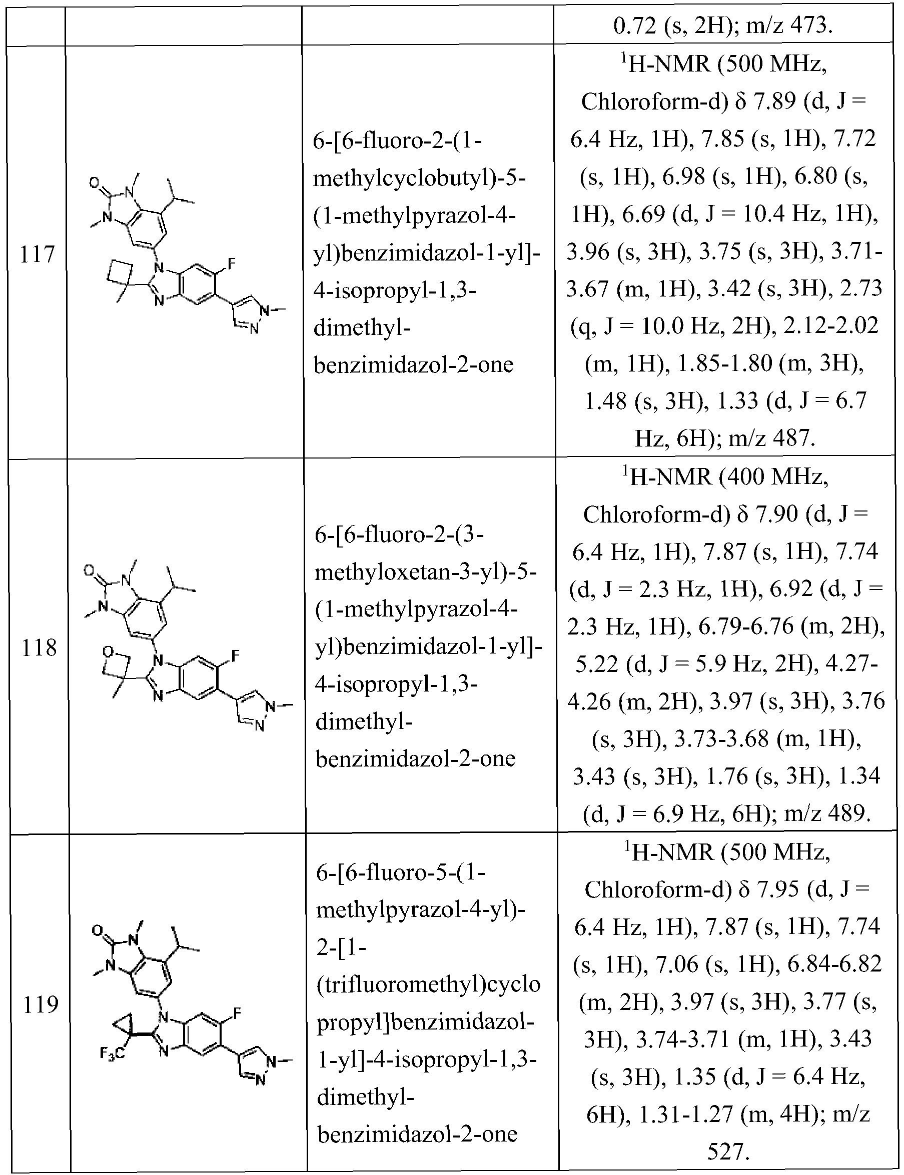

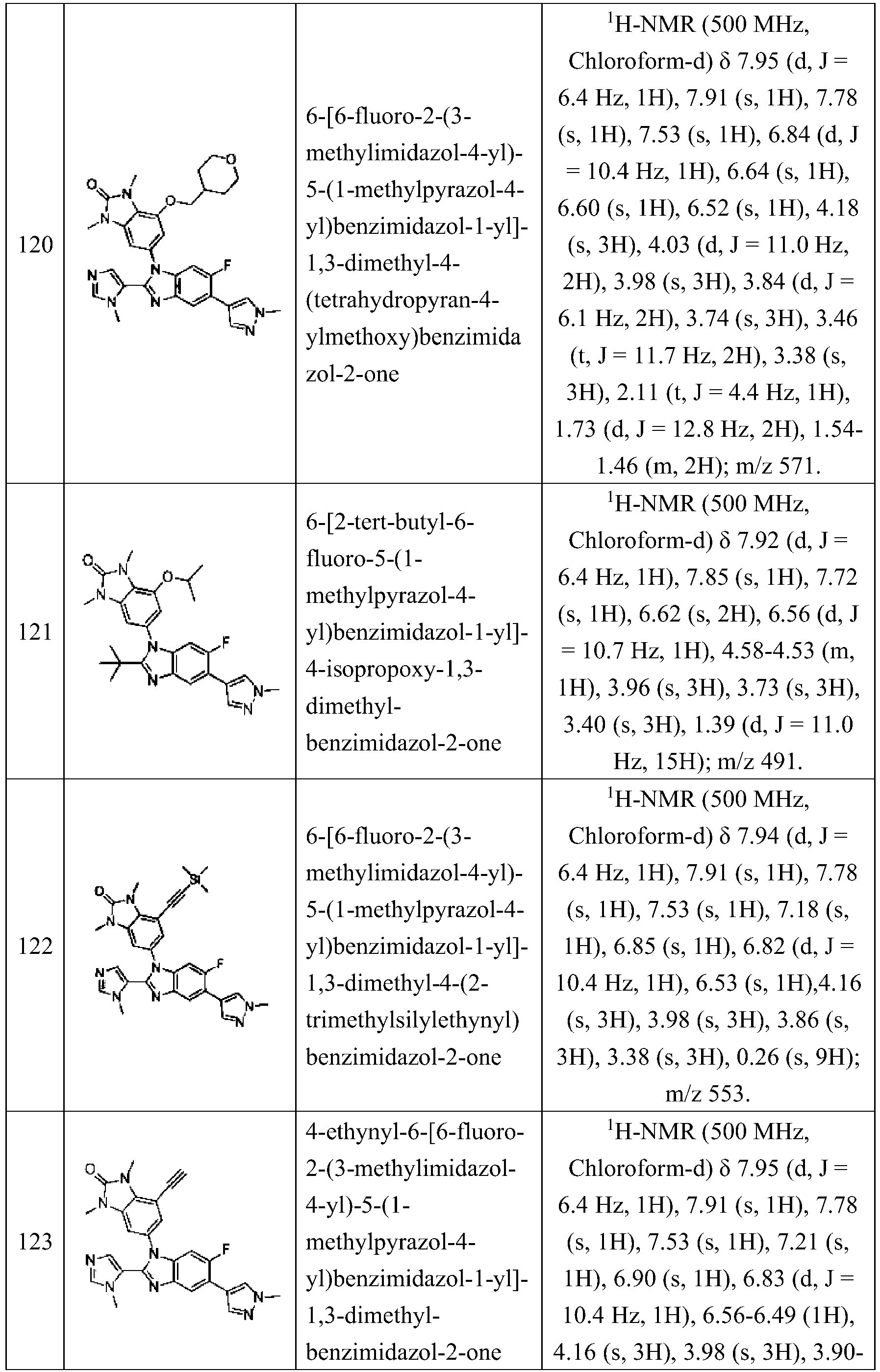

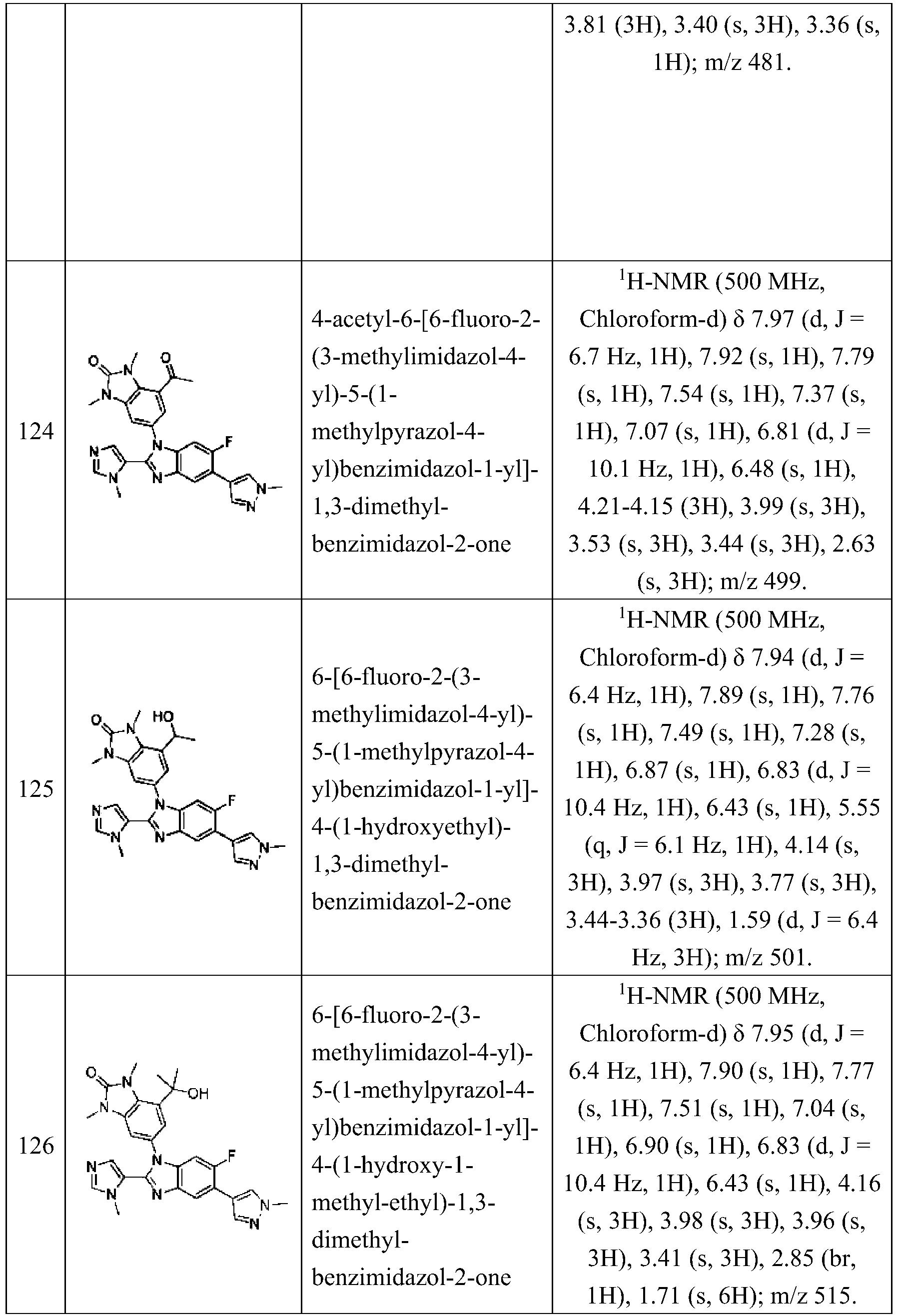

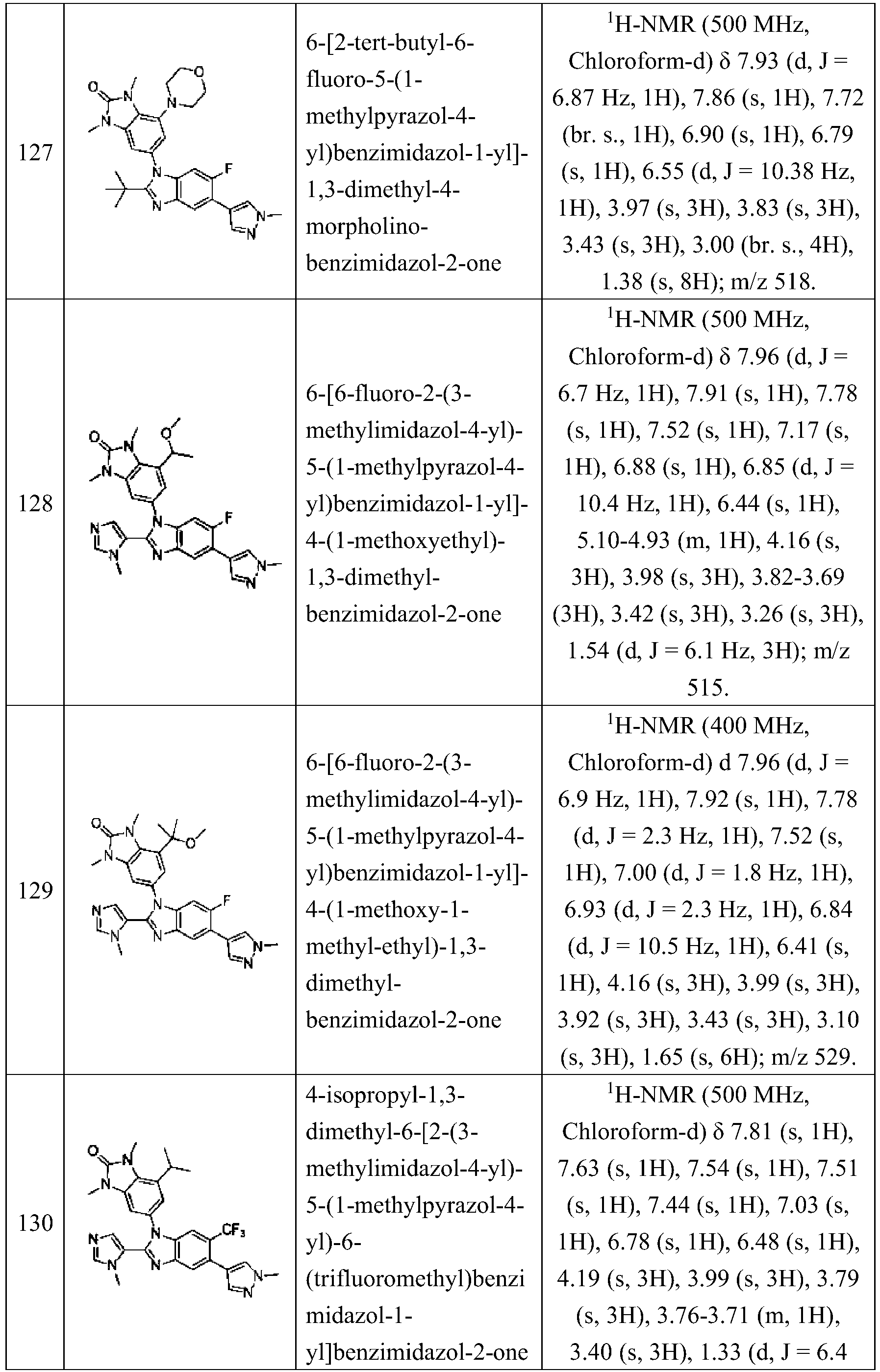

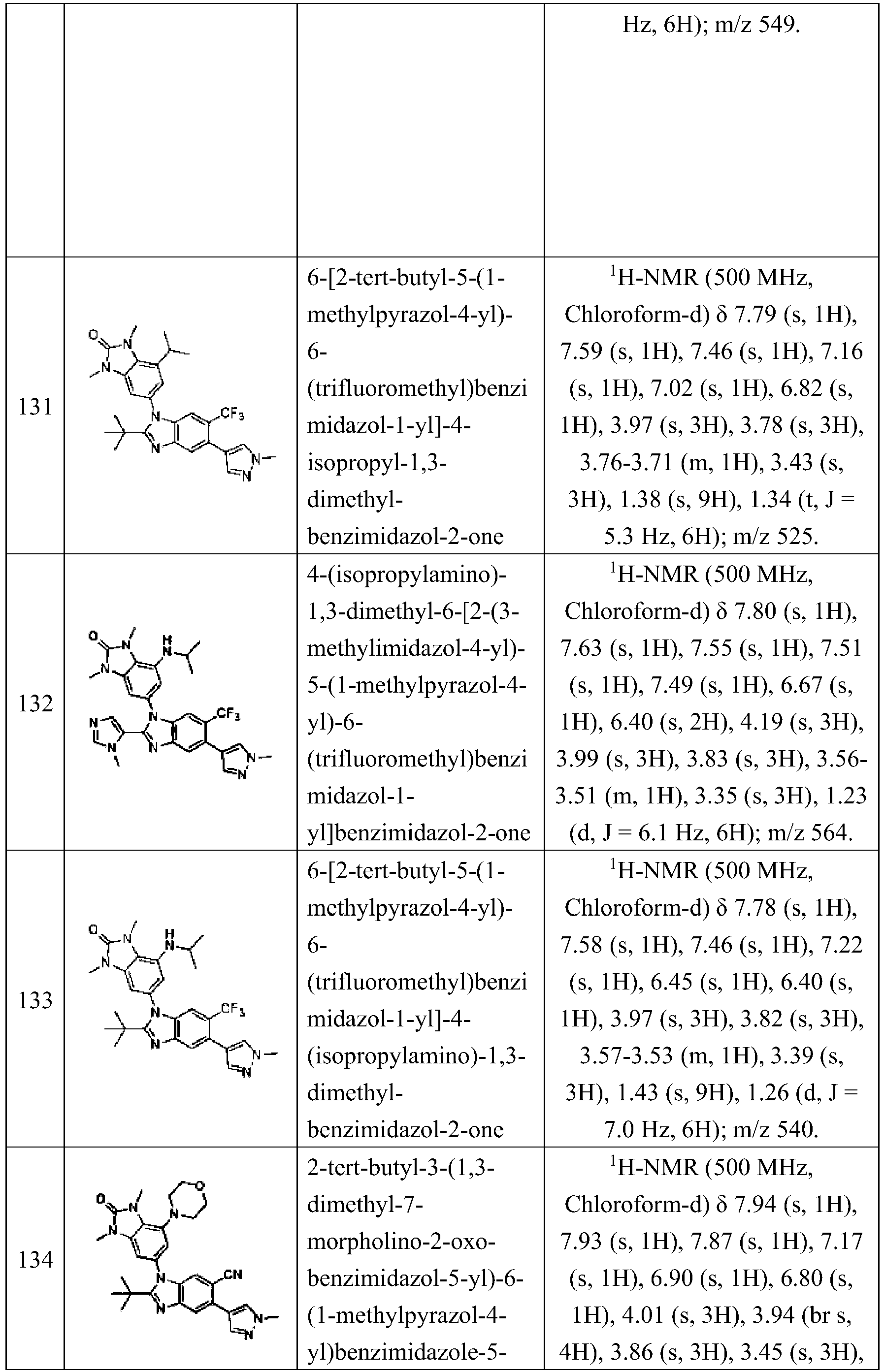

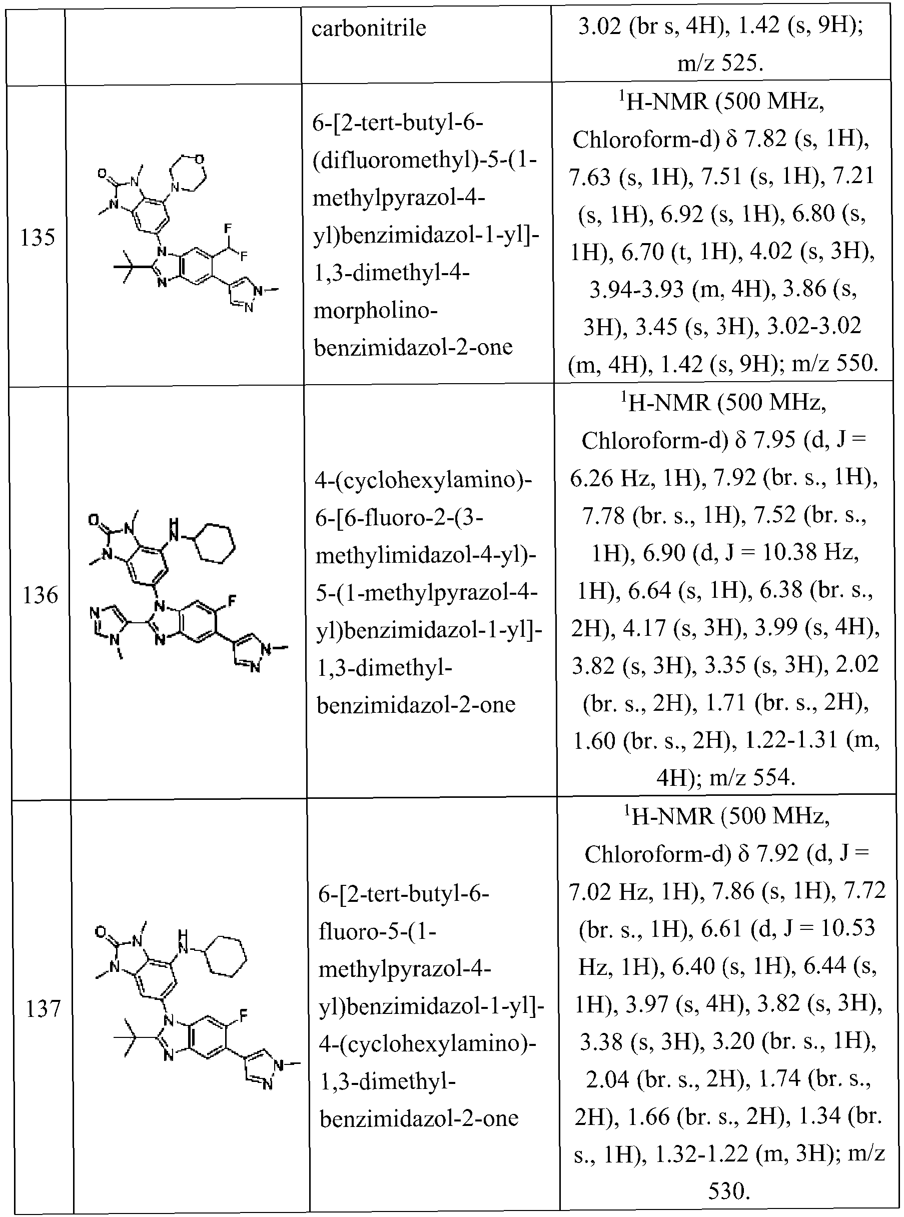

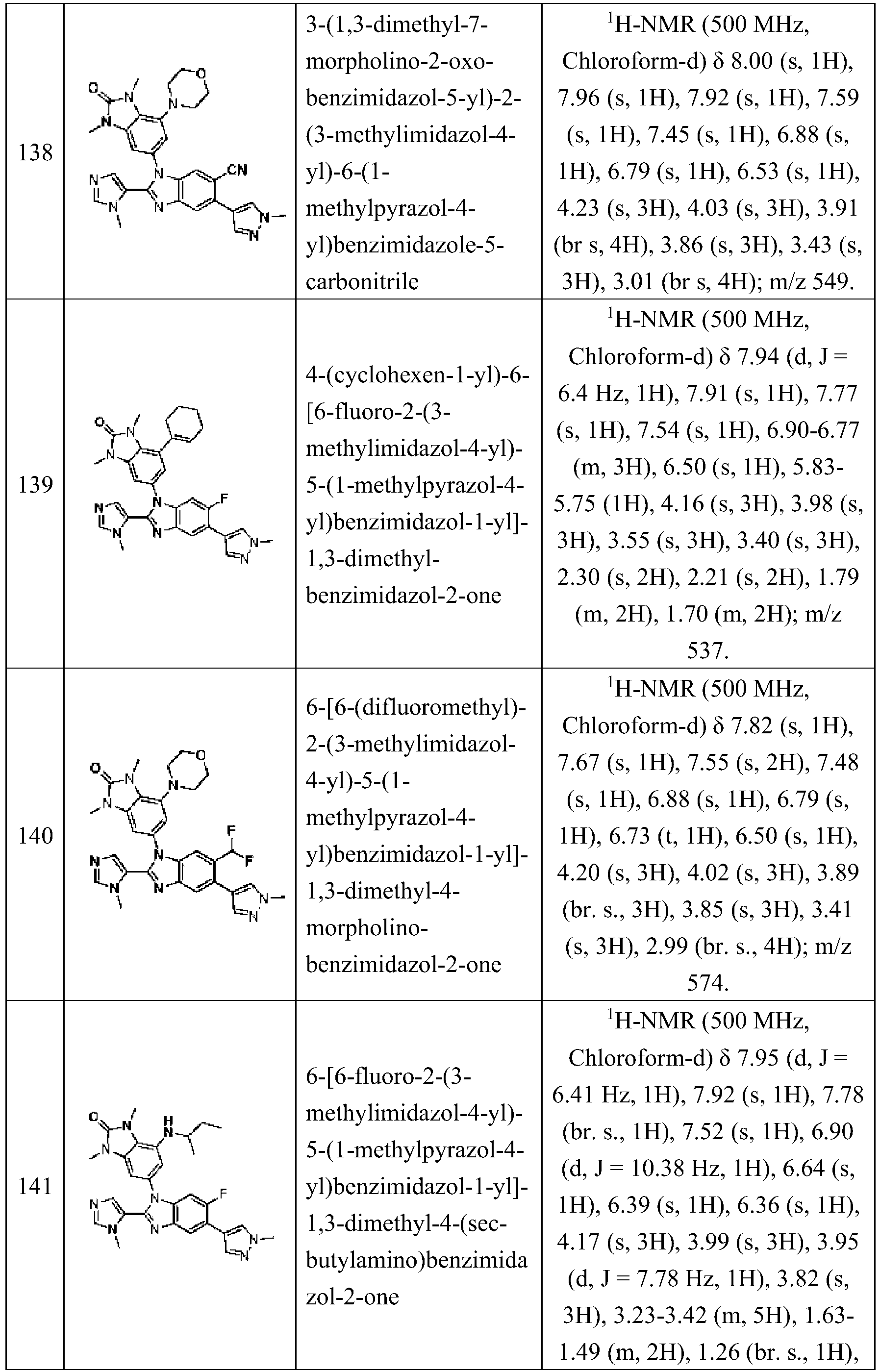

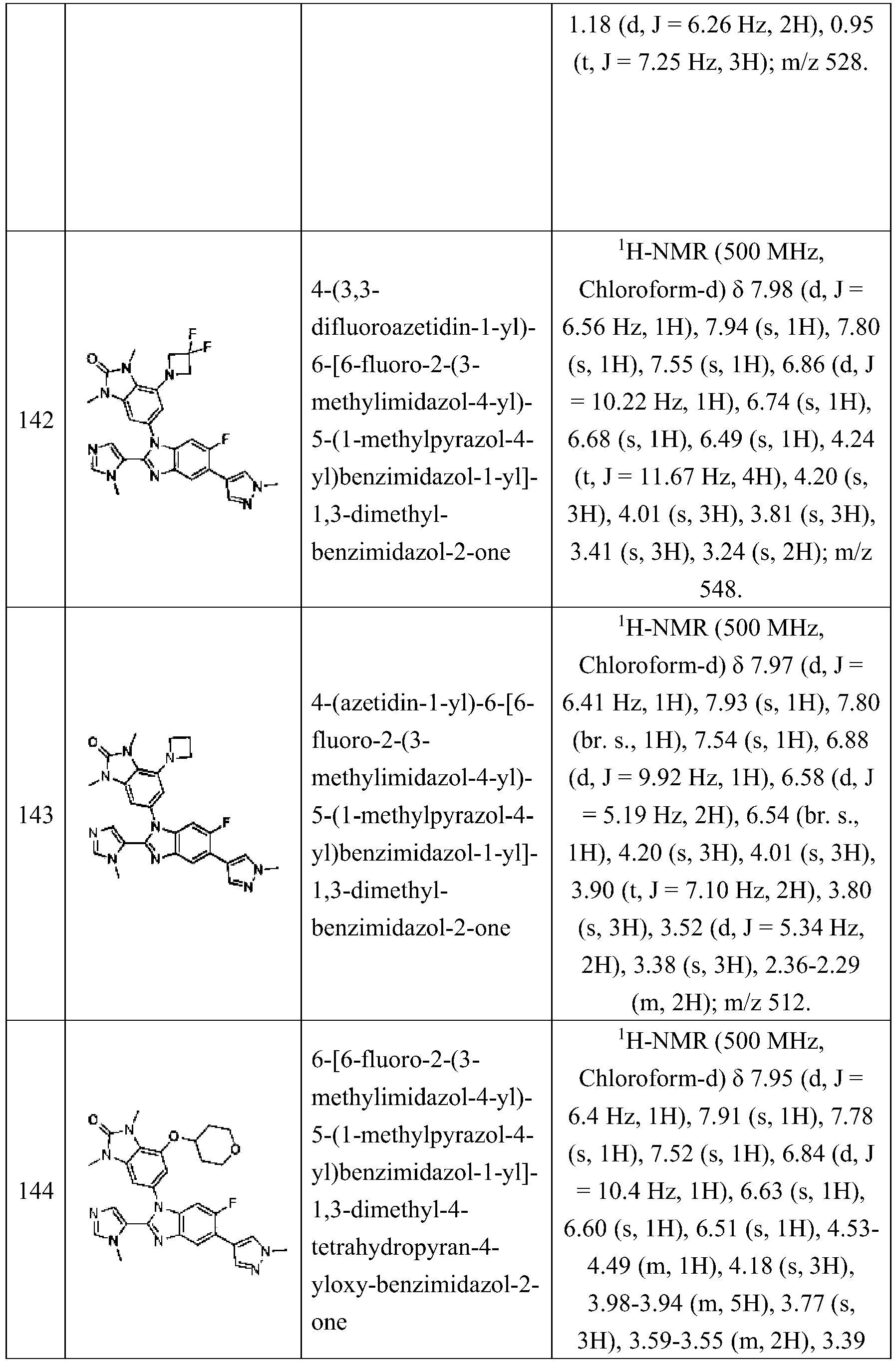

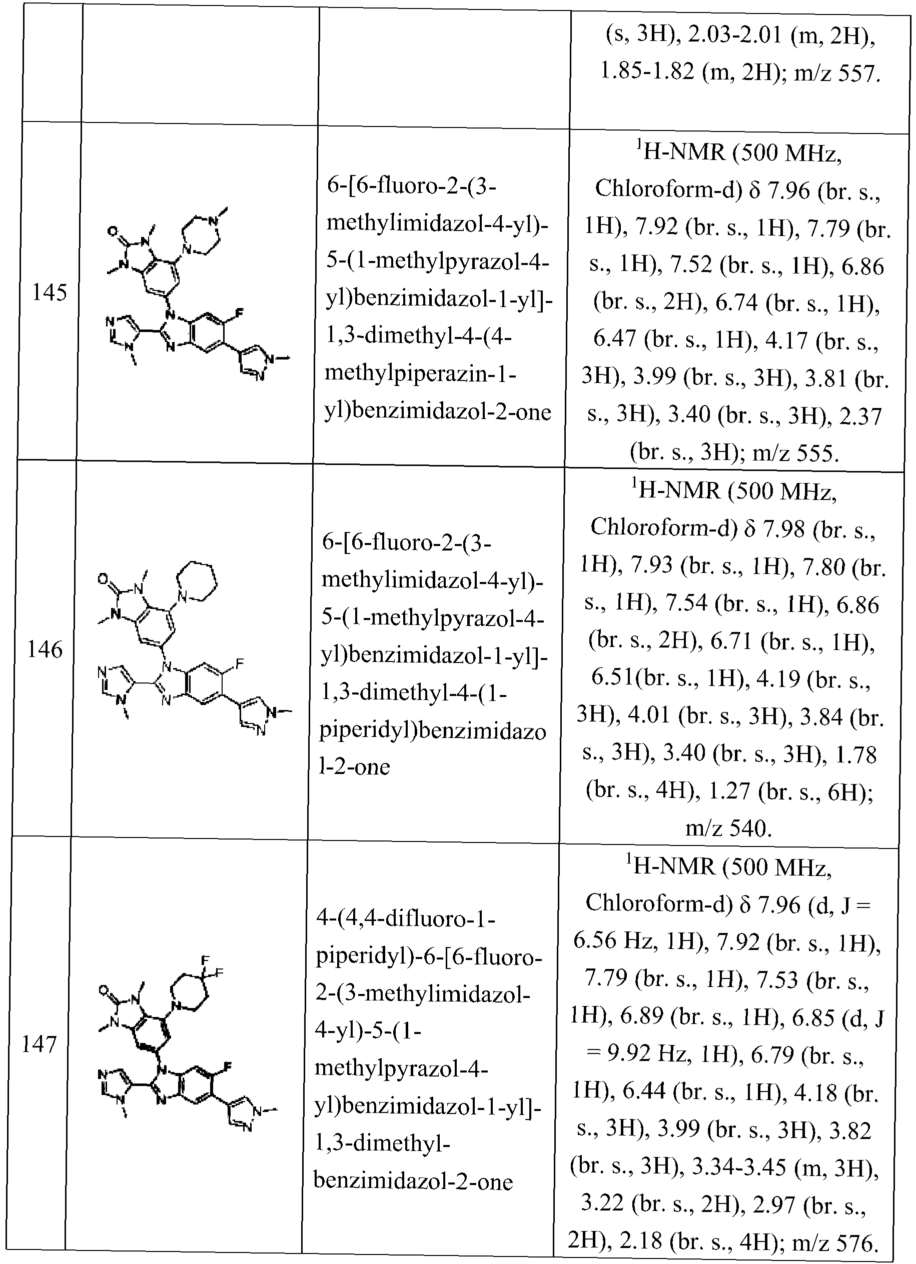

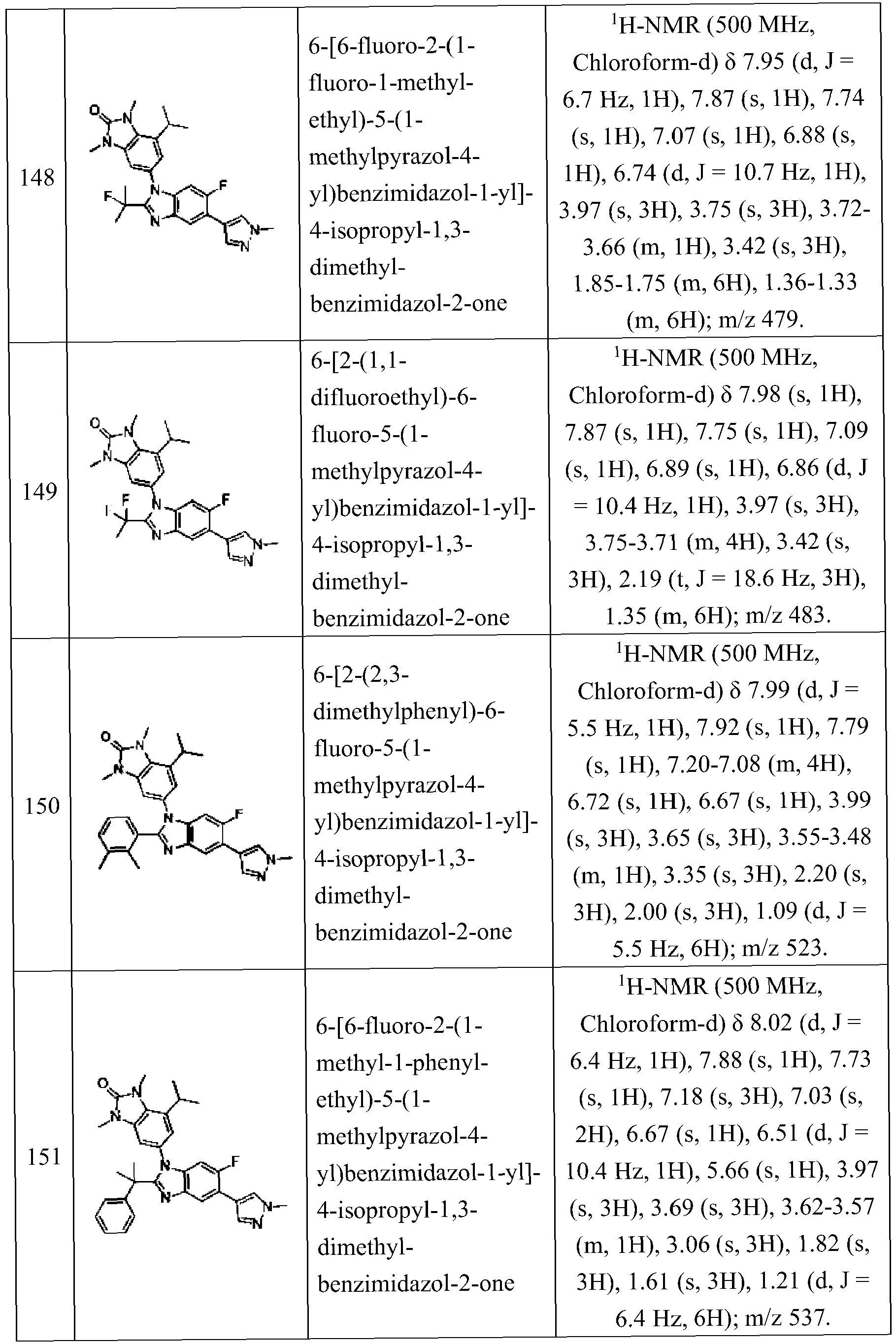

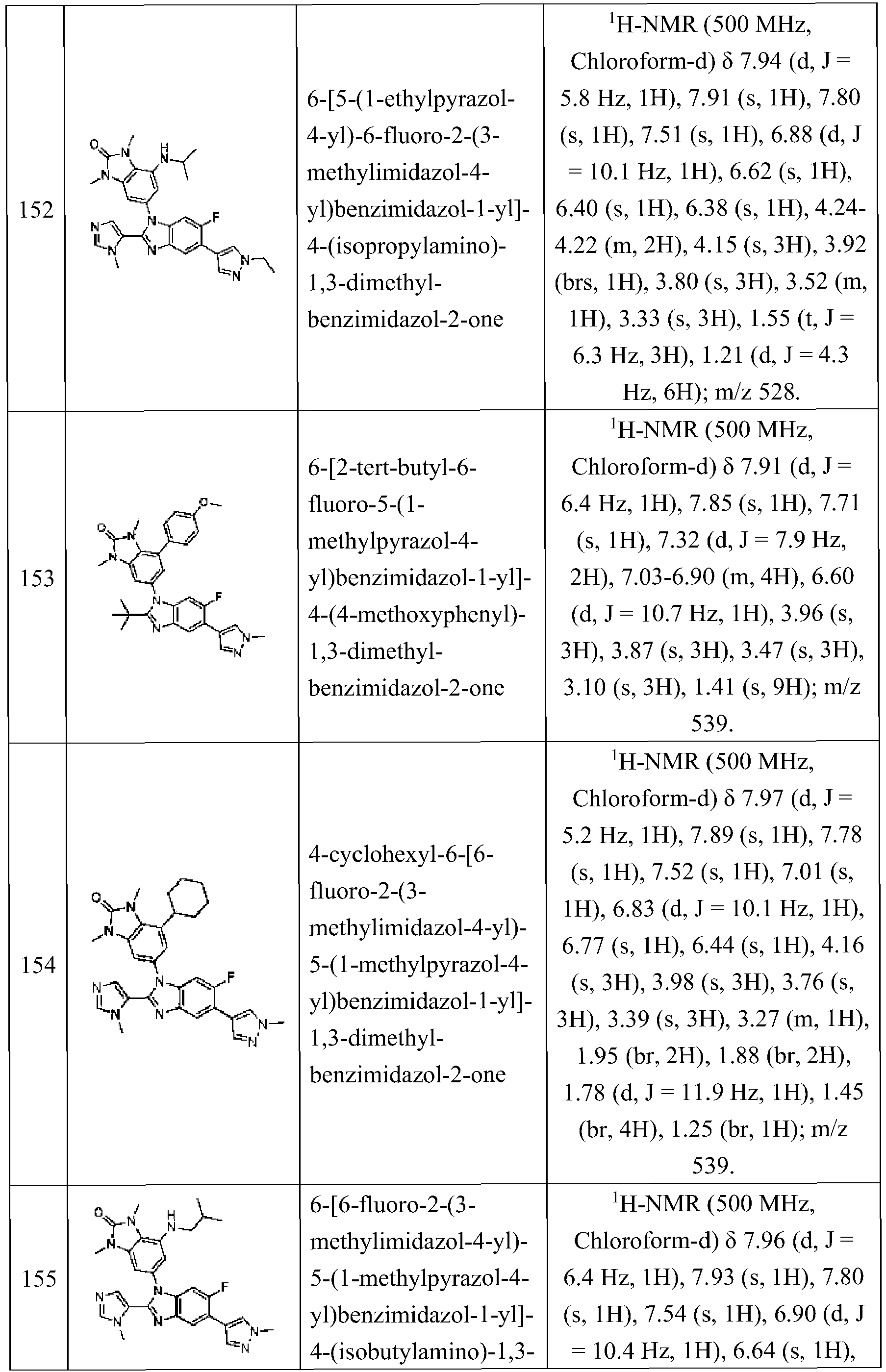

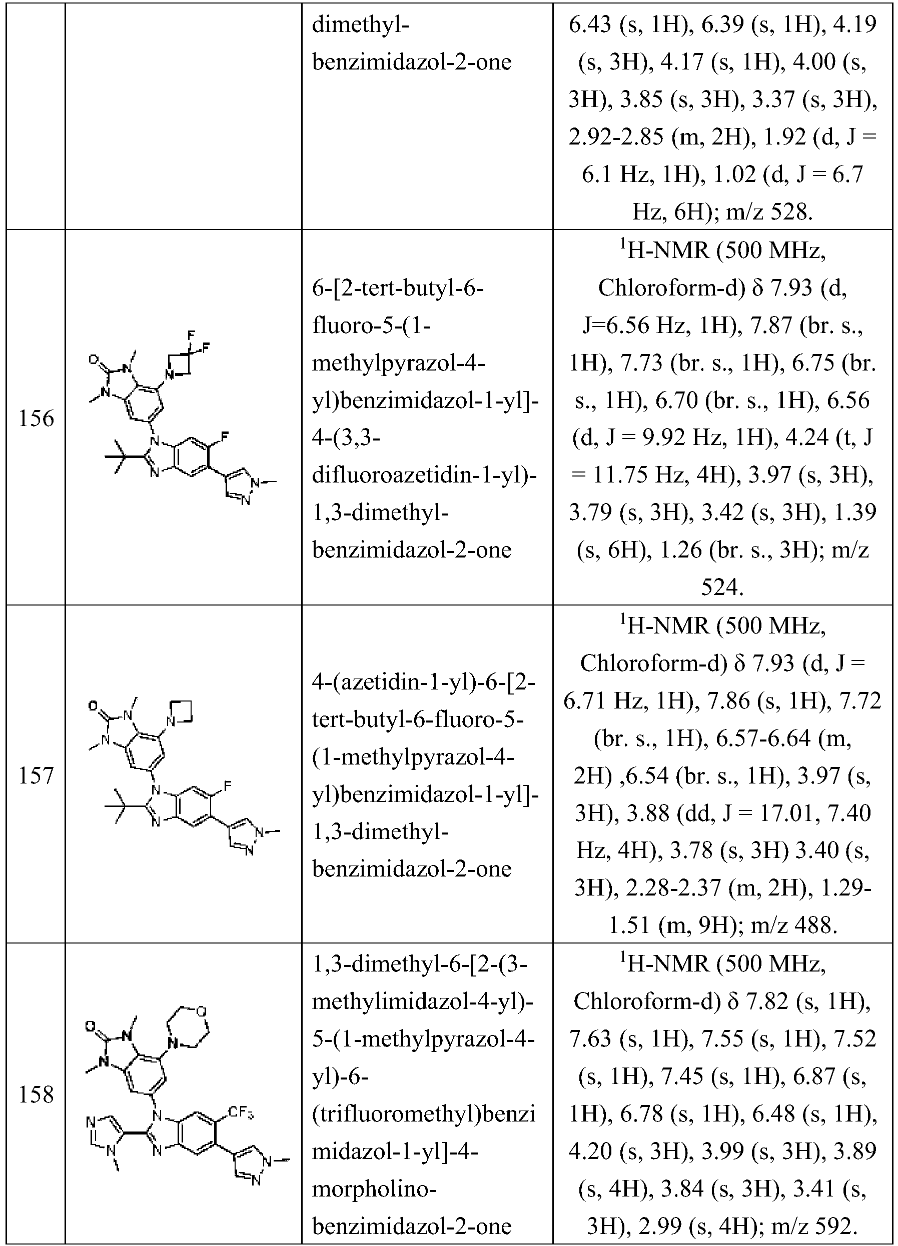

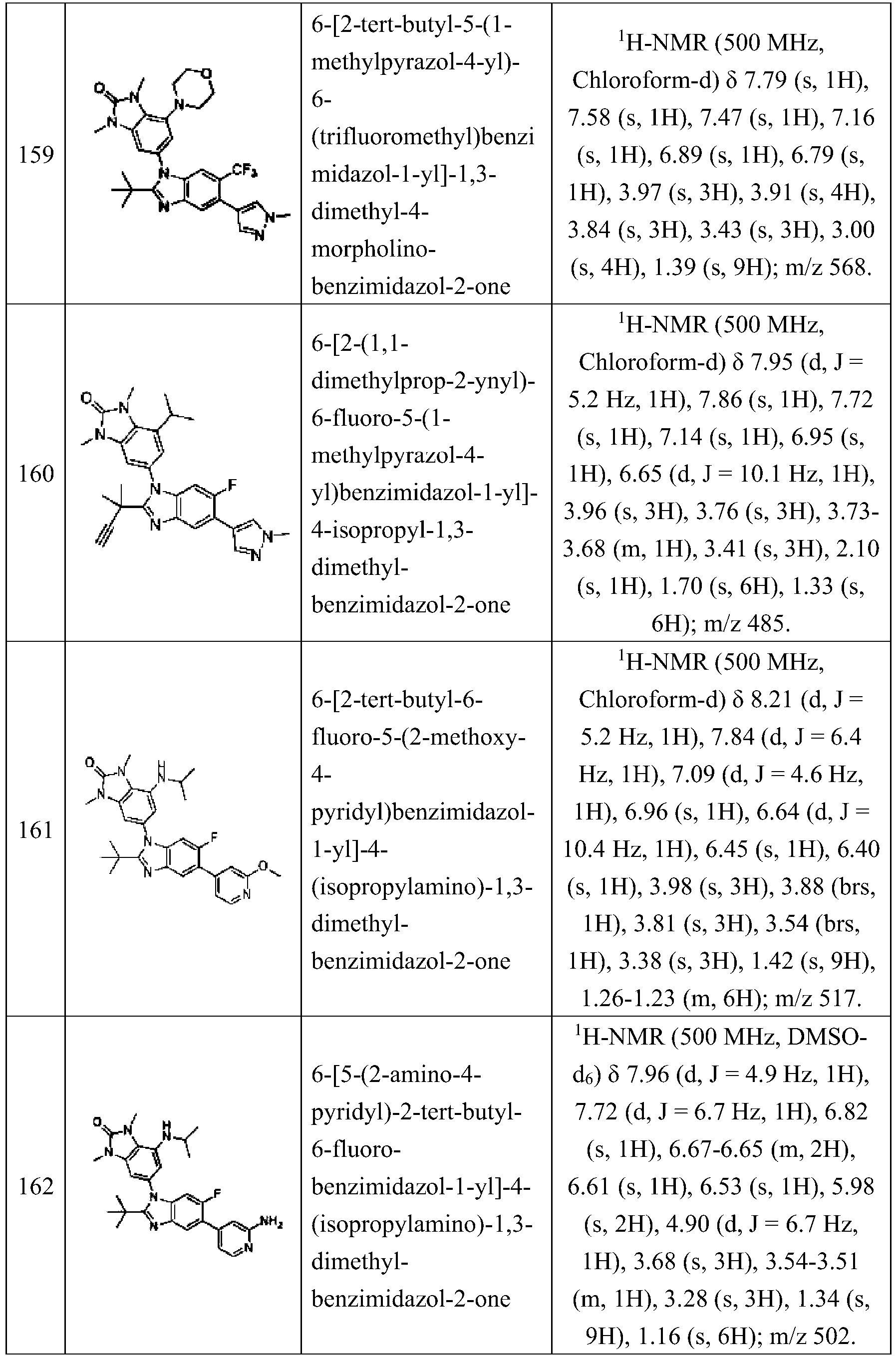

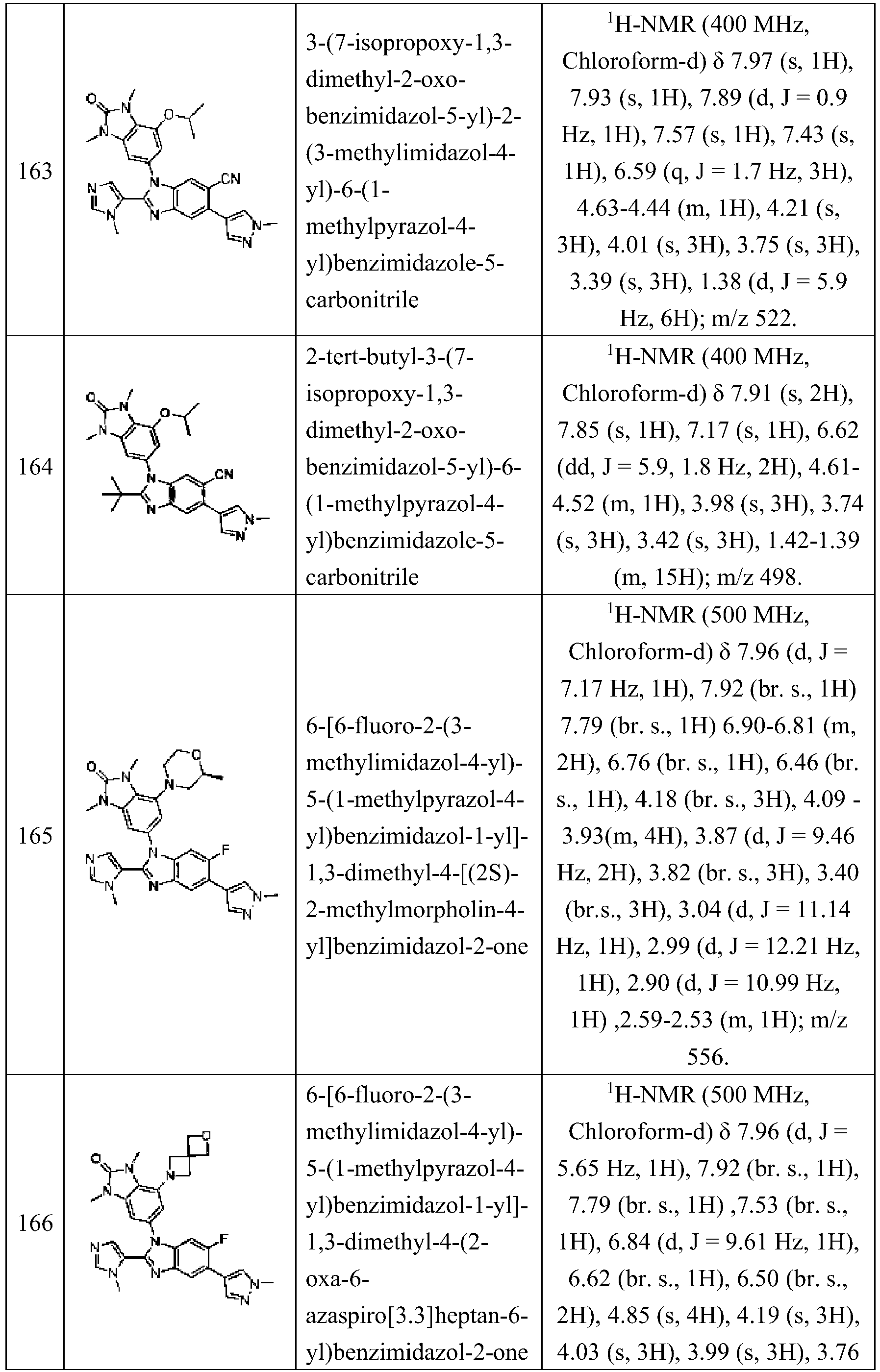

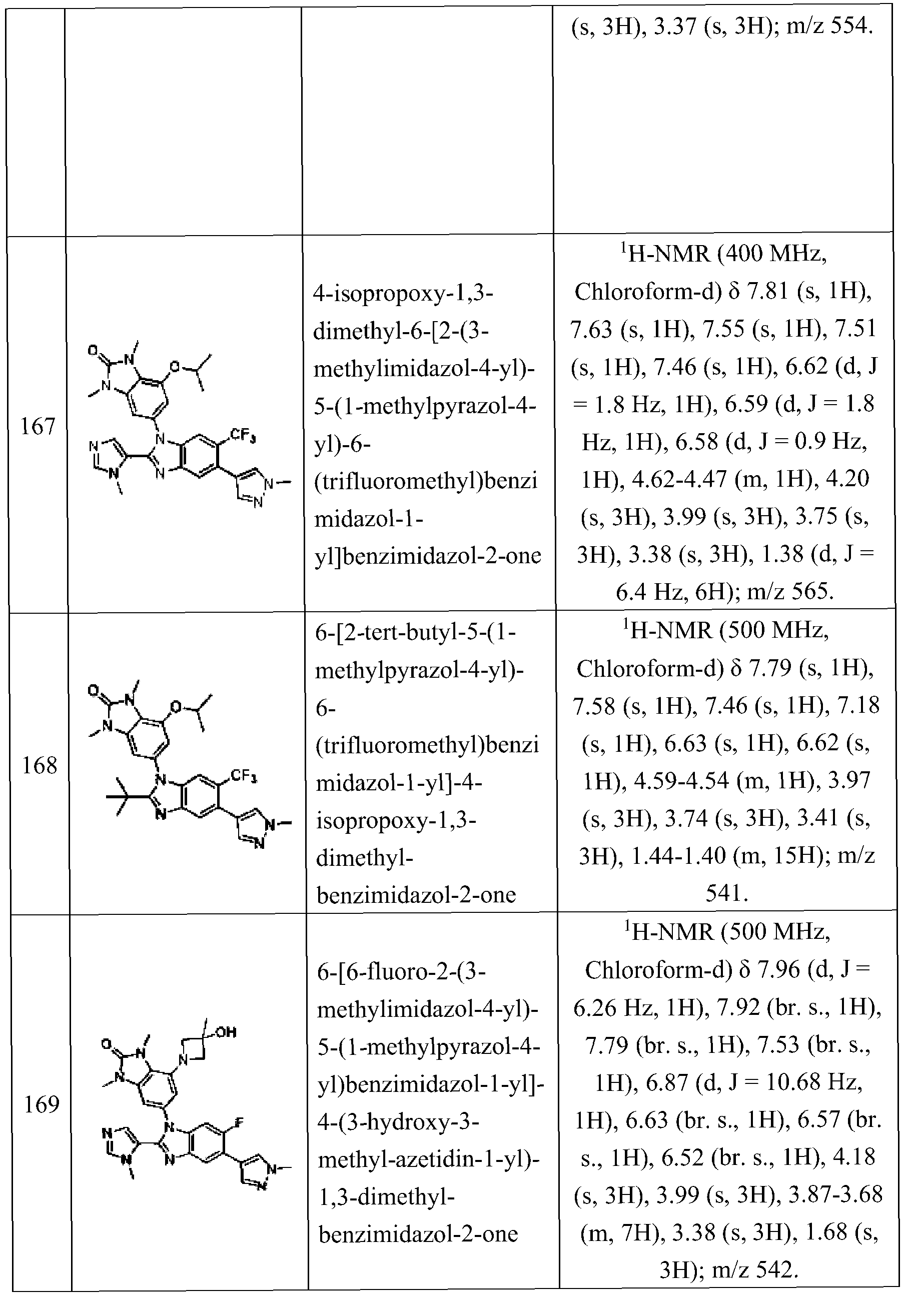

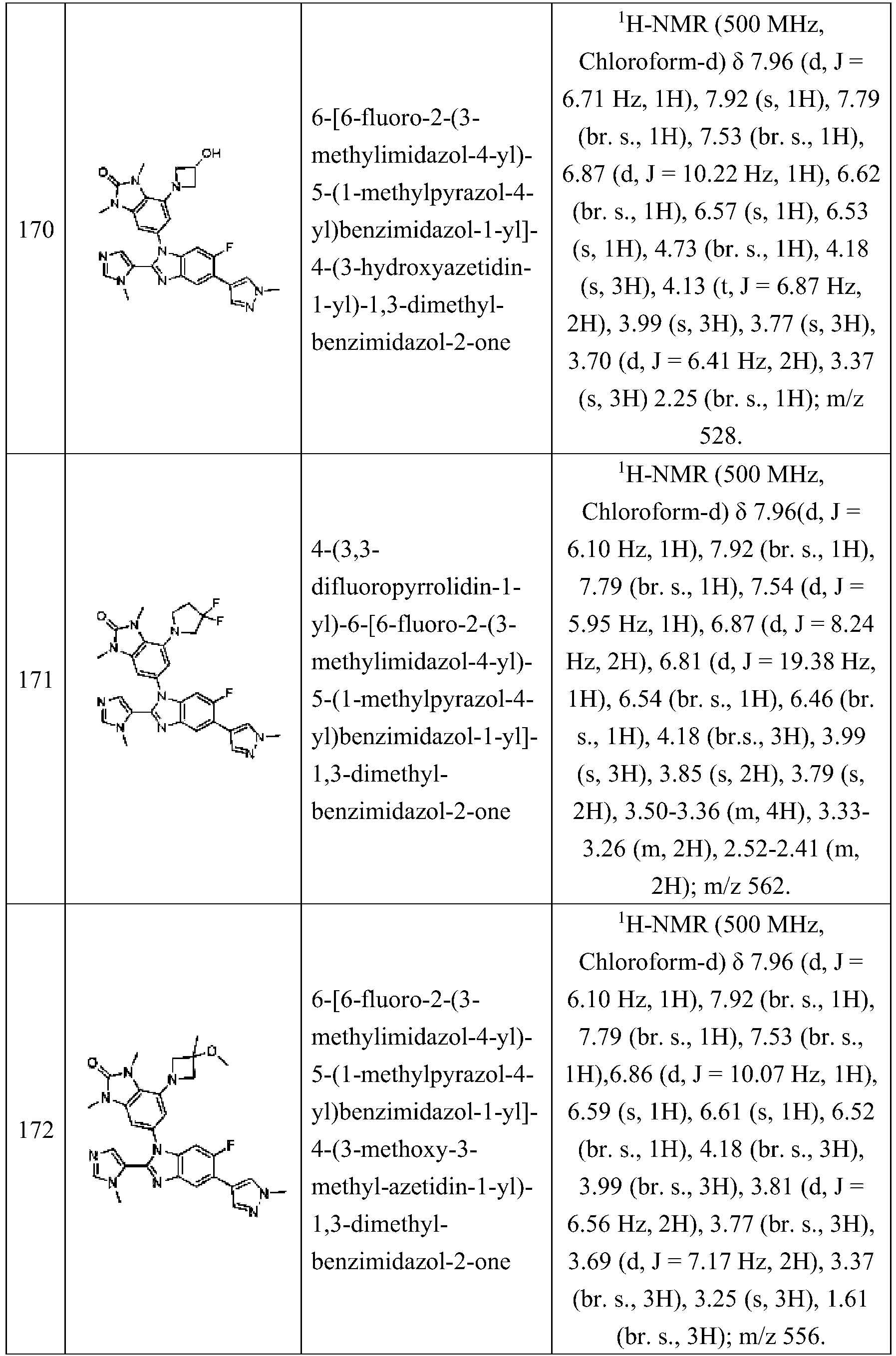

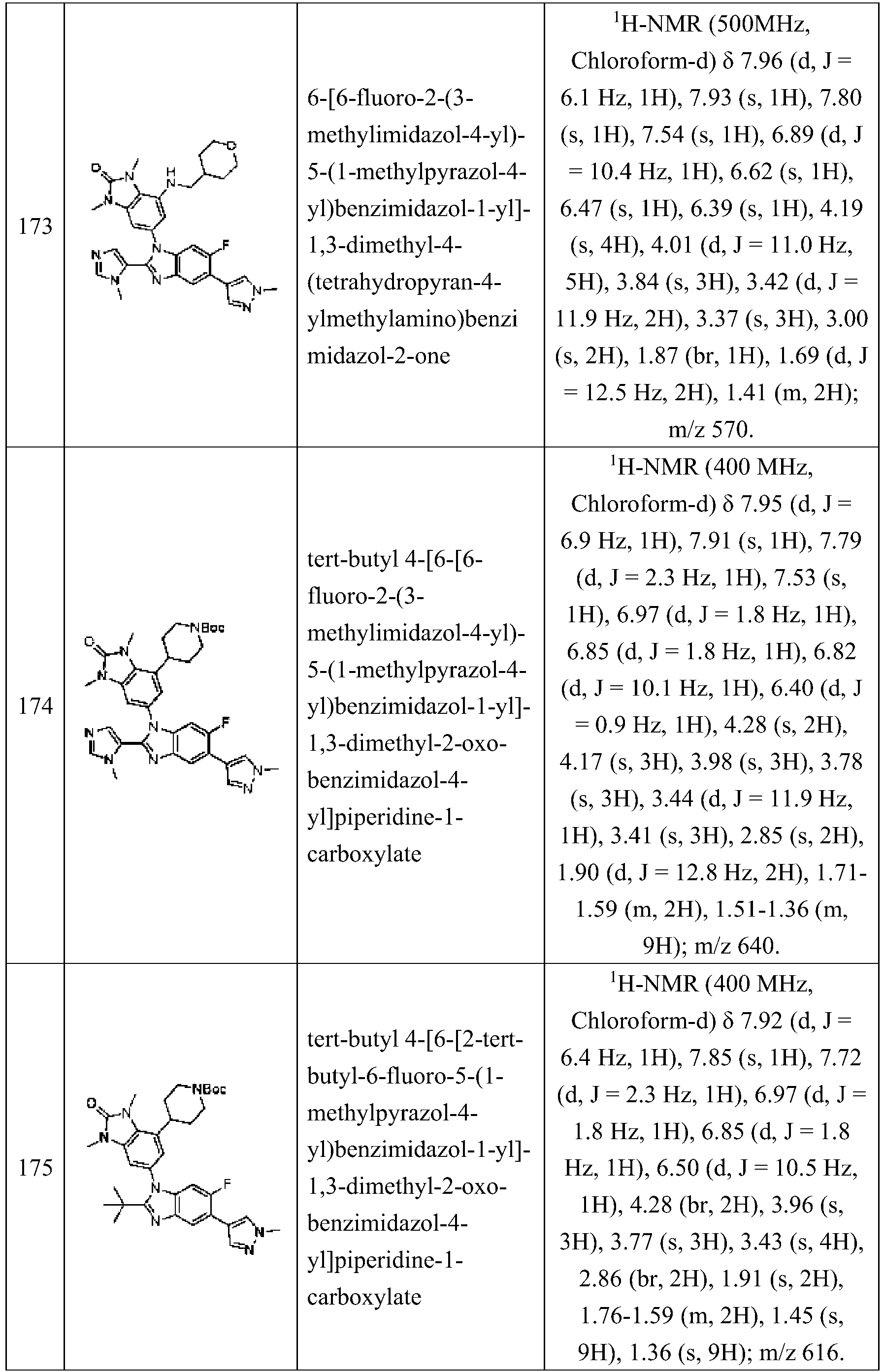

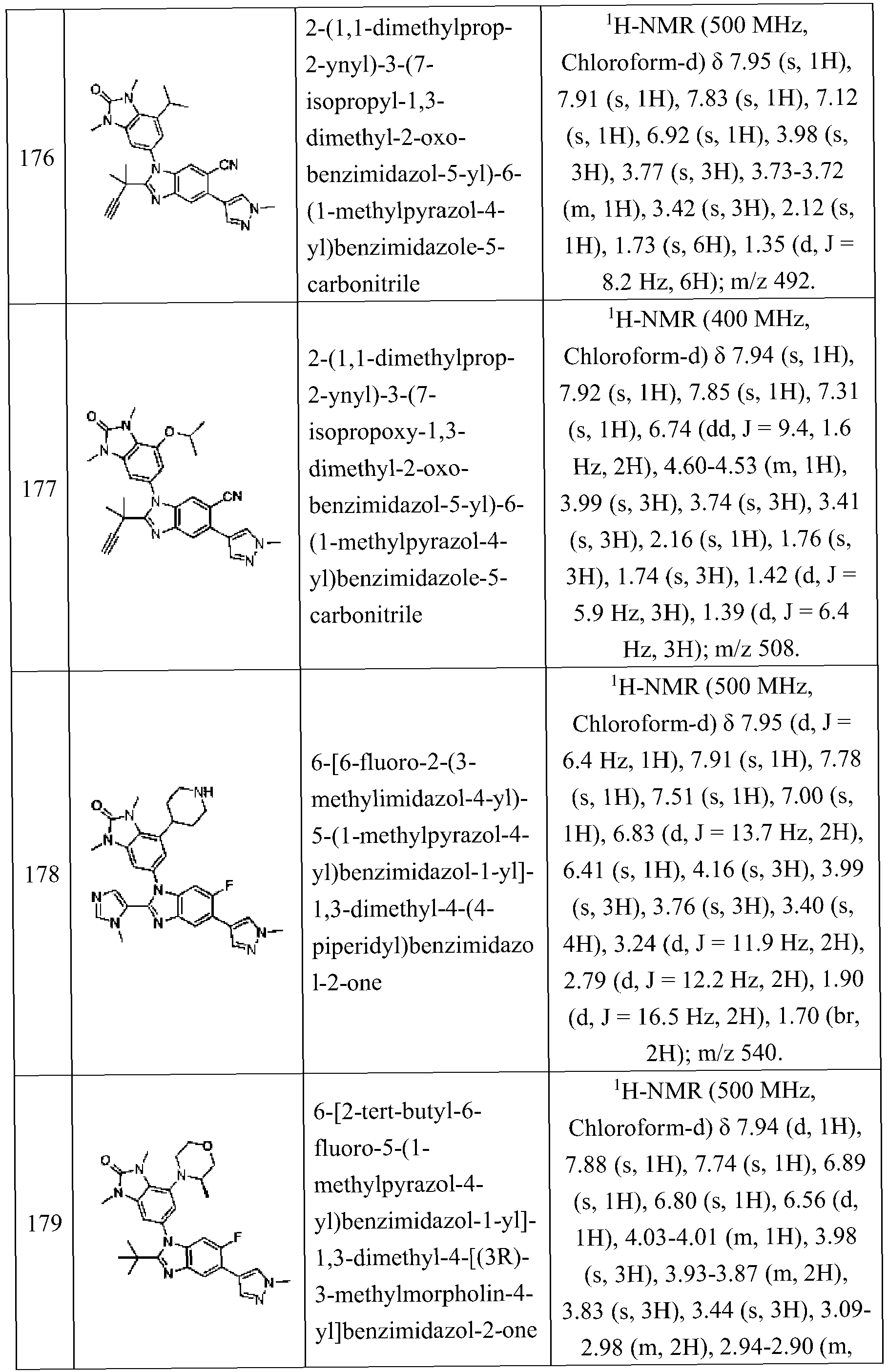

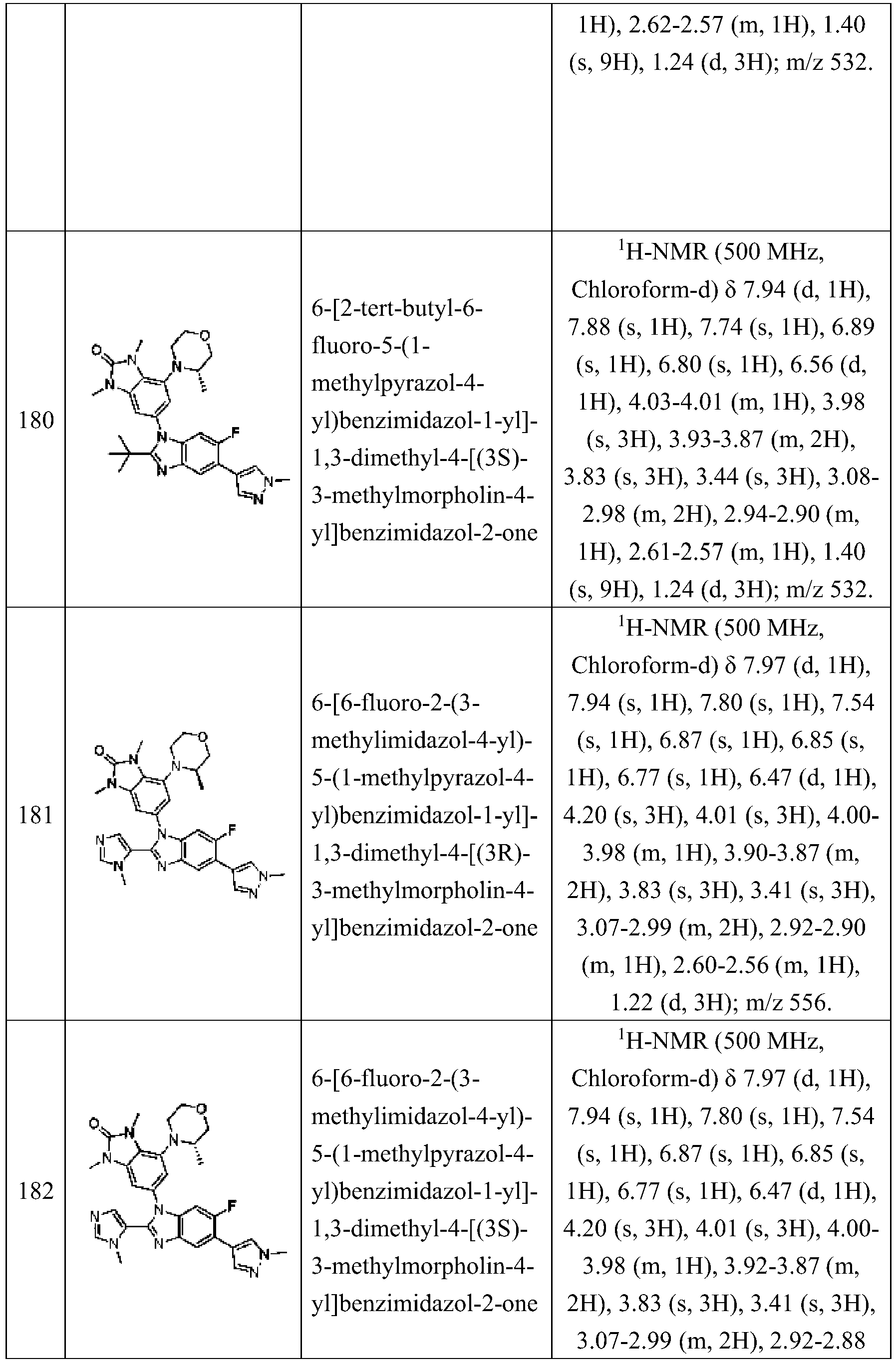

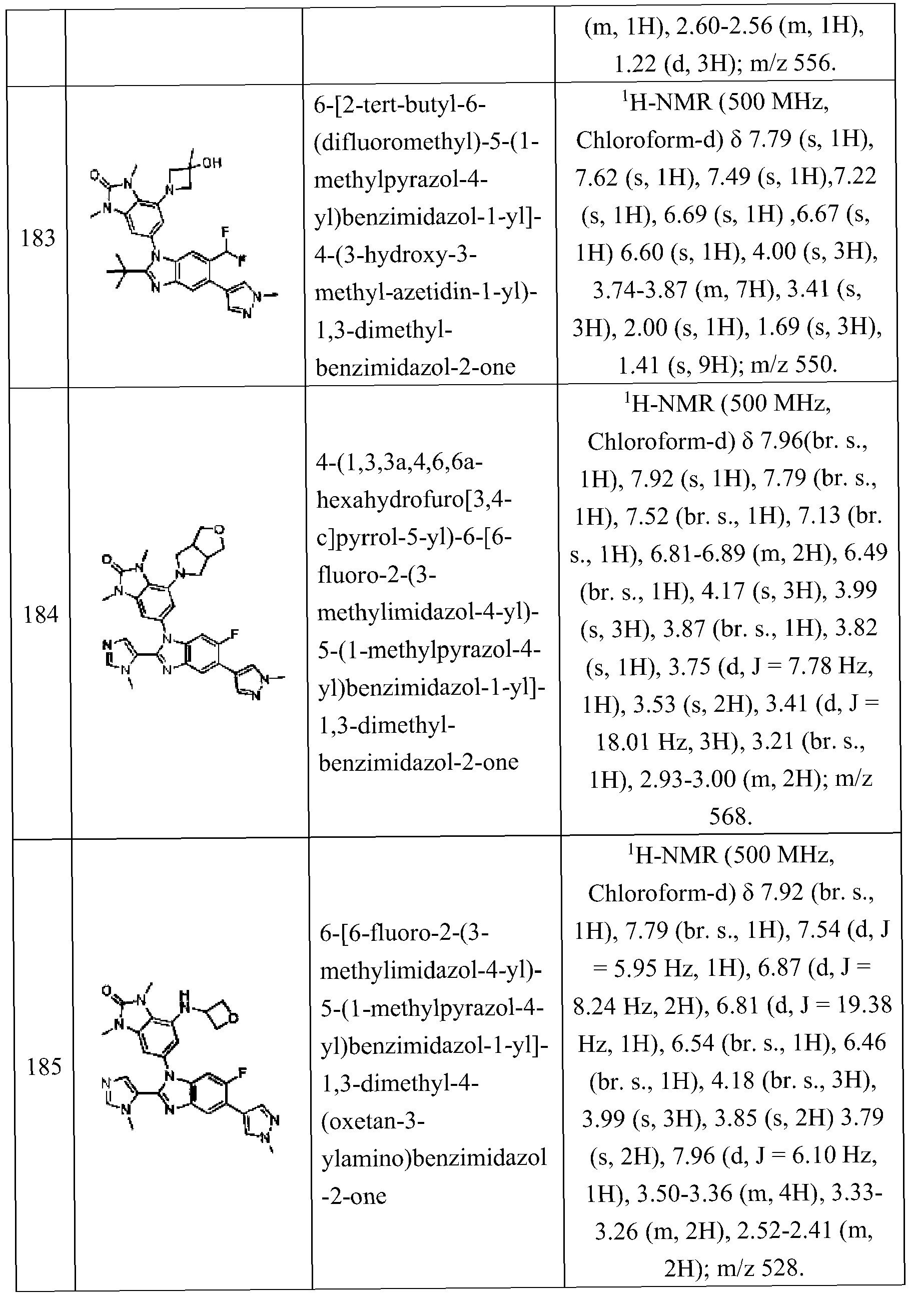

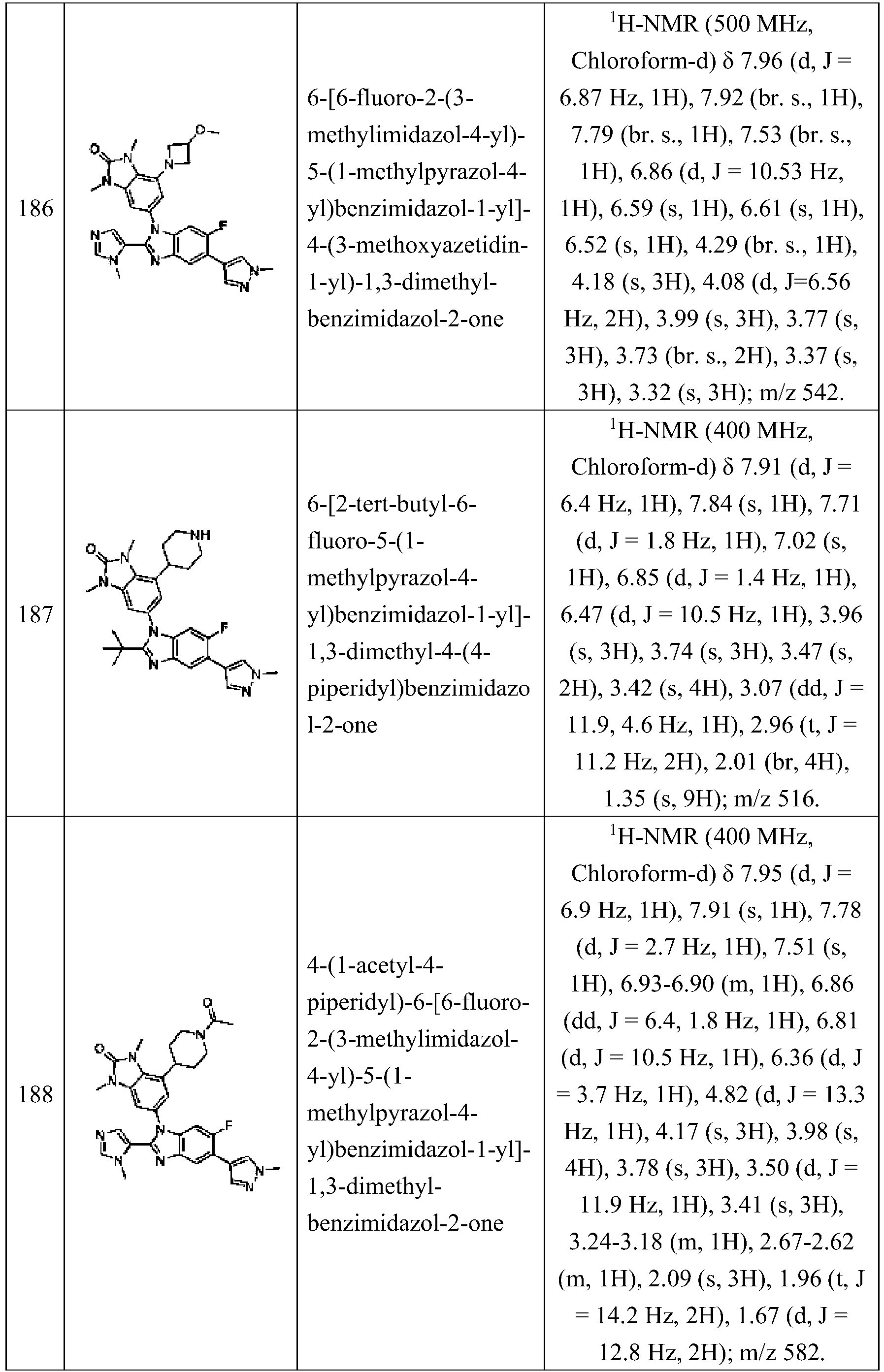

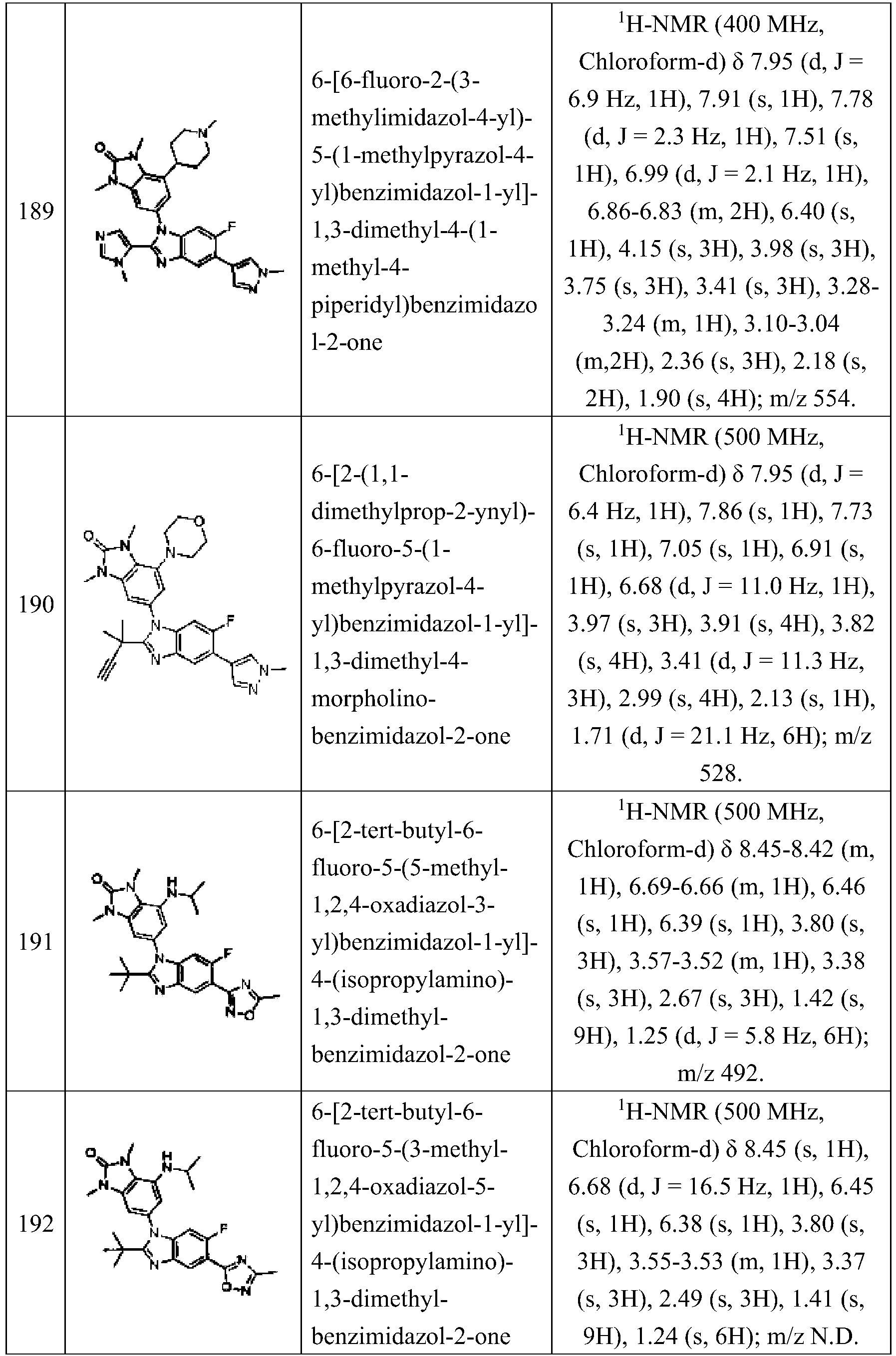

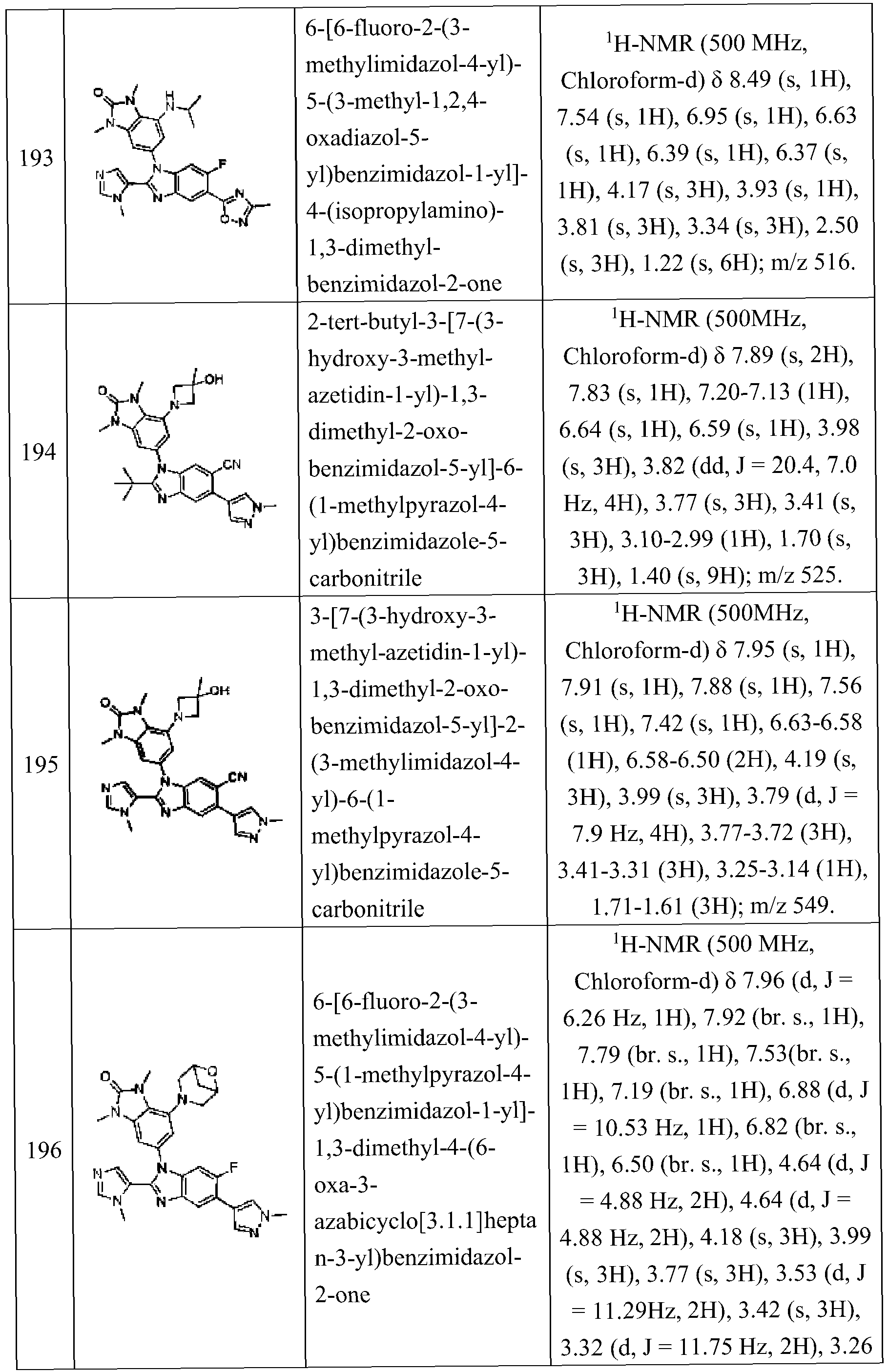

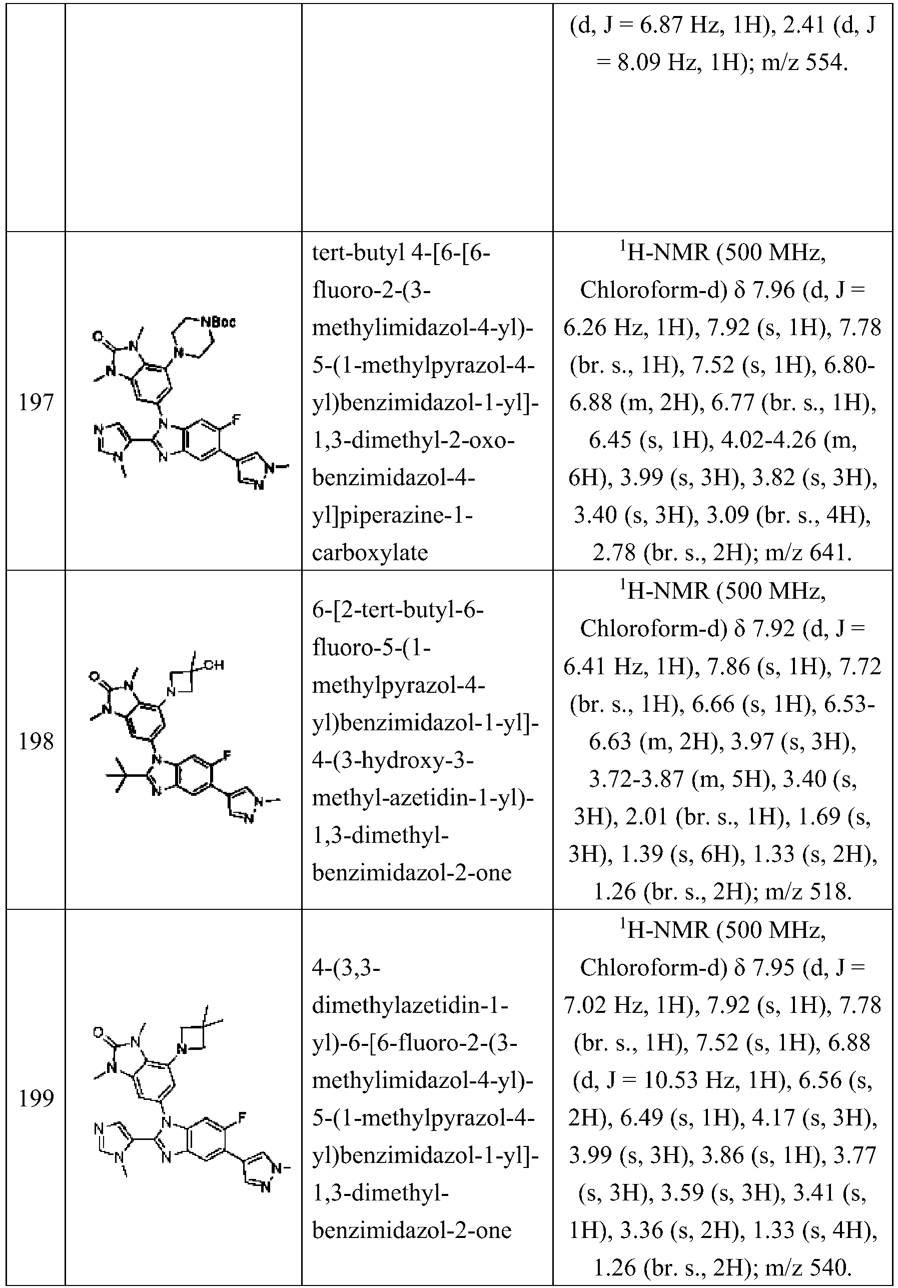

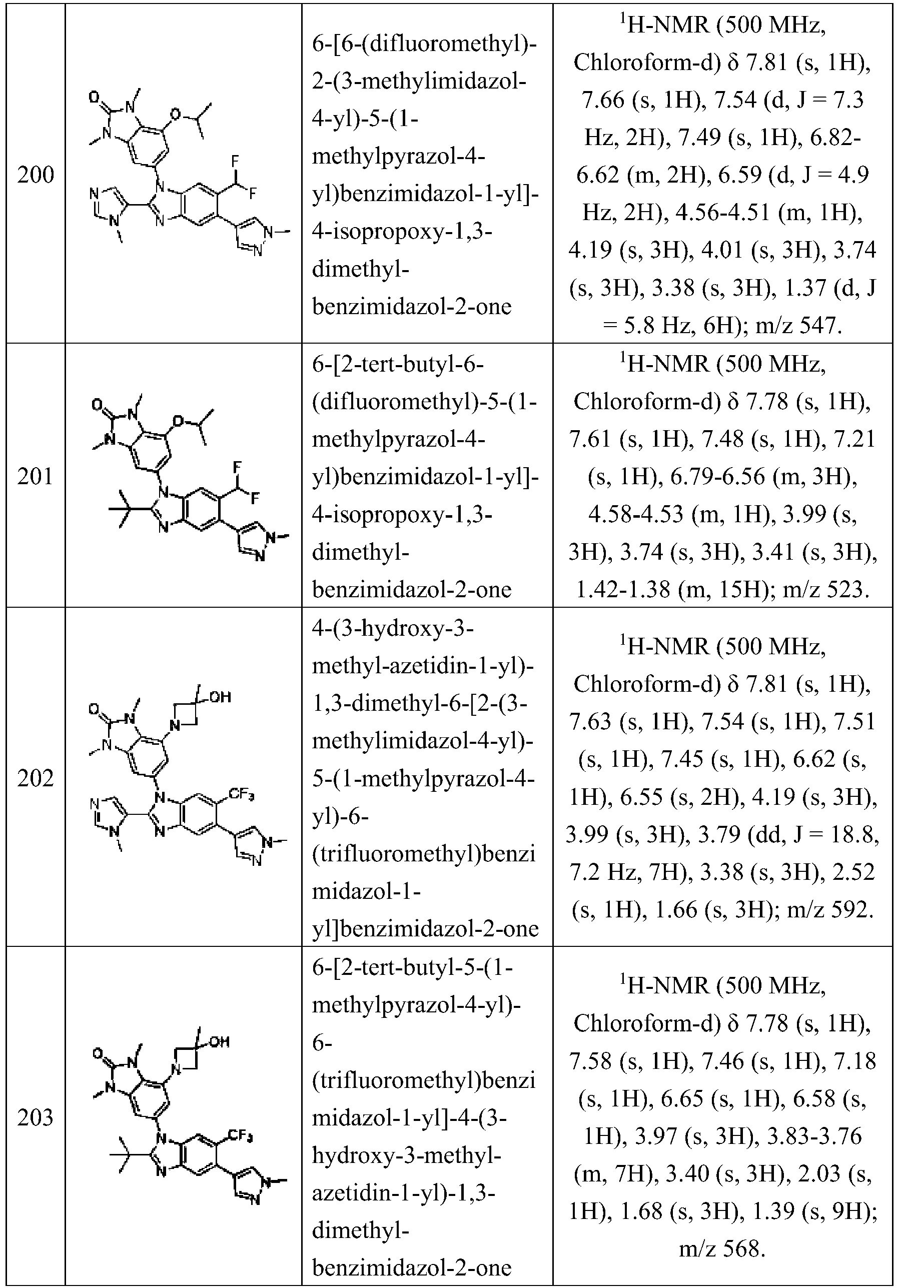

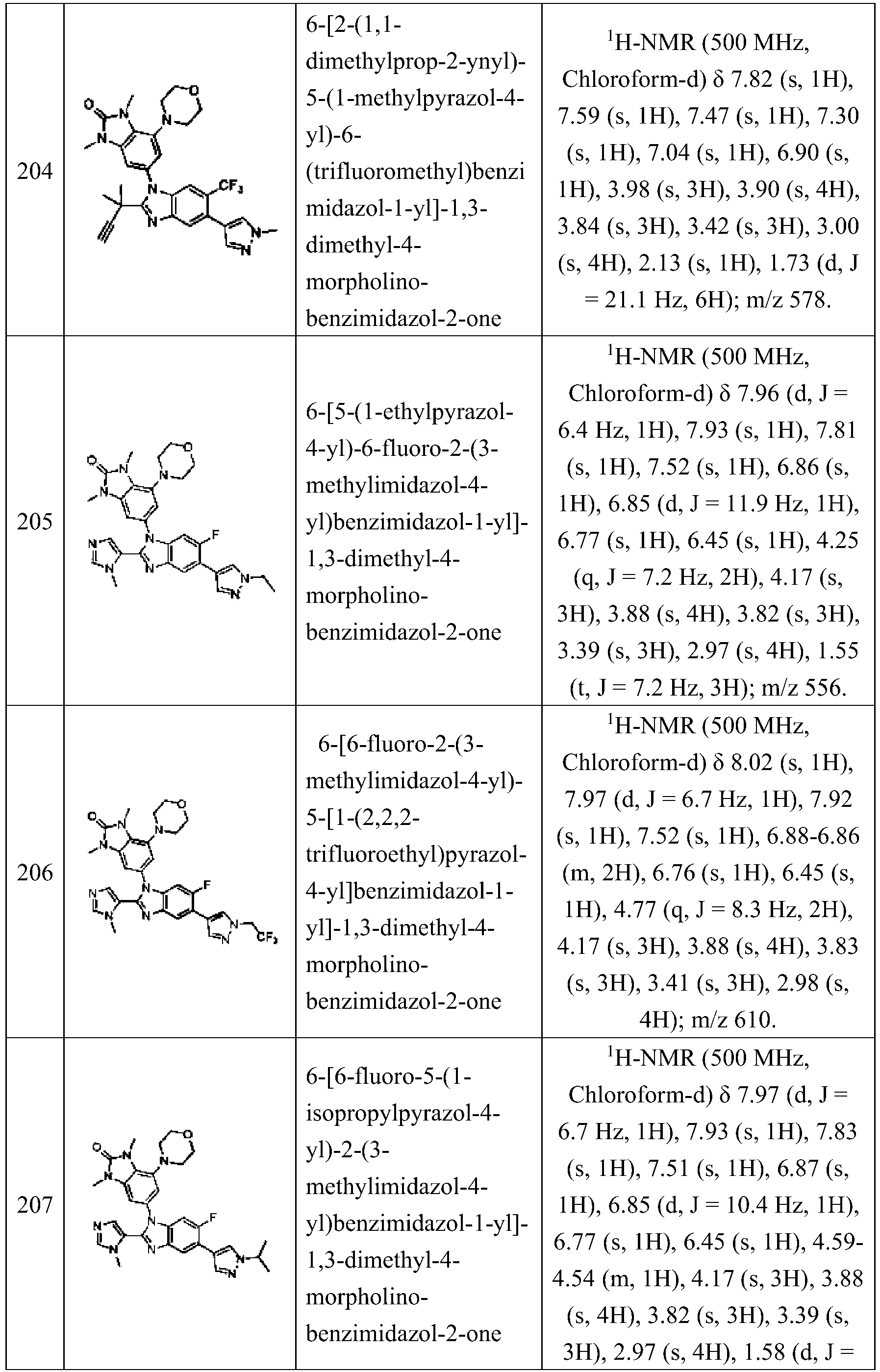

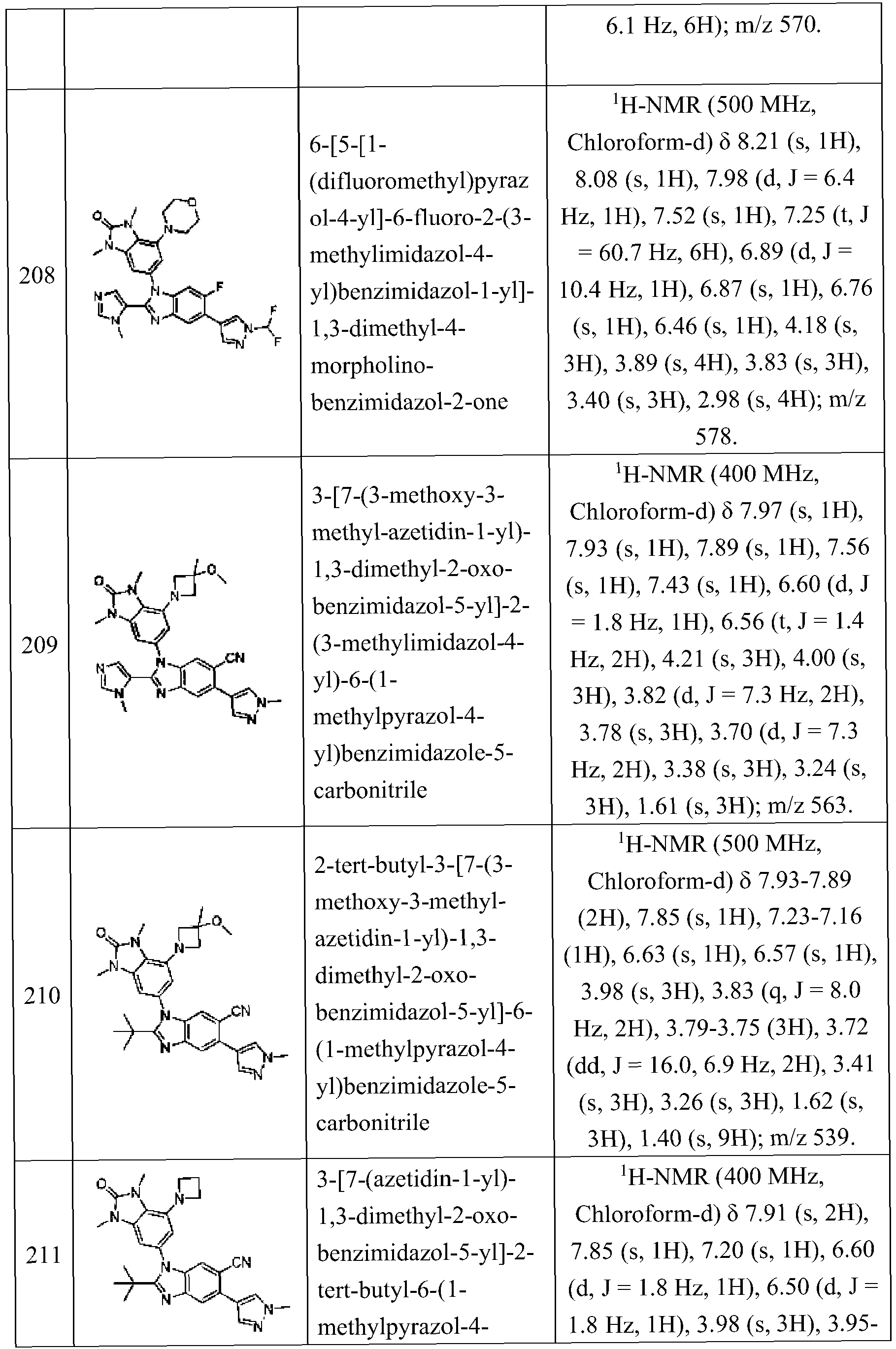

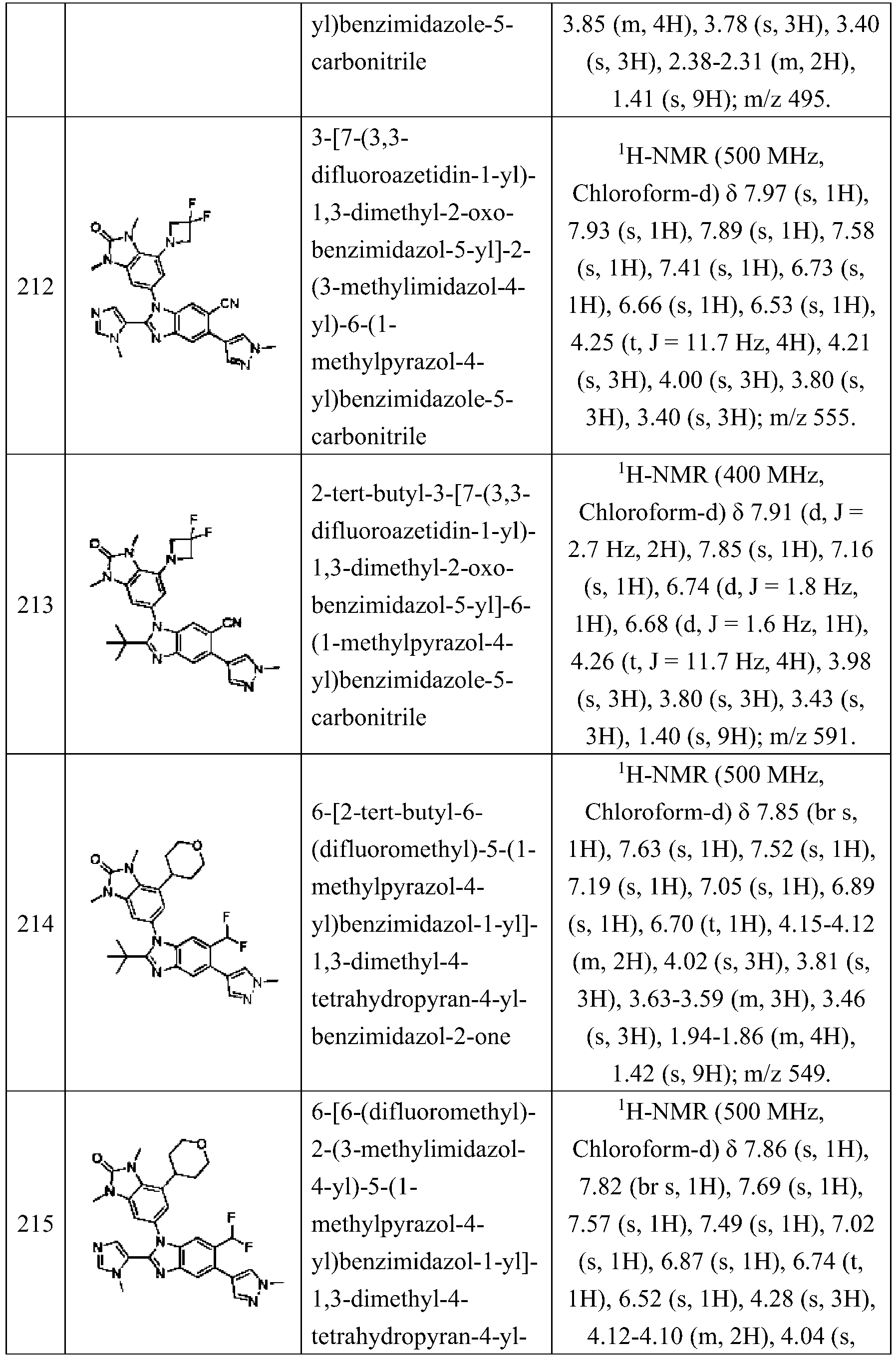

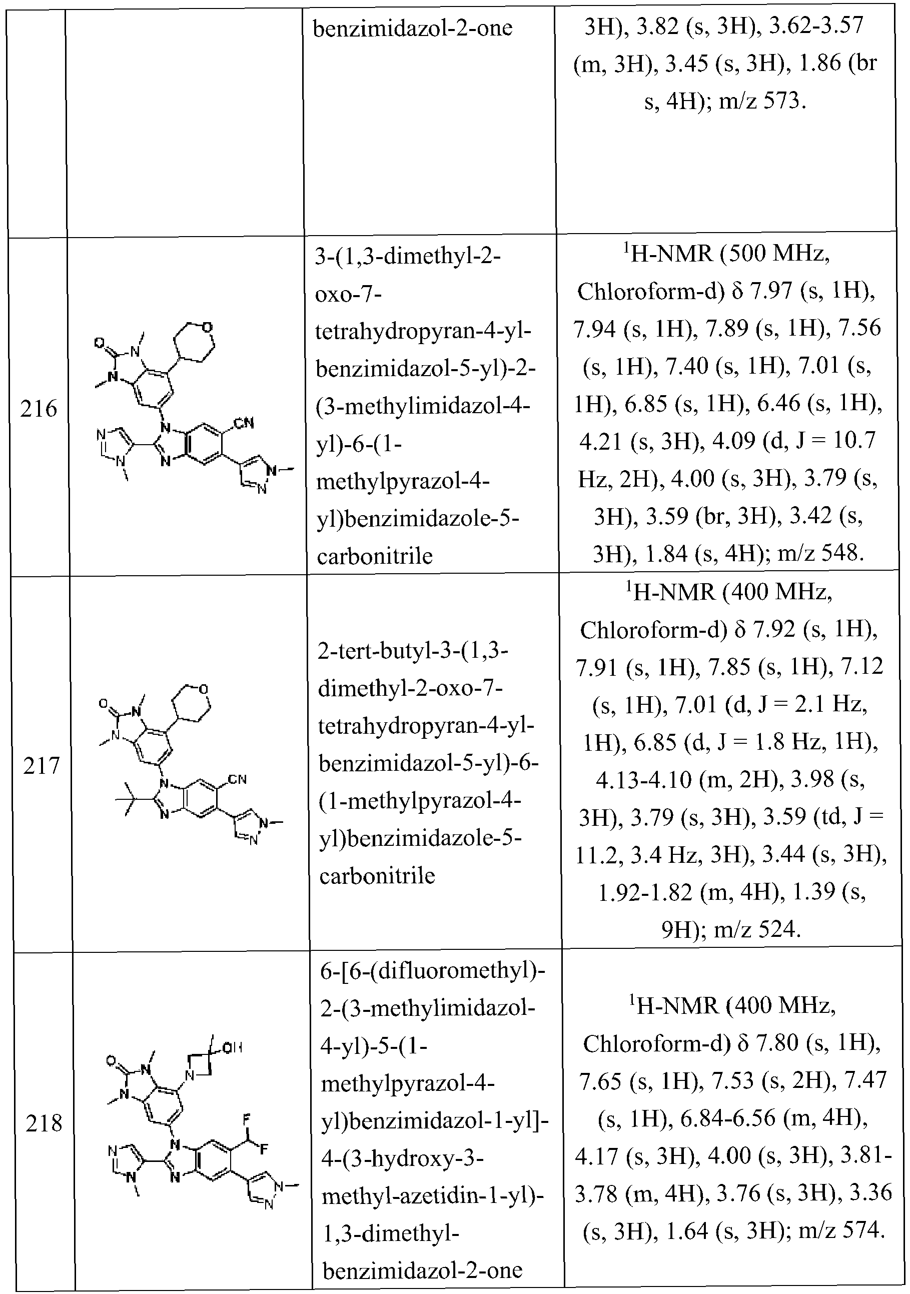

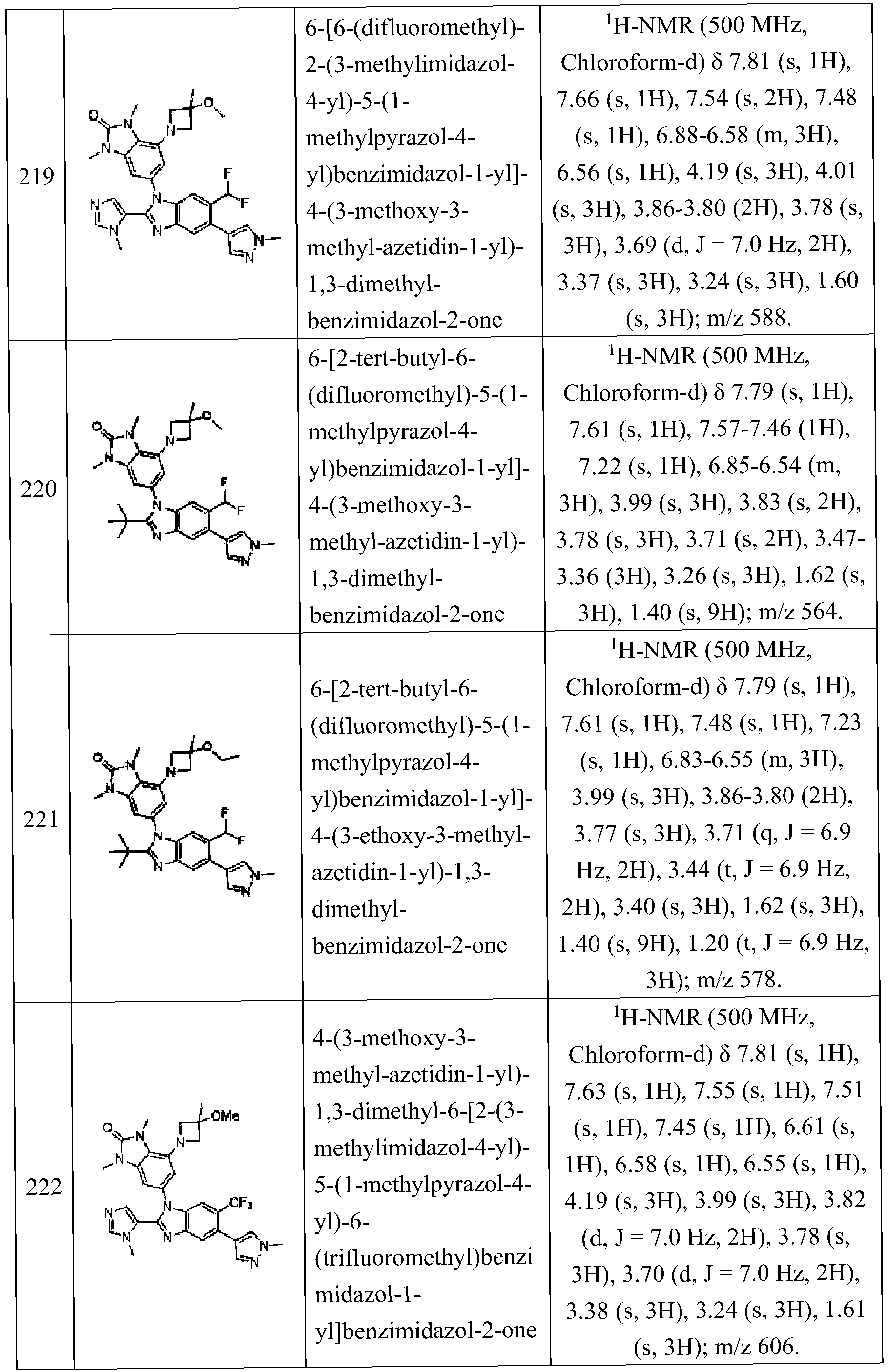

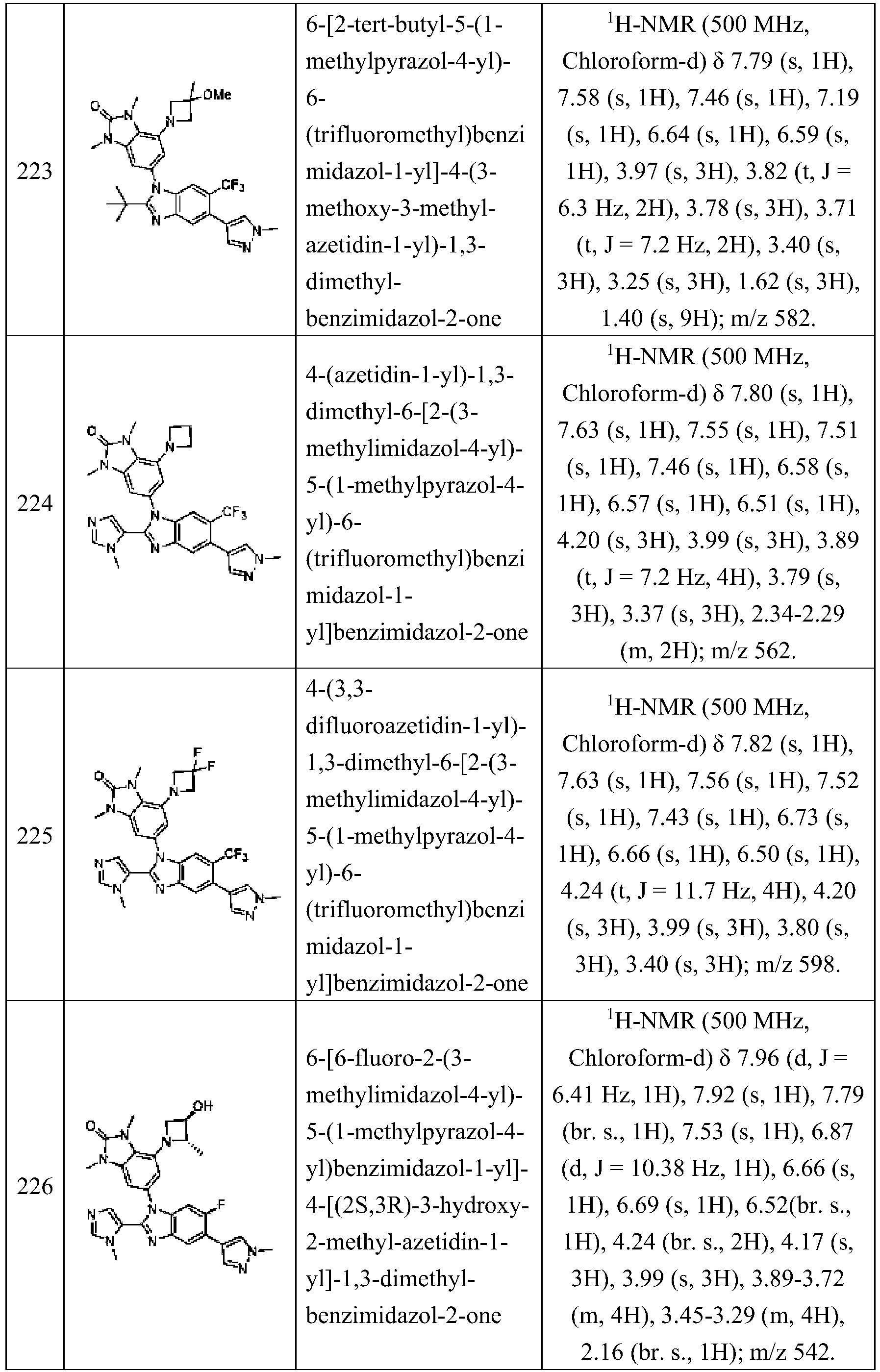

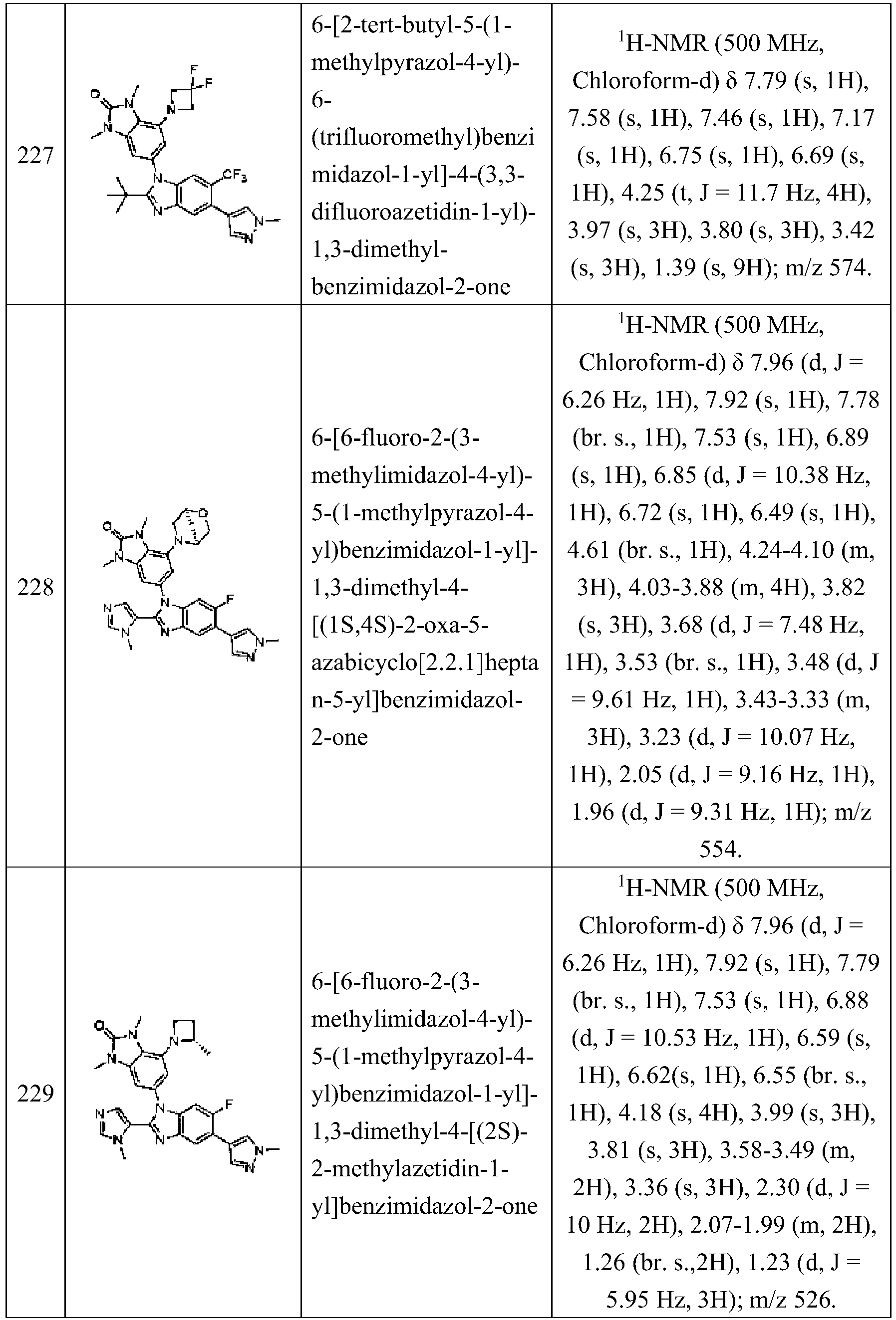

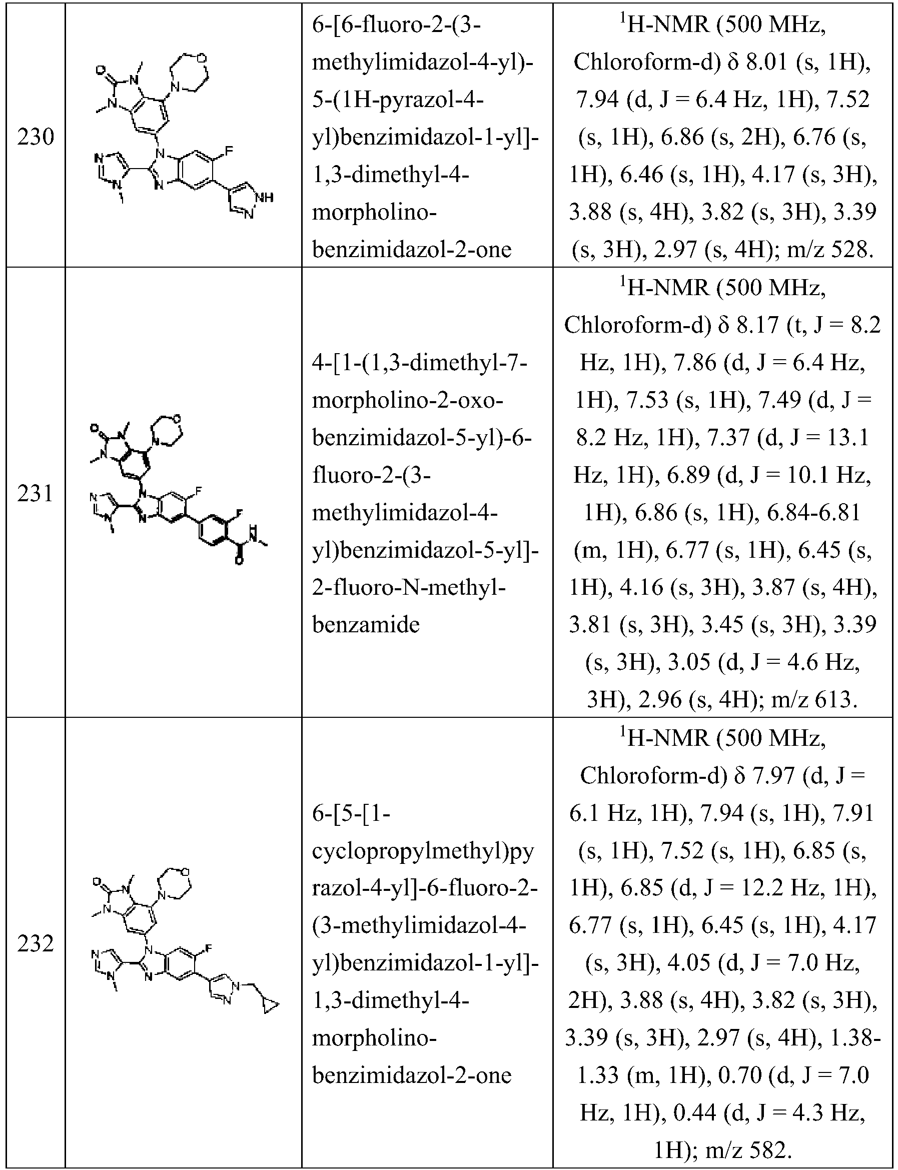

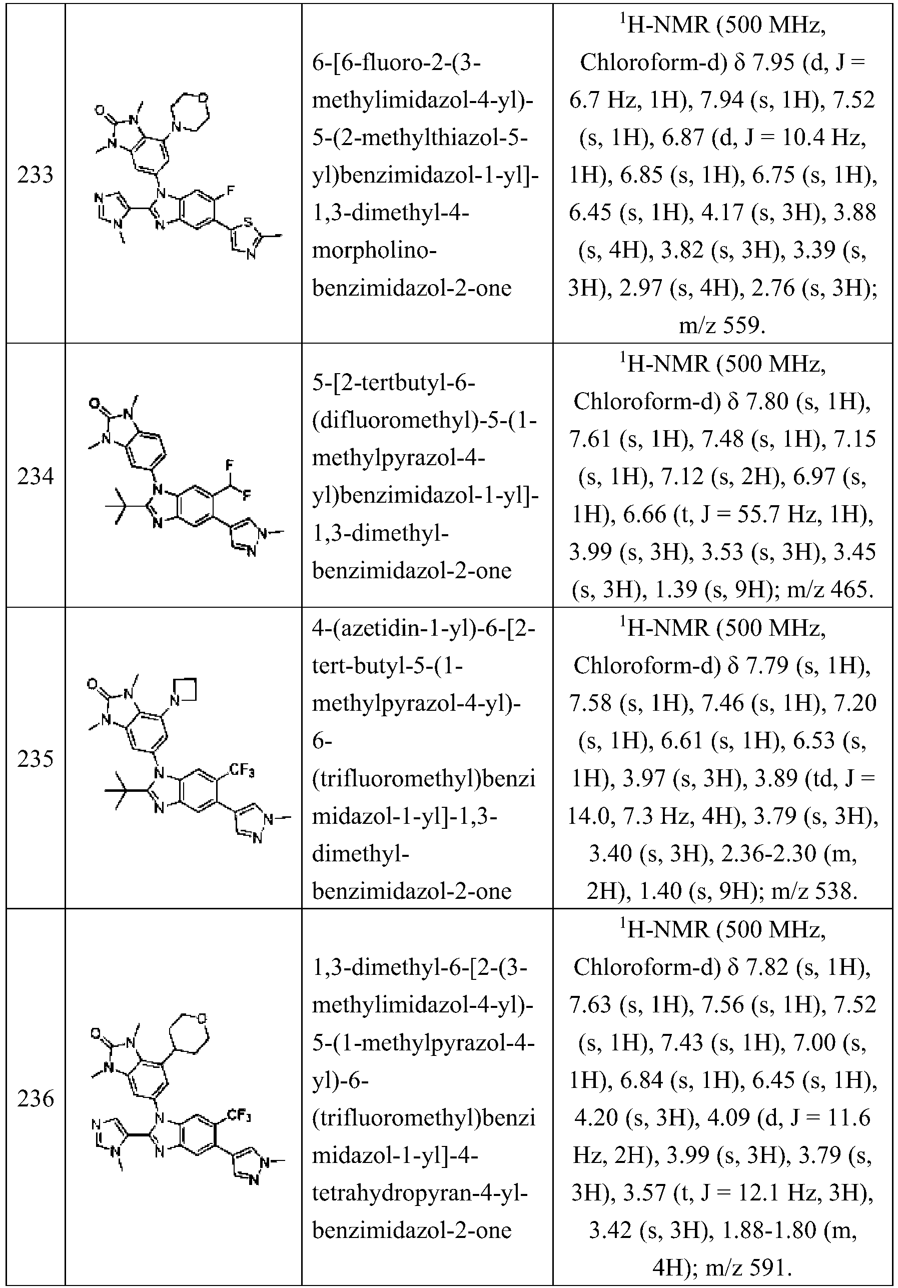

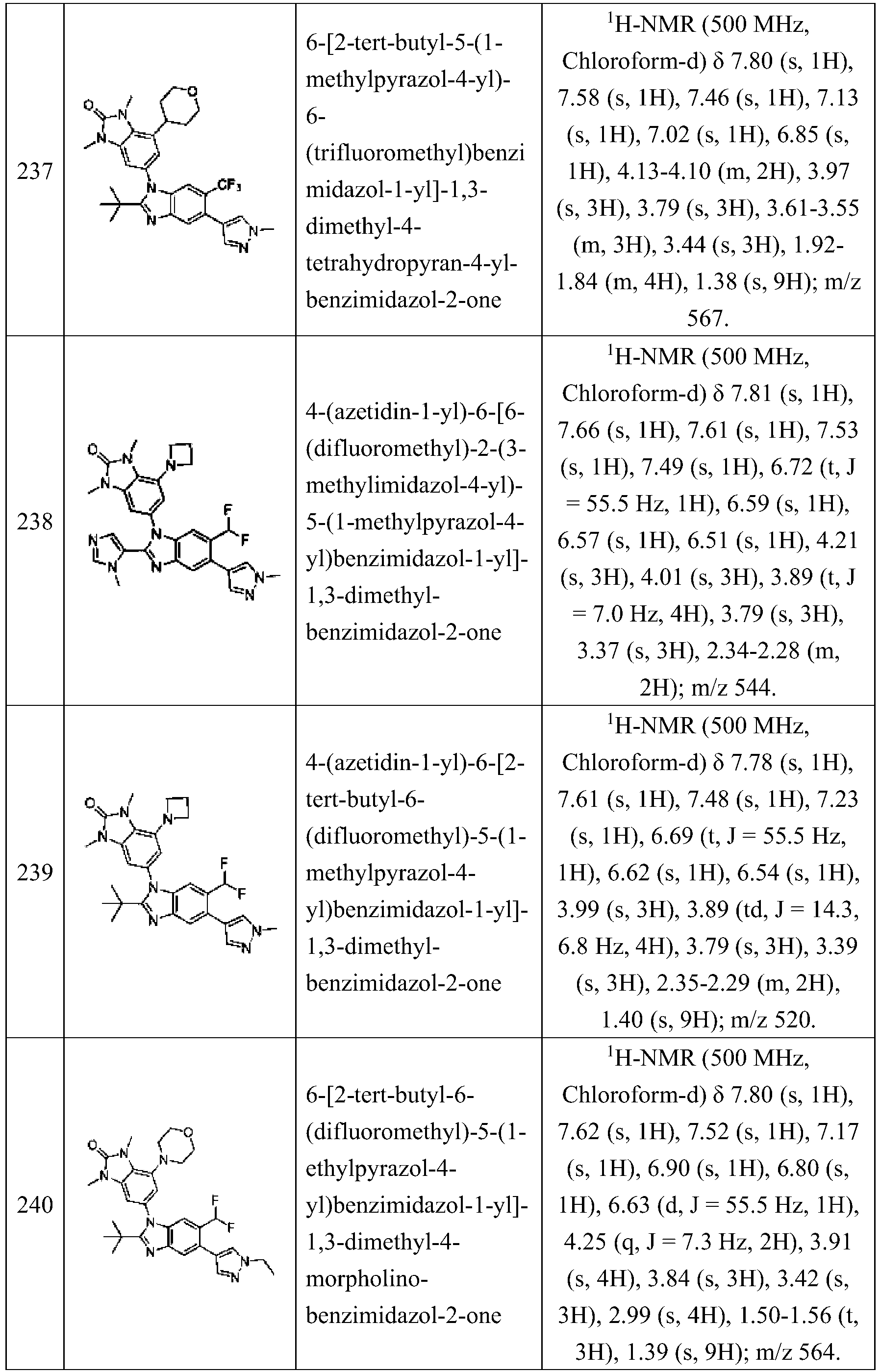

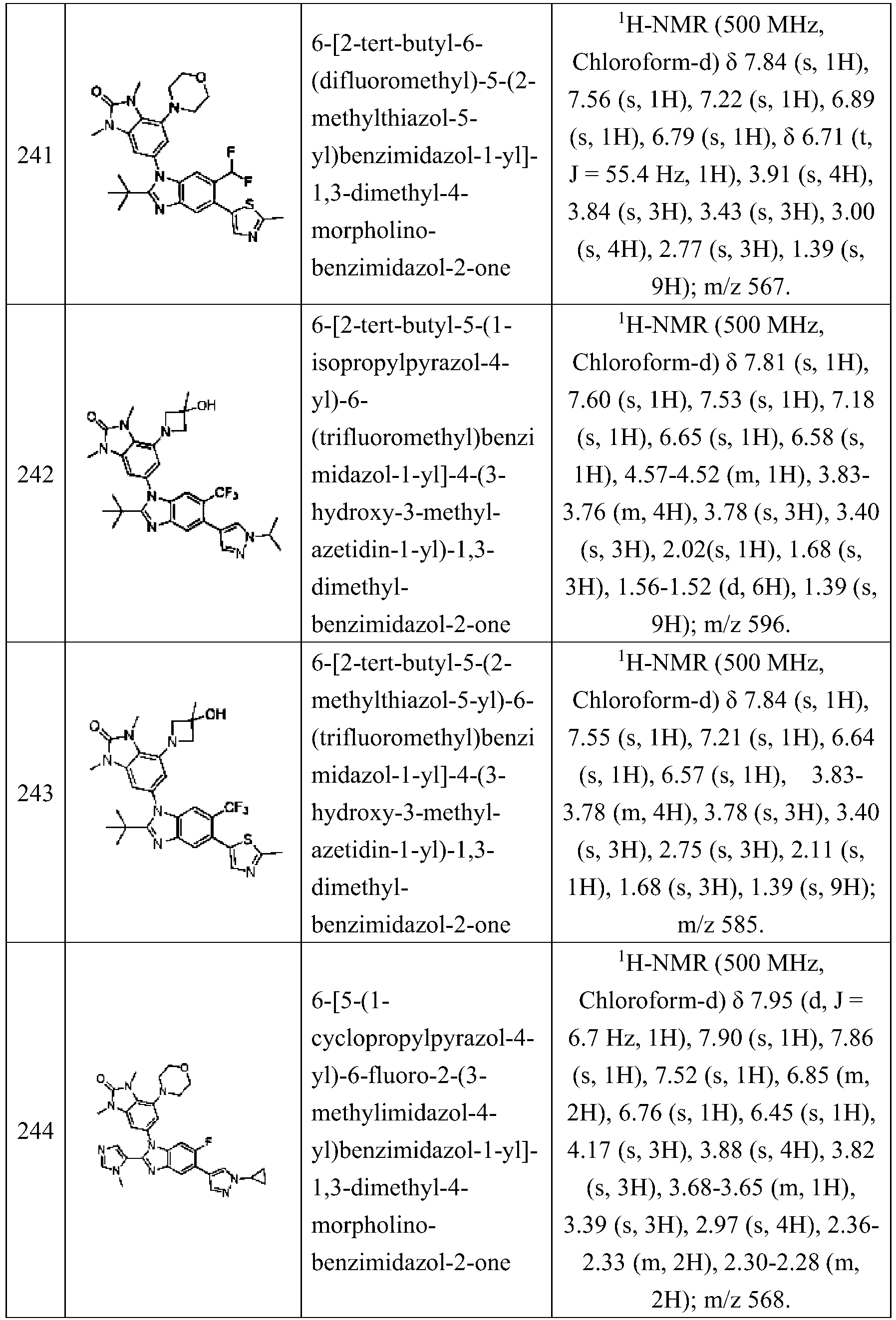

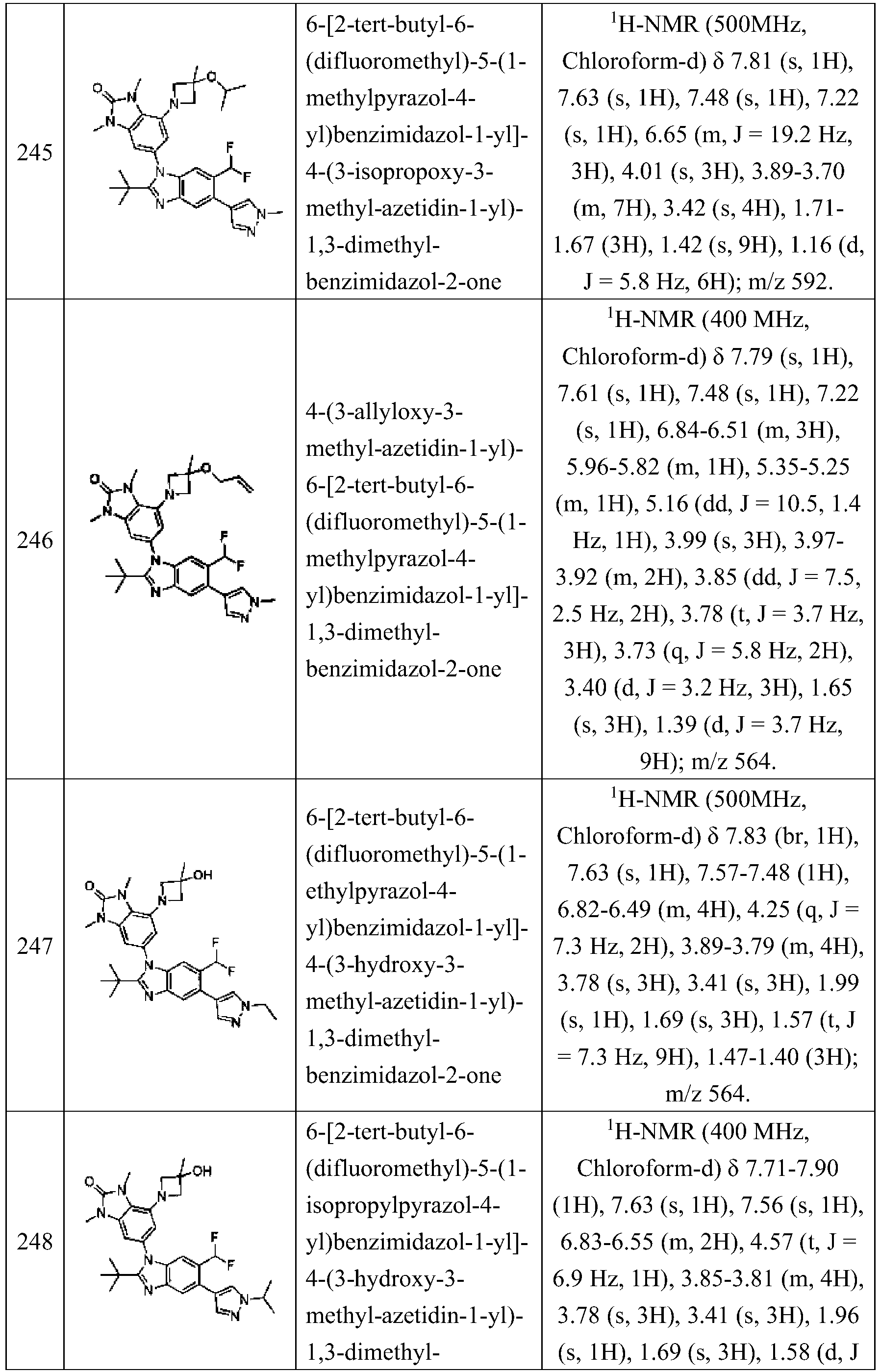

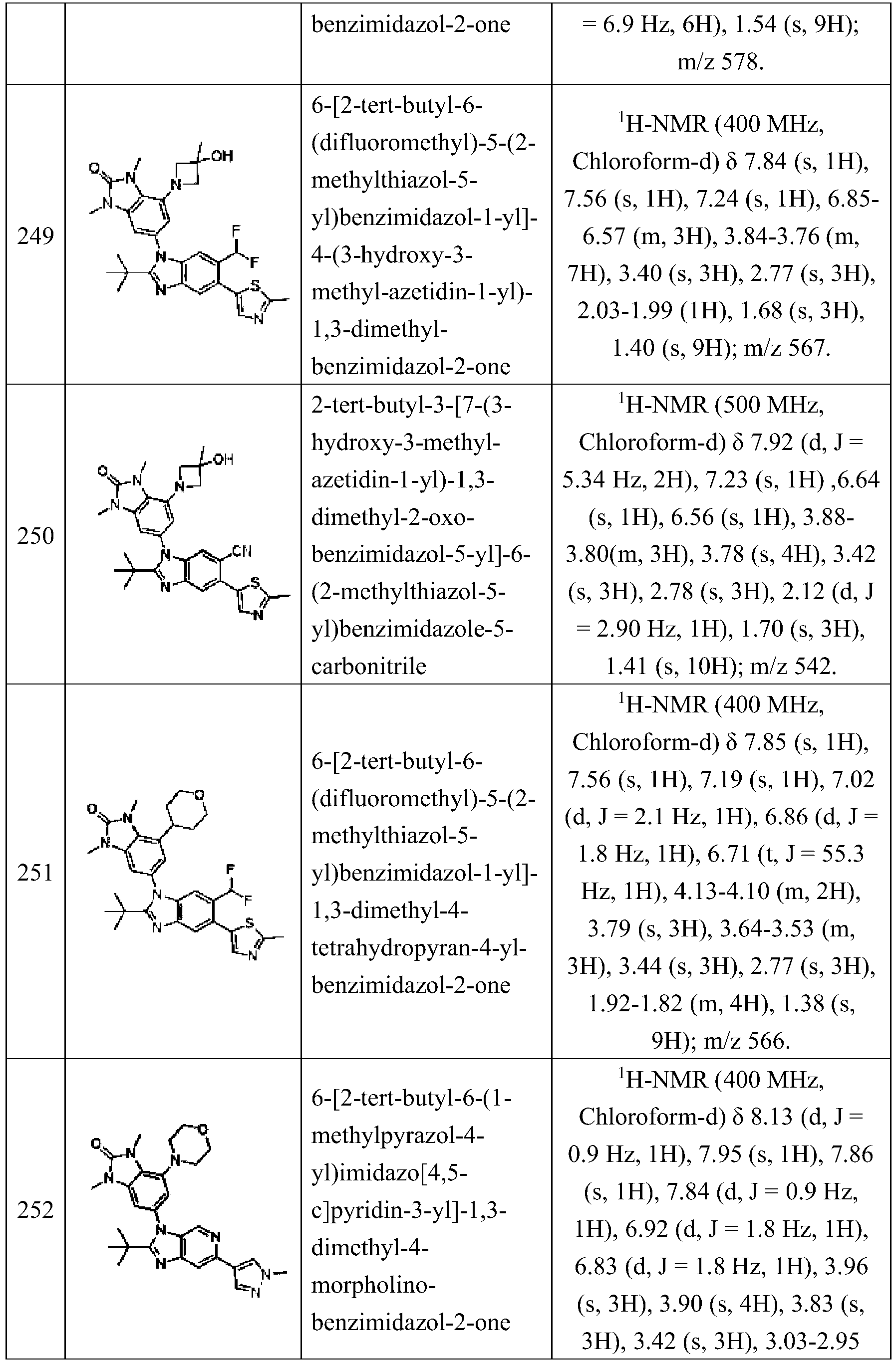

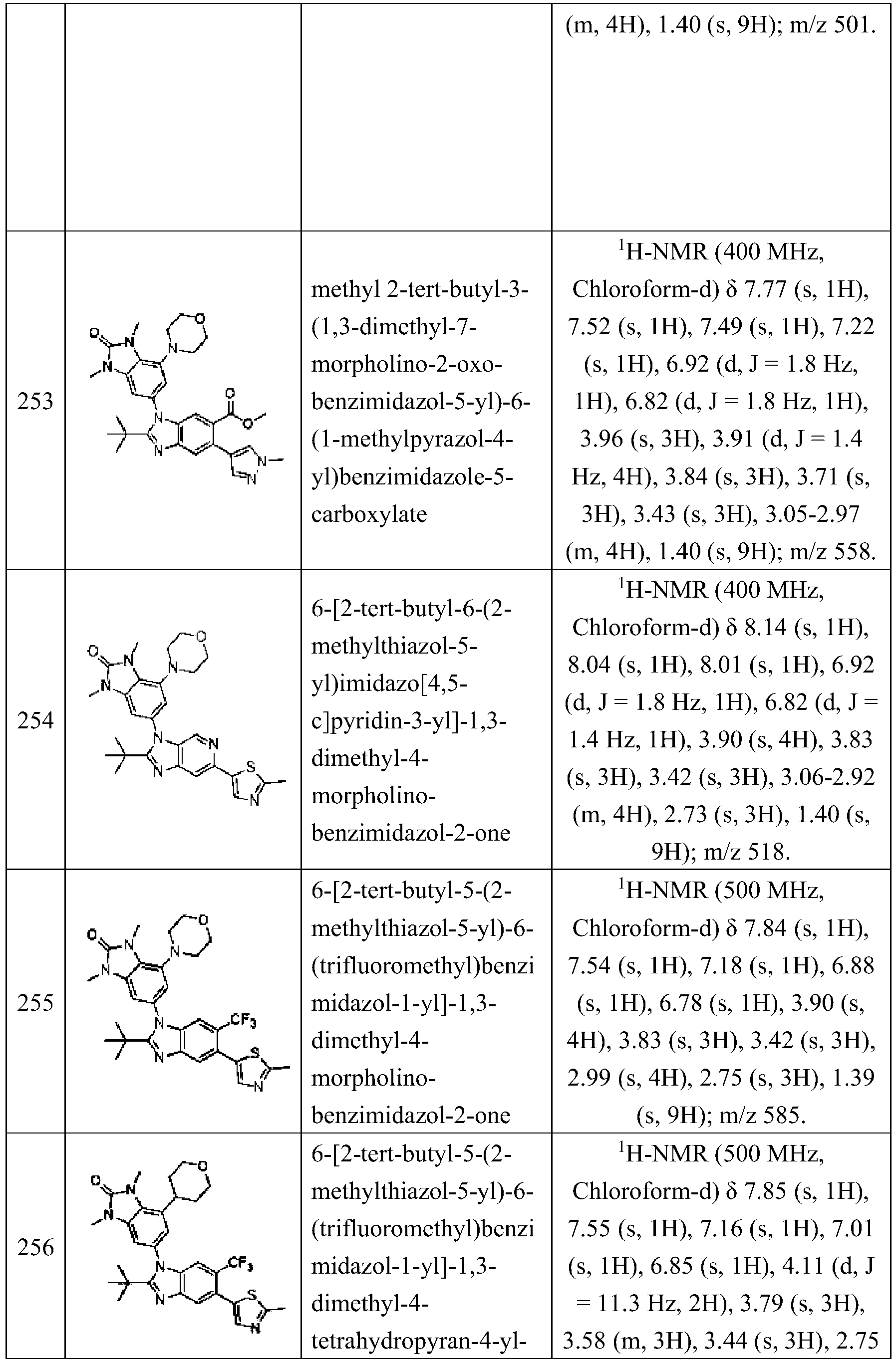

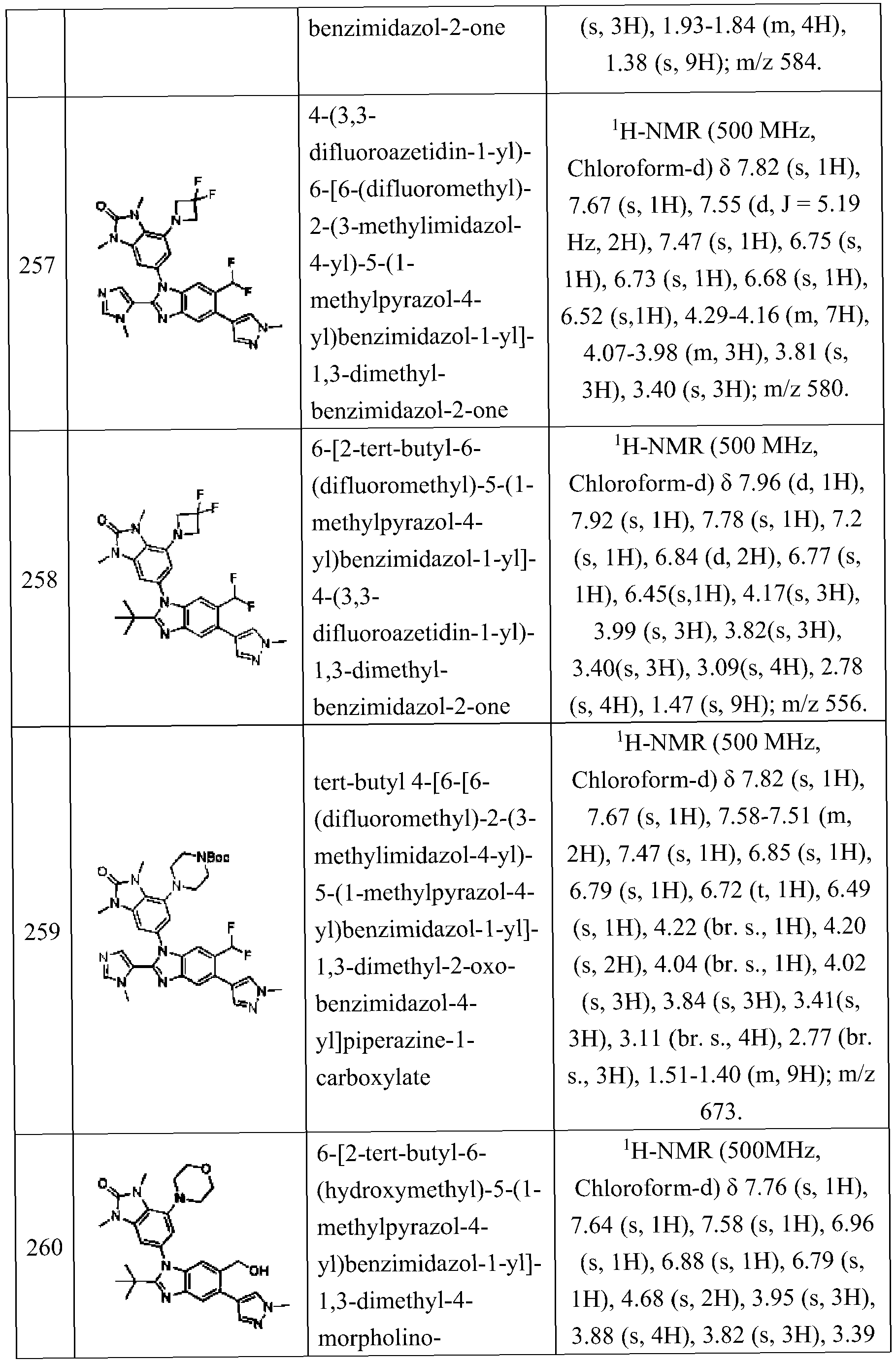

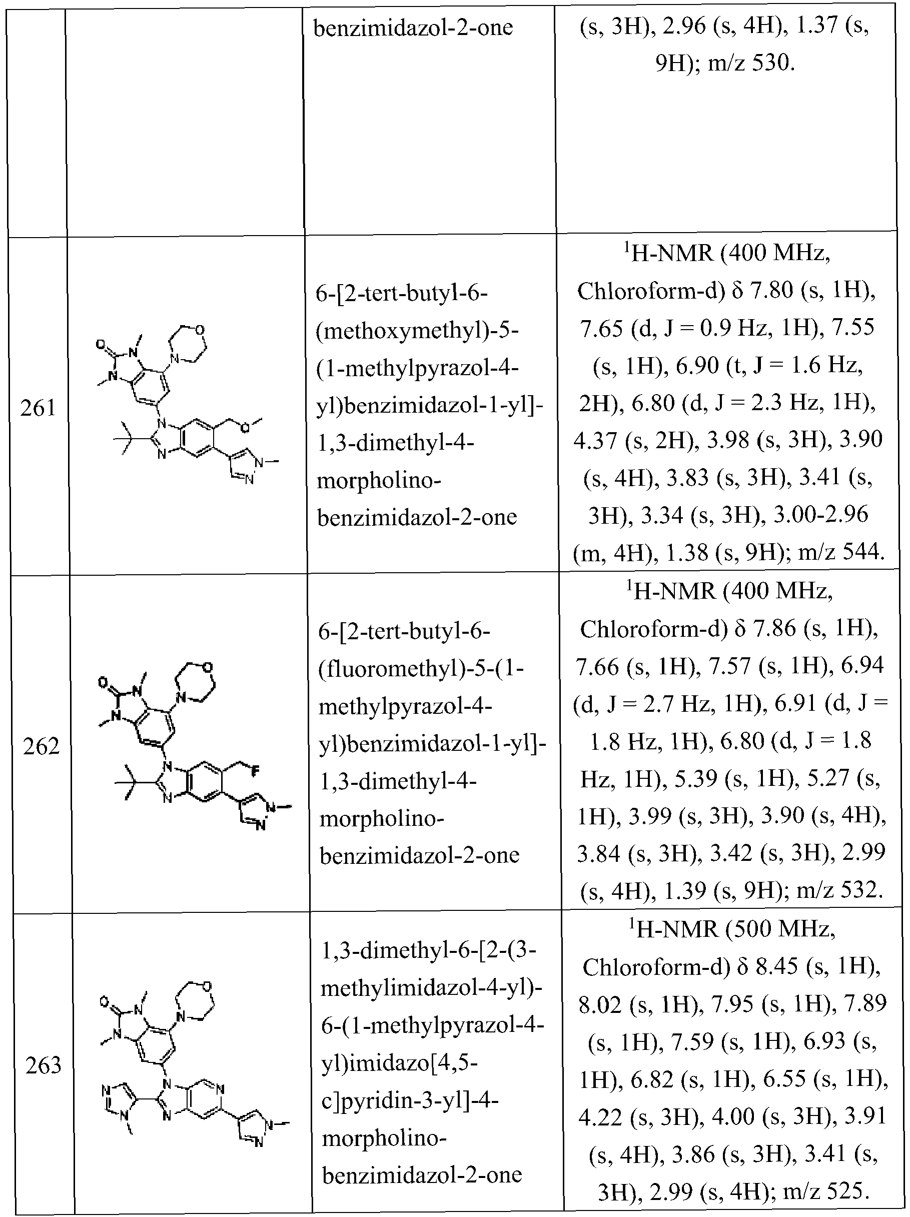

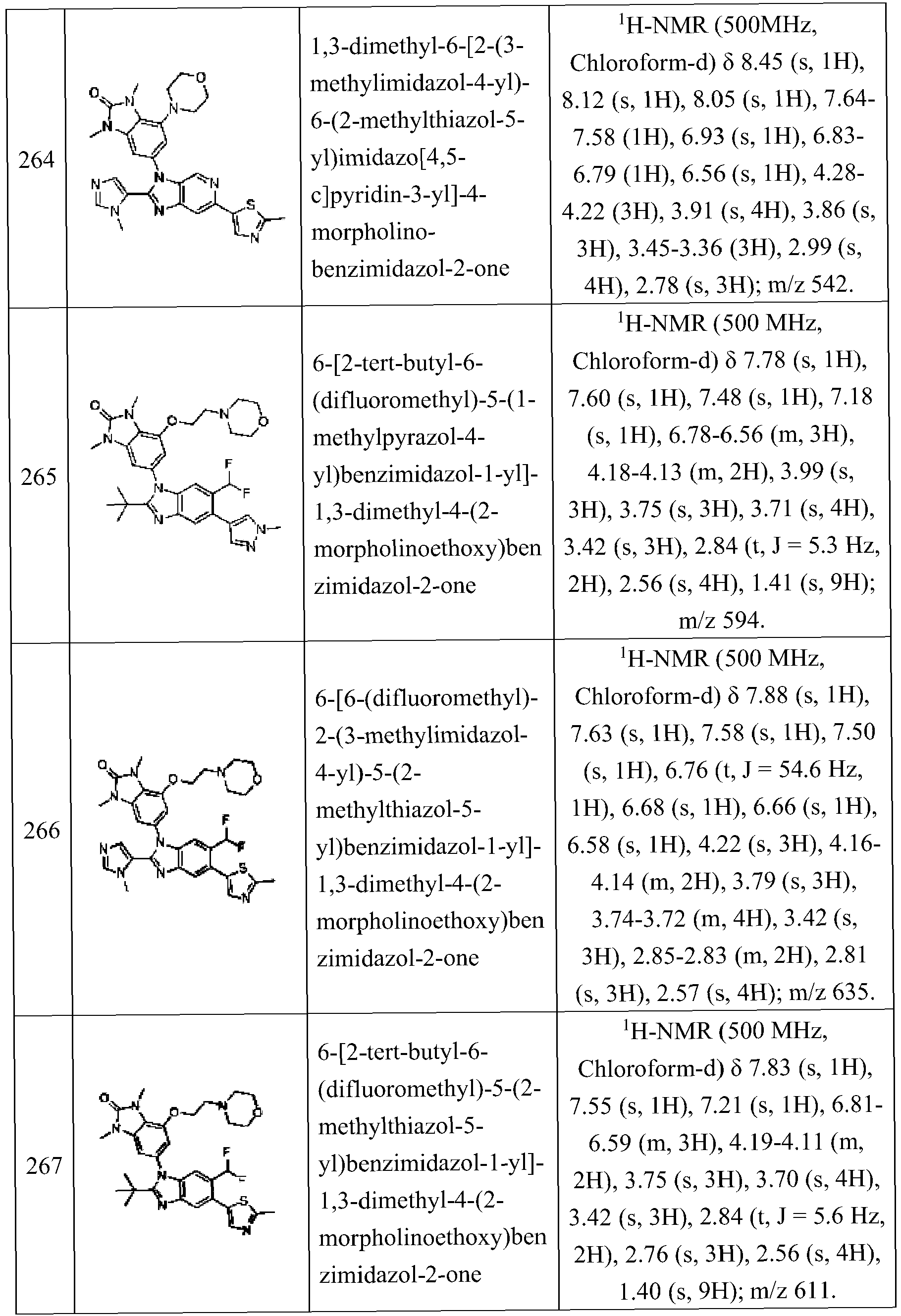

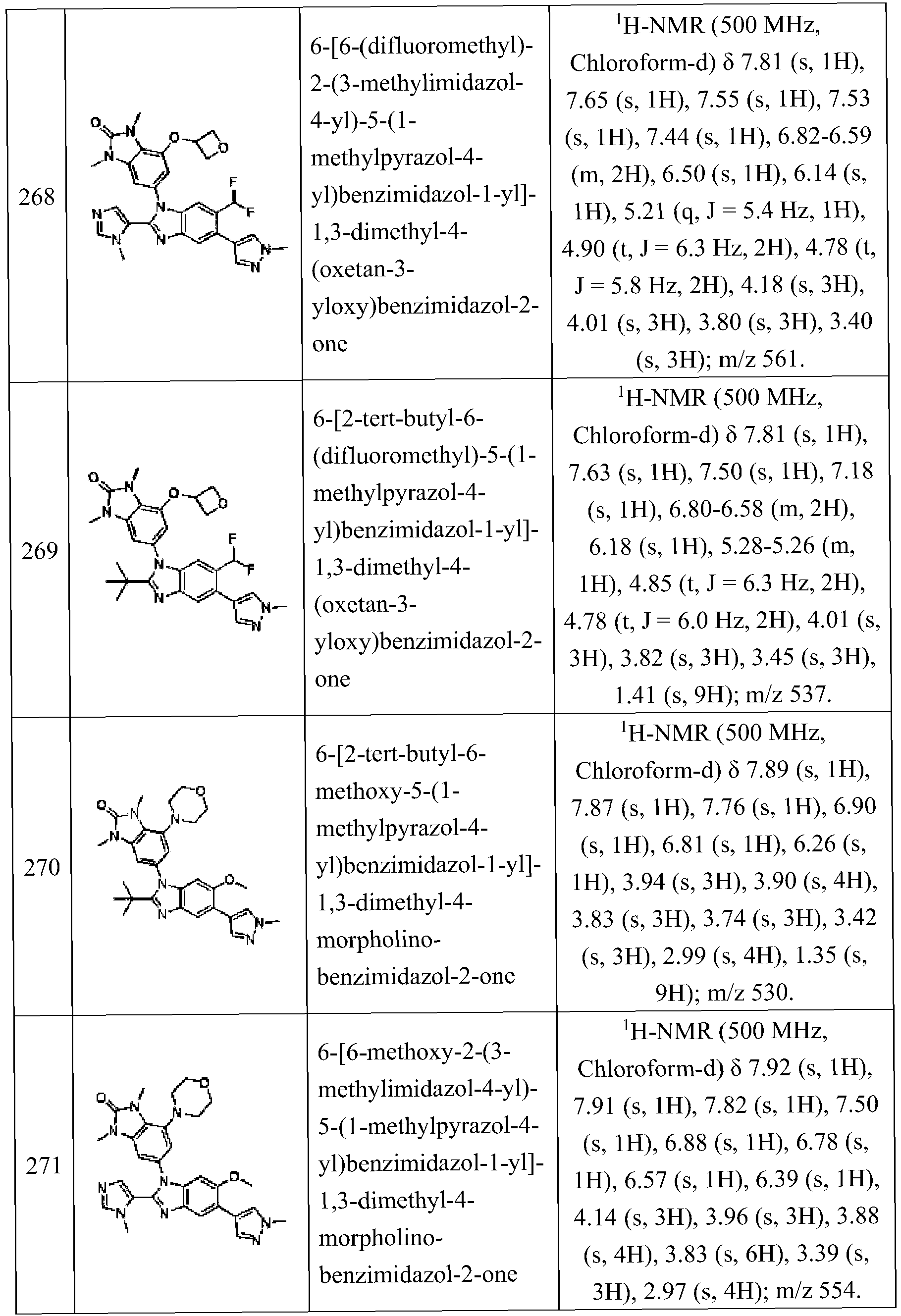

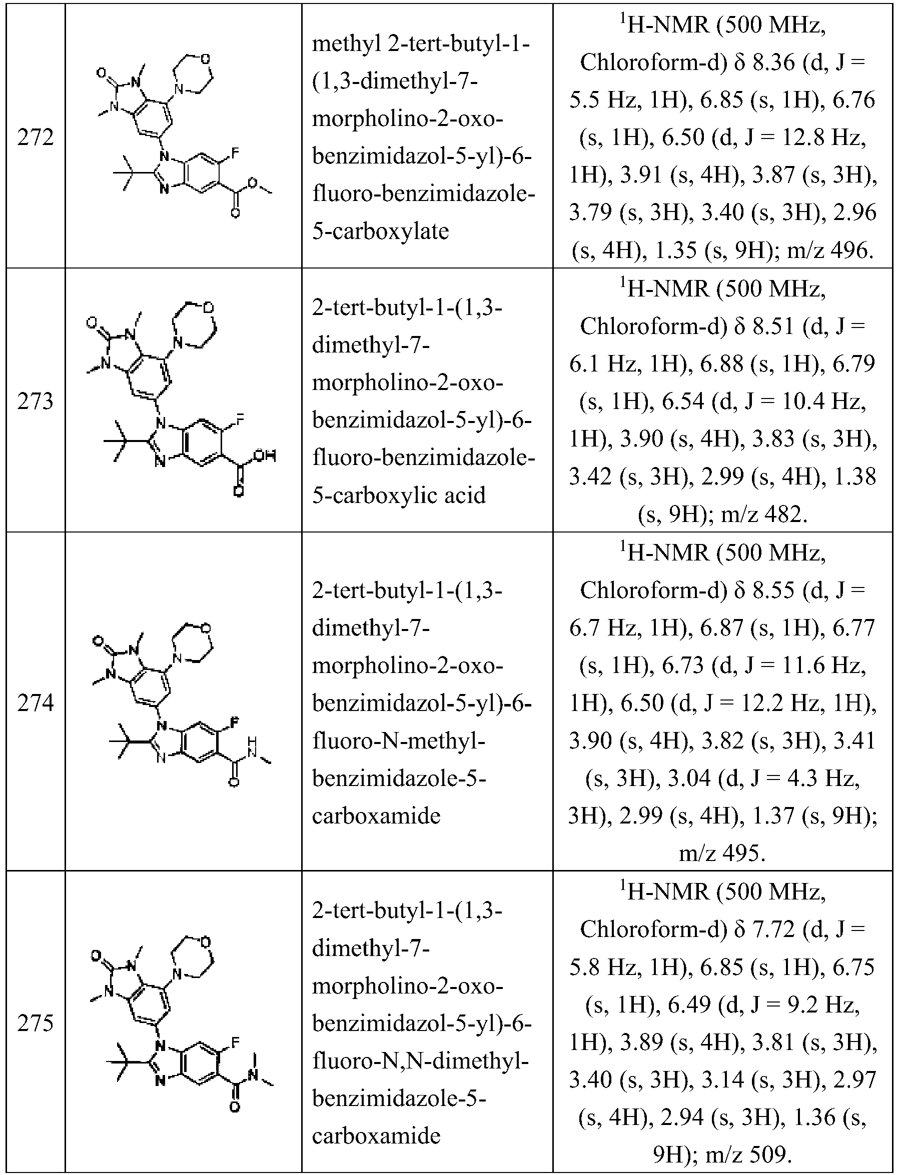

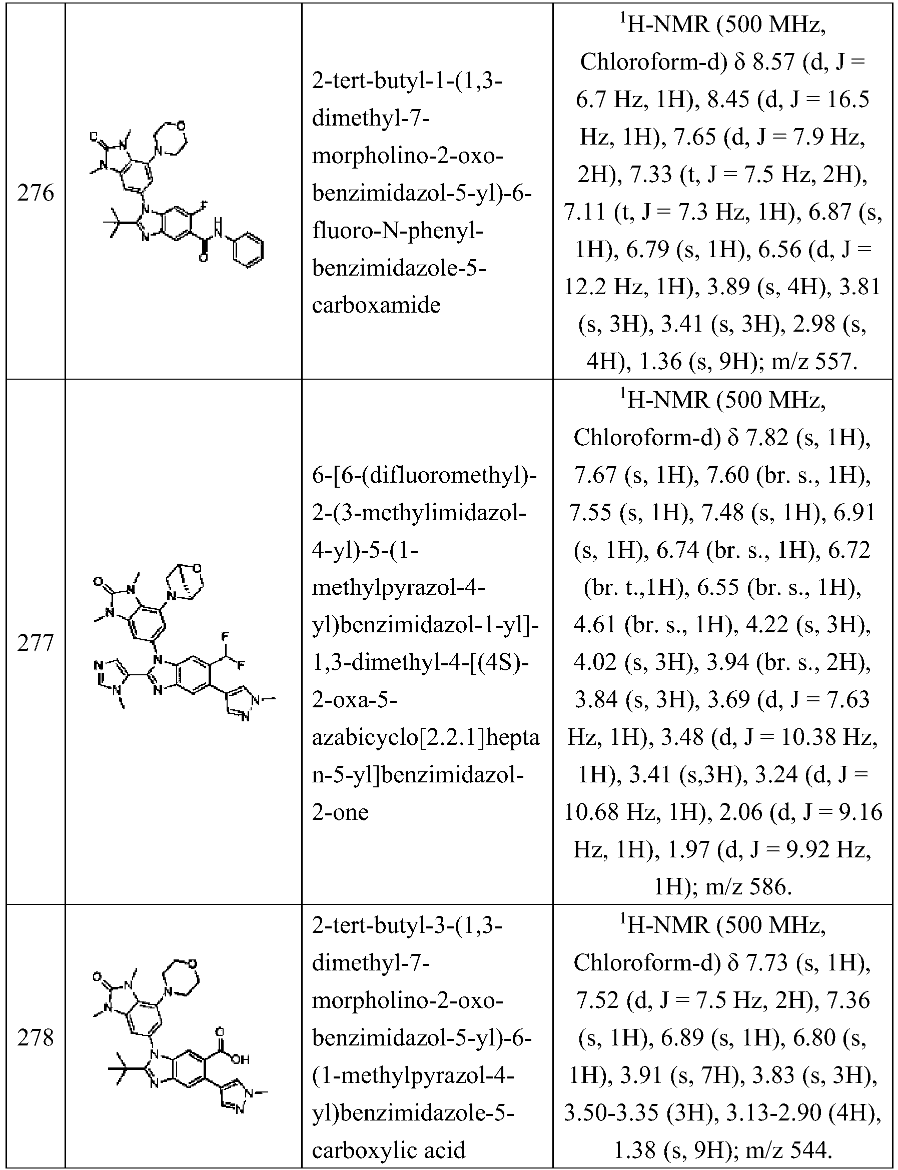

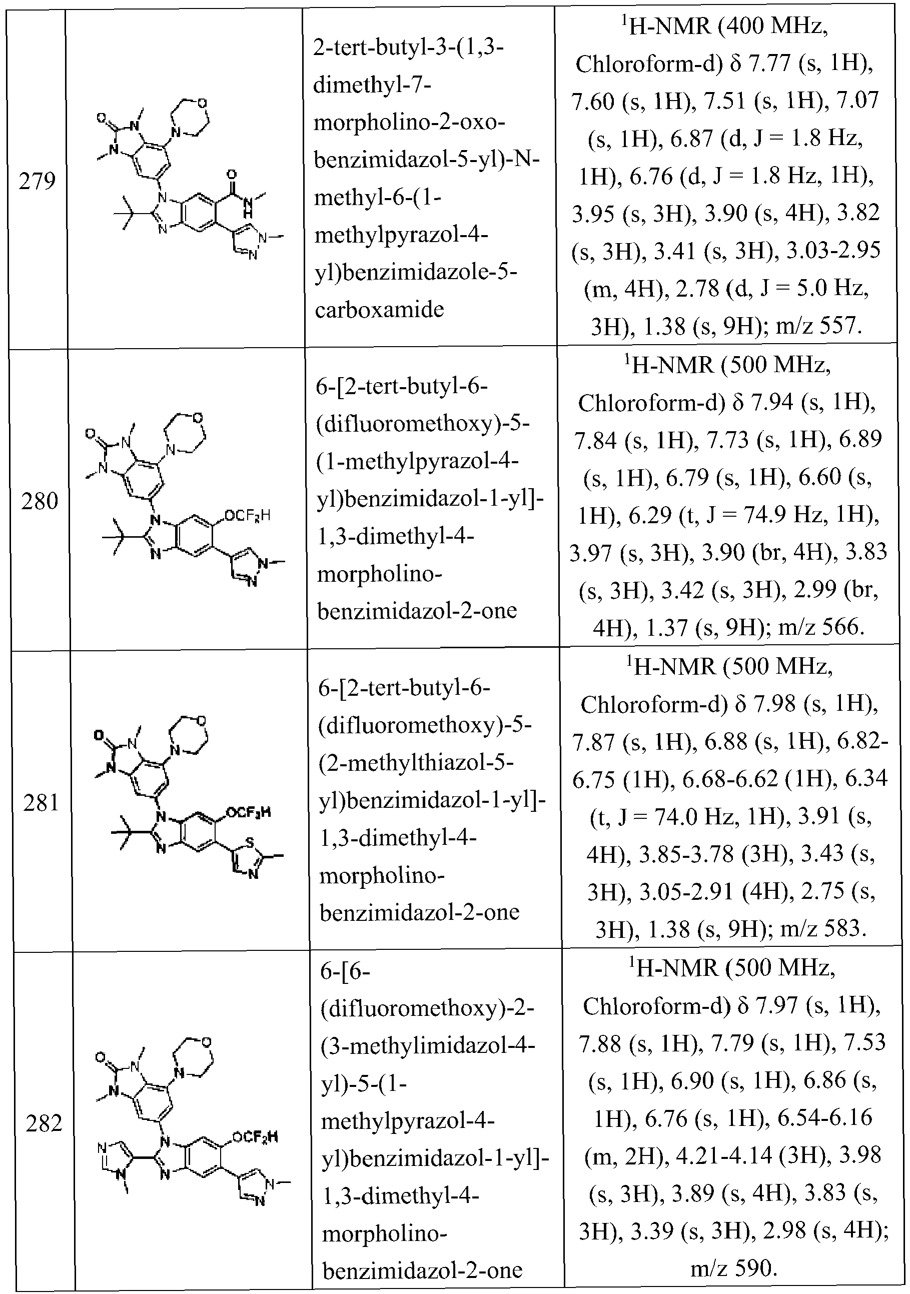

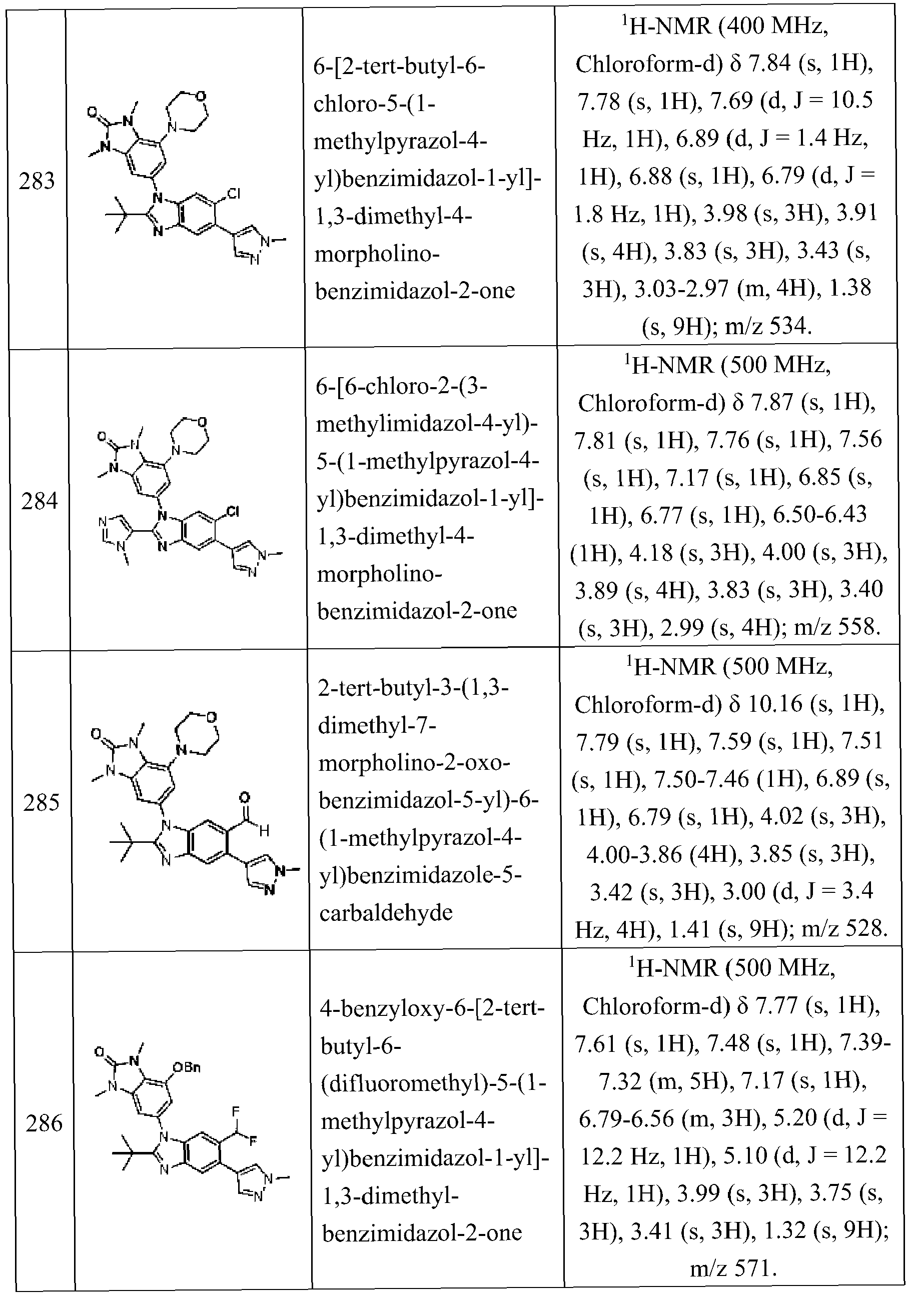

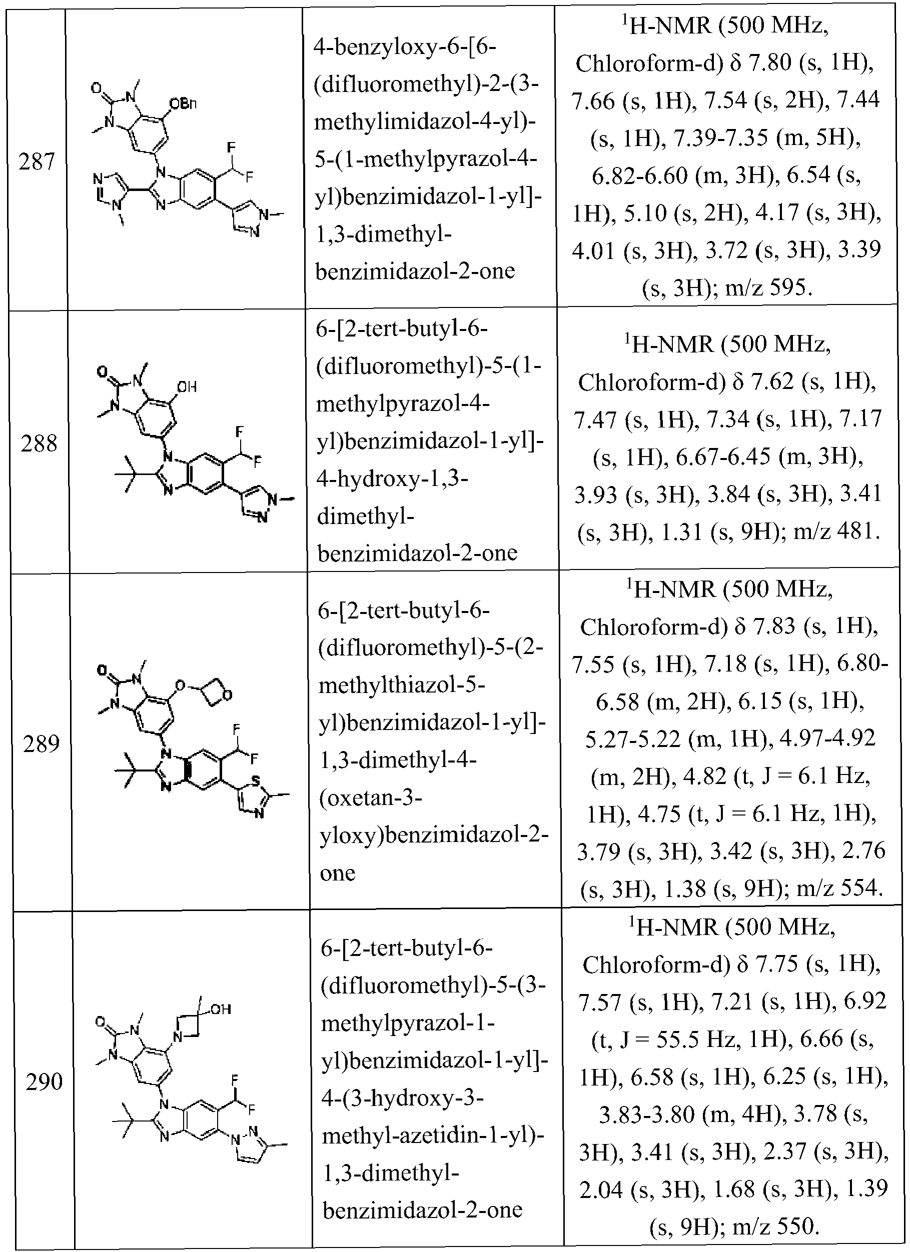

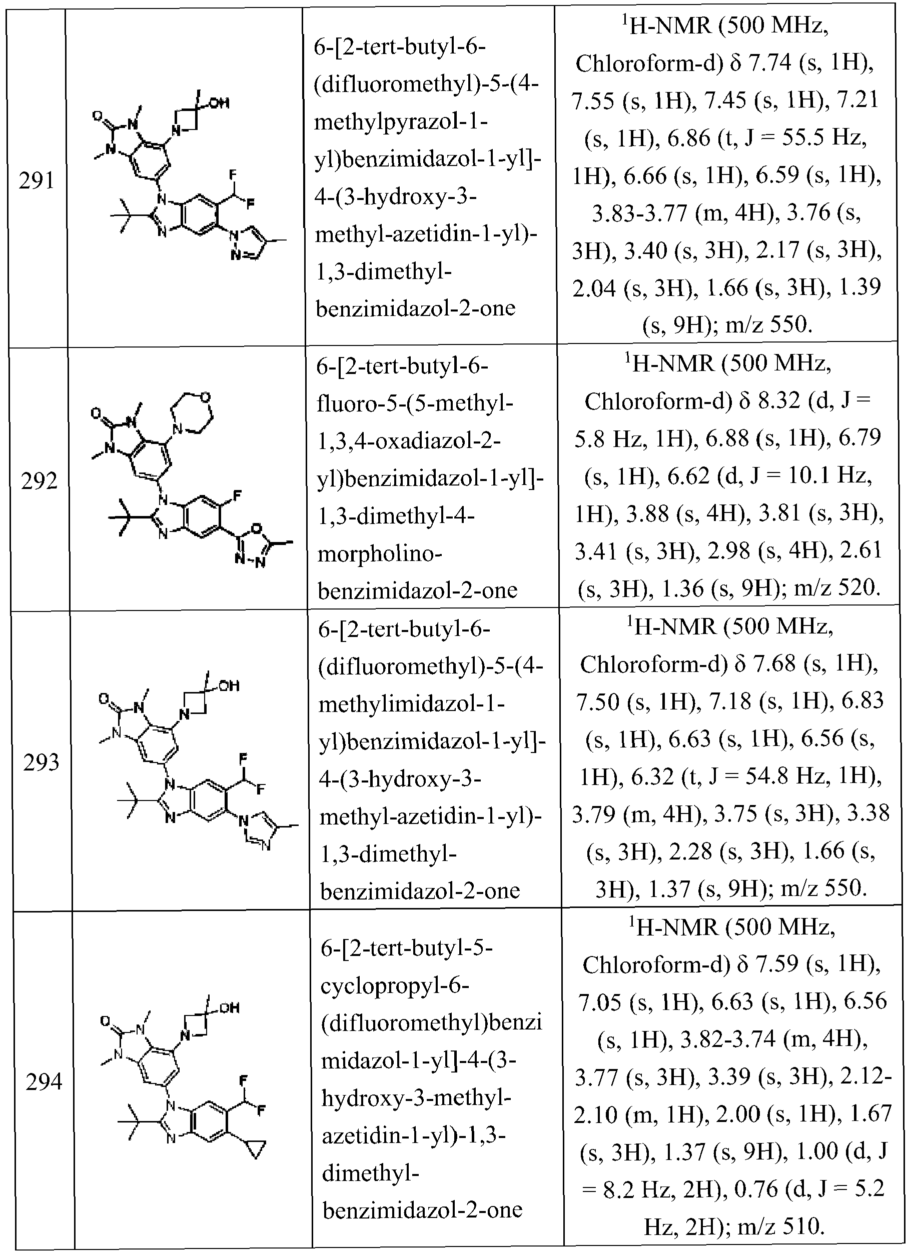

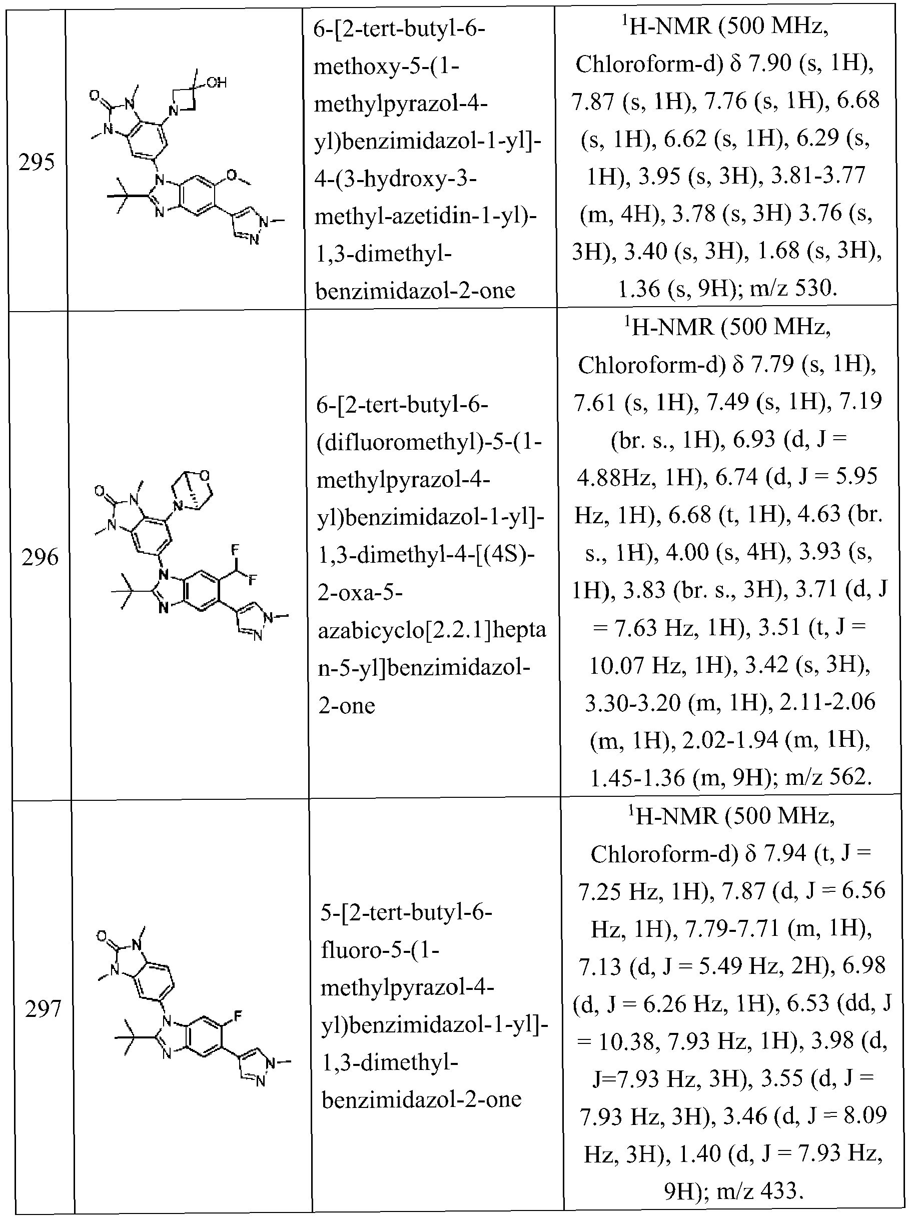

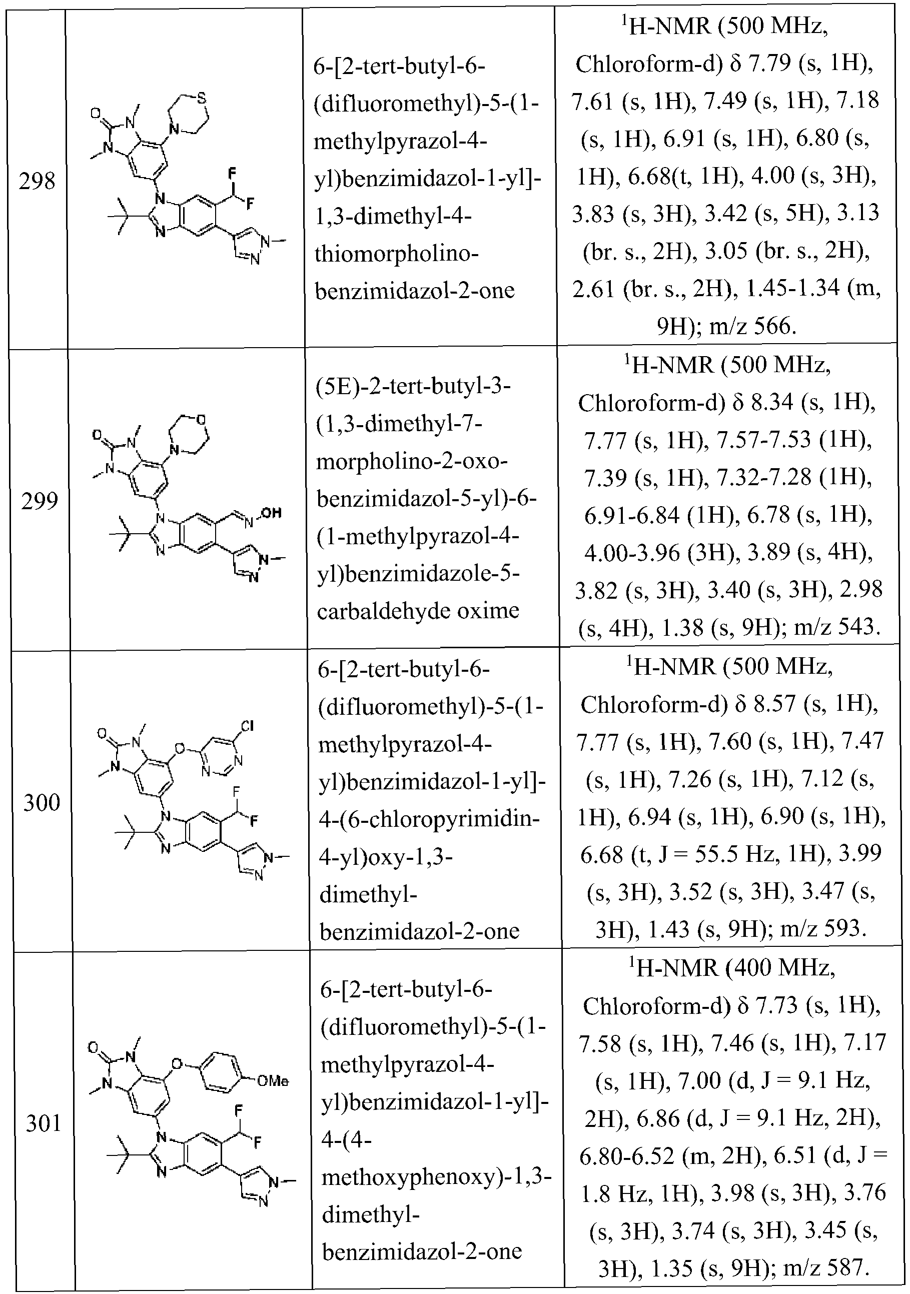

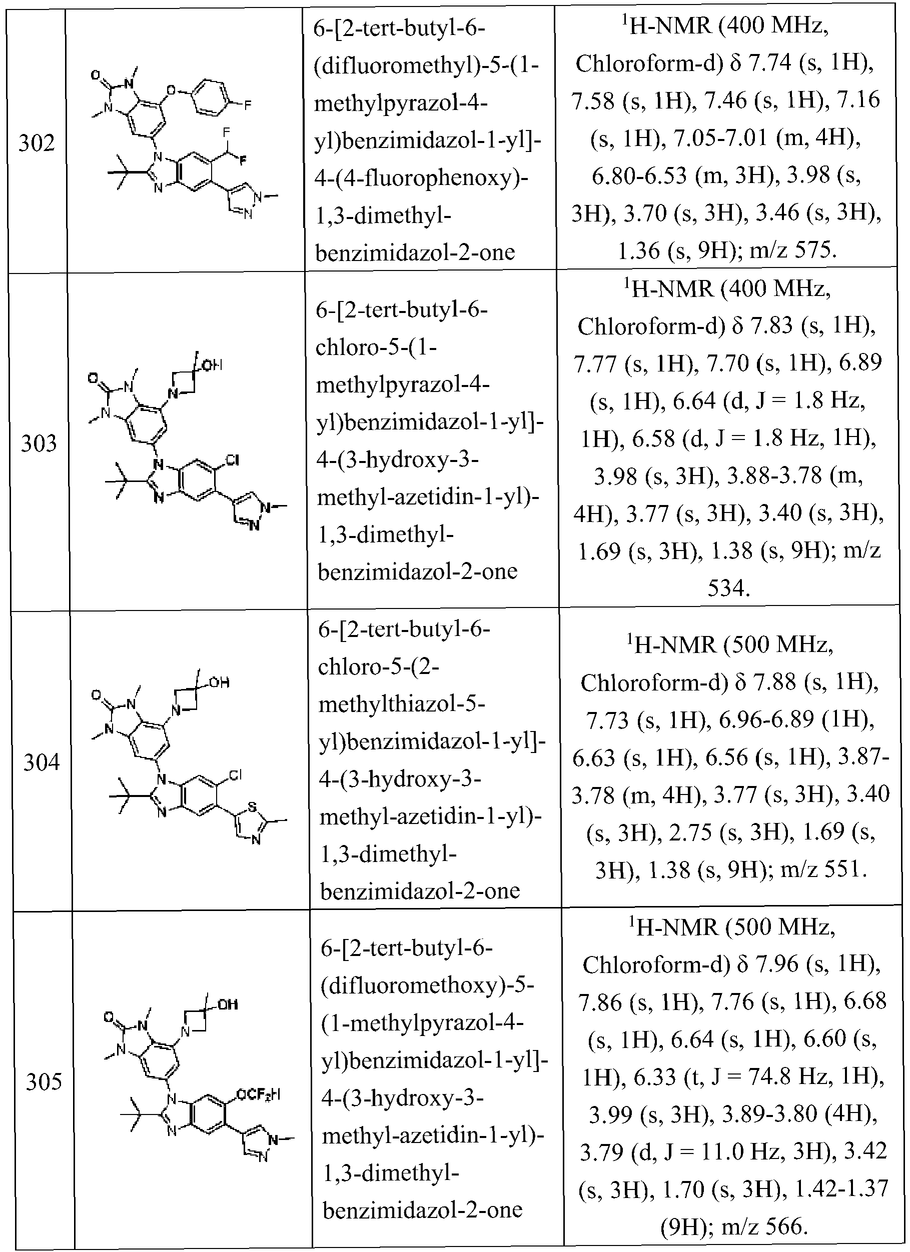

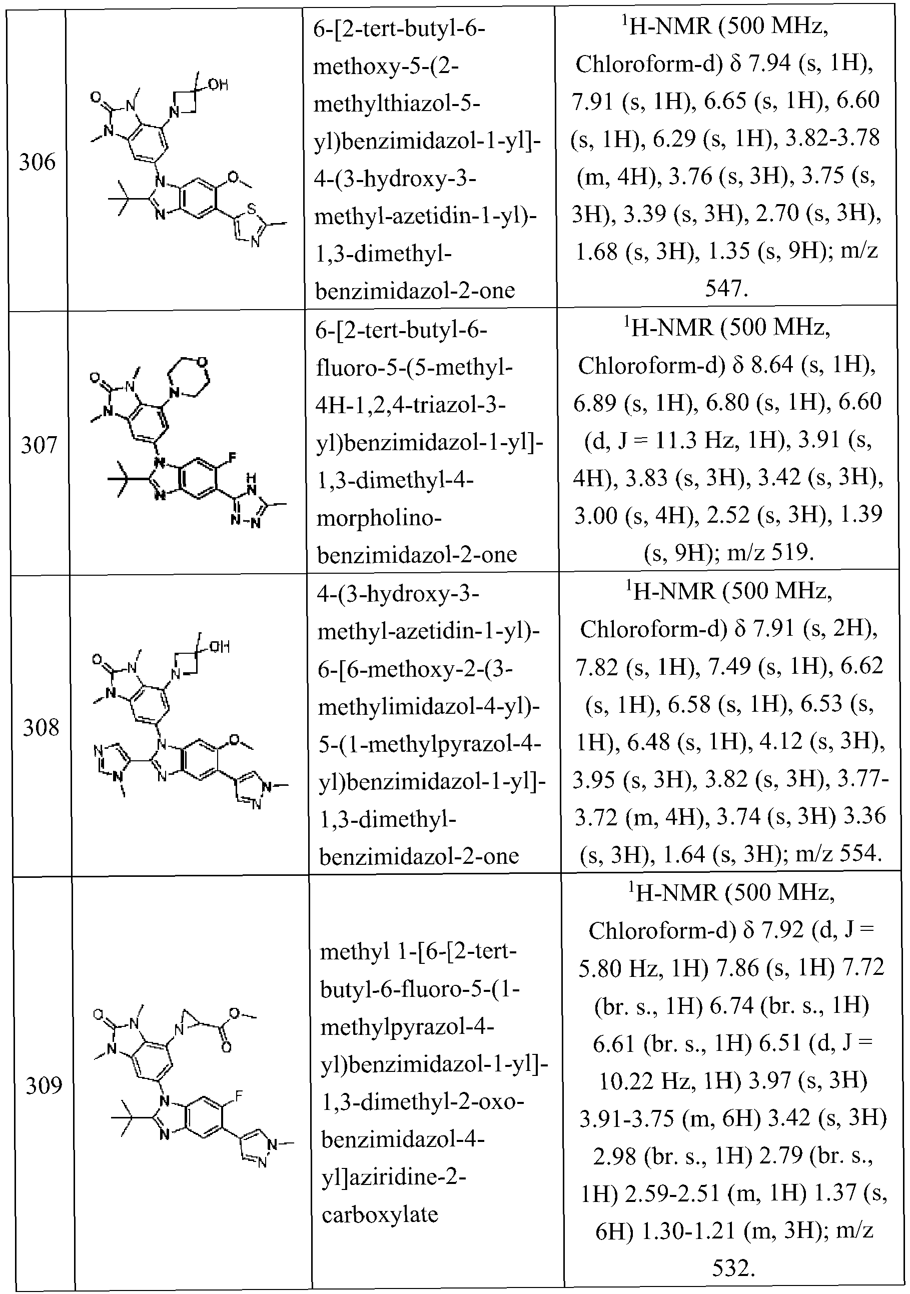

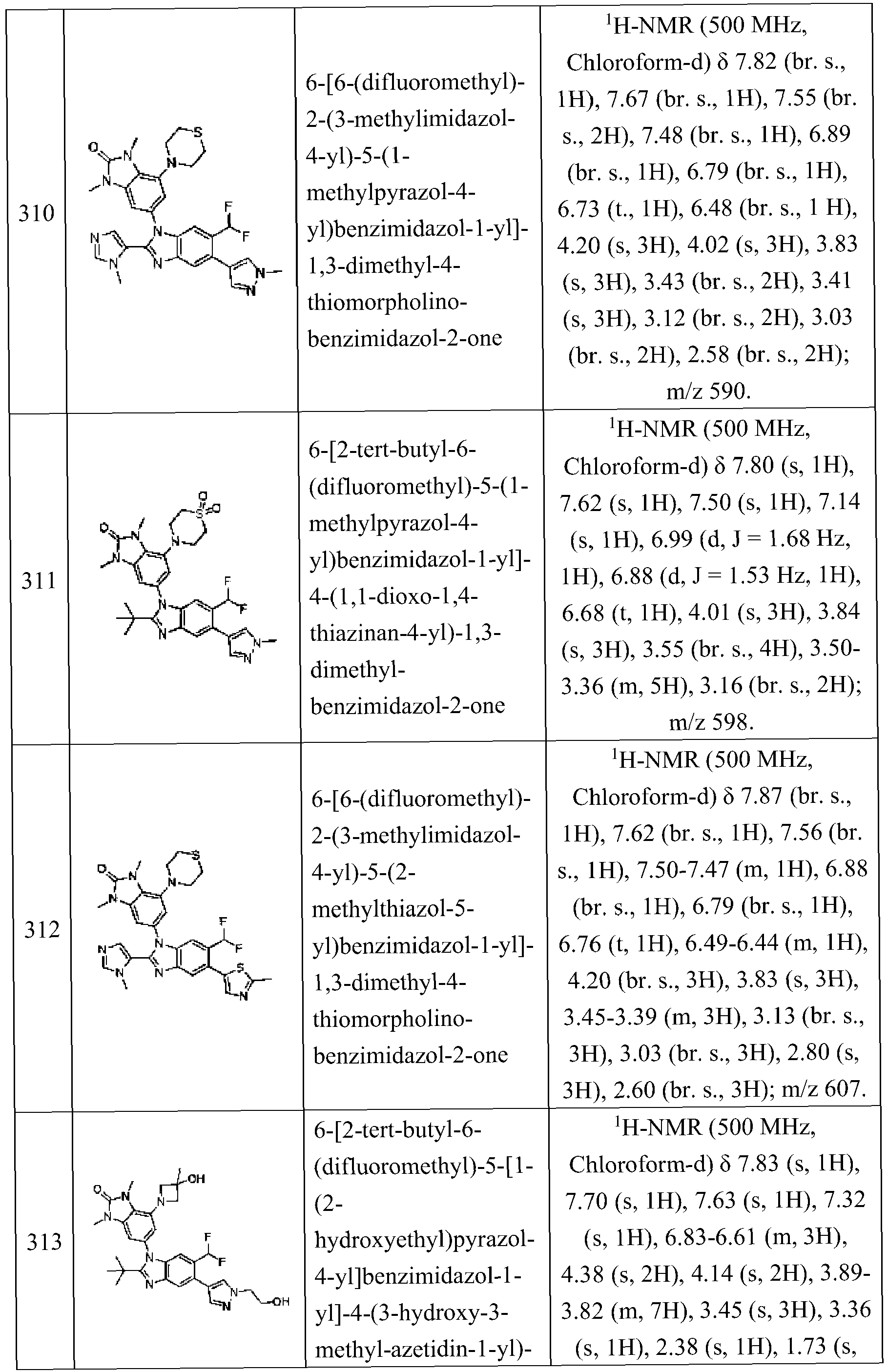

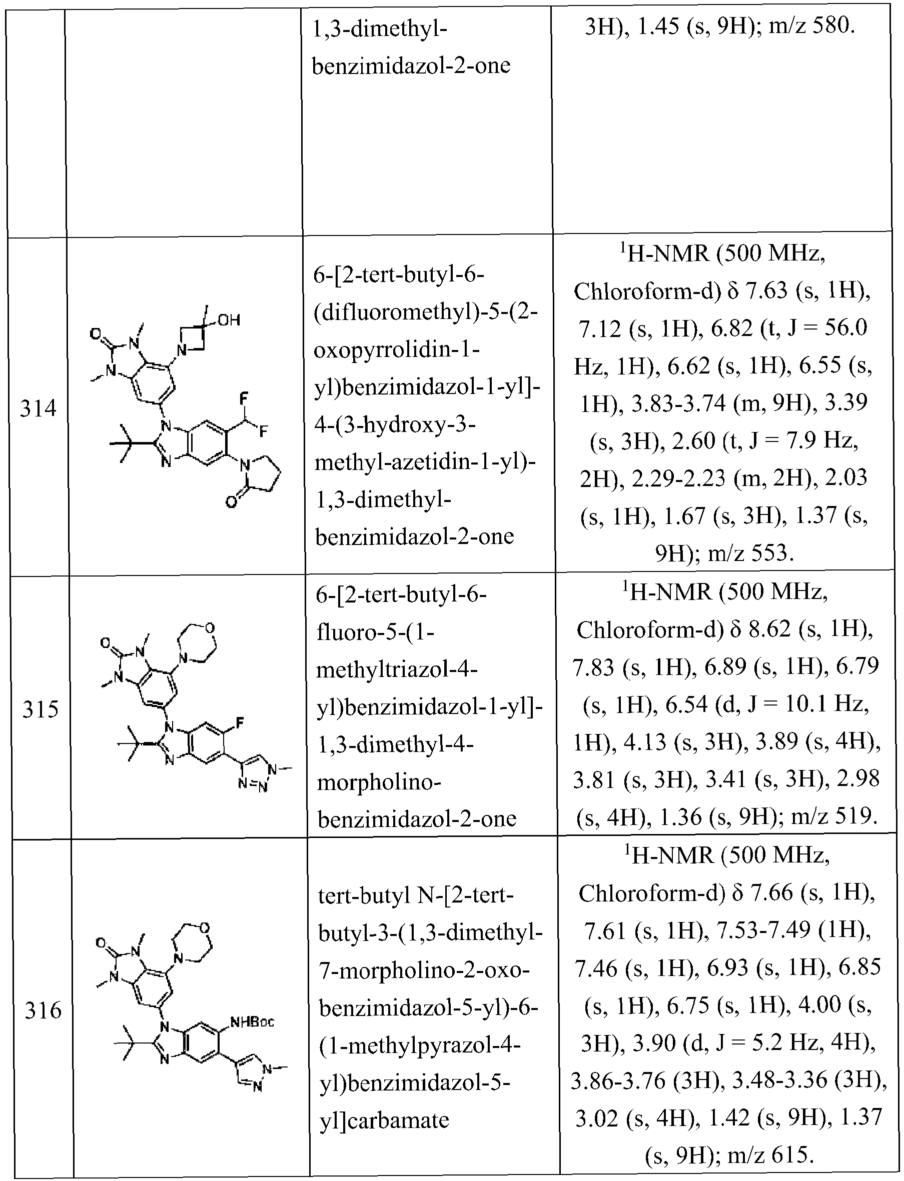

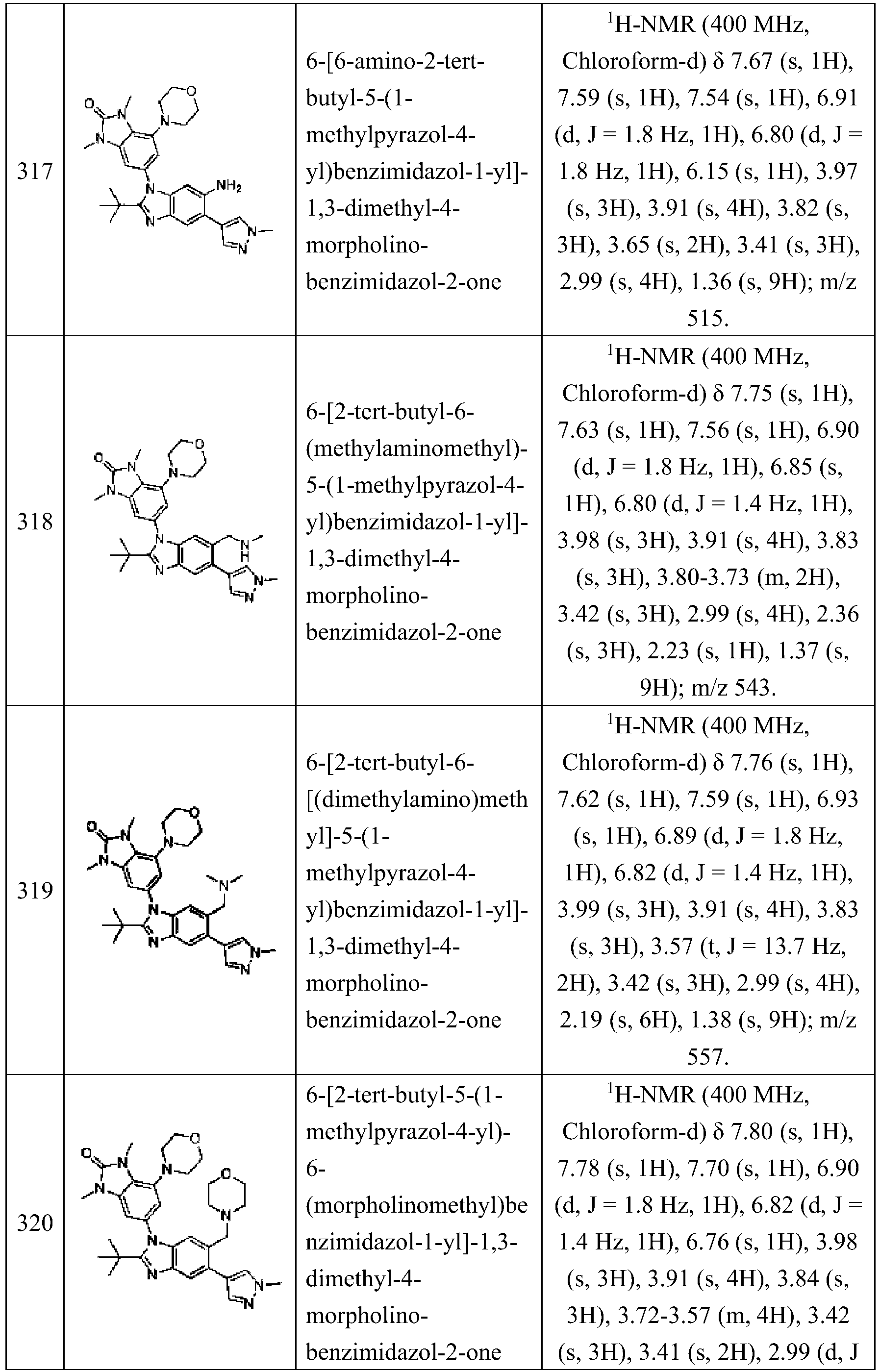

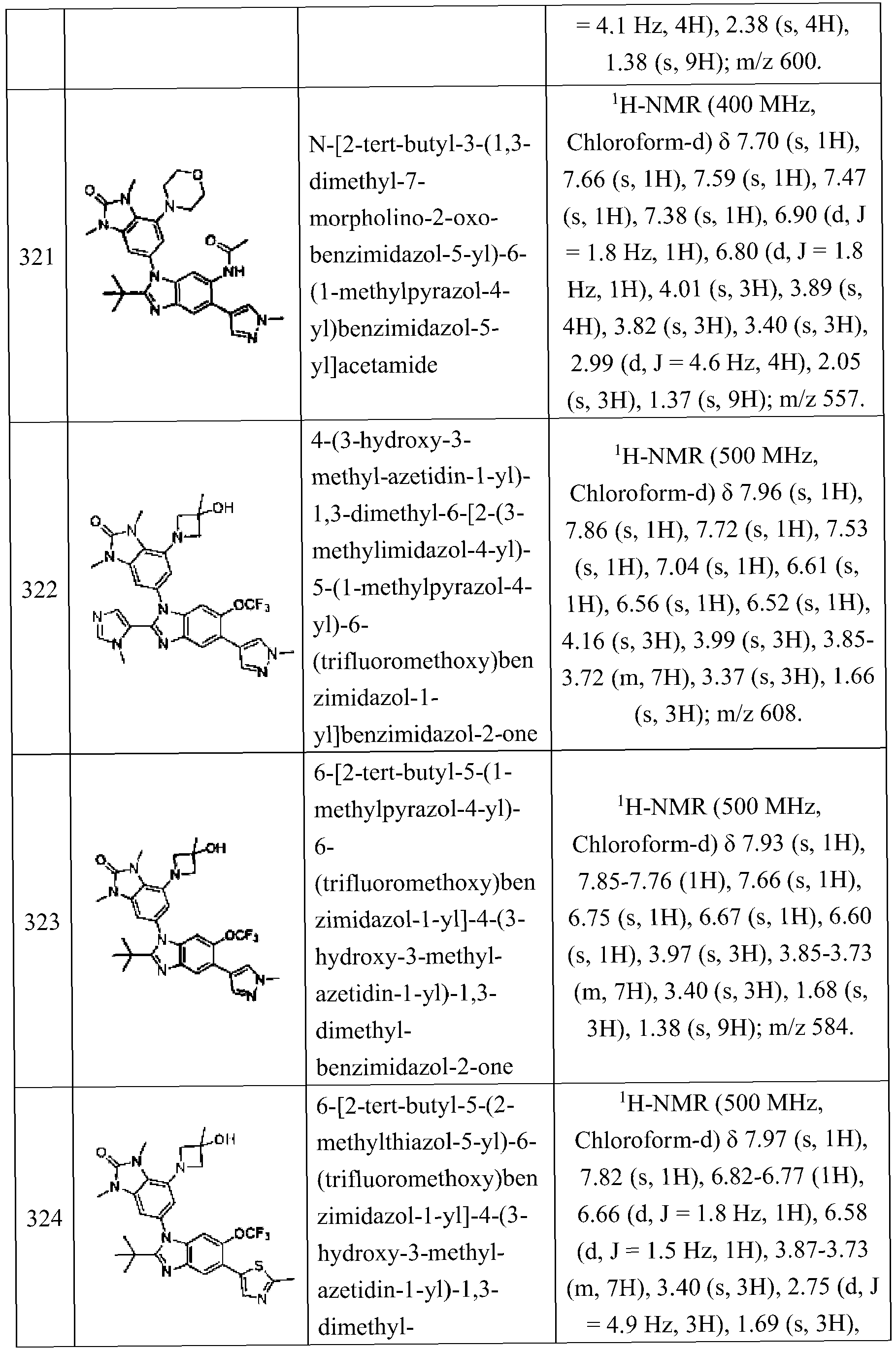

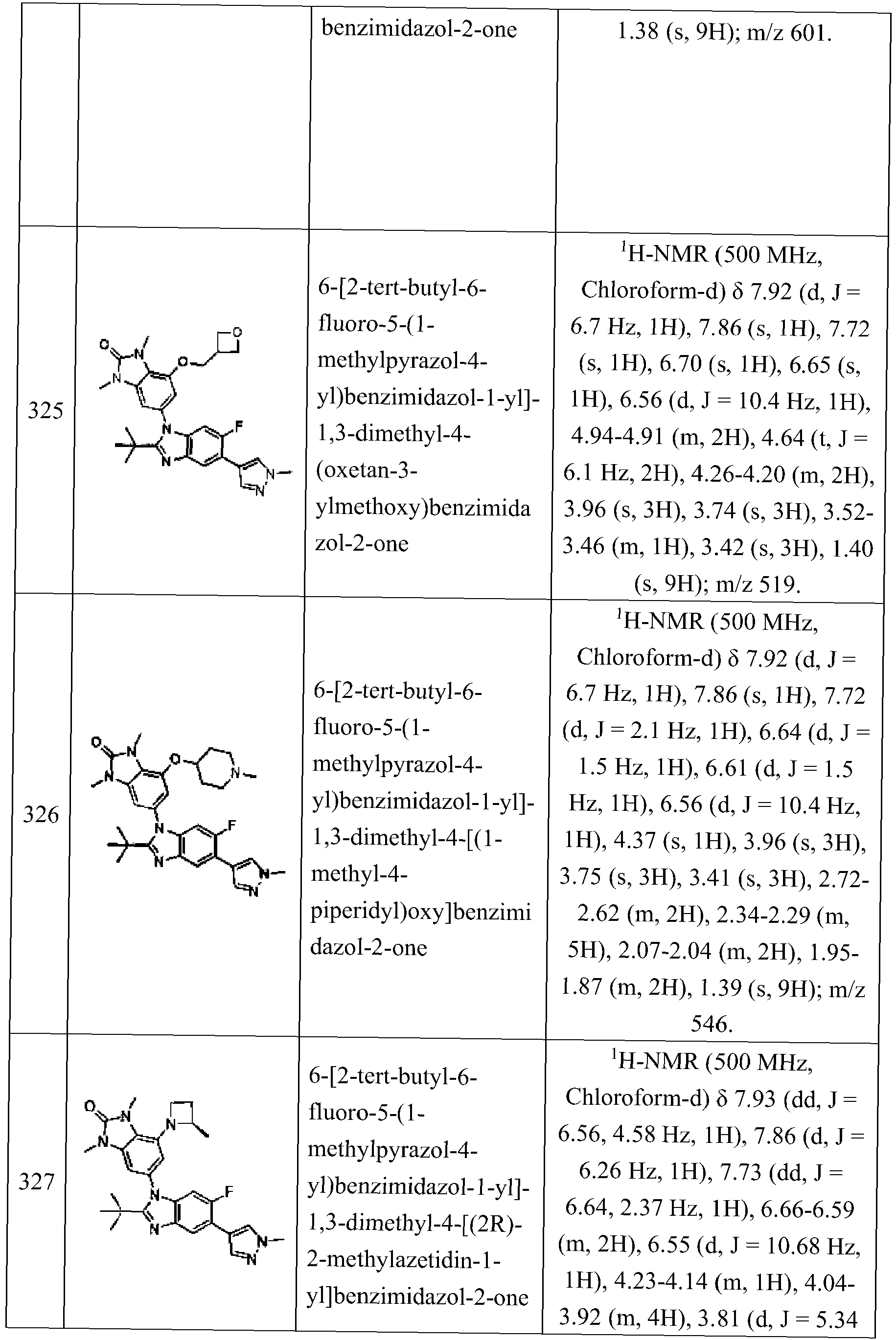

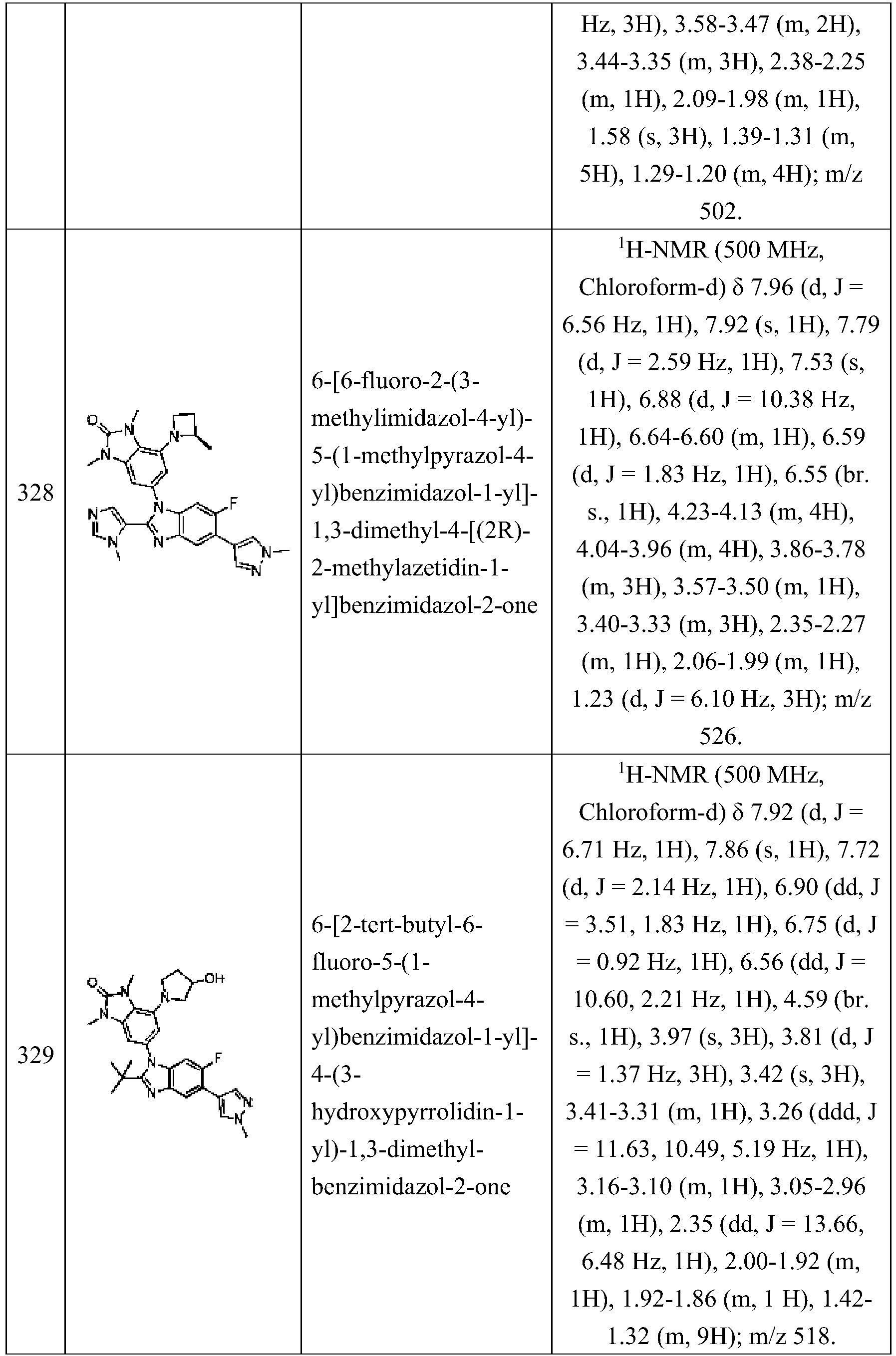

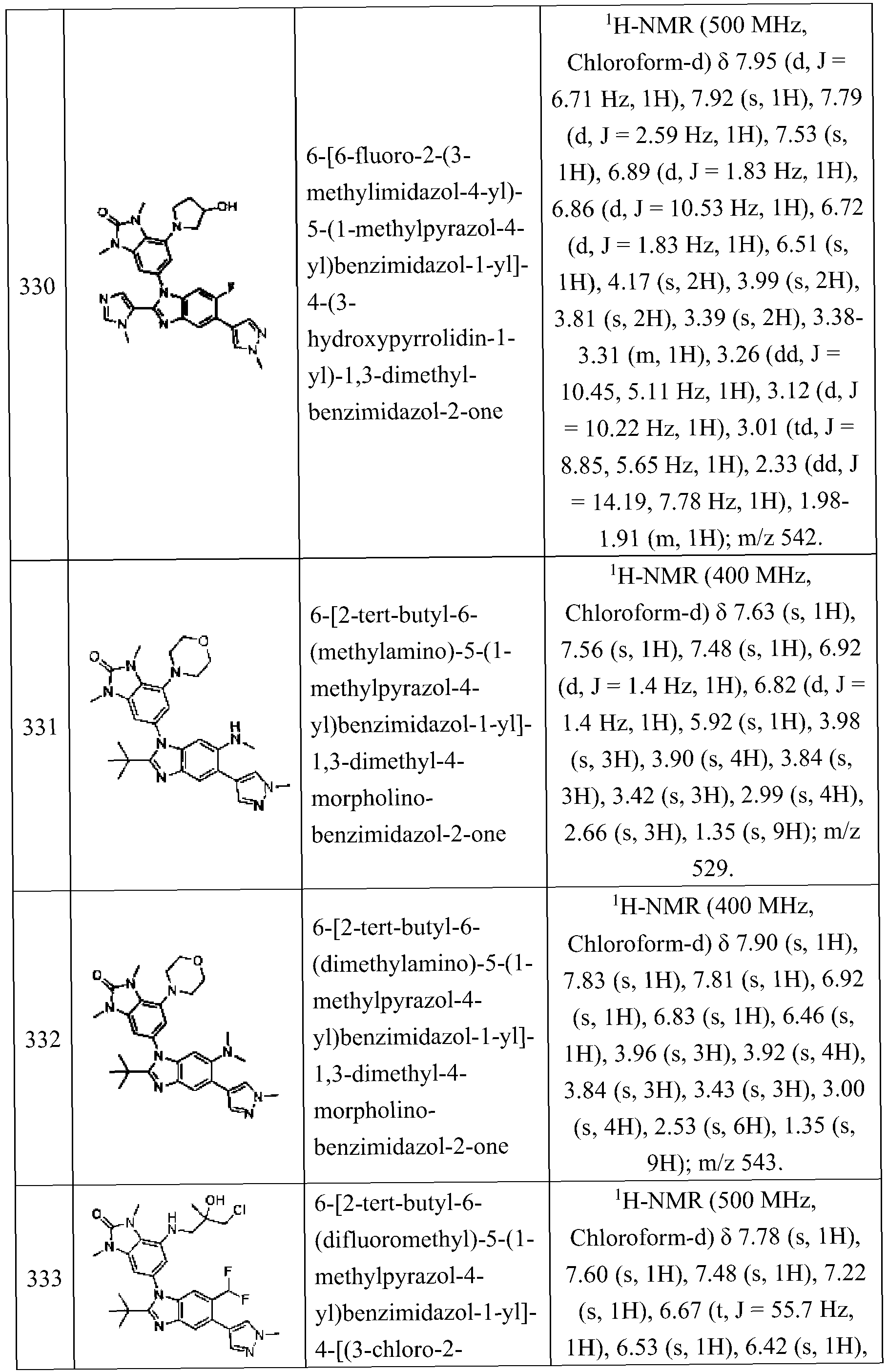

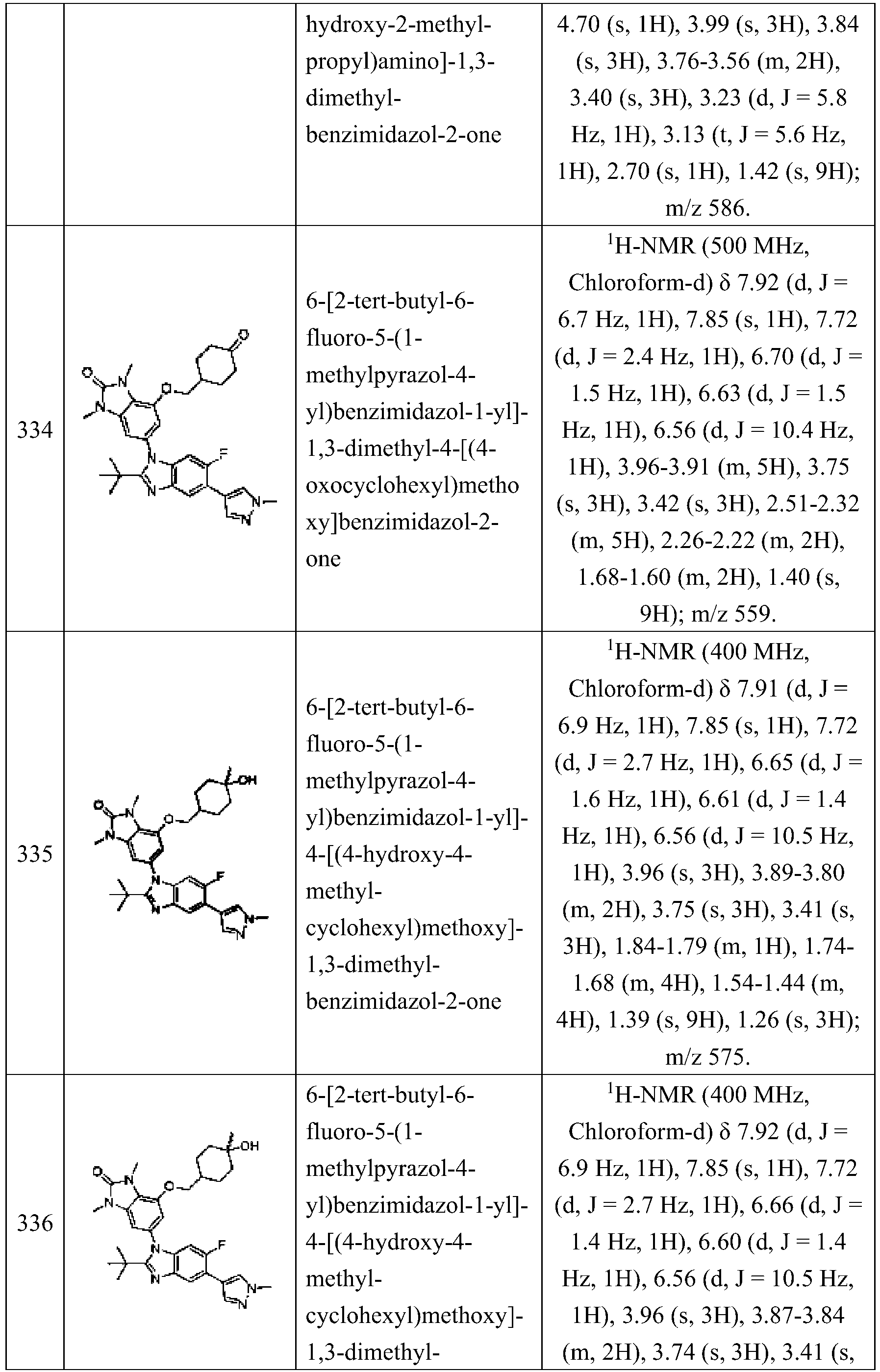

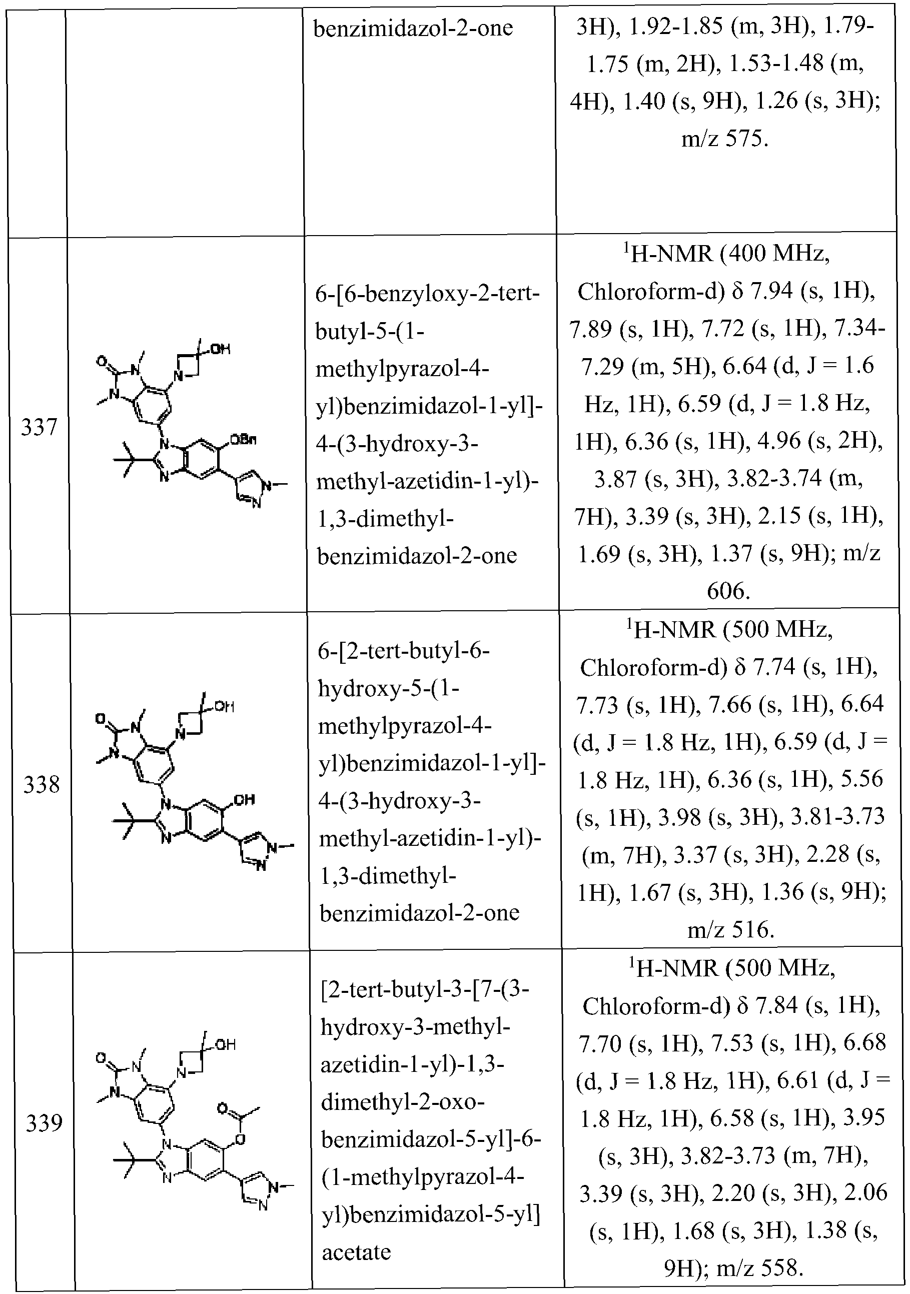

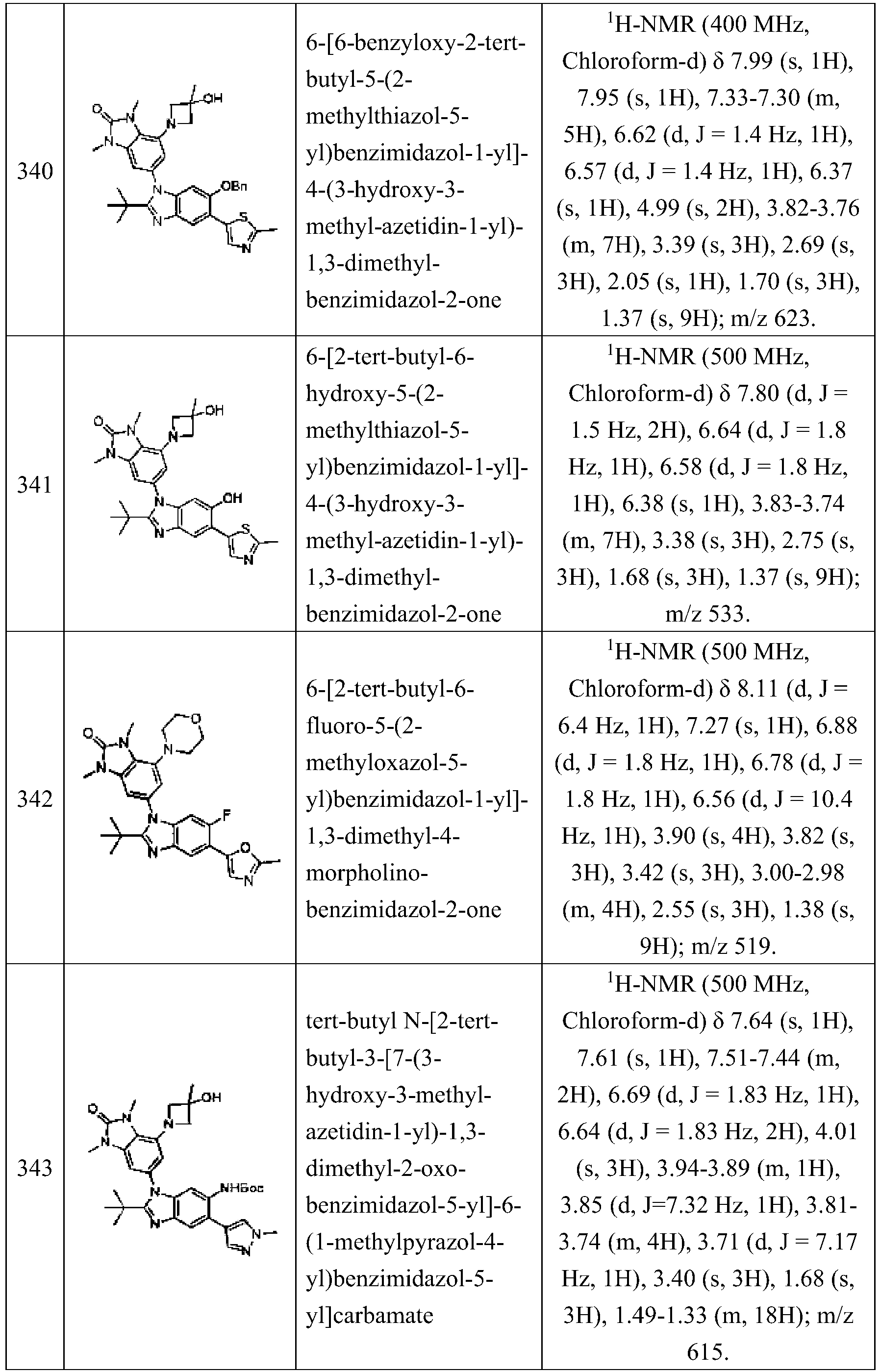

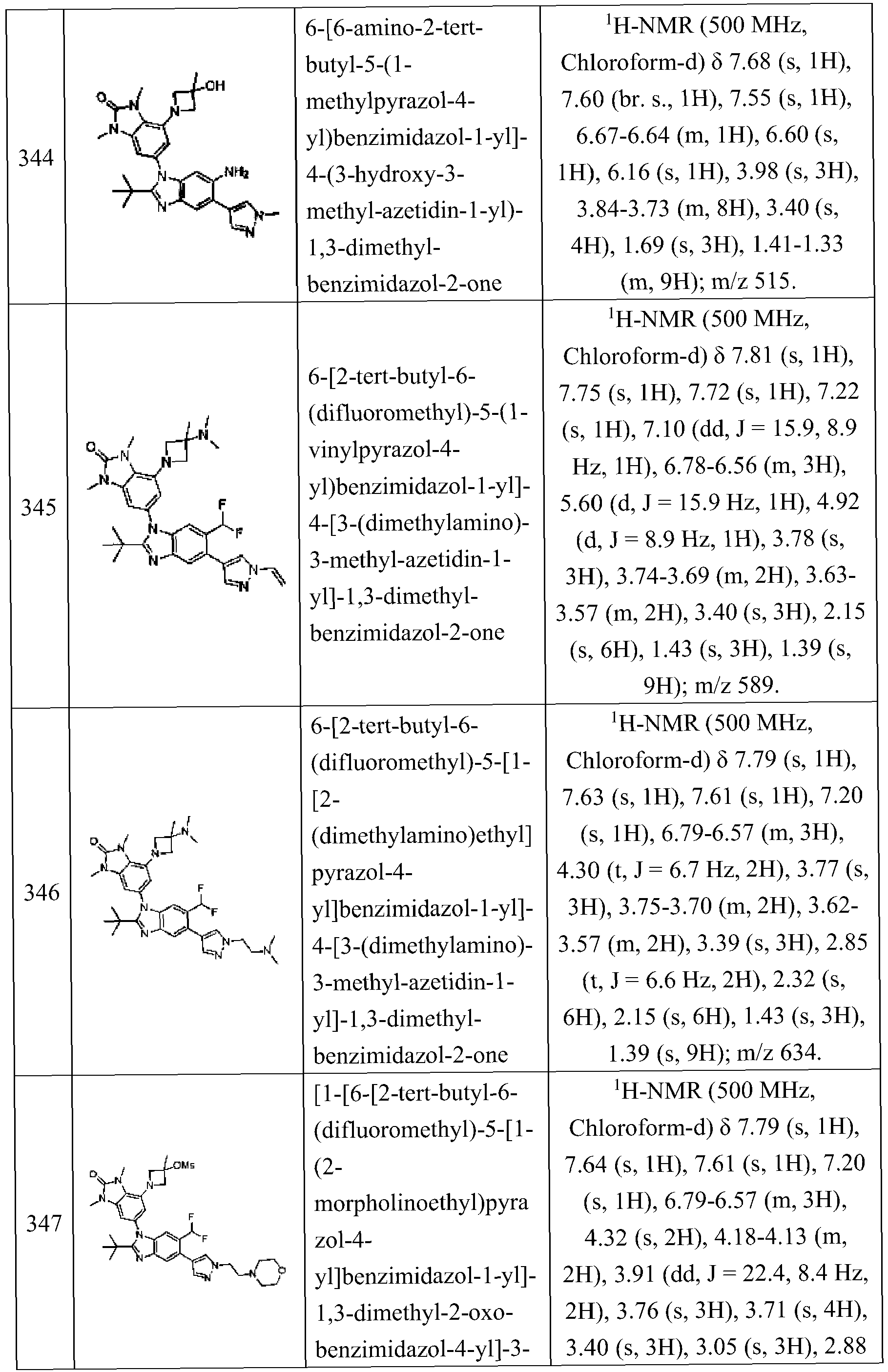

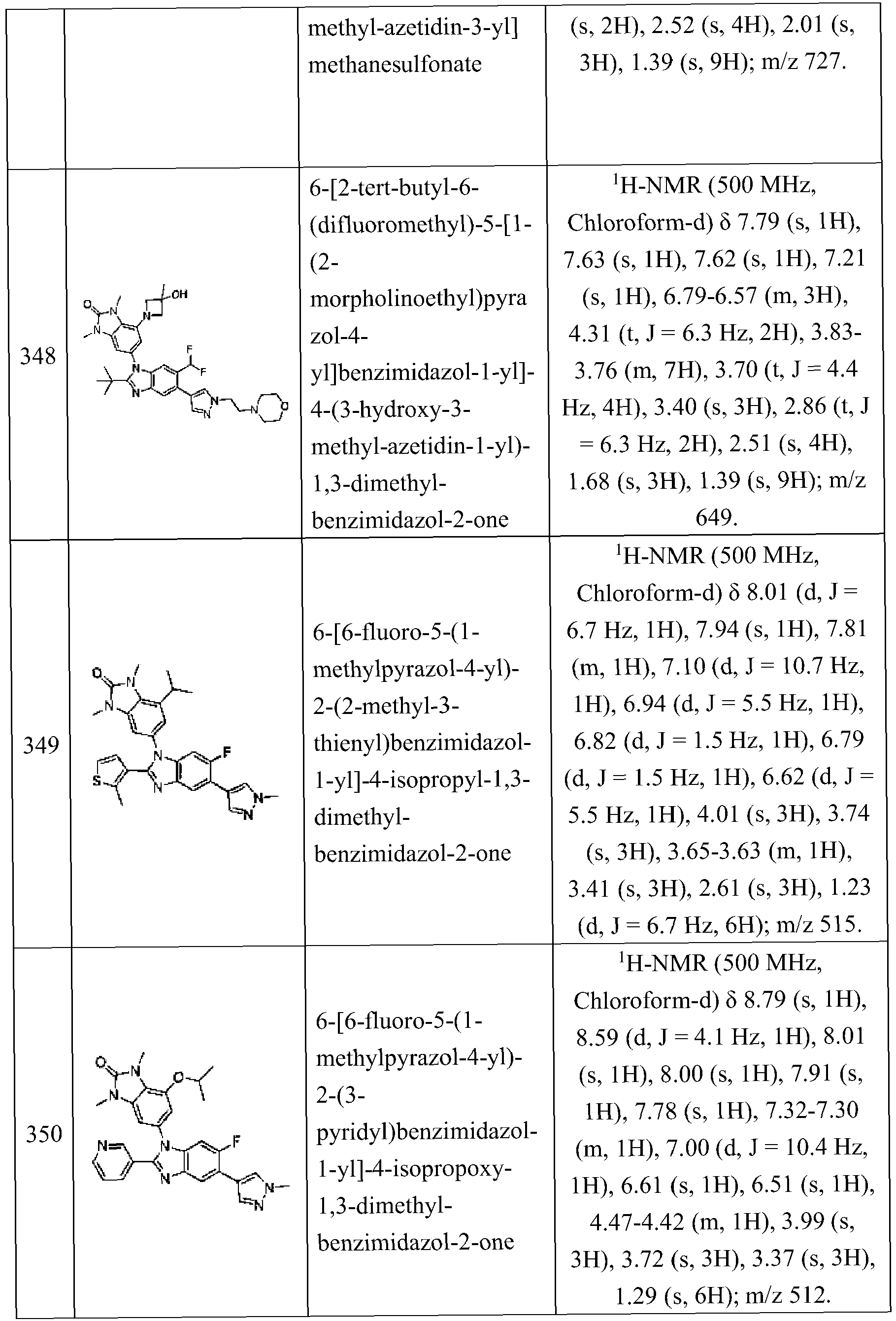

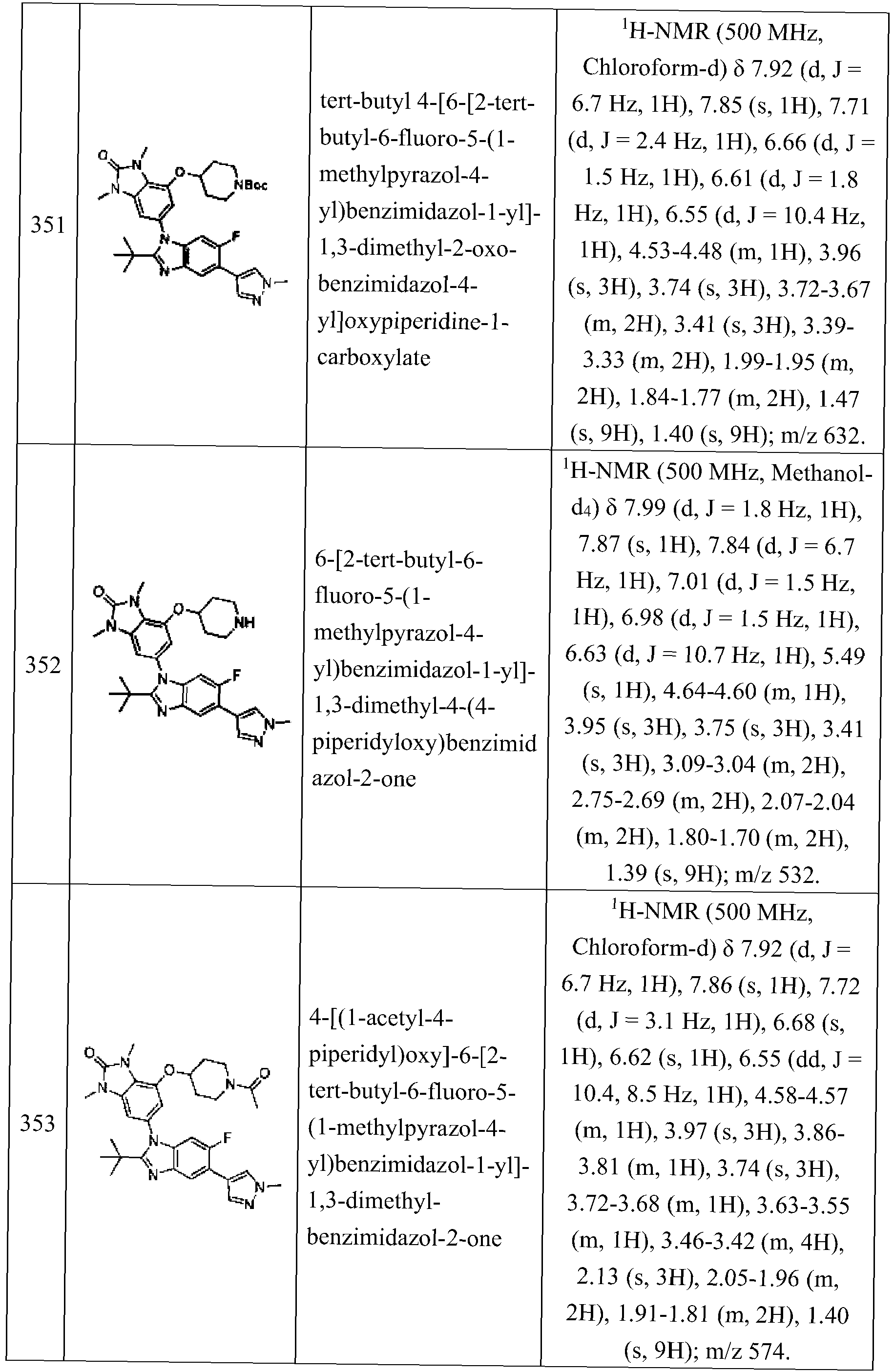

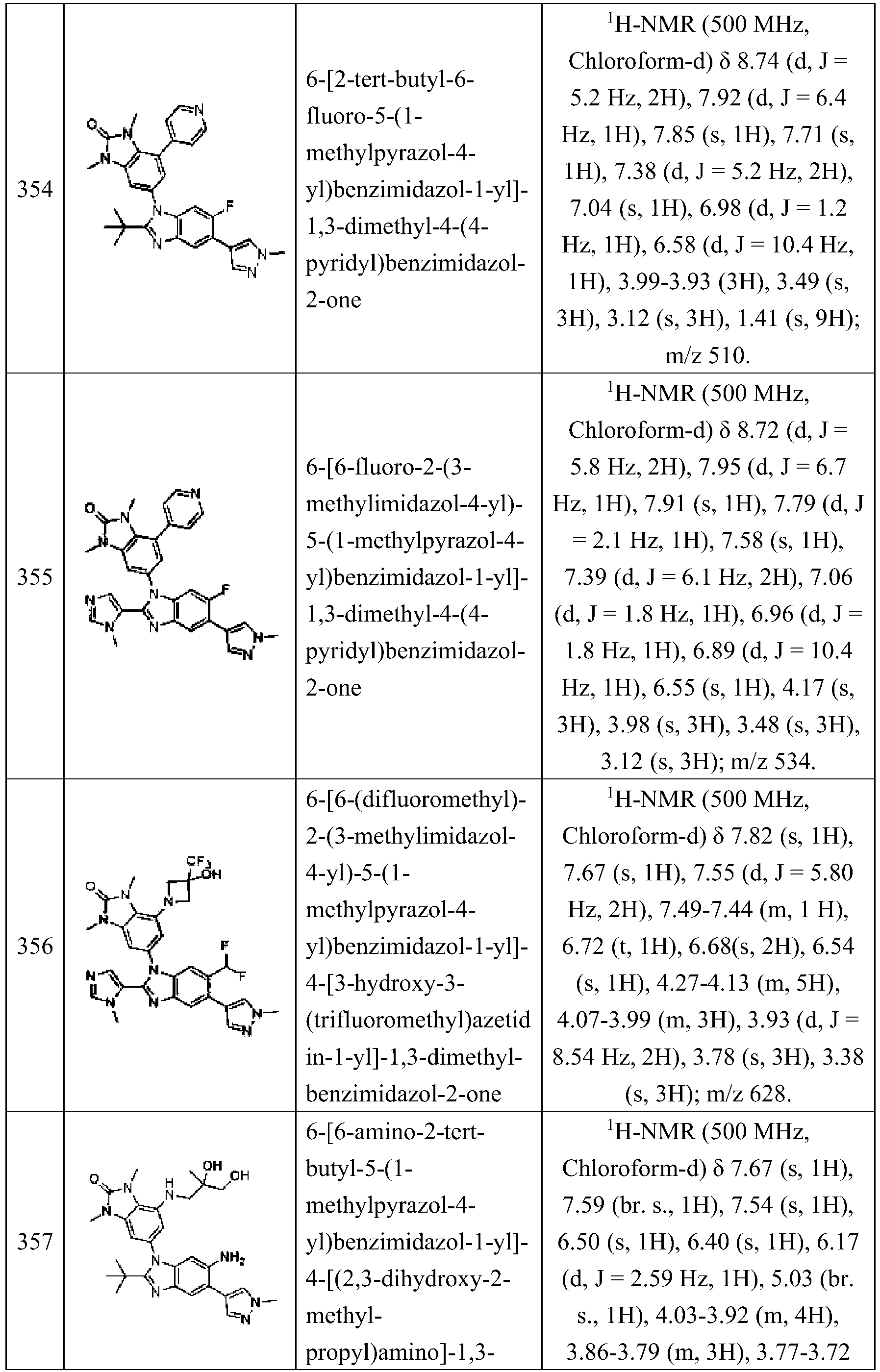

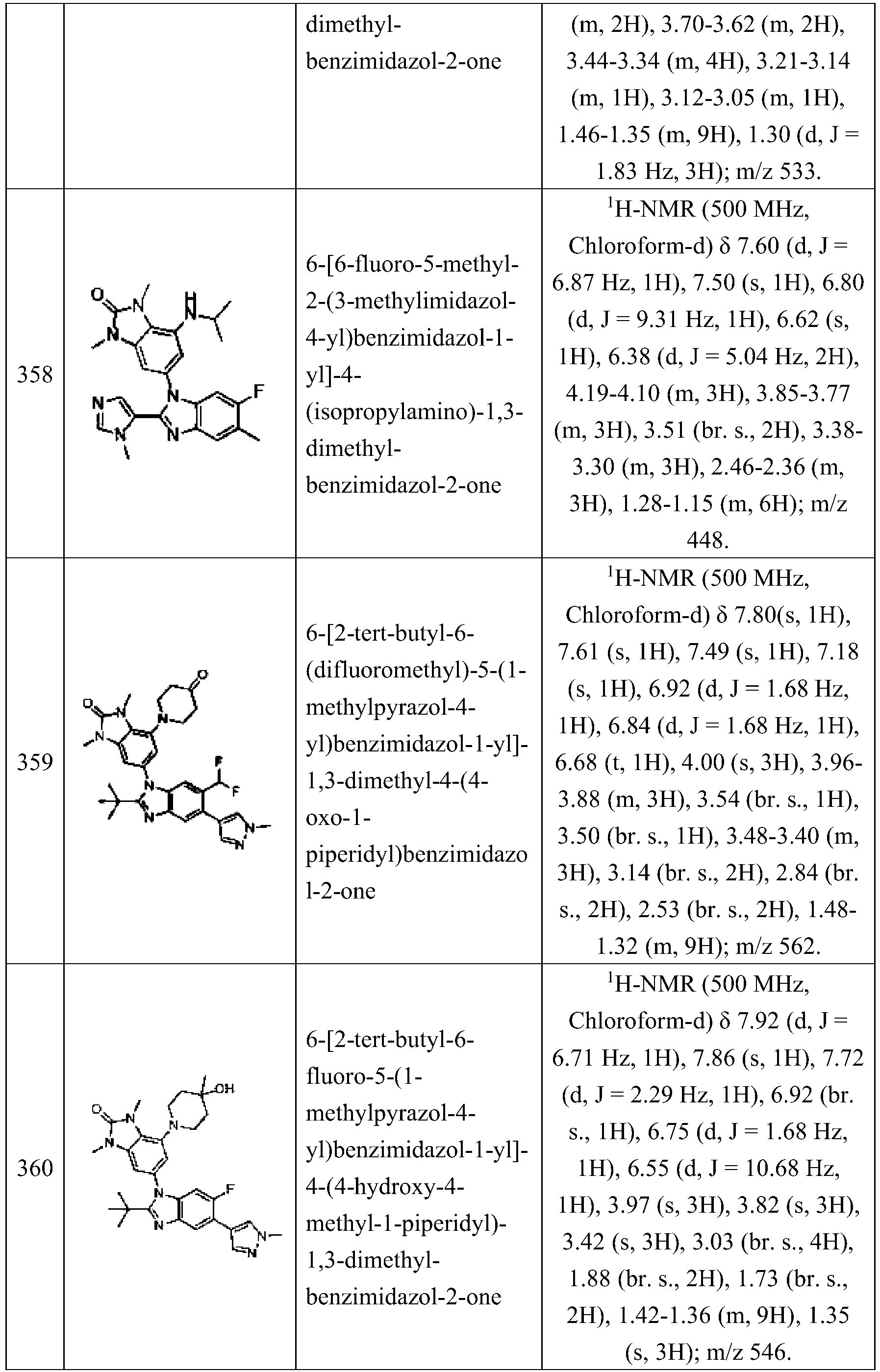

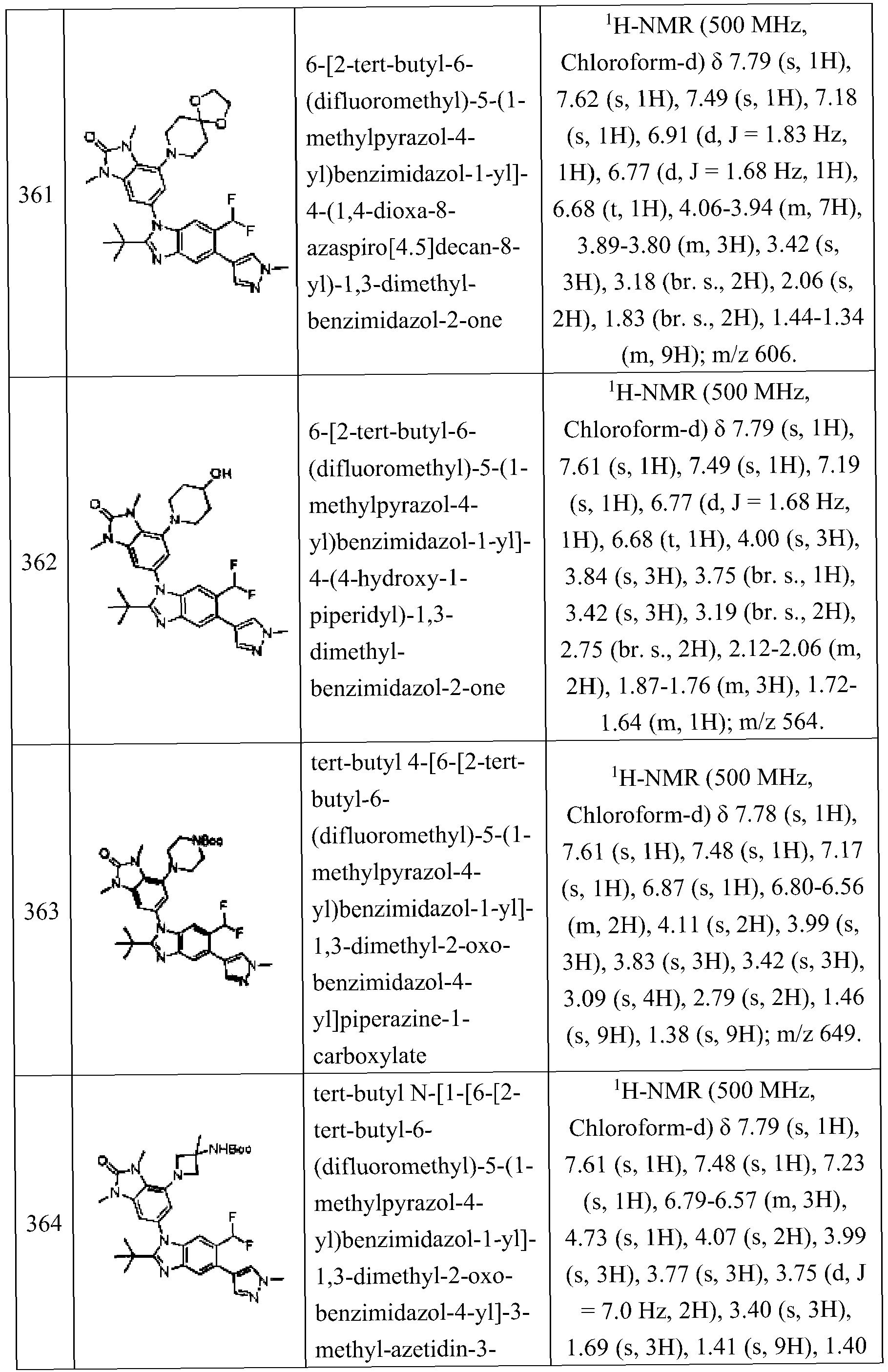

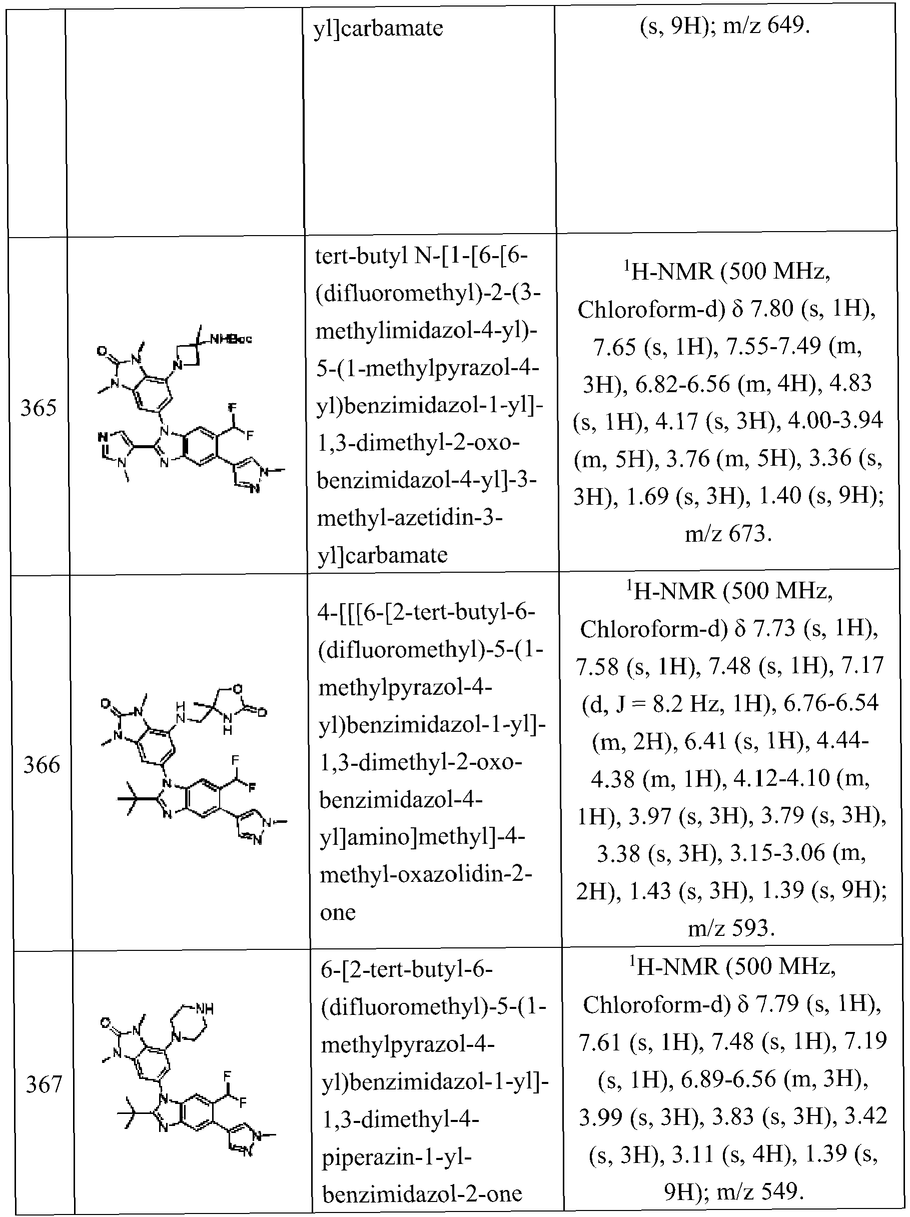

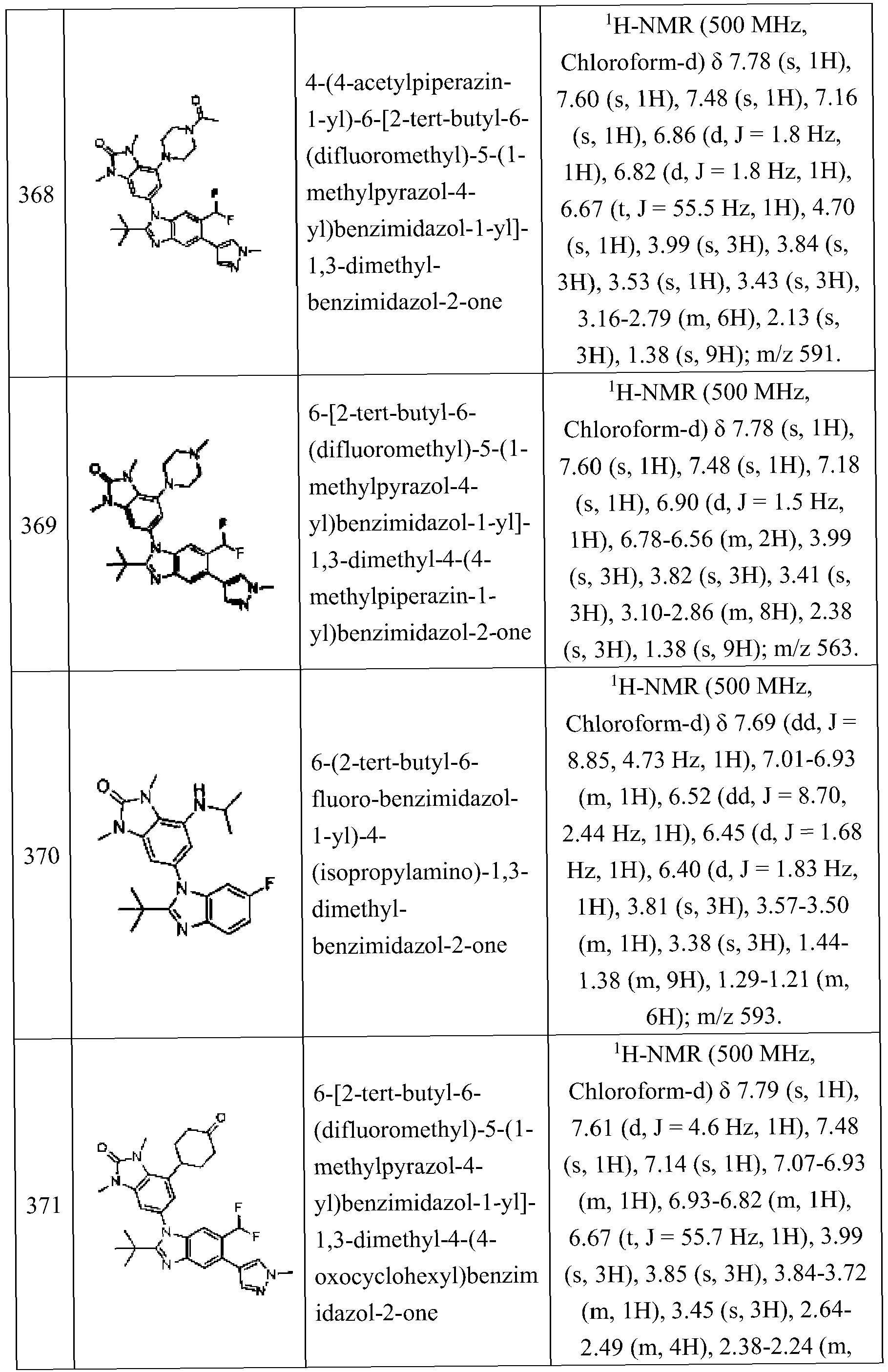

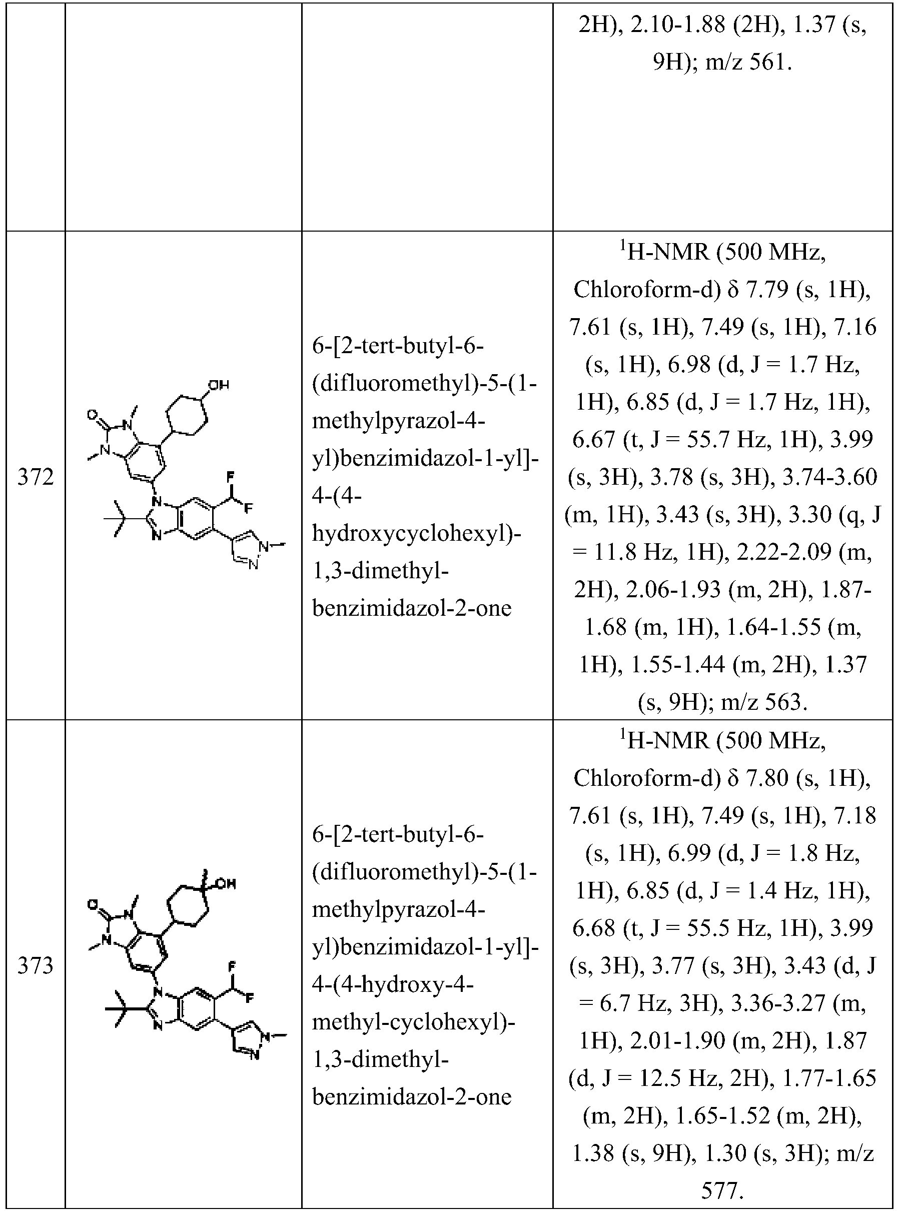

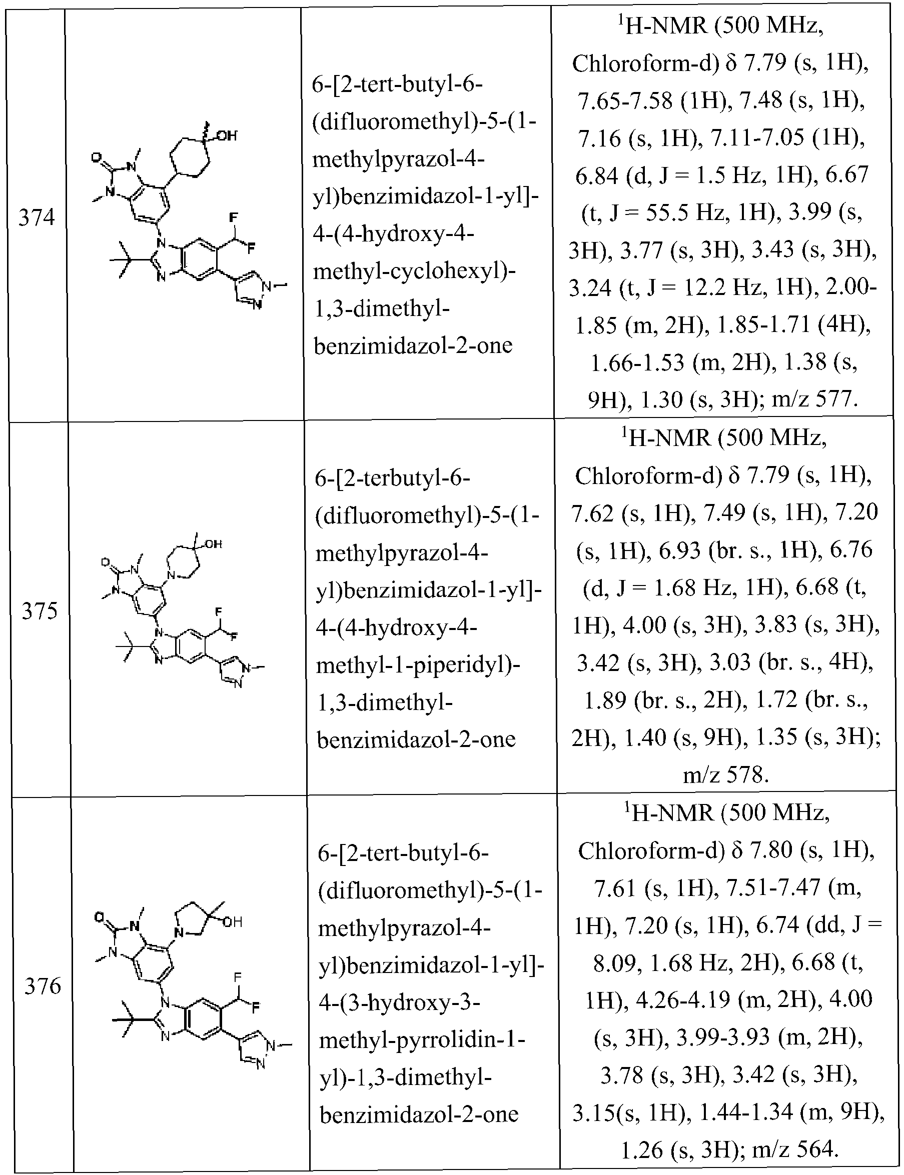

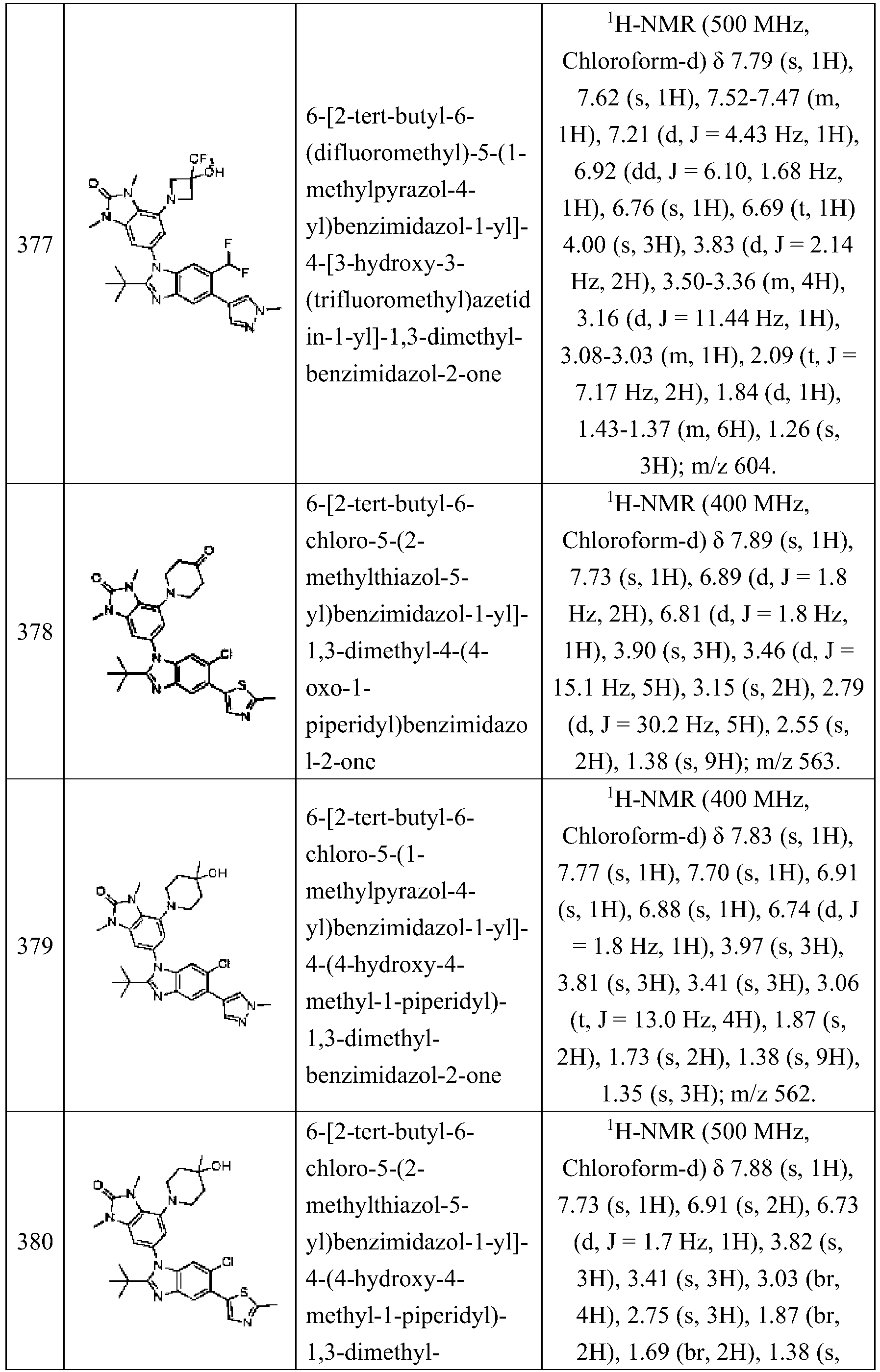

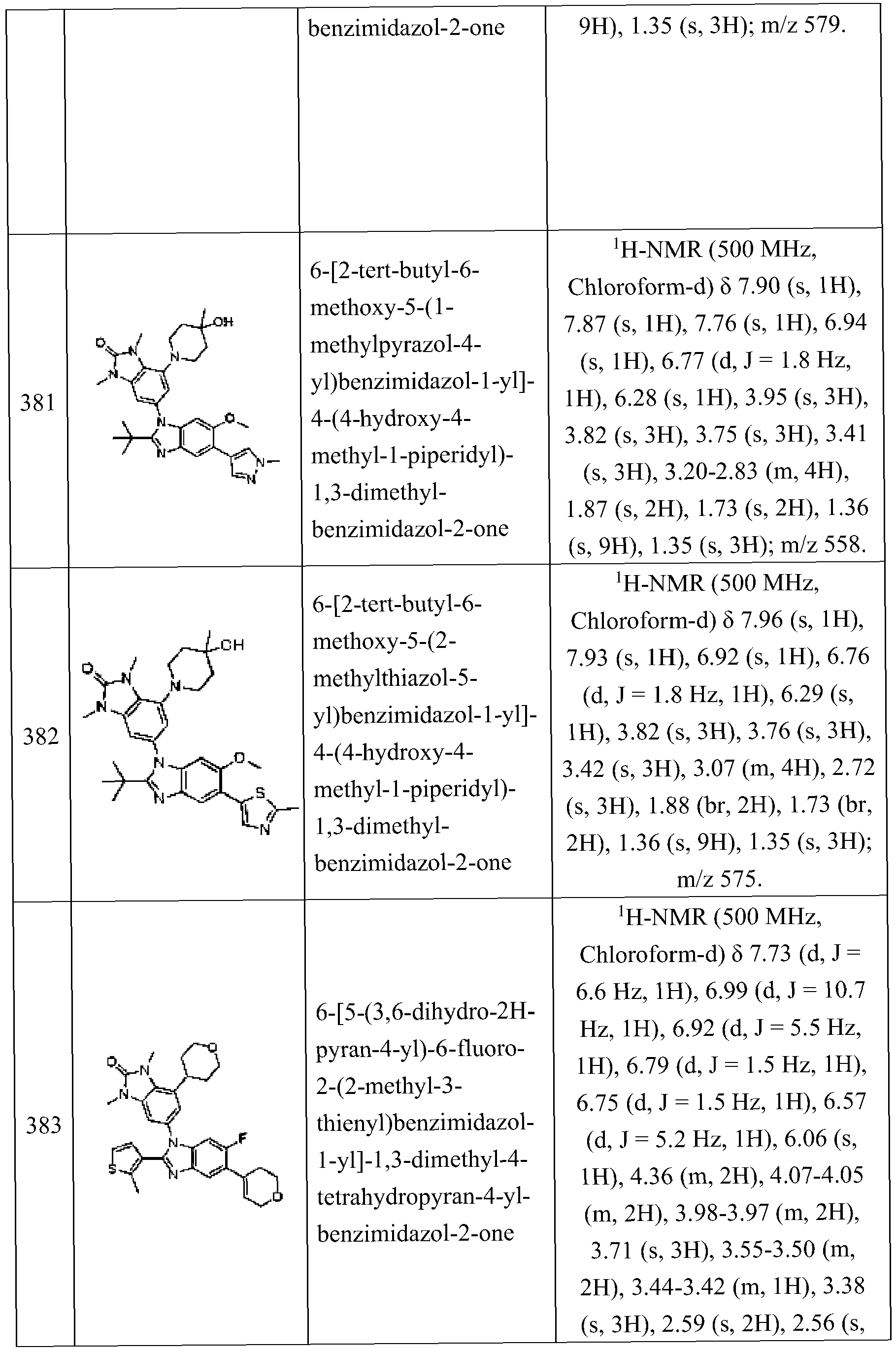

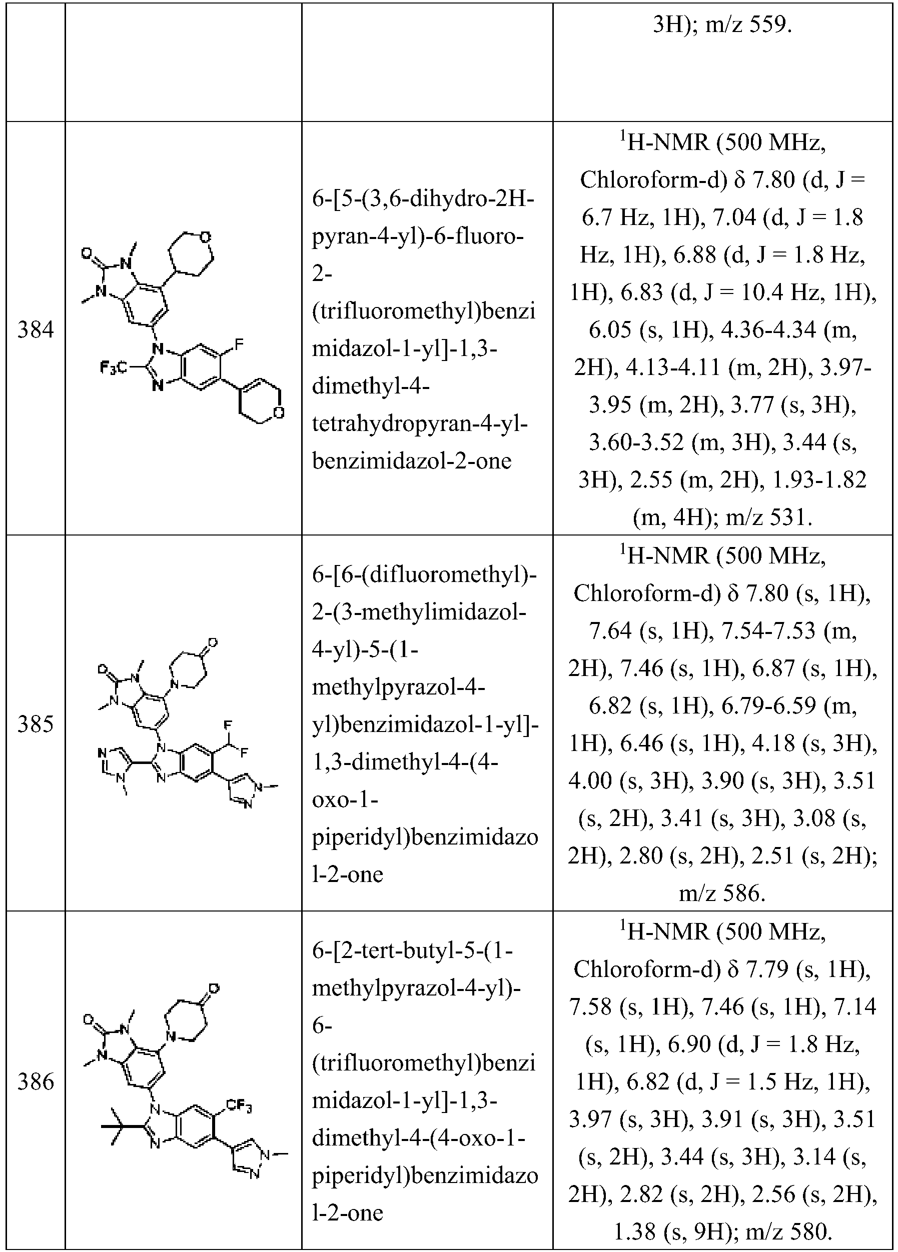

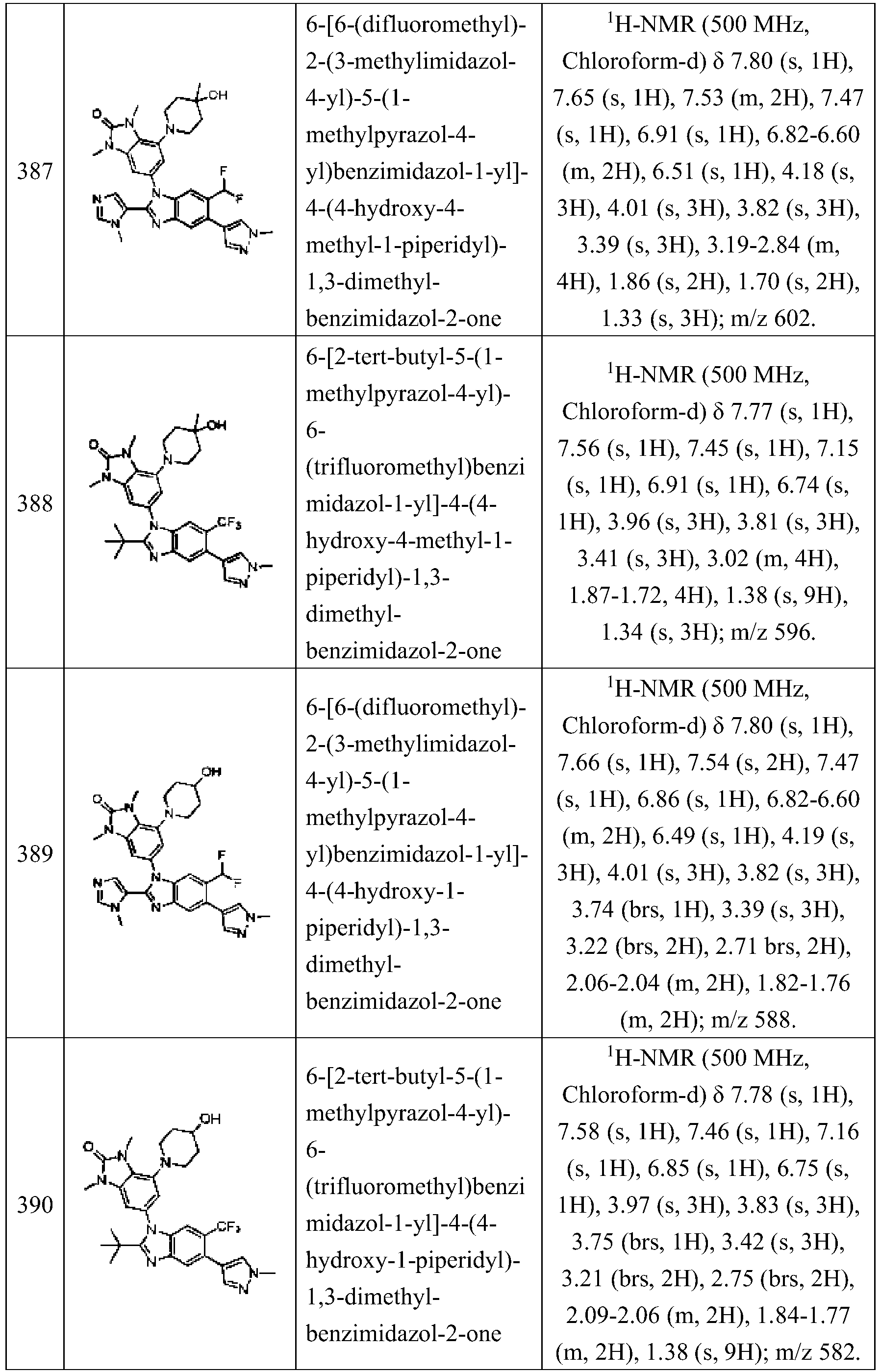

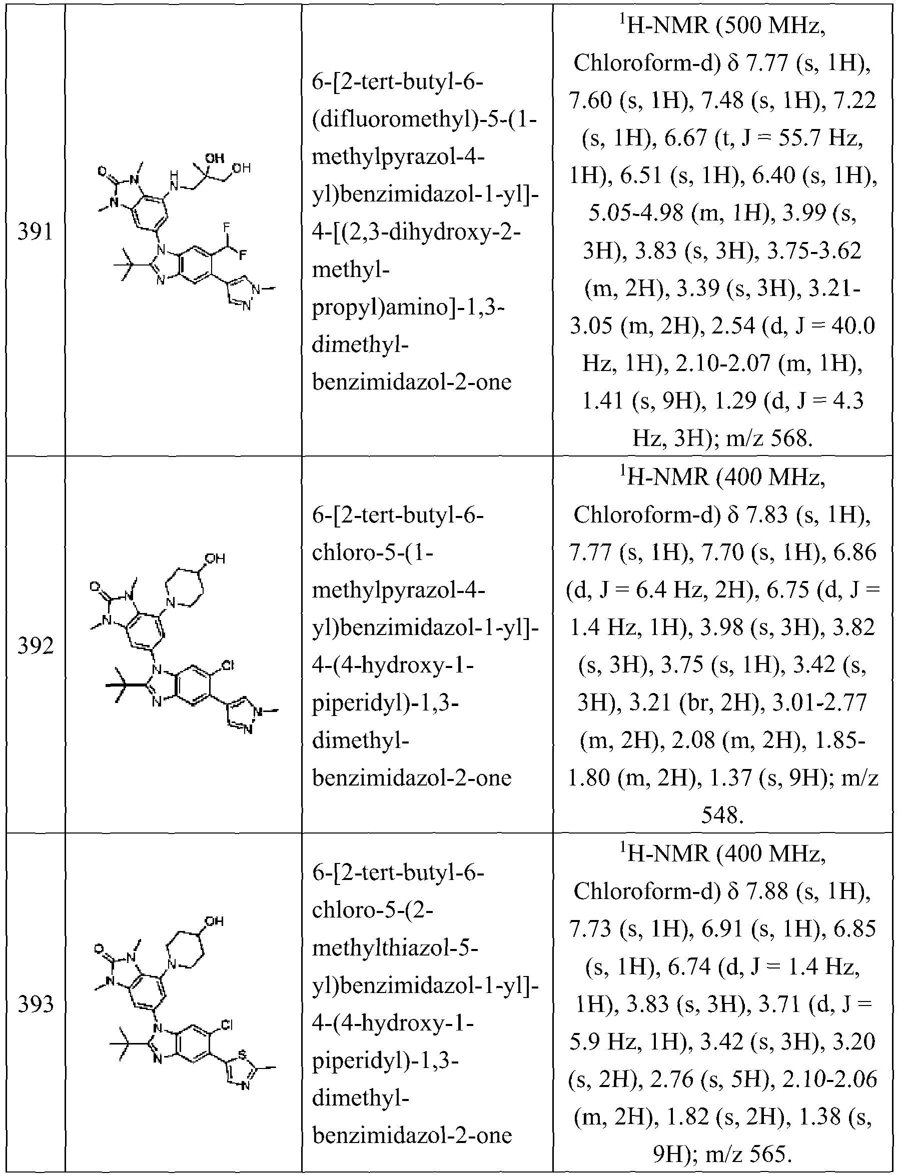

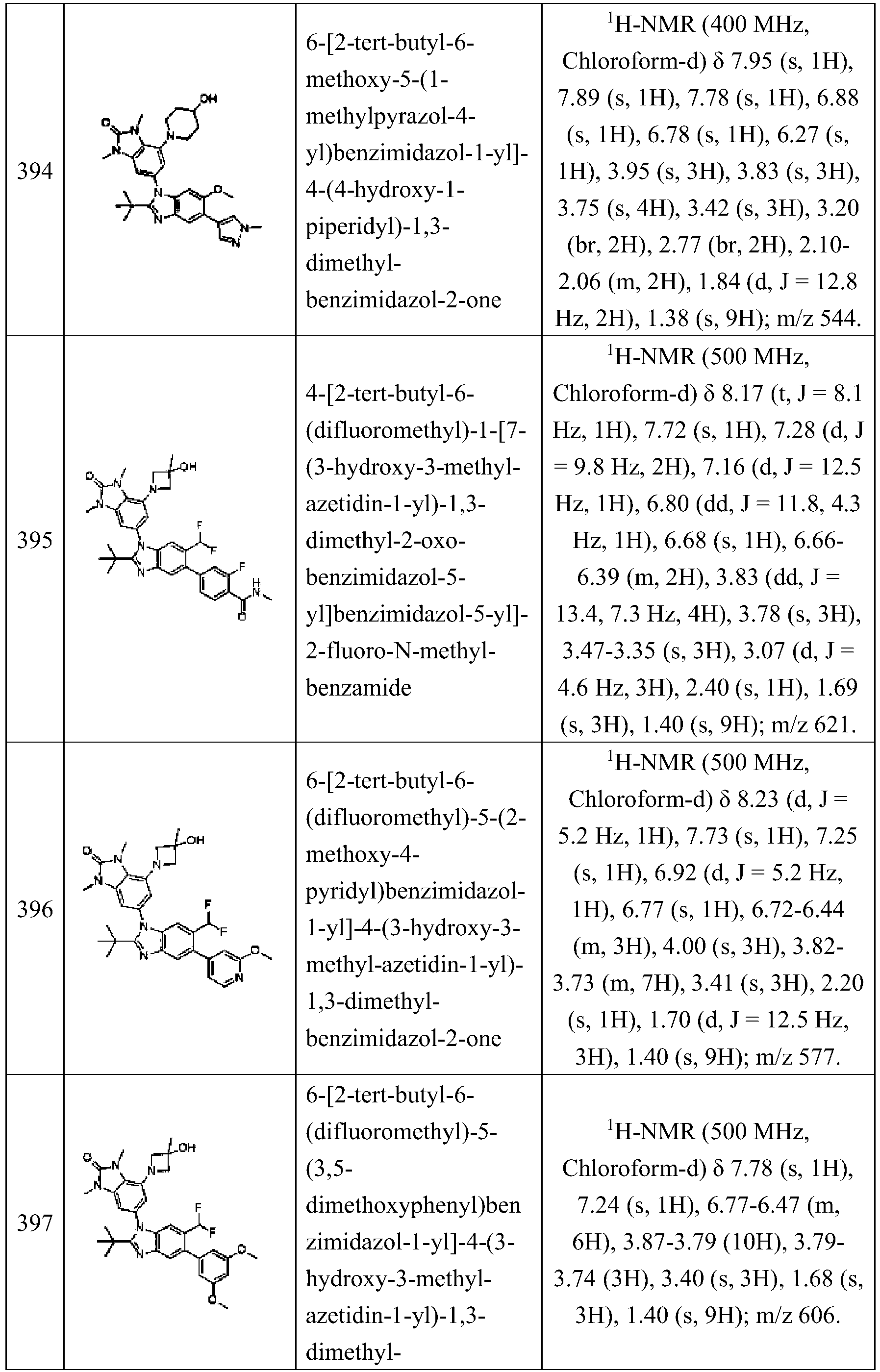

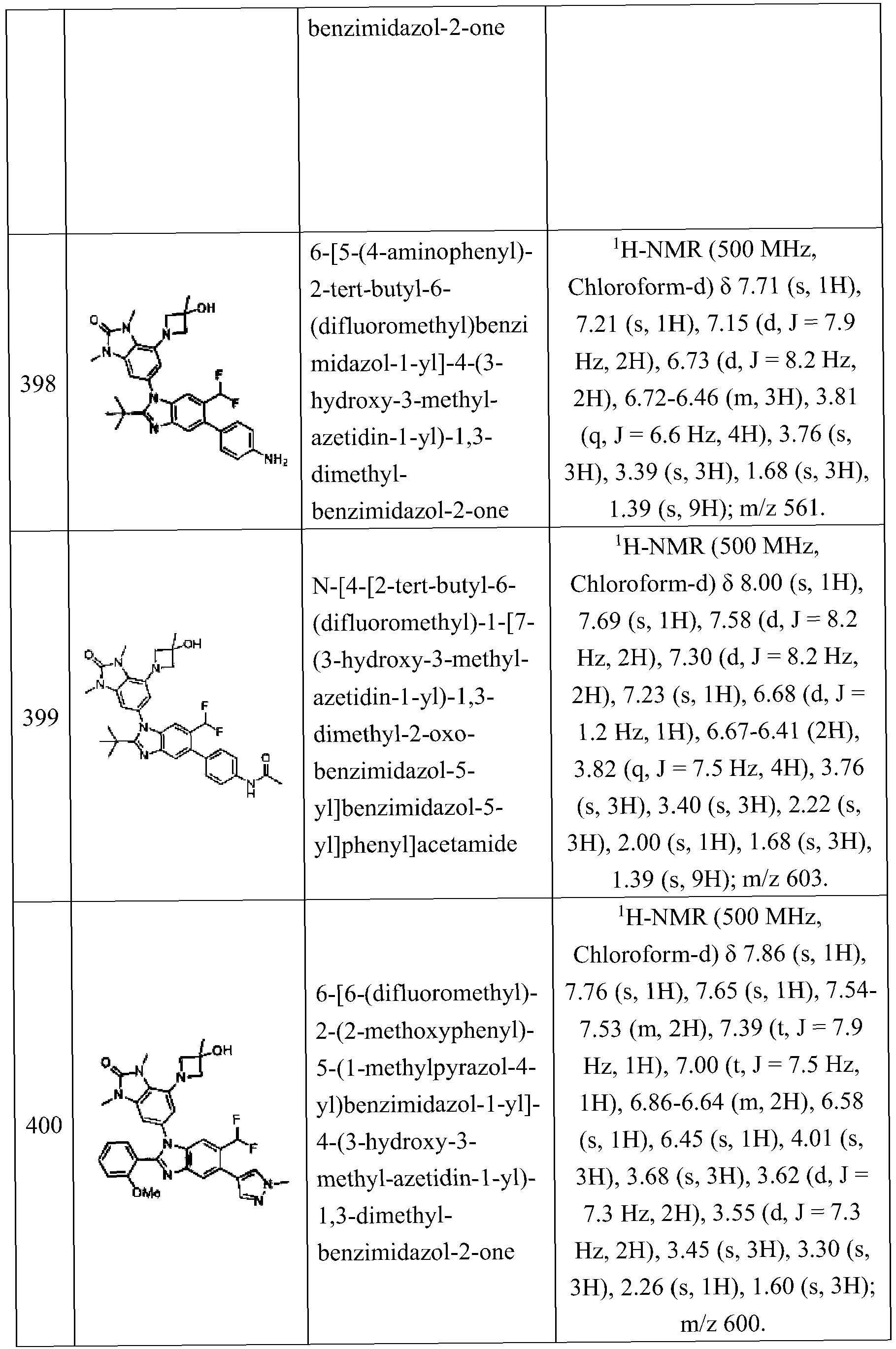

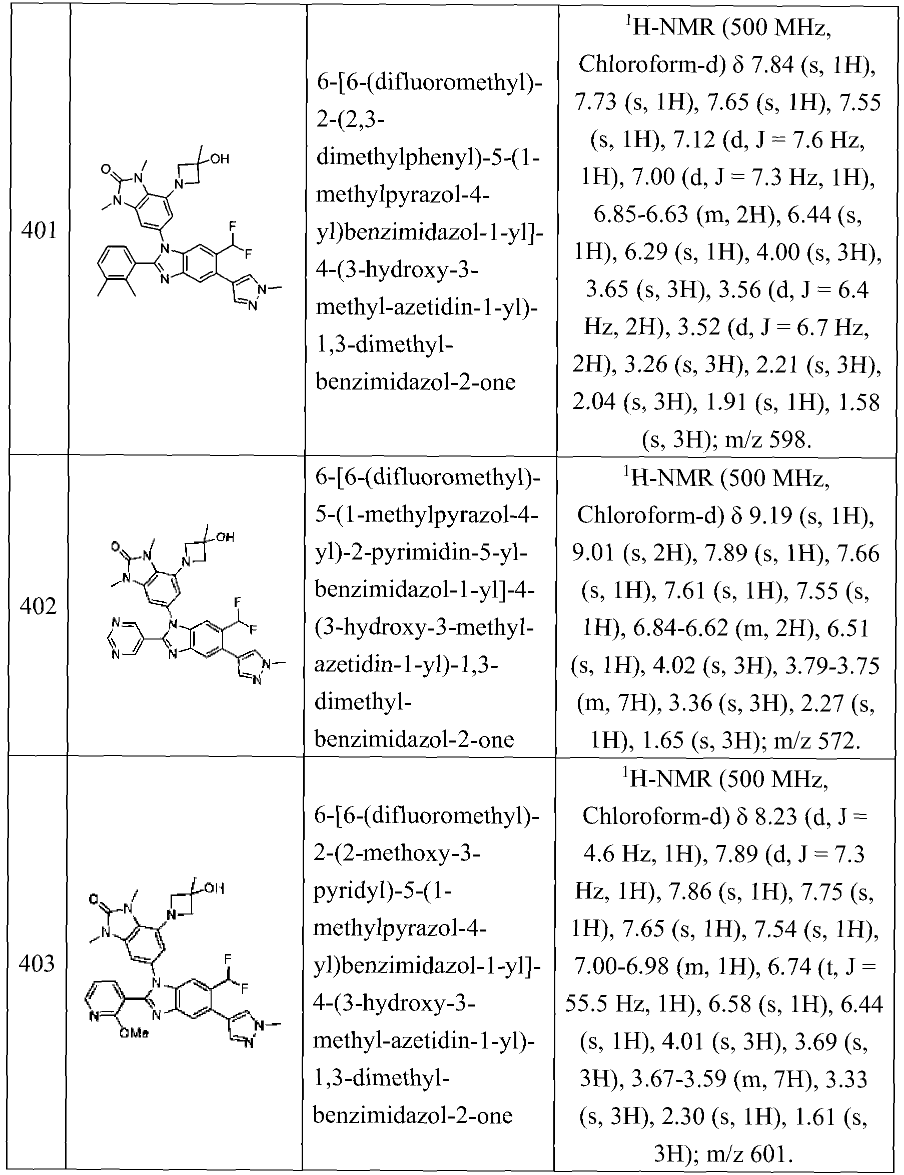

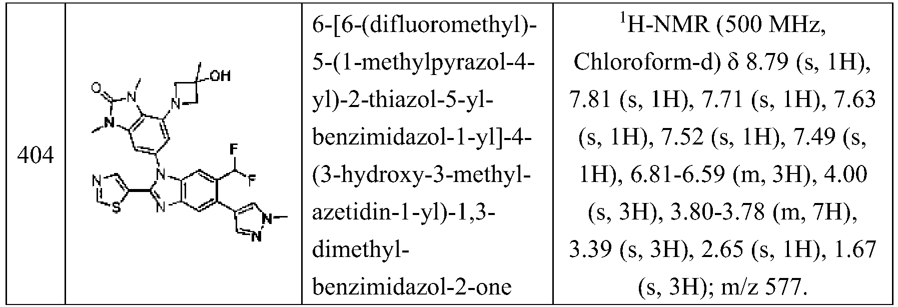

(Each of Examples 335 and 336, and Examples 373 and 374 differ in that the cyclohexyl group is cis or trans)

### Experimental Example 1. CBP Inhibitory Activity

A time resolved fluorescence resonance energy transfer (TR-FRET) experiment was used to verify the activity of CBP bromodomain.

The assay was performed in a final volume of 14 µL, optimized based on a CREBBP time resolved fluorescence resonance energy transfer (TR-FRET) assay kit (BPS Bioscience, cat. 32619) to maximize an assay window, and performed in a final volume of 14 µL, while maximizing the assay window. A TR-FRET fluorescence acceptor and a Tb donor were diluted in an assay buffer containing 2-[4-(2-hydroxyethyl)piperazin-1-yl]ethanesulfonic acid (HEPES) (hereafter, referred to as an assay buffer, BPS Bioscience, cat. 33012), and 3 µL of each solution was transferred to all wells of a white 384-low-volume well plate (Corning, cat. 4513). 2 µL of 4X Ac-H4 peptide (final concentration 85 nM) was transferred to high control wells and test wells. Each compound was prepared at 10 concentration points by serially diluting 3-fold from the highest concentration in a dimethyl sulfoxide solvent. The compound at each concentration was then diluted in the assay buffer to prepare a diluted compound solution that was 4-fold more concentrated than the final concentration. 2 µL of each compound or dimethyl sulfoxide prepared in this way was distributed into an assay plate so that the final test concentration was a minimum of 0.5 µM to a maximum of 10 µM (final dimethyl sulfoxide concentration 1%). 6 µL of 1X assay buffer was added to low control wells and 2 µl was added to high control and test wells. Then, 2 µL of 4X CBP BRD protein (final concentration 7.5 nM, BPS bioscience, cat 31128) was added to initiate a PPI reaction. The plate was reacted at room temperature for 2 hours before measuring the signal. The TR-FRET signal was measured using an EnVision Multilabel plate reader (PerkinElmer; Mirror LNACE/DELFIA Dual/Bias). For data analysis, the values of each well were obtained using a TR-FRET ratio ((Signal 665 nm/Signal 615 nm)×20000). Data analysis was performed using the TR-FRET ratio ((Signal 665 nm/Signal 615 nm)×20000). Data were reported as percent inhibition compared to control wells based on the following equation: %Activity = ((FRETcompound - FRETLow av)/(FRETHigh av - FRETLow av))×100. IC50 analysis was determined by standard 4 parameter curve fitting according to an algorithm using GraphPad Prism 7.0 (GraphPad Software Inc., La Jolla, CA, USA). All data are expressed as mean ± standard error of the mean (SEM).

### Experimental Example 2. BRD Inhibitory Activity

BRD inhibitory activity was measured using a time resolved fluorescence resonance energy transfer (TR-FRET) experiment to verify the activity of BRD4 bromodomain.

The assay was performed in a final volume of 14 µL, optimized based on a CREBBP time resolved fluorescence resonance energy transfer (TR-FRET) assay kit (BPS Bioscience, cat. 32613) to maximize an assay window, and performed in a final volume of 14 µL, while maximizing the assay window. A TR-FRET fluorescence acceptor and a Tb donor were diluted in an assay buffer containing 2-[4-(2-hydroxyethyl)piperazin-1-yl]ethanesulfonic acid (HEPES) (hereafter, referred to as an assay buffer, BPS Bioscience, cat. 33012), and 3 µL of each solution was transferred to all wells of a white 384-low-volume well plate (Corning, cat. 4513). 2 µL of 4X Ac-H4 peptide (final concentration 15 nM) was transferred to high control wells and test wells. Each compound was prepared at 10 concentration points by serially diluting 3-fold from the highest concentration in a dimethyl sulfoxide (DMSO) solvent. The compound at each concentration was then diluted in the assay buffer to prepare a diluted compound solution that was 4-fold more concentrated than the final concentration. 2 µL of each compound or dimethyl sulfoxide prepared in this way was distributed into an assay plate so that the final test concentration was a minimum of 0.5 µM to a maximum of 10 µM (final DMSO concentration 1%). 6 µL of 1X assay buffer was added to low control wells and 2 µL was added to high control and test wells. Then, 2 µL of 4X BRD4 (BD1) protein (final concentration 7.5 nM, BPS bioscience, cat 31040) was added to initiate a PPI reaction. The plate was reacted at room temperature for 2 hours before measuring the signal. The TR-FRET signal was measured using an EnVision Multilabel plate reader (PerkinElmer; Mirror LNACE/DELFIA Dual/Bias). For data analysis, the values of each well were obtained using the TR-FRET ratio ((Signal 665 nm/Signal 615 nm)×20000). Data analysis was performed using the TR-FRET ratio ((Signal 665 nm/Signal 615 nm)×20000). Data were reported as percent inhibition compared to control wells based on the following equation: %Activity = ((FRETcompound - FRETLow av)/(FRETHigh av - FRETLow av))×100. IC50 analysis was determined by standard 4 parameter curve fitting according to an algorithm using GraphPad Prism 7.0 (GraphPad Software Inc., La Jolla, CA, USA). All data are expressed as mean ± standard error of the mean (SEM).

The results according to Experimental Examples 1 and 2 are shown in Tables 3 and 4 below.

In Tables 3 and 4 below, a case where the activity value is 0 to 500 nM is indicated as "+++", a case with greater than 500 nM to 5 µM is indicated as "++", and a case with greater than 5 µM is indicated as "+."

**[Table 3]**

| **Example** | **CBP BRD IC₅₀** | **Example** | **CBP BRD IC₅₀** |
|---|---|---|---|
| 1 | N.D. | 203 | +++ |
| 2 | +++ | 204 | +++ |
| 3 | ++ | 205 | +++ |
| 4 | +++ | 206 | +++ |
| 5 | ++ | 207 | +++ |
| 6 | +++ | 208 | +++ |
| 7 | +++ | 209 | +++ |
| 8 | +++ | 210 | +++ |
| 9 | +++ | 211 | +++ |
| 10 | +++ | 212 | +++ |
| 11 | +++ | 213 | +++ |
| 12 | +++ | 214 | +++ |
| 13 | + | 215 | +++ |
| 14 | ++ | 216 | +++ |
| 15 | + | 217 | +++ |
| 16 | ++ | 218 | +++ |
| 17 | + | 219 | +++ |
| 18 | + | 220 | +++ |
| 19 | ++ | 221 | +++ |
| 20 | +++ | 222 | +++ |
| 21 | +++ | 223 | +++ |
| 22 | +++ | 224 | +++ |
| 23 | +++ | 225 | +++ |
| 24 | +++ | 226 | +++ |
| 25 | ++ | 227 | +++ |
| 26 | ++ | 228 | +++ |
| 27 | +++ | 229 | +++ |
| 28 | +++ | 230 | +++ |
| 29 | +++ | 231 | +++ |
| 30 | +++ | 232 | +++ |
| 31 | +++ | 233 | +++ |
| 32 | +++ | 234 | +++ |
| 33 | +++ | 235 | +++ |
| 34 | +++ | 236 | +++ |
| 35 | +++ | 237 | +++ |
| 36 | +++ | 238 | +++ |
| 37 | +++ | 239 | +++ |
| 38 | ++ | 240 | +++ |
| 39 | ++ | 241 | +++ |
| 40 | +++ | 242 | +++ |
| 41 | +++ | 243 | +++ |
| 42 | +++ | 244 | +++ |
| 43 | +++ | 245 | +++ |
| 44 | +++ | 246 | +++ |
| 45 | +++ | 247 | +++ |
| 46 | +++ | 248 | +++ |
| 47 | +++ | 249 | +++ |
| 48 | ++ | 250 | +++ |
| 49 | +++ | 251 | +++ |
| 50 | ++ | 252 | +++ |
| 51 | ++ | 253 | +++ |
| 52 | ++ | 254 | +++ |
| 53 | +++ | 255 | +++ |
| 54 | +++ | 256 | +++ |
| 55 | +++ | 257 | +++ |
| 56 | +++ | 258 | +++ |
| 57 | +++ | 259 | +++ |
| 58 | +++ | 260 | +++ |
| 59 | +++ | 261 | +++ |
| 60 | +++ | 262 | +++ |
| 61 | +++ | 263 | +++ |
| 62 | +++ | 264 | +++ |
| 63 | +++ | 265 | +++ |
| 64 | +++ | 266 | +++ |
| 65 | +++ | 267 | +++ |
| 66 | +++ | 268 | +++ |
| 67 | +++ | 269 | +++ |
| 68 | +++ | 270 | +++ |
| 69 | +++ | 271 | +++ |
| 70 | ++ | 272 | +++ |
| 71 | ++ | 273 | ++ |
| 72 | +++ | 274 | ++ |
| 73 | +++ | 275 | ++ |
| 74 | ++ | 276 | +++ |
| 75 | ++ | 277 | +++ |
| 76 | +++ | 278 | +++ |
| 77 | +++ | 279 | ++ |
| 78 | +++ | 280 | +++ |
| 79 | +++ | 281 | +++ |
| 80 | +++ | 282 | +++ |
| 81 | +++ | 283 | +++ |
| 82 | +++ | 284 | +++ |
| 83 | +++ | 285 | +++ |
| 84 | +++ | 286 | ++ |
| 85 | +++ | 287 | +++ |
| 86 | +++ | 288 | +++ |
| 87 | +++ | 289 | +++ |
| 88 | +++ | 290 | +++ |
| 89 | +++ | 291 | +++ |
| 90 | ++ | 292 | +++ |
| 91 | +++ | 293 | +++ |
| 92 | +++ | 294 | +++ |
| 93 | +++ | 295 | +++ |
| 94 | +++ | 296 | +++ |
| 95 | +++ | 297 | +++ |
| 96 | +++ | 298 | +++ |
| 97 | +++ | 299 | +++ |
| 98 | +++ | 300 | +++ |
| 99 | +++ | 301 | +++ |
| 100 | +++ | 302 | +++ |
| 101 | +++ | 303 | +++ |
| 102 | +++ | 304 | +++ |
| 103 | +++ | 305 | +++ |
| 104 | ++ | 306 | +++ |
| 105 | ++ | 307 | +++ |
| 106 | +++ | 308 | +++ |
| 107 | +++ | 309 | +++ |
| 108 | +++ | 310 | +++ |
| 109 | +++ | 311 | +++ |
| 110 | ++ | 312 | +++ |
| 111 | ++ | 313 | +++ |
| 112 | +++ | 314 | +++ |
| 113 | +++ | 315 | +++ |
| 114 | +++ | 316 | + |
| 115 | +++ | 317 | +++ |
| 116 | ++ | 318 | ++ |
| 117 | ++ | 319 | + |
| 118 | ++ | 320 | ++ |
| 119 | + | 321 | + |
| 120 | +++ | 322 | +++ |
| 121 | +++ | 323 | +++ |
| 122 | ++ | 324 | +++ |
| 123 | +++ | 325 | +++ |
| 124 | ++ | 326 | +++ |
| 125 | ++ | 327 | +++ |
| 126 | ++ | 328 | +++ |
| 127 | +++ | 329 | +++ |
| 128 | ++ | 330 | +++ |
| 129 | ++ | 331 | +++ |
| 130 | +++ | 332 | +++ |
| 131 | ++ | 333 | +++ |
| 132 | +++ | 334 | +++ |
| 133 | +++ | 335 | +++ |
| 134 | +++ | 336 | +++ |
| 135 | +++ | 337 | +++ |
| 136 | +++ | 338 | +++ |
| 137 | +++ | 339 | +++ |
| 138 | +++ | 340 | +++ |
| 139 | +++ | 341 | +++ |
| 140 | +++ | 342 | +++ |
| 141 | +++ | 343 | + |
| 142 | +++ | 344 | +++ |
| 143 | +++ | 345 | +++ |
| 144 | +++ | 346 | +++ |
| 145 | +++ | 347 | +++ |
| 146 | +++ | 348 | +++ |
| 147 | +++ | 349 | +++ |
| 148 | ++ | 350 | +++ |
| 149 | ++ | 351 | +++ |
| 150 | +++ | 352 | +++ |
| 151 | + | 353 | +++ |
| 152 | +++ | 354 | +++ |
| 153 | +++ | 355 | +++ |
| 154 | +++ | 356 | +++ |
| 155 | +++ | 357 | +++ |
| 156 | +++ | 358 | ++ |
| 157 | +++ | 359 | +++ |
| 158 | +++ | 360 | +++ |
| 159 | +++ | 361 | +++ |
| 160 | +++ | 362 | +++ |
| 161 | ++ | 363 | +++ |
| 162 | ++ | 364 | +++ |
| 163 | +++ | 365 | +++ |
| 164 | +++ | 366 | +++ |
| 165 | +++ | 367 | +++ |
| 166 | +++ | 368 | +++ |
| 167 | +++ | 369 | +++ |
| 168 | +++ | 370 | + |
| 169 | +++ | 371 | +++ |
| 170 | +++ | 372 | +++ |
| 171 | +++ | 373 | +++ |
| 172 | +++ | 374 | +++ |
| 173 | +++ | 375 | +++ |
| 174 | +++ | 376 | +++ |
| 175 | ++ | 377 | +++ |
| 176 | +++ | 378 | +++ |
| 177 | +++ | 379 | +++ |
| 178 | +++ | 380 | +++ |
| 179 | +++ | 381 | +++ |
| 180 | +++ | 382 | +++ |
| 181 | +++ | 383 | ++ |
| 182 | +++ | 384 | + |
| 183 | +++ | 385 | +++ |
| 184 | +++ | 386 | +++ |
| 185 | +++ | 387 | +++ |
| 186 | +++ | 388 | +++ |
| 187 | +++ | 389 | +++ |
| 188 | +++ | 390 | +++ |
| 189 | +++ | 391 | +++ |
| 190 | +++ | 392 | +++ |
| 191 | +++ | 393 | +++ |
| 192 | +++ | 394 | +++ |
| 193 | +++ | 395 | +++ |
| 194 | +++ | 396 | +++ |
| 195 | +++ | 397 | +++ |
| 196 | +++ | 398 | +++ |
| 197 | +++ | 399 | +++ |
| 198 | +++ | 400 | +++ |
| 199 | +++ | 401 | +++ |
| 200 | +++ | 402 | +++ |
| 201 | +++ | 403 | +++ |
| 202 | +++ | 404 | +++ |

**[Table 4]**

| **Example** | **BRD4 (BD1) IC₅₀** | **Example** | **BRD4 (BD1) IC₅₀** |
|---|---|---|---|
| 1 | N.D. | 203 | + |
| 2 | + | 204 | + |
| 3 | ++ | 205 | + |
| 4 | ++ | 206 | + |
| 5 | ++ | 207 | + |
| 6 | + | 208 | + |
| 7 | ++ | 209 | + |
| 8 | ++ | 210 | + |
| 9 | + | 211 | + |
| 10 | + | 212 | + |
| 11 | + | 213 | + |
| 12 | ++ | 214 | + |
| 13 | + | 215 | + |
| 14 | + | 216 | + |
| 15 | + | 217 | + |
| 16 | + | 218 | ++ |
| 17 | + | 219 | ++ |
| 18 | + | 220 | + |
| 19 | + | 221 | + |
| 20 | ++ | 222 | ++ |
| 21 | + | 223 | + |
| 22 | + | 224 | ++ |
| 23 | + | 225 | + |
| 24 | + | 226 | + |
| 25 | ++ | 227 | + |
| 26 | + | 228 | ++ |
| 27 | + | 229 | + |
| 28 | ++ | 230 | + |
| 29 | ++ | 231 | ++ |
| 30 | + | 232 | + |
| 31 | + | 233 | + |
| 32 | ++ | 234 | + |
| 33 | ++ | 235 | + |
| 34 | + | 236 | + |
| 35 | + | 237 | + |
| 36 | + | 238 | ++ |
| 37 | + | 239 | + |
| 38 | + | 240 | + |
| 39 | + | 241 | + |
| 40 | + | 242 | + |
| 41 | ++ | 243 | + |
| 42 | + | 244 | + |
| 43 | + | 245 | + |
| 44 | ++ | 246 | + |
| 45 | + | 247 | + |
| 46 | ++ | 248 | + |
| 47 | + | 249 | ++ |
| 48 | + | 250 | + |
| 49 | + | 251 | + |
| 50 | + | 252 | + |
| 51 | + | 253 | ++ |
| 52 | + | 254 | + |
| 53 | + | 255 | + |
| 54 | + | 256 | + |
| 55 | + | 257 | + |
| 56 | + | 258 | + |
| 57 | + | 259 | + |
| 58 | + | 260 | + |
| 59 | ++ | 261 | + |
| 60 | ++ | 262 | + |
| 61 | ++ | 263 | + |
| 62 | ++ | 264 | + |
| 63 | + | 265 | + |
| 64 | + | 266 | ++ |
| 65 | + | 267 | + |
| 66 | + | 268 | + |
| 67 | ++ | 269 | + |
| 68 | ++ | 270 | + |
| 69 | ++ | 271 | ++ |
| 70 | + | 272 | + |
| 71 | + | 273 | + |
| 72 | + | 274 | + |
| 73 | ++ | 275 | + |
| 74 | + | 276 | + |
| 75 | + | 277 | +++ |
| 76 | + | 278 | + |
| 77 | + | 279 | ++ |
| 78 | + | 280 | + |
| 79 | + | 281 | + |
| 80 | + | 282 | ++ |
| 81 | + | 283 | + |
| 82 | + | 284 | ++ |
| 83 | + | 285 | + |
| 84 | + | 286 | + |
| 85 | + | 287 | + |
| 86 | + | 288 | + |
| 87 | + | 289 | + |
| 88 | + | 290 | + |
| 89 | + | 291 | + |
| 90 | + | 292 | + |
| 91 | + | 293 | + |
| 92 | + | 294 | + |
| 93 | + | 295 | ++ |
| 94 | + | 296 | +++ |
| 95 | + | 297 | + |
| 96 | + | 298 | + |
| 97 | + | 299 | + |
| 98 | + | 300 | + |
| 99 | + | 301 | + |
| 100 | + | 302 | + |
| 101 | + | 303 | ++ |
| 102 | + | 304 | + |
| 103 | + | 305 | ++ |
| 104 | + | 306 | ++ |
| 105 | + | 307 | + |
| 106 | + | 308 | +++ |
| 107 | ++ | 309 | + |
| 108 | + | 310 | + |
| 109 | + | 311 | + |
| 110 | + | 312 | + |
| 111 | + | 313 | + |
| 112 | + | 314 | + |
| 113 | + | 315 | + |
| 114 | + | 316 | + |
| 115 | + | 317 | + |
| 116 | + | 318 | + |
| 117 | + | 319 | + |
| 118 | + | 320 | + |
| 119 | + | 321 | + |
| 120 | + | 322 | ++ |
| 121 | + | 323 | ++ |
| 122 | + | 324 | + |
| 123 | + | 325 | + |
| 124 | + | 326 | + |
| 125 | + | 327 | + |
| 126 | + | 328 | ++ |
| 127 | + | 329 | + |
| 128 | + | 330 | ++ |
| 129 | + | 331 | ++ |
| 130 | + | 332 | ++ |
| 131 | + | 333 | + |
| 132 | + | 334 | + |
| 133 | + | 335 | + |
| 134 | + | 336 | + |
| 135 | + | 337 | + |
| 136 | + | 338 | + |
| 137 | + | 339 | + |
| 138 | + | 340 | + |
| 139 | + | 341 | + |
| 140 | + | 342 | + |
| 141 | ++ | 343 | + |
| 142 | ++ | 344 | + |
| 143 | ++ | 345 | + |
| 144 | + | 346 | + |
| 145 | + | 347 | + |
| 146 | + | 348 | + |
| 147 | + | 349 | + |
| 148 | + | 350 | + |
| 149 | + | 351 | + |
| 150 | + | 352 | + |
| 151 | + | 353 | + |
| 152 | ++ | 354 | + |
| 153 | + | 355 | + |
| 154 | + | 356 | ++ |
| 155 | + | 357 | + |
| 156 | + | 358 | + |
| 157 | + | 359 | + |
| 158 | + | 360 | + |
| 159 | + | 361 | + |
| 160 | + | 362 | + |
| 161 | + | 363 | + |
| 162 | + | 364 | + |
| 163 | + | 365 | + |
| 164 | + | 366 | + |
| 165 | + | 367 | + |
| 166 | ++ | 368 | + |
| 167 | + | 369 | + |
| 168 | + | 370 | + |
| 169 | ++ | 371 | + |
| 170 | ++ | 372 | + |
| 171 | + | 373 | + |
| 172 | ++ | 374 | + |
| 173 | + | 375 | ++ |
| 174 | + | 376 | + |
| 175 | + | 377 | + |
| 176 | + | 378 | + |
| 177 | + | 379 | ++ |
| 178 | + | 380 | + |
| 179 | + | 381 | ++ |
| 180 | + | 382 | ++ |
| 181 | + | 383 | + |
| 182 | + | 384 | + |
| 183 | ++ | 385 | ++ |
| 184 | ++ | 386 | + |
| 185 | + | 387 | ++ |
| 186 | ++ | 388 | ++ |
| 187 | + | 389 | ++ |
| 188 | + | 390 | + |
| 189 | + | 391 | + |
| 190 | + | 392 | ++ |
| 191 | + | 393 | + |
| 192 | + | 394 | + |
| 193 | + | 395 | + |
| 194 | + | 396 | + |
| 195 | ++ | 397 | + |
| 196 | + | 398 | + |
| 197 | + | 399 | + |
| 198 | + | 400 | + |
| 199 | ++ | 401 | ++ |
| 200 | ++ | 402 | + |
| 201 | + | 403 | + |
| 202 | ++ | 404 | ++ |

## Claims

1. A compound represented by the following Formula I: 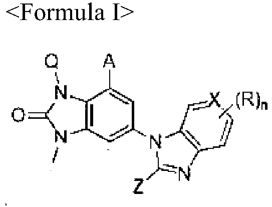 where
X is C or N,
n is an integer from 0 to 3, where if n is an integer of 2 or more, Rs are the same or different,
Rs are each independently C₁₋₇ alkyl, C₃₋₇ cycloalkyl, C₁₋₅ haloalkyl, C₆₋₁₀ aryl, 5- to 9-membered heteroaryl, 3- to 14-membered heterocycloalkyl, 4- to 9-membered heterocycloalkenyl, halogen, cyano, carbonyl, formaldoxime, NR^{a}R^{b}, SO₂NHR^{c}, CONR^{d}R^{e}, CO₂R^{f}, NHCOR^{g} or OR^{h}, and R^{a} to R^{f} are each independently selected from the group consisting of hydrogen, C₁₋₅ alkyl, C₃₋₇ cycloalkyl, C₁₋₄ haloalkyl, C₆₋₁₀ aryl, halogen, carbonyl and a Boc group,
Q is hydrogen, C₁₋₇ alkyl or C₃₋₇ cycloalkyl,
A is hydrogen, C₁₋₈ alkyl, C₂₋₈ alkenyl, C₂₋₆ alkynyl, C₃₋₁₀ cycloalkyl, C₄₋₁₀ cycloalkenyl, C₆₋₁₀ aryl, 3- to 16-membered heterocycloalkyl, 3- to 16-membered heterocycloalkenyl, 5- to 9-membered heteroaryl, halogen, carbonyl, NRⁱR^{j} or OR^{k}, and Rⁱ to R^{k} are each independently selected from the group consisting of hydrogen, C₁₋₈ alkyl, C₃₋₁₀ cycloalkyl, C₆₋₁₀ aryl, 5- to 10-membered heteroaryl, 3- to 10-membered heterocycloalkyl and a Boc group, and
Z is C₁₋₈ alkyl, C₃₋₇ cycloalkyl, C₆₋₁₀ aryl, 5- to 13-membered heteroaryl or 3- to 8-membered heterocycloalkyl,
where R, R^{a} to R^{k}, Q, A and Z are each substituted or unsubstituted with Substituent Group I:
Substituent Group I: substituted or unsubstituted C₁₋₁₀ alkyl, substituted or unsubstituted C₂₋₁₀ alkenyl, substituted or unsubstituted C₂₋₁₀ alkynyl, substituted or unsubstituted C₃₋₂₀ carbocycle, substituted or unsubstituted C₃₋₂₀ cycloalkyl, substituted or unsubstituted C₆₋₃₀ aryl, substituted or unsubstituted 3- to 30-membered heterocycle, substituted or unsubstituted C₁₋₁₀ alkoxy, substituted or unsubstituted C₂₋₁₀ alkenyloxy, substituted or unsubstituted C₃₋₁₀ cycloalkyloxy, substituted or unsubstituted C₆₋₃₀ aryloxy, substituted or unsubstituted 3- to 30-membered heterocycleoxy, substituted or unsubstituted C₁₋₁₀ alkylketone, substituted or unsubstituted C₂₋₁₀ alkenylketone, substituted or unsubstituted C₃₋₁₀ cycloalkylketone, substituted or unsubstituted C₆₋₃₀ arylketone, substituted or unsubstituted 3- to 30-membered heterocycleketone, substituted or unsubstituted C₁₋₁₀ alkylthio, substituted or unsubstituted C₂₋₁₀ alkenylthio, substituted or unsubstituted C₃₋₁₀ cycloalkylthio, substituted or unsubstituted C₆₋₃₀ arylthio, substituted or unsubstituted 3- to 30-membered heterocyclethio, substituted or unsubstituted carbonyl, carboxy, halogen, substituted or unsubstituted C₁₋₁₀ haloalkyl, hydroxy, substituted or unsubstituted amino, imine, cyano, nitro, amide, thiol, sulfone and phosphoric acid,
where a C₁₋₁₀ alkyl moiety is interposed by a heteroatom group selected from - O-, -S-, -SO-, -SO₂-, -NR^{m}- (R^{m} is hydrogen or substituted or unsubstituted C₁₋₁₀ alkyl), -N=, =N-, -POR^{m}- and -PO₄R^{m}-, and a heterocycle moiety comprises at least one of S, N, P and O as a ring-forming atom.

2. The compound according to claim 1, wherein R, R^{a} to R^{k}, Q, A and Z are each substituted or unsubstituted with Substituent Group I-1:
Substituent Group I-1: substituted or unsubstituted C₁₋₆ alkyl, substituted or unsubstituted C₁₋₅ alkenyl, substituted or unsubstituted C₂₋₇ alkynyl, substituted or unsubstituted C₃₋₆ cycloalkyl, substituted or unsubstituted C₁₋₅ haloalkyl, substituted or unsubstituted C₆₋₈ aryl, substituted or unsubstituted carbonyl, substituted or unsubstituted 3- to 9-membered heterocycloalkyl, halogen, cyano, a Boc group, OR^{p1}, NR^{p2}R^{p3}, CONR^{p4}R^{p5}, NHCOR^{p6} or CO₂R^{p7}, where R^{p1} to R^{p7} are each independently hydrogen, substituted or unsubstituted methyl, substituted or unsubstituted ethyl, substituted or unsubstituted propyl, substituted or unsubstituted propenyl, substituted or unsubstituted mesylate, carbonyl and a Boc group.

3. The compound according to claim 1, wherein the compound of Formula I is represented by any one of the following Formula I-1 or I-2: 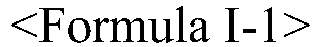 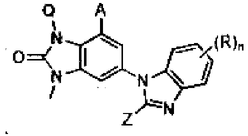 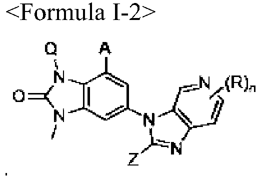 where
n, R, Q, A and Z are the same as defined in claim 1.

4. The compound according to claim 3, wherein the compound of Formula I-1 is represented by any one of the following Formula I-1-1 or I-1-2: 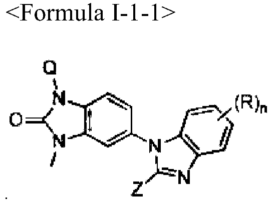 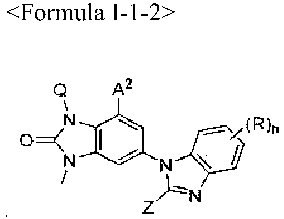 where
A² is C₁₋₈ alkyl, C₂₋₈ alkenyl, C₂₋₆ alkynyl, C₃₋₁₀ cycloalkyl, C₄₋₁₀ cycloalkenyl, C₆₋₁₀ aryl, 3- to 16-membered heterocycloalkyl, 3- to 16-membered heterocycloalkenyl, 5- to 9-membered heteroaryl, halogen, carbonyl, NRⁱR^{j} or OR^{k}, which is substituted or unsubstituted with substituent group I, and
Rⁱ to R^{k}, n, R, Q and Z are the same as defined in claim 1.

5. The compound according to claim 3, wherein the compound of Formula I-2 is represented by any one of the following Formula I-2-1 or I-2-2: 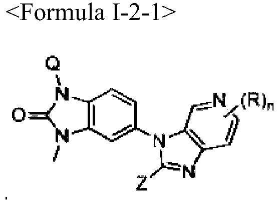 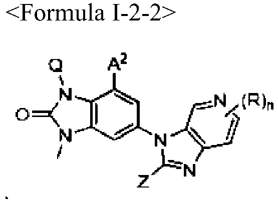 where
A² is the same as defined in claim 4, and
n, R, Q and Z are the same as defined in claim 1.

6. The compound according to claim 4, wherein the compound of Formula I-1-1 is represented by any one of the following Formula I-1-1-1 or I-1-1-2: 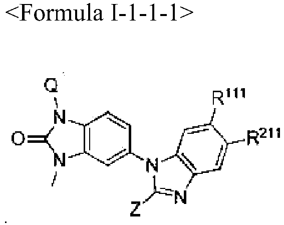 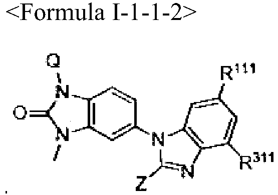 where
R¹¹¹, R²¹¹ and R³¹¹ are each independently C₁₋₅ alkyl, C₁₋₃ haloalkyl, C₆₋₈ aryl, 5-to 7-membered heteroaryl, halogen, cyano, carbonyl, CONR^{d}R^{e} or CO₂R^{f}, which is substituted or unsubstituted with substituent group I, and
R^{d} to R^{f}, Q and Z are the same as defined in claim 1.

7. The compound according to claim 6, wherein
R¹¹¹, R²¹¹ and R³¹¹ are each independently methyl, propyl, CF₂H, phenyl, pyrazolyl, bromo, fluoro, cyano, aldehyde, CONR^{d}R^{e} or CO₂R^{f}, where R^{d} to R^{f} are each independently hydrogen or substituted or unsubstituted methyl, and
R¹¹¹ and R²¹¹ are each independently substituted or unsubstituted with Substituent Group I-1-1:
Substituent Group I-1-1: substituted or unsubstituted C₁₋₃ alkyl, halogen, OR^{p1}, NR^{p2}R^{p3}, CONR^{p4}R^{p5} or NHCOR^{p6}, where R^{p1} to R^{p6} are each independently hydrogen, substituted or unsubstituted methyl or substituted or unsubstituted propyl.

8. The compound according to claim 4, wherein the compound of Formula I-1-2 is represented by any one of the following Formula I-1-2-1 to I-1-2-3: 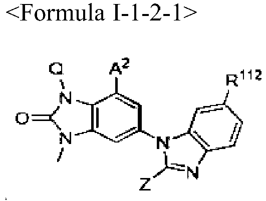 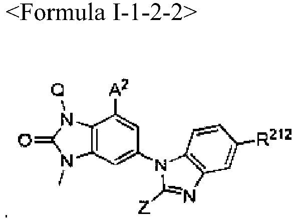 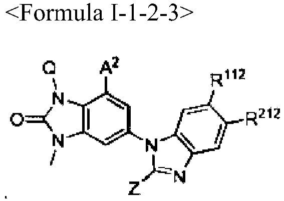 where
A² is the same as defined in claim 4,
R¹¹² and R²¹² are each independently C₁₋₅ alkyl, C₃₋₇ cycloalkyl, C₁₋₅ haloalkyl, C₆₋₁₀ aryl, 5- to 9-membered heteroaryl, 3- to 14-membered heterocycloalkyl, 4- to 9-membered heterocycloalkenyl, halogen, cyano, carbonyl, formaldoxime, NR^{a}R^{b}, SO₂NHR^{c}, CONR^{d}R^{e}, CO₂R^{f}, NHCOR^{g} or OR^{h}, where R¹¹² and R²¹² are each substituted or unsubstituted with Substituent Group I, and
R^{a} to R^{h}, Q and Z are the same as defined in claim 1.

9. The compound according to claim 8, wherein
R¹¹² and R²¹² are each independently methyl, cyclopropyl, CF₃, CF₂H, CFH₂, phenyl, oxazolyl, pyrazolyl, thiazolyl, pyridinyl, oxadiazolyl, imidazolyl, triazolyl, lactamyl, oxindolyl, quinolinonyl, dihydroquinolinonyl, benzoxazinonyl, dihydropyranyl, fluoro, chloro, bromo, cyano, aldehyde, formaldoxime, NR^{a}R^{b}, SO₂NHR^{c}, CONR^{d}R^{e}, CO₂R^{f}, NHCOR^{g} or OR^{h}, where R^{a} to R^{h} are each independently hydrogen, substituted or unsubstituted methyl, CF₂H, CF₃, substituted or unsubstituted phenyl, substituted or unsubstituted benzyl, substituted or unsubstituted carbonyl or a Boc group, and
R¹¹² and R²¹² are each independently substituted or unsubstituted with Substituent Group I-1-2:
Substituent Group I-1-2: substituted or unsubstituted C₁₋₅ alkyl, substituted or unsubstituted C₃₋₅ cycloalkyl, substituted or unsubstituted C₁₋₅ alkenyl, substituted or unsubstituted C₁₋₄ haloalkyl, halogen, 4-membered to 8-membered heterocycloalkyl, OR^{p1}, NR^{p2}R^{p3}, CONR^{p4}R^{p5} or NHCOR^{p6}, where R^{p1} to R^{p6} are each independently hydrogen or substituted or unsubstituted methyl.

10. The compound according to claim 5, wherein the compound of Formula I-2-1 is represented by any one of the following Formula I-2-1-1 or I-2-1-2: 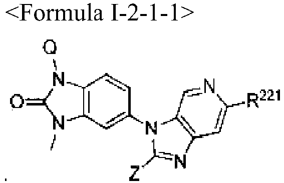 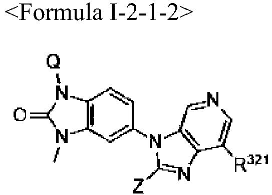 where
R²²¹ and R³¹² are each independently C₆₋₉ aryl, 5- to 9-membered heteroaryl, halogen or CONR^{d}R^{e}, where R²²¹ and R³¹² are each substituted or unsubstituted with Substituent Group I, and
R^{d} to R^{e}, Q and Z are the same as defined in claim 1.

11. The compound according to claim 10, wherein
R²²¹ and R³¹² are each independently phenyl, pyrazolyl, bromo or CONR^{d}R^{e}, where R^{d} to R^{e} are each independently hydrogen or substituted or unsubstituted methyl, and
R²²¹ and R³¹² are each independently substituted or unsubstituted with Substituent Group I-1-3:
Substituent Group 1-1-3: substituted or unsubstituted C₁₋₃ alkyl, halogen or CONR^{p4}R^{p5}, where R^{p4} to R^{p5} are each independently hydrogen or substituted or unsubstituted methyl.

12. The compound according to claim 5, wherein the compound of Formula 1-2-2 is represented by any one of the following Formula I-2-2-1 or I-2-2-2: 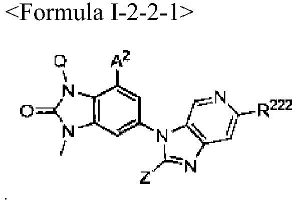 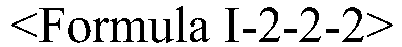 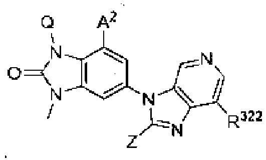 where
A² is the same as defined in claim 3,
R²²² and R³²² are each independently 5- to 9-membered heteroaryl, where R²²² and R³²² are each substituted or unsubstituted with Substituent Group I, and
Q and Z are the same as defined in claim 1.

13. The compound according to claim 12, wherein
R²²² and R³²² are each independently pyrazolyl or thiazolyl, where R²²² and R³²² are each independently substituted or unsubstituted with Substituent Group I-1-4:
Substituent Group I-1-4: substituted or unsubstituted C₁₋₃ alkyl or substituted or unsubstituted C₁₋₃ haloalkyl.

14. The compound according to claim 1, wherein Q is hydrogen, substituted or unsubstituted methyl, substituted or unsubstituted ethyl or substituted or unsubstituted propyl.

15. The compound according to claim 1, wherein Z is substituted or unsubstituted methyl, substituted or unsubstituted ethyl, substituted or unsubstituted propyl, substituted or unsubstituted butyl, substituted or unsubstituted cyclopropyl, substituted or unsubstituted cyclobutyl, substituted or unsubstituted phenyl, substituted or unsubstituted imidazolyl, substituted or unsubstituted indolyl, substituted or unsubstituted thiophenyl, substituted or unsubstituted pyridinyl, substituted or unsubstituted pyrimidinyl, substituted or unsubstituted thiazolyl, or substituted or unsubstituted oxetanyl.

16. The compound according to claim 1, being any one selected from the following group:
3-(7-chloro-1,3-dimethyl-2-oxo-benzimidazol-5-yl)-N-methyl-2-(6-methyl-1H-indol-3-yl)benzimidazole-5-sulfonamide;
3-(1,3-dimethyl-2-oxo-7-tetrahydropyran-4-yl-benzimidazol-5-yl)-N-methyl-2-(6-methyl-1H-indol-3-yl)benzimidazole-5-sulfonamide;
methyl 6-bromo-3-(1,3-dimethyl-2-oxo-benzimidazol-5-yl)-2-(3-methylimidazol-4-yl)benzimidazole-5-carboxylate;
methyl 3-(1,3-dimethyl-2-oxo-benzimidazol-5-yl)-6-[3-fluoro-4-(methylcarbamoyl)phenyl]-2-(3-methylimidazol-4-yl)benzimidazole-5-carboxylate;
methyl 3-(1,3-dimethyl-2-oxo-benzimidazol-5-yl)-2-(6-methyl-1H-indol-3-yl)-6-(1-methylpyrazol-4-yl)benzimidazole-5-carboxylate;
3-(1,3-dimethyl-2-oxo-benzimidazol-5-yl)-2-(3-methylimidazol-4-yl)-6-(1-methylpyrazol-4-yl)benzimidazole-5-carbonitrile;
5-[6-(hydroxymethyl)-2-(6-methyl-1H-indol-3-yl)-5-(1-methylpyrazol-4-yl)benzimidazol-1-yl]-1,3-dimethyl-benzimidazol-2-one;
3-(1,3-dimethyl-2-oxo-benzimidazol-5-yl)-2-(6-methyl-1H-indol-3-yl)-6-(1-methylpyrazol-4-yl)benzimidazole-5-carbaldehyde;
5-[6-(difluoromethyl)-2-(6-methyl-1H-indol-3-yl)-5-(1-methylpyrazol-4-yl)benzimidazol-1-yl]-1,3-dimethyl-benzimidazol-2-one;
3-(1,3-dimethyl-2-oxo-benzimidazol-5-yl)-2-(6-methyl-1H-indol-3-yl)-6-(1-methylpyrazol-4-yl)benzimidazole-5-carboxylic acid;
1,3-dimethyl-6-[2-(3-methylimidazol-4-yl)-5-(1-methylpyrazol-4-yl)benzimidazol-1-yl]-4-tetrahydropyran-4-yl-benzimidazol-2-one;
5-[6-(1-hydroxy-1-methyl-ethyl)-2-(6-methyl-1H-indol-3-yl)-5-(1-methylpyrazol-4-yl)benzimidazol-1-yl]-1,3-dimethyl-benzimidazol-2-one;
5-[6-bromo-2-(6-methyl-1H-indol-3-yl)imidazo[4,5-c]pyridin-3-yl]-1,3-dimethyl-benzimidazol-2-one;
1,3-dimethyl-5-[2-(6-methyl-1H-indol-3-yl)-6-(1-methylpyrazol-4-yl)imidazo[4,5-c]pyridin-3-yl]benzimidazol-2-one;
6-bromo-3-(1,3-dimethyl-2-oxo-benzimidazol-5-yl)-2-(6-methyl-1H-indol-3-yl)benzimidazole-5-carbonitrile;
3-(1,3-dimethyl-2-oxo-benzimidazol-5-yl)-2-(6-methyl-1H-indol-3-yl)-6-(1-methylpyrazol-4-yl)benzimidazole-5-carbonitrile;
3-(1,3-dimethyl-2-oxo-benzimidazol-5-yl)-2-methyl-6-(1-methylpyrazol-4-yl)benzimidazole-5-carboxylic acid;
methyl 3-(1,3-dimethyl-2-oxo-benzimidazol-5-yl)-2-methyl-6-(1-methylpyrazol-4-yl)benzimidazole-5-carboxylate;
3-(1,3-dimethyl-2-oxo-benzimidazol-5-yl)-2-(3-methylimidazol-4-yl)-6-(1-methylpyrazol-4-yl)benzimidazole-5-carboxylic acid;
methyl 3-(1,3-dimethyl-2-oxo-benzimidazol-5-yl)-2-(3-methylimidazol-4-yl)-6-(1-methylpyrazol-4-yl)benzimidazole-5-carboxylate;
4-[6-cyano-1-(1,3-dimethyl-2-oxo-benzimidazol-5-yl)-2-(3-methylimidazol-4-yl)benzimidazol-5-yl]-2-fluoro-N-methyl-benzamide;
3-(1,3-dimethyl-2-oxo-benzimidazol-5-yl)-6-[3-fluoro-4-(methylcarbamoyl)phenyl]-2-(3-methylimidazol-4-yl)benzimidazole-5-carboxylic acid;
4-[1-(1,3-dimethyl-2-oxo-7-tetrahydropyran-4-yl-benzimidazol-5-yl)-2-(3-methylimidazol-4-yl)benzimidazol-5-yl]-2-fluoro-N-methyl-benzamide;
5-[6-fluoro-2-(3-methylimidazol-4-yl)-5-(1-methylpyrazol-4-yl)benzimidazol-1-yl]-1,3-dimethyl-benzimidazol-2-one;
4-[1-(1,3-dimethyl-2-oxo-benzimidazol-5-yl)-6-(1-hydroxy-1-methyl-ethyl)-2-(3-methylimidazol-4-yl)benzimidazol-5-yl]-2-fluoro-N-methyl-benzamide;
3-(1,3-dimethyl-2-oxo-benzimidazol-5-yl)-6-[3-fluoro-4-(methylcarbamoyl)phenyl]-N-methyl-2-(3-methylimidazol-4-yl)benzimidazole-5-carboxamide;
4-[3-(1,3-dimethyl-2-oxo-benzimidazol-5-yl)-2-(3-methylimidazol-4-yl)imidazo[4,5-c]pyridin-6-yl]-2-fluoro-N-methyl-benzamide;
4-[1-(1,3-dimethyl-2-oxo-benzimidazol-5-yl)-6-fluoro-2-(3-methylimidazol-4-yl)benzimidazol-5-yl]-2-fluoro-N-methyl-benzamide;
N-[4-[1-(1,3-dimethyl-2-oxo-benzimidazol-5-yl)-6-fluoro-2-(3-methylimidazol-4-yl)benzimidazol-5-yl]phenyl]acetamide;
5-[6-fluoro-5-(3-methoxyphenyl)-2-(3-methylimidazol-4-yl)benzimidazol-1-yl]-1,3-dimethyl-benzimidazol-2-one;
N-[4-[1-(1,3-dimethyl-2-oxo-benzimidazol-5-yl)-6-fluoro-2-(3-methylimidazol-4-yl)benzimidazol-5-yl]phenyl]-2-methyl-propanamide;
5-[5-(4-aminophenyl)-6-fluoro-2-(3-methylimidazol-4-yl)benzimidazol-1-yl]-1,3-dimethyl-benzimidazol-2-one;
N-[4-[1-(1,3-dimethyl-2-oxo-7-tetrahydropyran-4-yl-benzimidazol-5-yl)-6-fluoro-2-(3-methylimidazol-4-yl)benzimidazol-5-yl]phenyl]acetamide;
6-[5-(4-aminophenyl)-6-fluoro-2-(3-methylimidazol-4-yl)benzimidazol-1-yl]-1,3-dimethyl-4-tetrahydropyran-4-yl-benzimidazol-2-one;
6-[5-[4-(dimethylamino)phenyl]-6-fluoro-2-(3-methylimidazol-4-yl)benzimidazol-1-yl]-1,3-dimethyl-4-tetrahydropyran-4-yl-benzimidazol-2-one;
4-[1-(1,3-dimethyl-2-oxo-7-tetrahydropyran-4-yl-benzimidazol-5-yl)-6-fluoro-2-(3-methylimidazol-4-yl)benzimidazol-5-yl]-2-fluoro-N-methyl-benzamide;
6-[6-fluoro-2-(3-methylimidazol-4-yl)-5-(1-methylpyrazol-4-yl)benzimidazol-1-yl]-1,3-dimethyl-4-tetrahydropyran-4-yl-benzimidazol-2-one;
6-[6-fluoro-5-[4-(methylamino)phenyl]-2-(3-methylimidazol-4-yl)benzimidazol-1-yl]-1,3-dimethyl-4-tetrahydropyran-4-yl-benzimidazol-2-one;
tert-butyl 4-[6-[5-(4-acetamidophenyl)-6-fluoro-2-(3-methylimidazol-4-yl)benzimidazol-1-yl]-1,3-dimethyl-2-oxo-benzimidazol-4-yl]-3,6-dihydro-2H-pyridine-1-carboxylate;
N-[4-[1-[1,3-dimethyl-7-(1-methyl-3,6-dihydro-2H-pyridin-4-yl)-2-oxo-benzimidazol-5-yl]-6-fluoro-2-(3-methylimidazol-4-yl)benzimidazol-5-yl]phenyl]acetamide;
N-[4-[1-[1,3-dimethyl-7-(2-morpholinoethylamino)-2-oxo-benzimidazol-5-yl]-6-fluoro-2-(3-methylimidazol-4-yl)benzimidazol-5-yl]phenyl]acetamide;
N-[4-[1-[7-(cyclopentylamino)-1,3-dimethyl-2-oxo-benzimidazol-5-yl]-6-fluoro-2-(3-methylimidazol-4-yl)benzimidazol-5-yl]phenyl]acetamide;
N-[4-[1-[1,3-dimethyl-2-oxo-7-(propylamino)benzimidazol-5-yl]-6-fluoro-2-(3-methylimidazol-4-yl)benzimidazol-5-yl]phenyl]acetamide;
N-[4-[6-fluoro-1-[7-(isopropylamino)-1,3-dimethyl-2-oxo-benzimidazol-5-yl]-2-(3-methylimidazol-4-yl)benzimidazol-5-yl]phenyl]acetamide;
N-[4-[1-[7-(cyclopropylamino)-1,3-dimethyl-2-oxo-benzimidazol-5-yl]-6-fluoro-2-(3-methylimidazol-4-yl)benzimidazol-5-yl]phenyl]acetamide;
N-[4-[1-(1,3-dimethyl-7-morpholino-2-oxo-benzimidazol-5-yl)-6-fluoro-2-(3-methylimidazol-4-yl)benzimidazol-5-yl]phenyl]acetamide;
6-[6-fluoro-2-(3-methylimidazol-4-yl)-5-(1-methylpyrazol-4-yl)benzimidazol-1-yl]-1,3-dimethyl-4-morpholino-benzimidazol-2-one;
5-[6-fluoro-2-(3-methylimidazol-4-yl)-5-(1-methylpyrazol-4-yl)benzimidazol-1-yl]-3-methyl-1H-benzimidazol-2-one;
1-ethyl-5-[6-fluoro-2-(3-methylimidazol-4-yl)-5-(1-methylpyrazol-4-yl)benzimidazol-1-yl]-3-methyl-benzimidazol-2-one;
5-[6-fluoro-2-(3-methylimidazol-4-yl)-5-(1-methylpyrazol-4-yl)benzimidazol-1-yl]-1-isopropyl-3-methyl-benzimidazol-2-one;
1-(cyclopropylmethyl)-5-[6-fluoro-2-(3-methylimidazol-4-yl)-5-(1-methylpyrazol-4-yl)benzimidazol-1-yl]-3-methyl-benzimidazol-2-one;
5-[6-fluoro-2-(3-methylimidazol-4-yl)-5-(1-methylpyrazol-4-yl)benzimidazol-1-yl]-1-(2-hydroxyethyl)-3-methyl-benzimidazol-2-one;
4-chloro-6-[6-fluoro-2-(3-methylimidazol-4-yl)-5-(1-methylpyrazol-4-yl)benzimidazol-1-yl]-1,3-dimethyl-benzimidazol-2-one;
6-[6-fluoro-2-(3-methylimidazol-4-yl)-5-(1-methylpyrazol-4-yl)benzimidazol-1-yl]-4-isopropenyl-1,3-dimethyl-benzimidazol-2-one;
6-[6-fluoro-2-(3-methylimidazol-4-yl)-5-(1-methylpyrazol-4-yl)benzimidazol-1-yl]-1,3-dimethyl-4-(1-methylpyrazol-4-yl)benzimidazol-2-one;
4-cyclopropyl-6-[6-fluoro-2-(3-methylimidazol-4-yl)-5-(1-methylpyrazol-4-yl)benzimidazol-1-yl]-1,3-dimethyl-benzimidazol-2-one;
6-[6-fluoro-2-(3-methylimidazol-4-yl)-5-(1-methylpyrazol-4-yl)benzimidazol-1-yl]-4-(4-fluorophenyl)-1,3-dimethyl-benzimidazol-2-one;
6-[6-fluoro-2-(3-methylimidazol-4-yl)-5-(1-methylpyrazol-4-yl)benzimidazol-1-yl]-4-isopropyl-1,3-dimethyl-benzimidazol-2-one;
6-[6-fluoro-2-(3-methylimidazol-4-yl)-5-(1-methylpyrazol-4-yl)benzimidazol-1-yl]-4-(isopropylamino)-1,3-dimethyl-benzimidazol-2-one;
6-[6-fluoro-2-(3-methylimidazol-4-yl)-5-(1-methylpyrazol-4-yl)benzimidazol-1-yl]-1,3-dimethyl-4-(propylamino)benzimidazol-2-one;
N-[4-[1-(1,3-dimethyl-2-oxo-7-pyrrolidin-1-yl-benzimidazol-5-yl)-6-fluoro-2-(3-methylimidazol-4-yl)benzimidazol-5-yl]phenyl]acetamide;
6-[6-fluoro-2-(3-methylimidazol-4-yl)-5-(1-methylpyrazol-4-yl)benzimidazol-1-yl]-1,3-dimethyl-4-pyrrolidin-1-yl-benzimidazol-2-one;
6-[6-fluoro-2-(3-methylimidazol-4-yl)-5-(1-methylpyrazol-4-yl)benzimidazol-1-yl]-4-isobutyl-1,3-dimethyl-benzimidazol-2-one;
6-[6-fluoro-2-(3-methylimidazol-4-yl)-5-(1-methylpyrazol-4-yl)benzimidazol-1-yl]-1,3-dimethyl-4-(2-methylprop-1-enyl)benzimidazol-2-one;
tert-butyl 4-[6-[6-fluoro-2-(3-methylimidazol-4-yl)-5-(1-methylpyrazol-4-yl)benzimidazol-1-yl]-1,3-dimethyl-2-oxo-benzimidazol-4-yl]-3,6-dihydro-2H-pyridine-1-carboxylate;
4-(cyclopropylmethylamino)-6-[6-fluoro-2-(3-methylimidazol-4-yl)-5-(1-methylpyrazol-4-yl)benzimidazol-1-yl]-1,3-dimethyl-benzimidazol-2-one;
6-[1-(1,3-dimethyl-2-oxo-7-tetrahydropyran-4-yl-benzimidazol-5-yl)-6-fluoro-2-(3-methylimidazol-4-yl)benzimidazol-5-yl]-3,4-dihydro-1H-quinolin-2-one;
6-[6-fluoro-2-(3-methylimidazol-4-yl)-5-(2-oxoindolin-5-yl)benzimidazol-1-yl]-1,3-dimethyl-4-tetrahydropyran-4-yl-benzimidazol-2-one;
7-[1-(1,3-dimethyl-2-oxo-7-tetrahydropyran-4-yl-benzimidazol-5-yl)-6-fluoro-2-(3-methylimidazol-4-yl)benzimidazol-5-yl]-4H-1,4-benzoxazin-3-one;
6-[5-(4-chloro-3-methoxy-phenyl)-6-fluoro-2-(3-methylimidazol-4-yl)benzimidazol-1-yl]-1,3-dimethyl-4-tetrahydropyran-4-yl-benzimidazol-2-one;
6-[5-(3-chloro-4-methoxy-phenyl)-6-fluoro-2-(3-methylimidazol-4-yl)benzimidazol-1-yl]-1,3-dimethyl-4-tetrahydropyran-4-yl-benzimidazol-2-one;
6-[1-(1,3-dimethyl-2-oxo-7-tetrahydropyran-4-yl-benzimidazol-5-yl)-6-fluoro-2-(3-methylimidazol-4-yl)benzimidazol-5-yl]-4H-1,4-benzoxazin-3-one;
6-[1-(1,3-dimethyl-2-oxo-7-tetrahydropyran-4-yl-benzimidazol-5-yl)-6-fluoro-2-(3-methylimidazol-4-yl)benzimidazol-5-yl]-1H-quinolin-2-one;
6-[6-fluoro-2-(3-methylimidazol-4-yl)-5-[1-methyl-3-(trifluoromethyl)pyrazol-4-yl]benzimidazol-1-yl]-1,3-dimethyl-4-tetrahydropyran-4-yl-benzimidazol-2-one;
6-[6-fluoro-2-(3-methylimidazol-4-yl)-5-(1-methylpyrazol-4-yl)benzimidazol-1-yl]-1,3-dimethyl-4-(1,2,3,6-tetrahydropyridin-4-yl)benzimidazol-2-one;
6-[6-fluoro-2-(3-methylimidazol-4-yl)-5-(1-methylpyrazol-4-yl)benzimidazol-1-yl]-1,3-dimethyl-4-(1-methyl-3,6-dihydro-2H-pyridin-4-yl)benzimidazol-2-one;
6-[6-fluoro-2-(3-methylimidazol-4-yl)-5-(1-methylpyrazol-4-yl)benzimidazol-1-yl]-4-(4-methoxyphenyl)-1,3-dimethyl-benzimidazol-2-one;
5-[6-(difluoromethyl)-2-(3-methylimidazol-4-yl)-5-(1-methylpyrazol-4-yl)benzimidazol-1-yl]-1,3-dimethyl-benzimidazol-2-one;
2-[6-fluoro-1-[7-(isopropylamino)-1,3-dimethyl-2-oxo-benzimidazol-5-yl]-5-(1-methylpyrazol-4-yl)benzimidazol-2-yl]-2-methyl-propanenitrile;
2-[6-fluoro-1-[7-(isopropylamino)-1,3-dimethyl-2-oxo-benzimidazol-5-yl]-5-(3-oxo-4H-1,4-benzoxazin-7-yl)benzimidazol-2-yl]-2-methyl-propanenitrile;
4-(cyclohexylmethylamino)-6-[6-fluoro-2-(3-methylimidazol-4-yl)-5-(1-methylpyrazol-4-yl)benzimidazol-1-yl]-1,3-dimethyl-benzimidazol-2-one;
6-[6-fluoro-2-(3-methylimidazol-4-yl)-5-(1-methylpyrazol-4-yl)benzimidazol-1-yl]-1,3-dimethyl-4-(3,3,3-trifluoropropylamino)benzimidazol-2-one;
6-[2-tert-butyl-6-fluoro-5-(1-methylpyrazol-4-yl)benzimidazol-1-yl]-4-(isopropylamino)-1,3-dimethyl-benzimidazol-2-one;
7-[2-tert-butyl-6-fluoro-1-[7-(isopropylamino)-1,3-dimethyl-2-oxo-benzimidazol-5-yl]benzimidazol-5-yl]-4H-1,4-benzoxazin-3-one;
4-(cyclopentylamino)-6-[6-fluoro-2-(3-methylimidazol-4-yl)-5-(1-methylpyrazol-4-yl)benzimidazol-1-yl]-1,3-dimethyl-benzimidazol-2-one;
6-[6-(difluoromethyl)-2-(3-methylimidazol-4-yl)-5-(1-methylpyrazol-4-yl)benzimidazol-1-yl]-4-(isopropylamino)-1,3-dimethyl-benzimidazol-2-one;
6-[2-tert-butyl-6-(difluoromethyl)-5-(1-methylpyrazol-4-yl)benzimidazol-1-yl]-4-(isopropylamino)-1,3-dimethyl-benzimidazol-2-one;
6-[6-fluoro-2-(3-methylimidazol-4-yl)-5-(1-methylpyrazol-3-yl)benzimidazol-1-yl]-4-(isopropylamino)-1,3-dimethyl-benzimidazol-2-one;
6-[6-fluoro-2-(3-methylimidazol-4-yl)-5-(2-methylthiazol-5-yl)benzimidazol-1-yl]-4-(isopropylamino)-1,3-dimethyl-benzimidazol-2-one;
6-[6-fluoro-2-(3-methylimidazol-4-yl)-5-(1-methylpyrazol-4-yl)benzimidazol-1-yl]-4-(2-methoxyethylamino)-1,3-dimethyl-benzimidazol-2-one;
4-[2-(dimethylamino)ethylamino]-6-[6-fluoro-2-(3-methylimidazol-4-yl)-5-(1-methylpyrazol-4-yl)benzimidazol-1-yl]-1,3-dimethyl-benzimidazol-2-one;
4-(cyclobutylamino)-6-[6-fluoro-2-(3-methylimidazol-4-yl)-5-(1-methylpyrazol-4-yl)benzimidazol-1-yl]-1,3-dimethyl-benzimidazol-2-one;
4-(tert-butylamino)-6-[6-fluoro-2-(3-methylimidazol-4-yl)-5-(1-methylpyrazol-4-yl)benzimidazol-1-yl]-1,3-dimethyl-benzimidazol-2-one;
6-[6-fluoro-2-(3-methylimidazol-4-yl)-5-(1-methylpyrazol-4-yl)benzimidazol-1-yl]-4-isopropoxy-1,3-dimethyl-benzimidazol-2-one;
6-[2-tert-butyl-6-fluoro-5-(1-methylpyrazol-4-yl)benzimidazol-1-yl]-4-(cyclobutylamino)-1,3-dimethyl-benzimidazol-2-one;
6-[6-fluoro-2-(3-methylimidazol-4-yl)-5-(1-methylpyrazol-4-yl)benzimidazol-1-yl]-4-isobutoxy-1,3-dimethyl-benzimidazol-2-one;
6-[6-fluoro-2-(3-methylimidazol-4-yl)-5-(1-methylpyrazol-4-yl)benzimidazol-1-yl]-4-hydroxy-1,3-dimethyl-benzimidazol-2-one;
4-(cyclopentoxy)-6-[6-fluoro-2-(3-methylimidazol-4-yl)-5-(1-methylpyrazol-4-yl)benzimidazol-1-yl]-1,3-dimethyl-benzimidazol-2-one;
6-[6-fluoro-2-(3-methylimidazol-4-yl)-5-(1-methylpyrazol-4-yl)benzimidazol-1-yl]-1,3-dimethyl-4-propoxy-benzimidazol-2-one;
4-(cyclopropylmethoxy)-6-[6-fluoro-2-(3-methylimidazol-4-yl)-5-(1-methylpyrazol-4-yl)benzimidazol-1-yl]-1,3-dimethyl-benzimidazol-2-one;
4-(cyclobutylmethylamino)-6-[6-fluoro-2-(3-methylimidazol-4-yl)-5-(1-methylpyrazol-4-yl)benzimidazol-1-yl]-1,3-dimethyl-benzimidazol-2-one;
6-[2-tert-butyl-6-fluoro-5-(1-methylpyrazol-4-yl)benzimidazol-1-yl]-4-isopropyl-1,3-dimethyl-benzimidazol-2-one;
6-[6-fluoro-2-(3-methylimidazol-4-yl)-5-(1-methylpyrazol-4-yl)benzimidazol-1-yl]-4-(2-methoxyphenyl)-1,3-dimethyl-benzimidazol-2-one;
methyl 2-tert-butyl-3-(1,3-dimethyl-2-oxo-benzimidazol-5-yl)-6-(1-methylpyrazol-4-yl)benzimidazole-5-carboxylate;
2-tert-butyl-3-(1,3-dimethyl-2-oxo-benzimidazol-5-yl)-6-(1-methylpyrazol-4-yl)benzimidazole-5-carboxylic acid;
6-[2-tert-butyl-6-fluoro-5-(1-methylpyrazol-4-yl)benzimidazol-1-yl]-4-(cyclopentylamino)-1,3-dimethyl-benzimidazol-2-one;
6-[6-fluoro-2-(3-methylimidazol-4-yl)-5-(1-methylpyrazol-4-yl)benzimidazol-1-yl]-4-[isopropyl(methyl)amino]-1,3-dimethyl-benzimidazol-2-one;
2-tert-butyl-3-(7-isopropyl-1,3-dimethyl-2-oxo-benzimidazol-5-yl)-6-(1-methylpyrazol-4-yl)benzimidazole-5-carbonitrile;
2-tert-butyl-3-[7-(isopropylamino)-1,3-dimethyl-2-oxo-benzimidazol-5-yl]-6-(1-methylpyrazol-4-yl)benzimidazole-5-carbonitrile;
4-anilino-6-[6-fluoro-2-(3-methylimidazol-4-yl)-5-(1-methylpyrazol-4-yl)benzimidazol-1-yl]-1,3-dimethyl-benzimidazol-2-one;
4-(4-fluoroanilino)-6-[6-fluoro-2-(3-methylimidazol-4-yl)-5-(1-methylpyrazol-4-yl)benzimidazol-1-yl]-1,3-dimethyl-benzimidazol-2-one;
4-[4-(dimethylamino)anilino]-6-[6-fluoro-2-(3-methylimidazol-4-yl)-5-(1-methylpyrazol-4-yl)benzimidazol-1-yl]-1,3-dimethyl-benzimidazol-2-one;
3-(7-isopropyl-1,3-dimethyl-2-oxo-benzimidazol-5-yl)-2-(3-methylimidazol-4-yl)-6-(1-methylpyrazol-4-yl)benzimidazole-5-carbonitrile;
3-[7-(isopropylamino)-1,3-dimethyl-2-oxo-benzimidazol-5-yl]-2-(3-methylimidazol-4-yl)-6-(1-methylpyrazol-4-yl)benzimidazole-5-carbonitrile;
6-[6-fluoro-2-(3-methylimidazol-4-yl)-5-(1-methylpyrazol-4-yl)benzimidazol-1-yl]-4-(4-methoxyanilino)-1,3-dimethyl-benzimidazol-2-one;
6-[6-fluoro-2-(1-methylcyclopropyl)-5-(1-methylpyrazol-4-yl)benzimidazol-1-yl]-4-isopropyl-1,3-dimethyl-benzimidazol-2-one;
6-[6-fluoro-2-(1-methylcyclobutyl)-5-(1-methylpyrazol-4-yl)benzimidazol-1-yl]-4-isopropyl-1,3-dimethyl-benzimidazol-2-one;
6-[6-fluoro-2-(3-methyloxetan-3-yl)-5-(1-methylpyrazol-4-yl)benzimidazol-1-yl]-4-isopropyl-1,3-dimethyl-benzimidazol-2-one;
6-[6-fluoro-5-(1-methylpyrazol-4-yl)-2-[1-(trifluoromethyl)cyclopropyl]benzimidazol-1-yl]-4-isopropyl-1,3-dimethyl-benzimidazol-2-one;
6-[6-fluoro-2-(3-methylimidazol-4-yl)-5-(1-methylpyrazol-4-yl)benzimidazol-1-yl]-1,3-dimethyl-4-(tetrahydropyran-4-ylmethoxy)benzimidazol-2-one;
6-[2-tert-butyl-6-fluoro-5-(1-methylpyrazol-4-yl)benzimidazol-1-yl]-4-isopropoxy-1,3-dimethyl-benzimidazol-2-one;
6-[6-fluoro-2-(3-methylimidazol-4-yl)-5-(1-methylpyrazol-4-yl)benzimidazol-1-yl]-1,3-dimethyl-4-(2-trimethylsilylethynyl)benzimidazol-2-one;
4-ethynyl-6-[6-fluoro-2-(3-methylimidazol-4-yl)-5-(1-methylpyrazol-4-yl)benzimidazol-1-yl]-1,3-dimethyl-benzimidazol-2-one;
4-acetyl-6-[6-fluoro-2-(3-methylimidazol-4-yl)-5-(1-methylpyrazol-4-yl)benzimidazol-1-yl]-1,3-dimethyl-benzimidazol-2-one;
6-[6-fluoro-2-(3-methylimidazol-4-yl)-5-(1-methylpyrazol-4-yl)benzimidazol-1-yl]-4-(1-hydroxyethyl)-1,3-dimethyl-benzimidazol-2-one;
6-[6-fluoro-2-(3-methylimidazol-4-yl)-5-(1-methylpyrazol-4-yl)benzimidazol-1-yl]-4-(1-hydroxy-1-methyl-ethyl)-1,3-dimethyl-benzimidazol-2-one;
6-[2-tert-butyl-6-fluoro-5-(1-methylpyrazol-4-yl)benzimidazol-1-yl]-1,3-dimethyl-4-morpholino-benzimidazol-2-one;
6-[6-fluoro-2-(3-methylimidazol-4-yl)-5-(1-methylpyrazol-4-yl)benzimidazol-1-yl]-4-(1-methoxyethyl)-1,3-dimethyl-benzimidazol-2-one;
6-[6-fluoro-2-(3-methylimidazol-4-yl)-5-(1-methylpyrazol-4-yl)benzimidazol-1-yl]-4-(1-methoxy-1-methyl-ethyl)-1,3-dimethyl-benzimidazol-2-one;
4-isopropyl-1,3-dimethyl-6-[2-(3-methylimidazol-4-yl)-5-(1-methylpyrazol-4-yl)-6-(trifluoromethyl)benzimidazol-1-yl]benzimidazol-2-one;
6-[2-tert-butyl-5-(1-methylpyrazol-4-yl)-6-(trifluoromethyl)benzimidazol-1-yl]-4-isopropyl-1,3-dimethyl-benzimidazol-2-one;
4-(isopropylamino)-1,3-dimethyl-6-[2-(3-methylimidazol-4-yl)-5-(1-methylpyrazol-4-yl)-6-(trifluoromethyl)benzimidazol-1-yl]benzimidazol-2-one;
6-[2-tert-butyl-5-(1-methylpyrazol-4-yl)-6-(trifluoromethyl)benzimidazol-1-yl]-4-(isopropylamino)-1,3-dimethyl-benzimidazol-2-one;
2-tert-butyl-3-(1,3-dimethyl-7-morpholino-2-oxo-benzimidazol-5-yl)-6-(1-methylpyrazol-4-yl)benzimidazole-5-carbonitrile;
6-[2-tert-butyl-6-(difluoromethyl)-5-(1-methylpyrazol-4-yl)benzimidazol-1-yl]-1,3-dimethyl-4-morpholino-benzimidazol-2-one;
4-(cyclohexylamino)-6-[6-fluoro-2-(3-methylimidazol-4-yl)-5-(1-methylpyrazol-4-yl)benzimidazol-1-yl]-1,3-dimethyl-benzimidazol-2-one;
6-[2-tert-butyl-6-fluoro-5-(1-methylpyrazol-4-yl)benzimidazol-1-yl]-4-(cyclohexylamino)-1,3-dimethyl-benzimidazol-2-one;
3-(1,3-dimethyl-7-morpholino-2-oxo-benzimidazol-5-yl)-2-(3-methylimidazol-4-yl)-6-(1-methylpyrazol-4-yl)benzimidazole-5-carbonitrile;
4-(cyclohexen-1-yl)-6-[6-fluoro-2-(3-methylimidazol-4-yl)-5-(1-methylpyrazol-4-yl)benzimidazol-1-yl]-1,3-dimethyl-benzimidazol-2-one;
6-[6-(difluoromethyl)-2-(3-methylimidazol-4-yl)-5-(1-methylpyrazol-4-yl)benzimidazol-1-yl]-1,3-dimethyl-4-morpholino-benzimidazol-2-one;
6-[6-fluoro-2-(3-methylimidazol-4-yl)-5-(1-methylpyrazol-4-yl)benzimidazol-1-yl]-1,3-dimethyl-4-(sec-butylamino)benzimidazol-2-one;
4-(3,3-difluoroazetidin-1-yl)-6-[6-fluoro-2-(3-methylimidazol-4-yl)-5-(1-methylpyrazol-4-yl)benzimidazol-1-yl]-1,3-dimethyl-benzimidazol-2-one;
4-(azetidin-1-yl)-6-[6-fluoro-2-(3-methylimidazol-4-yl)-5-(1-methylpyrazol-4-yl)benzimidazol-1-yl]-1,3-dimethyl-benzimidazol-2-one;
6-[6-fluoro-2-(3-methylimidazol-4-yl)-5-(1-methylpyrazol-4-yl)benzimidazol-1-yl]-1,3-dimethyl-4-tetrahydropyran-4-yloxy-benzimidazol-2-one;
6-[6-fluoro-2-(3-methylimidazol-4-yl)-5-(1-methylpyrazol-4-yl)benzimidazol-1-yl]-1,3-dimethyl-4-(4-methylpiperazin-1-yl)benzimidazol-2-one;
6-[6-fluoro-2-(3-methylimidazol-4-yl)-5-(1-methylpyrazol-4-yl)benzimidazol-1-yl]-1,3-dimethyl-4-(1-piperidyl)benzimidazol-2-one;
4-(4,4-difluoro-1-piperidyl)-6-[6-fluoro-2-(3-methylimidazol-4-yl)-5-(1-methylpyrazol-4-yl)benzimidazol-1-yl]-1,3-dimethyl-benzimidazol-2-one;
6-[6-fluoro-2-(1-fluoro-1-methyl-ethyl)-5-(1-methylpyrazol-4-yl)benzimidazol-1-yl]-4-isopropyl-1,3-dimethyl-benzimidazol-2-one;
6-[2-(1,1-difluoroethyl)-6-fluoro-5-(1-methylpyrazol-4-yl)benzimidazol-1-yl]-4-isopropyl-1,3-dimethyl-benzimidazol-2-one;
6-[2-(2,3-dimethylphenyl)-6-fluoro-5-(1-methylpyrazol-4-yl)benzimidazol-1-yl]-4-isopropyl-1,3-dimethyl-benzimidazol-2-one;
6-[6-fluoro-2-(1-methyl-1-phenyl-ethyl)-5-(1-methylpyrazol-4-yl)benzimidazol-1-yl]-4-isopropyl-1,3-dimethyl-benzimidazol-2-one;
6-[5-(1-ethylpyrazol-4-yl)-6-fluoro-2-(3-methylimidazol-4-yl)benzimidazol-1-yl]-4-(isopropylamino)-1,3-dimethyl-benzimidazol-2-one;
6-[2-tert-butyl-6-fluoro-5-(1-methylpyrazol-4-yl)benzimidazol-1-yl]-4-(4-methoxyphenyl)-1,3-dimethyl-benzimidazol-2-one;
4-cyclohexyl-6-[6-fluoro-2-(3-methylimidazol-4-yl)-5-(1-methylpyrazol-4-yl)benzimidazol-1-yl]-1,3-dimethyl-benzimidazol-2-one;
6-[6-fluoro-2-(3-methylimidazol-4-yl)-5-(1-methylpyrazol-4-yl)benzimidazol-1-yl]-4-(isobutylamino)-1,3-dimethyl-benzimidazol-2-one;
6-[2-tert-butyl-6-fluoro-5-(1-methylpyrazol-4-yl)benzimidazol-1-yl]-4-(3,3-difluoroazetidin-1-yl)-1,3-dimethyl-benzimidazol-2-one;
4-(azetidin-1-yl)-6-[2-tert-butyl-6-fluoro-5-(1-methylpyrazol-4-yl)benzimidazol-1-yl]-1,3-dimethyl-benzimidazol-2-one;
1,3-dimethyl-6-[2-(3-methylimidazol-4-yl)-5-(1-methylpyrazol-4-yl)-6-(trifluoromethyl)benzimidazol-1-yl]-4-morpholino-benzimidazol-2-one;
6-[2-tert-butyl-5-(1-methylpyrazol-4-yl)-6-(trifluoromethyl)benzimidazol-1-yl]-1,3-dimethyl-4-morpholino-benzimidazol-2-one;
6-[2-(1,1-dimethylprop-2-ynyl)-6-fluoro-5-(1-methylpyrazol-4-yl)benzimidazol-1-yl]-4-isopropyl-1,3-dimethyl-benzimidazol-2-one;
6-[2-tert-butyl-6-fluoro-5-(2-methoxy-4-pyridyl)benzimidazol-1-yl]-4-(isopropylamino)-1,3-dimethyl-benzimidazol-2-one;
6-[5-(2-amino-4-pyridyl)-2-tert-butyl-6-fluoro-benzimidazol-1-yl]-4-(isopropylamino)-1,3-dimethyl-benzimidazol-2-one;
3-(7-isopropoxy-1,3-dimethyl-2-oxo-benzimidazol-5-yl)-2-(3-methylimidazol-4-yl)-6-(1-methylpyrazol-4-yl)benzimidazole-5-carbonitrile;
2-tert-butyl-3-(7-isopropoxy-1,3-dimethyl-2-oxo-benzimidazol-5-yl)-6-(1-methylpyrazol-4-yl)benzimidazole-5-carbonitrile;
6-[6-fluoro-2-(3-methylimidazol-4-yl)-5-(1-methylpyrazol-4-yl)benzimidazol-1-yl]-1,3-dimethyl-4-[(2S)-2-methylmorpholin-4-yl]benzimidazol-2-one;
6-[6-fluoro-2-(3-methylimidazol-4-yl)-5-(1-methylpyrazol-4-yl)benzimidazol-1-yl]-1,3-dimethyl-4-(2-oxa-6-azaspiro[3.3]heptan-6-yl)benzimidazol-2-one;
4-isopropoxy-1,3-dimethyl-6-[2-(3-methylimidazol-4-yl)-5-(1-methylpyrazol-4-yl)-6-(trifluoromethyl)benzimidazol-1-yl]benzimidazol-2-one;
6-[2-tert-butyl-5-(1-methylpyrazol-4-yl)-6-(trifluoromethyl)benzimidazol-1-yl]-4-isopropoxy-1,3-dimethyl-benzimidazol-2-one;
6-[6-fluoro-2-(3-methylimidazol-4-yl)-5-(1-methylpyrazol-4-yl)benzimidazol-1-yl]-4-(3-hydroxy-3-methyl-azetidin-1-yl)-1,3-dimethyl-benzimidazol-2-one;
6-[6-fluoro-2-(3-methylimidazol-4-yl)-5-(1-methylpyrazol-4-yl)benzimidazol-1-yl]-4-(3-hydroxyazetidin-1-yl)-1,3-dimethyl-benzimidazol-2-one;
4-(3,3-difluoropyrrolidin-1-yl)-6-[6-fluoro-2-(3-methylimidazol-4-yl)-5-(1-methylpyrazol-4-yl)benzimidazol-1-yl]-1,3-dimethyl-benzimidazol-2-one;
6-[6-fluoro-2-(3-methylimidazol-4-yl)-5-(1-methylpyrazol-4-yl)benzimidazol-1-yl]-4-(3-methoxy-3-methyl-azetidin-1-yl)-1,3-dimethyl-benzimidazol-2-one;
6-[6-fluoro-2-(3-methylimidazol-4-yl)-5-(1-methylpyrazol-4-yl)benzimidazol-1-yl]-1,3-dimethyl-4-(tetrahydropyran-4-ylmethylamino)benzimidazol-2-one;
tert-butyl 4-[6-[6-fluoro-2-(3-methylimidazol-4-yl)-5-(1-methylpyrazol-4-yl)benzimidazol-1-yl]-1,3-dimethyl-2-oxo-benzimidazol-4-yl]piperidine-1-carboxylate;
tert-butyl 4-[6-[2-tert-butyl-6-fluoro-5-(1-methylpyrazol-4-yl)benzimidazol-1-yl]-1,3-dimethyl-2-oxo-benzimidazol-4-yl]piperidine-1-carboxylate;
2-(1,1-dimethylprop-2-ynyl)-3-(7-isopropyl-1,3-dimethyl-2-oxo-benzimidazol-5-yl)-6-(1-methylpyrazol-4-yl)benzimidazole-5-carbonitrile;
2-(1,1-dimethylprop-2-ynyl)-3-(7-isopropoxy-1,3-dimethyl-2-oxo-benzimidazol-5-yl)-6-(1-methylpyrazol-4-yl)benzimidazole-5-carbonitrile;
6-[6-fluoro-2-(3-methylimidazol-4-yl)-5-(1-methylpyrazol-4-yl)benzimidazol-1-yl]-1,3-dimethyl-4-(4-piperidyl)benzimidazol-2-one;
6-[2-tert-butyl-6-fluoro-5-(1-methylpyrazol-4-yl)benzimidazol-1-yl]-1,3-dimethyl-4-[rac-(3R)-3-methylmorpholin-4-yl]benzimidazol-2-one;
6-[2-tert-butyl-6-fluoro-5-(1-methylpyrazol-4-yl)benzimidazol-1-yl]-1,3-dimethyl-4-[rac-(3S)-3-methylmorpholin-4-yl]benzimidazol-2-one;
6-[6-fluoro-2-(3-methylimidazol-4-yl)-5-(1-methylpyrazol-4-yl)benzimidazol-1-yl]-1,3-dimethyl-4-[rac-(3R)-3-methylmorpholin-4-yl]benzimidazol-2-one;
6-[6-fluoro-2-(3-methylimidazol-4-yl)-5-(1-methylpyrazol-4-yl)benzimidazol-1-yl]-1,3-dimethyl-4-[rac-(3S)-3-methylmorpholin-4-yl]benzimidazol-2-one;
6-[2-tert-butyl-6-(difluoromethyl)-5-(1-methylpyrazol-4-yl)benzimidazol-1-yl]-4-(3-hydroxy-3-methyl-azetidin-1-yl)-1,3-dimethyl-benzimidazol-2-one;
4-(1,3,3a,4,6,6a-hexahydrofuro[3,4-c]pyrrol-5-yl)-6-[6-fluoro-2-(3-methylimidazol-4-yl)-5-(1-methylpyrazol-4-yl)benzimidazol-1-yl]-1,3-dimethyl-benzimidazol-2-one;
6-[6-fluoro-2-(3-methylimidazol-4-yl)-5-(1-methylpyrazol-4-yl)benzimidazol-1-yl]-1,3-dimethyl-4-(oxetan-3-ylamino)benzimidazol-2-one;
6-[6-fluoro-2-(3-methylimidazol-4-yl)-5-(1-methylpyrazol-4-yl)benzimidazol-1-yl]-4-(3-methoxyazetidin-1-yl)-1,3-dimethyl-benzimidazol-2-one;
6-[2-tert-butyl-6-fluoro-5-(1-methylpyrazol-4-yl)benzimidazol-1-yl]-1,3-dimethyl-4-(4-piperidyl)benzimidazol-2-one;
4-(1-acetyl-4-piperidyl)-6-[6-fluoro-2-(3-methylimidazol-4-yl)-5-(1-methylpyrazol-4-yl)benzimidazol-1-yl]-1,3-dimethyl-benzimidazol-2-one;
6-[6-fluoro-2-(3-methylimidazol-4-yl)-5-(1-methylpyrazol-4-yl)benzimidazol-1-yl]-1,3-dimethyl-4-(1-methyl-4-piperidyl)benzimidazol-2-one;
6-[2-(1,1-dimethylprop-2-ynyl)-6-fluoro-5-(1-methylpyrazol-4-yl)benzimidazol-1-yl]-1,3-dimethyl-4-morpholino-benzimidazol-2-one;
6-[2-tert-butyl-6-fluoro-5-(5-methyl-1,2,4-oxadiazol-3-yl)benzimidazol-1-yl]-4-(isopropylamino)-1,3-dimethyl-benzimidazol-2-one;
6-[2-tert-butyl-6-fluoro-5-(3-methyl-1,2,4-oxadiazol-5-yl)benzimidazol-1-yl]-4-(isopropylamino)-1,3-dimethyl-benzimidazol-2-one;
6-[6-fluoro-2-(3-methylimidazol-4-yl)-5-(3-methyl-1,2,4-oxadiazol-5-yl)benzimidazol-1-yl]-4-(isopropylamino)-1,3-dimethyl-benzimidazol-2-one;
2-tert-butyl-3-[7-(3-hydroxy-3-methyl-azetidin-1-yl)-1,3-dimethyl-2-oxo-benzimidazol-5-yl]-6-(1-methylpyrazol-4-yl)benzimidazole-5-carbonitrile;
3-[7-(3-hydroxy-3-methyl-azetidin-1-yl)-1,3-dimethyl-2-oxo-benzimidazol-5-yl]-2-(3-methylimidazol-4-yl)-6-(1-methylpyrazol-4-yl)benzimidazole-5-carbonitrile;
6-[6-fluoro-2-(3-methylimidazol-4-yl)-5-(1-methylpyrazol-4-yl)benzimidazol-1-yl]-1,3-dimethyl-4-(6-oxa-3-azabicyclo[3.1.1]heptan-3-yl)benzimidazol-2-one;
tert-butyl 4-[6-[6-fluoro-2-(3-methylimidazol-4-yl)-5-(1-methylpyrazol-4-yl)benzimidazol-1-yl]-1,3-dimethyl-2-oxo-benzimidazol-4-yl]piperazine-1-carboxylate;
6-[2-tert-butyl-6-fluoro-5-(1-methylpyrazol-4-yl)benzimidazol-1-yl]-4-(3-hydroxy-3-methyl-azetidin-1-yl)-1,3-dimethyl-benzimidazol-2-one;
4-(3,3-dimethylazetidin-1-yl)-6-[6-fluoro-2-(3-methylimidazol-4-yl)-5-(1-methylpyrazol-4-yl)benzimidazol-1-yl]-1,3-dimethyl-benzimidazol-2-one;
6-[6-(difluoromethyl)-2-(3-methylimidazol-4-yl)-5-(1-methylpyrazol-4-yl)benzimidazol-1-yl]-4-isopropoxy-1,3-dimethyl-benzimidazol-2-one;
6-[2-tert-butyl-6-(difluoromethyl)-5-(1-methylpyrazol-4-yl)benzimidazol-1-yl]-4-isopropoxy-1,3-dimethyl-benzimidazol-2-one;
4-(3-hydroxy-3-methyl-azetidin-1-yl)-1,3-dimethyl-6-[2-(3-methylimidazol-4-yl)-5-(1-methylpyrazol-4-yl)-6-(trifluoromethyl)benzimidazol-1-yl]benzimidazol-2-one;
6-[2-tert-butyl-5-(1-methylpyrazol-4-yl)-6-(trifluoromethyl)benzimidazol-1-yl]-4-(3-hydroxy-3-methyl-azetidin-1-yl)-1,3-dimethyl-benzimidazol-2-one;
6-[2-(1,1-dimethylprop-2-ynyl)-5-(1-methylpyrazol-4-yl)-6-(trifluoromethyl)benzimidazol-1-yl]-1,3-dimethyl-4-morpholino-benzimidazol-2-one;
6-[5-(1-ethylpyrazol-4-yl)-6-fluoro-2-(3-methylimidazol-4-yl)benzimidazol-1-yl]-1,3-dimethyl-4-morpholino-benzimidazol-2-one;
6-[6-fluoro-2-(3-methylimidazol-4-yl)-5-[1-(2,2,2-trifluoroethyl)pyrazol-4-yl]benzimidazol-1-yl]-1,3-dimethyl-4-morpholino-benzimidazol-2-one;
6-[6-fluoro-5-(1-isopropylpyrazol-4-yl)-2-(3-methylimidazol-4-yl)benzimidazol-1-yl]-1,3-dimethyl-4-morpholino-benzimidazol-2-one;
6-[5-[1-(difluoromethyl)pyrazol-4-yl]-6-fluoro-2-(3-methylimidazol-4-yl)benzimidazol-1-yl]-1,3-dimethyl-4-morpholino-benzimidazol-2-one;
3-[7-(3-methoxy-3-methyl-azetidin-1-yl)-1,3-dimethyl-2-oxo-benzimidazol-5-yl]-2-(3-methylimidazol-4-yl)-6-(1-methylpyrazol-4-yl)benzimidazole-5-carbonitrile;
2-tert-butyl-3-[7-(3-methoxy-3-methyl-azetidin-1-yl)-1,3-dimethyl-2-oxo-benzimidazol-5-yl]-6-(1-methylpyrazol-4-yl)benzimidazole-5-carbonitrile;
3-[7-(azetidin-1-yl)-1,3-dimethyl-2-oxo-benzimidazol-5-yl]-2-tert-butyl-6-(1-methylpyrazol-4-yl)benzimidazole-5-carbonitrile;
3-[7-(3,3-difluoroazetidin-1-yl)-1,3-dimethyl-2-oxo-benzimidazol-5-yl]-2-(3-methylimidazol-4-yl)-6-(1-methylpyrazol-4-yl)benzimidazole-5-carbonitrile;
2-tert-butyl-3-[7-(3,3-difluoroazetidin-1-yl)-1,3-dimethyl-2-oxo-benzimidazol-5-yl]-6-(1-methylpyrazol-4-yl)benzimidazole-5-carbonitrile;
6-[2-tert-butyl-6-(difluoromethyl)-5-(1-methylpyrazol-4-yl)benzimidazol-1-yl]-1,3-dimethyl-4-tetrahydropyran-4-yl-benzimidazol-2-one;
6-[6-(difluoromethyl)-2-(3-methylimidazol-4-yl)-5-(1-methylpyrazol-4-yl)benzimidazol-1-yl]-1,3-dimethyl-4-tetrahydropyran-4-yl-benzimidazol-2-one;
3-(1,3-dimethyl-2-oxo-7-tetrahydropyran-4-yl-benzimidazol-5-yl)-2-(3-methylimidazol-4-yl)-6-(1-methylpyrazol-4-yl)benzimidazole-5-carbonitrile;
2-tert-butyl-3-(1,3-dimethyl-2-oxo-7-tetrahydropyran-4-yl-benzimidazol-5-yl)-6-(1-methylpyrazol-4-yl)benzimidazole-5-carbonitrile;
6-[6-(difluoromethyl)-2-(3-methylimidazol-4-yl)-5-(1-methylpyrazol-4-yl)benzimidazol-1-yl]-4-(3-hydroxy-3-methyl-azetidin-1-yl)-1,3-dimethyl-benzimidazol-2-one;
6-[6-(difluoromethyl)-2-(3-methylimidazol-4-yl)-5-(1-methylpyrazol-4-yl)benzimidazol-1-yl]-4-(3-methoxy-3-methyl-azetidin-1-yl)-1,3-dimethyl-benzimidazol-2-one;
6-[2-tert-butyl-6-(difluoromethyl)-5-(1-methylpyrazol-4-yl)benzimidazol-1-yl]-4-(3-methoxy-3-methyl-azetidin-1-yl)-1,3-dimethyl-benzimidazol-2-one;
6-[2-tert-butyl-6-(difluoromethyl)-5-(1-methylpyrazol-4-yl)benzimidazol-1-yl]-4-(3-ethoxy-3-methyl-azetidin-1-yl)-1,3-dimethyl-benzimidazol-2-one;
4-(3-methoxy-3-methyl-azetidin-1-yl)-1,3-dimethyl-6-[2-(3-methylimidazol-4-yl)-5-(1-methylpyrazol-4-yl)-6-(trifluoromethyl)benzimidazol-1-yl]benzimidazol-2-one;
6-[2-tert-butyl-5-(1-methylpyrazol-4-yl)-6-(trifluoromethyl)benzimidazol-1-yl]-4-(3-methoxy-3-methyl-azetidin-1-yl)-1,3-dimethyl-benzimidazol-2-one;
4-(azetidin-1-yl)-1,3-dimethyl-6-[2-(3-methylimidazol-4-yl)-5-(1-methylpyrazol-4-yl)-6-(trifluoromethyl)benzimidazol-1-yl]benzimidazol-2-one;
4-(3,3-difluoroazetidin-1-yl)-1,3-dimethyl-6-[2-(3-methylimidazol-4-yl)-5-(1-methylpyrazol-4-yl)-6-(trifluoromethyl)benzimidazol-1-yl]benzimidazol-2-one;
6-[6-fluoro-2-(3-methylimidazol-4-yl)-5-(1-methylpyrazol-4-yl)benzimidazol-1-yl]-4-[(2S,3R)-3-hydroxy-2-methyl-azetidin-1-yl]-1,3-dimethyl-benzimidazol-2-one;
6-[2-tert-butyl-5-(1-methylpyrazol-4-yl)-6-(trifluoromethyl)benzimidazol-1-yl]-4-(3,3-difluoroazetidin-1-yl)-1,3-dimethyl-benzimidazol-2-one;
6-[6-fluoro-2-(3-methylimidazol-4-yl)-5-(1-methylpyrazol-4-yl)benzimidazol-1-yl]-1,3-dimethyl-4-[(1S,4S)-2-oxa-5-azabicyclo[2.2.1]heptan-5-yl]benzimidazol-2-one;
6-[6-fluoro-2-(3-methylimidazol-4-yl)-5-(1-methylpyrazol-4-yl)benzimidazol-1-yl]-1,3-dimethyl-4-[(2S)-2-methylazetidin-1-yl]benzimidazol-2-one;
6-[6-fluoro-2-(3-methylimidazol-4-yl)-5-(1H-pyrazol-4-yl)benzimidazol-1-yl]-1,3-dimethyl-4-morpholino-benzimidazol-2-one;
4-[1-(1,3-dimethyl-7-morpholino-2-oxo-benzimidazol-5-yl)-6-fluoro-2-(3-methylimidazol-4-yl)benzimidazol-5-yl]-2-fluoro-N-methyl-benzamide;
6-[5-[1-(cyclopropylmethyl)pyrazol-4-yl]-6-fluoro-2-(3-methylimidazol-4-yl)benzimidazol-1-yl]-1,3-dimethyl-4-morpholino-benzimidazol-2-one;
6-[6-fluoro-2-(3-methylimidazol-4-yl)-5-(2-methylthiazol-5-yl)benzimidazol-1-yl]-1,3-dimethyl-4-morpholino-benzimidazol-2-one;
5-[2-tert-butyl-6-(difluoromethyl)-5-(1-methylpyrazol-4-yl)benzimidazol-1-yl]-1,3-dimethyl-benzimidazol-2-one;
4-(azetidin-1-yl)-6-[2-tert-butyl-5-(1-methylpyrazol-4-yl)-6-(trifluoromethyl)benzimidazol-1-yl]-1,3-dimethyl-benzimidazol-2-one;
1,3-dimethyl-6-[2-(3-methylimidazol-4-yl)-5-(1-methylpyrazol-4-yl)-6-(trifluoromethyl)benzimidazol-1-yl]-4-tetrahydropyran-4-yl-benzimidazol-2-one;
6-[2-tert-butyl-5-(1-methylpyrazol-4-yl)-6-(trifluoromethyl)benzimidazol-1-yl]-1,3-dimethyl-4-tetrahydropyran-4-yl-benzimidazol-2-one;
4-(azetidin-1-yl)-6-[6-(difluoromethyl)-2-(3-methylimidazol-4-yl)-5-(1-methylpyrazol-4-yl)benzimidazol-1-yl]-1,3-dimethyl-benzimidazol-2-one;
4-(azetidin-1-yl)-6-[2-tert-butyl-6-(difluoromethyl)-5-(1-methylpyrazol-4-yl)benzimidazol-1-yl]-1,3-dimethyl-benzimidazol-2-one;
6-[2-tert-butyl-6-(difluoromethyl)-5-(1-ethylpyrazol-4-yl)benzimidazol-1-yl]-1,3-dimethyl-4-morpholino-benzimidazol-2-one;
6-[2-tert-butyl-6-(difluoromethyl)-5-(2-methylthiazol-5-yl)benzimidazol-1-yl]-1,3-dimethyl-4-morpholino-benzimidazol-2-one;
6-[2-tert-butyl-5-(1-isopropylpyrazol-4-yl)-6-(trifluoromethyl)benzimidazol-1-yl]-4-(3-hydroxy-3-methyl-azetidin-1-yl)-1,3-dimethyl-benzimidazol-2-one;
6-[2-tert-butyl-5-(2-methylthiazol-5-yl)-6-(trifluoromethyl)benzimidazol-1-yl]-4-(3-hydroxy-3-methyl-azetidin-1-yl)-1,3-dimethyl-benzimidazol-2-one;
6-[5-(1-cyclopropylpyrazol-4-yl)-6-fluoro-2-(3-methylimidazol-4-yl)benzimidazol-1-yl]-1,3-dimethyl-4-morpholino-benzimidazol-2-one;
6-[2-tert-butyl-6-(difluoromethyl)-5-(1-methylpyrazol-4-yl)benzimidazol-1-yl]-4-(3-isopropoxy-3-methyl-azetidin-1-yl)-1,3-dimethyl-benzimidazol-2-one;
4-(3-allyloxy-3-methyl-azetidin-1-yl)-6-[2-tert-butyl-6-(difluoromethyl)-5-(1-methylpyrazol-4-yl)benzimidazol-1-yl]-1,3-dimethyl-benzimidazol-2-one;
6-[2-tert-butyl-6-(difluoromethyl)-5-(1-ethylpyrazol-4-yl)benzimidazol-1-yl]-4-(3-hydroxy-3-methyl-azetidin-1-yl)-1,3-dimethyl-benzimidazol-2-one;
6-[2-tert-butyl-6-(difluoromethyl)-5-(1-isopropylpyrazol-4-yl)benzimidazol-1-yl]-4-(3-hydroxy-3-methyl-azetidin-1-yl)-1,3-dimethyl-benzimidazol-2-one;
6-[2-tert-butyl-6-(difluoromethyl)-5-(2-methylthiazol-5-yl)benzimidazol-1-yl]-4-(3-hydroxy-3-methyl-azetidin-1-yl)-1,3-dimethyl-benzimidazol-2-one;
2-tert-butyl-3-[7-(3-hydroxy-3-methyl-azetidin-1-yl)-1,3-dimethyl-2-oxo-benzimidazol-5-yl]-6-(2-methylthiazol-5-yl)benzimidazole-5-carbonitrile;
6-[2-tert-butyl-6-(difluoromethyl)-5-(2-methylthiazol-5-yl)benzimidazol-1-yl]-1,3-dimethyl-4-tetrahydropyran-4-yl-benzimidazol-2-one;
6-[2-tert-butyl-6-(1-methylpyrazol-4-yl)imidazo[4,5-c]pyridin-3-yl]-1,3-dimethyl-4-morpholino-benzimidazol-2-one;
methyl 2-tert-butyl-3-(1,3-dimethyl-7-morpholino-2-oxo-benzimidazol-5-yl)-6-(1-methylpyrazol-4-yl)benzimidazole-5-carboxylate;
6-[2-tert-butyl-6-(2-methylthiazol-5-yl)imidazo[4,5-c]pyridin-3-yl]-1,3-dimethyl-4-morpholino-benzimidazol-2-one;
6-[2-tert-butyl-5-(2-methylthiazol-5-yl)-6-(trifluoromethyl)benzimidazol-1-yl]-1,3-dimethyl-4-morpholino-benzimidazol-2-one;
6-[2-tert-butyl-5-(2-methylthiazol-5-yl)-6-(trifluoromethyl)benzimidazol-1-yl]-1,3-dimethyl-4-tetrahydropyran-4-yl-benzimidazol-2-one;
4-(3,3-difluoroazetidin-1-yl)-6-[6-(difluoromethyl)-2-(3-methylimidazol-4-yl)-5-(1-methylpyrazol-4-yl)benzimidazol-1-yl]-1,3-dimethyl-benzimidazol-2-one;
6-[2-tert-butyl-6-(difluoromethyl)-5-(1-methylpyrazol-4-yl)benzimidazol-1-yl]-4-(3,3-difluoroazetidin-1-yl)-1,3-dimethyl-benzimidazol-2-one;
tert-butyl 4-[6-[6-(difluoromethyl)-2-(3-methylimidazol-4-yl)-5-(1-methylpyrazol-4-yl)benzimidazol-1-yl]-1,3-dimethyl-2-oxo-benzimidazol-4-yl]piperazine-1-carboxylate;
6-[2-tert-butyl-6-(hydroxymethyl)-5-(1-methylpyrazol-4-yl)benzimidazol-1-yl]-1,3-dimethyl-4-morpholino-benzimidazol-2-one;
6-[2-tert-butyl-6-(methoxymethyl)-5-(1-methylpyrazol-4-yl)benzimidazol-1-yl]-1,3-dimethyl-4-morpholino-benzimidazol-2-one;
6-[2-tert-butyl-6-(fluoromethyl)-5-(1-methylpyrazol-4-yl)benzimidazol-1-yl]-1,3-dimethyl-4-morpholino-benzimidazol-2-one;
1,3-dimethyl-6-[2-(3-methylimidazol-4-yl)-6-(1-methylpyrazol-4-yl)imidazo[4,5-c]pyridin-3-yl]-4-morpholino-benzimidazol-2-one;
1,3-dimethyl-6-[2-(3-methylimidazol-4-yl)-6-(2-methylthiazol-5-yl)imidazo[4,5-c]pyridin-3-yl]-4-morpholino-benzimidazol-2-one;
6-[2-tert-butyl-6-(difluoromethyl)-5-(1-methylpyrazol-4-yl)benzimidazol-1-yl]-1,3-dimethyl-4-(2-morpholinoethoxy)benzimidazol-2-one;
6-[6-(difluoromethyl)-2-(3-methylimidazol-4-yl)-5-(2-methylthiazol-5-yl)benzimidazol-1-yl]-1,3-dimethyl-4-(2-morpholinoethoxy)benzimidazol-2-one;
6-[2-tert-butyl-6-(difluoromethyl)-5-(2-methylthiazol-5-yl)benzimidazol-1-yl]-1,3-dimethyl-4-(2-morpholinoethoxy)benzimidazol-2-one;
6-[6-(difluoromethyl)-2-(3-methylimidazol-4-yl)-5-(1-methylpyrazol-4-yl)benzimidazol-1-yl]-1,3-dimethyl-4-(oxetan-3-yloxy)benzimidazol-2-one;
6-[2-tert-butyl-6-(difluoromethyl)-5-(1-methylpyrazol-4-yl)benzimidazol-1-yl]-1,3-dimethyl-4-(oxetan-3-yloxy)benzimidazol-2-one;
6-[2-tert-butyl-6-methoxy-5-(1-methylpyrazol-4-yl)benzimidazol-1-yl]-1,3-dimethyl-4-morpholino-benzimidazol-2-one;
6-[6-methoxy-2-(3-methylimidazol-4-yl)-5-(1-methylpyrazol-4-yl)benzimidazol-1-yl]-1,3-dimethyl-4-morpholino-benzimidazol-2-one;
methyl 2-tert-butyl-1-(1,3-dimethyl-7-morpholino-2-oxo-benzimidazol-5-yl)-6-fluoro-benzimidazole-5-carboxylate;
2-tert-butyl-1-(1,3-dimethyl-7-morpholino-2-oxo-benzimidazol-5-yl)-6-fluoro-benzimidazole-5-carboxylic acid;
2-tert-butyl-1-(1,3-dimethyl-7-morpholino-2-oxo-benzimidazol-5-yl)-6-fluoro-N-methyl-benzimidazole-5-carboxamide;
2-tert-butyl-1-(1,3-dimethyl-7-morpholino-2-oxo-benzimidazol-5-yl)-6-fluoro-N,N-dimethyl-benzimidazole-5-carboxamide;
2-tert-butyl-1-(1,3-dimethyl-7-morpholino-2-oxo-benzimidazol-5-yl)-6-fluoro-N-phenyl-benzimidazole-5-carboxamide;
6-[6-(difluoromethyl)-2-(3-methylimidazol-4-yl)-5-(1-methylpyrazol-4-yl)benzimidazol-1-yl]-1,3-dimethyl-4-[(4S)-2-oxa-5-azabicyclo[2.2.1]heptan-5-yl]benzimidazol-2-one;
2-tert-butyl-3-(1,3-dimethyl-7-morpholino-2-oxo-benzimidazol-5-yl)-6-(1-methylpyrazol-4-yl)benzimidazole-5-carboxylic acid;
2-tert-butyl-3-(1,3-dimethyl-7-morpholino-2-oxo-benzimidazol-5-yl)-N-methyl-6-(1-methylpyrazol-4-yl)benzimidazole-5-carboxamide;
6-[2-tert-butyl-6-(difluoromethoxy)-5-(1-methylpyrazol-4-yl)benzimidazol-1-yl]-1,3-dimethyl-4-morpholino-benzimidazol-2-one;
6-[2-tert-butyl-6-(difluoromethoxy)-5-(2-methylthiazol-5-yl)benzimidazol-1-yl]-1,3-dimethyl-4-morpholino-benzimidazol-2-one;
6-[6-(difluoromethoxy)-2-(3-methylimidazol-4-yl)-5-(1-methylpyrazol-4-yl)benzimidazol-1-yl]-1,3-dimethyl-4-morpholino-benzimidazol-2-one;
6-[2-tert-butyl-6-chloro-5-(1-methylpyrazol-4-yl)benzimidazol-1-yl]-1,3-dimethyl-4-morpholino-benzimidazol-2-one;
6-[6-chloro-2-(3-methylimidazol-4-yl)-5-(1-methylpyrazol-4-yl)benzimidazol-1-yl]-1,3-dimethyl-4-morpholino-benzimidazol-2-one;
2-tert-butyl-3-(1,3-dimethyl-7-morpholino-2-oxo-benzimidazol-5-yl)-6-(1-methylpyrazol-4-yl)benzimidazole-5-carbaldehyde;
4-benzyloxy-6-[2-tert-butyl-6-(difluoromethyl)-5-(1-methylpyrazol-4-yl)benzimidazol-1-yl]-1,3-dimethyl-benzimidazol-2-one;
4-benzyloxy-6-[6-(difluoromethyl)-2-(3-methylimidazol-4-yl)-5-(1-methylpyrazol-4-yl)benzimidazol-1-yl]-1,3-dimethyl-benzimidazol-2-one;
6-[2-tert-butyl-6-(difluoromethyl)-5-(1-methylpyrazol-4-yl)benzimidazol-1-yl]-4-hydroxy-1,3-dimethyl-benzimidazol-2-one;
6-[2-tert-butyl-6-(difluoromethyl)-5-(2-methylthiazol-5-yl)benzimidazol-1-yl]-1,3-dimethyl-4-(oxetan-3-yloxy)benzimidazol-2-one;
6-[2-tert-butyl-6-(difluoromethyl)-5-(3-methylpyrazol-1-yl)benzimidazol-1-yl]-4-(3-hydroxy-3-methyl-azetidin-1-yl)-1,3-dimethyl-benzimidazol-2-one;
6-[2-tert-butyl-6-(difluoromethyl)-5-(4-methylpyrazol-1-yl)benzimidazol-1-yl]-4-(3-hydroxy-3-methyl-azetidin-1-yl)-1,3-dimethyl-benzimidazol-2-one;
6-[2-tert-butyl-6-fluoro-5-(5-methyl-1,3,4-oxadiazol-2-yl)benzimidazol-1-yl]-1,3-dimethyl-4-morpholino-benzimidazol-2-one;
6-[2-tert-butyl-6-(difluoromethyl)-5-(4-methylimidazol-1-yl)benzimidazol-1-yl]-4-(3-hydroxy-3-methyl-azetidin-1-yl)-1,3-dimethyl-benzimidazol-2-one;
6-[2-tert-butyl-5-cyclopropyl-6-(difluoromethyl)benzimidazol-1-yl]-4-(3-hydroxy-3-methyl-azetidin-1-yl)-1,3-dimethyl-benzimidazol-2-one;
6-[2-tert-butyl-6-methoxy-5-(1-methylpyrazol-4-yl)benzimidazol-1-yl]-4-(3-hydroxy-3-methyl-azetidin-1-yl)-1,3-dimethyl-benzimidazol-2-one;
6-[2-tert-butyl-6-(difluoromethyl)-5-(1-methylpyrazol-4-yl)benzimidazol-1-yl]-1,3-dimethyl-4-[(4S)-2-oxa-5-azabicyclo[2.2.1]heptan-5-yl]benzimidazol-2-one;
5-[2-tert-butyl-6-fluoro-5-(1-methylpyrazol-4-yl)benzimidazol-1-yl]-1,3-dimethyl-benzimidazol-2-one;
6-[2-tert-butyl-6-(difluoromethyl)-5-(1-methylpyrazol-4-yl)benzimidazol-1-yl]-1,3-dimethyl-4-thiomorpholino-benzimidazol-2-one;
(5E)-2-tert-butyl-3-(1,3-dimethyl-7-morpholino-2-oxo-benzimidazol-5-yl)-6-(1-methylpyrazol-4-yl)benzimidazole-5-carbaldehyde oxime;
6-[2-tert-butyl-6-(difluoromethyl)-5-(1-methylpyrazol-4-yl)benzimidazol-1-yl]-4-(6-chloropyrimidin-4-yl)oxy-1,3-dimethyl-benzimidazol-2-one;
6-[2-tert-butyl-6-(difluoromethyl)-5-(1-methylpyrazol-4-yl)benzimidazol-1-yl]-4-(4-methoxyphenoxy)-1,3-dimethyl-benzimidazol-2-one;
6-[2-tert-butyl-6-(difluoromethyl)-5-(1-methylpyrazol-4-yl)benzimidazol-1-yl]-4-(4-fluorophenoxy)-1,3-dimethyl-benzimidazol-2-one;
6-[2-tert-butyl-6-chloro-5-(1-methylpyrazol-4-yl)benzimidazol-1-yl]-4-(3-hydroxy-3-methyl-azetidin-1-yl)-1,3-dimethyl-benzimidazol-2-one;
6-[2-tert-butyl-6-chloro-5-(2-methylthiazol-5-yl)benzimidazol-1-yl]-4-(3-hydroxy-3-methyl-azetidin-1-yl)-1,3-dimethyl-benzimidazol-2-one;
6-[2-tert-butyl-6-(difluoromethoxy)-5-(1-methylpyrazol-4-yl)benzimidazol-1-yl]-4-(3-hydroxy-3-methyl-azetidin-1-yl)-1,3-dimethyl-benzimidazol-2-one;
6-[2-tert-butyl-6-methoxy-5-(2-methylthiazol-5-yl)benzimidazol-1-yl]-4-(3-hydroxy-3-methyl-azetidin-1-yl)-1,3-dimethyl-benzimidazol-2-one;
6-[2-tert-butyl-6-fluoro-5-(5-methyl-4H-1,2,4-triazol-3-yl)benzimidazol-1-yl]-1,3-dimethyl-4-morpholino-benzimidazol-2-one;
4-(3-hydroxy-3-methyl-azetidin-1-yl)-6-[6-methoxy-2-(3-methylimidazol-4-yl)-5-(1-methylpyrazol-4-yl)benzimidazol-1-yl]-1,3-dimethyl-benzimidazol-2-one;
methyl 1-[6-[2-tert-butyl-6-fluoro-5-(1-methylpyrazol-4-yl)benzimidazol-1-yl]-1,3-dimethyl-2-oxo-benzimidazol-4-yl]aziridine-2-carboxylate;
6-[6-(difluoromethyl)-2-(3-methylimidazol-4-yl)-5-(1-methylpyrazol-4-yl)benzimidazol-1-yl]-1,3-dimethyl-4-thiomorpholino-benzimidazol-2-one;
6-[2-tert-butyl-6-(difluoromethyl)-5-(1-methylpyrazol-4-yl)benzimidazol-1-yl]-4-(1,1-dioxo-1,4-thiazinan-4-yl)-1,3-dimethyl-benzimidazol-2-one;
6-[6-(difluoromethyl)-2-(3-methylimidazol-4-yl)-5-(2-methylthiazol-5-yl)benzimidazol-1-yl]-1,3-dimethyl-4-thiomorpholino-benzimidazol-2-one;
6-[2-tert-butyl-6-(difluoromethyl)-5-[1-(2-hydroxyethyl)pyrazol-4-yl]benzimidazol-1-yl]-4-(3-hydroxy-3-methyl-azetidin-1-yl)-1,3-dimethyl-benzimidazol-2-one;
6-[2-tert-butyl-6-(difluoromethyl)-5-(2-oxopyrrolidin-1-yl)benzimidazol-1-yl]-4-(3-hydroxy-3-methyl-azetidin-1-yl)-1,3-dimethyl-benzimidazol-2-one;
6-[2-tert-butyl-6-fluoro-5-(1-methyltriazol-4-yl)benzimidazol-1-yl]-1,3-dimethyl-4-morpholino-benzimidazol-2-one;
tert-butyl N-[2-tert-butyl-3-(1,3-dimethyl-7-morpholino-2-oxo-benzimidazol-5-yl)-6-(1-methylpyrazol-4-yl)benzimidazol-5-yl]carbamate;
6-[6-amino-2-tert-butyl-5-(1-methylpyrazol-4-yl)benzimidazol-1-yl]-1,3-dimethyl-4-morpholino-benzimidazol-2-one;
6-[2-tert-butyl-6-(methylaminomethyl)-5-(1-methylpyrazol-4-yl)benzimidazol-1-yl]-1,3-dimethyl-4-morpholino-benzimidazol-2-one;
6-[2-tert-butyl-6-[(dimethylamino)methyl]-5-(1-methylpyrazol-4-yl)benzimidazol-1-yl]-1,3-dimethyl-4-morpholino-benzimidazol-2-one;
6-[2-tert-butyl-5-(1-methylpyrazol-4-yl)-6-(morpholinomethyl)benzimidazol-1-yl]-1,3-dimethyl-4-morpholino-benzimidazol-2-one;
N-[2-tert-butyl-3-(1,3-dimethyl-7-morpholino-2-oxo-benzimidazol-5-yl)-6-(1-methylpyrazol-4-yl)benzimidazol-5-yl]acetamide;
4-(3-hydroxy-3-methyl-azetidin-1-yl)-1,3-dimethyl-6-[2-(3-methylimidazol-4-yl)-5-(1-methylpyrazol-4-yl)-6-(trifluoromethoxy)benzimidazol-1-yl]benzimidazol-2-one;
6-[2-tert-butyl-5-(1-methylpyrazol-4-yl)-6-(trifluoromethoxy)benzimidazol-1-yl]-4-(3-hydroxy-3-methyl-azetidin-1-yl)-1,3-dimethyl-benzimidazol-2-one;
6-[2-tert-butyl-5-(2-methylthiazol-5-yl)-6-(trifluoromethoxy)benzimidazol-1-yl]-4-(3-hydroxy-3-methyl-azetidin-1-yl)-1,3-dimethyl-benzimidazol-2-one;
6-[2-tert-butyl-6-fluoro-5-(1-methylpyrazol-4-yl)benzimidazol-1-yl]-1,3-dimethyl-4-(oxetan-3-ylmethoxy)benzimidazol-2-one;
6-[2-tert-butyl-6-fluoro-5-(1-methylpyrazol-4-yl)benzimidazol-1-yl]-1,3-dimethyl-4-[(1-methyl-4-piperidyl)oxy]benzimidazol-2-one;
6-[2-tert-butyl-6-fluoro-5-(1-methylpyrazol-4-yl)benzimidazol-1-yl]-1,3-dimethyl-4-[(2R)-2-methylazetidin-1-yl]benzimidazol-2-one;
6-[6-fluoro-2-(3-methylimidazol-4-yl)-5-(1-methylpyrazol-4-yl)benzimidazol-1-yl]-1,3-dimethyl-4-[(2R)-2-methylazetidin-1-yl]benzimidazol-2-one;
6-[2-tert-butyl-6-fluoro-5-(1-methylpyrazol-4-yl)benzimidazol-1-yl]-4-(3-hydroxypyrrolidin-1-yl)-1,3-dimethyl-benzimidazol-2-one;
6-[6-fluoro-2-(3-methylimidazol-4-yl)-5-(1-methylpyrazol-4-yl)benzimidazol-1-yl]-4-(3-hydroxypyrrolidin-1-yl)-1,3-dimethyl-benzimidazol-2-one;
6-[2-tert-butyl-6-(methylamino)-5-(1-methylpyrazol-4-yl)benzimidazol-1-yl]-1,3-dimethyl-4-morpholino-benzimidazol-2-one;
6-[2-tert-butyl-6-(dimethylamino)-5-(1-methylpyrazol-4-yl)benzimidazol-1-yl]-1,3-dimethyl-4-morpholino-benzimidazol-2-one;
6-[2-tert-butyl-6-(difluoromethyl)-5-(1-methylpyrazol-4-yl)benzimidazol-1-yl]-4-[(3-chloro-2-hydroxy-2-methyl-propyl)amino]-1,3-dimethyl-benzimidazol-2-one;
6-[2-tert-butyl-6-fluoro-5-(1-methylpyrazol-4-yl)benzimidazol-1-yl]-1,3-dimethyl-4-[(4-oxocyclohexyl)methoxy]benzimidazol-2-one;
6-[2-tert-butyl-6-fluoro-5-(1-methylpyrazol-4-yl)benzimidazol-1-yl]-4-[(4-hydroxy-4-methyl-cyclohexyl)methoxy]-1,3-dimethyl-benzimidazol-2-one;
6-[2-tert-butyl-6-fluoro-5-(1-methylpyrazol-4-yl)benzimidazol-1-yl]-4-[(4-hydroxy-4-methyl-cyclohexyl)methoxy]-1,3-dimethyl-benzimidazol-2-one;
6-[6-benzyloxy-2-tert-butyl-5-(1-methylpyrazol-4-yl)benzimidazol-1-yl]-4-(3-hydroxy-3-methyl-azetidin-1-yl)-1,3-dimethyl-benzimidazol-2-one;
6-[2-tert-butyl-6-hydroxy-5-(1-methylpyrazol-4-yl)benzimidazol-1-yl]-4-(3-hydroxy-3-methyl-azetidin-1-yl)-1,3-dimethyl-benzimidazol-2-one;
[2-tert-butyl-3-[7-(3-hydroxy-3-methyl-azetidin-1-yl)-1,3-dimethyl-2-oxo-benzimidazol-5-yl]-6-(1-methylpyrazol-4-yl)benzimidazol-5-yl] acetate;
6-[6-benzyloxy-2-tert-butyl-5-(2-methylthiazol-5-yl)benzimidazol-1-yl]-4-(3-hydroxy-3-methyl-azetidin-1-yl)-1,3-dimethyl-benzimidazol-2-one;
6-[2-tert-butyl-6-hydroxy-5-(2-methylthiazol-5-yl)benzimidazol-1-yl]-4-(3-hydroxy-3-methyl-azetidin-1-yl)-1,3-dimethyl-benzimidazol-2-one;
6-[2-tert-butyl-6-fluoro-5-(2-methyloxazol-5-yl)benzimidazol-1-yl]-1,3-dimethyl-4-morpholino-benzimidazol-2-one;
tert-butyl N-[2-tert-butyl-3-[7-(3-hydroxy-3-methyl-azetidin-1-yl)-1,3-dimethyl-2-oxo-benzimidazol-5-yl]-6-(1-methylpyrazol-4-yl)benzimidazol-5-yl]carbamate;
6-[6-amino-2-tert-butyl-5-(1-methylpyrazol-4-yl)benzimidazol-1-yl]-4-(3-hydroxy-3-methyl-azetidin-1-yl)-1,3-dimethyl-benzimidazol-2-one;
6-[2-tert-butyl-6-(difluoromethyl)-5-(1-vinylpyrazol-4-yl)benzimidazol-1-yl]-4-[3-(dimethylamino)-3-methyl-azetidin-1-yl]-1,3-dimethyl-benzimidazol-2-one;
6-[2-tert-butyl-6-(difluoromethyl)-5-[1-[2-(dimethylamino)ethyl]pyrazol-4-yl]benzimidazol-1-yl]-4-[3-(dimethylamino)-3-methyl-azetidin-1-yl]-1,3-dimethyl-benzimidazol-2-one;
[1-[6-[2-tert-butyl-6-(difluoromethyl)-5-[1-(2-morpholinoethyl)pyrazol-4-yl]benzimidazol-1-yl]-1,3-dimethyl-2-oxo-benzimidazol-4-yl]-3-methyl-azetidin-3-yl] methanesulfonate;
6-[2-tert-butyl-6-(difluoromethyl)-5-[1-(2-morpholinoethyl)pyrazol-4-yl]benzimidazol-1-yl]-4-(3-hydroxy-3-methyl-azetidin-1-yl)-1,3-dimethyl-benzimidazol-2-one;
6-[6-fluoro-5-(1-methylpyrazol-4-yl)-2-(2-methyl-3-thienyl)benzimidazol-1-yl]-4-isopropyl-1,3-dimethyl-benzimidazol-2-one;
6-[6-fluoro-5-(1-methylpyrazol-4-yl)-2-(3-pyridyl)benzimidazol-1-yl]-4-isopropoxy-1,3-dimethyl-benzimidazol-2-one;
tert-butyl 4-[6-[2-tert-butyl-6-fluoro-5-(1-methylpyrazol-4-yl)benzimidazol-1-yl]-1,3-dimethyl-2-oxo-benzimidazol-4-yl]oxypiperidine-1-carboxylate;
6-[2-tert-butyl-6-fluoro-5-(1-methylpyrazol-4-yl)benzimidazol-1-yl]-1,3-dimethyl-4-(4-piperidyloxy)benzimidazol-2-one;
4-[(1-acetyl-4-piperidyl)oxy]-6-[2-tert-butyl-6-fluoro-5-(1-methylpyrazol-4-yl)benzimidazol-1-yl]-1,3-dimethyl-benzimidazol-2-one;
6-[2-tert-butyl-6-fluoro-5-(1-methylpyrazol-4-yl)benzimidazol-1-yl]-1,3-dimethyl-4-(4-pyridyl)benzimidazol-2-one;
6-[6-fluoro-2-(3-methylimidazol-4-yl)-5-(1-methylpyrazol-4-yl)benzimidazol-1-yl]-1,3-dimethyl-4-(4-pyridyl)benzimidazol-2-one;
6-[6-(difluoromethyl)-2-(3-methylimidazol-4-yl)-5-(1-methylpyrazol-4-yl)benzimidazol-1-yl]-4-[3-hydroxy-3-(trifluoromethyl)azetidin-1-yl]-1,3-dimethyl-benzimidazol-2-one;
6-[6-amino-2-tert-butyl-5-(1-methylpyrazol-4-yl)benzimidazol-1-yl]-4-[(2,3-dihydroxy-2-methyl-propyl)amino]-1,3-dimethyl-benzimidazol-2-one;
6-[6-fluoro-5-methyl-2-(3-methylimidazol-4-yl)benzimidazol-1-yl]-4-(isopropylamino)-1,3-dimethyl-benzimidazol-2-one;
6-[2-tert-butyl-6-(difluoromethyl)-5-(1-methylpyrazol-4-yl)benzimidazol-1-yl]-1,3-dimethyl-4-(4-oxo-1-piperidyl)benzimidazol-2-one;
6-[2-tert-butyl-6-fluoro-5-(1-methylpyrazol-4-yl)benzimidazol-1-yl]-4-(4-hydroxy-4-methyl-1-piperidyl)-1,3-dimethyl-benzimidazol-2-one;
6-[2-tert-butyl-6-(difluoromethyl)-5-(1-methylpyrazol-4-yl)benzimidazol-1-yl]-4-(1,4-dioxa-8-azaspiro[4.5]decan-8-yl)-1,3-dimethyl-benzimidazol-2-one;
6-[2-tert-butyl-6-(difluoromethyl)-5-(1-methylpyrazol-4-yl)benzimidazol-1-yl]-4-(4-hydroxy-1-piperidyl)-1,3-dimethyl-benzimidazol-2-one;
tert-butyl 4-[6-[2-tert-butyl-6-(difluoromethyl)-5-(1-methylpyrazol-4-yl)benzimidazol-1-yl]-1,3-dimethyl-2-oxo-benzimidazol-4-yl]piperazine-1-carboxylate;
tert-butyl N-[1-[6-[2-tert-butyl-6-(difluoromethyl)-5-(1-methylpyrazol-4-yl)benzimidazol-1-yl]-1,3-dimethyl-2-oxo-benzimidazol-4-yl]-3-methyl-azetidin-3-yl]carbamate;
tert-butyl N-[1-[6-[6-(difluoromethyl)-2-(3-methylimidazol-4-yl)-5-(1-methylpyrazol-4-yl)benzimidazol-1-yl]-1,3-dimethyl-2-oxo-benzimidazol-4-yl]-3-methyl-azetidin-3-yl]carbamate;
4-[[[6-[2-tert-butyl-6-(difluoromethyl)-5-(1-methylpyrazol-4-yl)benzimidazol-1-yl]-1,3-dimethyl-2-oxo-benzimidazol-4-yl]amino]methyl]-4-methyl-oxazolidin-2-one;
6-[2-tert-butyl-6-(difluoromethyl)-5-(1-methylpyrazol-4-yl)benzimidazol-1-yl]-1,3-dimethyl-4-piperazin-1-yl-benzimidazol-2-one;
4-(4-acetylpiperazin-1-yl)-6-[2-tert-butyl-6-(difluoromethyl)-5-(1-methylpyrazol-4-yl)benzimidazol-1-yl]-1,3-dimethyl-benzimidazol-2-one;
6-[2-tert-butyl-6-(difluoromethyl)-5-(1-methylpyrazol-4-yl)benzimidazol-1-yl]-1,3-dimethyl-4-(4-methylpiperazin-1-yl)benzimidazol-2-one;
6-(2-tert-butyl-6-fluoro-benzimidazol-1-yl)-4-(isopropylamino)-1,3-dimethyl-benzimidazol-2-one;
6-[2-tert-butyl-6-(difluoromethyl)-5-(1-methylpyrazol-4-yl)benzimidazol-1-yl]-1,3-dimethyl-4-(4-oxocyclohexyl)benzimidazol-2-one;
6-[2-tert-butyl-6-(difluoromethyl)-5-(1-methylpyrazol-4-yl)benzimidazol-1-yl]-4-(4-hydroxycyclohexyl)-1,3-dimethyl-benzimidazol-2-one;
6-[2-tert-butyl-6-(difluoromethyl)-5-(1-methylpyrazol-4-yl)benzimidazol-1-yl]-4-(4-hydroxy-4-methyl-cyclohexyl)-1,3-dimethyl-benzimidazol-2-one;
6-[2-tert-butyl-6-(difluoromethyl)-5-(1-methylpyrazol-4-yl)benzimidazol-1-yl]-4-(4-hydroxy-4-methyl-cyclohexyl)-1,3-dimethyl-benzimidazol-2-one;
6-[2-tert-butyl-6-(difluoromethyl)-5-(1-methylpyrazol-4-yl)benzimidazol-1-yl]-4-(4-hydroxy-4-methyl-1-piperidyl)-1,3-dimethyl-benzimidazol-2-one;
6-[2-tert-butyl-6-(difluoromethyl)-5-(1-methylpyrazol-4-yl)benzimidazol-1-yl]-4-(3-hydroxy-3-methyl-pyrrolidin-1-yl)-1,3-dimethyl-benzimidazol-2-one;
6-[2-tert-butyl-6-(difluoromethyl)-5-(1-methylpyrazol-4-yl)benzimidazol-1-yl]-4-[3-hydroxy-3-(trifluoromethyl)azetidin-1-yl]-1,3-dimethyl-benzimidazol-2-one;
6-[2-tert-butyl-6-chloro-5-(2-methylthiazol-5-yl)benzimidazol-1-yl]-1,3-dimethyl-4-(4-oxo-1-piperidyl)benzimidazol-2-one;
6-[2-tert-butyl-6-chloro-5-(1-methylpyrazol-4-yl)benzimidazol-1-yl]-4-(4-hydroxy-4-methyl-1-piperidyl)-1,3-dimethyl-benzimidazol-2-one;
6-[2-tert-butyl-6-chloro-5-(2-methylthiazol-5-yl)benzimidazol-1-yl]-4-(4-hydroxy-4-methyl-1-piperidyl)-1,3-dimethyl-benzimidazol-2-one;
6-[2-tert-butyl-6-methoxy-5-(1-methylpyrazol-4-yl)benzimidazol-1-yl]-4-(4-hydroxy-4-methyl-1-piperidyl)-1,3-dimethyl-benzimidazol-2-one;
6-[2-tert-butyl-6-methoxy-5-(2-methylthiazol-5-yl)benzimidazol-1-yl]-4-(4-hydroxy-4-methyl-1-piperidyl)-1,3-dimethyl-benzimidazol-2-one;
6-[5-(3,6-dihydro-2H-pyran-4-yl)-6-fluoro-2-(2-methyl-3-thienyl)benzimidazol-1-yl]-1,3-dimethyl-4-tetrahydropyran-4-yl-benzimidazol-2-one;
6-[5-(3,6-dihydro-2H-pyran-4-yl)-6-fluoro-2-(trifluoromethyl)benzimidazol-1-yl]-1,3-dimethyl-4-tetrahydropyran-4-yl-benzimidazol-2-one;
6-[6-(difluoromethyl)-2-(3-methylimidazol-4-yl)-5-(1-methylpyrazol-4-yl)benzimidazol-1-yl]-1,3-dimethyl-4-(4-oxo-1-piperidyl)benzimidazol-2-one;
6-[2-tert-butyl-5-(1-methylpyrazol-4-yl)-6-(trifluoromethyl)benzimidazol-1-yl]-1,3-dimethyl-4-(4-oxo-1-piperidyl)benzimidazol-2-one;
6-[6-(difluoromethyl)-2-(3-methylimidazol-4-yl)-5-(1-methylpyrazol-4-yl)benzimidazol-1-yl]-4-(4-hydroxy-4-methyl-1-piperidyl)-1,3-dimethyl-benzimidazol-2-one;
6-[2-tert-butyl-5-(1-methylpyrazol-4-yl)-6-(trifluoromethyl)benzimidazol-1-yl]-4-(4-hydroxy-4-methyl-1-piperidyl)-1,3-dimethyl-benzimidazol-2-one;
6-[6-(difluoromethyl)-2-(3-methylimidazol-4-yl)-5-(1-methylpyrazol-4-yl)benzimidazol-1-yl]-4-(4-hydroxy-1-piperidyl)-1,3-dimethyl-benzimidazol-2-one;
6-[2-tert-butyl-5-(1-methylpyrazol-4-yl)-6-(trifluoromethyl)benzimidazol-1-yl]-4-(4-hydroxy-1-piperidyl)-1,3-dimethyl-benzimidazol-2-one;
6-[2-tert-butyl-6-(difluoromethyl)-5-(1-methylpyrazol-4-yl)benzimidazol-1-yl]-4-[(2,3-dihydroxy-2-methyl-propyl)amino]-1,3-dimethyl-benzimidazol-2-one;
6-[2-tert-butyl-6-chloro-5-(1-methylpyrazol-4-yl)benzimidazol-1-yl]-4-(4-hydroxy-1-piperidyl)-1,3-dimethyl-benzimidazol-2-one;
6-[2-tert-butyl-6-chloro-5-(2-methylthiazol-5-yl)benzimidazol-1-yl]-4-(4-hydroxy-1-piperidyl)-1,3-dimethyl-benzimidazol-2-one;
6-[2-tert-butyl-6-methoxy-5-(1-methylpyrazol-4-yl)benzimidazol-1-yl]-4-(4-hydroxy-1-piperidyl)-1,3-dimethyl-benzimidazol-2-one;
4-[2-tert-butyl-6-(difluoromethyl)-1-[7-(3-hydroxy-3-methyl-azetidin-1-yl)-1,3-dimethyl-2-oxo-benzimidazol-5-yl]benzimidazol-5-yl]-2-fluoro-N-methyl-benzamide;
6-[2-tert-butyl-6-(difluoromethyl)-5-(2-methoxy-4-pyridyl)benzimidazol-1-yl]-4-(3-hydroxy-3-methyl-azetidin-1-yl)-1,3-dimethyl-benzimidazol-2-one;
6-[2-tert-butyl-6-(difluoromethyl)-5-(3,5-dimethoxyphenyl)benzimidazol-1-yl]-4-(3-hydroxy-3-methyl-azetidin-1-yl)-1,3-dimethyl-benzimidazol-2-one;
6-[5-(4-aminophenyl)-2-tert-butyl-6-(difluoromethyl)benzimidazol-1-yl]-4-(3-hydroxy-3-methyl-azetidin-1-yl)-1,3-dimethyl-benzimidazol-2-one;
N-[4-[2-tert-butyl-6-(difluoromethyl)-1-[7-(3-hydroxy-3-methyl-azetidin-1-yl)-1,3-dimethyl-2-oxo-benzimidazol-5-yl]benzimidazol-5-yl]phenyl]acetamide;
6-[6-(difluoromethyl)-2-(2-methoxyphenyl)-5-(1-methylpyrazol-4-yl)benzimidazol-1-yl]-4-(3-hydroxy-3-methyl-azetidin-1-yl)-1,3-dimethyl-benzimidazol-2-one;
6-[6-(difluoromethyl)-2-(2,3-dimethylphenyl)-5-(1-methylpyrazol-4-yl)benzimidazol-1-yl]-4-(3-hydroxy-3-methyl-azetidin-1-yl)-1,3-dimethyl-benzimidazol-2-one;
[6-6-(difluoromethyl)-5-(1-methylpyrazol-4-yl)-2-pyrimidin-5-yl-benzimidazol-1-yl]-4-(3-hydroxy-3-methyl-azetidin-1-yl)-1,3-dimethyl-benzimidazol-2-one;
6-[6-(difluoromethyl)-2-(2-methoxy-3-pyridyl)-5-(1-methylpyrazol-4-yl)benzimidazol-1-yl]-4-(3-hydroxy-3-methyl-azetidin-1-yl)-1,3-dimethyl-benzimidazol-2-one;
6-[6-(difluoromethyl)-5-(1-methylpyrazol-4-yl)-2-thiazol-5-yl-benzimidazol-1-yl]-4-(3-hydroxy-3-methyl-azetidin-1-yl)-1,3-dimethyl-benzimidazol-2-one.

17. The compound according to claim 1, used as a CBP/p300 inhibitor.

18. An isomer of the compound according to claim 1, a pharmaceutically acceptable salt thereof, a hydrate thereof or a solvate thereof.

19. A pharmaceutical composition for preventing or treating cancer, inflammatory disorders or autoimmune diseases, the composition comprising at least one among the compound according to claim 1, an isomer thereof, a pharmaceutically acceptable salt thereof, a hydrate thereof and a solvate thereof, as an active ingredient.

20. The pharmaceutical composition according to claim 19, wherein the cancer is cancer dependent on oncogene MYC, tumor harboring a loss of function mutation of CBP or p300, or cancer with activation of CBP and/or p300 function.

21. The pharmaceutical composition according to claim 19, wherein the cancer is one or more selected from the group consisting of blood cancer, prostate cancer, breast cancer, lung cancer, pancreatic cancer, colorectal cancer, and melanoma..

22. The pharmaceutical composition according to claim 19, wherein the cancer is one or more selected from the group consisting of acoustic neuroma, acute leukemia, acute lymphoblastic leukemia, acute myeloid leukemia, acute T-cell leukemia, basal cell carcinoma, cholangiocarcinoma, bladder cancer, brain cancer, breast cancer, bronchial carcinoma, cervical cancer, chondrosarcoma, chordoma, choriocarcinoma, chronic leukemia, chronic lymphocytic leukemia, chronic myeloid leukemia, chronic myeloid leukemia, colon cancer, colon cancer, craniopharyngioma, cystadenocarcinoma, diffuse large B-cell lymphoma, dysproliferative changes, embryonal carcinoma, endometrial cancer, endotheliosarcoma, ependymoma, epithelial carcinoma, erythroleukemia, esophageal cancer, estrogen receptor-positive breast cancer, essential thrombocythemia, Ewing's tumor, fibrosarcoma, follicular lymphoma, germ cell testicular cancer, glioma, glioblastoma, pedunculated tumor, heavy chain disease head and neck cancer, hemangioblastoma, hepatoma, hepatocellular carcinoma, hormone-insensitive prostate cancer, leiomyosarcoma, leukemia, liposarcoma, lung cancer, lymphoendotheliosarcoma, lymphangiosarcoma, lymphocytic leukemia, lymphoma, lymphoid malignancy of T-cell or B-cell origin, medullary carcinoma, medulloblastoma, melanoma, meningioma, mesothelioma, multiple myeloma, myeloid leukemia, myeloma, myxosarcoma, neuroblastoma, NUT midline carcinoma (NMC), non-small cell lung cancer, oligodendroglioma, oral cancer, osteosarcoma, ovarian cancer, pancreatic cancer, papillary adenocarcinoma, papillary carcinoma, pinealoma, polycythemia vera, prostate cancer, rectal cancer, renal cell carcinoma, retinoblastoma, rhabdomyosarcoma, sarcoma, sebaceous carcinoma, seminoma, skin cancer, small cell lung carcinoma, solid tumors (carcinomas and sarcomas), small cell lung cancer, stomach cancer, squamous cell carcinoma, synovioma, sweat gland carcinoma, thyroid cancer, Waldenstrom macroglobulinemia, testicular tumor, uterine cancer and Wilms tumor.

23. The pharmaceutical composition according to claim 19, wherein the inflammatory disorder or autoimmune disease is one or more selected from the group consisting of Addison's disease, acute gout, ankylosing spondylitis, asthma, atherosclerosis, Behcet's disease, bullous skin disease, chronic obstructive pulmonary disease, Crohn's disease, dermatitis, eczema, giant cell arteritis, fibrosis, glomerulonephritis, hepatic vascular occlusion, hepatitis, hypophysitis, immunodeficiency syndrome, inflammatory bowel disease, Kawasaki disease, lupus nephritis, multiple sclerosis, myocarditis, myositis, nephritis, organ transplant rejection, osteoarthritis, pancreatitis, pericarditis, polyarteritis nodosa pneumonia, primary biliary cirrhosis, psoriasis, psoriatic arthritis, rheumatoid arthritis, scleritis, sclerosing cholangitis, sepsis, systemic lupus erythematosus, Takayasu's arteritis, toxic shock, thyroiditis, type 1 diabetes, ulcerative colitis, uveitis, vitiligo, vasculitis, and Wegener's granulomatosis.
